(19) Europäisches Patentamt
European Patent Office
Office européen des brevets

(11) EP 3 558 391 B1

(12) **EUROPEAN PATENT SPECIFICATION**

(45) Date of publication and mention of the grant of the patent:
**02.02.2022 Bulletin 2022/05**

(21) Application number: **17835533.5**

(22) Date of filing: **21.12.2017**

(51) International Patent Classification (IPC):
***A61K 47/68*** *(2017.01)* ***A61P 35/00*** *(2006.01)*
***C07K 16/40*** *(2006.01)* ***C07K 16/28*** *(2006.01)*
***C07K 16/30*** *(2006.01)*

(52) Cooperative Patent Classification (CPC):
**A61K 47/6871; A61K 47/6803; A61P 35/00; A61P 35/02; C07K 16/2896; C07K 16/30; C07K 16/40;** C07K 2317/24; C07K 2317/33; C07K 2317/524; C07K 2317/526; C07K 2317/71; C07K 2317/72; C07K 2317/73; C07K 2317/76;
(Cont.)

(86) International application number:
**PCT/US2017/067823**

(87) International publication number:
**WO 2018/119196 (28.06.2018 Gazette 2018/26)**

(54) **IMMUNOCONJUGATES TARGETING ADAM9 AND METHODS OF USE THEREOF**

AUF ADAM9 GERICHTETE IMMUNOKONJUGATE UND VERFAHREN ZUR VERWENDUNG DAVON

IMMUNOCONJUGUÉS CIBLANT L'ADAM9 ET LEURS MÉTHODES D'UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD TN**

(30) Priority: **23.12.2016 US 201662438488 P**
**31.03.2017 US 201762480201 P**

(43) Date of publication of application:
**30.10.2019 Bulletin 2019/44**

(73) Proprietors:
- **Immunogen, Inc.**
  **Waltham, MA 02451 (US)**
- **MacroGenics, Inc.**
  **Rockville, MD 20850 (US)**

(72) Inventors:
- **HICKS, Stuart William**
  **North Andover, MA 01845 (US)**
- **YODER, Nicholas C.**
  **Brookline, MA 02446 (US)**
- **BARAT, Bhaswati**
  **Derwood, MD 20855 (US)**
- **BONVINI, Ezio**
  **Potomac, Maryland 20854 (US)**
- **DIEDRICH, Gundo**
  **North Potomac, MD 20878 (US)**
- **JOHNSON, Leslie S.**
  **Darnestown, MD 20874 (US)**
- **LOO, Deryk**
  **Belmont, CA 94002 (US)**
- **SCRIBNER, Juniper A.**
  **Burlingame, CA 94010 (US)**

(74) Representative: **Gibbs, Richard**
**Marks & Clerk LLP**
**Aurora**
**120 Bothwell Street**
**Glasgow G2 7JS (GB)**

(56) References cited:
**DE-A1- 10 337 368** **US-A1- 2009 285 840**
**US-A1- 2016 095 938**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

- **STUART W HICKS ET AL: "Abstract #37: Novel Antibody-Drug Conjugates Targeting ADAM9-expressing Solid Tumors Demonstrate Potent Preclinical Activity Introduction", PROCEEDINGS OF THE AMERICAN ASSOCIATION FOR CANCER RESEARCH ANNUAL MEETING 2017, vol. 77, no. 13 Suppl, 1 April 2017 (2017-04-01), XP055464251, DOI: 10.1158/1538-7445.AM2017-37 -& STUART W HICKS ET AL: "Abstract 37: Novel antibody-drug conjugates targeting ADAM9-expressing solid tumors demonstrate potent preclinical activity | Cancer Research", PROCEEDINGS OF THE AMERICAN ASSOCIATION FOR CANCER RESEARCH ANNUAL MEETING 2017, vol. 77, no. 13 Suppl, 1 July 2017 (2017-07-01), XP055464261, DOI: 10.1158/1538-7445.AM2017-37**
- **JUNIPER A. SCRIBNER ET AL: "Abstract 38: Target validation, antibody discovery and preclinical data supporting ADAM9 as an antibody-drug conjugate therapeutic target for solid tumors", CANCER RESEARCH, vol. 77, no. 13 Supplement, 1 April 2017 (2017-04-01), pages 38-38, XP055464265, US ISSN: 0008-5472, DOI: 10.1158/1538-7445.AM2017-38 -& STUART W HICKS ET AL: "Abstract 38: Target validation, antibody discovery and preclinical data supporting ADAM9 as an antibody-drug conjugate therapeutic target for solid tumors | Cancer Research", PROCEEDINGS OF THE AMERICAN ASSOCIATION FOR CANCER RESEARCH ANNUAL MEETING 2017, vol. 77, no. 13 Suppl, 1 July 2017 (2017-07-01), XP055464262, DOI: 10.1158/1538-7445.AM2017-38**


(52) Cooperative Patent Classification (CPC): (Cont.)
C07K 2317/77

## Description

## FIELD OF THE INVENTION

The present invention is directed to immunoconjugates comprising an antibody or fragment thereof capable of specifically binding to "Disintegrin and Metalloproteinase Domain-containing Protein 9" **("ADAM9")** conjugated to at least one pharmacological agent. The invention particularly concerns such immunoconjugates that are cross-reactive with human ADAM9 and the ADAM9 of a non-human primate (such as a cynomolgus monkey). The invention additionally pertains to all such immunoconjugates that comprise a Light Chain Variable (VL) Domain and/or a Heavy Chain Variable (VH) Domain that has been humanized and optionally deimmunized so as to exhibit reduced immunogenicity upon administration of such immunoconjugates to a recipient subject. The invention is also directed to pharmaceutical compositions that contain any of such immunoconjugates, and to immunoconjugates and pharmaceutical compositions for use in the treatment of a disease or condition associated with, or characterized by, the expression of ADAM9 (such as cancer).

## BACKGROUND OF THE INVENTION

ADAM is a family of proteins involved in various physiologic and pathologic processes (Amendola, R.S. et al. (2015) "ADAM9 Disintegrin Domain Activates Human Neutrophils Through An Autocrine Circuit Involving Integrins And CXCR2," J. Leukocyte Biol. 97(5):951-962; Edwars, D.R. et al. (2008) "The ADAM Metalloproteases," Molec. Aspects Med. 29:258-289). At least 40 gene members of the family have been identified, and at least 21 of such members are believed to be functional in humans (Li, J. et al. (2016) "Overexpression of ADAM9 Promotes Colon Cancer Cells Invasion," J. Invest. Surg. 26(3):127-133; Duffy, M.J. et al. (2011) "The ADAMs Family Of Proteases: New Biomarkers And Therapeutic Targets For Cancer?," Clin. Proteomics 8:9:1-13; see also US Patent Publication No. 2013/0045244).

ADAM family members have a well-conserved structure with 8 domains, among which are a metalloprotease domain and an integrin-binding (disintegrin) domain (Duffy, M.J. et al. (2009) "The Role Of ADAMs In Disease Pathophysiology," Clin. Chim. Acta 403:31-36). The ADAM metalloprotease domain acts as a sheddase and has been reported to modulate a series of biologic processes by cleaving transmembrane proteins, which then can act as soluble ligands and regulate cellular signaling (Amendola, R.S. et al. (2015) "ADAM9 Disintegrin Domain Activates Human Neutrophils Through An Autocrine Circuit Involving Integrins And CXCR2," J. Leukocyte Biol. 97(5):951-962; Ito, N. et al. (2004) "ADAMs, A Disintegrin And Metalloproteinases, Mediate Shedding Of Oxytocinase," Biochem. Biophys. Res. Commun. 314 (2004) 1008-1013).

ADAM9 is a member of the ADAM family of molecule. It is synthesized as an inactive form which is proteolytically cleaved to generate an active enzyme. Processing at the upstream site is particularly important for activation of the proenzyme. ADAM9 is expressed in fibroblasts (Zigrino, P. et al. (2011) "The Disintegrin-Like And Cysteine-Rich Domains Of ADAM-9 Mediate Interactions Between Melanoma Cells And Fibroblasts," J. Biol. Chem. 286:6801-6807), activated vascular smooth muscle cells (Sun, C. et al. (2010) "ADAM15 Regulates Endothelial Permeability And Neutrophil Migration Via Src/ERK1/2 Signalling," Cardiovasc. Res. 87:348-355), monocytes (Namba, K. et al. (2001) "Involvement Of ADAM9 In Multinucleated Giant Cell Formation Of Blood Monocytes," Cell. Immunol. 213:104-113), activated macrophages (Oksala, N. et al. (2009) "ADAM-9, ADAM-15, And ADAM-17 Are Upregulated In Macrophages In Advanced Human Atherosclerotic Plaques In Aorta And Carotid And Femoral Arteries - Tampere Vascular Study," Ann. Med. 41:279-290).

ADAM9's metalloprotease activity participates in the degradation of matrix components, to thereby allow migration of tumor cells (Amendola, R.S. et al. (2015) "ADAM9 Disintegrin Domain Activates Human Neutrophils Through An Autocrine Circuit Involving Integrins And CXCR2," J. Leukocyte Biol. 97(5):951-962). Its disintegrin domain, which is highly homologous to many snake-venom disintegrins, allows the interaction between ADAM9 and integrins, and enables ADAM9 to modulate, positively or negatively, cell adhesion events (Zigrino, P. et al. (2011) "The Disintegrin-Like And Cysteine-Rich Domains Of ADAM-9 Mediate Interactions Between Melanoma Cells And Fibroblasts," J. Biol. Chem. 286:6801-6807; Karadag, A. et al. (2006) "ADAM-9 (MDC-9/Meltringamma), A Member Of The A Disintegrin And Metalloproteinase Family, Regulates Myeloma-Cell-Induced Interleukin-6 Production In Osteoblasts By Direct Interaction With The Alpha(v)Beta5 Integrin," Blood 107:3271-3278; Cominetti, M.R. et al. (2009) "Inhibition Of Platelets And Tumor Cell Adhesion By The Disintegrin Domain Of Human ADAM9 To Collagen I Under Dynamic Flow Conditions," Biochimie, 91:1045-1052). The ADAM9 disintegrin domain has been shown to interact with the $\alpha6\beta1$, $\alpha6\beta4$, $\alpha v\beta5$ and $\alpha9\beta1$ integrins.

The expression of ADAM9 has been found to be relevant to disease, especially cancer. ADAM9 has been found to cleave and release a number of molecules with important roles in tumorigenesis and angiogenesis, such as TEK, KDR, EPHB4, CD40, VCAM1 and CDH5. ADAM9 is expressed by many types of tumor cells, including tumor cells of breast cancers, colon cancers, gastric cancers, gliomas, liver cancers, non-small cell lung cancers, melanomas, myelomas, pancreatic cancers and prostate cancers (Yoshimasu, T. et al. (2004) "Overexpression Of ADAM9 In Non-Small

Cell Lung Cancer Correlates With Brain Metastasis," Cancer Res. 64:4190-4196; Peduto, L. et al. (2005) "Critical Function For ADAM9 In Mouse Prostate Cancer," Cancer Res. 65:9312-9319; Zigrino, P. et al. (2005) "ADAM-9 Expression And Regulation In Human Skin Melanoma And Melanoma Cell Lines," Int. J. Cancer 116:853-859; Fritzsche, F.R. et al. (2008) "ADAM9 Is Highly Expressed In Renal Cell Cancer And Is Associated With Tumour Progression," BMC Cancer 8:179:1-9; Fry, J.L. et al. (2010) "Secreted And Membrane-Bound Isoforms Of Protease ADAM9 Have Opposing Effects On Breast Cancer Cell Migration," Cancer Res. 70, 8187-8198; Chang, L. et al. (2016) "Combined Rnai Targeting Human Stat3 And ADAM9 As Gene Therapy For Non-Small Cell Lung Cancer," Oncology Letters 11:1242-1250; Fan, X. et al. (2016) "ADAM9 Expression Is Associate with Glioma Tumor Grade and Histological Type, and Acts as a Prognostic Factor in Lower-Grade Gliomas," Int. J. Mol. Sci. 17:1276:1-11).

Significantly, increased ADAM9 expression has been found to correlate positively with tumor malignancy and metastatic potential (Amendola, R.S. et al. (2015) "ADAM9 Disintegrin Domain Activates Human Neutrophils Through An Autocrine Circuit Involving Integrins And CXCR2," J. Leukocyte Biol. 97(5):951-962; Fan, X. et al. (2016) "ADAM9 Expression Is Associate with Glioma Tumor Grade and Histological Type, and Acts as a Prognostic Factor in Lower-Grade Gliomas," Int. J. Mol. Sci. 17:1276:1-11; Li, J. et al. (2016) "Overexpression of ADAM9 Promotes Colon Cancer Cells Invasion," J. Invest. Surg. 26(3):127-133). Additionally, ADAM9 and its secreted soluble isoform seem to be crucial for cancer cells to disseminate (Amendola, R.S. et al. (2015) "ADAM9 Disintegrin Domain Activates Human Neutrophils Through An Autocrine Circuit Involving Integrins And CXCR2," J. Leukocyte Biol. 97(5):951-962; Fry, J.L. et al. (2010) "Secreted And Membrane-Bound Isoforms Of Protease ADAM9 Have Opposing Effects On Breast Cancer Cell Migration," Cancer Res. 70, 8187-8198; Mazzocca, A. (2005) "A Secreted Form OfADAM9 Promotes Carcinoma Invasion Through Tumor-Stromal Interactions," Cancer Res. 65:4728-4738; see also US Patent Nos. 9,150,656; 7,585,634; 7,829,277; 8,101,361; and 8,445,198 and US Patent Publication No. 2009/0023149).

A number of studies have thus identified ADAM9 as a potential target for anticancer therapy (Peduto, L. (2009) "ADAM9 As A Potential Target Molecule In Cancer," Curr. Pharm. Des. 15:2282-2287; Duffy, M.J. et al. (2009) "Role Of ADAMs In Cancer Formation And Progression" Clin. Cancer Res. 15:1140-1144; Duffy, M.J. et al. (2011) "The ADAMs Family Of Proteases: New Biomarkers And Therapeutic Targets For Cancer?," Clin. Proteomics 8:9:1-13; Josson, S. et al. (2011) "Inhibition of ADAM9 Expression Induces Epithelial Phenotypic Alterations and Sensitizes Human Prostate Cancer Cells to Radiation and Chemotherapy," Prostate 71(3):232-240; see also US Patent Publication Nos. 2016/0138113, 2016/0068909, 2016/0024582, 2015/0368352, 2015/0337356, 2015/0337048, 2015/0010575, 2014/0342946, 2012/0077694, 2011/0151536, 2011/0129450, 2010/0291063, 2010/0233079, 2010/0112713, 2009/0203051, 2004/0092466, 2003/0091568, and 2002/0068062, and PCT Publication Nos. WO 2016/077505, WO 2014/205293, WO 2014/186364, WO 2014/124326, WO 2014/108480, WO 2013/119960, WO 2013/098797, WO 2013/049704, and WO 2011/100362). US2009/0285840A1 describes compositions and methods that inhibit expression of Adam9 gene products, such as ADAM9 mRNA and/or ADAM9 polypeptides, as a therapeutic approach for the treatment of pathological neovascularization and conditions associated with angiogenesis. DE10337368A1 describes diagnosis of pancreatic cancer by detecting expression of ADAM9 protein using specific antibodies and also the therapeutic use of these antibodies and of ADAM9-specific nucleic acids. Additionally, the expression of ADAM9 has also been found to be relevant to pulmonary disease and inflammation (see, *e.g.,* US Patent Publication Nos. 2016/0068909; 2012/0149595; 2009/0233300; 2006/0270618; and 2009/0142301). Antibodies that bind to ADAM9 are commercially available from Abcam, Thermofisher, Sigma-Aldrich, and other companies.

However, despite all prior advances, a need remains for high affinity ADAM9 targeting immunoconjugates that exhibit minimal binding to normal tissues and are capable binding to human and non-human ADAM9 with similar high affinity. The present invention addresses this need and the need for improved therapeutics for cancer.

## SUMMARY OF THE INVENTION

According to an aspect of the invention there is provided immunoconjugates comprising an antibody or fragment thereof capable of specifically binding to "Disintegrin and Metalloproteinase Domain-containing Protein 9" **("ADAM9")** conjugated to at least one pharmacological agent as defined in claim 1. The immunoconjugates are cross-reactive with human ADAM9 and the ADAM9 of a non-human primate (a cynomolgus monkey). The immunoconjugates comprise a Light Chain Variable (VL) Domain and/or a Heavy Chain Variable (VH) Domain that have been humanized (and optionally deimmunized) so as to exhibit reduced immunogenicity upon administration of such immunoconjugates to a recipient subject. According to further aspects of the invention there are provided a pharmaceutical composition as defined in claim 15, and an immunoconjugate or pharmaceutical composition for use in the treatment of a disease or condition associated with, or characterized by, the expression of ADAM9 (such as cancer) as defined in claims 16 and 17..

In detail, the present invention provides an immunoconjugate comprising a humanized anti-ADAM9 antibody or ADAM9-binding fragment thereof that specifically binds to human ADAM9 and cyno ADAM9:

(I) wherein said humanized anti-ADAM9 antibody or ADAM9-binding fragment thereof is conjugated to a pharmacological agent; and

(II) wherein said humanized anti-ADAM9 antibody or ADAM9-binding fragment thereof comprises a Light Chain Variable (VL) Domain and a Heavy Chain Variable (VH) Domain, wherein said Heavy Chain Variable Domain comprises a $CDR_H1$ Domain, a $CDR_H2$ Domain and a $CDR_H3$ Domain, and said Light Chain Variable Domain comprises a $CDR_L1$ Domain, a $CDR_L2$ Domain, and a $CDR_L3$ Domain, wherein:

(A) said $CDR_H1$ Domain comprises the amino acid sequence of SEQ ID NO:8 or SEQ ID NO:34; and

(B) said $CDR_H2$ Domain comprises the amino acid sequence of SEQ ID NO: 9, SEQ ID NO:35 or SEQ ID NO:36; and

(C) said $CDR_H3$ Domain comprises amino acid sequence of any one of SEQ ID NO:37, SEQ ID NO:38, SEQ ID NO:39, SEQ ID N0:40, SEQ ID NO:41, SEQ ID NO:42, SEQ ID NO:43, SEQ ID NO:44, SEQ ID NO:45 or SEQ ID NO:46; and

wherein:

(A) said $CDR_L1$ Domain has the amino acid sequence of SEQ ID NO:12, SEQ ID NO:62, SEQ ID NO:63, or SEQ ID NO:64; and

(B) said $CDR_L2$ Domain has the amino acid sequence of SEQ ID NO: 13; and

(C) said $CDR_L3$ Domain has the amino acid sequence of SEQ ID NO: 14 or SEQ ID NO:65.

In certain embodiments, the Heavy Chain Variable (VH) Domain of the optimized variant of MAB-A is selected from the group consisting of:

(5) hMAB-A VH(2A) **(SEQ ID NO:20);**
(6) hMAB-A VH(2B) **(SEQ ID NO:21);**
(7) hMAB-A VH(2C) **(SEQ ID NO:22);**
(8) hMAB-A VH(2D) **(SEQ ID NO:23);**
(9) hMAB-A VH(2E) **(SEQ ID NO:24);**
(10) hMAB-A VH(2F) **(SEQ ID NO:25);**
(11) hMAB-A VH(2G) **(SEQ ID NO:26);**
(12) hMAB-A VH(2H) **(SEQ ID NO:27);**
(13) hMAB-A VH(2I) **(SEQ ID NO:28);** and
(14) hMAB-A VH(2J) **(SEQ ID NO:29).**

In certain embodiments, the Light Chain Variable (VL) Domain of the optimized variant of MAB-A is selected from the group consisting of:

(1) hMAB-A VL(1) **(SEQ ID NO:54);**
(2) hMAB-A VL(2) **(SEQ ID NO:55);**
(3) hMAB-A VL(3) **(SEQ ID NO:56);**
(4) hMAB-A VL(4) **(SEQ ID NO:57).**

In certain embodiments, the $CDR_H1$ Domain comprises the amino acid sequence SYWMH **(SEQ ID NO:8),** the $CDR_H2$ Domain comprises the amino acid sequence E I I P I FGHTNYNEKFKS **(SEQ ID NO:35),** and the $CDR_H3$ Domain comprises the amino acid sequence GGYYYYPRQGFLDY **(SEQ ID NO:45)**
In certain embodiments, the $CDR_L1$ Domain comprises the amino acid sequence KASQSVDYSGDSYMN **(SEQ ID NO:62),** the $CDR_L2$ Domain comprises the amino acid sequence AASDLES **(SEQ ID NO:13),** and the $CDR_L3$ Domain comprises the amino acid sequence QQSHEDPFT **(SEQ ID NO:14).**
In certain embodiments, the immunoconjugate comprises:

(A) the Heavy Chain Variable (VH) Domain of hMAB-A **(2I.2) (SEQ ID NO:28);** or
(B) the Light Chain Variable (VL) Domain of hMAB-A **(2I.2) (SEQ ID NO:55);** or
(C) the Heavy Chain Variable (VH) Domain of hMAB-A **(2I.2) (SEQ ID NO:28)** and the Light Chain Variable (VL)

Domain of hMAB-A **(2I.2) (SEQ ID NO:55)**

In certain embodiments, the immunoconjugate comprises an Fc Region. In some embodiments, the Fc Region is a variant Fc Region that comprises: (a) one or more amino acid modification(s) that reduce(s) the affinity of the variant Fc Region for an FcγR; and/or (b) one or more amino acid modification(s) that introduces a cysteine residue. In some embodiments, the one or more amino acid modification(s) that reduce(s) the affinity of the variant Fc Region for an FcγR comprise: (A) L234A; (B) L235A; or (C) L234A and L235A; wherein said numbering is that of the EU index as in Kabat. In some embodiments, the one or more amino acid modification(s) that that introduces a cysteine residue comprises S442C, wherein said numbering is that of the EU index as in Kabat.

The immunoconjugate of the present invention comprises a humanized anti-ADAM9 antibody or ADAM9-binding fragment thereof that specifically binds to human ADAM9 and cyno ADAM9, wherein the humanized anti-ADAM9 antibody or ADAM9-binding fragment thereof is conjugated to the pharmacological agent.

In certain embodiments, the humanized anti-ADAM9 antibody or ADAM9-binding fragment thereof is optimized to have at least a 100-fold enhancement in binding affinity to cyno ADAM9 and retains high affinity binding to human ADAM9 as compared to the chimeric or murine parental antibody.

In certain embodiments, the anti-ADAM9 antibody or ADAM9-binding fragment thereof comprises a $CDR_H1$ domain, a $CDR_H2$ domain, and a $CDR_H3$ domain and a $CDR_L1$ domain, a $CDR_L2$ domain, and a $CDR_L3$ domain having the sequences selected from the group consisting of:

(a) SEQ ID NOs: 8, 35, and 37 and SEQ ID NOs: 62, 13, 14, respectively;
(b) SEQ ID NOs: 8, 35, and 38 and SEQ ID NOs: 62, 13, 14, respectively;
(c) SEQ ID NOs: 8, 35, and 39 and SEQ ID NOs: 62, 13, 14, respectively;
(d) SEQ ID NOs: 8, 35, and 40 and SEQ ID NOs: 62, 13, 14, respectively;
(e) SEQ ID NOs: 8, 35, and 41 and SEQ ID NOs: 62, 13, 14, respectively;
(f) SEQ ID NOs: 8, 35, and 42 and SEQ ID NOs: 62, 13, 14, respectively;
(g) SEQ ID NOs: 8, 35, and 43 and SEQ ID NOs: 62, 13, 14, respectively;
(h) SEQ ID NOs: 8, 35, and 44 and SEQ ID NOs: 62, 13, 14, respectively;
(i) SEQ ID NOs: 8, 35, and 45 and SEQ ID NOs: 62, 13, 14, respectively; and
(j) SEQ ID NOs: 8, 35, and 46 and SEQ ID NOs: 62, 13, 14, respectively.

In certain embodiments, the humanized anti-ADAM9 antibody or ADAM9-binding fragment thereof comprises a heavy chain variable domain (VH) and a light chain variable domain (VL) having sequences that are at least 90%, at least 95%, or at least 99% identical to sequences selected from the group consisting of:

(a) SEQ ID NO:20 and SEQ ID NO:55, respectively;
(b) SEQ ID NO:21 and SEQ ID NO:55, respectively;
(c) SEQ ID NO:22 and SEQ ID NO:55, respectively;
(d) SEQ ID NO:23 and SEQ ID NO:55, respectively;
(e) SEQ ID NO:24 and SEQ ID NO:55, respectively;
(f) SEQ ID NO:25 and SEQ ID NO:55, respectively;
(g) SEQ ID NO:26 and SEQ ID NO:55, respectively;
(h) SEQ ID NO:27 and SEQ ID NO:55, respectively;
(i) SEQ ID NO:28 and SEQ ID NO:55, respectively; and
(j) SEQ ID NO:29 and SEQ ID NO:55, respectively.

In certain embodiments, the humanized anti-ADAM9 antibody or ADAM9-binding fragment thereof comprises a heavy chain variable domain (VH) and a light chain variable domain (VL) having the sequences selected from the group consisting of:

(a) SEQ ID NO:20 and SEQ ID NO:55, respectively;
(b) SEQ ID NO:21 and SEQ ID NO:55, respectively;
(c) SEQ ID NO:22 and SEQ ID NO:55, respectively;
(d) SEQ ID NO:23 and SEQ ID NO:55, respectively;
(e) SEQ ID NO:24 and SEQ ID NO:55, respectively;
(f) SEQ ID NO:25 and SEQ ID NO:55, respectively;
(g) SEQ ID NO:26 and SEQ ID NO:55, respectively;
(h) SEQ ID NO:27 and SEQ ID NO:55, respectively;
(i) SEQ ID NO:28 and SEQ ID NO:55, respectively; and

(j) SEQ ID NO:29 and SEQ ID NO:55, respectively.

In certain embodiments, the humanized anti-ADAM9 antibody is a full length antibody comprising an Fc region. In some embodiments, the Fc region is a variant Fc region that comprises: (a) one or more amino acid modification(s) that reduces(s) the affinity of the variant Fc region for an FcγR selected from the group consisting of: L234A, L235A, and L234A and L235A; and/or (b) an amino acid modification that introduces a cysteine residue at S442, wherein said numbering is that of the EU index as in Kabat.

In some embodiments, the humanized anti-ADAM9 antibody comprises a heavy chain and a light chain having the sequences selected from the group consisting of:

(a) SEQ ID NO:51 and SEQ ID NO:68, respectively; and
(b) SEQ ID NO:52 and SEQ ID NO:68, respectively.

In certain embodiments, the humanized anti-ADAM9 antibody comprises a heavy chain and a light chain having the sequences selected from the group consisting of:

(a) SEQ ID NO:141 and SEQ ID NO:68, respectively;
(b) SEQ ID NO:142 and SEQ ID NO:68, respectively;
(c) SEQ ID NO:143 and SEQ ID NO:68, respectively;
(d) SEQ ID NO:151 and SEQ ID NO:68, respectively;
(e) SEQ ID NO:152 and SEQ ID NO:68, respectively;
(f) SEQ ID NO:153 and SEQ ID NO:68, respectively; and
(g) SEQ ID NO:154 and SEQ ID NO:68, respectively.

In certain embodiments, the immunoconjugate of the present invention is represented by the following formula:

$$\mathrm{CBA}\!\left(\!-\mathrm{Cy}^{L1}\right)_{W_L},$$

wherein:

CBA is an anti-ADAM9 antibody or ADAM9-binding fragment thereof as described herein that is covalently linked to $Cy^{L1}$ through a lysine residue;
$W_L$ is an integer from 1 to 20; and
$Cy^{L1}$ is represented by the following formula:

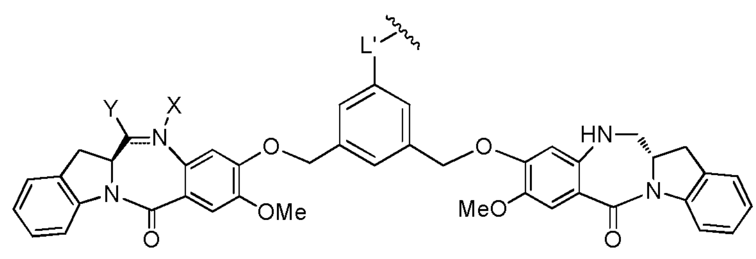

, or

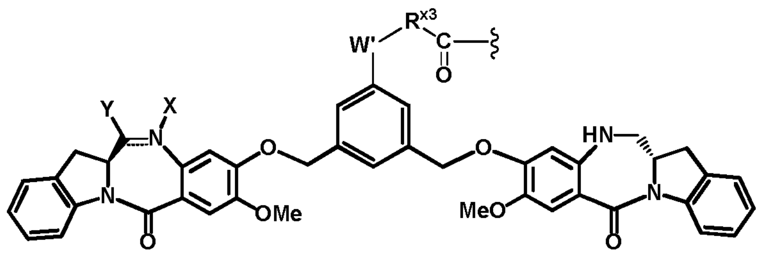

or a pharmaceutically acceptable salt thereof, wherein:

the double line == between N and C represents a single bond or a double bond, provided that when it is a double bond, X is absent and Y is -H or a $(C_1-C_4)$alkyl; and when it is a single bond, X is -H or an amine protecting moiety, and Y is -OH or $-SO_3H$ or a pharmaceutically acceptable salt thereof;

W' is $-NR^{e'}$,

$R^{e'}$ is $-(CH_2-CH_2-O)_n-R^k$;

n is an integer from 2 to 6;

$R^k$ is -H or -Me;

$R^{x3}$ is a $(C_1-C_6)$alkyl;

L' is represented by the following formula:

- $NR_5$-P-C(=O)-$(CR_aR_b)_m$-C(=O)- (B1'); or
- $NR_5$-P-C(=O)-$(CR_aR_b)_m$-S-$Z^{s1}$- (B2');

$R_5$ is -H or a $(C_1-C_3)$alkyl;

P is an amino acid residue or a peptide containing between 2 to 20 amino acid residues;

$R_a$ and $R_b$, for each occurrence, are each independently -H, $(C_1-C_3)$alkyl, or a charged substituent or an ionizable group Q;

m is an integer from 1 to 6; and

$Z^{s1}$ is selected from any one of the following formulas:

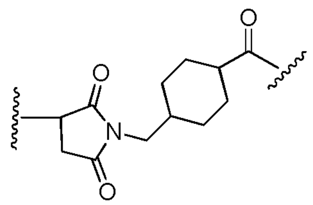 (b1);
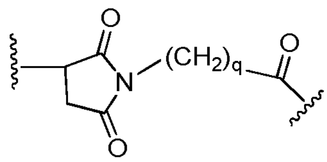 (b2);
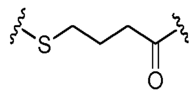 (b3);

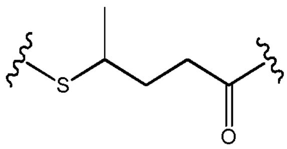 (b4);
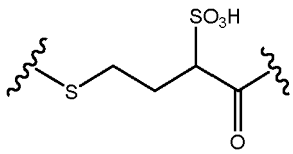 (b5),

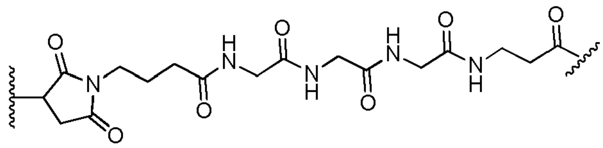 (b6),

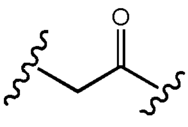 (b7);
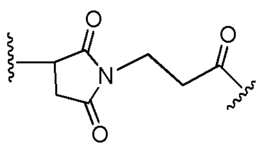 (b8);
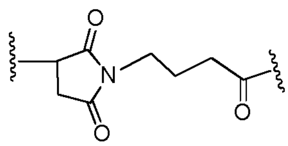 (b9); and

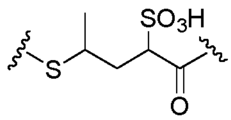 (b10),

wherein q is an integer from 1 to 5.

In certain embodiments, the immunoconjugate of the present invention is represented by the following formula:

$$CBA-\left(-Cy^{L2}\right)_{W_L},$$

wherein:

CBA is an anti-ADAM9 antibody or ADAM9-binding fragment thereof as described herein that is covalently linked to $Cy^{L2}$ through a lysine residue;
$W_L$ is an integer from 1 to 20; and
$Cy^{L2}$ is represented by the following formula:

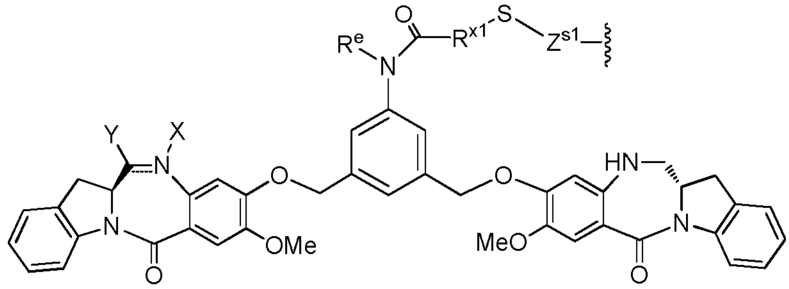

;

or

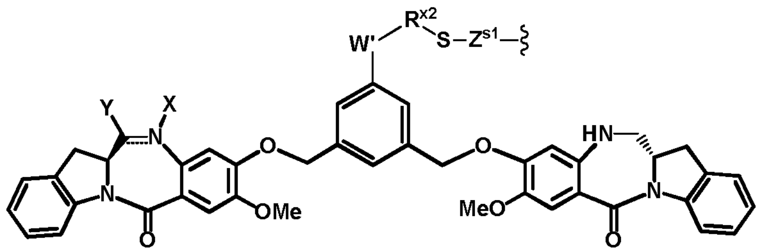

;

or a pharmaceutically acceptable salt thereof, wherein:
the double line

$$=\!=$$

between N and C represents a single bond or a double bond, provided that when it is a double bond, X is absent and Y is -H or a $(C_1\text{-}C_4)$alkyl; and when it is a single bond, X is -H or an amine protecting moiety, and Y is -OH or $-SO_3H$;
$R^{x1}$ and $R^{x2}$ are independently $(C_1\text{-}C_6)$alkyl;
$R^e$ is -H or a $(C_1\text{-}C_6)$alkyl;
W' is $-NR^{e'}$,
$R^{e'}$ is $-(CH_2\text{-}CH_2\text{-}O)_n\text{-}R^k$;
n is an integer from 2 to 6;
$R^k$ is -H or -Me;
$Z^{s1}$ is selected from any one of the following formulas:

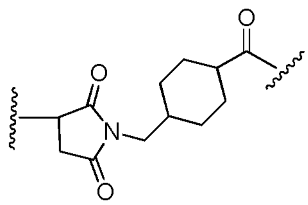
(b1);
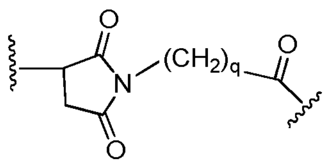
(b2);
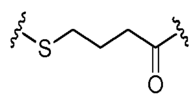
(b3);

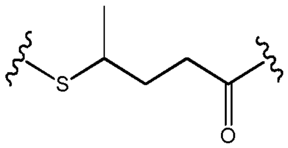 (b4); 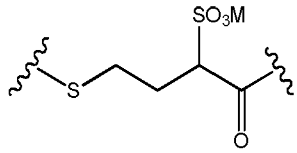 (b5),

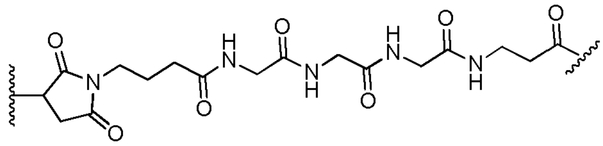 (b6),

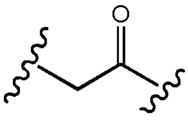 (b7); 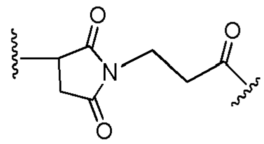 (b8); 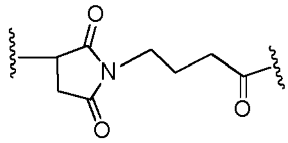 (b9); and

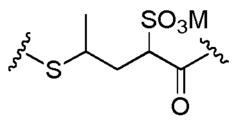 (b10),

wherein q is an integer from 1 to 5.

In certain embodiments, the immunoconjugate of the present invention is represented by the following formula:

$$CBA\left(-Cy^{L3}\right)_{W_L},$$

wherein:

CBA is an anti-ADAM9 antibody or ADAM9-binding fragment thereof as described herein that is covalently linked to $Cy^{L3}$ through a lysine residue;

$W_L$ is an integer from 1 to 20;

$Cy^{L3}$ is represented by the following formula:

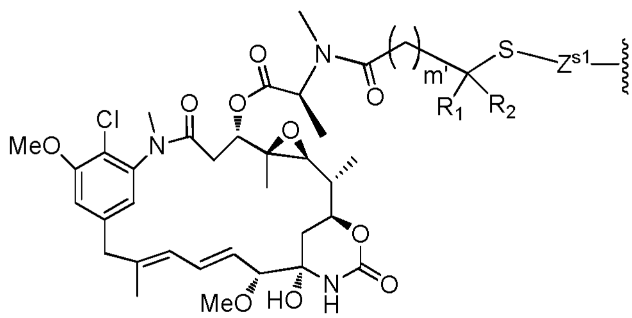

,

m' is 1 or 2;

$R_1$ and $R_2$, are each independently H or a $(C_1\text{-}C_3)$alkyl; and

$Z^{s1}$ is selected from any one of the following formulas:

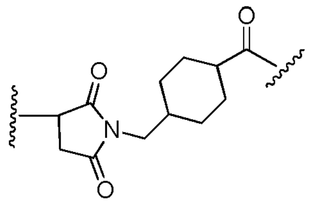 (b1); 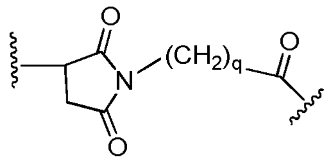 (b2); 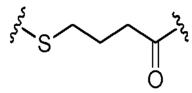 (b3);

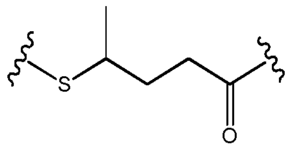 (b4); 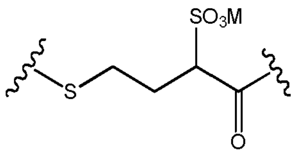 (b5),

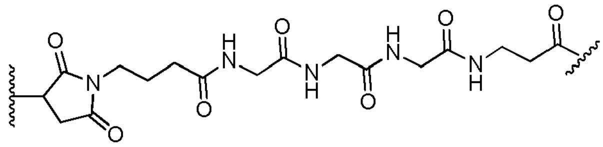 (b6),

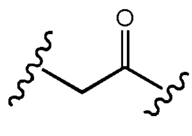 (b7); 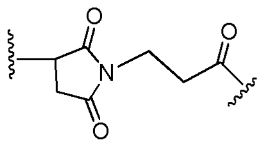 (b8); 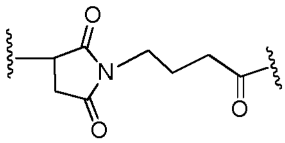 (b9),

and

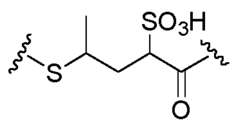 (b10),

wherein q is an integer from 1 to 5.

In certain embodiments, the immunoconjugate of the present invention comprises an humanized anti-ADAM9 antibody or ADAM9-binding fragment thereof linked to the maytansinoid DM4 via *N*-succinimidyl-4-(2-pyridyldithio)2-sulfo butanoate (sulfo-SPDB), wherein an humanized anti-ADAM9 antibody or ADAM9-binding fragment thereof comprising a $CDR_H1$ domain, a $CDR_H2$ domain, and a $CDR_H3$ domain and a $CDR_L1$ domain, a $CDR_L2$ domain, and a $CDR_L3$ domain having the sequences of SEQ ID NOs: 8, 35, and 45 and SEQ ID NOs: 62, 13, 14, respectively. In certain embodiments, the humanized anti-ADAM9 antibody or ADAM9-binding fragment thereof comprises a heavy chain variable domain (VH) and a light chain variable domain (VL) having sequences of SEQ ID NO:28 and SEQ ID NO:55, respectively. In some embodiments, the humanized anti-ADAM9 antibody comprises a heavy chain and a light chain having the sequences of SEQ ID NO:52 and SEQ ID NO:68, respectively. In some embodiments, the humanized anti-ADAM9 antibody comprises a heavy chain and a light chain having the sequences of SEQ ID NO: 151 and SEQ ID NO:68, respectively. In some embodiments, X in SEQ ID NO:52 or SEQ ID NO:151 is lysine. In some embodiments, X in SEQ ID NO:52 or SEQ ID NO:151 is absent.

In certain embodiments, the immunoconjugate of the present invention is represented by the following formula:

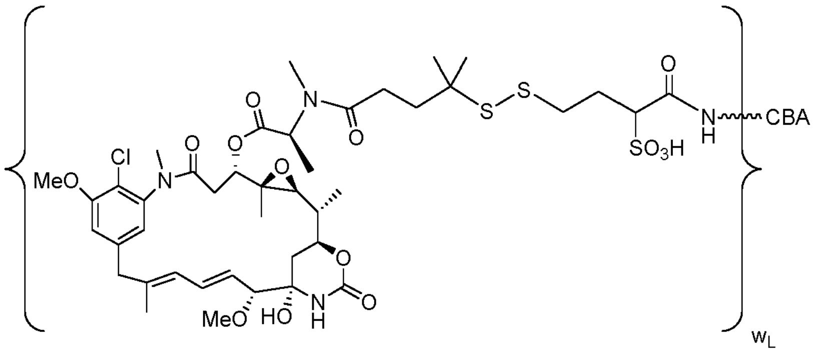

or a pharmaceutically acceptable salt thereof, wherein:

$W_L$ is an integer from 1 to 10;

CBA is an humanized anti-ADAM9 antibody or ADAM9-binding fragment thereof comprising a $CDR_H1$ domain, a $CDR_H2$ domain, and a $CDR_H3$ domain and a $CDR_L1$ domain, a $CDR_L2$ domain, and a $CDR_L3$ domain having the sequences of SEQ ID NOs: 8, 35, and 45 and SEQ ID NOs: 62, 13, 14, respectively. In certain embodiments, the humanized anti-ADAM9 antibody or ADAM9-binding fragment thereof comprises a heavy chain variable domain (VH) and a light chain variable domain (VL) having sequences of SEQ ID NO:28 and SEQ ID NO:55, respectively. In some embodiments, the humanized anti-ADAM9 antibody comprises a heavy chain and a light chain having the sequences of SEQ ID NO:52 and SEQ ID NO:68, respectively. In some embodiments, the humanized anti-ADAM9 antibody comprises a heavy chain and a light chain having the sequences of SEQ ID NO: 151 and SEQ ID NO:68, respectively. In some embodiments, In some embodiments, X in SEQ ID NO:52 or SEQ ID NO:151 is lysine. In some embodiments, In some embodiments, X in SEQ ID NO:52 or SEQ ID NO:151 is absent. In some embodiments, the DAR value for a composition (*e.g.*, pharmaceutical compositions) comprising the immunoconjugate is in the range of 1.0 to 5.0, 1.0 to 4.0, 1.0 to 3.4, 1.0 to 3.0, 1.5 to 2.5, 2.0 to 2.5, or 1.8 to 2.2. In some embodiments, the DAR is less than 4.0, less than 3.8, less than 3.6, less than 3.5, less than 3.0 or less than 2.5.

In certain embodiments, the immunoconjugate of the present invention is represented by the following formula:

$$CBA\left(-Cy^{C1}\right)_{W_C},$$

wherein:

CBA is an anti-ADAM9 antibody or ADAM9-binding fragment thereof as described herein covalently linked to $Cy^{C1}$ through a cysteine residue;

$W_C$ is 1 or 2;

$Cy^{C1}$ is represented by the following formula:

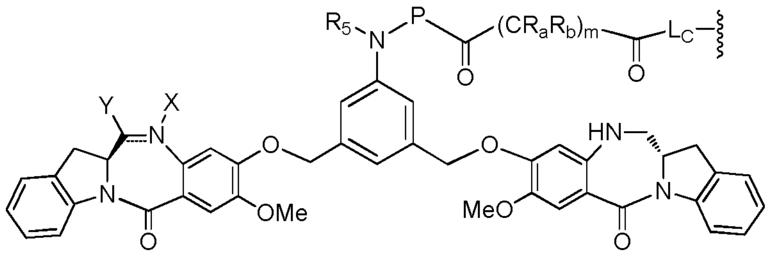

;

or

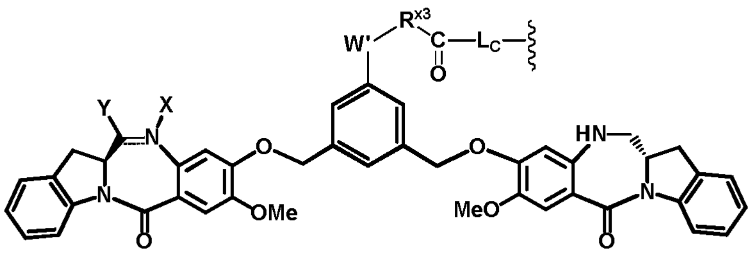

,

or a pharmaceutically acceptable salt thereof, wherein:
the double line



between N and C represents a single bond or a double bond, provided that when it is a double bond, X is absent and Y is -H or a $(C_1-C_4)$alkyl; and when it is a single bond, X is -H or an amine protecting moiety, Y is -OH or $-SO_3H$ or a pharmaceutically acceptable salt thereof;
$R_5$ is -H or a $(C_1-C_3)$alkyl;
P is an amino acid residue or a peptide containing 2 to 20 amino acid residues;
$R_a$ and $R_b$, for each occurrence, are independently -H, $(C_1-C_3)$alkyl, or a charged substituent or an ionizable group Q;
W' is $-NR^{e'}$,
$R^{e'}$ is $-(CH_2-CH_2-O)_n-R^k$;
n is an integer from 2 to 6;
$R^k$ is -H or -Me;
$R^{x3}$ is a $(C_1-C_6)$alkyl; and,
$L_C$ is represented by

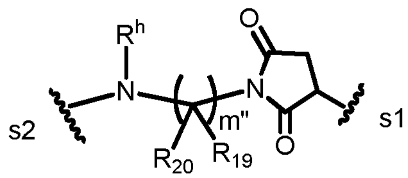

,

s1 is the site covalently linked to CBA, and s2 is the site covalently linked to the -C(=O)- group on $Cy^{C1}$; wherein:
$R_{19}$ and $R_{20}$, for each occurrence, are independently -H or a $(C_1-C_3)$alkyl;

m" is an integer between 1 and 10; and
$R^h$ is -H or a $(C_1-C_3)$alkyl.

In certain embodiments, the immunoconjugate of the present invention is represented by the following formula:

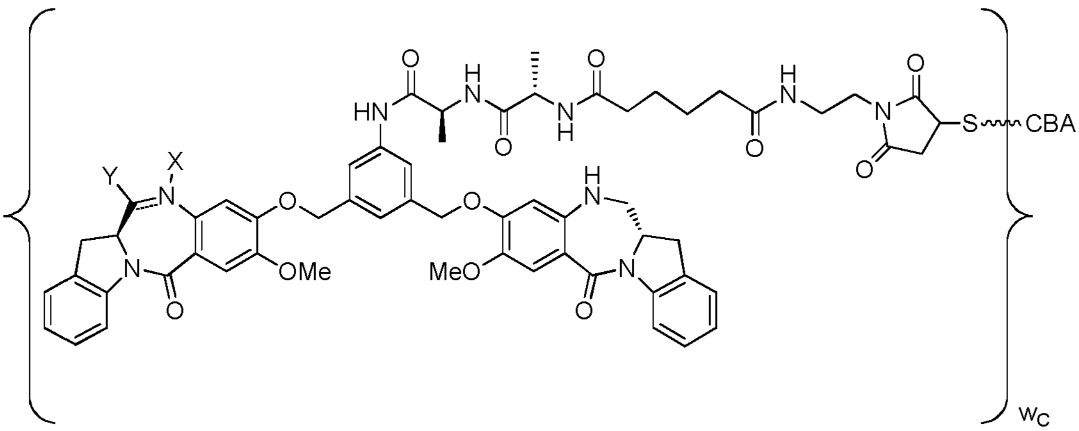

;

or a pharmaceutically acceptable salt thereof, wherein:
the double line



between N and C represents a single bond or a double bond, provided that when it is a double bond, X is absent and Y is -H, and when it is a single bond, X is -H, and Y is $-SO_3H$ or a pharmaceutically acceptable salt thereof;

CBA is an humanized anti-ADAM9 antibody or ADAM9-binding fragment thereof comprising a $CDR_H1$ domain, a $CDR_H2$ domain, and a $CDR_H3$ domain and a $CDR_L1$ domain, a $CDR_L2$ domain, and a $CDR_L3$ domain having the sequences of SEQ ID NOs: 8, 35, and 45 and SEQ ID NOs: 62, 13, 14, respectively. In certain embodiments, the humanized anti-ADAM9 antibody or ADAM9-binding fragment thereof comprises a heavy chain variable domain (VH) and a light chain variable domain (VL) having sequences of SEQ ID NO:28 and SEQ ID NO:55, respectively. In some embodiments, humanized anti-ADAM9 antibody comprises a heavy chain and a light chain having the sequences of SEQ ID NO: 142 and SEQ ID NO:68, respectively. In some embodiments, X in SEQ ID NO:142 is lysine. In some embodiments, the humanized anti-ADAM9 antibody comprises a heavy chain and a light chain having the sequences of SEQ ID NO:152 and SEQ ID NO:68, respectively. In some embodiments, X in SEQ ID NO:142 or SEQ ID NO:152 is lysine. In some embodiments, X in SEQ ID NO:142 or SEQ ID NO:152 is absent.

In certain embodiments, the immunoconjugate is represented by the following formula:

$$CBA\left(-Cy^{C2}\right)_{W_C},$$

wherein:

CBA is an anti-ADAM9 antibody or ADAM9-binding fragment thereof as described herein covalently linked to $Cy^{C2}$ through a cysteine residue;
$W_C$ is 1 or 2;
$Cy^{C2}$ is represented by the following formula:

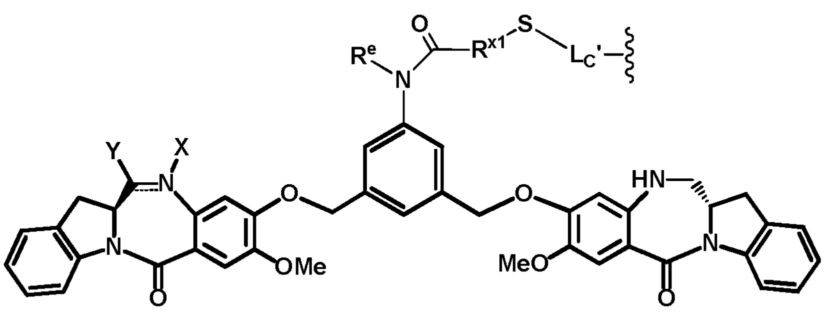

,

or

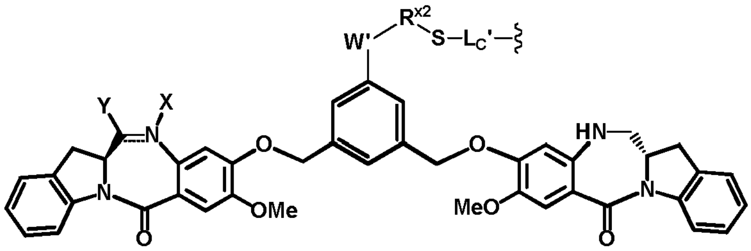

,

or a pharmaceutically acceptable salt thereof, wherein:

the double line



between N and C represents a single bond or a double bond, provided that when it is a double bond, X is absent and Y is -H or a $(C_1-C_4)$alkyl; and when it is a single bond, X is -H or an amine protecting moiety, Y is -OH or $-SO_3H$ or a pharmaceutically acceptable salt thereof;
$R^{x1}$ is a $(C_1-C_6)$alkyl;
$R^e$ is -H or a $(C_1-C_6)$alkyl;
W' is $-NR^{e'}$;
$R^{e'}$ is $-(CH_2-CH_2-O)_n-R^k$;
n is an integer from 2 to 6;
$R^k$ is -H or -Me;
$R^{x2}$ is a $(C_1-C_6)$alkyl;
$L_C$' is represented by the following formula:

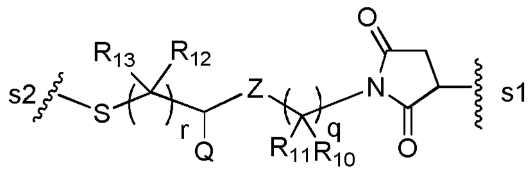

;

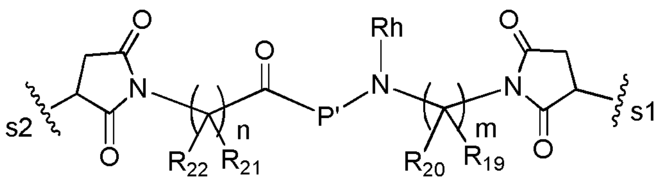

;

wherein:

s1 is the site covalently linked to the CBA and s2 is the site covalently linked to -S-group on $Cy^{C2}$;
Z is $-C(=O)-NR_9-$, or $-NR_9-C(=O)-$;
Q is -H, a charged substituent, or an ionizable group;
$R_9$, $R_{10}$, $R_{11}$, $R_{12}$, $R_{13}$, $R_{19}$, $R_{20}$, $R_{21}$ and $R_{22}$, for each occurrence, are independently - H or a $(C_1-C_3)$alkyl;
q and r, for each occurrence, are independently an integer between 0 and 10;
m and n are each independently an integer between 0 and 10;
$R^h$ is -H or a $(C_1-C_3)$alkyl; and
P' is an amino acid residue or a peptide containing 2 to 20 amino acid residues.

In certain embodiments, the immunoconjugate of the present invention is represented by the following formula:

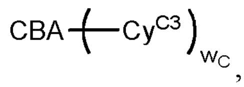

wherein:

CBA is an anti-ADAM9 antibody or ADAM9-binding fragment as described herein covalently linked to $Cy^{C3}$ through a cysteine residue;
$W_C$ is 1 or 2;
$Cy^{C3}$ is represented by the following formula:

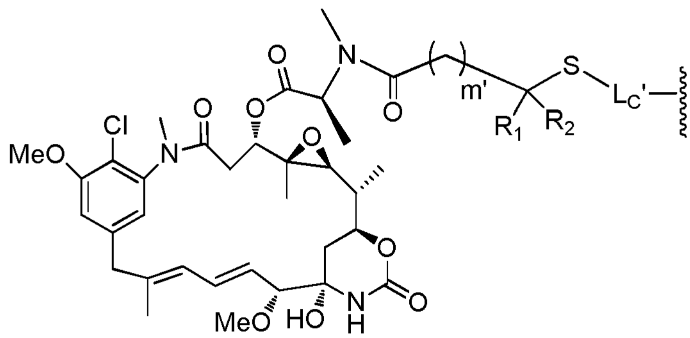

wherein:

m' is 1 or 2;
$R_1$ and $R_2$, are each independently -H or a $(C_1\text{-}C_3)$alkyl;
$L_C$' is represented by the following formula:

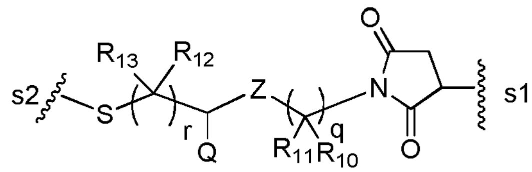

;

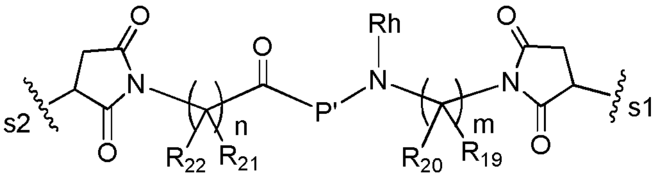

;

wherein:

s1 is the site covalently linked to the CBA and s2 is the site covalently linked to -S-group on $Cy^{C3}$;
Z is $-C(=O)-NR_9-$, or $-NR_9-C(=O)-$;
Q is H, a charged substituent, or an ionizable group;
$R_9$, $R_{10}$, $R_{11}$, $R_{12}$, $R_{13}$, $R_{19}$, $R_{20}$, $R_{21}$ and $R_{22}$, for each occurrence, are independently - H or a $(C_1\text{-}C_3)$alkyl;
q and r, for each occurrence, are independently an integer between 0 and 10;
m and n are each independently an integer between 0 and 10;
$R^h$ is -H or a $(C_1\text{-}C_3)$alkyl; and
P' is an amino acid residue or a peptide containing 2 to 20 amino acid residues.

In certain embodiments, the immunoconjugate of the present invention is represented by the following formula:

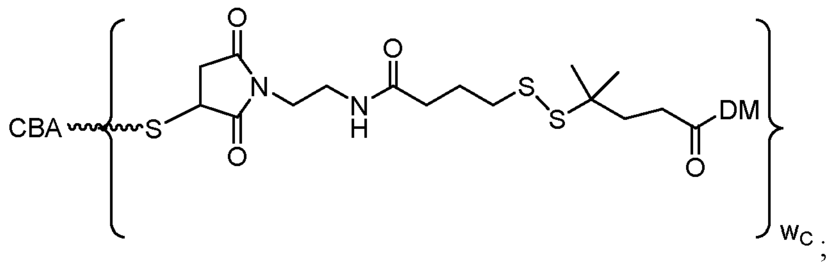

or a pharmaceutically acceptable salt thereof, wherein:

DM is a drug moiety represented by the following formula:

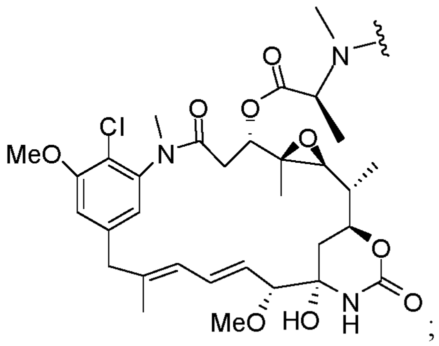

$W_C$ is 1 or 2;

CBA is an humanized anti-ADAM9 antibody or ADAM9-binding fragment thereof comprising a $CDR_H1$ domain, a $CDR_H2$ domain, and a $CDR_H3$ domain and a $CDR_L1$ domain, a $CDR_L2$ domain, and a $CDR_L3$ domain having the sequences of SEQ ID NOs: 8, 35, and 45 and SEQ ID NOs: 62, 13, 14, respectively. In certain embodiments, the humanized anti-ADAM9 antibody or ADAM9-binding fragment thereof comprises a heavy chain variable domain (VH) and a light chain variable domain (VL) having sequences of SEQ ID NO:28 and SEQ ID NO:55, respectively. In some embodiments, the humanized anti-ADAM9 antibody comprises a heavy chain and a light chain having the sequences of SEQ ID NO: 142 and SEQ ID NO:68, respectively. In some embodiments, the humanized anti-ADAM9 antibody comprises a heavy chain and a light chain having the sequences of SEQ ID NO: 152 and SEQ ID NO:68, respectively. In some embodiments, X in SEQ ID NO:142 or SEQ ID NO: 152 is lysine. In some embodiments, X in SEQ ID NO:142 or SEQ ID NO: 152 is absent. In some embodiments, Wc is 2.

In certain embodiments, the immunoconjugate of the present invention is represented by the following formula:

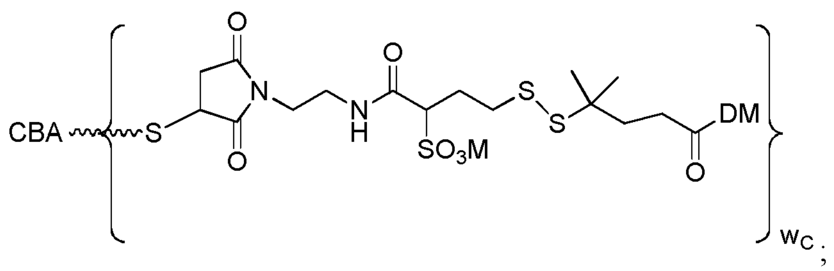

or a pharmaceutically acceptable salt thereof, wherein:

DM is a drug moiety represented by the following formula:

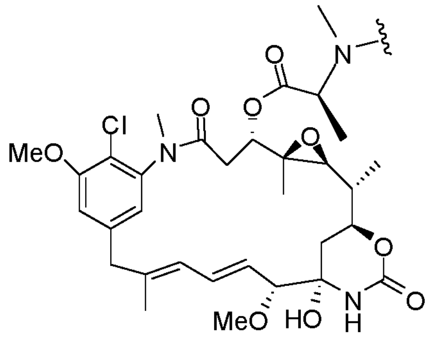

CBA is an humanized anti-ADAM9 antibody or ADAM9-binding fragment thereof comprising a $CDR_H1$ domain, a $CDR_H2$ domain, and a $CDR_H3$ domain and a $CDR_L1$ domain, a $CDR_L2$ domain, and a $CDR_L3$ domain having the sequences of SEQ ID NOs: 8, 35, and 45 and SEQ ID NOs: 62, 13, 14, respectively. In certain embodiments, the humanized anti-ADAM9 antibody or ADAM9-binding fragment thereof comprises a heavy chain variable domain (VH) and a light chain variable domain (VL) having sequences of SEQ ID NO:28 and SEQ ID NO:55, respectively. In some embodiments, the humanized anti-ADAM9 antibody comprises a heavy chain and a light chain having the sequences of SEQ ID NO: 142 and SEQ ID NO:68, respectively. In some embodiments, the humanized anti-ADAM9 antibody comprises a heavy chain and a light chain having the sequences of SEQ ID NO: 152 and SEQ ID NO:68, respectively; and

Wc is 1 or 2. In some embodiments, Wc is 2.

Another aspect of the present invention is set out in claim 15 and provides a pharmaceutical composition comprising an effective amount of the immunoconjugate as described herein and a pharmaceutically acceptable carrier, excipient or diluent.

Another aspect of the present invention is set out in claim 16 and provides the immunoconjugate or the pharmaceutical composition for use in a method for treating a disease or condition associated with, or characterized by, the expression of ADAM9 in a subject comprising administering to said subject an effective amount of the immunoconjugate or the pharmaceutical composition.

In certain embodiments, the disease or condition associated with, or characterized by, the expression of ADAM9 is cancer. In some embodiments, the cancer is selected from the group consisting of non-small-cell lung cancer, colorectal cancer, gastric cancer, pancreatic cancer, renal cell carcinoma, prostate cancer, esophageal cancer, breast cancer, head and neck cancer, ovarian cancer, liver cancer, cervical cancer, thyroid cancer, testicular cancer, myeloid cancer, melanoma, and lymphoid cancer. In certain embodiments, the non-small-cell lung cancer is squamous cell carcinoma, adenocarcinoma, or large-cell undifferentiated carcinoma. In certain embodiments, the colorectal cancer is adenocarcinoma, gastrointestinal carcinoid tumors, gastrointestinal stromal tumors, primary colorectal lymphoma, leiomyosarcoma, or squamous cell carcinoma.

## BRIEF DESCRIPTION OF THE DRAWINGS

In the following examples, immunoconjugates comprising chMAB-A or hMAB-A(2.2) are shown for illustration purposes only and do not form part of the presently-claimed invention.

**FIGs. 1A-1C** present the results of an immunohistochemistry (IHC) studies and show the ability of MAB-A to specifically label a variety of non-small cell lung cancer types **(FIG. 1A),** breast cancer cells, prostate cancer cells, gastric cancer cells **(FIG. 1B),** and colon cancer cells **(FIG. 1C)** while the isotype control failed to specifically label any of these cancer cell types **(FIGs. 1A-1C).**

**FIG. 2** presents the results of cell staining studies and show that MAB-A binds to human ADAM9, and to a lesser extent, cynomolgus monkey ADAM9, transiently expressed on the surface of 293-FT and CHO-K cells (top and bottom panels respectively).

**FIGs. 3A-3B** depict the amino acid sequences of the murine anti-ADAM9-VH Domain aligned with several humanized/optimized variants of MAB-A **(FIG. 3A, SEQ ID NOs:7, 16, 17, 18,19, 21, 22, 23** and **28)** and the murine anti-ADAM9-VL Domain aligned with several humanized/optimized variants of MAB-A **(FIG. 3B, SEQ ID NOs:11, 51, 52, 53** and **54).** Positions substituted within the CDRs during the initial optimization are underlined as follows:

potential deamidation and isomeration sites are indicated with a single underline, lysine residues are indicated with double underline, additional labile residues are indicated with a double dashed underline.**FIGs. 4A-4B** present the ELISA binding curves of the ten selected optimized hMAB-A clones comprising $CDR_H3$ variants, the parental hMAB-A (2.2), and an isotype control antibody. **FIG. 4A** presents the binding curves for cynoADAM9 and **FIG. 4B** presents the binding curves for huADAM9.

**FIGs. 5A-5B** present the ELISA binding curves of the Fc variants. **FIG. 5A** presents the binding curves for cynoADAM9 and **FIG. 5B** presents the binding curves for huADAM9.

**FIGs. 6A-6B** show ADAM9 IHC membrane staining in a 20 carcinoma tissue microarray and ADAM IHC membrane and cytoplasmic staining in eight selected indications, respectively.

**FIGs. 7A-7B** show pulse internalization and continuous internalization of various anti-ADAM9 antibody conjugates.

**FIGs. 8A-8D** show FACS binding curves of chMAB-A, hMAB-A(2.2), hMAB-A(2C.2), and hMAB-A(2I.2) antibodies and their corresponding immunoconjugates.

**FIGs. 9A-9H** show *in vitro* cytotoxicity of various anti-ADAM9 immunoconjugates (hMAB-A(2.2)-sSPDB-DM4, hMAB-A(2I.2)-sSPDB-DM4, hMAB-A(2.2)-DGN549, and hMAB-A(2I.2)-S442C-DGN549) on a broad panel of ADAM9 positive tumor cell lines. The non-targeting IgG1 based conjugates are included as negative controls.

**FIG. 10** shows the anti-tumor activity of hMAB-A(2.2)-sSPDB-DM4 (1.25 mg/kg, 2.5 mg/kg, 5 mg/kg, by antibody) and chMAB-A-DGN549 (10μg/kg, by payload) in the Calu-3 human non-small cell lung adenocarcinoma xenograft model.

**FIG. 11** shows the anti-tumor activity of hMAB-A(2I.2)-S442C-DGN549 (0.5μg/kg, 1μg/kg, 3μg/kg, and 10μg/kg, by payload) in the Calu-3 human non-small cell lung adenocarcinoma xenograft model.

**FIG. 12** shows the anti-tumor activity of hMAB-A(2.2)-sSPDB-DM4 (1.25 mg/kg, 2.5 mg/kg, 5 mg/kg, by antibody) in the H1703 non-small cell lung squamous cell carcinoma xenograft model.

**FIG. 13** shows the anti-tumor activity of chMAB-A-sSPDB-DM4 (1.25 mg/kg, 2.5 mg/kg, 5 mg/kg) in the Detroit562 head and neck squamous cell carcinoma model (tumor volume 95.76 to 450.83 $mm^3$).

**FIG. 14** shows the anti-tumor activity of chMAB-A-sSPDB-DM4 (1.25 mg/kg and 5 mg/kg) in the Detroi562 head and neck squamous cell carcinoma model (tumor volume 277.51 to 503.49 $mm^3$).

**FIG. 15** shows the anti-tumor activity of huMAB-A(2I.2)-S442C-DGN549 in the SNU-5 gastric carcinoma xenograft model.

**FIG. 16** shows the anti-tumor activity of huMAB-A(2.2)-sSPDB-DM4 in the SNU-5 gastric carcinoma xenograft model.

**FIG. 17** shows the anti-tumor activity of huMAB-A(2I.2)-S442C-DGN549 in the SW48 colorectal cancer xenograft model.

**FIG. 18** shows the anti-tumor activity of huMAB-A(2.2)-sSPDB-DM4 in the SW48 colorectal cancer xenograft model.

**FIG. 19A** shows the binding of 250nM & 1000nM huFcRn to captured anti-ADAM9 antibodies with and without the YTE mutation at pH 6.0.

**FIG. 19B** shows the binding of 25nM & 100nM anti-ADAM9 antibodies with and without the YTE mutation to immobilized FcRn at pH 6.0.

**FIG. 20** shows the antibody-drug conjugate PK data for hMAB-A(2I.2)-sSPDB-DM4 and hMAB-A(2I.2)-YTE-sSPDB-DM4 in cynomolgus monkey at both 4 mg/kg and 12 mg/kg (antibody dose).

## DETAILED DESCRIPTION OF THE INVENTION

The present invention is directed to immunoconjugates comprising an antibody or fragment thereof capable of specifically binding to "Disintegrin and Metalloproteinase Domain-containing Protein 9" **("ADAM9")** conjugated to at least one pharmacological agent as defined in claim 1. The immunoconjugates are cross-reactive with human ADAM9 and the ADAM9 of a non-human primate (a cynomolgus monkey). The immunoconjugates comprise a Light Chain Variable (VL) Domain and/or a Heavy Chain Variable (VH) Domain that has been humanized (and optionally deimmunized) so as to exhibit reduced immunogenicity upon administration of such immunoconjugates to a recipient subject. The invention is also directed to pharmaceutical compositions as defined in claim 15 that contain any of such immunoconjugates, and to immunoconjugates or pharmaceutical compositions for use in the treatment of a disease or condition associated with, or characterized by, the expression of ADAM9 (such as cancer) as defined in claims 16 and 17.

### I. Antibodies and Their Binding Domains

The immunoconjugates of the present invention comprise an antibody that binds to ADAM9 or an ADAM9-binding fragment thereof. **"Antibodies"** are immunoglobulin molecules capable of specific binding to a target, such as a carbohydrate, polynucleotide, lipid, polypeptide, *etc.,* through at least one antigen recognition site, located in the **Variable Domain** of the immunoglobulin molecule. As used herein, the terms **"antibody"** and **"antibodies"** refer to monoclonal antibodies, multispecific antibodies, human antibodies, humanized antibodies, synthetic antibodies, chimeric antibodies, polyclonal antibodies, camelized antibodies, single-chain Fvs (scFv), single-chain antibodies, Fab fragments, F(ab') fragments, intrabodies, and epitope-binding fragments of any of the above. In particular, the term "antibody" includes immunoglobulin molecules and immunologically active fragments of immunoglobulin molecules, *i.e.,* molecules that contain an epitope-binding site. Immunoglobulin molecules can be of any type (*e.g.,* IgG, IgE, IgM, IgD, IgA and IgY), class (*e.g.,* $IgG_1$, $IgG_2$, $IgG_3$, $IgG_4$, $IgA_1$ and $IgA_2$) or subclass. The last few decades have seen a revival of interest in the therapeutic potential of antibodies, and antibodies have become one of the leading classes of biotechnology-derived drugs (Chan, C.E. et al. (2009) "The Use Of Antibodies In The Treatment Of Infectious Diseases," Singapore Med. J. 50(7):663-666). In addition to their use in diagnostics, antibodies have been shown to be useful as therapeutic agents. Over 200 antibody-based drugs have been approved for use or are under development.

Antibodies are capable of **"immunospecifically binding"** to a polypeptide or protein or a non-protein molecule due to the presence on such molecule of a particular domain or moiety or conformation (an **"epitope").** An epitope-containing molecule may have immunogenic activity, such that it elicits an antibody production response in an animal; such molecules are termed **"antigens."** As used herein, an antibody is said to **"immunospecifically"** bind a region of another molecule (*i.e.,* an epitope) if it reacts or associates more frequently, more rapidly, with greater duration and/or with greater affinity with that epitope relative to alternative epitopes. For example, an antibody that immunospecifically binds to a viral epitope is an antibody that binds that viral epitope with greater affinity, avidity, more readily, and/or with greater duration than it immunospecifically binds to other viral epitopes or to non-viral epitopes. It is also understood by reading this definition that, for example, an antibody (or moiety or epitope) that immunospecifically binds to a first target may or may not specifically or preferentially bind to a second target. As such, "immunospecific binding" to a particular epitope does not necessarily require (although it can include) exclusive binding to that epitope. Generally, but not necessarily, reference to binding means "immunospecific" binding. Two molecules are said to be capable of binding to one another in a **"physiospecific"** manner, if such binding exhibits the specificity with which receptors bind to their respective ligands.

The term **"monoclonal antibody"** refers to a homogeneous antibody population wherein the monoclonal antibody is comprised of amino acids (naturally occurring or non-naturally occurring) that are involved in the selective binding of an antigen. Monoclonal antibodies are highly specific, being directed against a single epitope (or antigenic site). The term "monoclonal antibody" encompasses not only intact monoclonal antibodies and full-length monoclonal antibodies, but also fragments thereof (such as Fab, Fab', $F(ab')_2$ Fv), single-chain (scFv), mutants thereof, fusion proteins comprising an antibody portion, humanized monoclonal antibodies, chimeric monoclonal antibodies, and any other modified configuration of the immunoglobulin molecule that comprises an antigen recognition site of the required specificity and the ability to bind to an antigen. The term is not intended to be limited as regards to the source of the antibody or the manner in which it is made (*e.g.,* by hybridoma, phage selection, recombinant expression, transgenic animals, *etc.*). The term includes whole immunoglobulins as well as the fragments *etc.* described above under the definition of "antibody." Methods of making monoclonal antibodies are known in the art. One method which may be employed is the method of Kohler, G. et al. (1975) "Continuous Cultures Of Fused Cells Secreting Antibody Of Predefined Specificity," Nature 256:495-497, or a modification thereof. Typically, monoclonal antibodies are developed in mice, rats or rabbits. The antibodies are produced by immunizing an animal with an immunogenic amount of cells, cell extracts, or protein preparations that contain the desired epitope. The immunogen can be, but is not limited to, primary cells, cultured cell lines, cancerous cells, proteins, peptides, nucleic acids, or tissue. Cells used for immunization may be

cultured for a period of time (*e.g.,* at least 24 hours) prior to their use as an immunogen. Cells may be used as immunogens by themselves or in combination with a non-denaturing adjuvant, such as Ribi (see, *e.g.,* Jennings, V.M. (1995) "Review of Selected Adjuvants Used in Antibody Production," ILAR J. 37(3):119-125). In general, cells should be kept intact and preferably viable when used as immunogens. Intact cells may allow antigens to be better detected than ruptured cells by the immunized animal. Use of denaturing or harsh adjuvants, *e.g.*, Freund's adjuvant, may rupture cells and therefore is discouraged. The immunogen may be administered multiple times at periodic intervals such as, bi weekly, or weekly, or may be administered in such a way as to maintain viability in the animal (e.g., in a tissue recombinant). Alternatively, existing monoclonal antibodies and any other equivalent antibodies that are immunospecific for a desired pathogenic epitope can be sequenced and produced recombinantly by any means known in the art. In one embodiment, such an antibody is sequenced and the polynucleotide sequence is then cloned into a vector for expression or propagation. The sequence encoding the antibody of interest may be maintained in a vector in a host cell and the host cell can then be expanded and frozen for future use. The polynucleotide sequence of such antibodies may be used for genetic manipulation to generate an affinity optimized, a chimeric antibody, a humanized antibody, and/or a caninized antibody, to improve the affinity, or other characteristics of the antibody, as well as the immunoconjugates of the invention. The general principle in humanizing an antibody involves retaining the basic sequence of the antigen-binding portion of the antibody, while swapping the non-human remainder of the antibody with human antibody sequences.

Natural antibodies (such as natural IgG antibodies) are composed of two "**Light Chains**" complexed with two "**Heavy Chains**." Each Light Chain contains a Variable Domain ("**VL**") and a Constant Domain ("**CL**"). Each Heavy Chain contains a Variable Domain ("**VH**"), three Constant Domains ("**CH1**," "**CH2**" and "**CH3**"), and a "**Hinge**" Region ("**H**") located between the **CH1** and **CH2** Domains. In contrast, scFvs are single chain molecules made by linking Light and Heavy Chain Variable Domains together via a short linking peptide.

The basic structural unit of naturally occurring immunoglobulins (*e.g.*, IgG) is thus a tetramer having two light chains and two heavy chains, usually expressed as a glycoprotein of about 150,000 Da. The amino-terminal **("N-terminal")** portion of each chain includes a Variable Domain of about 100 to 110 or more amino acids primarily responsible for antigen recognition. The carboxy-terminal ("**C-terminal**") portion of each chain defines a constant region, with light chains having a single Constant Domain and heavy chains usually having three Constant Domains and a Hinge Region. Thus, the structure of the light chains of an IgG molecule is n-VL-CL-c and the structure of the IgG heavy chains is **n-VH-CH1-H-CH2-CH3-c** (where n and c represent, respectively, the N-terminus and the C-terminus of the polypeptide).

### A. Characteristics of Antibody Variable Domains

The Variable Domains of an IgG molecule consist of 1, 2, and most commonly 3, complementarity determining regions **("CDR",** *i.e.,* **CDR1, CDR2** and **CDR3,** respectively), which contain the residues in contact with epitope, and non-CDR segments, referred to as **framework regions ("FR"),** which in general maintain the structure and determine the positioning of the CDR regions so as to permit such contacting (although certain framework residues may also contact the epitope). Thus, the VL and VH Domains typically have the structure: **n-FR1-CDR1-FR2-CDR2-FR3-CDR3-FR4-c** (where "n" denotes the N-terminus and "c" denotes the C-terminus). Polypeptides that are (or may serve as) the first, second, third, and fourth FR of the Light Chain of an antibody are herein respectively designated as: **$FR_L1$ Domain, $FR_L2$ Domain, $FR_L3$ Domain,** and **$FR_L4$ Domain.** Similarly, polypeptides that are (or may serve as) the first, second, third and fourth FR of the Heavy Chain of an antibody are herein respectively designated as: **$FR_H1$ Domain, $FR_H2$ Domain, $FR_H3$ Domain** and **$FR_H4$ Domain.** Polypeptides that are (or may serve as) the first, second and third CDR of the Light Chain of an antibody are herein respectively designated as: **$CDR_L1$ Domain, $CDR_L2$ Domain,** and **$CDR_L3$ Domain.** Similarly, polypeptides that are (or may serve as) the first, second and third CDR of the Heavy Chain of an antibody are herein respectively designated as: **$CDR_H1$ Domain, $CDR_H2$ Domain,** and **$CDR_H3$ Domain.** Thus, the terms $CDR_L1$ Domain, $CDR_L2$ Domain, $CDR_L3$ Domain, $CDR_H1$ Domain, $CDR_H2$ Domain, and $CDR_H3$ Domain are directed to polypeptides that when incorporated into an antibody causes the antibody to be able to bind to a specific epitope.

Throughout the present specification, the numbering of the residues in the Variable Domains of the mature heavy and light chains of immunoglobulins are designated by the position of an amino acid in the chain. Kabat described numerous amino acid sequences for antibodies, identified an amino acid consensus sequence for each subgroup, and assigned a residue number to each amino acid, and the CDRs are identified as defined by Kabat (it will be understood that $CDR_H1$ as defined by Chothia, C. & Lesk, A. M. ((1987) "Canonical structures for the hypervariable regions of immunoglobulins," J. Mol. Biol. 196:901-917) begins five residues earlier). Kabat's numbering scheme is extendible to antibodies not included in his compendium by aligning the antibody in question with one of the consensus sequences in Kabat by reference to conserved amino acids. This method for assigning residue numbers has become standard in the field and readily identifies amino acids at equivalent positions in different antibodies, including chimeric or humanized variants. For example, an amino acid at position 50 of a human antibody light chain occupies the equivalent position to an amino acid at position 50 of a mouse antibody light chain.

The ability of an antibody to bind an epitope of an antigen depends upon the presence and amino acid sequence

of the antibody's VL and VH Domains. Interaction of an antibody's Light Chain and Heavy Chain and, in particular, interaction of its VL and VH Domains forms one of the two epitope-binding sites of a natural antibody, such as an IgG. Natural antibodies are capable of binding to only one epitope species (*i.e.,* they are monospecific), although they can bind multiple copies of that epitope species (*i.e.,* exhibiting bivalency or multivalency).

Accordingly, as used herein, the term "**epitope-binding fragment**" means a fragment of an antibody capable of immunospecifically binding to an epitope, and the term "**epitope-binding site**" refers to a portion of a molecule comprising an epitope-binding fragment. An epitope-binding fragment may contain any 1, 2, 3, 4, or 5 the CDR Domains of an antibody, or may contain all 6 of the CDR Domains of an antibody and, although capable of immunospecifically binding to such epitope, may exhibit an immunospecificity, affinity or selectivity toward such epitope that differs from that of such antibody. Preferably, however, an epitope-binding fragment will contain all 6 of the CDR Domains of such antibody. An epitope-binding fragment of an antibody may be a single polypeptide chain (*e.g.,* an scFv), or may comprise two or more polypeptide chains, each having an amino terminus and a carboxy terminus (*e.g.,* a Fab fragment, an $Fab_2$ fragment, *etc.*). Unless specifically noted, the order of domains of the protein molecules described herein is in the "**N-terminal to C-terminal**" direction.

The invention also encompasses immunoconjugates comprising single-chain Variable Domain fragments **("scFv")** comprising an anti-ADAM9-VL and/or VH Domain of the invention. Single-chain Variable Domain fragments comprise VL and VH Domains that are linked together using a short "Linker" peptide. Such Linkers can be modified to provide additional functions, such as to permit the attachment of a drug or to permit attachment to a solid support. The single-chain variants can be produced either recombinantly or synthetically. For synthetic production of scFv, an automated synthesizer can be used. For recombinant production of scFv, a suitable plasmid containing polynucleotide that encodes the scFv can be introduced into a suitable host cell, either eukaryotic, such as yeast, plant, insect or mammalian cells, or prokaryotic, such as E. coli. Polynucleotides encoding the scFv of interest can be made by routine manipulations such as ligation of polynucleotides. The resultant scFv can be isolated using standard protein purification techniques known in the art.

The term "**humanized**" antibody refers to a chimeric molecule having an epitope-binding site of an immunoglobulin from a non-human species and a remaining immunoglobulin structure that is based upon the structure and /or sequence of a human immunoglobulin. Humanized antibodies are generally prepared using recombinant techniques. The immunoconjugates of the present invention may comprise humanized, chimeric or caninized variants of an antibody that is designated herein as **"MAB-A."** The polynucleotide sequences that encode the Variable Domains of MAB-A may be used for genetic manipulation to generate MAB-A derivatives possessing improved or altered characteristics *(e.g.,* affinity, cross-reactivity, specificity, *etc.).* The general principle in humanizing an antibody involves retaining the basic sequence of the epitope-binding portion of the antibody, while swapping the non-human remainder of the antibody with human antibody sequences. There are four general steps to humanize a monoclonal antibody. These are: (1) determining the nucleotide and predicted amino acid sequence of the starting antibody light and heavy variable domains; (2) designing the humanized antibody or caninized antibody, *i.e.,* deciding which antibody framework region to use during the humanizing or canonizing process; (3) employing the actual humanizing or caninizing methodologies/techniques; and (4) transfecting and expressing the humanized antibody. See, for example, U.S. Patent Nos. 4,816,567; 5,807,715; 5,866,692; and 6,331,415. The term "**optimized**" antibody refers to an antibody having at least one amino acid which is different from the parent antibody in at least one complementarity determining region (CDR) in the light or heavy chain variable region, which confers a higher binding affinity, (e.g., a 2-fold or more fold) higher binding affinity, to human ADAM9 and/or cynomolgus monkey ADAM9 as compared to the parental antibody. It will be understood from the teaching provided herein that the antibodies of the invention are humanized, and optionally optimized.

The epitope-binding site may comprise either a complete Variable Domain fused to one or more Constant Domains or only the CDRs of such Variable Domain grafted to appropriate framework regions. Epitope-binding sites may be wild-type or may be modified by one or more amino acid substitutions, insertions or deletions. Such action partially or completely eliminates the ability of the Constant Region to serve as an immunogen in recipients (*e.g.*, human individuals), however, the possibility of an immune response to the foreign Variable Domain remains (LoBuglio, A.F. et al. (1989) "Mouse/Human Chimeric Monoclonal Antibody In Man: Kinetics And Immune Response," Proc. Natl. Acad. Sci. (U.S.A.) 86:4220-4224). Another approach focuses not only on providing human-derived constant regions, but on modifying the Variable Domains as well so as to reshape them as closely as possible to a form found in human immunoglobulins. It is known that the Variable Domains of both the Heavy and Light Chains of antibodies contain three CDRs which vary in response to the antigens in question and determine binding capability, flanked by the four framework regions, which are relatively conserved in a given species and which putatively provide a scaffolding for the CDRs. When non-human antibodies are prepared with respect to a particular antigen, the variable domains can be "reshaped" or "humanized" by grafting CDRs derived from non-human antibody on the FRs present in the human antibody to be modified. Application of this approach to various antibodies has been reported by Sato, K. et al. (1993) Cancer Res 53:851-856. Riechmann, L. et al. (1988) "Reshaping Human Antibodies for Therapy," Nature 332:323-327; Verhoeyen, M. et al. (1988) "Reshaping Human Antibodies: Grafting An Antilysozyme Activity," Science 239:1534-1536; Kettleborough, C. A. et al. (1991) "Humanization Of A Mouse Monoclonal Antibody By CDR-Grafting: The Importance Of Framework Residues On Loop Conformation," Protein Engineering 4:773-3783; Maeda, H. et al. (1991) "Construction Of Reshaped Human Antibodies With HIV-Neutralizing Activity," Human Antibodies Hybridoma 2:124-134; Gorman, S. D. et al. (1991) "Reshaping A Therapeutic CD4 Antibody," Proc. Natl. Acad. Sci. (U.S.A.) 88:4181-4185; Tempest, P.R. et al. (1991) "Reshaping A Human Monoclonal Antibody To Inhibit Human Respiratory Syncytial Virus Infection in vivo," Bio/Technology 9:266-271; Co, M. S. et al. (1991) "HumanizedAntibodies For Antiviral Therapy," Proc. Natl. Acad. Sci. (U.S.A.) 88:2869-2873; Carter, P. et al. (1992) "Humanization Of An Anti-p185her2 Antibody For Human Cancer Therapy," Proc. Natl. Acad. Sci. (U.S.A.) 89:4285-4289; and Co, M.S. et al. (1992) "Chimeric And Humanized Antibodies With Specificity For The CD33 Antigen," J. Immunol. 148:1149-1154. According to the claimed invention, the humanized antibodies have one or more CDRs (one, two, three, four, or five) that differ in sequence relative to the CDRs of the original antibody.

A number of humanized antibody molecules comprising an epitope-binding site derived from a non-human immunoglobulin have been described, including chimeric antibodies having rodent or modified rodent Variable Domain and their associated complementarity determining regions (CDRs) fused to human constant domains (see, for example, Winter et al. (1991) "Man-made Antibodies," Nature 349:293-299; Lobuglio et al. (1989) "Mouse/Human Chimeric Monoclonal Antibody In Man: Kinetics And Immune Response," Proc. Natl. Acad. Sci. (U.S.A.) 86:4220-4224; Shaw et al. (1987) "Characterization Of A Mouse/Human Chimeric Monoclonal Antibody (17-1A) To A Colon Cancer Tumor-Associated Antigen," J. Immunol. 138:4534-4538; and Brown et al. (1987) "Tumor-Specific Genetically Engineered Murine/Human Chimeric Monoclonal Antibody," Cancer Res. 47:3577-3583). Other references describe rodent CDRs grafted into a human supporting framework region (FR) prior to fusion with an appropriate human antibody Constant Domain (see, for example, Riechmann, L. et al. (1988) "Reshaping Human Antibodies for Therapy," Nature 332:323-327; Verhoeyen, M. et al. (1988) "Reshaping Human Antibodies: Grafting An Antilysozyme Activity," Science 239:1534-1536; and Jones et al. (1986) "Replacing The Complementarity-Determining Regions In A Human Antibody With Those From A Mouse," Nature 321:522-525). Another reference describes rodent CDRs supported by recombinantly veneered rodent framework regions (see, for example, European Patent Publication No. 519,596). These "humanized" molecules are designed to minimize unwanted immunological response towards rodent anti-human antibody molecules, which limits the duration and effectiveness of therapeutic applications of those moieties in human recipients. Other methods of humanizing antibodies that may also be utilized are disclosed by Daugherty et al. (1991) "Polymerase Chain Reaction Facilitates The Cloning, CDR-Grafting, And Rapid Expression Of A Murine Monoclonal Antibody Directed Against The CD18 Component Of Leukocyte Integrins," Nucl. Acids Res. 19:2471-2476 and in U.S. Patent Nos. 6,180,377; 6,054,297; 5,997,867; and 5,866,692.

### B. Characteristics of Antibody Constant Domains

Throughout the present specification, the numbering of the residues in the constant region of an IgG heavy chain is that of the EU index as in Kabat et al., SEQUENCES OF PROTEINS OF IMMUNOLOGICAL INTEREST, 5th Ed. Public Health Service, NH1, MD (1991) ("Kab**at").** The term **"the EU index as set forth in Kabat"** refers to the numbering of the Constant Domains of human IgG1 EU antibody provided in Kabat. This method for assigning residue numbers has become standard in the field and readily identifies amino acids at equivalent positions in the constant regions of different antibody isotypes.

#### 1. Constant Regions of the Light Chain

As indicated above, each Light Chain of an antibody contains a Variable Domain **("VL")** and a Constant Domain **("CL").**

A preferred CL Domain is a human IgG CL Kappa Domain. The amino acid sequence of an exemplary human CL Kappa Domain is **(SEQ ID NO:69):**

```
RTVAAPSVFI FPPSDEQLKS GTASVVCLLN NFYPREAKVQ WKVDNALQSG
NSQESVTEQD SKDSTYSLSS TLTLSKADYE KHKVYACEVT HQGLSSPVTK
SFNRGEC
```

Alternatively, an exemplary CL Domain is a human IgG CL Lambda Domain. The amino acid sequence of an exemplary human CL Lambda Domain is **(SEQ ID NO:70):**

QPKAAPSVTL FPPSSEELQA NKATLVCLIS DFYPGAVTVA WKADSSPVKA
GVETTPSKQS NNKYAASSYL SLTPEQWKSH RSYSCQVTHE GSTVEKTVAP
TECS

### 2. Constant Regions of the Heavy Chain

#### a. Naturally-Occurring Fc Regions

As provided herein, the immunoconjugates of the invention may comprise an Fc Region. The Fc Region of such immunoconjugates the invention may be of any isotype (*e.g.*, IgG1, IgG2, IgG3, or IgG4). The immunoconjugates of the invention may further comprise a CH1 Domain and/or a Hinge Region. When present, the CH1 Domain and/or Hinge Region may be of any isotype (*e.g.,* IgG1, IgG2, IgG3, or IgG4), and is preferably of the same isotype as the desired Fc Region.

The Fc Region of the Fc Region-containing immunoconjugates of the present invention may be either a complete Fc Region (*e.g.,* a complete IgG Fc Region) or only a fragment of an Fc Region. Optionally, the Fc Region of the Fc Region-containing immunoconjugates of the present invention lacks the C-terminal lysine amino acid residue.

The CH1 Domains of the two heavy chains of an antibody complex with the antibody's Light Chain's "CL" constant region, and are attached to the heavy chains CH2 Domains via an intervening Hinge Domain.

An exemplary CH1 Domain is a human IgG1 CH1 Domain. The amino acid sequence of an exemplary human IgG1 CH1 Domain is (**SEQ ID NO:71**):

ASTKGPSVFP LAPSSKSTSG GTAALGCLVK DYFPEPVTVS WNSGALTSGV
HTFPAVLQSS GLYSLSSVVT VPSSSLGTQT YICNVNHKPS NTKVDKRV

An exemplary CH1 Domain is a human IgG2 CH1 Domain. The amino acid sequence of an exemplary human IgG2 CH1 Domain is (**SEQ ID NO:72**):

ASTKGPSVFP LAPCSRSTSE STAALGCLVK DYFPEPVTVS WNSGALTSGV
HTFPAVLQSS GLYSLSSVVT VPSSNFGTQT YTCNVDHKPS NTKVDKTV

An exemplary CH1 Domain is a human IgG4 CH1 Domain. The amino acid sequence of an exemplary human IgG4 CH1 Domain is (**SEQ ID NO:73**):

ASTKGPSVFP LAPCSRSTSE STAALGCLVK DYFPEPVTVS WNSGALTSGV
HTFPAVLQSS GLYSLSSVVT VPSSSLGTKT YTCNVDHKPS NTKVDKRV

One exemplary Hinge Region is a human IgG1 Hinge Region. The amino acid sequence of an exemplary human IgG1 Hinge Region is **(SEQ ID NO:74):** EPKSCDKTHTCPPCP.

Another exemplary Hinge Region is a human IgG2 Hinge Region. The amino acid sequence of an exemplary human IgG2 Hinge Region is **(SEQ ID NO:75):** ERKCCVECPPCP.

Another exemplary Hinge Region is a human IgG4 Hinge Region. The amino acid sequence of an exemplary human IgG4 Hinge Region is **(SEQ ID NO:76):** ESKYGPPCPSCP. As described above, an IgG4 Hinge Region may comprise a stabilizing mutation, such as the S228P substitution. The amino acid sequence of an exemplary stabilized IgG4 Hinge Region is **(SEQ ID NO:77):** ESKYGPPCPPCP.

The CH2 and CH3 Domains of the two Heavy Chains of an antibody interact to form an **"Fc Region,"** which is a domain that is recognized by cellular **"Fc Receptors,"** including but not limited to Fc gamma Receptors **("FcγRs").** As used herein, the term "Fc Region" is used to define the C-terminal region of an IgG Heavy Chain that comprises the CH2 and CH3 Domains of that chain. An Fc Region is said to be of a particular IgG isotype, class or subclass if its amino acid sequence is most homologous to that isotype, relative to other IgG isotypes.

The amino acid sequence of the CH2-CH3 Domain of an exemplary human IgG1 is (SEQ ID NO:1)

```
231       240        250        260        270        280
APELLGGPSV FLFPPKPKDT LMISRTPEVT CVVVDVSHED PEVKFNWYVD

          290        300        310        320        330
GVEVHNAKTK PREEQYNSTY RVVSVLTVLH QDWLNGKEYK CKVSNKALPA

          340        350        360        370        380
PIEKTISKAK GQPREPQVYT LPPSREEMTK NQVSLTCLVK GFYPSDIAVE

          390        400        410        420        430
WESNGQPENN YKTTPPVLDS DGSFFLYSKL TVDKSRWQQG NVFSCSVMHE

          440     447
ALHNHYTQKS LSLSPGX
```

as numbered by the EU index as set forth in Kabat, wherein X is a lysine (K) or is absent.

The amino acid sequence of the CH2-CH3 Domain of an exemplary human IgG2 is (SEQ ID NO:2):

```
231       240        250        260        270        280
APPVA-GPSV FLFPPKPKDT LMISRTPEVT CVVVDVSHED PEVQFNWYVD

          290        300        310        320        330
GVEVHNAKTK PREEQFNSTF RVVSVLTVVH QDWLNGKEYK CKVSNKGLPA

          340        350        360        370        380
PIEKTISKTK GQPREPQVYT LPPSREEMTK NQVSLTCLVK GFYPSDISVE

          390        400        410        420        430
WESNGQPENN YKTTPPMLDS DGSFFLYSKL TVDKSRWQQG NVFSCSVMHE

          440     447
ALHNHYTQKS LSLSPGX
```

as numbered by the EU index as set forth in Kabat, wherein X is a lysine (K) or is absent.

The amino acid sequence of the CH2-CH3 Domain of an exemplary human IgG3 is **(SEQ ID NO:3):**

```
231       240        250        260        270        280
APELLGGPSV FLFPPKPKDT LMISRTPEVT CVVVDVSHED PEVQFKWYVD

          290        300        310        320        330
GVEVHNAKTK PREEQYNSTF RVVSVLTVLH QDWLNGKEYK CKVSNKALPA

          340        350        360        370        380
PIEKTISKTK GQPREPQVYT LPPSREEMTK NQVSLTCLVK GFYPSDIAVE

          390        400        410        420        430
WESSGQPENN YNTTPPMLDS DGSFFLYSKL TVDKSRWQQG NIFSCSVMHE

          440     447
ALHNRFTQKS LSLSPGX
```

as numbered by the EU index as set forth in Kabat, wherein X is a lysine (K) or is absent.

The amino acid sequence of the CH2-CH3 Domain of an exemplary human IgG4 is (SEQ ID NO:4):

```
231       240        250        260        270        280
APEFLGGPSV FLFPPKPKDT LMISRTPEVT CVVVDVSQED PEVQFNWYVD

          290        300        310        320        330
GVEVHNAKTK PREEQFNSTY RVVSVLTVLH QDWLNGKEYK CKVSNKGLPS

          340        350        360        370        380
SIEKTISKAK GQPREPQVYT LPPSQEEMTK NQVSLTCLVK GFYPSDIAVE

          390        400        410        420        430
WESNGQPENN YKTTPPVLDS DGSFFLYSRL TVDKSRWQEG NVFSCSVMHE

          440     447
ALHNHYTQKS LSLSLGX
```

as numbered by the EU index as set forth in Kabat, wherein X is a lysine (K) or is absent.

Polymorphisms have been observed at a number of different positions within antibody constant regions (*e.g.,* Fc positions, including but not limited to positions 270, 272, 312, 315, 356, and 358 as numbered by the EU index as set forth in Kabat), and thus slight differences between the presented sequence and sequences in the prior art can exist. Polymorphic forms of human immunoglobulins have been well-characterized. At present, 18 Gm allotypes are known: G1m (1, 2, 3, 17) or G1m (a, x, f, z), G2m (23) or G2m (n), G3m (5, 6, 10, 11, 13, 14, 15, 16, 21, 24, 26, 27, 28) or G3m (b1, c3, b3, b0, b3, b4, s, t, g1, c5, u, v, g5) (Lefranc, et al., "The Human IgG Subclasses: Molecular Analysis of Structure, Function And Regulation." Pergamon, Oxford, pp. 43-78 (1990); Lefranc, G. et al., 1979, Hum. Genet.: 50, 199-211). It is specifically contemplated that the antibodies of the present invention may incorporate any allotype, isoallotype, or haplotype of any immunoglobulin gene, and are not limited to the allotype, isoallotype or haplotype of the sequences provided herein. Furthermore, in some expression systems the C-terminal amino acid residue (bolded above) of the CH3 Domain may be post-translationally removed. Accordingly, the C-terminal residue of the CH3 Domain is an optional amino acid residue in the immunoconjugates of the invention. Specifically encompassed by the instant invention are immunoconjugates lacking the C-terminal residue of the CH3 Domain. Also specifically encompassed by the instant invention are such constructs comprising the C-terminal lysine residue of the CH3 Domain.

**b. Fcγ Receptors (FcyRs)**

In traditional immune function, the interaction of antibody-antigen complexes with cells of the immune system results in a wide array of responses, ranging from effector functions such as antibody dependent cytotoxicity, mast cell degranulation, and phagocytosis to immunomodulatory signals such as regulating lymphocyte proliferation and antibody secretion. All of these interactions are initiated through the binding of the Fc Region of antibodies or immune complexes to specialized cell surface receptors on hematopoietic cells, and particularly to receptors (singularly referred to as an **"Fc gamma receptor" "FcyR,"** and collectively as **"FcyRs"**) found on the surfaces of multiple types of immune system cells (*e.g.,* B lymphocytes, follicular dendritic cells, natural killer cells, macrophages, neutrophils, eosinophils, basophils and mast cells).

The diversity of cellular responses triggered by antibodies and immune complexes results from the structural heterogeneity of the three Fc receptors: FcγRI (CD64), FcγRII (CD32), and FcγRIII (CD16). FcγRI (CD64), FcγRIIA (CD32A) and FcγRIII (CD16) are activating (*i.e.,* immune system enhancing) receptors; FcγRIIB (CD32B) is an inhibiting (*i.e.,* immune system dampening) receptor. In addition, interaction with the neonatal Fc Receptor (FcRn) mediates the recycling of IgG molecules from the endosome to the cell surface and release into the blood. The amino acid sequence of exemplary wild-type IgG1 **(SEQ ID NO:1),** IgG2 **(SEQ ID NO:2),** IgG3 **(SEQ ID NO:3),** and IgG4 **(SEQ ID NO:4)** are presented above.

The ability of the different FcγRs to mediate diametrically opposing functions reflects structural differences among the different FcγRs, and in particular reflects whether the bound FcγR possesses an Immunoreceptor Tyrosine-Based Activation Motif **("ITAM")** or an Immunoreceptor Tyrosine-Based Inhibitory Motif **("ITIM").** The recruitment of different cytoplasmic enzymes to these structures dictates the outcome of the FcγR-mediated cellular responses. ITAM-containing FcγRs include FcγRI, FcγRIIA, FcγRIIIA, and activate the immune system when bound to Fc Regions (*e.g.*, aggregated Fc Regions present in an immune complex). FcγRIIB is the only currently known natural ITIM-containing FcγR; it acts to dampen or inhibit the immune system when bound to aggregated Fc Regions. Human neutrophils express the FcγRIIA gene. FcγRIIA clustering via immune complexes or specific antibody cross-linking serves to aggregate ITAMs with receptor-associated kinases which facilitate ITAM phosphorylation. ITAM phosphorylation serves as a docking site for Syk kinase, the activation of which results in the activation of downstream substrates (*e.g.*, $PI_3K$). Cellular activation leads to release of pro-inflammatory mediators. The FcγRIIB gene is expressed on B lymphocytes; its extracellular domain is 96% identical to FcγRIIA and binds IgG complexes in an indistinguishable manner. The presence of an ITIM in the cytoplasmic domain of FcγRIIB defines this inhibitory subclass of FcγR. Recently the molecular basis of this inhibition was established. When co-ligated along with an activating FcγR, the ITIM in FcγRIIB becomes phosphorylated and attracts the SH2 domain of the inositol polyphosphate 5'-phosphatase (SHIP), which hydrolyzes phosphoinositol messengers released as a consequence of ITAM-containing FcγR- mediated tyrosine kinase activation, consequently preventing the influx of intracellular $Ca^{++}$. Thus cross-linking of FcγRIIB dampens the activating response to FcγR ligation and inhibits cellular responsiveness. B-cell activation, B-cell proliferation and antibody secretion is thus aborted.

**c. Variant Fc Regions**

Modification of the Fc Region may lead to an altered phenotype, for example altered serum half-life, altered stability, altered susceptibility to cellular enzymes or altered effector function. It may therefore be desirable to modify an Fc Region-containing molecule of the present invention with respect to effector function, for example, so as to enhance the effectiveness of such molecule in treating cancer. Reduction or elimination of effector function is desirable in certain cases, for example in the case of antibodies whose mechanism of action involves blocking or antagonism, but not killing of the cells bearing a target antigen. Increased effector function is generally desirable when directed to undesirable cells, such as tumor and foreign cells, where the FcγRs are expressed at low levels, for example, tumor-specific B cells with low levels of FcγRIIB (*e.g.*, non-Hodgkin's lymphoma, CLL, and Burkitt's lymphoma). Immunoconjugates of the invention possessing such conferred or altered effector function activity are useful for the treatment and/or prevention of a disease, disorder or infection in which an enhanced efficacy of effector function activity is desired.

Accordingly, in certain embodiments, the Fc Region of the Fc Region-containing immunoconjugates of the present invention may be an engineered variant Fc Region. Although the Fc Region of immunoconjugates of the present invention may possess the ability to bind to one or more Fc receptors (*e.g.*, FcγR(s)), more preferably such variant Fc Region have altered binding to FcγRIA (CD64), FcγRIIA (CD32A), FcγRIIB (CD32B), FcγRIIIA (CD16a) or FcγRIIIB (CD16b) (relative to the binding exhibited by a wild-type Fc Region), *e.g.*, will have enhanced binding to an activating receptor and/or will have substantially reduced or no ability to bind to inhibitory receptor(s). Thus, the Fc Region of the immunoconjugates of the present invention may include some or all of the CH2 Domain and/or some or all of the CH3 Domain of a complete Fc Region, or may comprise a variant CH2 and/or a variant CH3 sequence (that may include, for example, one or more insertions and/or one or more deletions with respect to the CH2 or CH3 domains of a complete Fc Region). Such Fc Regions may comprise non-Fc polypeptide portions, or may comprise portions of non-naturally

complete Fc Regions, or may comprise non-naturally occurring orientations of CH2 and/or CH3 Domains (such as, for example, two CH2 domains or two CH3 domains, or in the N-terminal to C-terminal direction, a CH3 Domain linked to a CH2 Domain, *etc.*).

Fc Region modifications identified as altering effector function are known in the art, including modifications that increase binding to activating receptors (*e.g.*, FcγRIIA (CD16A) and reduce binding to inhibitory receptors (*e.g.,* FcγRIIB (CD32B) (see, *e.g.,* Stavenhagen, J.B. et al. (2007) "Fc Optimization Of Therapeutic Antibodies Enhances Their Ability To Kill Tumor Cells In Vitro And Controls Tumor Expansion In Vivo Via Low-Affinity Activating Fcgamma Receptors," Cancer Res. 57(18):8882-8890). **Table 1** lists exemplary single, double, triple, quadruple and quintuple substitutions (numbering is that of the EU index as in Kabat, and substitutions are relative to the amino acid sequence of **SEQ ID NO:1**) of exemplary modification that increase binding to activating receptors and/or reduce binding to inhibitory receptors.

| **Table** 1 **Variations of Preferred Activating Fc Regions** | | | |
|---|---|---|---|
| **Single-Site Variations** | | | |
| F243L | R292G | D270E | R292P |
| Y300L | P396L | | |
| **Double-Site Variations** | | | |
| F243L and R292P | F243L and Y300L | F243L and P396L | R292P and Y300L |
| D270E and P396L | R292P and V305I | P396L and Q419H | P247L and N421K |
| R292P and P396L | Y300L and P396L | R255L and P396L | R292P and P305I |
| K392T and P396L | | | |
| **Triple-Site Variations** | | | |
| F243L, P247L and N421K | | P247L, D270E and N421K | |
| F243L, R292P and Y300L | | R255L, D270E and P396L | |
| F243L, R292P and V305I | | D270E, G316D and R416G | |
| F243L, R292P and P396L | | D270E, K392T and P396L | |
| F243L, Y300L and P396L | | D270E, P396L and Q419H | |
| V284M, R292L and K370N | | R292P, Y300L and P396L | |
| **Quadruple-Site Variations** | | | |
| L234F, F243L, R292P and Y300L | | F243L, P247L, D270E and N421K | |
| L234F, F243L, R292P and Y300L | | F243L, R255L, D270E and P396L | |
| L235I, F243L, R292P and Y300L | | F243L, D270E, G316D and R416G | |
| L235Q, F243L, R292P and Y300L | | F243L, D270E, K392T and P396L | |
| P247L, D270E, Y300L and N421K | | F243L, R292P, Y300L, and P396L | |
| R255L, D270E, R292G and P396L | | F243L, R292P, V305I and P396L | |
| R255L, D270E, Y300L and P396L | | F243L, D270E, P396L and Q419H | |
| D270E, G316D, P396L and R416G | | | |
| **Quintuple-Site Variations** | | | |
| L235V, F243L, R292P, Y300L and P396L | | F243L, R292P, V305I, Y300L and P396L | |
| L235P, F243L, R292P, Y300L and P396L | | | |

Exemplary variants of human IgG1 Fc Regions with reduced binding to CD32B and/or increased binding to CD16A contain F243L, R292P, Y300L, V305I or P396L substitutions, wherein the numbering is that of the EU index as in Kabat. These amino acid substitutions may be present in a human IgG1 Fc Region in any combination. In one embodiment, the variant human IgG1 Fc Region contains a F243L, R292P and Y300L substitution. In another embodiment, the variant human IgG1 Fc Region contains a F243L, R292P, Y300L, V305I and P396L substitution.

In certain embodiments, it is preferred for the Fc Regions of the immunoconjugates of the present invention to exhibit decreased (or substantially no) binding to FcγRIA (CD64), FcγRIIA (CD32A), FcγRIIB (CD32B), FcγRIIIA (CD16a) or FcγRIIIB (CD16b) (relative to the binding exhibited by the wild-type IgG1 Fc Region **(SEQ ID NO:1).** In a specific embodiment, the immunoconjugates of the present invention comprise an IgG Fc Region that exhibits reduced ADCC effector function. In a preferred embodiment the CH2-CH3 Domains of immunoconjugates include any 1, 2, 3, or 4 of the substitutions: L234A, L235A, D265A, N297Q, and N297G, wherein the numbering is that of the EU index as in Kabat. In another embodiment, the CH2-CH3 Domains contain an N297Q substitution, an N297G substitution, L234A and L235A substitutions or a D265A substitution, as these mutations abolish FcR binding. Alternatively, a CH2-CH3 Domain of a naturally occurring Fc region that inherently exhibits decreased (or substantially no) binding to FcγRIIIA (CD16a) and/or reduced effector function (relative to the binding and effector function exhibited by the wild-type IgG1 Fc Region (**SEQ ID NO:1**)) is utilized. In a specific embodiment, the immunoconjugates of the present invention comprise an IgG2 Fc Region (**SEQ ID NO:2**) or an IgG4 Fc Region (**SEQ ID:NO:4).** When an IgG4 Fc Region is utilized, the instant invention also encompasses the introduction of a stabilizing mutation, such as the Hinge Region S228P substitution described above (see, *e.g.,* **SEQ ID NO:77**). Since the N297G, N297Q, L234A, L235A and D265A substitutions abolish effector function, in circumstances in which effector function is desired, these substitutions would preferably not be employed.

A preferred IgG1 sequence for the CH2 and CH3 Domains of the Fc Region-containing immunoconjugates of the present invention having reduced or abolished effector function will comprise the substitutions L234A/L235A (shown underlined) **(SEQ ID NO:78):**

APE**AA**GGPSV FLFPPKPKDT LMISRTPEVT CVVVDVSHED PEVKFNWYVD
GVEVHNAKTK PREEQYNSTY RVVSVLTVLH QDWLNGKEYK CKVSNKALPA
PIEKTISKAK GQPREPQVYT LPPSREEMTK NQVSLTCLVK GFYPSDIAVE
WESNGQPENN YKTTPPVLDS DGSFFLYSKL TVDKSRWQQG NVFSCSVMHE
ALHNHYTQKS LSLSPG**X**

wherein, X is a lysine (K) or is absent.

A second preferred IgG1 sequence for the CH2 and CH3 Domains of the Fc Region-containing immunoconjugates of the present invention comprises an S442C substitution (shown underlined), that permits two CH3 domains to be covalently bonded to one another via a disulfide bond or conjugation of a pharmaceutical agent. The amino acid sequence of such molecule is (SEQ ID NO:79):

APELLGGPSV FLFPPKPKDT LMISRTPEVT CVVVDVSHED PEVKFNWYVD
GVEVHNAKTK PREEQYNSTY RVVSVLTVLH QDWLNGKEYK CKVSNKALPA
PIEKTISKAK GQPREPQVYT LPPSREEMTK NQVSLTCLVK GFYPSDIAVE
WESNGQPENN YKTTPPVLDS DGSFFLYSKL TVDKSRWQQG NVFSCSVMHE
ALHNHYTQKS L**C**LSPG**X**

wherein, **X** is a lysine (K) or is absent.

A third preferred IgG1 sequence for the CH2 and CH3 Domains of the Fc Region-containing immunoconjugates of the present invention comprises the L234A/L235A substitutions (shown underlined) that reduce or abolish effector function and the S442C substitution (shown underlined) that permits two CH3 domains to be covalently bonded to one another via a disulfide bond or conjugation of a pharmaceutical agent. The amino acid sequence of such molecule is **(SEQ ID NO:80):**

APE**AA**GGPSV FLFPPKPKDT LMISRTPEVT CVVVDVSHED PEVKFNWYVD
GVEVHNAKTK PREEQYNSTY RVVSVLTVLH QDWLNGKEYK CKVSNKALPA
PIEKTISKAK GQPREPQVYT LPPSREEMTK NQVSLTCLVK GFYPSDIAVE
WESNGQPENN YKTTPPVLDS DGSFFLYSKL TVDKSRWQQG NVFSCSVMHE
ALHNHYTQKS L**C**LSPG**X**

wherein, **X** is a lysine (K) or is absent.

The serum half-life of proteins comprising Fc Regions may be increased by increasing the binding affinity of

the Fc Region for FcRn. The term "half-life" as used herein means a pharmacokinetic property of a molecule that is a measure of the mean survival time of the molecules following their administration. Half-life can be expressed as the time required to eliminate fifty percent (50%) of a known quantity of the molecule from a subject's (*e.g.,* a human patient or other mammal) body or a specific compartment thereof, for example, as measured in serum, *i.e.,* circulating half-life, or in other tissues. In general, an increase in half-life results in an increase in mean residence time (MRT) in circulation for the administered molecule.

In some embodiments, the immunoconjugates of the present invention comprise a variant Fc Region that comprises at least one amino acid modification relative to a wild-type Fc Region, such that said molecule has an increased half-life (relative to a molecule comprising a wild-type Fc Region). In some embodiments, the immunoconjugates of the present invention comprise a variant IgG Fc Region, wherein said variant Fc Region comprises a half-life extending amino acid substitution at one or more positions selected from the group consisting of 238, 250, 252, 254, 256, 257, 256, 265, 272, 286, 288, 303, 305, 307, 308, 309, 311, 312, 317, 340, 356, 360, 362, 376, 378, 380, 382, 413, 424, 428, 433, 434, 435, and 436, wherein the numbering is that of the EU index as in Kabat. Numerous mutations capable of increasing the half-life of an Fc Region-containing molecule are known in the art and include, for example M252Y, S254T, T256E, and combinations thereof. For example, see the mutations described in U.S. Patent Nos. 6,277,375, 7,083,784; 7,217,797, 8,088,376; U.S. Publication Nos. 2002/0147311; 2007/0148164; and PCT Publication Nos. WO 98/23289; WO 2009/058492; and WO 2010/033279. Immunoconjugates with enhanced half-life also include those possessing variant Fc Regions comprising substitutions at two or more of Fc Region residues 250, 252, 254, 256, 257, 288, 307, 308, 309, 311, 378, 428, 433, 434, 435 and 436, wherein the numbering is that of the EU index as in Kabat. In particular, two or more substitutions selected from: T250Q, M252Y, S254T, T256E, K288D, T307Q, V308P, A378V, M428L, N434A, H435K, and Y436I, wherein the numbering is that of the EU index as in Kabat.

In a specific embodiment, an immunoconjugate of the present invention possesses a variant IgG Fc Region comprising the substitutions:

(A) M252Y, S254T and T256E;
(B) M252Y and S254T;
(C) M252Y and T256E;
(D) T250Q and M428L;
(E) T307Q and N434A;
(F) A378V and N434A;
(G) N434A and Y436I;
(H) V308P and N434A; or
(I) K288D and H435K.

In a preferred embodiment, the immunoconjugate of the present invention possesses a variant IgG Fc Region comprising any 1, 2, or 3 of the substitutions: M252Y, S254T and T256E. The invention further encompasses immunoconjugates possessing variant Fc Regions comprising:

(A) one or more mutations which alter effector function and/or FcγR; and
(B) one or more mutations which extend serum half-life.

A fourth preferred IgG1 sequence for the CH2 and CH3 Domains of the Fc Region-containing immunoconjugates of the present invention comprises the M252Y, S254T and T256E substitutions (shown underlined), so as to extend the serum half-life. The amino acid sequence of such molecule is **(SEQ ID NO:147):**

```
APELLGGPSV FLFPPKPKDT LYITREPEVT CVVVDVSHED PEVKFNWYVD
GVEVHNAKTK PREEQYNSTY RVVSVLTVLH QDWLNGKEYK CKVSNKALPA
PIEKTISKAK GQPREPQVYT LPPSREEMTK NQVSLTCLVK GFYPSDIAVE
WESNGQPENN YKTTPPVLDS DGSFFLYSKL TVDKSRWQQG NVFSCSVMHE
ALHNHYTQKS LSLSPGX
```

wherein, **X** is a lysine (K) or is absent.

A fifth preferred IgG1 sequence for the CH2 and CH3 Domains of the Fc Region-containing immunoconjugates of the present invention comprises the M252Y, S254T and T256E substitutions (shown underlined), so as to extend the serum half-life, and the S442C substitution (shown underlined), so as to permit two CH3 domains to be covalently bonded to one another via a disulfide bond or to permit conjugation of a drug moiety. The amino acid sequence of such molecule

is **(SEQ ID NO: 148):**

APELLGGPSV FLFPPKPKDT L**Y**I**T**R**E**PEVT CVVVDVSHED PEVKFNWYVD
GVEVHNAKTK PREEQYNSTY RVVSVLTVLH QDWLNGKEYK CKVSNKALPA
PIEKTISKAK GQPREPQVYT LPPSREEMTK NQVSLTCLVK GFYPSDIAVE
WESNGQPENN YKTTPPVLDS DGSFFLYSKL TVDKSRWQQG NVFSCSVMHE
ALHNHYTQKS L**C**LSPG**X**

wherein, **X** is a lysine (K) or is absent.

A sixth preferred IgG1 sequence for the CH2 and CH3 Domains of the Fc Region-containing immunoconjugates of the present invention comprises the L234A/L235A substitutions (shown underlined) that reduce or abolish effector function and the M252Y, S254T and T256E substitutions (shown underlined), so as to extend the serum half-life. The amino acid sequence of such molecule is **(SEQ ID NO: 149):**

APE**AA**GGPSV FLFPPKPKDT L**Y**I**T**R**E**PEVT CVVVDVSHED PEVKFNWYVD
GVEVHNAKTK PREEQYNSTY RVVSVLTVLH QDWLNGKEYK CKVSNKALPA
PIEKTISKAK GQPREPQVYT LPPSREEMTK NQVSLTCLVK GFYPSDIAVE
WESNGQPENN YKTTPPVLDS DGSFFLYSKL TVDKSRWQQG NVFSCSVMHE
ALHNHYTQKS LSLSPG**X**

wherein, **X** is a lysine (K) or is absent.

A seventh preferred IgG1 sequence for the CH2 and CH3 Domains of the Fc Region-containing immunoconjugates of the present invention comprises the L234A/L235A substitutions (shown underlined) that reduce or abolish effector function and the M252Y, S254T and T256E substitutions (shown underlined), so as to extend the serum half-life and the S442C substitution (shown underlined), so as to permit two CH3 domains to be covalently bonded to one another via a disulfide bond or to permit conjugation of a drug moiety. The amino acid sequence of such molecule is **(SEQ ID NO:150):**

APE**AA**GGPSV FLFPPKPKDT L**Y**I**T**R**E**PEVT CVVVDVSHED PEVKFNWYVD
GVEVHNAKTK PREEQYNSTY RVVSVLTVLH QDWLNGKEYK CKVSNKALPA
PIEKTISKAK GQPREPQVYT LPPSREEMTK NQVSLTCLVK GFYPSDIAVE
WESNGQPENN YKTTPPVLDS DGSFFLYSKL TVDKSRWQQG NVFSCSVMHE
ALHNHYTQKS L**C**LSPG**X**

wherein, **X** is a lysine (K) or is absent.

**II. Exemplary Anti-ADAM9 Antibodies**

Particular antibodies and antigen-binding fragments thereof capable of specifically binding to ADAM9 are useful in the generation of the immunoconjugates of the invention.

A representative human ADAM9 polypeptide (NCBI Sequence NP_003807, including a 28 amino acid residue signal sequence, shown underlined), shown for illustration purposes only and not according to the claimed invention, has the amino acid sequence **(SEQ ID NO:5):**

**MGSGARFPSG TLRVRWLLLL GLVGPVLG**AA RPGFQQTSHL SSYEIITPWR
LTRERREAPR PYSKQVSYVI QAEGKEHIIH LERNKDLLPE DFVVYTYNKE
GTLITDHPNI QNHCHYRGYV EGVHNSSIAL SDCFGLRGLL HLENASYGIE
PLQNSSHFEH IIYRMDDVYK EPLKCGVSNK DIEKETAKDE EEEPPSMTQL
LRRRRAVLPQ TRYVELFIVV DKERYDMMGR NQTAVREEMI LLANYLDSMY
IMLNIRIVLV GLEIWTNGNL INIVGGAGDV LGNFVQWREK FLITRRRHDS
AQLVLKKGFG GTAGMAFVGT VCSRSHAGGI NVFGQITVET FASIVAHELG
HNLGMNHDDG RDCSCGAKSC IMNSGASGSR NFSSCSAEDF EKLTLNKGGN
CLLNIPKPDE AYSAPSCGNK LVDAGEECDC GTPKECELDP CCEGSTCKLK
SFAECAYGDC CKDCRFLPGG TLCRGKTSEC DVPEYCNGSS QFCQPDVFIQ
NGYPCQNNKA YCYNGMCQYY DAQCQVIFGS KAKAAPKDCF IEVNSKGDRF
GNCGFSGNEY KKCATGNALC GKLQCENVQE IPVFGIVPAI IQTPSRGTKC
WGVDFQLGSD VPDPGMVNEG TKCGAGKICR NFQCVDASVL NYDCDVQKKC
HGHGVCNSNK NCHCENGWAP PNCETKGYGG SVDSGPTYNE MNTALRDGLL
VFFFLIVPLI VCAIFIFIKR DQLWRSYFRK KRSQTYESDG KNQANPSRQP
GSVPRHVSPV TPPREVPIYA NRFAVPTYAA KQPQQFPSRP PPPQPKVSSQ
GNLIPARPAP APPLYSSLT

Of the 819 amino acid residues of ADAM9 **(SEQ ID NO:5),** residues 1-28 are a signal sequence, residues 29-697 are the Extracellular Domain, residues 698-718 are the Transmembrane Domain, and residues 719-819 are the Intracellular Domain. Three structural domains are located within the Extracellular Domain: a Reprolysin (M12B) Family Zinc Metalloprotease Domain (at approximately residues 212-406); a Disintegrin Domain (at approximately residues 423-497); and an EGF-like Domain (at approximately residues 644-697). A number of post-translational modifications and isoforms have been identified and the protein is proteolytically cleaved in the trans-Golgi network before it reaches the plasma membrane to generate a mature protein. The removal of the pro-domain occurs via cleavage at two different sites. Processed most likely by a pro-protein convertase such as furin, at the boundary between the pro-domain and the catalytic domain (Arg-205/Ala-206). An additional upstream cleavage pro-protein convertase site (Arg-56/Glu-57) has an important role in the activation of ADAM9.

A representative cynomolgus monkey ADAM9 polypeptide (NCBI Sequence XM_005563126.2, including a possible 28 amino acid residue signal sequence, shown underlined), shown for illustration purposes only and not according to the claimed invention, has the amino acid sequence **(SEQ ID NO:6):**

MGSGVGSPSG TLRVRWLLLL CLVGPVLGAA RPGFQQTSHL SSYEIITPWR
LTRERREAPR PYSKQVSYLI QAEGKEHIIH LERNKDLLPE DFVVYTYNKE
GTVITDHPNI QNHCHFRGYV EGVYNSSVAL SNCFGLRGLL HLENASYGIE
PLQNSSHFEH IIYRMDDVHK EPLKCGVSNK DIEKETTKDE EEEPPSMTQL
LRRRRAVLPQ TRYVELFIVV DKERYDMMGR NQTAVREEMI LLANYLDSMY
IMLNIRIVLV GLEIWTNGNL INIAGGAGDV LGNFVQWREK FLITRRRHDS
AQLVLKKGFG GTAGMAFVGT VCSRSHAGGI NVFGHITVET FASIVAHELG
HNLGMNHDDG RDCSCGAKSC IMNSGASGSR NFSSCSAEDF EKLTLNKGGN
CLLNIPKPDE AYSAPSCGNK LVDAGEECDC GTPKECELDP CCEGSTCKLK
SFAECAYGDC CKDCRFLPGG TLCRGKTSEC DVPEYCNGSS QFCQPDVFIQ
NGYPCQNNKA YCYNGMCQYY DAQCQVIFGS KAKAAPKDCF IEVNSKGDRF
GNCGFSGNEY KKCATGNALC GKLQCENVQE IPVFGIVPAI IQTPSRGTKC
WGVDFQLGSD VPDPGMVNEG TKCGADKICR NFQCVDASVL NYDCDIQKKC
HGHGVCNSNK NCHCENGWAP PNCETKGYGG SVDSGPTYNE MNTALRDGLL
VFFFLIVPLI VCAIFIFIKR DQLWRRYFRK KRSQTYESDG KNQANPSRQP
VSVPRHVSPV TPPREVPIYA NRFPVPTYAA KQPQQFPSRP PPPQPKVSSQ
GNLIPARPAP APPLYSSLT

The Reprolysin (M12B) Family Zinc Metalloprotease Domain of the protein is at approximately residues 212-406); the Disintegrin Domain of the protein is at approximately residues 423-497.

In certain embodiments, the immunoconjugates comprising the anti-ADAM9 antibodies and ADAM9-binding fragments thereof of the invention are characterized by any one, two, three, four, five, six, seven, eight, or nine of the following criteria:

(1) the ability to immunospecifically bind human ADAM9 as endogenously expressed on the surface of a cancer cell;
(2) specifically binds human and non-human primate ADAM9 (e.g., ADAM9 of cynomolgus monkey) with a similar binding affinity;
(3) specifically binds human ADAM9 with an equilibrium binding constant ($K_D$) of 4 nM or less;
(4) specifically binds non-human primate ADAM9 with an equilibrium binding constant ($K_D$) of 4 nM or less
(5) specifically binds human ADAM9 with an on rate (ka) of $5 \times 10^5$ $M^{-1}min^{-1}$ or more;
(6) specifically binds non-human primate ADAM9 with an on rate (ka) of $1 \times 10^6$ $M^{-1}min^{-1}$ or more;
(7) specifically binds human ADAM9 with an off rate (kd) of $1 \times 10^{-3}$ $min^{-1}$ or less;
(8) specifically binds non-human primate ADAM9 with an off rate (kd) of $9 \times 10^{-4}$ $min^{-1}$ or less.
(9) optimized to have at least 100-fold enhancement (e.g., at least 100-fold, at least 150-fold, at least 200-fold, at least 250-fold, at least 300-fold, at least 350-fold, at least 400-fold, at least 450-fold, at least 500-fold, at least 550-fold, or at least 600-fold enhancement) in binding affinity (e.g., as measured by BIACORE® analysis) to cyno ADAM9 and retains high affinity binding to human ADAM9 (e.g., BIACORE® analysis) as compared to the chimeric or murine parental antibody.

As described herein, the binding constants of an anti-ADAM9 antibody or ADAM9-binding fragment thereof may be determined using surface plasmon resonance *e.g.*, via a BIACORE® analysis. Surface plasmon resonance data may be fitted to a 1:1 Langmuir binding model (simultaneous ka kd) and an equilibrium binding constant $K_D$ calculated from the ratio of rate constants kd/ka. Such binding constants may be determined for a monovalent anti-ADAM9 antibody or ADAM9-binding fragment thereof (*i.e.,* a molecule comprising a single ADAM9 epitope-binding site), a bivalent anti-ADAM9 antibody or ADAM9-binding fragment thereof (*i.e.,* a molecule comprising two ADAM9 epitope-binding sites), or anti-ADAM9 antibodies and ADAM9-binding fragments thereof having higher valency (*e.g.,* a molecule comprising three, four, or more ADAM9 epitope-binding sites).

The present invention particularly encompasses immunoconjugates possessing an anti-ADAM9 antibody or an ADAM9-binding fragment thereof comprising an anti-ADAM9 Light Chain Variable (VL) Domain and an anti-ADAM9 Heavy Chain Variable (VH) Domain that immunospecifically bind to an epitope of a human ADAM9 polypeptide. Unless otherwise stated, all such anti-ADAM9 antibodies and ADAM9-binding fragment thereof are capable of immunospecifically binding to human ADAM9. As used herein such ADAM9 Variable Domains are referred to as **"anti-ADAM9-VL"**

and **"anti-ADAM9-VH,"** respectively.

**A. Murine Anti-Human ADAM9 Antibodies**

A murine anti-ADAM9 antibody that blocks the target protein processing activity of ADAM9, is internalized and having anti-tumor activity was identified (see, *e.g.,* US Patent No. 8,361,475). This antibody, designated in US Patent Nos. 7,674,619 and 8,361,475 as an "anti-KID24" antibody produced by hybridoma clone ATCC PTA-5174, is designated herein as **"MAB-A."** MAB-A exhibits strong preferential binding to tumors over normal tissues (see, FIGs. 7A-7C). MAB-A exhibited little or no staining across a large panel of normal cell types (**Table 2**).

| Table 2 | |
|---|---|
| **Tissue** | MAB-A **(1.25 μg/mL)** |
| **Adrenal** | Negative |
| **Bladder** | Negative |
| **Bone Marrow** | Negative |
| **Breast** | Negative |
| **Cerebellum** | Negative |
| **Cerebrum** | ND |
| **Cervix** | Negative |
| **Colon** | Negative |
| **Esophagus** | Smooth Muscle +/- to 1+ (gr c) <5% |
| **Tissue** | MAB-A **(1.25 μg/mL)** |
| **Ovaduct** | Negative |
| **Heart** | Negative |
| **Kidney** | Negative |
| **Liver** | Negative |
| **Lung** | Negative |
| **Lymph Node** | Negative |
| **Ovary** | Negative |
| **Pancreas** | Very rare (possible acinar) 1+ (c) |
| **Parathyroid** | Epithelium parenchymal cells 1+ (gr c), 1% Cells (favor chief cells) 2+ (m,c)5% 1+ (m,c) 10% apical primarily |
| **Pituitary** | Posterior lobe cells (possibly neural cells and/or pituicytes 1+ (c>m) <5% |
| **Placenta** | Vascular lining cells within chorionic plate 1+ (gr c>m) Mesenchymal cells of chorionic plate 1-2+ (gr c),5% |
| **Prostate** | Glandular epithelium 2+ (gr c)5% and 1+ (gr c) 5% |
| **Retina + Ciliary Body** | Favor negative (pigmented epi layer 3-4+ (gr c) due to pigment not stained) |
| **Submandibular Gland** | Ductal epi +/- (c) 10% |
| **Skeletal Muscle** | Negative |
| **Skin** | Negative |
| **Small Intestine** | Negative |
| **Spinal Cord** | Neuropil 1+ (gr c) <1% |
| **Spleen** | Negative |

(continued)

| Table 2 | |
|---|---|
| **Stomach** | Negative |
| **Testis** | Seminiferous tubule 1+ (gr c) <5% Interstitial cells (possibly Leydig cells) 2-3+ (gr c) <5% and 1+ (gr c) 10% |
| **Thyroid** | Negative |
| **Tonsil** | Endo cells 2-3+ (c,m) <5% and 1+ (m,c) 15% |
| **Ureter** | Transitional epithelium 1+ (m,c) <5% and 1+ (m,c) 5%; Endo cells 1+ (c) <5% |
| **Tissue** | MAB-A **(1.25 μg/mL)** |
| **Uterus** | Negative |
| **A498 Cell Pellet** | 2-3+ (m,c), 50%, 1+ (m,c) 45% |

As shown in **FIG. 2,** MAB-A binds human ADAM9 with high affinity, but binds non-human primate (*e.g.*, cynomolgus monkey) ADAM9 to a lesser extent.

The amino acid sequences of the VL and VH Domains of MAB-A are provided below for illustration purposes and are not according to the claimed invention. The VH and VL Domains of MAB-A were humanized and the CDRs optimized to improve affinity and/or to remove potential amino acid liabilities. The $CDR_H3$ was further optimized to enhance binding to non-human primate ADAM9 while maintaining its high affinity for human ADAM9.

The preferred immunoconjugates of the present invention comprising 1, 2 or all 3 of the $CDR_H$s of a VH Domain and 1 or 2 of the $CDR_L$s of the VL Domain of an optimized variant of MAB-A, and preferably further possess the humanized framework regions **("FRs")** of the VH and/or VL Domains of humanized MAB-A. Other preferred immunoconjugates of the present invention possess the entire VH Domain of a humanized/optimized variant of MAB-A.

The invention particularly relates to immunoconjugates comprising:

(A)

(1) the three $CDR_L$s of the VL Domain of MAB-A; and
(2) the four FRs of the VL Domain of a humanized variant of MAB-A; or

(B) the three $CDR_H$s of the VH Domain of an optimized variant of MAB-A; and the three $CDR_L$s of the VL Domain of MAB-A; or

(C)

(1) the three $CDR_H$s of the VH Domain of an optimized variant of MAB-A; and
(2) the four FRs of the VH Domain of a humanized variant of MAB-A; or

(D)

(1) the VH Domain of a humanized/optimized variant of MAB-A; and
(2) the VL Domain of a humanized/optimized variant of MAB-A Murine Antibody "MAB-A"

The amino acid sequence of the VH Domain of the murine anti-ADAM9 antibody MAB-A is **SEQ ID NO:7,** shown for illustration purposes only and not according to the claimed invention, (the $CDR_H$ residues are shown underlined):

QVQLQQPGAE LVKPGASVKL SCKASGYTFT **SYWMH**WVKQR PGQGLEWIG**E**
**IIPINGHTNY NEKFKS**KATL TLDKSSSTAY MQLSSLASED SAVYYCAR**GG**
**YYYYGSRDYF DY**WGQGTTLT VSS

The amino acid sequence of the $CDR_H1$ Domain of MAB-A is **(SEQ ID NO:8):** SYWMH.

The amino acid sequence of the $CDR_H2$ Domain of MAB-A is **(SEQ ID NO:9):** EIIPINGHTNYNEKFKS.
The amino acid sequence of the $CDR_H3$ Domain of MAB-A is **(SEQ ID NO:10)**: GGYYYYGSRDYFDY.
The amino acid sequence of the VL Domain of the murine anti-ADAM9 antibody MAB-A is **SEQ ID NO:11,** shown for illustration purposes only and not according to the claimed invention, (the $CDR_L$ residues are shown underlined):

DIVLTQSPAS LAVSLGQRAT ISCKASQSVD YDGDSYMNWY QQIPGQPPKL LIYAASDLES GIPARFSGSG SGTDFTLNIH PVEEEDAATY YCQQSHEDPF TFGGGTKLEI K

The amino acid sequence of the $CDR_L1$ Domain of MAB-A is **(SEQ ID NO: 12):** KASQSVDYDGDSYMN.
The amino acid sequence of the $CDR_L2$ Domain of MAB-A is **(SEQ ID NO: 13):** AASDLES.
The amino acid sequence of the $CDR_L3$ Domain of MAB-A is **(SEQ ID NO: 14):** QQSHEDPFT.

**B. Exemplary Humanized/Optimized Anti-ADAM9-VH and VL Domains**

**1. Variant VH Domains of MAB-A**

The amino acid sequences of certain preferred humanized/optimized anti-ADAM9-VH Domains of MAB-A are variants of the ADAM9-VH Domain of MAB-A (**SEQ ID NO:7**).
The amino acid sequences of certain preferred humanized/optimized anti-ADAM9-VH Domains of MAB-A:

| | |
|---|---|
| hMAB-A VH(2A) (**SEQ ID NO:20**) | hMAB-A VH(2D) (**SEQ ID NO:23**) |
| hMAB-A VH(2B) (**SEQ ID NO:21**) | hMAB-A VH(2E) (**SEQ ID NO:24**) |
| hMAB-A VH(2C) (**SEQ ID NO:22**) | hMAB-A VH(2F) (**SEQ ID NO:25**) |
| | hMAB-A VH(2G) (**SEQ ID NO:26**) |
| | hMAB-A VH(2H) (**SEQ ID NO:27**) |
| | hMAB-A VH(2I) (**SEQ ID NO:28**) |
| and hMAB-A VH(2J) (**SEQ ID NO:29**) | |

are presented below ($CDR_H$ residues are shown in single underline; differences relative to hMAB-A VH(1) (**SEQ ID NO:7**) are shown in double underline).
The amino acid sequences of humanized/optimized anti-ADAM9-VH Domains of MAB-A:

hMAB-A VH(1) (**SEQ ID NO:16**)
hMAB-A VH(2) (**SEQ ID NO:17**)
hMAB-A VH(3) (**SEQ ID NO:18**)
hMAB-A VH(4) (**SEQ ID NO:19**)

are also given below but the above sequences do not form part of the presently-claimed invention.

hMAB-A VH(1) (**SEQ ID NO:16**):

EVQLVESGGG LVKPGGSLRL SCAASGFTFS SYWMHWVRQA PGKGLEWVGE IIPINGHTNY NEKFKSRFTI SLDNSKNTLY LQMGSLRAED TAVYYCARGG YYYYGSRDYF DYWGQGTTVT VSS

hMAB-A VH(2) (**SEQ ID NO:17**):

EVQLVESGGG LVKPGGSLRL SCAASGFTFS **SYWMH**WVRQA PGKGLEWVG**E**
**IIPIFGHTNY NEKFKS**RFTI SLDNSKNTLY LQMGSLRAED TAVYYCAR**GG**
**YYYYGSRDYF DY**WGQGTTVT VSS

hMAB-A VH(3) (**SEQ ID NO:18**):

EVQLVESGGG LVKPGGSLRL SCAASGFTFS **SYWMH**WVRQA PGKGLEWVG**E**
**IIPIFGHTNY NERFQG**RFTI SLDNSKNTLY LQMGSLRAED TAVYYCAR**GG**
**YYYYGSRDYF DY**WGQGTTVT VSS

hMAB-A VH(4) (**SEQ ID NO:19**):

EVQLVESGGG LVKPGGSLRL SCAASGFTFS **SYWIH**WVRQA PGKGLEWVG**E**
**IIPIFGHTNY NERFQG**RFTI SLDNSKNTLY LQMGSLRAED TAVYYCAR**GG**
**YYYYGSRDYF DY**WGQGTTVT VSS

hMAB-A VH(2A) (**SEQ ID NO:20**):

EVQLVESGGG LVKPGGSLRL SCAASGFTFS **SYWMH**WVRQA PGKGLEWVG**E**
**IIPIFGHTNY NEKFKS**RFTI SLDNSKNTLY LQMGSLRAED TAVYYCAR**GG**
**YYYYFNSGTL DY**WGQGTTVT VSS

hMAB-A VH(2B) (**SEQ ID NO:21**):

EVQLVESGGG LVKPGGSLRL SCAASGFTFS **SYWMH**WVRQA PGKGLEWVG**E**
**IIPIFGHTNY NEKFKS**RFTI SLDNSKNTLY LQMGSLRAED TAVYYCAR**GG**
**YYYYIGKGVL DY**WGQGTTVT VSS

hMAB-A VH(2C) (**SEQ ID NO:22**):

EVQLVESGGG LVKPGGSLRL SCAASGFTFS **SYWMH**WVRQA PGKGLEWVG**E**
**IIPIFGHTNY NEKFKS**RFTI SLDNSKNTLY LQMGSLRAED TAVYYCAR**GG**
**YYYYPRFGWL DY**WGQGTTVT VSS

hMAB-A VH(2D) (**SEQ ID NO:23**):

EVQLVESGGG LVKPGGSLRL SCAASGFTFS **SYWMH**WVRQA PGKGLEWVG**E**
**IIPIFGHTNY NEKFKS**RFTI SLDNSKNTLY LQMGSLRAED TAVYYCAR**GG**
**YYYYTGKGVL DY**WGQGTTVT VSS

hMAB-A VH(2E) (**SEQ ID NO:24**):

EVQLVESGGG LVKPGGSLRL SCAASGFTFS **SYWMH**WVRQA PGKGLEWVG**E IIPIFGHTNY NEKFKS**RFTI SLDNSKNTLY LQMGSLRAED TAVYYCAR**GG YYYYDSNAVL DY**WGQGTTVT VSS

hMAB-A VH(2F) (**SEQ ID NO:25**):

EVQLVESGGG LVKPGGSLRL SCAASGFTFS **SYWMH**WVRQA PGKGLEWVG**E IIPIFGHTNY NEKFKS**RFTI SLDNSKNTLY LQMGSLRAED TAVYYCAR**GG YYYYFHSGTL DY**WGQGTTVT VSS

hMAB-A VH(2G) (**SEQ ID NO:26**):

EVQLVESGGG LVKPGGSLRL SCAASGFTFS **SYWMH**WVRQA PGKGLEWVG**E IIPIFGHTNY NEKFKS**RFTI SLDNSKNTLY LQMGSLRAED TAVYYCAR**GG YYYYFNKAVL DY**WGQGTTVT VSS

hMAB-A VH(2H) (**SEQ ID NO:27**):

EVQLVESGGG LVKPGGSLRL SCAASGFTFS **SYWMH**WVRQA PGKGLEWVG**E IIPIFGHTNY NEKFKS**RFTI SLDNSKNTLY LQMGSLRAED TAVYYCAR**GG YYYYGGSGVL DY**WGQGTTVT VSS

hMAB-A VH(2I) (**SEQ ID NO:28**):

EVQLVESGGG LVKPGGSLRL SCAASGFTFS **SYWMH**WVRQA PGKGLEWVG**E IIPIFGHTNY NEKFKS**RFTI SLDNSKNTLY LQMGSLRAED TAVYYCAR**GG YYYYPRQGFL DY**WGQGTTVT VSS

hMAB-A VH(2J) (**SEQ ID NO:29**):

EVQLVESGGG LVKPGGSLRL SCAASGFTFS **SYWMH**WVRQA PGKGLEWVG**E IIPIFGHTNY NEKFKS**RFTI SLDNSKNTLY LQMGSLRAED TAVYYCAR**GG YYYYYNSGTL DY**WGQGTTVT VSS

Suitable amino acid sequences for the FRs of a humanized and/or optimized anti-ADAM9-VH Domain of MAB-A are:

$FR_H1$ Domain (**SEQ ID NO:30**): EVQLVESGGGLVKPGGSLRLSCAASGFTFS

$FR_H2$ Domain (**SEQ ID NO:31**): WVRQAPGKGLEWVG

$FR_H3$ Domain (**SEQ ID NO:32**): RFTISLDNSKNTLYLQMGSLRAEDTAVYYCAR

$FR_H4$ Domain (**SEQ ID NO:33**): WGQGTTVTVSS

The amino acid sequence for the $CDR_H1$ Domain of an anti-ADAM9-VH Domain is one of:

**SEQ ID NO:8**: SYWMH
**SEQ ID NO:34**: SYWIH

The amino acid sequence for the $CDR_H2$ Domain of an anti-ADAM9-VH Domain is one of:

**SEQ ID** N**O:9**: EIIPINGHTNYNEKFKS
**SEQ ID NO:35**: EIIPIFGHTNYNEKFKS
**SEQ ID NO:36**: EIIPIFGHTNYNERFQG

The amino acid sequence for the $CDR_H3$ Domain of an anti-ADAM9-VH Domain is one of:

**SEQ ID NO:37**: GGYYYYFNSGTLDY
**SEQ ID NO:38**: GGYYYYIGKGVLDY
**SEQ ID NO:39**: GGYYYYPRFGWLDY
**SEQ ID NO:40**: GGYYYYTGKGVLDY
**SEQ ID NO:41**: GGYYYYDSNAVLDY
**SEQ ID NO:42**: GGYYYYFHSGTLDY
**SEQ ID NO:43**: GGYYYYFNKAVLDY
**SEQ ID NO:44**: GGYYYYGGSGVLDY
**SEQ ID NO:45**: GGYYYYPRQGFLDY
**SEQ ID NO:46**: GGYYYYYNSGTLDY

A first exemplary humanized/optimized IgG1 Heavy Chain of a derivative/variant of MAB-A that falls outside of the scope of the presently-claimed invention contains the hMAB-A **VH (2)** Domain (**SEQ ID NO:17**), and has the amino acid sequence (**SEQ ID NO:50**):

```
EVQLVESGGG LVKPGGSLRL SCAASGFTFS SYWMHWVRQA PGKGLEWVGE
IIPIFGHTNY NEKFKSRFTI SLDNSKNTLY LQMGSLRAED TAVYYCARGG
YYYYGSRDYF DYWGQGTTVT VSSASTKGPS VFPLAPSSKS TSGGTAALGC
LVKDYFPEPV TVSWNSGALT SGVHTFPAVL QSSGLYSLSS VVTVPSSSLG
TQTYICNVNH KPSNTKVDKR VEPKSCDKTH TCPPCPAPEL LGGPSVFLFP
PKPKDTLMIS RTPEVTCVVV DVSHEDPEVK FNWYVDGVEV HNAKTKPREE
QYNSTYRVVS VLTVLHQDWL NGKEYKCKVS NKALPAPIEK TISKAKGQPR
EPQVYTLPPS REEMTKNQVS LTCLVKGFYP SDIAVEWESN GQPENNYKTT
PPVLDSDGSF FLYSKLTVDK SRWQQGNVFS CSVMHEALHN HYTQKSLSLS
PGX
```

wherein X is a lysine (K) or is absent.

A second exemplary humanized/optimized IgG1 Heavy Chain of a derivative/variant of MAB-A contains the hMAB-A **VH (2C)** Domain (**SEQ ID NO:22**), and has the amino acid sequence (**SEQ ID NO:51**):

EVQLVESGGG LVKPGGSLRL SCAASGFTFS SYWMHWVRQA PGKGLEWVGE
IIPIFGHTNY NEKFKSRFTI SLDNSKNTLY LQMGSLRAED TAVYYCARGG
YYYYPRFGWL DYWGQGTTVT VSSASTKGPS VFPLAPSSKS TSGGTAALGC
LVKDYFPEPV TVSWNSGALT SGVHTFPAVL QSSGLYSLSS VVTVPSSSLG
TQTYICNVNH KPSNTKVDKR VEPKSCDKTH TCPPCPAPEL LGGPSVFLFP
PKPKDTLMIS RTPEVTCVVV DVSHEDPEVK FNWYVDGVEV HNAKTKPREE
QYNSTYRVVS VLTVLHQDWL NGKEYKCKVS NKALPAPIEK TISKAKGQPR
EPQVYTLPPS REEMTKNQVS LTCLVKGFYP SDIAVEWESN GQPENNYKTT
PPVLDSDGSF FLYSKLTVDK SRWQQGNVFS CSVMHEALHN HYTQKSLSLS
PGX

wherein X is a lysine (K) or is absent.

A third exemplary humanized/optimized IgG1 Heavy Chain of a derivative/variant of MAB-A contains the hMAB-A **VH (2I)** Domain (**SEQ ID NO:28**), and has the amino acid sequence (**SEQ ID NO:52**):

EVQLVESGGG LVKPGGSLRL SCAASGFTFS SYWMHWVRQA PGKGLEWVGE
IIPIFGHTNY NEKFKSRFTI SLDNSKNTLY LQMGSLRAED TAVYYCARGG
YYYYPRQGFL DYWGQGTTVT VSSASTKGPS VFPLAPSSKS TSGGTAALGC
LVKDYFPEPV TVSWNSGALT SGVHTFPAVL QSSGLYSLSS VVTVPSSSLG
TQTYICNVNH KPSNTKVDKR VEPKSCDKTH TCPPCPAPEL LGGPSVFLFP
PKPKDTLMIS RTPEVTCVVV DVSHEDPEVK FNWYVDGVEV HNAKTKPREE
QYNSTYRVVS VLTVLHQDWL NGKEYKCKVS NKALPAPIEK TISKAKGQPR
EPQVYTLPPS REEMTKNQVS LTCLVKGFYP SDIAVEWESN GQPENNYKTT
PPVLDSDGSF FLYSKLTVDK SRWQQGNVFS CSVMHEALHN HYTQKSLSLS
PGX

wherein X is a lysine (K) or is absent.

As provided above, the CH2-CH3 Domains of the Fc Region may be engineered for example, to reduce effector function and/or to introduce a conjugation site and/or to extend the serum half-life. In certain embodiments, the CH2-CH3 Domains of the exemplary humanized/optimized IgG1 Heavy Chains of the invention comprise one or more substitutions selected from: L234A, L235A, M252Y, S254T, T256E and S442C.

Thus, a fourth exemplary humanized/optimized IgG1 Heavy Chain of a derivative/variant of MAB-A contains the hMAB-A **VH (21)** Domain (**SEQ ID NO:28**), and further comprises the substitutions L234A, and L235A in the CH2-CH3 Domains of the Fc Region (**SEQ ID NO:78**) and has the amino acid sequence (**SEQ ID NO:141**)

EVQLVESGGG LVKPGGSLRL SCAASGFTFS SYWMHWVRQA PGKGLEWVGE
IIPIFGHTNY NEKFKSRFTI SLDNSKNTLY LQMGSLRAED TAVYYCARGG
YYYYPRQGFL DYWGQGTTVT VSSASTKGPS VFPLAPSSKS TSGGTAALGC
LVKDYFPEPV TVSWNSGALT SGVHTFPAVL QSSGLYSLSS VVTVPSSSLG
TQTYICNVNH KPSNTKVDKR VEPKSCDKTH TCPPCPAPE**A** **A**GGPSVFLFP
PKPKDTLMIS RTPEVTCVVV DVSHEDPEVK FNWYVDGVEV HNAKTKPREE
QYNSTYRVVS VLTVLHQDWL NGKEYKCKVS NKALPAPIEK TISKAKGQPR
EPQVYTLPPS REEMTKNQVS LTCLVKGFYP SDIAVEWESN GQPENNYKTT
PPVLDSDGSF FLYSKLTVDK SRWQQGNVFS CSVMHEALHN HYTQKSLSLS
PGX

wherein X is a lysine (K) or is absent.

A fifth exemplary humanized/optimized IgG1 Heavy Chain of a derivative/variant of MAB-A contains the hMAB-A **VH (2I)** Domain (**SEQ ID NO:28**), and further comprises the S442C substitution in the CH2-CH3 Domains of the Fc Region (**SEQ ID NO:79**) and has the amino acid sequence (**SEQ ID NO:142**):

EVQLVESGGG LVKPGGSLRL SCAASGFTFS SYWMHWVRQA PGKGLEWVGE
IIPIFGHTNY NEKFKSRFTI SLDNSKNTLY LQMGSLRAED TAVYYCARGG
YYYYPRQGFL DYWGQGTTVT VSSASTKGPS VFPLAPSSKS TSGGTAALGC
LVKDYFPEPV TVSWNSGALT SGVHTFPAVL QSSGLYSLSS VVTVPSSSLG
TQTYICNVNH KPSNTKVDKR VEPKSCDKTH TCPPCPAPEL LGGPSVFLFP
PKPKDTLMIS RTPEVTCVVV DVSHEDPEVK FNWYVDGVEV HNAKTKPREE
QYNSTYRVVS VLTVLHQDWL NGKEYKCKVS NKALPAPIEK TISKAKGQPR
EPQVYTLPPS REEMTKNQVS LTCLVKGFYP SDIAVEWESN GQPENNYKTT
PPVLDSDGSF FLYSKLTVDK SRWQQGNVFS CSVMHEALHN HYTQKSL**C**LS
PGX

wherein X is a lysine (K) or is absent.

A sixth exemplary humanized/optimized IgG1 Heavy Chain of a derivative/variant of MAB-A contains the hMAB-A **VH (2I)** Domain (**SEQ ID NO:28**), and further comprises the substitutions L234A, L235A and S442C in the CH2-CH3 Domains of the Fc Region (**SEQ ID NO:80**) and has the amino acid sequence (**SEQ ID NO:143**):

EVQLVESGGG LVKPGGSLRL SCAASGFTFS SYWMHWVRQA PGKGLEWVGE
IIPIFGHTNY NEKFKSRFTI SLDNSKNTLY LQMGSLRAED TAVYYCARGG
YYYYPRQGFL DYWGQGTTVT VSSASTKGPS VFPLAPSSKS TSGGTAALGC
LVKDYFPEPV TVSWNSGALT SGVHTFPAVL QSSGLYSLSS VVTVPSSSLG
TQTYICNVNH KPSNTKVDKR VEPKSCDKTH TCPPCPAPE**A** **A**GGPSVFLFP
PKPKDTLMIS RTPEVTCVVV DVSHEDPEVK FNWYVDGVEV HNAKTKPREE
QYNSTYRVVS VLTVLHQDWL NGKEYKCKVS NKALPAPIEK TISKAKGQPR
EPQVYTLPPS REEMTKNQVS LTCLVKGFYP SDIAVEWESN GQPENNYKTT
PPVLDSDGSF FLYSKLTVDK SRWQQGNVFS CSVMHEALHN HYTQKSL**C**LS
PGX

wherein X is a lysine (K) or is absent.

A seventh exemplary humanized/optimized IgG1 Heavy Chain of a derivative/variant of MAB-A contains the hMAB-A **VH (2I)** Domain (**SEQ ID NO:28**), and further comprises the substitutions M252Y, S254T and T256E in the CH2-CH3 Domains of the Fc Region (**SEQ ID NO:147**) and has the amino acid sequence (**SEQ ID NO:151**):

EVQLVESGGG LVKPGGSLRL SCAASGFTFS SYWMHWVRQA PGKGLEWVGE
IIPIFGHTNY NEKFKSRFTI SLDNSKNTLY LQMGSLRAED TAVYYCARGG
YYYYPRQGFL DYWGQGTTVT VSSASTKGPS VFPLAPSSKS TSGGTAALGC
LVKDYFPEPV TVSWNSGALT SGVHTFPAVL QSSGLYSLSS VVTVPSSSLG
TQTYICNVNH KPSNTKVDKR VEPKSCDKTH TCPPCPAPEL LGGPSVFLFP
PKPKDTL**Y**I**T** R**E**PEVTCVVV DVSHEDPEVK FNWYVDGVEV HNAKTKPREE
QYNSTYRVVS VLTVLHQDWL NGKEYKCKVS NKALPAPIEK TISKAKGQPR
EPQVYTLPPS REEMTKNQVS LTCLVKGFYP SDIAVEWESN GQPENNYKTT
PPVLDSDGSF FLYSKLTVDK SRWQQGNVFS CSVMHEALHN HYTQKSLSLS
PGX

wherein **X** is a lysine (K) or is absent.

An eighth exemplary humanized/optimized IgG1 Heavy Chain of a derivative/variant of MAB-A contains the hMAB-A VH (21) Domain (SEQ ID NO:28), and further comprises the substitutions M252Y, S254T, T256E, and S442C in the CH2-CH3 Domains of the Fc Region (SEQ ID NO:148) and has the amino acid sequence (SEQ ID NO:152):

```
EVQLVESGGG LVKPGGSLRL SCAASGFTFS SYWMHWVRQA PGKGLEWVGE
IIPIFGHTNY NEKFKSRFTI SLDNSKNTLY LQMGSLRAED TAVYYCARGG
YYYYPRQGFL DYWGQGTTVT VSSASTKGPS VFPLAPSSKS TSGGTAALGC
LVKDYFPEPV TVSWNSGALT SGVHTFPAVL QSSGLYSLSS VVTVPSSSLG
TQTYICNVNH KPSNTKVDKR VEPKSCDKTH TCPPCPAPEL LGGPSVFLFP
PKPKDTLYIT REPEVTCVVV DVSHEDPEVK FNWYVDGVEV HNAKTKPREE
QYNSTYRVVS VLTVLHQDWL NGKEYKCKVS NKALPAPIEK TISKAKGQPR
EPQVYTLPPS REEMTKNQVS LTCLVKGFYP SDIAVEWESN GQPENNYKTT
PPVLDSDGSF FLYSKLTVDK SRWQQGNVFS CSVMHEALHN HYTQKSLCLS
PGX
```

wherein **X** is a lysine (K) or is absent.

A ninth exemplary humanized/optimized IgG1 Heavy Chain of a derivative/variant of MAB-A contains the hMAB-A **VH (2I)** Domain (**SEQ ID NO:28**), and further comprises the substitutions L234A, L235A, M252Y, S254T and T256E in the CH2-CH3 Domains of the Fc Region (**SEQ ID NO:149**) and has the amino acid sequence (**SEQ ID NO:153**):

```
EVQLVESGGG LVKPGGSLRL SCAASGFTFS SYWMHWVRQA PGKGLEWVGE
IIPIFGHTNY NEKFKSRFTI SLDNSKNTLY LQMGSLRAED TAVYYCARGG
YYYYPRQGFL DYWGQGTTVT VSSASTKGPS VFPLAPSSKS TSGGTAALGC
LVKDYFPEPV TVSWNSGALT SGVHTFPAVL QSSGLYSLSS VVTVPSSSLG
TQTYICNVNH KPSNTKVDKR VEPKSCDKTH TCPPCPAPEA AGGPSVFLFP
PKPKDTLYIT REPEVTCVVV DVSHEDPEVK FNWYVDGVEV HNAKTKPREE
QYNSTYRVVS VLTVLHQDWL NGKEYKCKVS NKALPAPIEK TISKAKGQPR
EPQVYTLPPS REEMTKNQVS LTCLVKGFYP SDIAVEWESN GQPENNYKTT
PPVLDSDGSF FLYSKLTVDK SRWQQGNVFS CSVMHEALHN HYTQKSLSLS
PGX
```

wherein **X** is a lysine (K) or is absent.

A tenth exemplary humanized/optimized IgG1 Heavy Chain of a derivative/variant of MAB-A contains the hMAB-A **VH (2I)** Domain (**SEQ ID NO:28**), and further comprises the substitutions L234A, L235A, M252Y, S254T, T256E, and S442C in the CH2-CH3 Domains of the Fc Region (**SEQ ID NO:150**) and has the amino acid sequence (**SEQ ID NO:154**):

```
EVQLVESGGG LVKPGGSLRL SCAASGFTFS SYWMHWVRQA PGKGLEWVGE
IIPIFGHTNY NEKFKSRFTI SLDNSKNTLY LQMGSLRAED TAVYYCARGG
YYYYPRQGFL DYWGQGTTVT VSSASTKGPS VFPLAPSSKS TSGGTAALGC
LVKDYFPEPV TVSWNSGALT SGVHTFPAVL QSSGLYSLSS VVTVPSSSLG
TQTYICNVNH KPSNTKVDKR VEPKSCDKTH TCPPCPAPEA AGGPSVFLFP
PKPKDTLYIT REPEVTCVVV DVSHEDPEVK FNWYVDGVEV HNAKTKPREE
QYNSTYRVVS VLTVLHQDWL NGKEYKCKVS NKALPAPIEK TISKAKGQPR
EPQVYTLPPS REEMTKNQVS LTCLVKGFYP SDIAVEWESN GQPENNYKTT
PPVLDSDGSF FLYSKLTVDK SRWQQGNVFS CSVMHEALHN HYTQKSLCLS
PGX
```

wherein **X** is a lysine (K) or is absent.

**2. Variant VL Domains of** MAB-A

The amino acid sequences of preferred humanized/optimized anti-ADAM9-VL Domains of MAB-A are variants of the ADAM9-VL Domain of MAB-A (**SEQ ID NO:11**).

The amino acid sequences of a preferred humanized anti-ADAM9-VL Domain of MAB-A: hMAB-A VL(1) (**SEQ ID NO:54**), and of certain preferred humanized/optimized anti-ADAM9-VL Domains of MAB-A: hMAB-A VL(2) (**SEQ ID NO:55**), hMAB-A VL(3) (**SEQ ID NO:56**), and hMAB-A VL(4) (**SEQ ID NO:57**), are presented below ($CDR_L$ residues are shown in single underline; differences relative to hMAB-A VL(1) (**SEQ ID NO:54**) are shown in double underline).

hMAB-A VL(1) (**SEQ ID NO:54**):

DIVMTQSPDS LAVSLGERAT ISC**KASQSVD YDGDSYMN**WY QQKPGQPPKL
LIY**AASDLES** GIPARFSGSG SGTDFTLTIS SLEPEDFATY YC**QQSHEDPF**
**T**FGQGTKLEI K

hMAB-A VL(2) (**SEQ ID NO:55**):

DIVMTQSPDS LAVSLGERAT ISC**KASQSVD YSGDSYMN**WY QQKPGQPPKL
LIY**AASDLES** GIPARFSGSG SGTDFTLTIS SLEPEDFATY YC**QQSHEDPF**
**T**FGQGTKLEI K

hMAB-A VL(3) (**SEQ ID NO:56**):

DIVMTQSPDS LAVSLGERAT ISC**RASQSVD YSGDSYMN**WY QQKPGQPPKL
LIY**AASDLES** GIPARFSGSG SGTDFTLTIS SLEPEDFATY YC**QQSHEDPF**
**T**FGQGTKLEI K

hMAB-A VL(4) (**SEQ ID NO:57**):

DIVMTQSPDS LAVSLGERAT ISC**RASQSVD YSGDSYLN**WY QQKPGQPPKL
LIY**AASDLES** GIPARFSGSG SGTDFTLTIS SLEPEDFATY YC**QQSYSTPF**
**T**FGQGTKLEI K

Accordingly, suitable amino acid sequences for the FRs of a humanized and/or optimized anti-ADAM9-VL Domain of MAB-A are:

$FR_L1$ Domain (**SEQ ID NO:58**): DIVMTQSPDSLAVSLGERATISC

$FR_L2$ Domain (**SEQ ID NO:59**): WYQQKPGQPPKLLIY

$FR_L3$ Domain (**SEQ ID NO:60**): GIPARFSGSGSGTDFTLTISSLEPEDFATYYC

$FR_L4$ Domain (**SEQ ID NO:61**): FGQGTKLEIK

The amino acid sequence for the $CDR_L1$ Domain of an anti-ADAM9-VL Domain is one ofe:

**SEQ ID NO:12**: KASQSVDYDGDSYMN
**SEQ ID NO:62**: KASQSVDYSGDSYMN

**SEQ ID NO:63**: RASQSVDYSGDSYMN
**SEQ ID NO:64**: RASQSVDYSGDSYLN

The amino acid sequence for the $CDR_L3$ Domain of an anti-ADAM9-VL Domain is one of:

**SEQ ID NO:14**: QQSHEDPFT
**SEQ ID NO:65**: QQSYSTPFT

An exemplary humanized/optimized IgG1 Light Chain of a derivative/variant of MAB-A contains the hMAB-A VL (2) Domain (**SEQ ID NO:55**), and has the amino acid sequence (**SEQ ID NO:68**):

```
DIVMTQSPDS LAVSLGERAT ISCKASQSVD YSGDSYMNWY QQKPGQPPKL
LIYAASDLES GIPARFSGSG SGTDFTLTIS SLEPEDFATY YCQQSHEDPF
TFGQGTKLEI KRTVAAPSVF IFPPSDEQLK SGTASVVCLL NNFYPREAKV
QWKVDNALQS GNSQESVTEQ DSKDSTYSLS STLTLSKADY EKHKVYACEV
THQGLSSPVT KSFNRGEC
```

The invention further contemplates such immunoconjugates that further comprise any of the above-provided humanized MAB-A $FR_H1$, $FR_H2$, $FR_H3$, or $FR_H4$, $FR_L1$, $FR_L2$, $FR_L3$, or $FR_L4$, and particularly contemplates such immunoconjugates that comprise $FR_H1$, $FR_H2$, $FR_H3$, and $FR_H4$, and/or that comprise $FR_L1$, $FR_L2$, $FR_L3$, $FR_L4$ and $FR_H1$.

In some embodiments, the humanized/optimized anti-ADAM9 antibody or ADAM9-binding fragment thereof includes a $CDR_H1$ domain, a $CDR_H2$ domain, and a $CDR_H3$ domain and a $CDR_L1$ domain, a $CDR_L2$ domain, and a $CDR_L3$ domain having the sequences selected from the group consisting of:

(a) SEQ ID NOs: 8, 35, and 37 and SEQ ID NOs: 62, 13, 14, respectively;
b) SEQ ID NOs: 8, 35, and 38 and SEQ ID NOs: 62, 13, 14, respectively;
(c) SEQ ID NOs: 8, 35, and 39 and SEQ ID NOs: 62, 13, 14, respectively;
(d) SEQ ID NOs: 8, 35, and 40 and SEQ ID NOs: 62, 13, 14, respectively;
(e) SEQ ID NOs: 8, 35, and 41 and SEQ ID NOs: 62, 13, 14, respectively;
(f) SEQ ID NOs: 8, 35, and 42 and SEQ ID NOs: 62, 13, 14, respectively;
(g) SEQ ID NOs: 8, 35, and 43 and SEQ ID NOs: 62, 13, 14, respectively;
(h) SEQ ID NOs: 8, 35, and 44 and SEQ ID NOs: 62, 13, 14, respectively;
(i) SEQ ID NOs: 8, 35, and 45 and SEQ ID NOs: 62, 13, 14, respectively; and
(j) SEQ ID NOs: 8, 35, and 46 and SEQ ID NOs: 62, 13, 14, respectively.

In particular embodiments, the humanized/optimized anti-ADAM9 antibody or ADAM9-binding fragment thereof includes a $CDR_H1$ domain, a $CDR_H2$ domain, and a $CDR_H3$ domain and a $CDR_L1$ domain, a $CDR_L2$ domain, and a $CDR_L3$ domain having the sequences of SEQ ID NOs: 8, 35, and 45 and SEQ ID NOs: 62, 13, 14, respectively.

In some embodiments, the humanized/optimized anti-ADAM9 antibody or ADAM9-binding fragment thereof includes a heavy chain variable domain (VH) and a light chain variable domain (VL) having sequences that are at least 90%, at least 95%, at least 99%, or are 100% identical to the sequences as follows:

(a) SEQ ID N0:20 and SEQ ID NO:55, respectively;
(b) SEQ ID NO:21 and SEQ ID NO:55, respectively;
(c) SEQ ID NO:22 and SEQ ID NO:55, respectively;
(d) SEQ ID NO:23 and SEQ ID NO:55, respectively;
(e) SEQ ID NO:24 and SEQ ID NO:55, respectively;
(f) SEQ ID NO:25 and SEQ ID NO:55, respectively;
(g) SEQ ID NO:26 and SEQ ID NO:55, respectively;
(h) SEQ ID NO:27 and SEQ ID NO:55, respectively;
(i) SEQ ID NO:28 and SEQ ID NO:55, respectively; and
(j) SEQ ID NO:29 and SEQ ID NO:55, respectively

By "substantially identical" or "identical" is meant a polypeptide exhibiting at least 50% identity to a reference amino acid sequence (for example, any one of the amino acid sequences described herein). Preferably, such a sequence is at least 60%, more preferably at least 80% or at least 85%, and more preferably at least 90%, at least 95% at least

99%, or even 100% identical at the amino acid level or nucleic acid to the sequence used for comparison.

Sequence identity is typically measured using sequence analysis software (for example, Sequence Analysis Software Package of the Genetics Computer Group, University of Wisconsin Biotechnology Center, 1710 University Avenue, Madison, Wis. 53705, BLAST, BESTFIT, GAP, or PILEUP/PRETTYBOX programs). Such software matches identical or similar sequences by assigning degrees of homology to various substitutions, deletions, and/or other modifications. Conservative substitutions typically include substitutions within the following groups: glycine, alanine; valine, isoleucine, leucine; aspartic acid, glutamic acid, asparagine, glutamine; serine, threonine; lysine, arginine; and phenylalanine, tyrosine. In an exemplary approach to determining the degree of identity, a BLAST program may be used, with a probability score between $e^{-3}$ and $e^{-100}$ indicating a closely related sequence.

In particular embodiments, the humanized/optimized anti-ADAM9 antibody or ADAM9-binding fragment thereof includes a heavy chain variable domain (VH) and a light chain variable domain (VL) having sequences that are at least 90%, at least 95%, at least 99%, or are 100% identical to the sequences of SEQ ID NO:28 and SEQ ID NO:55, respectively.

In certain embodiments, the humanized/optimized anti-ADAM9 antibody comprises a heavy chain and a light chain sequence as follows:

(a) SEQ ID NO:51 and SEQ ID NO:68, respectively;
(b) SEQ ID NO:52 and SEQ ID NO:68, respectively
(c) SEQ ID NO: 141 and SEQ ID NO:68, respectively;
(d) SEQ ID NO:142 and SEQ ID NO:68, respectively;
(e) SEQ ID NO: 143 and SEQ ID NO:68, respectively;
(f) SEQ ID NO: 151 and SEQ ID NO:68, respectively;
(g) SEQ ID NO: 152 and SEQ ID NO:68, respectively;
(h) SEQ ID NO:153 and SEQ ID NO:68, respectively; and
(i) SEQ ID NO: 154 and SEQ ID NO:68, respectively.

In particular embodiments, the humanized/optimized anti-ADAM9 antibody comprises a heavy chain having the sequence of SEQ ID NO:52 and a light chain having the sequence of SEQ ID NO:68. In other particular embodiments, the humanized/optimized anti-ADAM9 antibody comprises a heavy chain having the sequence of SEQ ID NO:142 and a light chain having the sequence of SEQ ID NO:68. In other embodiments, the humanized/optimized anti-ADAM9 antibody is engineered for extended serum half life and comprises a heavy chain having the sequence of SEQ ID NO:151 and a light chain having the sequence of SEQ ID NO:68. In other particular embodiments, the humanized/optimized anti-ADAM9 antibody is engineered for extended serum half life and for site specific conjugation and comprises a heavy chain having the sequence of SEQ ID NO: 152 and a light chain having the sequence of SEQ ID NO:68.

The present invention also expressly contemplates immunoconjugates that immunospecifically bind to an epitope of a human ADAM9 polypeptide, and that comprise any of the above-provided humanized/optimized anti-ADAM9 MAB-A VL or VH Domains. The present invention particularly contemplates such anti-ADAM9 antibodies and ADAM9-binding fragments thereof that comprise any of the following combinations of humanized/optimized anti-ADAM9 VL or VH Domains:

| hMAB-A **VH** / hMAB-A **VL Combinations** | |
|---|---|
| | hMAB-A VH(2D) / hMAB-A VL(1)<br>hMAB-A VH(2D) / hMAB-A VL(2)<br>hMAB-A VH(2D) / hMAB-A VL(3)<br>hMAB-A VH(2D) / hMAB-A VL(4) |
| | hMAB-A VH(2E) / hMAB-A VL(1)<br>hMAB-A VH(2E) / hMAB-A VL(2)<br>hMAB-A VH(2E) / hMAB-A VL(3)<br>hMAB-A VH(2E) / hMAB-A VL(4) |
| | hMAB-A VH(2F) / hMAB-A VL(1)<br>hMAB-A VH(2F) / hMAB-A VL(2)<br>hMAB-A VH(2F) / hMAB-A VL(3)<br>hMAB-A VH(2F) / hMAB-A VL(4) |

(continued)

| hMAB-A **VH** / hMAB-A **VL Combinations** | |
|---|---|
| | hMAB-A VH(2G) / hMAB-A VL(1)<br>hMAB-A VH(2G) / hMAB-A VL(2)<br>hMAB-A VH(2G) / hMAB-A VL(3)<br>hMAB-A VH(2G) / hMAB-A VL(4) |
| hMAB-A VH(2A) / hMAB-A VL(1)<br>hMAB-A VH(2A) / hMAB-A VL(2)<br>hMAB-A VH(2A) / hMAB-A VL(3)<br>hMAB-A VH(2A) / hMAB-A VL(4) | hMAB-A VH(2H) / hMAB-A VL(1)<br>hMAB-A VH(2H) / hMAB-A VL(2)<br>hMAB-A VH(2H) / hMAB-A VL(3)<br>hMAB-A VH(2H) / hMAB-A VL(4) |
| hMAB-A VH(2B) / hMAB-A VL(1)<br>hMAB-A VH(2B) / hMAB-A VL(2)<br>hMAB-A VH(2B) / hMAB-A VL(3)<br>hMAB-A VH(2B) / hMAB-A VL(4) | hMAB-A VH(2I) / hMAB-A VL(1)<br>hMAB-A VH(2I) / hMAB-A VL(2)<br>hMAB-A VH(2I) / hMAB-A VL(3)<br>hMAB-A VH(2I) / hMAB-A VL(4) |
| hMAB-A VH(2C) / hMAB-A VL(1)<br>hMAB-A VH(2C) / hMAB-A VL(2)<br>hMAB-A VH(2C) / hMAB-A VL(3)<br>hMAB-A VH(2C) / hMAB-A VL(4) | hMAB-A VH(2J) / hMAB-A VL(1)<br>hMAB-A VH(2J) / hMAB-A VL(2)<br>hMAB-A VH(2J) / hMAB-A VL(3)<br>hMAB-A VH(2J) / hMAB-A VL(4) |

The present invention specifically encompasses immunoconjugates comprising a humanized/optimized anti-ADAM9-VL and/or VH Domain as provided above. In particular embodiments, the immunoconjugates of the present invention comprise (i) a humanized/optimized anti-ADAM9-VL and/or VH Domain as provided above, and (ii) an Fc Region.

Although particular modifications to anti-ADAM9 VH and VL Domains are summarized above and compared in **FIGs. 3A-3B**, it is not necessary to modify all or most of these residues when engineering a humanized and/or optimized anti-ADAM9-VH or VL Domain of the invention. The present invention also encompasses minor variations of these VH and VL sequences including, for example, amino acid substitutions of the C-terminal and/or N-terminal amino acid residues which may be introduced to facilitate subcloning.

**III. Antibody Drug Conjugates**

*Definitions*

The term "**immunoconjugate**," "**conjugate**," or "**ADC**" as used herein refers to a compound or a derivative thereof (a pharmacological agent) that is linked to or conjugated to an anti-ADAM9 antibody or ADAM9-binding fragment thereof as described herein.

A "**linker**" is any chemical moiety that is capable of linking a pharmacological agent described herein (*e.g.*, maytansinoid or (indolinobenzodiazepine) compounds), to an anti-ADAM9 antibody or ADAM9-binding fragment thereof in a stable, covalent manner. Linkers can be susceptible to or be substantially resistant to acid-induced cleavage, light-induced cleavage, peptidase-induced cleavage, esterase-induced cleavage, and disulfide bond cleavage, at conditions under which the compound or the antibody remains active. Suitable linkers are well known in the art and include, for example, disulfide groups, thioether groups, acid labile groups, photolabile groups, peptidase labile groups and esterase labile groups. Linkers also include charged linkers, and hydrophilic forms thereof as described herein and know in the art.

**"Alkyl"** as used herein refers to a saturated linear or branched-chain monovalent hydrocarbon radical of one to twenty carbon atoms. Examples of alkyl include, but are not limited to, methyl, ethyl, 1-propyl, 2-propyl, 1-butyl, 2-methyl-1-propyl, $-CH_2CH(CH_3)_2$), 2-butyl, 2-methyl-2-propyl, 1-pentyl, 2-pentyl 3-pentyl, 2-methyl-2-butyl, 3-methyl-2-butyl, 3-methyl-1-butyl, 2-methyl-1-butyl, 1-hexyl), 2-hexyl, 3-hexyl, 2-methyl-2-pentyl, 3-methyl-2-pentyl, 4-methyl-2-pentyl, 3-methyl-3-pentyl, 2-methyl-3-pentyl, 2,3-dimethyl-2-butyl, 3,3-dimethyl-2-butyl, 1-heptyl, 1-octyl, and the like. Preferably, the alkyl has one to ten carbon atoms. More preferably, the alkyl has one to four carbon atoms.

The number of carbon atoms in a group can be specified herein by the prefix "$C_{x-xx}$", wherein x and xx are integers. For example, "$C_{1-4}$alkyl" is an alkyl group having from 1 to 4 carbon atoms.

The term "**compound**" or "**cytotoxic compound**," or "**cytotoxic agent**" are used interchangeably. They are intended to include compounds for which a structure or formula or any derivative thereof has been disclosed in the

present invention or a structure or formula or any derivative thereof that has been incorporated by reference. The term also includes, stereoisomers, geometric isomers, tautomers, solvates, metabolites, and salts (*e.g.,* pharmaceutically acceptable salts) of a compound of all the formulae disclosed in the present invention. The term also includes any solvates, hydrates, and polymorphs of any of the foregoing. The specific recitation of "stereoisomers," "geometric isomers," "tautomers," "solvates," "metabolites," "salt", "conjugates," "conjugates salt," "solvate," "hydrate," or "polymorph" in certain aspects of the invention described in this application shall not be interpreted as an intended omission of these forms in other aspects of the invention where the term "compound" is used without recitation of these other forms.

The term "**chiral**" refers to molecules that have the property of non-superimposability of the mirror image partner, while the term "achiral" refers to molecules that are superimposable on their mirror image partner.

The term "**stereoisomer**" refers to compounds that have identical chemical constitution and connectivity, but different orientations of their atoms in space that cannot be interconverted by rotation about single bonds.

"**Diastereomer**" refers to a stereoisomer with two or more centers of chirality and whose molecules are not mirror images of one another. Diastereomers have different physical properties, e.g. melting points, boiling points, spectral properties, and reactivities. Mixtures of diastereomers can separate under high resolution analytical procedures such as crystallization, electrophoresis and chromatography.

"**Enantiomers**" refer to two stereoisomers of a compound that are non-superimposable mirror images of one another.

Stereochemical definitions and conventions used herein generally follow S. P. Parker, Ed., McGraw-Hill, Dictionary of Chemical Terms (1984) McGraw-Hill Book Company, New York; and Eliel, E. and Wilen, S., Stereochemistry of Organic Compounds, John Wiley & Sons, Inc., New York, 1994. The compounds of the invention can contain asymmetric or chiral centers, and therefore exist in different stereoisomeric forms. It is intended that all stereoisomeric forms of the compounds of the invention, including diastereomers, enantiomers and atropisomers, as well as mixtures thereof such as racemic mixtures, form part of the present invention. Many organic compounds exist in optically active forms, *i.e.,* they have the ability to rotate the plane of plane-polarized light. In describing an optically active compound, the prefixes D and L, or R and S, are used to denote the absolute configuration of the molecule about its chiral center(s). The prefixes d and I or (+) and (-) are employed to designate the sign of rotation of plane-polarized light by the compound, with (-) or 1 meaning that the compound is levorotatory. A compound prefixed with (+) or d is dextrorotatory. For a given chemical structure, these stereoisomers are identical except that they are mirror images of one another. A specific stereoisomer can also be referred to as an enantiomer, and a mixture of such isomers is often called an enantiomeric mixture. A 50:50 mixture of enantiomers is referred to as a racemic mixture or a racemate, which can occur where there has been no stereoselection or stereospecificity in a chemical reaction or process. The terms "racemic mixture" and "racemate" refer to an equimolar mixture of two enantiomeric species, devoid of optical activity.

The term "**tautomer**" or "**tautomeric form**" refers to structural isomers of different energies that are interconvertible via a low energy barrier. For example, proton tautomers (also known as prototropic tautomers) include interconversions via migration of a proton, such as keto-enol and imine-enamine isomerizations. Valence tautomers include interconversions by reorganization of some of the bonding electrons.

The term "imine reactive reagent" refers to a reagent that is capable of reacting with an imine group. Examples of imine reactive reagent includes sulfites ($H_2SO_3$, $H_2SO_2$ or a salt of $HSO_3^-$, $SO_3^{2-}$ or $HSO_2^-$formed with a cation), metabisulfite ($H_2S_2O_5$ or a salt of $S_2O_5^{2-}$formed with a cation), mono, di, tri, and tetra- thiophosphates ($PO_3SH_3$, $PO_2S_2H_3$, $POS_3H_3$, $PS_4H_3$ or a salt of $PO_3S^{3-}$, $PO_2S_2^{3-}$, $POS_3^{3-}$ or $PS_4^{3-}$ formed with a cation), thio phosphate esters ($(R^iO)_2PS(OR^i)$, $R^iSH$, $R^iSOH$, $R^iSO_2H$, $R^iSO_3H$), various amines (hydroxyl amine (*e.g.*, $NH_2OH$), hydrazine (*e.g.*, $NH_2NH_2$), $NH_2O-R^i$, $R^{i'}NH-R^i$, $NH_2-R^i$), $NH_2-CO-NH_2$, $NH_2-C(=S)-NH_2$, thiosulfate ($H_2S_2O_3$ or a salt of $S_2O_3^{2-}$ formed with a cation), dithionite ($H_2S_2O_4$ or a salt of $S_2O_4^{2-}$ formed with a cation), phosphorodithioate ($P(=S)(OR^k)(SH)(OH)$ or a salt thereof formed with a cation), hydroxamic acid ($R^kC(=O)NHOH$ or a salt formed with a cation), hydrazide ($R^kCONHNH_2$), formaldehyde sulfoxylate ($HOCH_2SO_2H$ or a salt of $HOCH_2SO_2^-$ formed with a cation, such as $HOCH_2SO_2^-Na^+$), glycated nucleotide (such as GDP-mannose), fludarabine or a mixture thereof, wherein $R^i$ and $R^{i'}$ are each independently a linear or branched alkyl having 1 to 10 carbon atoms and are substituted with at least one substituent selected from $-N(R^j)_2$, $-CO_2H$, $-SO_3H$, and $-PO_3H$; $R^i$ and $R^{i'}$ can be further optionally substituted with a substituent for an alkyl described herein; $R^j$ is a linear or branched alkyl having 1 to 6 carbon atoms; and $R^k$ is a linear, branched or cyclic alkyl, alkenyl or alkynyl having 1 to 10 carbon atoms, aryl, heterocyclyl or heteroaryl (preferably, $R^k$ is a linear or branched alkyl having 1 to 4 carbon atoms; more preferably, $R^k$ is methyl, ethyl or propyl). Preferably, the cation is a monovalent cation, such as $Na^+$ or $K^+$. Preferably, the imine reactive reagent is selected from sulfites, hydroxyl amine, urea and hydrazine. More preferably, the imine reactive reagent is $NaHSO_3$ or $KHSO_3$.

The term "cation" refers to an ion with positive charge. The cation can be monovalent (e.g., Na+, K+, NH4+ etc.), bi-valent (e.g., Ca2+, Mg2+, etc.) or multi-valent (e.g., A13+ etc.). Preferably, the cation is monovalent

The phrase "**pharmaceutically acceptable salt**" as used herein, refers to pharmaceutically acceptable organic or inorganic salts of a compound of the invention. Exemplary salts include to sulfate, citrate, acetate, oxalate, chloride, bromide, iodide, nitrate, bisulfate, phosphate, acid phosphate, isonicotinate, lactate, salicylate, acid citrate, tartrate,

oleate, tannate, pantothenate, bitartrate, ascorbate, succinate, maleate, gentisinate, fumarate, gluconate, glucuronate, saccharate, formate, benzoate, glutamate, methanesulfonate "mesylate," ethanesulfonate, benzenesulfonate, p-toluenesulfonate, pamoate (*i.e.,* 1,1'-methylene-bis-(2-hydroxy-3-naphthoate)) salts, alkali metal (*e.g.*, sodium and potassium) salts, alkaline earth metal (*e.g.*, magnesium) salts, and ammonium salts. A pharmaceutically acceptable salt can involve the inclusion of another molecule such as an acetate ion, a succinate ion or other counter ion. The counter ion can be any organic or inorganic moiety that stabilizes the charge on the parent compound. Furthermore, a pharmaceutically acceptable salt can have more than one charged atom in its structure. Instances where multiple charged atoms are part of the pharmaceutically acceptable salt can have multiple counter ions. Hence, a pharmaceutically acceptable salt can have one or more charged atoms and/or one or more counter ion.

If the compound is a base, the desired pharmaceutically acceptable salt can be prepared by any suitable method available in the art, for example, treatment of the free base with an inorganic acid, such as hydrochloric acid, hydrobromic acid, sulfuric acid, nitric acid, methanesulfonic acid, phosphoric acid and the like, or with an organic acid, such as acetic acid, maleic acid, succinic acid, mandelic acid, fumaric acid, malonic acid, pyruvic acid, oxalic acid, glycolic acid, salicylic acid, a pyranosidyl acid, such as glucuronic acid or galacturonic acid, an alpha hydroxy acid, such as citric acid or tartaric acid, an amino acid, such as aspartic acid or glutamic acid, an aromatic acid, such as benzoic acid or cinnamic acid, a sulfonic acid, such as p-toluenesulfonic acid or ethanesulfonic acid, or the like.

If the compound is an acid, the desired pharmaceutically acceptable salt can be prepared by any suitable method, for example, treatment of the free acid with an inorganic or organic base, such as an amine (primary, secondary or tertiary), an alkali metal hydroxide or alkaline earth metal hydroxide, or the like. Illustrative examples of suitable salts include organic salts derived from amino acids, such as glycine and arginine, ammonia, primary, secondary, and tertiary amines, and cyclic amines, such as piperidine, morpholine and piperazine, and inorganic salts derived from sodium, calcium, potassium, magnesium, manganese, iron, copper, zinc, aluminum and lithium.

As used herein, the term "**solvate**" means a compound that further includes a stoichiometric or non-stoichiometric amount of solvent such as water, isopropanol, acetone, ethanol, methanol, DMSO, ethyl acetate, acetic acid, and ethanolamine dichloromethane, 2-propanol, or the like, bound by non-covalent intermolecular forces. Solvates or hydrates of the compounds are readily prepared by addition of at least one molar equivalent of a hydroxylic solvent such as methanol, ethanol, 1-propanol, 2-propanol or water to the compound to result in solvation or hydration of the imine moiety.

A "**metabolite**" or "**catabolite**" is a product produced through metabolism or catabolism in the body of a specified compound, a derivative thereof, or a conjugate thereof, or salt thereof. Metabolites of a compound, a derivative thereof, or a conjugate thereof, can be identified using routine techniques known in the art and their activities determined using tests such as those described herein. Such products can result for example from the oxidation, hydroxylation, reduction, hydrolysis, amidation, deamidation, esterification, deesterification, enzymatic cleavage, and the like, of the administered compound.

The phrase "**pharmaceutically acceptable**" indicates that the substance or composition must be compatible chemically and/or toxicologically, with the other ingredients comprising a formulation, and/or the mammal being treated therewith.

The term "**protecting group**" or "**protecting moiety**" refers to a substituent that is commonly employed to block or protect a particular functionality while reacting other functional groups on the compound, a derivative thereof, or a conjugate thereof. For example, an "amine-protecting group" or an "amino-protecting moiety" is a substituent attached to an amino group that blocks or protects the amino functionality in the compound. Such groups are well known in the art (see for example P. Wuts and T. Greene, 2007, Protective Groups in Organic Synthesis, Chapter 7, J. Wiley & Sons, NJ) and exemplified by carbamates such as methyl and ethyl carbamate, FMOC, substituted ethyl carbamates, carbamates cleaved by 1,6-β-elimination (also termed "self immolative"), ureas, amides, peptides, alkyl and aryl derivatives. Suitable amino-protecting groups include acetyl, trifluoroacetyl, t-butoxycarbonyl (BOC), benzyloxycarbonyl (CBZ) and 9-fluorenylmethylenoxycarbonyl (Fmoc). For a general description of protecting groups and their use, *see* P. G.M. Wuts & T. W. Greene, Protective Groups in Organic Synthesis, John Wiley & Sons, New York, 2007.

The term "**amino acid**" refers to naturally occurring amino acids or non-naturally occurring amino acid. In one embodiment, the amino acid is represented by $NH_2$-C($R^{aa'}R^{aa}$)-C(=O)OH, wherein $R^{aa}$ and $R^{aa'}$ are each independently H, an optionally substituted linear, branched or cyclic alkyl, alkenyl or alkynyl having 1 to 10 carbon atoms, aryl, heteroaryl or heterocyclyl or $R^{aa}$ and the N-terminal nitrogen atom can together form a heterocycyclic ring (*e.g.,* as in proline). The term "**amino acid residue**" refers to the corresponding residue when one hydrogen atom is removed from the amine and/or carboxy end of the amino acid, such as -NH-C($R^{aa'}R^{aa}$)-C(=O)O-.

The term "**peptide**" refers to short chains of amino acid monomers linked by peptide (amide) bonds. In some embodiments, the peptides contain 2 to 20 amino acid residues. In other embodiments, the peptides contain 2 to 10 amino acid residus. In yet other embodiments, the peptides contain 2 to 5 amino acid residues. As used herein, when a peptide is a portion of a cytotoxic agent or a linker described herein represented by a specific sequence of amino acids, the peptide can be connected to the rest of the cytotoxic agent or the linker in both directions. For example, a dipeptide XI-X2 includes XI-X2 and X2-X1. Similarly, a tripeptide X1-X2-X3 includes X1-X2-X3 and X3-X2-X1 and a

tetrapeptide X1-X2-X3-X4 includes X1-X2-X3-X4 and X4-X2-X3-X1. X1, X¬2, X3 and X4 represents an amino acid residue.

The term "**reactive ester group**" refers to a group an ester group that can readily react with an amine group to form amide bond. Exemplary reactive ester groups include N-hydroxysuccinimide esters, N-hydroxyphthalimide esters, N-hydroxy sulfo-succinimide esters, para-nitrophenyl esters, dinitrophenyl esters, pentafluorophenyl esters and their derivatives, wherein said derivatives facilitate amide bond formation. In certain embodiments, the reactive ester group is a N-hydroxysuccinimide ester or a N-hydroxy sulfo-succinimide ester.

The term "**amine reactive group**" refers to a group that can react with an amine group to form a covalent bond. Exemplary amine reactive groups include reactive ester groups, acyl halides, sulfonyl halide, imidoester, or a reactive thioester groups. In certain embodiments, the amine reactive group is a reactive ester group. In one embodiment, the amine reactive group is a N-hydroxysuccinimide ester or a N-hydroxy sulfo-succinimide ester.

The term "**thiol-reactive group**" refers to a group that can react with a thiol (-SH) group to form a covalent bond. Exemplary thiol-reactive groups include maleimide, haloacetyl, aloacetamide, vinyl sulfone, vinyl sulfonamide or vinyal pyridine. In one embodiment, the thiol-reactive group is maleimide.

As used in the present disclosure and claims, the singular forms "**a**," "**an**," and "**the**" include plural forms unless the context clearly dictates otherwise.

It is understood that wherever embodiments are described herein with the language "**comprising**," otherwise analogous embodiments described in terms of "**consisting of**" and/or "**consisting essentially of**" are also provided.

## A. Exemplary Immunoconjugates

The present invention relates to immunoconjugates comprising an anti-ADAM9 antibody or an ADAM9-binding fragment thereof (as defined herein) conjugated to at least one pharmacological agent. Pharmacological agents include but are not limited to cytotoxins, (*e.g.,* a cytostatic or cytocidal agent), therapeutic agents, and radioactive metal ions, *e.g.,* alpha-emitters. Cytotoxins or cytotoxic agents include any agent that is detrimental to cells such as, for example, Pseudomonas exotoxin, Diptheria toxin, a botulinum toxin A through F, ricin abrin, saporin, and cytotoxic fragments of such agents. Therapeutic agents include any agent having a therapeutic effect to prophylactically or therapeutically treat a disorder. Such therapeutic agents may be may be chemical therapeutic agents, protein or polypeptide therapeutic agents, and include therapeutic agents that possess a desired biological activity and/or modify a given biological response. Examples of therapeutic agents include alkylating agents, angiogenesis inhibitors, anti-mitotic agents, hormone therapy agents, and antibodies useful for the treatment of cell proliferative disorders. In certain embodiments, the therapeutic agents are maytansinoid compounds, such as those described in US Patent Nos. 5,208,020 and 7,276,497. In certain embodiments, the therapeutic agents are benzodiazepine compounds, such as pyrrolobenzodiazepine (PBD) (such as those described in WO2010/043880, WO2011/130616, WO2009/016516, WO 2013/177481 and WO 2012/112708) and indolinobenzodiazepine (IGN) compounds (such as those described in WO/2010/091150, and WO 2012/128868 and U.S. Application No. 15/195,269, filed on June 28, 2016, entitled "CONJUGATES OF CYSTEINE ENGINEERED ANTIBODIES ").

As used herein, a "**pyrrolobenzodiazepine**" (PBD) compound is a compound having a pyrrolobenzodiazepine core structure. The pyrrolobenzodiazepine can be substituted or unsubstituted. It also includes a compound having two pyrrolobenzodiazepine core linked by a linker. The imine functionality (-C=N-) as part of indolinobenzodiazepine core can be reduced.

In certain embodiments, the pyrrolobenzodiazepine compound comprises a core structure represented by

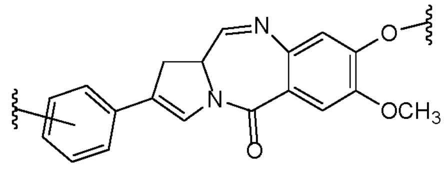

,

which can be optionally substituted.

In certain embodiments, the pyrrolobenzodiazepine compounds comprises a core structure represented by

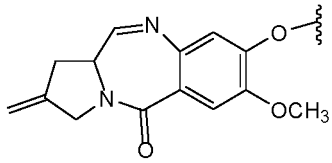

,

which can be optionally substituted.

As used herein, a "**indolinobenzodiazepine**" (IGN) compound is a compound having an indolinobenzodiazepine core structure. The indolinobenzodiazepine can be substituted or unsubstituted. It also includes a compound having two indolinobenzodiazepine core linked by a linker. The imine functionality (-C=N-) as part of indolinobenzodiazepine core can be reduced.

In certain embodiments, the indolinobenzodiazepine compound comprises a core structure represented by

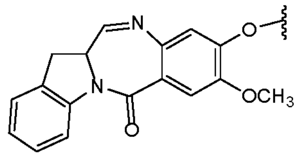

,

which can be optionally substituted.

In some embodiments, the indolinobenzodiazepine compound comprises a core structure represented by

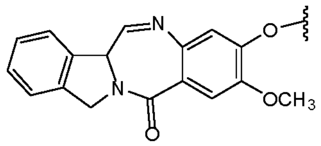

,

which can be further substituted.

The pharmacological agent may be coupled or conjugated either directly to the anti-ADAM9 antibody or ADAM9-binding fragment thereof or indirectly, through a linker using techniques known in the art to produce an "immunoconjugate," "conjugate," or "ADC."

In a first embodiment, the immunoconjugate of the present invention comprises an anti-ADAM9 antibody or an ADAM9-binding fragment thereof described herein covalently linked to a pharmacological agent described herein through the ε-amino group of one or more lysine residues located on the anti-ADAM9 antibody or an ADAM9-binding fragment thereof.

In a 1st specific embodiment of the first embodiment, the immunoconjugate of the present invention is represented by the following formula:

$$\mathrm{CBA}\left(-\mathrm{Cy}^{L1}\right)_{W_L} \quad \text{(L1)},$$

wherein:

CBA is an anti-ADAM9 antibody or an ADAM9-binding fragment thereof described herein above, that is covalently linked to $Cy^{L1}$ through a lysine residue;

$W_L$ is an integer from 1 to 20; and

$Cy^{L1}$ is a cytotoxic compound represented by the following formula:

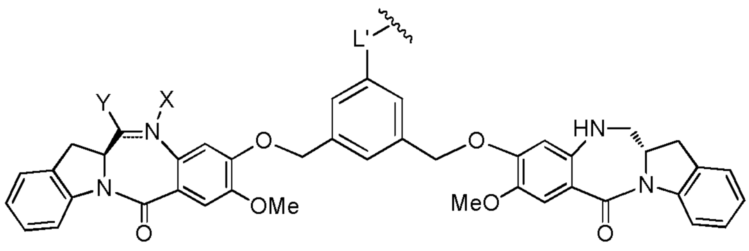
(L1a),

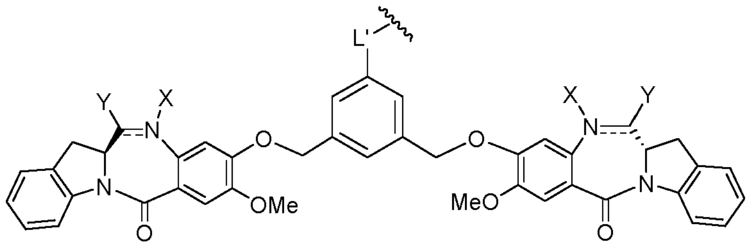
(L1a1),

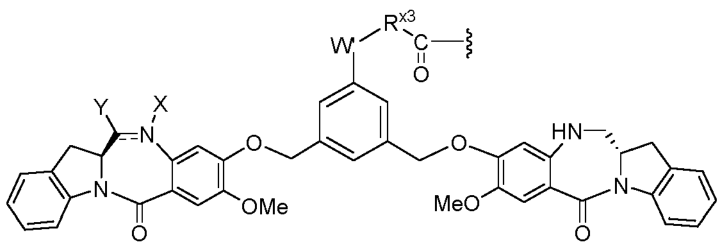
(L1b),

or

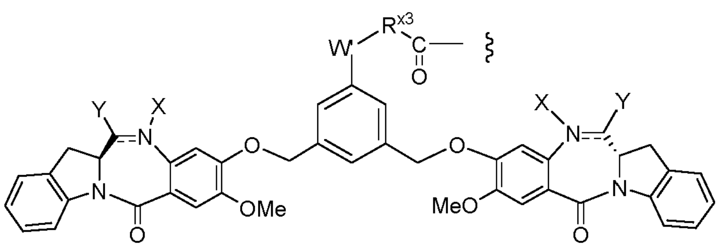
(L1b1);

or a pharmaceutically acceptable salt thereof, wherein:

the double line



between N and C represents a single bond or a double bond, provided that when it is a double bond, X is absent and Y is -H or a ($C_1$-$C_4$)alkyl; and when it is a single bond, X is -H or an amine protecting moiety, and Y is -OH or -$SO_3H$ or a pharmaceutically acceptable salt thereof;
W' is -$NR^{e'}$,
$R^{e'}$ is -$(CH_2$-$CH_2$-$O)_n$-$R^k$;
n is an integer from 2 to 6;
$R^k$ is -H or -Me;
$R^{x3}$ is a ($C_1$-$C_6$)alkyl;
L' is represented by the following formula:

- $NR_5$-P-C(=O)-$(CR_aR_b)_m$-C(=O)- (B1'); or
- $NR_5$-P-C(=O)-$(CR_aR_b)_m$-S-$Z^{s1}$- (B2');

$R_5$ is -H or a $(C_1-C_3)$alkyl;
P is an amino acid residue or a peptide containing between 2 to 20 amino acid residues;
$R_a$ and $R_b$, for each occurrence, are each independently -H, $(C_1-C_3)$alkyl, or a charged substituent or an ionizable group Q;
m is an integer from 1 to 6; and
$Z^{s1}$ is selected from any one of the following formulas:

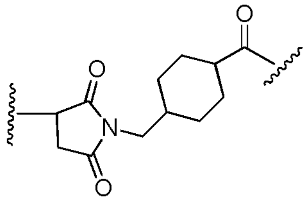 (b1); 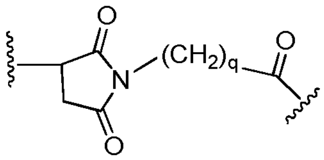 (b2); 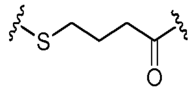 (b3);

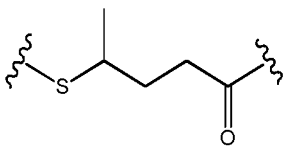 (b4); 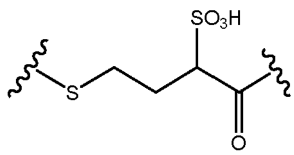 (b5),

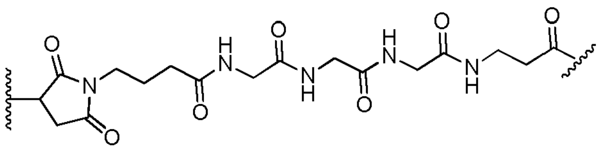 (b6),

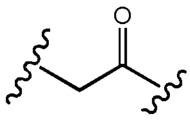 (b7); 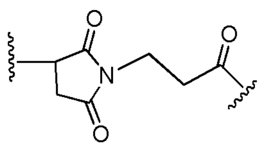 (b8); 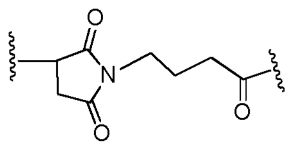 (b9);

and

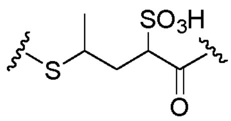 (b10),

wherein q is an integer from 1 to 5.

In a 2nd specific embodiment, for conjugates of formula (L1), $Cy^{L1}$ is represented by formula (L1a) or (L1a1); and the remaining variables are as described above in the 1st specific embodiment.

In a 3rd specific embodiment, for conjugates of formula (L1), $Cy^{L1}$ is represented by formula (L1b) or (L1b1); and the remaining variables are as described above in the 1st specific embodiment. More specifically, $R^{x3}$ is a $(C_2-C_4)$alkyl.

In a 4th specific embodiment, for conjugates of formula (L1), $Cy^{L1}$ is represented by formula (L1a); $R_a$ and $R_b$ are both H; $R_5$ is H or Me, and the remaining variables are as described above in the 1st specific embodiment.

In a 5th specific embodiment, P is a peptide containing 2 to 5 amino acid residues; and the remaining variables are described above in the 1st, 2nd or 4th specific embodiment. In a more specific embodiment, P is selected from the group consisting of Gly-Gly-Gly, Ala-Val, Val-Ala, Val-Cit, Val-Lys, Phe-Lys, Lys-Lys, Ala-Lys, Phe-Cit, Leu-Cit, Ile-Cit, Trp, Cit, Phe-Ala, Phe-$N^9$-tosyl-Arg, Phe-$N^9$-nitro-Arg, Phe-Phe-Lys, D-Phe-Phe-Lys, Gly-Phe-Lys, Leu-Ala-Leu, Ile-Ala-Leu, Val-Ala-Val, Ala-Leu-Ala-Leu (SEQ ID NO:144, β-Ala-Leu-Ala-Leu (SEQ ID NO:145), Gly-Phe-Leu-Gly (SEQ ID NO:146), Val-Arg, Arg-Val, Arg-Arg, Val-D-Cit, Val-D-Lys, Val-D-Arg, D-Val-Cit, D-Val-Lys, D-Val-Arg, D-Val-D-Cit, D-Val-D-Lys, D-Val-D-Arg, D-Arg-D-Arg, Ala-Ala, Ala-D-Ala, D-Ala-Ala, D-Ala-D-Ala, Ala-Met, Met-Ala, Gln-Val, Asn-Ala, Gln-Phe and Gln-Ala. More specifically, P is Gly-Gly-Gly, Ala-Val, Ala-Ala, Ala-D-Ala, D-Ala-Ala, or D-Ala-D-Ala.

In a 6th specific embodiment, Q is $-SO_3H$ or a pharmaceutically acceptable salt thereof; and the remaining variables are as described above in the 1st, 2nd, 4th or 5th specific embodiment or any more specific embodiments described therein.

In a 7th specific embodiment, the immunoconjugate of the first embodiment is represented by the following formula:

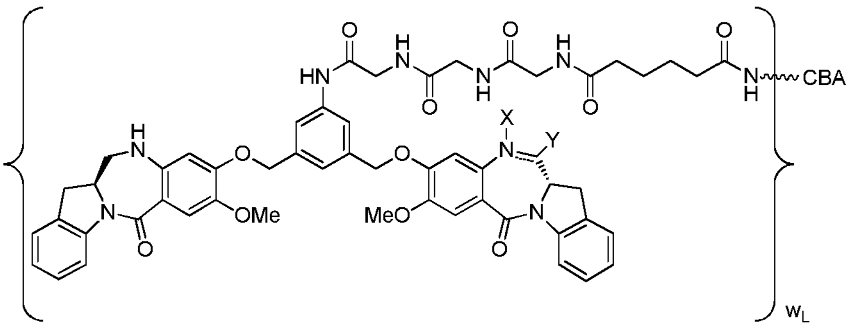
;

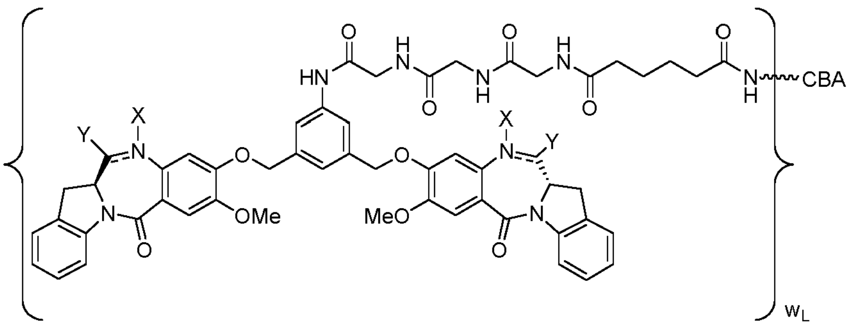
;

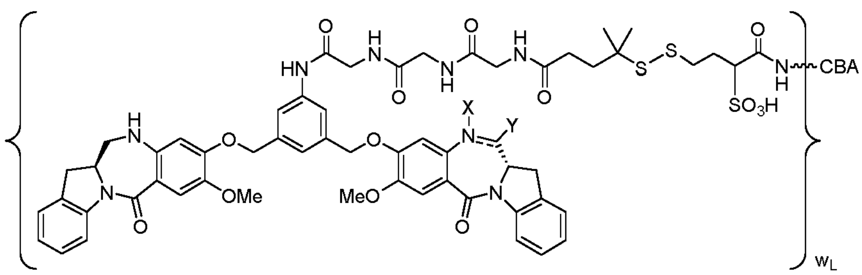
;

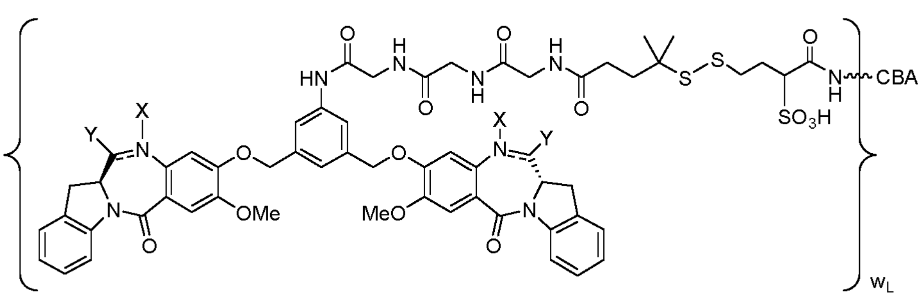
;

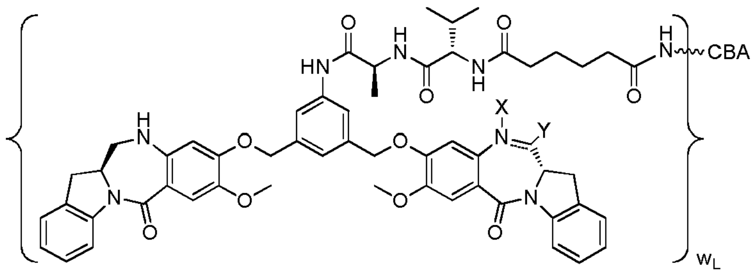

;

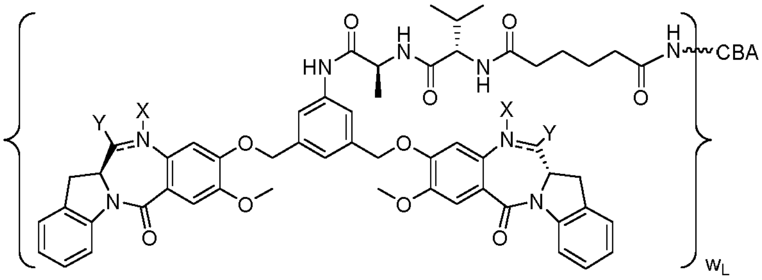

;

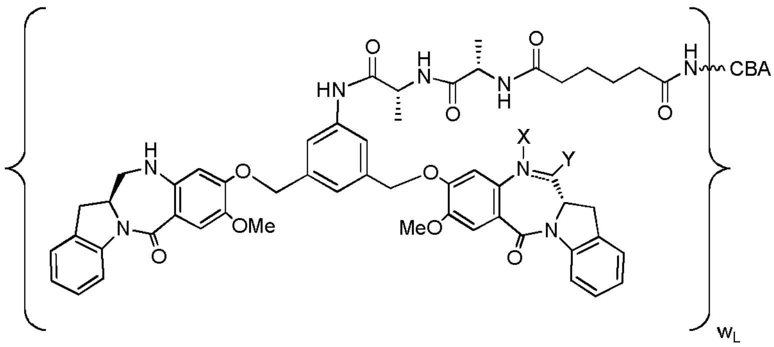

;

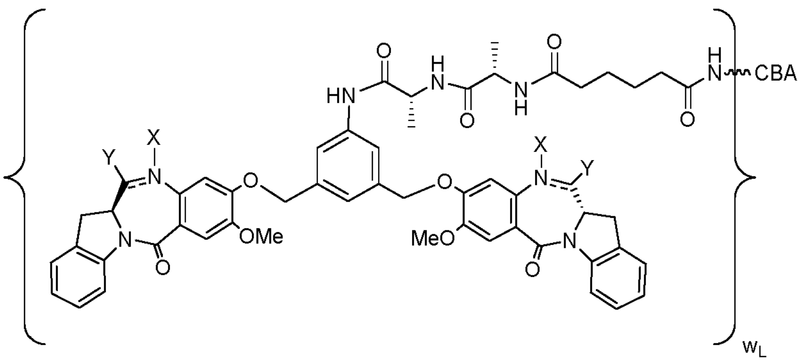

;

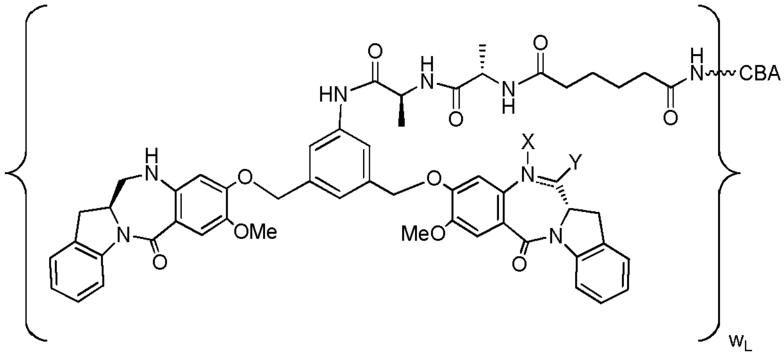

;

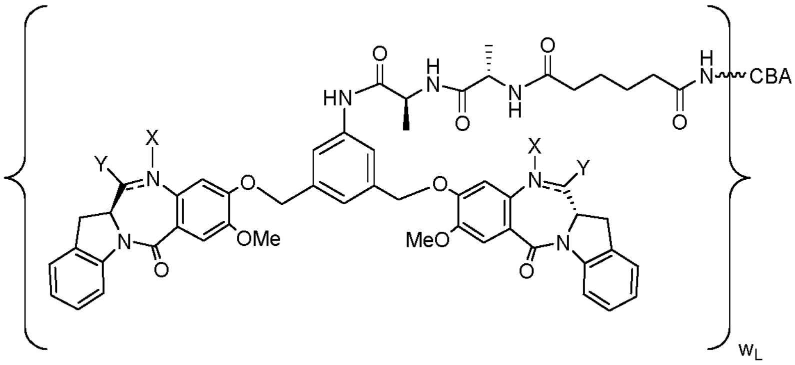

;

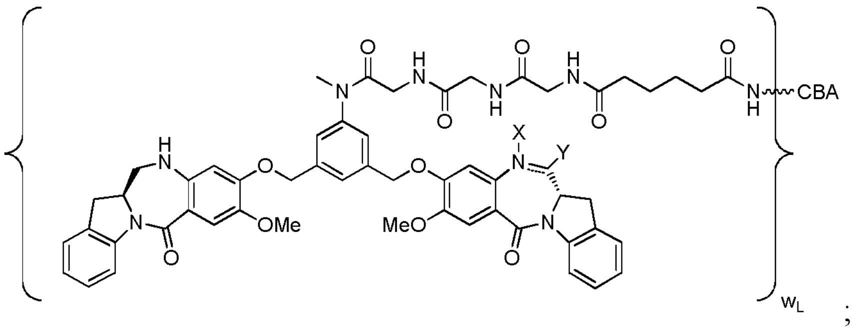

;

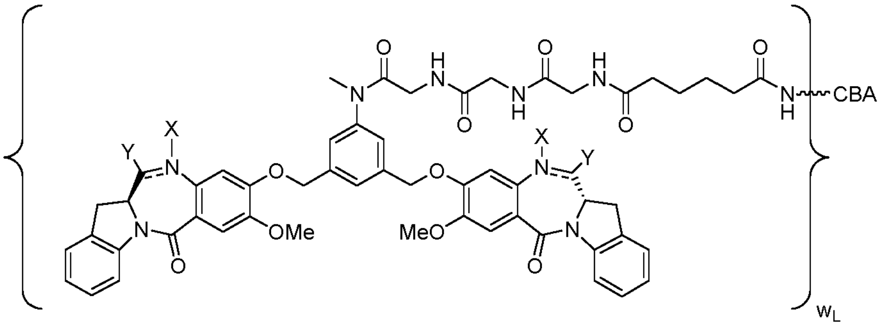

;

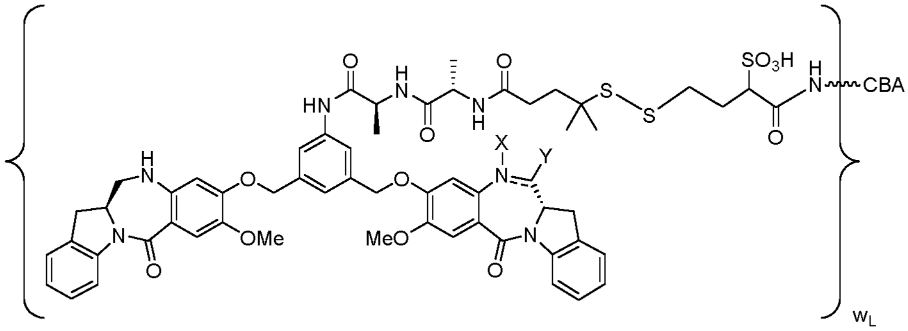

;

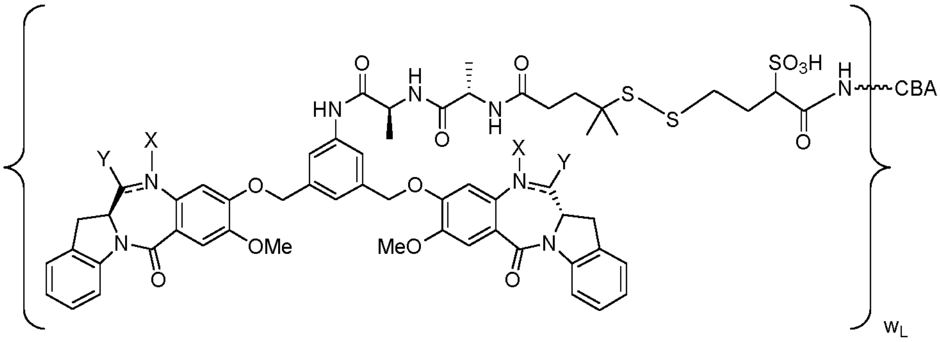

;

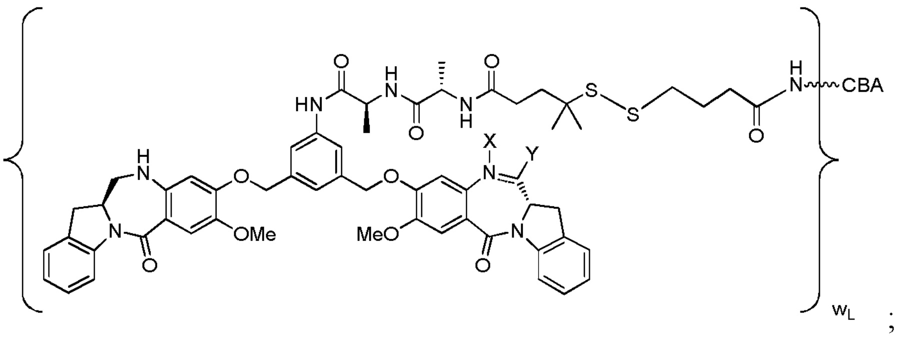

;

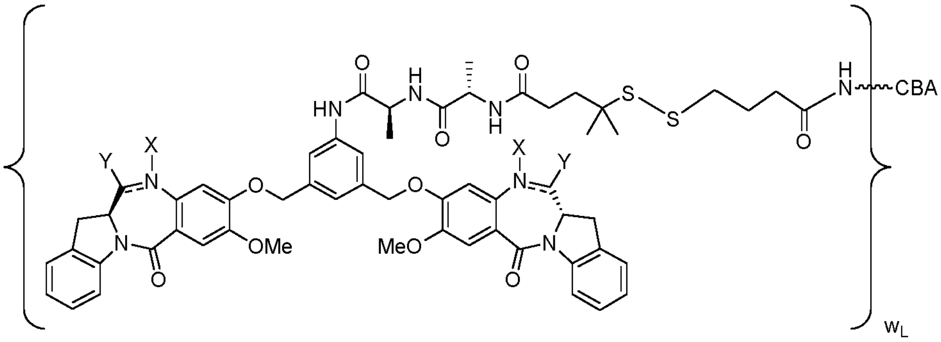

;

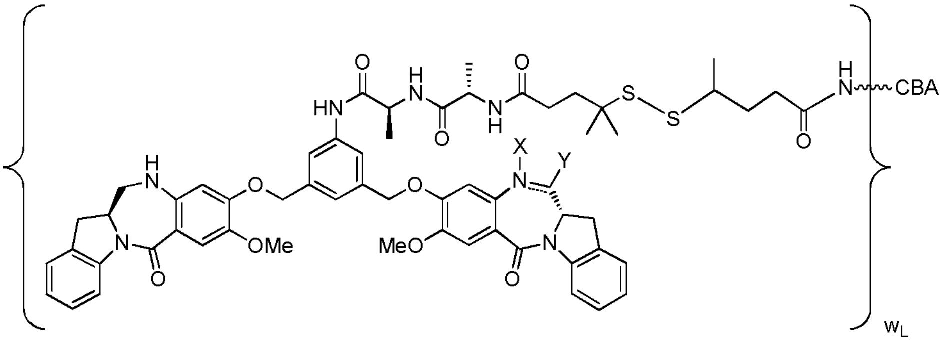

;

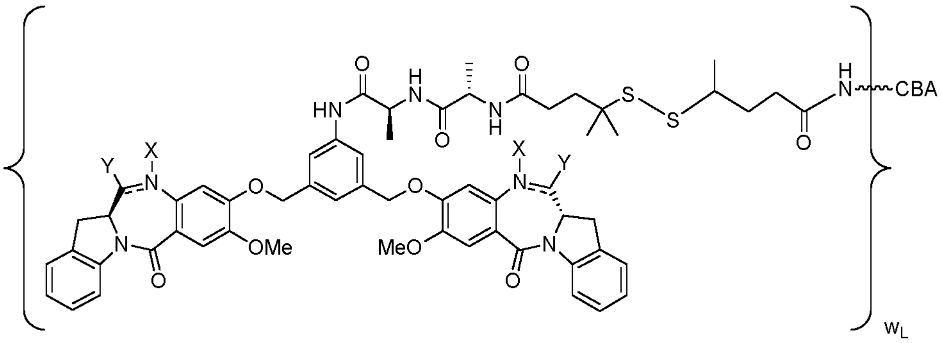

;

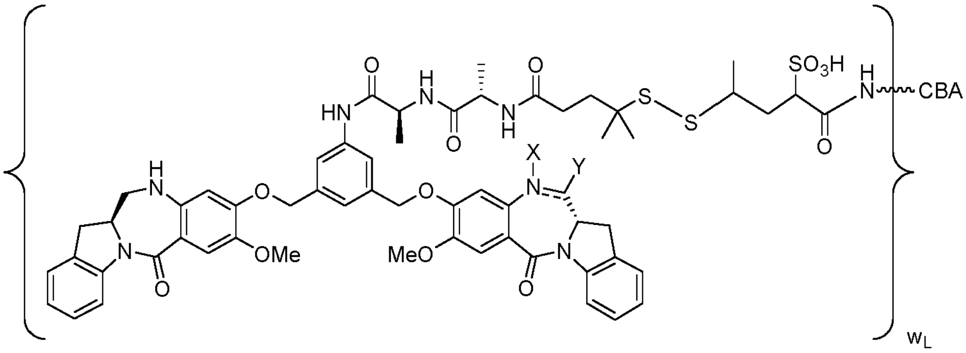

;

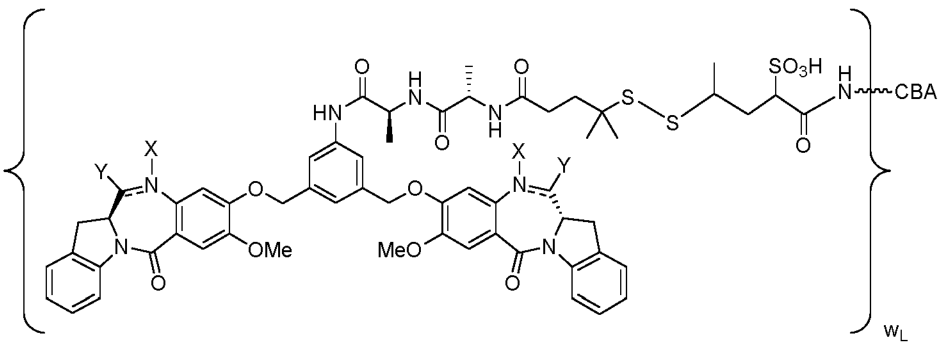

;

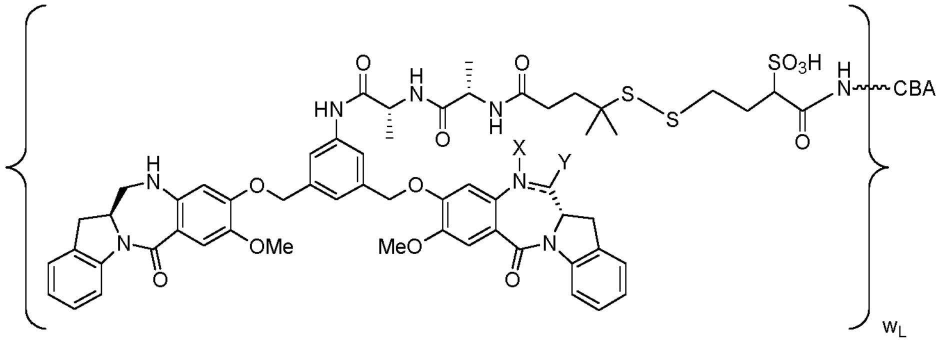

;

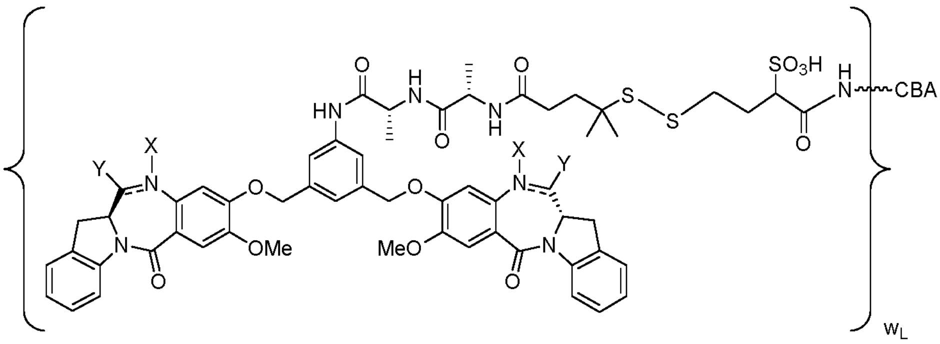

;

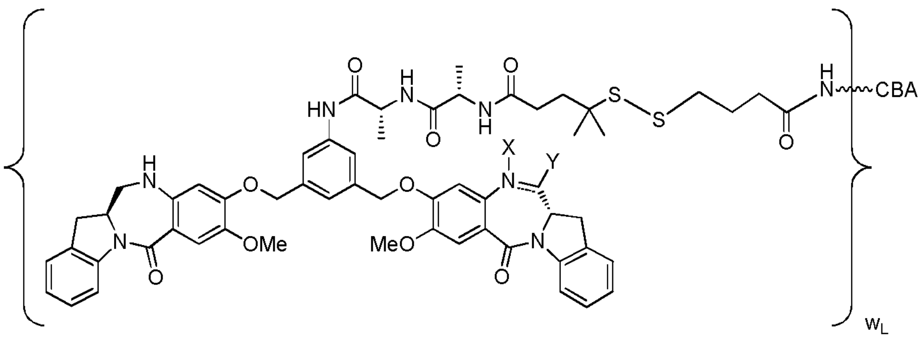

;

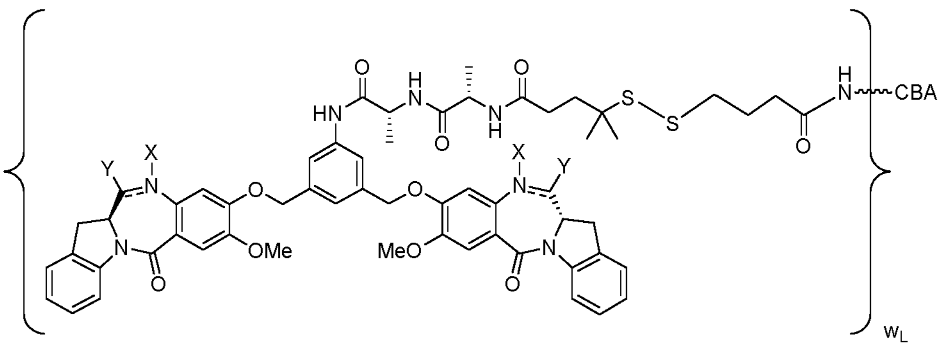

;

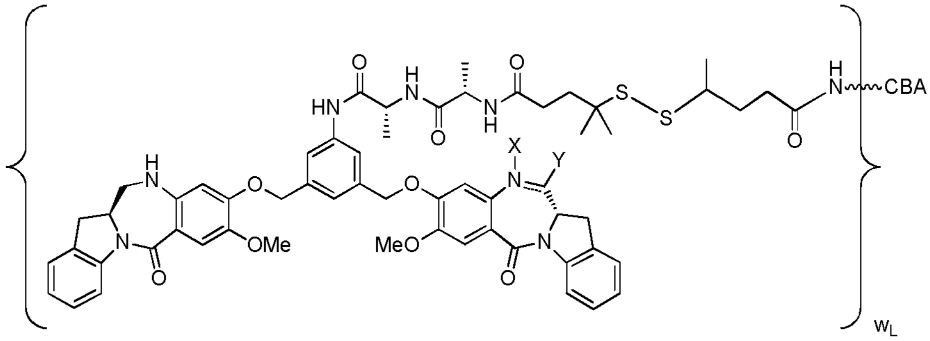

;

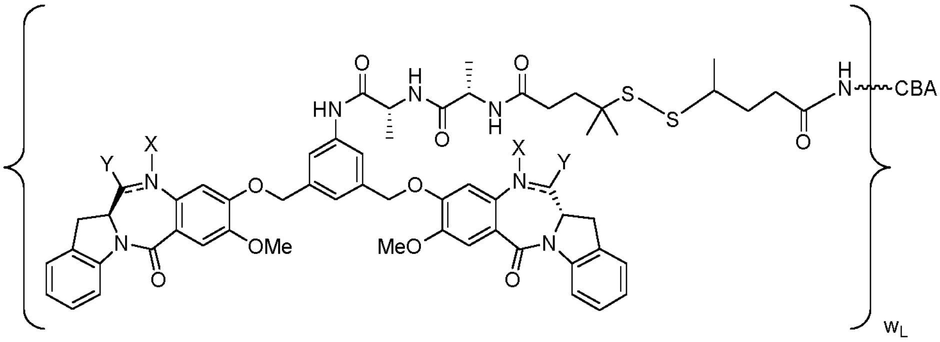

;

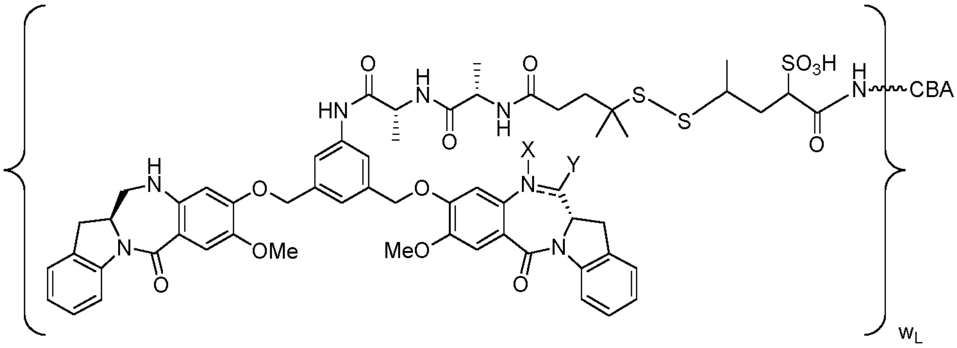

;

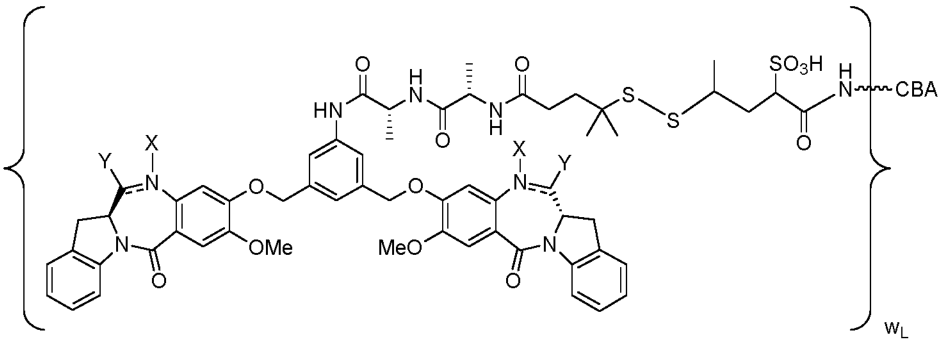

;

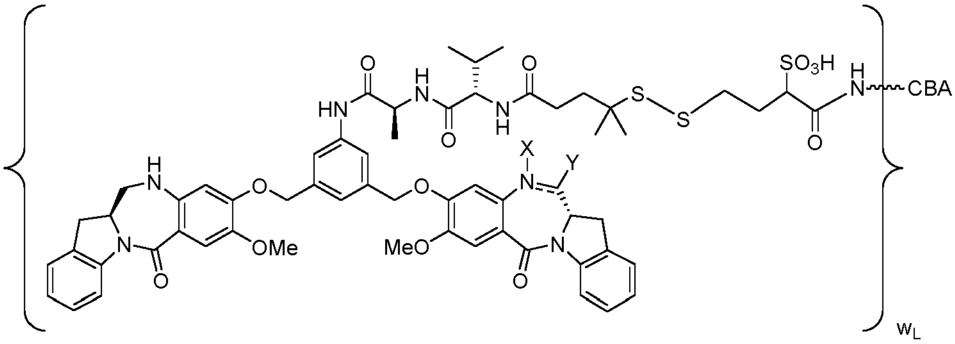
;

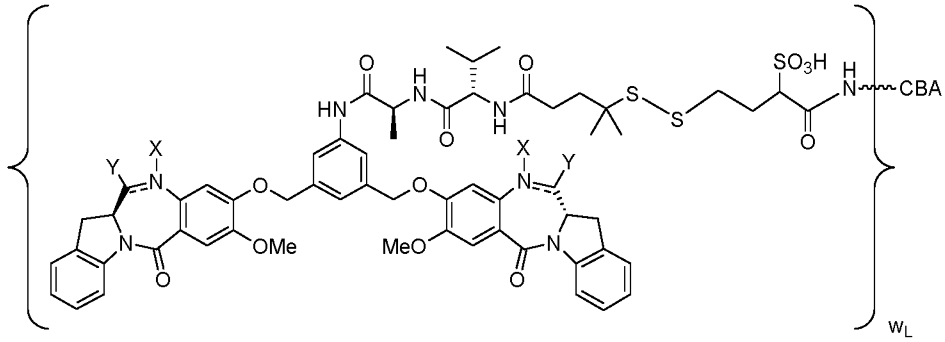
;

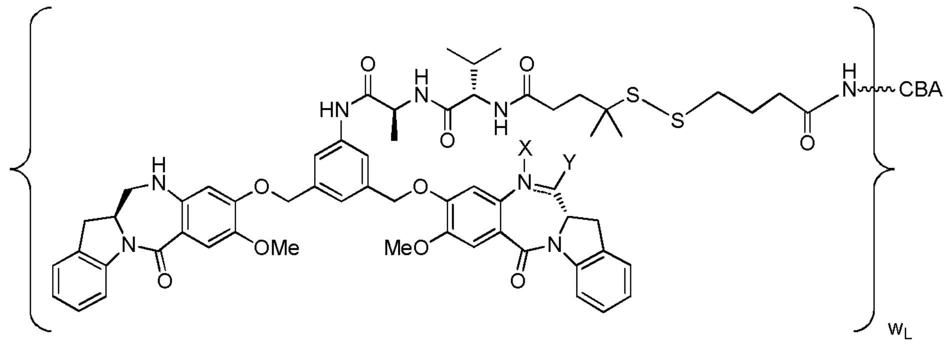
;

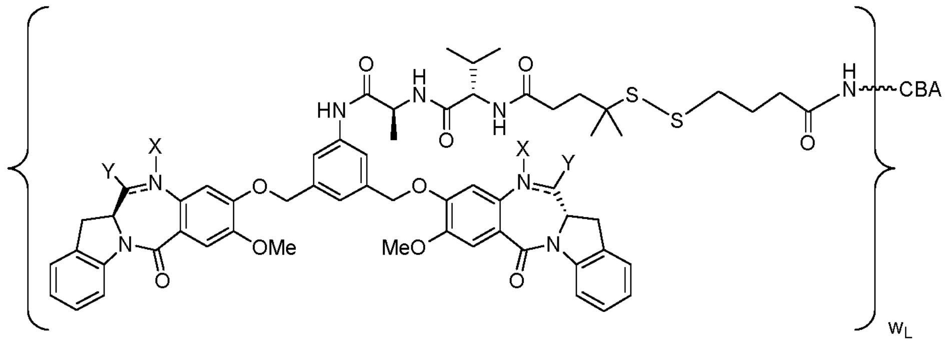
;

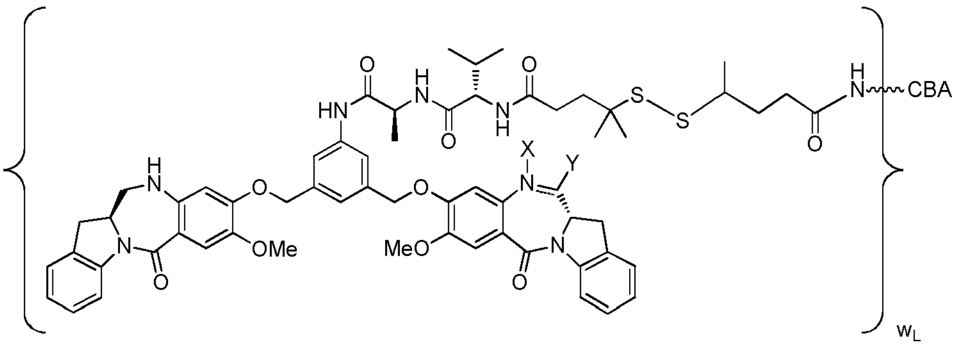

;

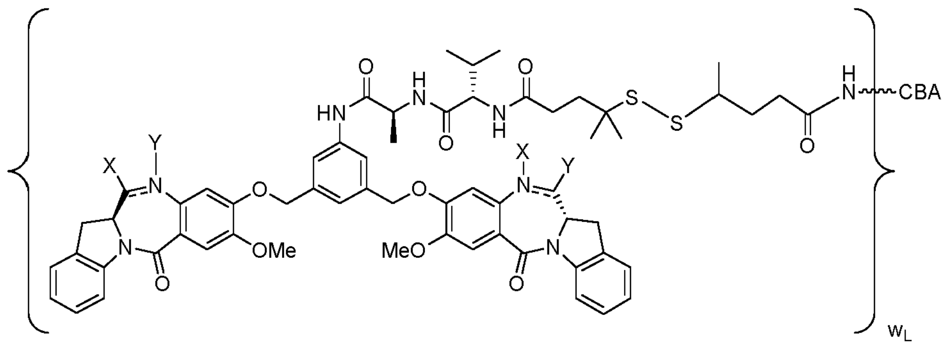

;

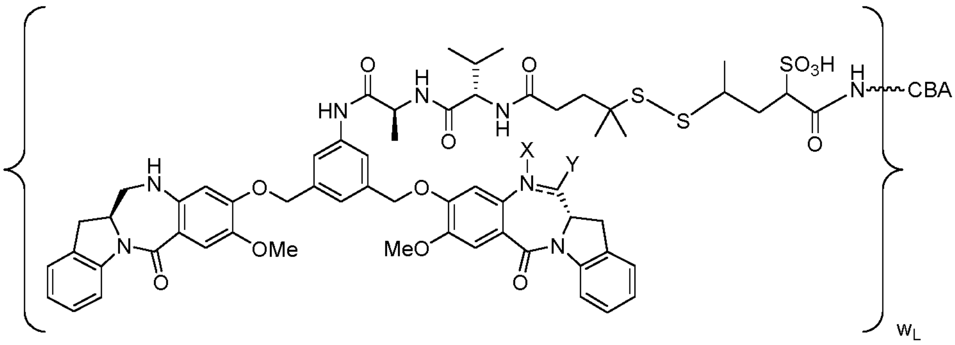

;

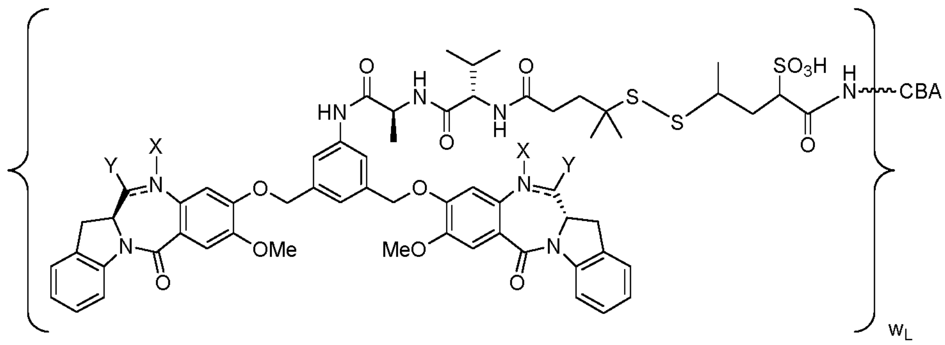

;

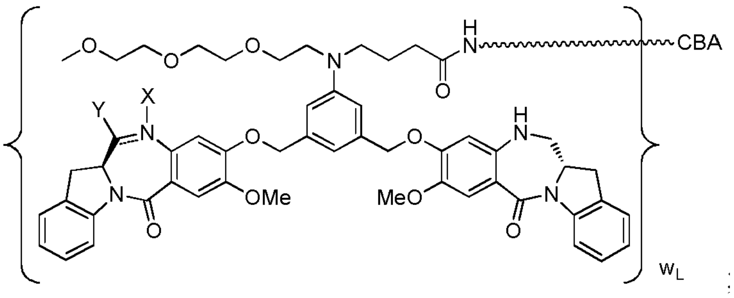

or

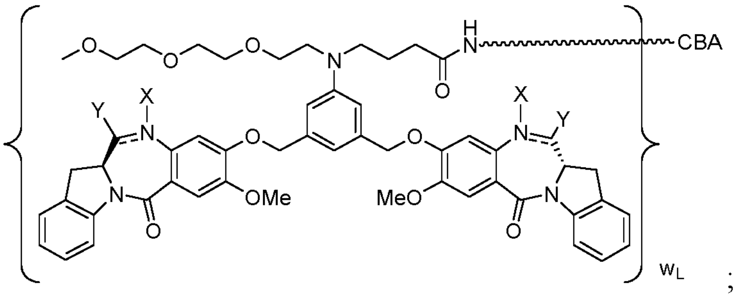

or a pharmaceutically acceptable salt thereof, wherein $W_L$ is an integer from 1 to 10; the double line



between N and C represents a single bond or a double bond, provided that when it is a double bond, X is absent and Y is -H; and when it is a single bond, X is -H, and Y is -OH or $-SO_3H$ or a pharmaceutically acceptable salt thereof. In a more specific embodiment, the double line



between N and C represents a double bond, X is absent and Y is -H. In another more specific embodiment, the double line



between N and C represents a single bond, X is -H and Y is $-SO_3H$ or a pharmaceutically acceptable salt thereof.

In a 8th specific embodiment, the immunoconjugate of the first embodiment is represented by the following formula:

$$CBA \left( -Cy^{L2} \right)_{W_L} \quad (L2),$$

wherein:

CBA is an anti-ADAM9 antibody or an ADAM9-binding fragment thereof described herein above, that is covalently linked to $Cy^{L2}$ through a lysine residue;

$W_L$ is an integer from 1 to 20; and

$Cy^{L2}$ is a cytotoxic compound represented by the following formula:

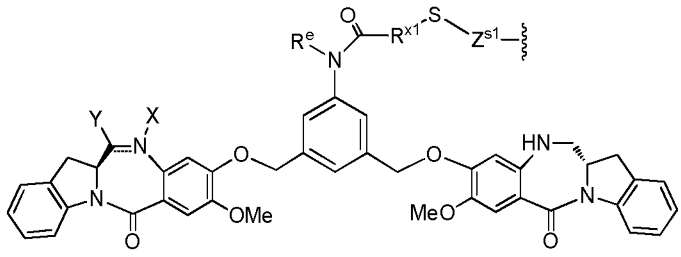
(L2a);

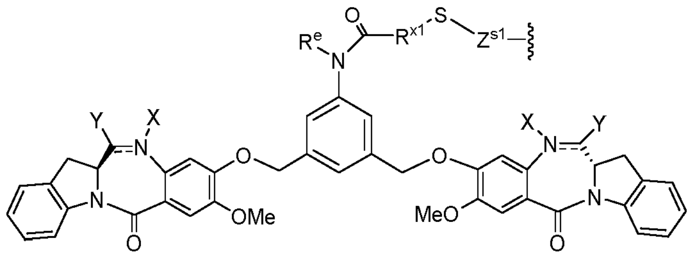
(L2a1);

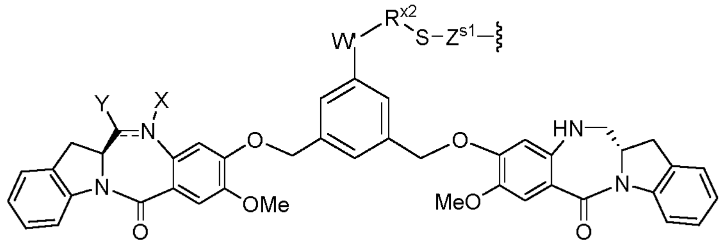
(L2b); or

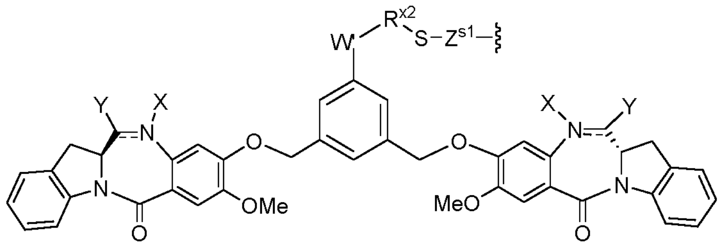
(L2b1)

or a pharmaceutically acceptable salt thereof, wherein:

the double line



between N and C represents a single bond or a double bond, provided that when it is a double bond, X is absent and Y is -H or a ($C_1$-$C_4$)alkyl; and when it is a single bond, X is -H or an amine protecting moiety, and Y is -OH or -$SO_3H$ or a pharmaceutically acceptable salt thereof;

$R^{x1}$ and $R^{x2}$ are independently ($C_1$-$C_6$)alkyl;

$R^e$ is -H or a ($C_1$-$C_6$)alkyl;

W' is -$NR^{e'}$,

$R^{e'}$ is -$(CH_2$-$CH_2$-$O)_n$-$R^k$;

n is an integer from 2 to 6;

$R^k$ is -H or -Me;

$Z^{s1}$ is selected from any one of the following formulas:

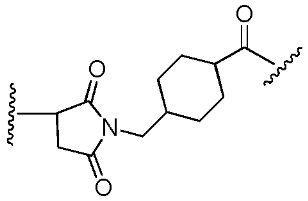 (b1); 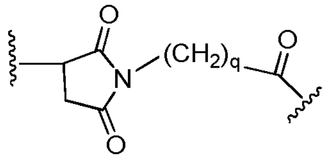 (b2); 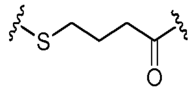 (b3);

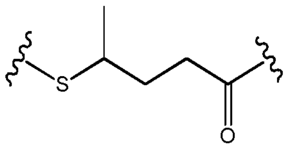 (b4); 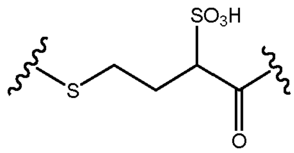 (b5),

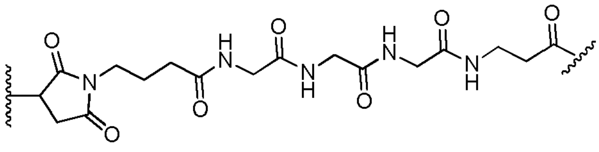 (b6),

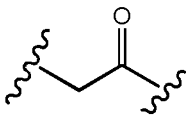 (b7); 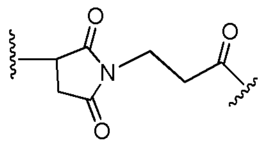 (b8); 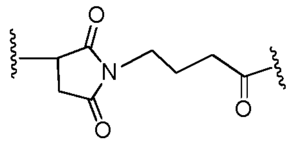 (b9);

and

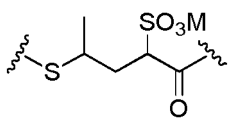 (b10),

wherein q is an integer from 1 to 5.

In a 9th specific embodiment, for immunoconjugates of formula (L2), $Cy^{L2}$ is represented by formula (L2a) or (L2a1); and the remaining variables are as described above in the 8th specific embodiment.

In a 10th specific embodiment, for immunoconjugates of formula (L2), $Cy^{L2}$ is represented by formula (L2b) or (L2b1); and the remaining variables are as described above in the 8th specific embodiment.

In a 11th specific embodiment, for immunoconjugates of formula (L2), $R^e$ is H or Me; $R^{x1}$ and $R^{x2}$ are independently $-(CH_2)_p-(CR^fR^g)-$, wherein $R^f$ and $R^g$ are each independently -H or a $(C_1-C_4)$alkyl; and p is 0, 1, 2 or 3; and the remaining variables are as described above in the 8th, 9th or 10th specific embodiment. More specifically, $R^f$ and $R^g$ are the same or different, and are selected from -H and -Me.

In a 12th specific embodiment, the immunoconjugate of the first embodiment is represented by the following formula:

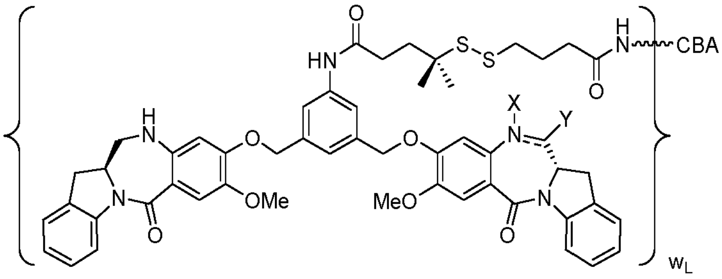

;

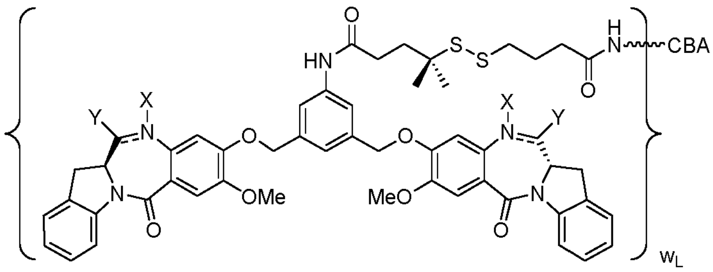

;

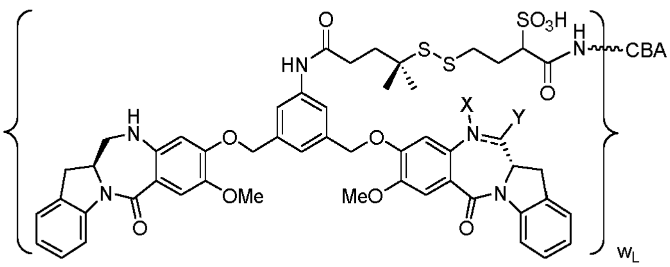

;

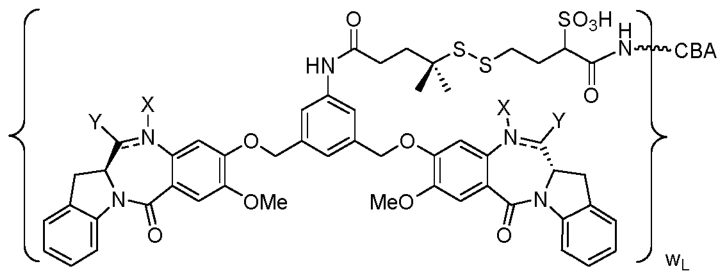

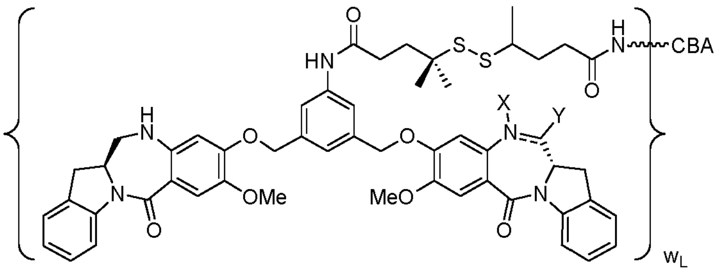

;

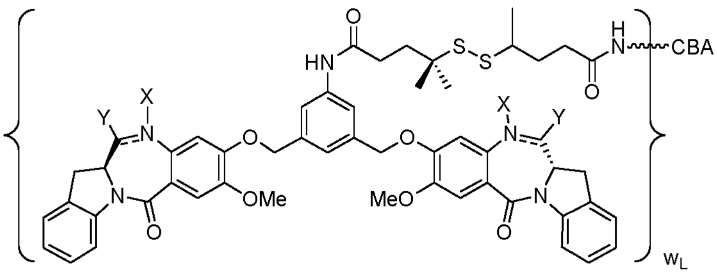

;

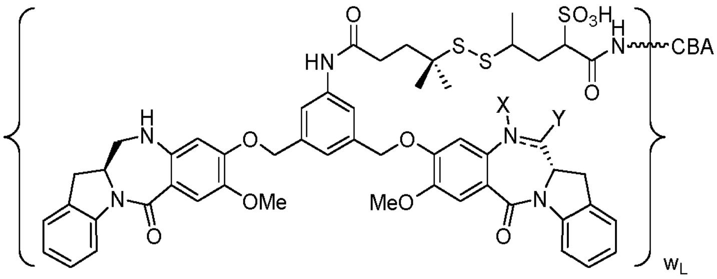

;

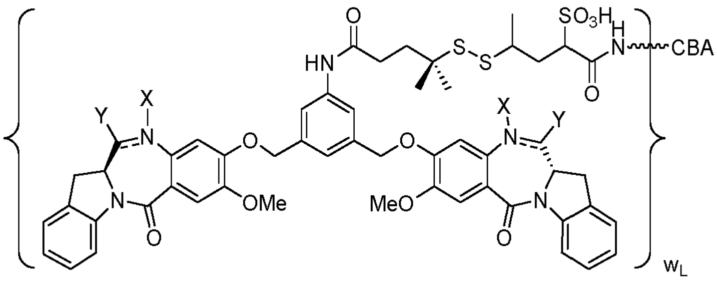

;

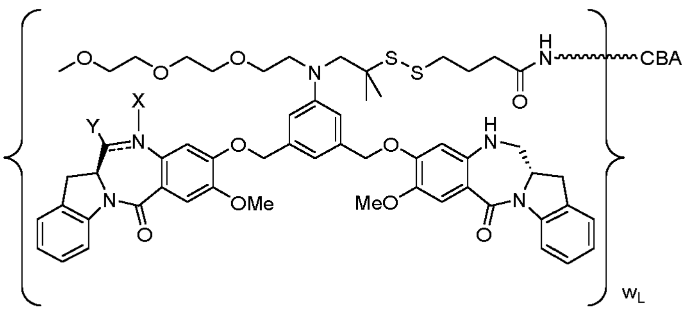

;

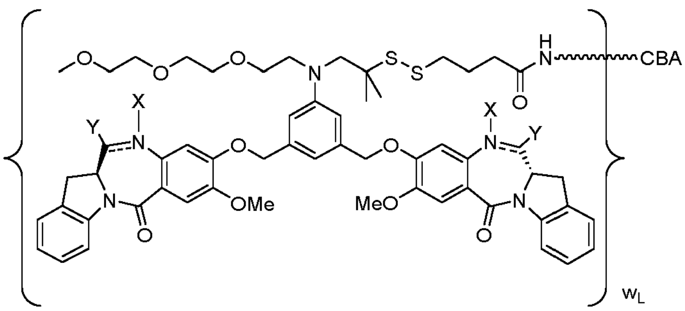

;

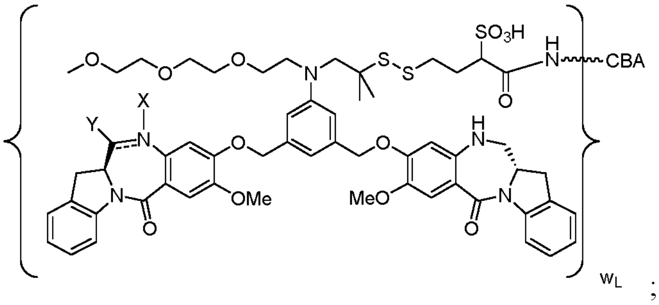

or

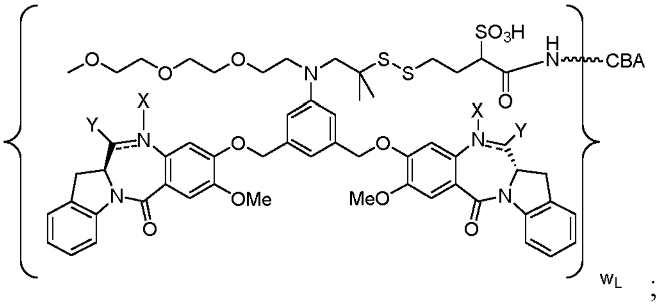

or a pharmaceutically acceptable salt thereof, wherein $W_L$ is an integer from 1 to 10; the double line

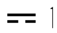

between N and C represents a single bond or a double bond, provided that when it is a double bond, X is absent and Y is -H; and when it is a single bond, X is -H and Y is -OH or $-SO_3H$ or a pharmaceutically acceptable salt thereof. In a more specific embodiment, the double line



between N and C represents a double bond. In another more specific embodiment, the double line



between N and C represents a single bond, X is -H and Y is $-SO_3H$ or a pharmaceutically acceptable salt thereof.

In a 13th specific embodiment, the immunoconjugates of the first embodiment is represented by the following formula:

$$CBA\left(-Cy^{L3}\right)_{W_L} \quad (L3),$$

wherein:

CBA is an anti-ADAM9 antibody or an ADAM9-binding fragment thereof described herein above, which is covalently linked to $Cy^{L3}$ through a Lys residue;

$W_L$ is an integer from 1 to 20;

$Cy^{L3}$ is represented by the following formula:

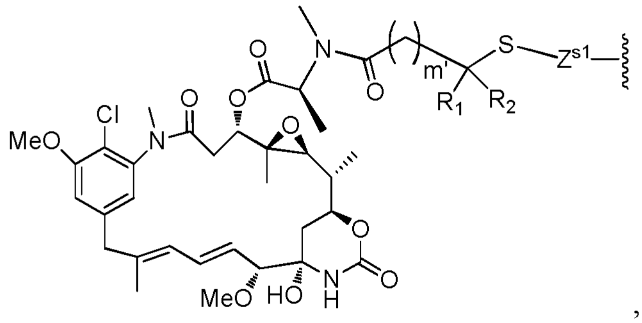

,

m' is 1 or 2;

$R_1$ and $R_2$, are each independently H or a $(C_1\text{-}C_3)$alkyl; and

$Z^{s1}$ is selected from any one of the following formulas:

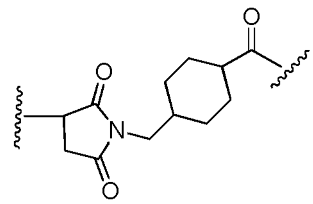

(b1);

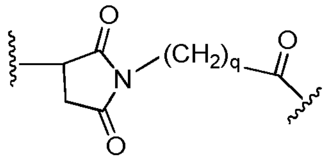

(b2);

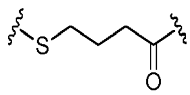

(b3);

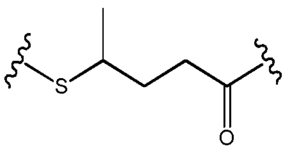

(b4);

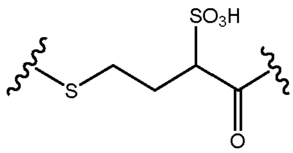

(b5),

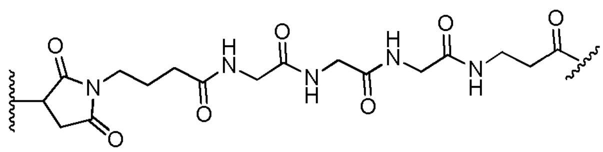

(b6),

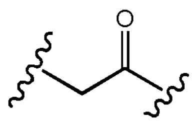

(b7);

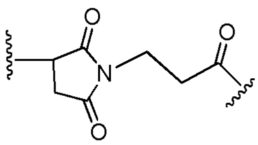

(b8);

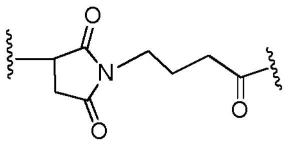

(b9),

and

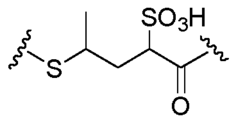

(b10),

wherein q is an integer from 1 to 5

In a 14th specific embodiment, for immunoconjugates of formula (L3), m' is 1, and $R_1$ and $R_2$ are both H; and the remaining variables are as described above in the 13th specific embodiment.

In a 15th specific embodiment, for immunoconjugates of formula (L3), m' is 2, and $R_1$ and $R_2$ are both Me; and the remaining variables are as described above in the 13th specific embodiment.

In a 16th specific embodiment, the immunoconjugates of the first embodiment is represented by the following

formula:

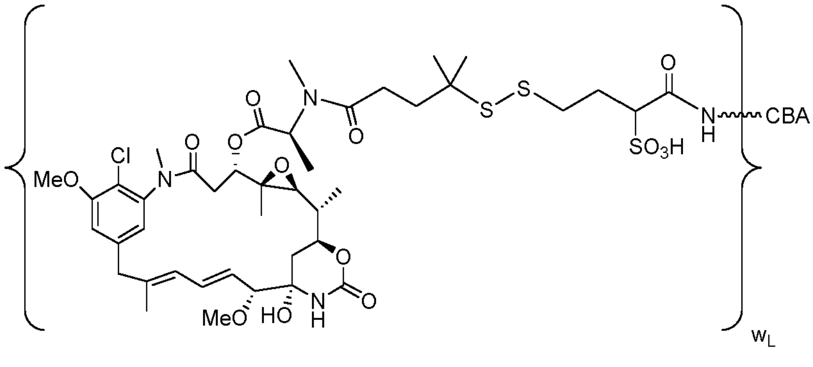

;

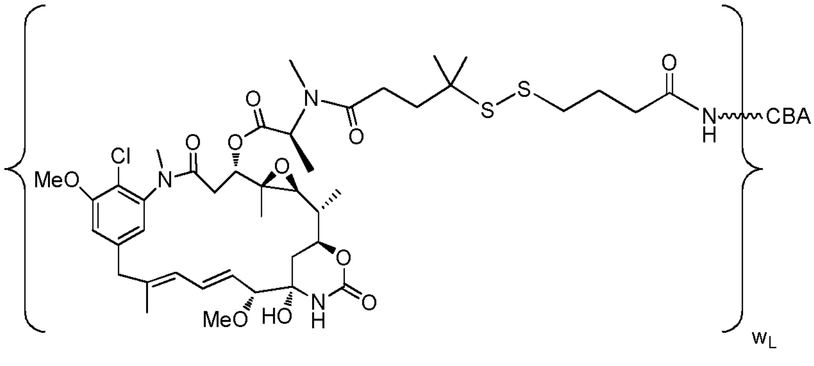

;

or

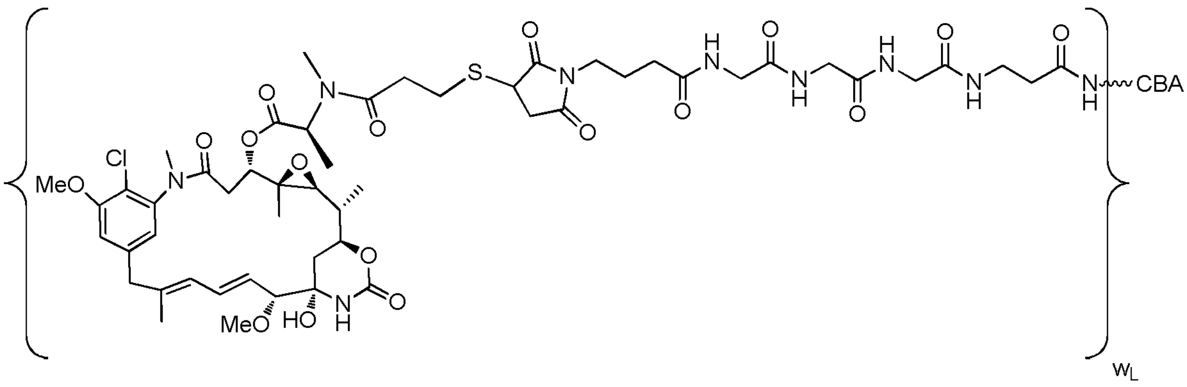

,

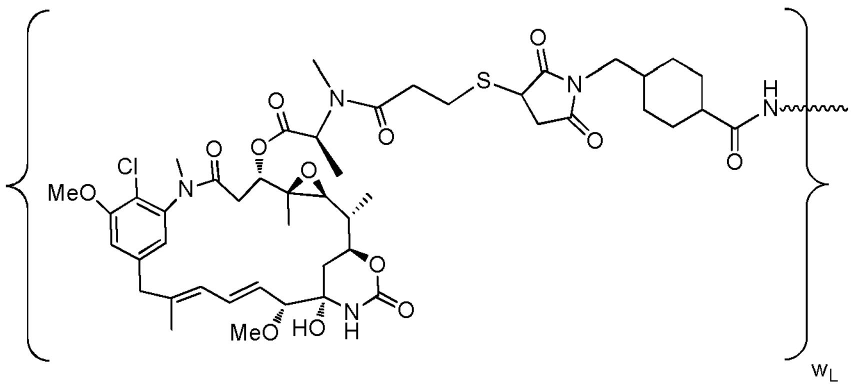

,

or a pharmaceutically acceptable salt thereof, wherein $W_L$ is an integer from 1 to 10.

In a 17th specific embodiment, for immunoconjugates of the first embodiment, Y is $-SO_3H$, $--SO_3Na$ or $-SO_3K$; and the remaining variables are as described above in any one of the 1st to 16th specific embodiment or any more specific embodiments described therein. In one embodiment, Y is $--SO_3Na$.

In certain embodiments, for compositions (*e.g.*, pharmaceutical compositions) comprising immunoconjugates of the first embodiment, or the 1st, 2nd, 3rd, 4th, 5th, 6th, 7th, 8th, 9th, 10th, 11th, 12th, 13th, 14th, 15th, 16th or 17th specific embodiment, the average number of the cytotoxic agent per antibody molecule (*i.e.,* average value of $w_L$), also known as Drug-Antibody Ratio (DAR) in the composition is in the range of 1.0 to 8.0. In some embodiments, DAR is in the range of 1.0 to 5.0, 1.0 to 4.0, 1.0 to 3.4, 1.0 to 3.0, 1.5 to 2.5, 2.0 to 2.5, or 1.8 to 2.2. In some embodiments, the DAR is less than 4.0, less than 3.8, less than 3.6, less than 3.5, less than 3.0 or less than 2.5.

In a second embodiment, the immunoconjugates of the present invention comprises an anti-ADAM9 antibody or an ADAM9-binding fragment thereof described herein above covalently linked to a cytotoxic agent described herein through the thiol group (-SH) of one or more cysteine residues located on the anti-ADAM9 antibody or an ADAM9-binding fragment thereof.

In a 1st specific embodiment, the immunoconjugate of the second embodiment is represented by the following formula:

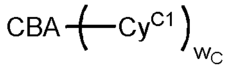

(C1),

wherein:

CBA is anti-ADAM9 antibody or an ADAM9-binding fragment thereof described herein covalently linked to $Cy^{C1}$ through a cysteine residue;

$W_C$ is 1 or 2;

$Cy^{C1}$ is represented by the following formula:

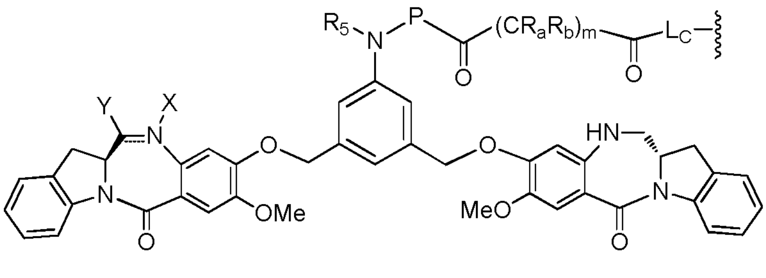

(C1a);

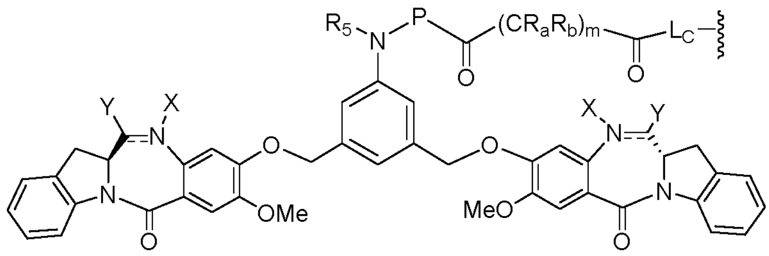

(C1a1);

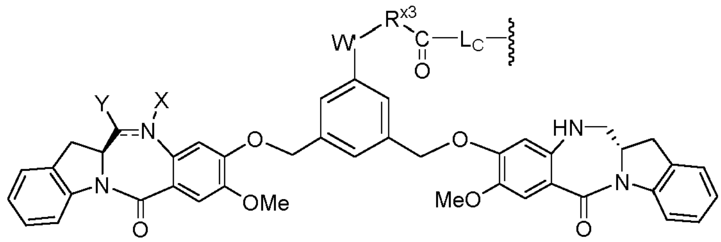

(C1b),

or

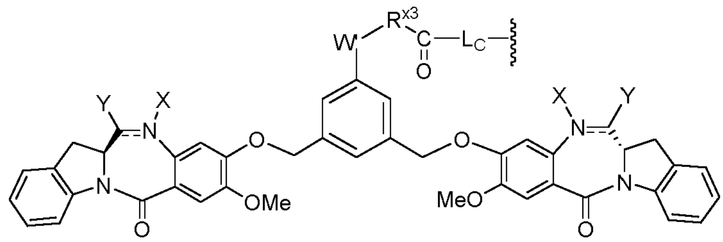

(C1b1)

or a pharmaceutically acceptable salt thereof, wherein:

the double line



between N and C represents a single bond or a double bond, provided that when it is a double bond, X is absent and Y is -H or a ($C_1$-$C_4$)alkyl; and when it is a single bond, X is -H or an amine protecting moiety, Y is -OH or $-SO_3H$ or a pharmaceutically acceptable salt thereof;

$R_5$ is -H or a ($C_1$-$C_3$)alkyl;

P is an amino acid residue or a peptide containing 2 to 20 amino acid residues;

$R_a$ and $R_b$, for each occurrence, are independently -H, ($C_1$-$C_3$)alkyl, or a charged substituent or an ionizable group Q;

W' is $-NR^{e'}$,

$R^{e'}$ is $-(CH_2-CH_2-O)_n-R^k$;

n is an integer from 2 to 6;

$R^k$ is -H or -Me;

$R^{x3}$ is a ($C_1$-$C_6$)alkyl; and,

$L_C$ is represented by:

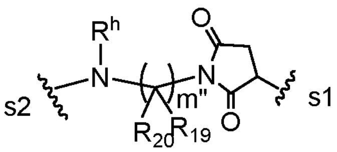

,

wherein s1 is the site covalently linked to CBA, and s2 is the site covalently linked to the -C(=O)- group on $Cy^{C1}$; wherein:

$R_{19}$ and $R_{20}$, for each occurrence, are independently -H or a $(C_1-C_3)$alkyl;
m" is an integer between 1 and 10; and
$R^h$ is -H or a $(C_1-C_3)$alkyl.

In a 2nd specific embodiment, for immunoconjugate of formula (C1), $Cy^{C1}$ is represented by formula (C1a) or (C1a1); and the remaining variables are as described above in the 1st specific embodiment of the second embodiment.

In a 3rd specific embodiment, for immunoconjugate of formula (C1), $Cy^{C1}$ is represented by formula (C1b) or (C1b1); and the remaining variables are as described above in the 1st specific embodiment of the second embodiment.

In a 4th specific embodiment, for immunoconjugate of formula (C1), $Cy^{C1}$ is represented by formula (C1a) or (C1a1); $R_a$ and $R_b$ are both H; and $R_5$ is H or Me; and the remaining variables are as described above in the 1st or 2nd specific embodiment of the second embodiment.

In a 5th specific embodiment, for immunoconjugate of formula (C1), P is a peptide containing 2 to 5 amino acid residues; and the remaining variables are as described above in the 1st, 2nd or 4th specific embodiment of the second embodiment. In a more specific embodiment, P is selected from Gly-Gly-Gly, Ala-Val, Val-Ala, Val-Cit, Val-Lys, Phe-Lys, Lys-Lys, Ala-Lys, Phe-Cit, Leu-Cit, Ile-Cit, Trp, Cit, Phe-Ala, Phe-$N^9$-tosyl-Arg, Phe-$N^9$-nitro-Arg, Phe-Phe-Lys, D-Phe-Phe-Lys, Gly-Phe-Lys, Leu-Ala-Leu, Ile-Ala-Leu, Val-Ala-Val, Ala-Leu-Ala-Leu (SEQ ID NO: 144), β-Ala-Leu-Ala-Leu (SEQ ID NO: 145), Gly-Phe-Leu-Gly (SEQ ID NO:146), Val-Arg, Arg-Val, Arg-Arg, Val-D-Cit, Val-D-Lys, Val-D-Arg, D-Val-Cit, D-Val-Lys, D-Val-Arg, D-Val-D-Cit, D-Val-D-Lys, D-Val-D-Arg, D-Arg-D-Arg, Ala-Ala, Ala-D-Ala, D-Ala-Ala, D-Ala-D-Ala, Ala-Met, Met-Ala, Gln-Val, Asn-Ala, Gln-Phe and Gln-Ala. In another more specific embodiment, P is Gly-Gly-Gly, Ala-Val, Ala-Ala, Ala-D-Ala, D-Ala-Ala, or D-Ala-D-Ala.

In a 6th specific embodiment, for immunoconjugates of formula (C1), Q is $-SO_3H$ or a pharmaceutically acceptable salt thereof; and the remaining variables are as describe above in the 1st, 2nd, 4th or 5th specific embodiment of the second embodiment or any more specific embodiments described therein.

In a 7th specific embodiment, for immunoconjugates of formula (C1), $R_{19}$ and $R_{20}$ are both H; and m" is an integer from 1 to 6; and the remaining variables are as described above in the 1st, 2nd, 3rd, 4th, 5th or 6th specific embodiment of the second embodiment or any more specific embodiments described therein.

In a 8th specific embodiment, for immunoconjugates of formula (C1), -L-Lc- is represented by the following formula:

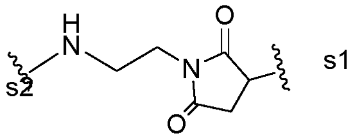

,

and the remaining variables are as described above in the 1st, 2nd, 3rd, 4th, 5th, 6th or 7th specific embodiment of the second embodiment or any more specific embodiments described therein.

In a 9th specific embodiment, the immunoconjugate of the second embodiment is represented by the following formula:

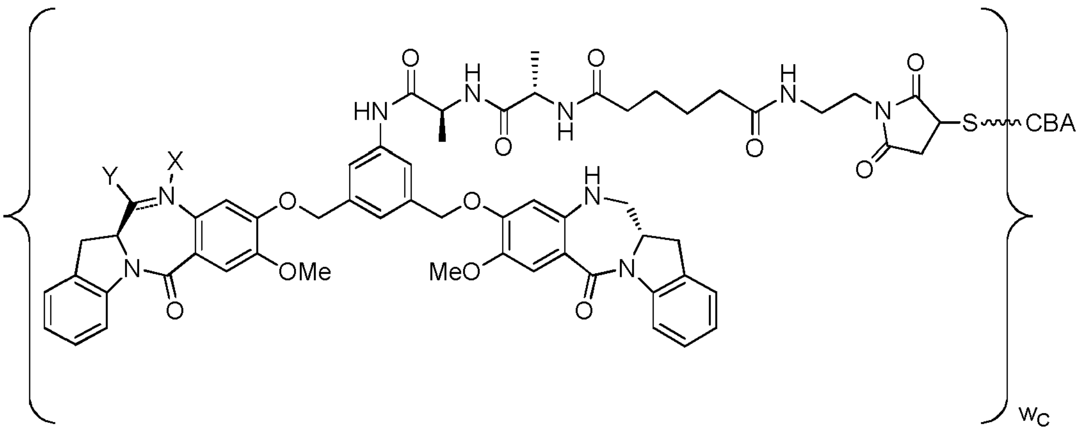

;

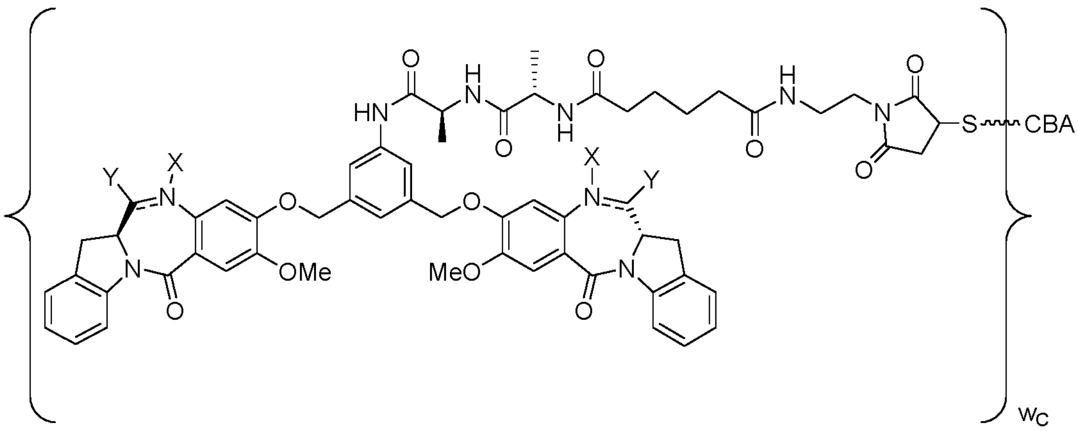

;

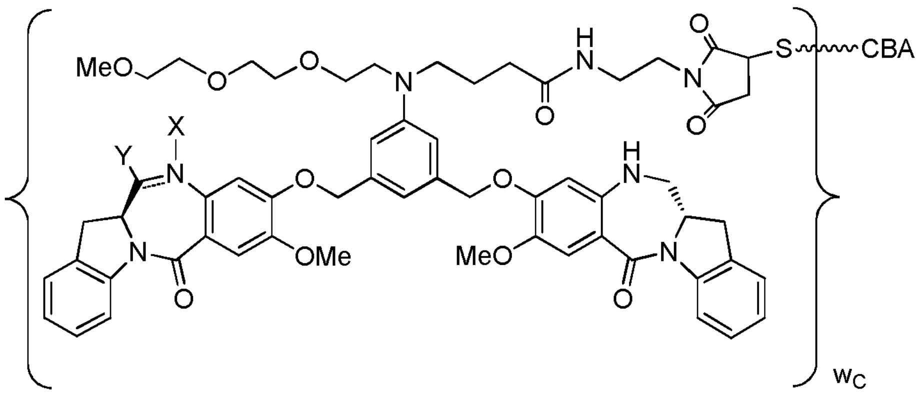

;

or

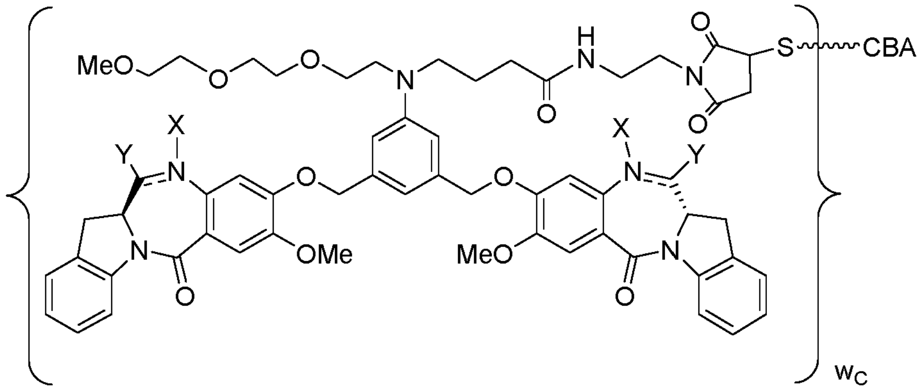

or a pharmaceutically acceptable salt thereof, wherein the double line



between N and C represents a single bond or a double bond, provided that when it is a double bond, X is absent and Y is -H, and when it is a single bond, X is -H, and Y is -OH or $-SO_3H$ or a pharmaceutically acceptable salt thereof. In a more specific embodiment, the double line



between N and C represents a double bond, X is absent and Y is -H. In another more specific embodiment, the double line



between N and C represents a single bond, X is -H and Y is $-SO_3H$ or a pharmaceutically acceptable salt thereof.

In a 10th specific embodiment, the immunoconjugate of the second embodiment is represented by the following formula:

$$CBA\left(-Cy^{C2}\right)_{W_C} \quad (C2),$$

wherein:

CBA is a an anti-ADAM9 antibody or an ADAM9-binding fragment thereof described herein above covalently linked to $Cy^{C2}$ through a cysteine residue;

$W_C$ is 1 or 2;

$Cy^{C2}$ is represented by the following formula:

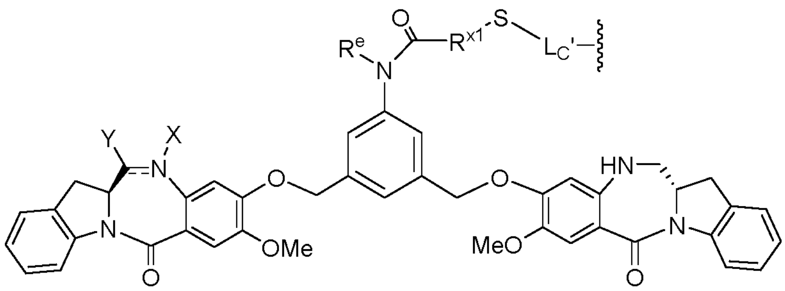

(C2a),

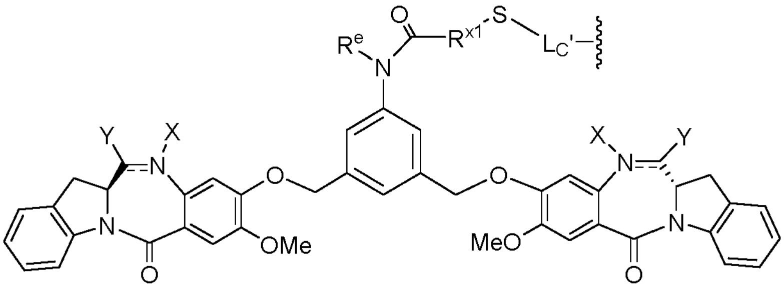

(C2a1),

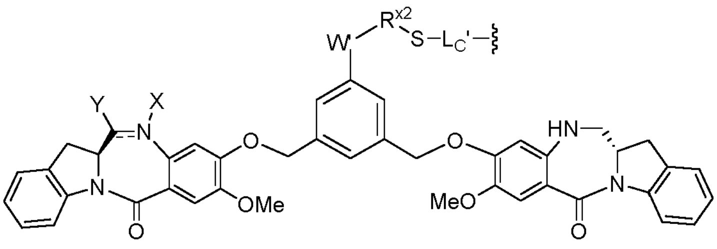

(C2b), or

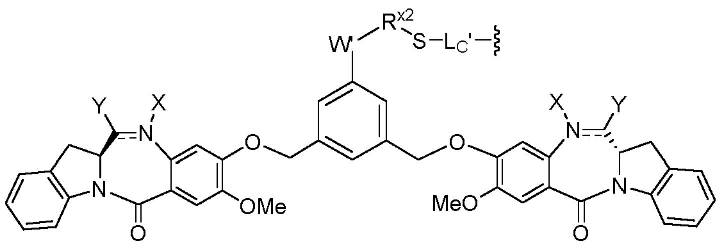

(C2b1),

or a pharmaceutically acceptable salt thereof, wherein:

the double line

==

between N and C represents a single bond or a double bond, provided that when it is a double bond, X is absent and Y is -H or a ($C_1$-$C_4$)alkyl; and when it is a single bond, X is -H or an amine protecting moiety, Y is -OH or -$SO_3H$ or a pharmaceutically acceptable salt thereof;
$R^{x1}$ is a ($C_1$-$C_6$)alkyl;
$R^e$ is -H or a ($C_1$-$C_6$)alkyl;
W' is -$NR^{e'}$;
$R^{e'}$ is -$(CH_2$-$CH_2$-$O)_n$-$R^k$;
n is an integer from 2 to 6;
$R^k$ is -H or -Me;
$R^{x2}$ is a ($C_1$-$C_6$)alkyl;
$L_C$' is represented by the following formula:

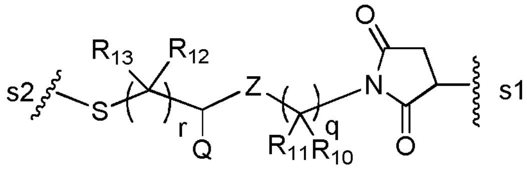

;

or

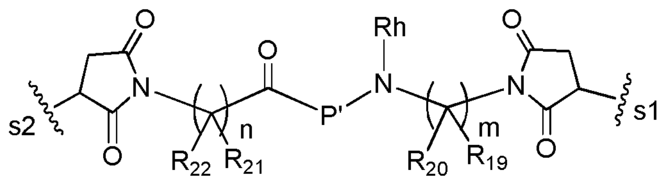

;

wherein:

s1 is the site covalently linked to the CBA and s2 is the site covalently linked to -S-group on $Cy^{C2}$;
Z is $-C(=O)-NR_9-$,or $-NR_9-C(=O)-$;
Q is -H, a charged substituent, or an ionizable group;
$R_9$, $R_{10}$, $R_{11}$, $R_{12}$, $R_{13}$, $R_{19}$, $R_{20}$, $R_{21}$ and $R_{22}$, for each occurrence, are independently - H or a $(C_1-C_3)$alkyl;
q and r, for each occurrence, are independently an integer between 0 and 10;
m and n are each independently an integer between 0 and 10;
$R^h$ is -H or a $(C_1-C_3)$alkyl; and
P' is an amino acid residue or a peptide containing 2 to 20 amino acid residues.

In a more specific embodiment, q and r are each independently an integer between 1 to 6, more specifically, an integer between 1 to 3. Even more specifically, $R_{10}$, $R_{11}$, $R_{12}$ and $R_{13}$ are all H.

In another more specific embodiment, m and n are each independently an integer between 1 and 6, more specifically, an integer between 1 to 3. Even more specifically, $R_{19}$, $R_{20}$, $R_{21}$ and $R_{22}$ are all H.

In a 11th specific embodiment, for immunoconjugates of formula (C2), $Cy^{C2}$ is represented by formula (C2a) or (C2a1); and the remaining variables are as described above in the 10th specific embodiment of the second embodiment or any more specific embodiments described therein.

In a 12th specific embodiment, for immunoconjugates of formula (C2), $Cy^{C2}$ is represented by formula (C2b) or (C2b1); and the remaining variables are as described above in the 10th specific embodiment of the second embodiment.

In a 13th specific embodiment, for immunoconjugates of formula (C2), P' is a peptide containing 2 to 5 amino acid residues; and the remaining variables are as described in the 10th, 11th or 12th specific embodiment of the second embodiment or any more specific embodiments described therein. In a more specific embodiment, P' is selected from Gly-Gly-Gly, Ala-Val, Val-Ala, Val-Cit, Val-Lys, Phe-Lys, Lys-Lys, Ala-Lys, Phe-Cit, Leu-Cit, Ile-Cit, Trp, Cit, Phe-Ala, Phe-$N^9$-tosyl-Arg, Phe-$N^9$-nitro-Arg, Phe-Phe-Lys, D-Phe-Phe-Lys, Gly-Phe-Lys, Leu-Ala-Leu, Ile-Ala-Leu, Val-Ala-Val, Ala-Leu-Ala-Leu (SEQ ID NO:144), β-Ala-Leu-Ala-Leu (SEQ ID NO: 145), Gly-Phe-Leu-Gly (SEQ ID NO: 146), Val-Arg, Arg-Val, Arg-Arg, Val-D-Cit, Val-D-Lys, Val-D-Arg, D-Val-Cit, D-Val-Lys, D-Val-Arg, D-Val-D-Cit, D-Val-D-Lys, D-Val-D-Arg, D-Arg-D-Arg, Ala-Ala, Ala-D-Ala, D-Ala-Ala, D-Ala-D-Ala, Ala-Met, Met-Ala, Gln-Val, Asn-Ala, Gln-Phe and Gln-Ala. In another more specific embodiment, P' is Gly-Gly-Gly, Ala-Val, Ala-Ala, Ala-D-Ala, D-Ala-Ala, or D-Ala-D-Ala.

In a 14th specific embodiment, for immunoconjugates of formula (C2), -Lc'- is represented by the following formula:

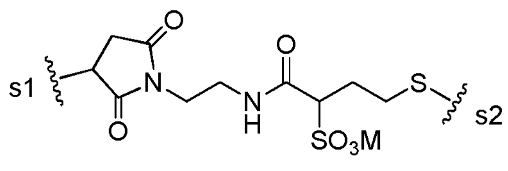

;

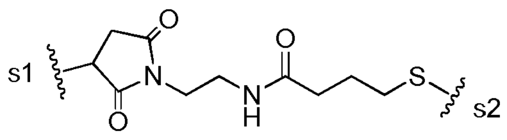

;



or

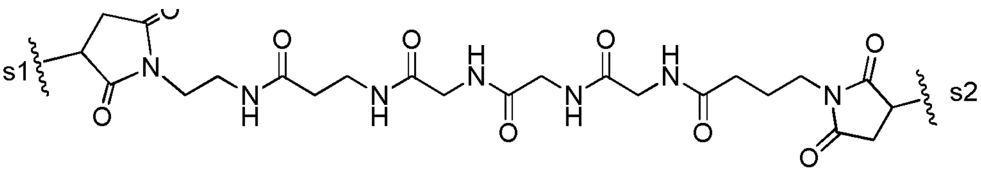

In a 15th specific embodiment, for immunoconjugates of (C2), $R^e$ is H or Me; $R^{x1}$ is $-(CH_2)_p-(CR^fR^g)-$, and $R^{x2}$ is $-(CH_2)_p-(CR^fR^g)-$, wherein $R^f$ and $R^g$ are each independently -H or a $(C_1-C_4)$alkyl; and p is 0, 1, 2 or 3; and the remaining variables are as described above in the 10th, 11th, 12th, 13th, or 14th specific embodiment of the second embodiment. More specifically, $R^f$ and $R^g$ are the same or different, and are selected from - H and -Me.

In a 16th specific embodiment, the immunoconjugate of the second embodiment is represented by the following formula:

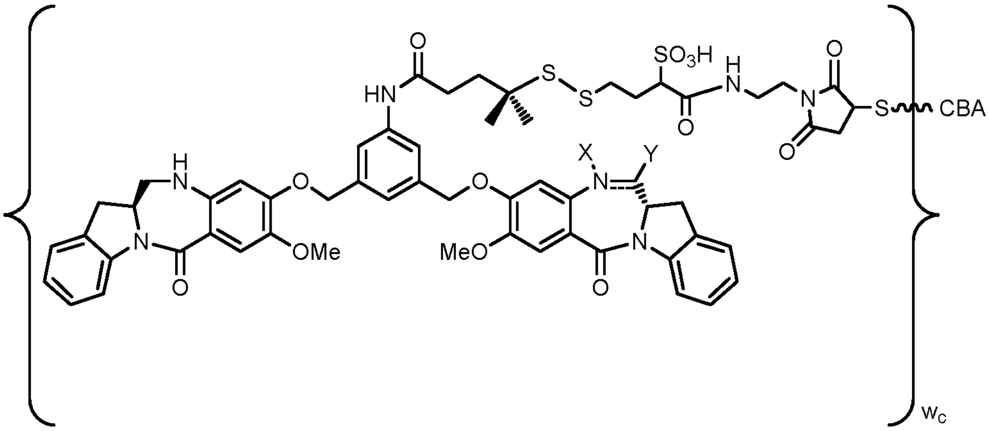

;

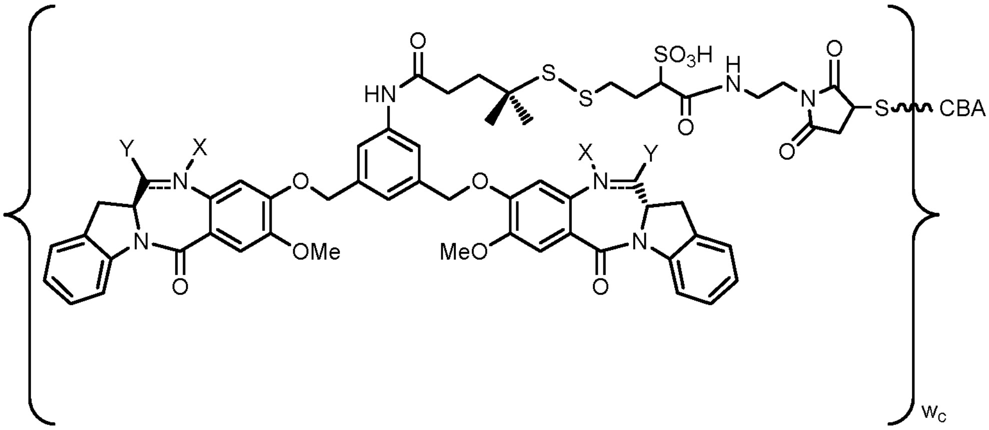

;

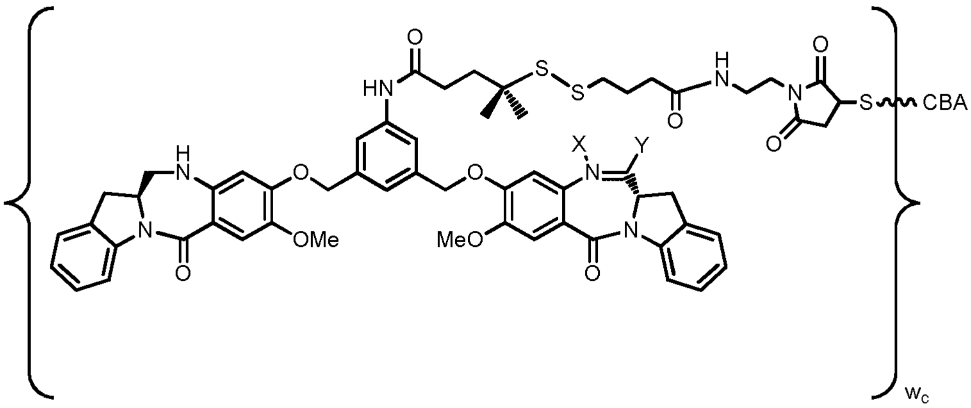

;

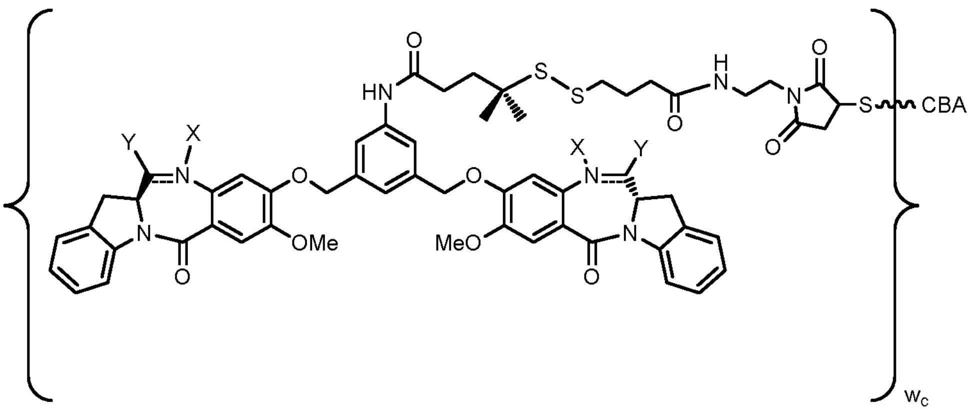

;

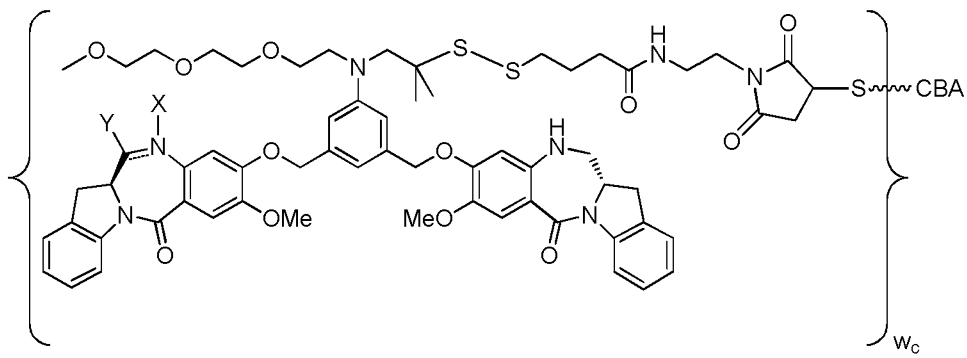

;

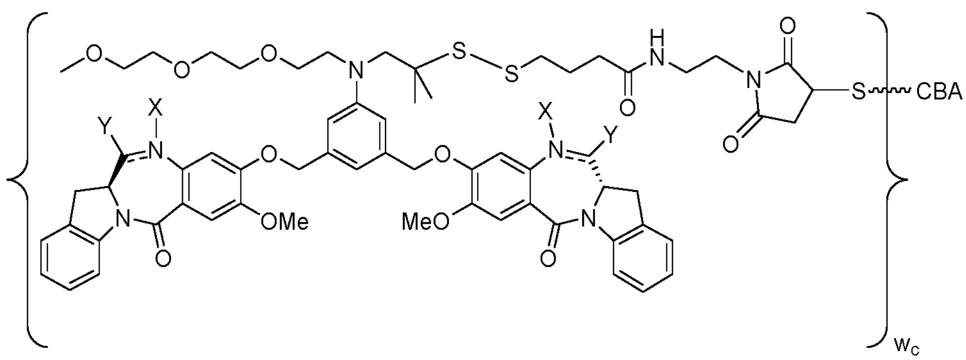

;

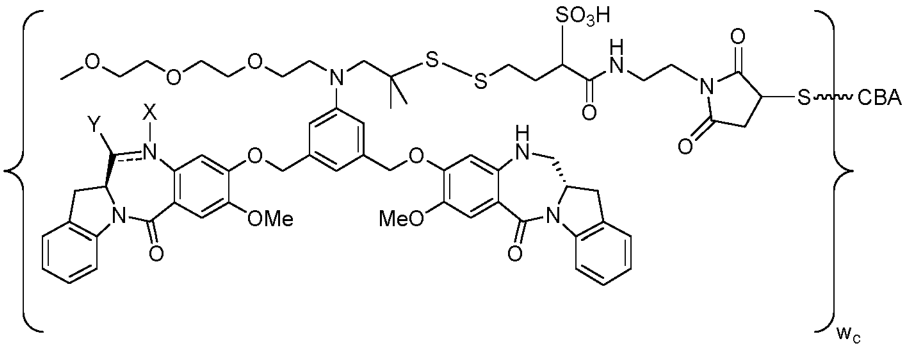

;

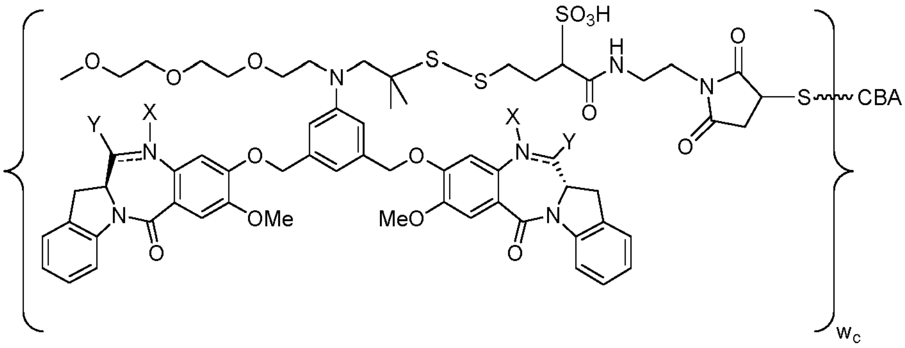

;

or a pharmaceutically acceptable salt thereof, wherein the double line



between N and C represents a single bond or a double bond, provided that when it is a double bond, X is absent and Y is -H, and when it is a single bond, X is -H, and Y is -OH or $-SO_3H$ or a pharmaceutically acceptable salt thereof. In a more specific embodiment, the double line



between N and C represents a double bond, X is absent and Y is -H. In another specific embodiment, the double line



between N and C represents a single bond, X is -H and Y is $-SO_3H$ or a pharmaceutically acceptable salt thereof.

In a 17th specific embodiment, the immunoconjugate of the second embodiment is represented by the following formula:

$$\text{CBA}\left(-\text{Cy}^{C3}\right)_{W_C} \quad \text{(C3)},$$

wherein:

CBA is an anti-ADAM9 antibody or an ADAM9-binding fragment thereof described herein above covalently linked to $Cy^{C3}$ through a cysteine residue;
$W_C$ is 1 or 2;
$Cy^{C3}$ is represented by the following formula:

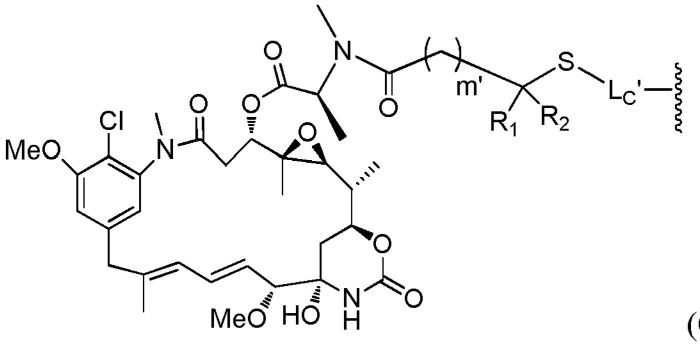

(C3a)

wherein:

m'is 1 or 2;
$R_1$ and $R_2$, are each independently -H or a $(C_1\text{-}C_3)$alkyl;
$L_C$' is represented by the following formula:

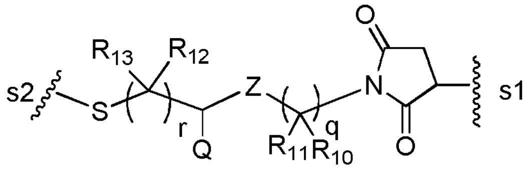

;

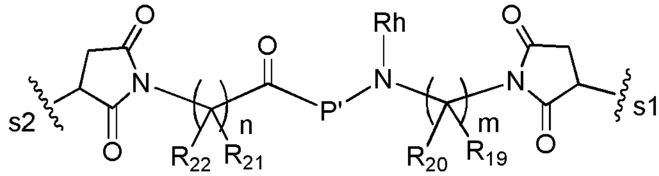

;

wherein:

s1 is the site covalently linked to the CBA and s2 is the site covalently linked to -S-group on $Cy^{C3}$;
Z is -C(=O)-$NR_9$-,or -$NR_9$-C(=O)-;
Q is H, a charged substituent, or an ionizable group;
$R_9$, $R_{10}$, $R_{11}$, $R_{12}$, $R_{13}$, $R_{19}$, $R_{20}$, $R_{21}$ and $R_{22}$, for each occurrence, are independently - H or a $(C_1\text{-}C_3)$alkyl;
q and r, for each occurrence, are independently an integer between 0 and 10;
m and n are each independently an integer between 0 and 10;
$R^h$ is -H or a $(C_1\text{-}C_3)$alkyl; and
P' is an amino acid residue or a peptide containing 2 to 20 amino acid residues.

In a more specific embodiment, q and r are each independently an integer between 1 to 6, more specifically, an integer from 1 to 3. Even more specifically, $R_{10}$, $R_{11}$, $R_{12}$ and $R_{13}$ are all H.

In another more specific embodiment, m and n are each independently an integer between 1 and 6, more specifically, an integer from 1 to 3. Even more specifically, $R_{19}$, $R_{20}$, $R_{21}$ and $R_{22}$ are all H.

In a 18th specific embodiment, for immunoconjugates of formula (C3), P' is a peptide containing 2 to 5 amino acid residues; and the remaining variables are as described above in the 17th specific embodiment of the second embodiment or any more specific embodiments described therein. In a more specific embodiment, P' is selected from Gly-Gly-Gly, Ala-Val, Val-Ala, Val-Cit, Val-Lys, Phe-Lys, Lys-Lys, Ala-Lys, Phe-Cit, Leu-Cit, Ile-Cit, Trp, Cit, Phe-Ala, Phe-$N^9$-tosyl-Arg, Phe-$N^9$-nitro-Arg, Phe-Phe-Lys, D-Phe-Phe-Lys, Gly-Phe-Lys, Leu-Ala-Leu, Ile-Ala-Leu, Val-Ala-Val, Ala-Leu-Ala-Leu (SEQ ID NO:144), β-Ala-Leu-Ala-Leu (SEQ ID NO: 145), Gly-Phe-Leu-Gly (SEQ ID NO: 146), Val-Arg, Arg-Val, Arg-Arg, Val-D-Cit, Val-D-Lys, Val-D-Arg, D-Val-Cit, D-Val-Lys, D-Val-Arg, D-Val-D-Cit, D-Val-D-Lys, D-Val-D-Arg, D-Arg-D-Arg, Ala-Ala, Ala-D-Ala, D-Ala-Ala, D-Ala-D-Ala, Ala-Met, Met-Ala, Gln-Val, Asn-Ala, Gln-Phe and Gln-Ala. In another more specific embodiment, P' is Gly-Gly-Gly, Ala-Val, Ala-Ala, Ala-D-Ala, D-Ala-Ala, or D-Ala-D-Ala.

In a 19th specific embodiment, for immunoconjugates of formula (C3), -Lc'- is represented by the following formula:

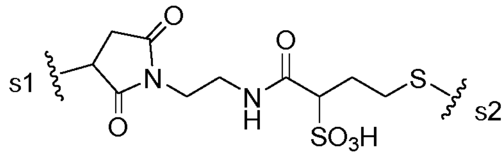

;

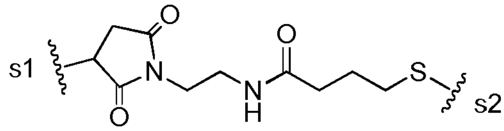

;

or

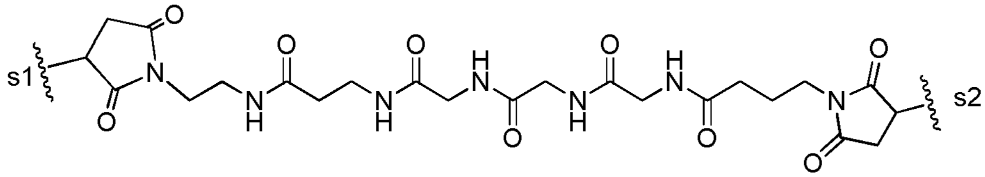

,

wherein M is $H^+$ or a cation; and the remaining variables are as described above in the 17th or 18th specific embodiment of the second embodiment or any more specific embodiments described therein.

In a 20th specific embodiment, for immunoconjugates of formula (C3), m' is 1 and $R_1$ and $R_2$ are both H; and the remaining variables are as described above in the 17th, 18th or 19th specific embodiment of the second embodiment or any more specific embodiments described therein.

In a 21st specific embodiment, for immunoconjugates of formula (C3), m' is 2 and $R_1$ and $R_2$ are both Me; and the remaining variables are as described above in the 17th, 18th or 19th specific embodiment of the second embodiment or any more specific embodiments described therein.

In a 22nd specific embodiment, the immunoconjugate of the second embodiment is represented by the following formula:

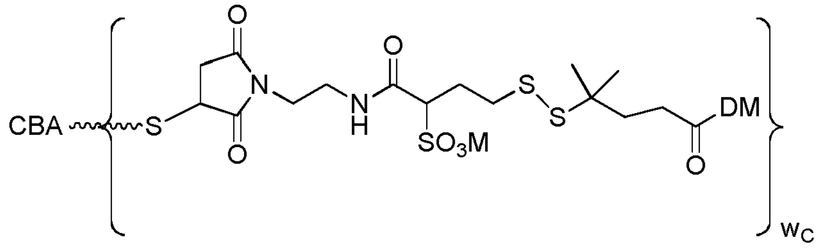

;

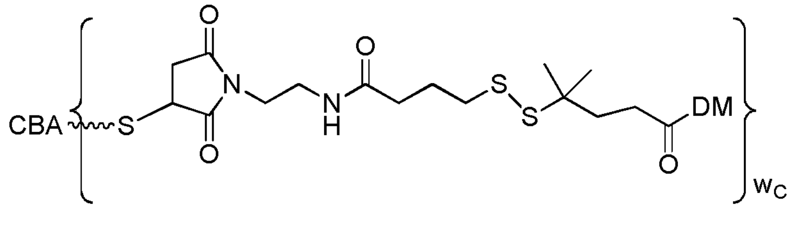

;

or

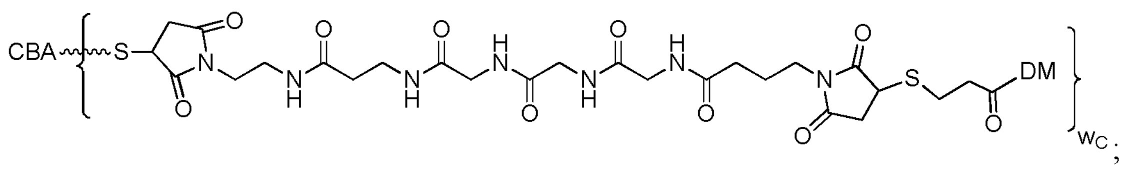

or a pharmaceutically acceptable salt thereof, wherein DM is a drug moiety represented by the following formula:

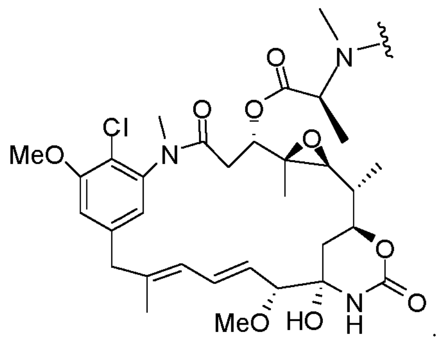

In a 23rd specific embodiment, for the immunoconjugates of the second embodiment, Y is $-SO_3H$, $-SO_3Na$ or $-SO_3K$; and the remaining variables are as described in any one of the 1st to 22nd specific embodiments of the second embodiment or any more specific embodiments described therein. In one embodiment, Y is $-SO_3Na$.

**C. Exemplary Linker Molecules**

Any suitable linkers known in the art can be used in preparing the immunoconjugates of the present invention. In certain embodiments, the linkers are bifunctional linkers. As used herein, the term "**bifunctional linker**" refers to modifying agents that possess two reactive groups; one of which is capable of reacting with a cell binding agent while the other one reacts with the cytotoxic compound to link the two moieties together. Such bifunctional crosslinkers are well known in the art (see, for example, Isalm and Dent in Bioconjugation chapter 5, p218-363, Groves Dictionaries Inc. New York, 1999). For example, bifunctional crosslinking agents that enable linkage via a thioether bond include *N*-succinimidyl-4-(N-maleimidomethyl)-cyclohexane-1-carboxylate (SMCC) to introduce maleimido groups, or with *N*-succinimidyl-4-(iodoacetyl)-aminobenzoate (SIAB) to introduce iodoacetyl groups. Other bifunctional crosslinking agents that introduce maleimido groups or haloacetyl groups on to a cell binding agent are well known in the art (see US Patent Publication Nos. 2008/0050310, 20050169933, available from Pierce Biotechnology Inc. P.O. Box 117, Rockland, IL 61105, USA) and include bis-maleimidopolyethyleneglycol (BMPEO), $BM(PEO)_2$, $BM(PEO)_3$, N-(β-maleimidopropyloxy)succinimide ester (BMPS), γ-maleimidobutyric acid N-succinimidyl ester (GMBS), ε-maleimidocaproic acid N-hydroxysuccinimide ester (EMCS), 5-maleimidovaleric acid NHS, HBVS, N-succinimidyl-4-(N-maleimidomethyl)-cyclohexane-1-carboxy-(6-amidocaproate), which is a "long chain" analog of SMCC (LC-SMCC), m-maleimidobenzoyl-N-hydroxysuccinimide ester (MBS), 4-(4-N-maleimidophenyl)-butyric acid hydrazide or HCl salt (MPBH), N-succinimidyl 3-(bromoacetamido)propionate (SBAP), N-succinimidyl iodoacetate (SIA), k-maleimidoundecanoic acid N-succinimidyl ester (KMUA), N-succinimidyl 4-(p-maleimidophenyl)-butyrate (SMPB), succinimidyl-6-(β-maleimidopropionamido)hexanoate (SMPH), succinimidyl-(4-vinylsulfonyl)benzoate (SVSB), dithiobis-maleimidoethane (DTME), 1,4-bis-maleimidobutane (BMB), 1,4-bismaleimidyl-2,3-dihydroxybutane (BMDB), bis-maleimidohexane (BMH), bis-maleimidoethane (BMOE), sulfosuccinimidyl 4-(N-maleimido-methyl)cyclohexane-1-carboxylate (sulfo-SMCC), sulfosuccinimidyl(4-iodo-acetyl)aminobenzoate (sulfo-SIAB), m-maleimidobenzoyl-N-hydroxysulfosuccinimide ester (sulfo-MBS), N-(y-maleimidobutryloxy)sulfosuccinimide ester (sulfo-GMBS), N-(ε-maleimidocaproyloxy)sulfosuccimido ester (sulfo-EMCS), N-(k-maleimidoundecanoyloxy)sulfosuccinimide ester (sulfo-KMUS), and sulfosuccinimidyl 4-(p-maleimidophenyl)butyrate (sulfo-SMPB).

Heterobifunctional crosslinking agents are bifunctional crosslinking agents having two different reactive groups. Heterobifunctional crosslinking agents containing both an amine-reactive *N*-hydroxysuccinimide group (NHS group) and a carbonyl-reactive hydrazine group can also be used to link the cytotoxic compounds described herein with a cell-binding agent (*e.g.*, antibody). Examples of such commercially available heterobifunctional crosslinking agents include succinimidyl 6-hydrazinonicotinamide acetone hydrazone (SANH), succinimidyl 4-hydrazidoterephthalate hydrochloride (SHTH) and succinimidyl hydrazinium nicotinate hydrochloride (SHNH). Conjugates bearing an acid-labile linkage can also be prepared using a hydrazine-bearing benzodiazepine derivative of the present invention. Examples of bifunctional

crosslinking agents that can be used include succinimidyl-p-formyl benzoate (SFB) and succinimidyl-p-formylphenoxyacetate (SFPA).

Bifunctional crosslinking agents that enable the linkage of cell binding agent with cytotoxic compounds via disulfide bonds are known in the art and include *N*-succinimidyl-3-(2-pyridyldithio)propionate (SPDP), *N*-succinimidyl-4-(2-pyridyldithio)pentanoate (SPP), *N*-succinimidyl-4-(2-pyridyldithio)butanoate (SPDB), *N*-succinimidyl-4-(2-pyridyldithio)2-sulfo butanoate (sulfo-SPDB) to introduce dithiopyridyl groups. Other bifunctional crosslinking agents that can be used to introduce disulfide groups are known in the art and are disclosed in U.S. Patents 6,913,748, 6,716,821 and US Patent Publications 20090274713 and 20100129314. Alternatively, crosslinking agents such as 2-iminothiolane, homocysteine thiolactone or S-acetylsuccinic anhydride that introduce thiol groups can also be used.

In certain embodiments, the bifunctional linkers are represented by any one of the formula (a1L) - (a10L) described below.

### D. Exemplary Pharmacological Agents

### 1. Maytansinoid

In certain embodiments, the pharmacological agent is a maytansinoid compound, such as those described in US Patent Nos. 5,208,020 and 7,276,497. In certain embodiments, the maytansinoid compound is represented by the following formula:

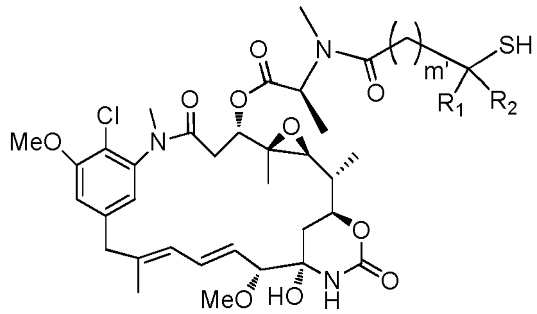

wherein the variables are as described above in any one of the 13th to 17th specific embodiments of the first embodiment above and any more specific embodiments described therein.

In a more specific embodiment, the maytansinoid compound is DM4:

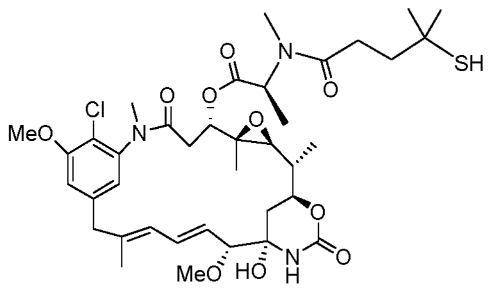

.

In another embodiment, the maytansinoid compound is DM1:

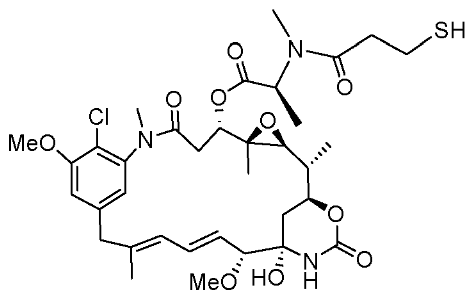

.

## 2. Benzodiazepine

In certain embodiments, the pharmacological agent is a benzodiazepine compound, such as pyrrolobenzodiazepine (PBD) (such as those described in WO2010/043880, WO2011/130616, WO2009/016516, WO 2013/177481 and WO 2012/112708) and indolinobenzodiazepine (IGN) compounds (such as those described in WO/2010/091150, and WO 2012/128868 and U.S. Application No. 15/195,269, filed on June 28, 2016, entitled "CONJUGATES OF CYSTEINE ENGINEERED ANTIBODIES". As used herein, a "benzodiazepine" compound is a compound having a benzodiazepine core structure. The benzodiazepine core can be substituted or unsubstituted, and/or fused with one or more ring structures. It also includes a compound having two benzodiazepine core linked by a linker. The imine functionality (-C=N-) as part of benzodiazepine core can be reduced.

As used herein, a "pyrrolobenzodiazepine" (PBD) compound is a compound having a pyrrolobenzodiazepine core structure. The pyrrolobenzodiazepine can be substituted or unsubstituted. It also includes a compound having two pyrrolobenzodiazepine core linked by a linker. The imine functionality (-C=N-) as part of indolinobenzodiazepine core can be reduced.

In certain embodiments, the pharmacological agent is an indolinobenzodiazepine compound represented by the following formula:

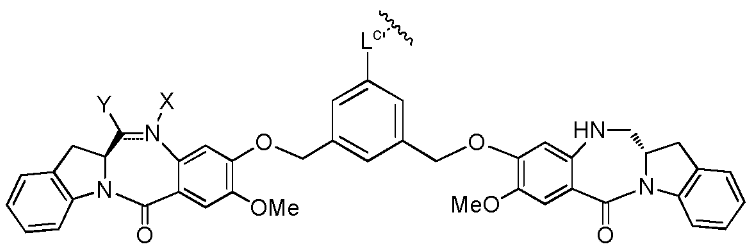

(L1a'),

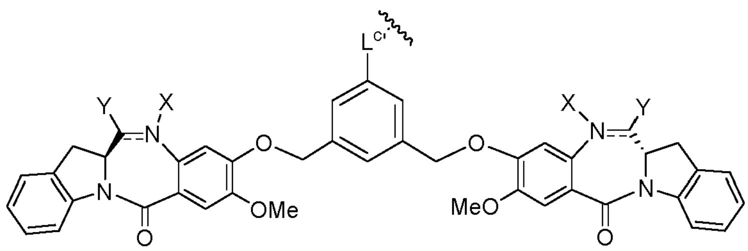

(L1a'1),

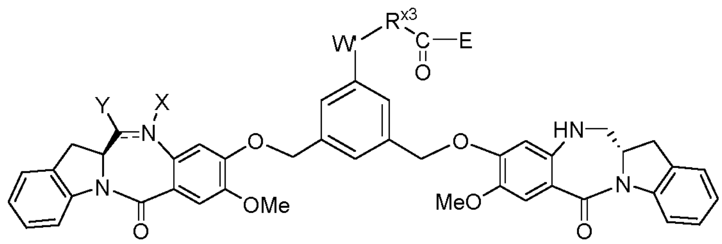

(L1b'),

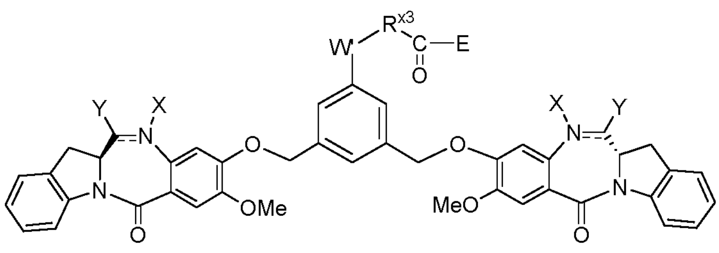

(L1b'1),

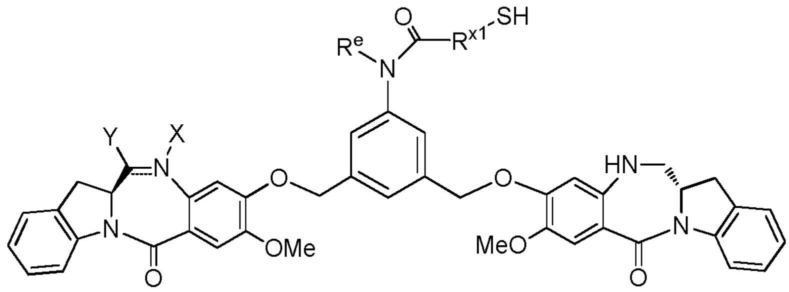

(L2a');

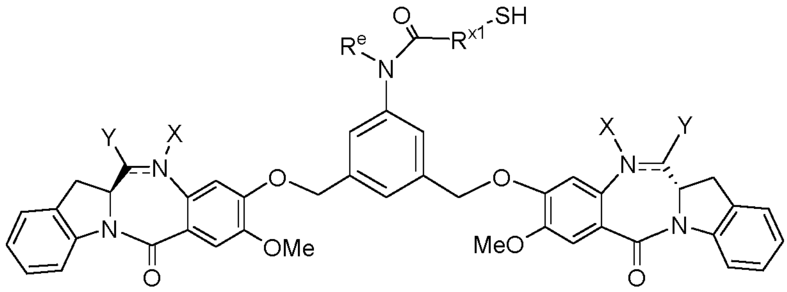

(L2a'1);

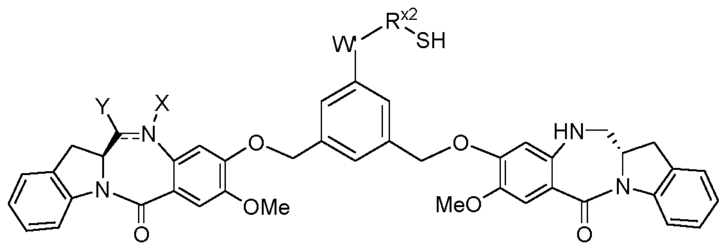

(L2b');

or

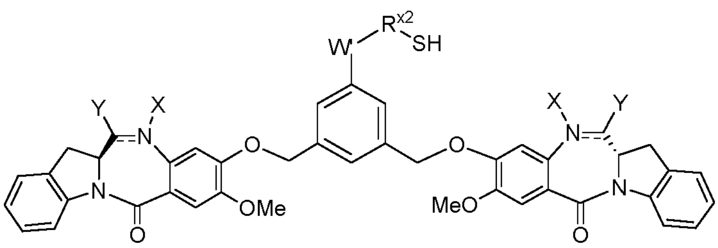

(L2b'1),

or a pharmaceutically acceptable salt thereof, wherein:

$L^{c'}$ is represented by the following formula:

- $NR_5$-P-C(=0)-$(CR_aR_b)_m$-C(=O)E (B1); or
- $NR_5$-P-C(=O)-$(CR_aR_b)_m$-S-$Z^s$ (B2)

C(=O)E is a reactive ester group, such as N-hydroxysuccinimide ester, N-hydroxy sulfosuccinimide ester, nitrophenyl (*e.g.,* 2 or 4-nitrophenyl) ester, dinitrophenyl (*e.g., 2,4*-dinitrophenyl) ester, sulfo-tetraflurophenyl (*e.g.*, 4-sulfo-2,3,5,6-tetrafluorophenyl) ester, or pentafluorophenyl ester, preferably N-hydroxysuccinimide ester;

$Z^s$ is represented by the following formula:

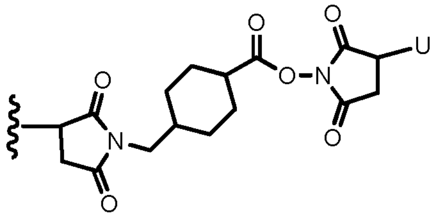 (a1); 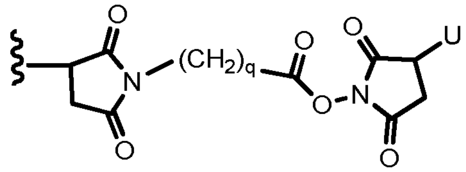 (a2);

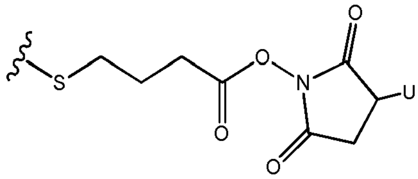 (a3);

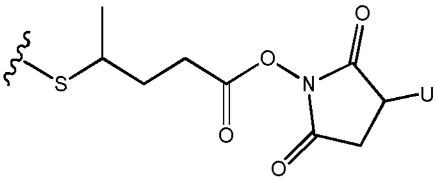 (a4); 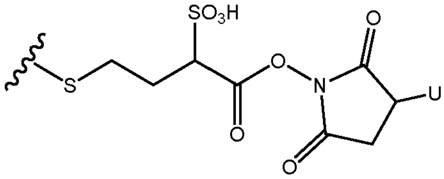 (a5),

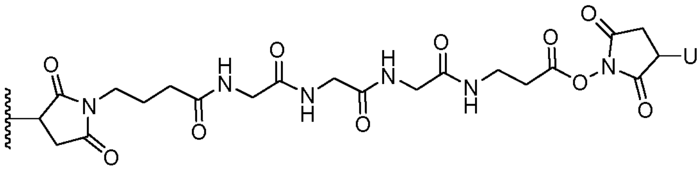 (a6),

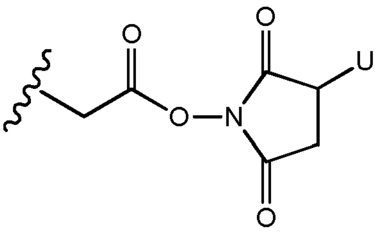 (a7); 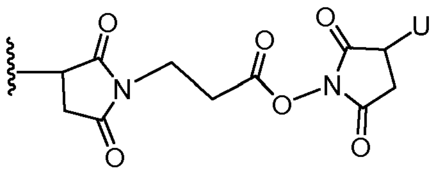 (a8);

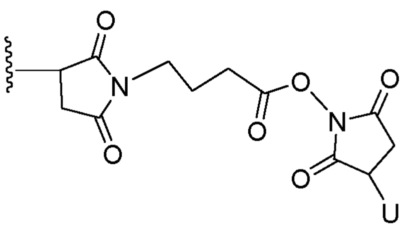 (a9);

and

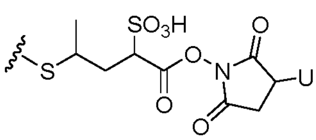 (a10),

wherein:

q is an integer from 1 to 5; and

U is -H or $SO_3H$ or a pharmaceutically acceptable salt thereof; and the remaining variables are as described in any one of the 1st to 12th and 17th specific embodiments of the first embodiment described above or any more specific embodiments described therein.

In certain embodiments, the pharmacological agent is an indolinobenzodiazepine compound represented by the following formula:

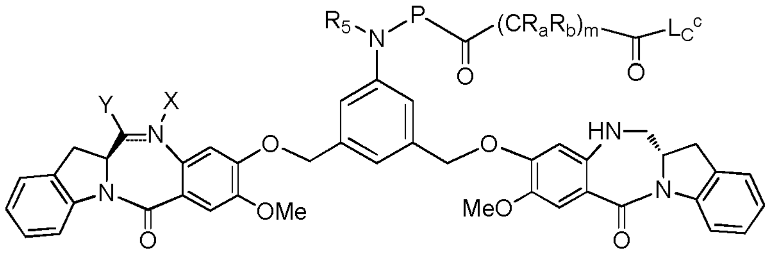

(C1a');

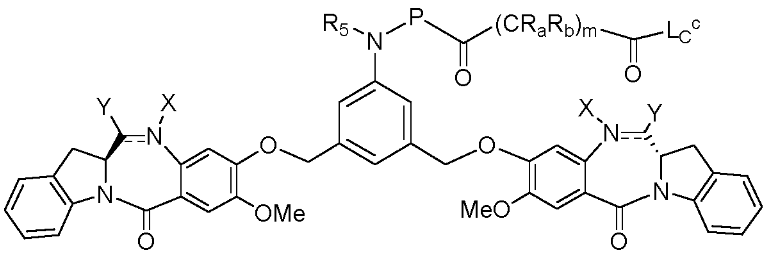

(C1a'1)

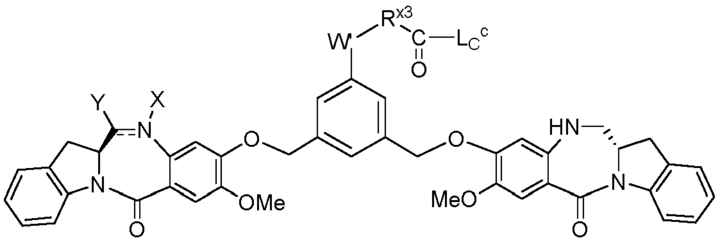

(C1b'),

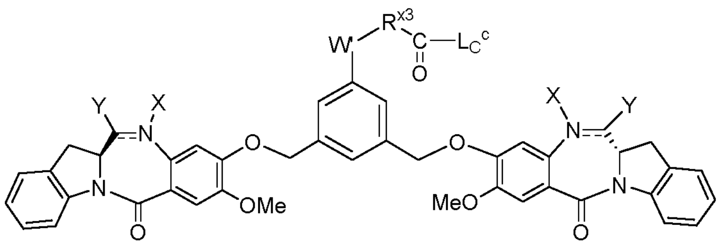

(C1b'1),

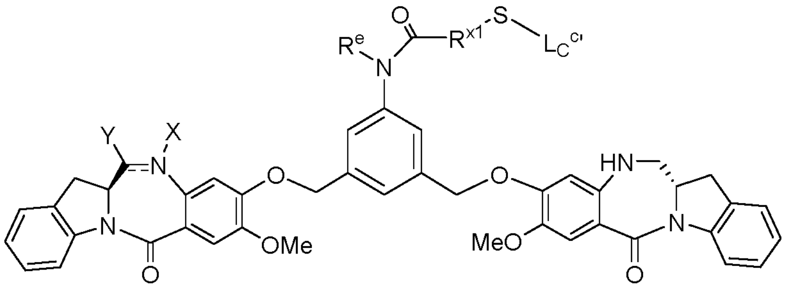

(C2a"),

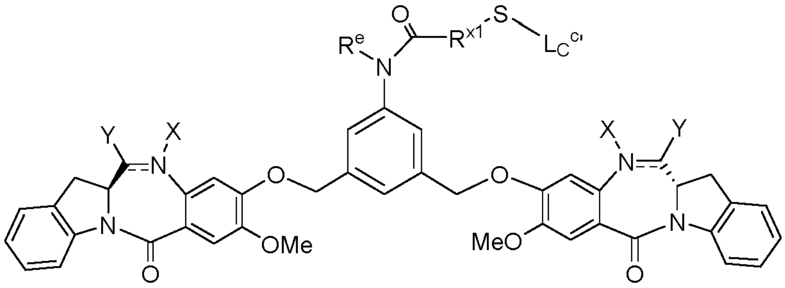

(C2a"1),

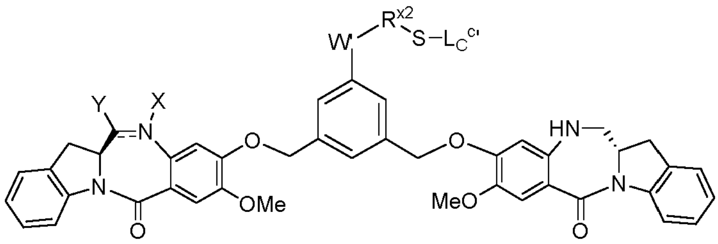

(C2b"),

or

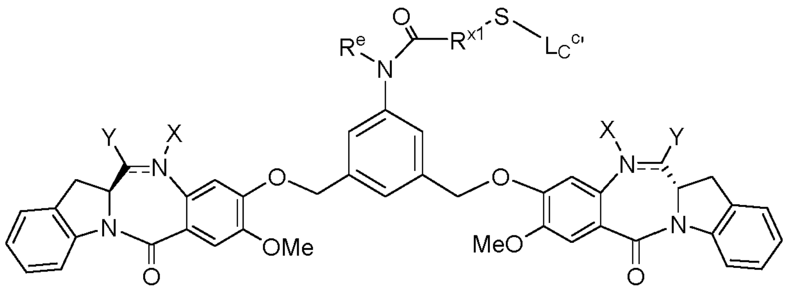

(C2b"1)

or a pharmaceutically acceptable salt thereof, wherein:

—$L_C{}^c$ for formulas (C1a'), (C1a'1), (C1b') and (C1b'1) is represented by the following formula:

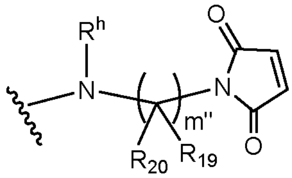

wherein the variables are as described above in any one of the 1st to 9th and 23rd specific embodiments of the second embodiment or any more specific embodiments described therein; and

$L_C^{c'}$ for formulas (C2a"), (C2a"1), (C2b") and (C2b"1) is represented by the following formula:

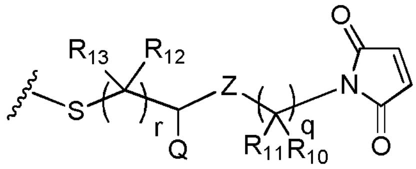

;

or

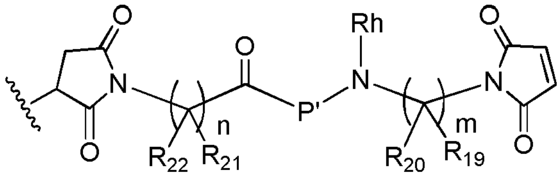

;

wherein the variables are as described above in any one of the 10th to 16th and 23rd specific embodiment of the second embodiment or any more specific embodiments described therein.

In certain embodiments, the pharmacological agent is an indolinobenzodiazepine compound of any one of the following or a pharmaceutically acceptable salt thereof:

| Compound No. | Structure |
|---|---|
| D1 | 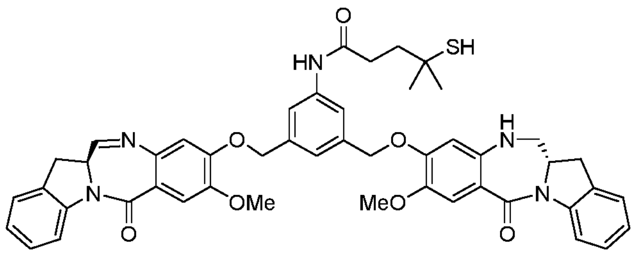 |
| sD1 | 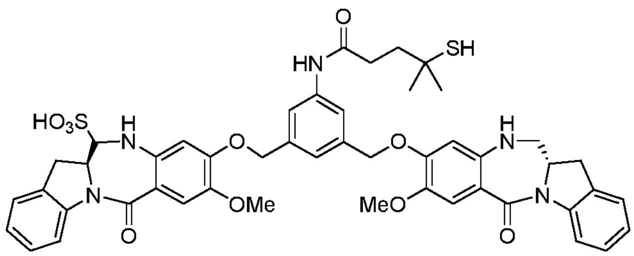 |
| D2 | 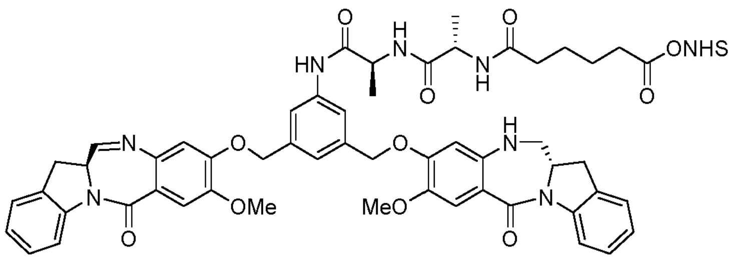 |

(continued)

| Compound No. | Structure |
|---|---|
| sD2 | 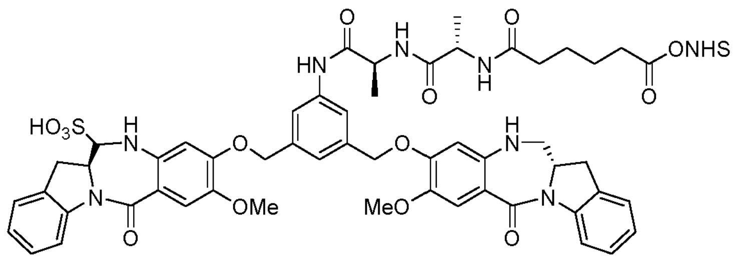 |
| DGN462 | 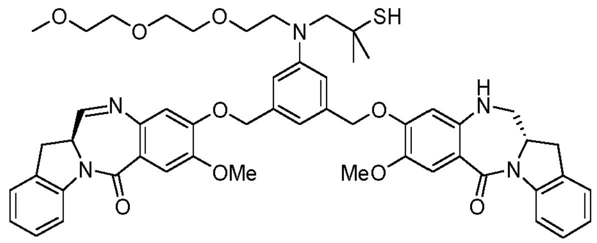 |
| sDGN462 | 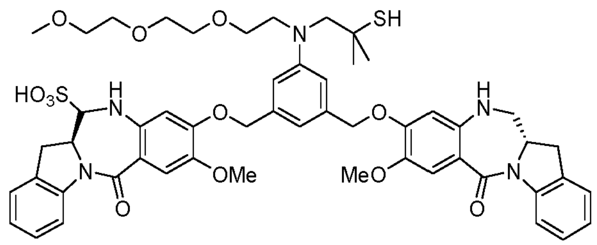 |
| D3 | 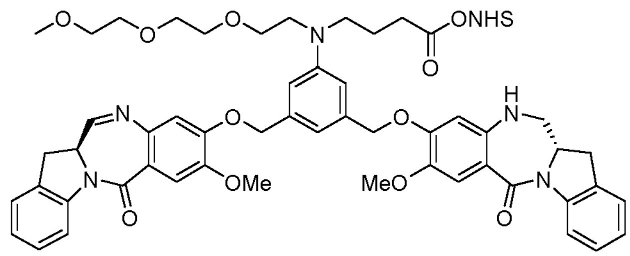 |
| sD3 | 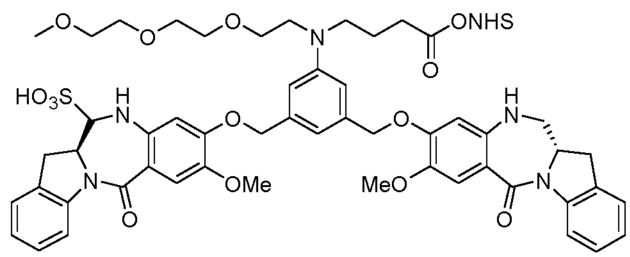 |
| D4 | 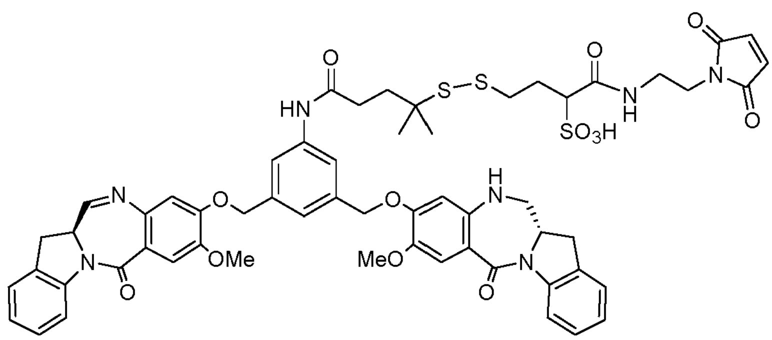 |

(continued)

| Compound No. | Structure |
|---|---|
| sD4 | 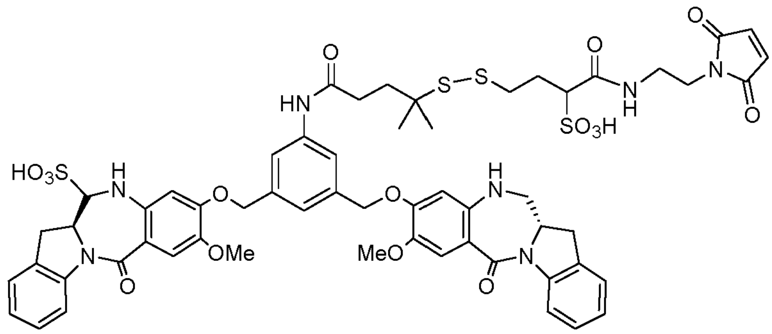 |
| D5 | 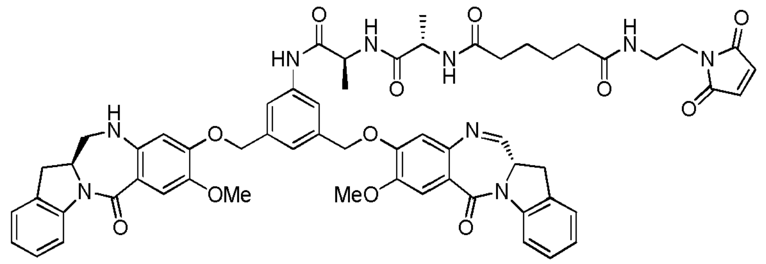 |
| sD5 | 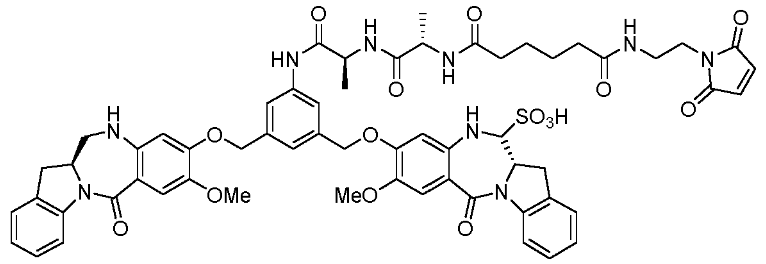 |
| D5' | 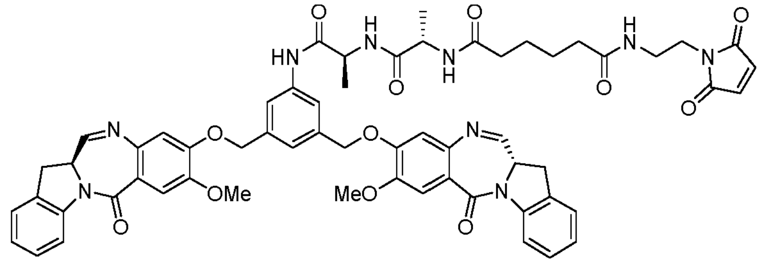 |
| sD5' | 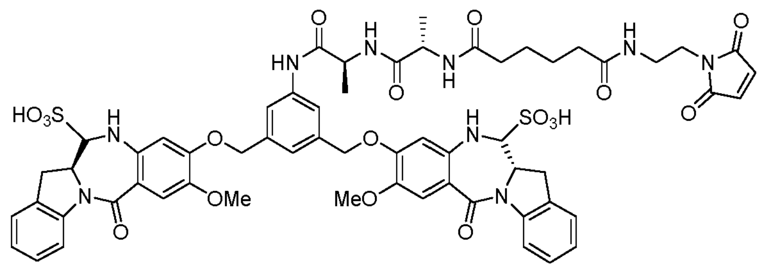 |

(continued)

| Compound No. | Structure |
|---|---|
| D6 | 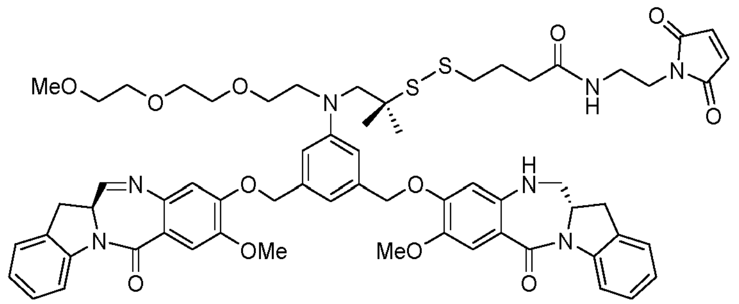 |
| sD6 | 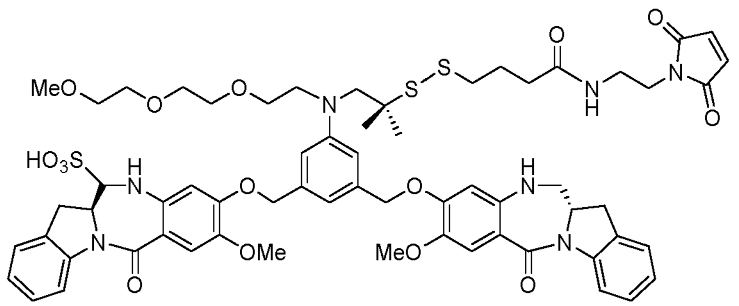 |
| D7 | 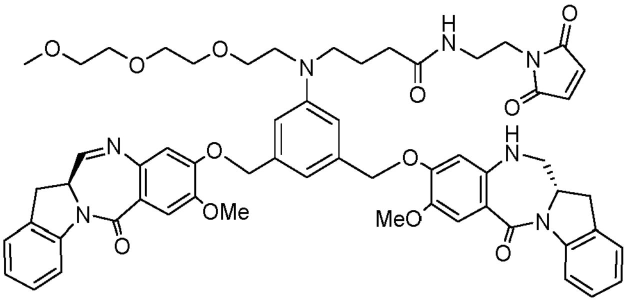 |
| sD7 | 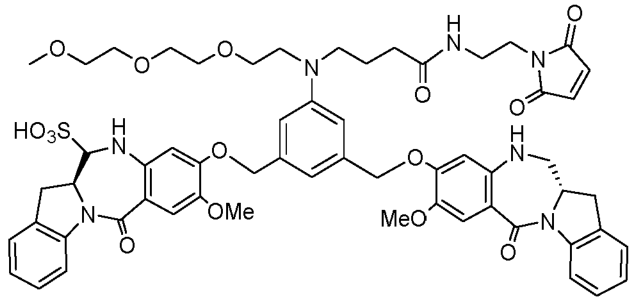 |

Compounds D1, sD1, D2, sD2, DGN462, sDGN462, D3 and sD3 shown above can be prepared according to procedures described in U.S. Patent Nos. 9,381,256, 8,765,740, 8,426,402, and 9,353,127, and U.S. Application Publication US2016/0082114.

In certain embodiments, the pharmaceutically acceptable salt of the compounds shown above (*e.g.,* sD1, sD2, sD4, sDGN462, sD3, sD4, sD5, sD5', sD6 or sD7) is a sodium or potassium salt. More specifically, the pharmaceutically acceptable salt is a sodium salt.

In a specific embodiment, the pharmacological agent is represented by the following formula:

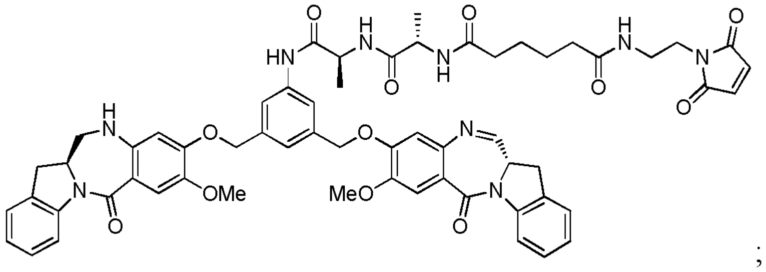

;

or

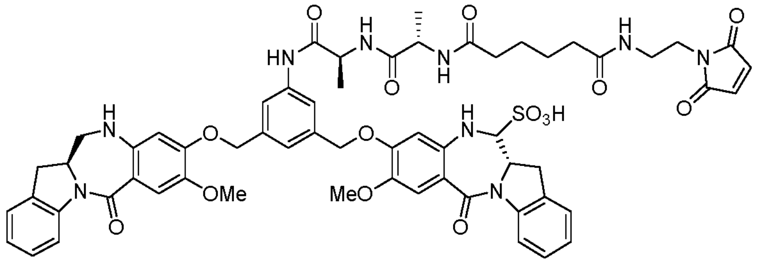

or a pharmaceutically acceptable salt thereof. In a specific embodiment, the pharmaceutically acceptable salt is a sodium or a potassium salt.

In another specific embodiment, the pharmacological agent is represented by the following formula:

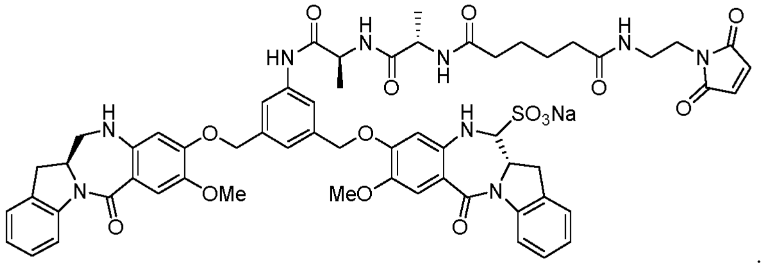

.

## IV. Methods of Production

The anti-ADAM9 antibodies and ADAM9-binding fragments thereof of the present invention are most preferably produced through the recombinant expression of nucleic acid molecules that encode such polypeptides, as is well-known in the art.

Polypeptides of the invention may be conveniently prepared using solid phase peptide synthesis (Merrifield, B. (1986) "Solid Phase Synthesis," Science 232(4748):341-347; Houghten, R.A. (1985) "General Method For The Rapid Solid-Phase Synthesis Of Large Numbers Of Peptides: Specificity Of Antigen-Antibody Interaction At The Level Of Individual Amino Acids," Proc. Natl. Acad. Sci. (U.S.A.) 82(15):5131-5135; Ganesan, A. (2006) "Solid-Phase Synthesis In The Twenty-First Century," Mini Rev. Med. Chem. 6(1):3-10).

In an alternative, antibodies may be made recombinantly and expressed using any method known in the art. Antibodies may be made recombinantly by first isolating the antibodies made from host animals, obtaining the gene sequence, and using the gene sequence to express the antibody recombinantly in host cells (*e.g.,* CHO cells). Another method that may be employed is to express the antibody sequence in plants {*e.g.*, tobacco) or transgenic milk. Suitable methods for expressing antibodies recombinantly in plants or milk have been disclosed (see, for example, Peeters et al. (2001) "Production Of Antibodies And Antibody Fragments In Plants," Vaccine 19:2756; Lonberg, N. et al. (1995) "Human Antibodies From Transgenic Mice," Int. Rev. Immunol 13:65-93; and Pollock et al. (1999) "Transgenic Milk As A Method For The Production Of Recombinant Antibodies," J. Immunol Methods 231:147-157). Suitable methods for making derivatives of antibodies, *e.g.*, humanized, single-chain, *etc.* are known in the art, and have been described above. In another alternative, antibodies may be made recombinantly by phage display technology (see, for example, U.S. Patent Nos. 5,565,332; 5,580,717; 5,733,743; 6,265,150; and Winter, G. et al. (1994) "Making Antibodies By Phage Display Technology," Annu. Rev. Immunol. 12.433-455).

Vectors containing polynucleotides of interest (*e.g.*, polynucleotides encoding the polypeptide chains of the anti-ADAM9 antibodies and ADAM9-binding fragments thereof of the present invention) can be introduced into the host cell by any of a number of appropriate means, including electroporation, transfection employing calcium chloride, rubidium chloride, calcium phosphate, DEAE- dextran, or other substances; microprojectile bombardment; lipofection; and infection (*e.g.*, where the vector is an infectious agent such as vaccinia virus). The choice of introducing vectors or polynucleotides will often depend on features of the host cell.

Any host cell capable of overexpressing heterologous DNAs can be used for the purpose of expressing a polypeptide or protein of interest. Non-limiting examples of suitable mammalian host cells include but are not limited to COS, HeLa, and CHO cells.

The invention includes immunoconjugates comprising an amino acid sequence of an anti-ADAM9 antibody or ADAM9-binding fragment thereof of this invention. The polypeptides of this invention can be made by procedures known in the art. The polypeptides can be produced by proteolytic or other degradation of the antibodies, by recombinant methods (*i.e.,* single or fusion polypeptides) as described above or by chemical synthesis. Polypeptides of the antibodies, especially shorter polypeptides up to about 50 amino acids, are conveniently made by chemical synthesis. Methods of chemical synthesis are known in the art and are commercially available.

Modification of polypeptides is routine practice in the art and need not be described in detail herein. Examples of modified polypeptides include polypeptides with conservative substitutions of amino acid residues, one or more deletions or additions of amino acids which do not significantly deleteriously change the functional activity, or use of chemical analogs. Amino acid residues that can be conservatively substituted for one another include: glycine/alanine; serine/threonine; valine/isoleucine/leucine; asparagine/glutamine; aspartic acid/glutamic acid; lysine/arginine; and phenylalanine/tyrosine. These polypeptides also include glycosylated and non-glycosylated polypeptides, as well as polypeptides with other post-translational modifications, such as, for example, glycosylation with different sugars, acetylation, and phosphorylation. Preferably, the amino acid substitutions would be conservative, *i.e.,* the substituted amino acid would possess similar chemical properties as that of the original amino acid. Such conservative substitutions are known in the art, and examples have been provided above. Amino acid modifications can range from changing or modifying one or more amino acids to complete redesign of a region, such as the Variable Domain. Changes in the Variable Domain can alter binding affinity and/or specificity. Other methods of modification include using coupling techniques known in the art, including enzymatic means, oxidative substitution and chelation. Modifications can be used, for example, for attachment of labels for immunoassay, such as the attachment of radioactive moieties for radioimmunoassay. Modified polypeptides are made using established procedures in the art and can be screened using standard assays known in the art.

The invention encompasses immunoconjugates comprising fusion proteins possessing one or more of the anti-ADAM9-VL and/or VH of this invention. In one embodiment, a fusion polypeptide is provided that comprises a light chain, a heavy chain or both a light and heavy chain. In another embodiment, the fusion polypeptide contains a heterologous immunoglobulin constant region. In another embodiment, the fusion polypeptide contains a Light Chain Variable Domain and a Heavy Chain Variable Domain of an antibody produced from a publicly-deposited hybridoma. For purposes of this invention, an antibody fusion protein contains one or more polypeptide domains that specifically bind to ADAM9 and another amino acid sequence to which it is not attached in the native molecule, for example, a heterologous sequence or a homologous sequence from another region.

## V. Drug Conjugation

The immunoconjugates comprising an anti-ADAM9 antibody or an ADAM9-binding fragment thereof covalently linked to a pharmacological agent through the ε-amino group of one or more lysine residues located on the anti-ADAM9 antibody or an ADAM9-binding fragment thereof as described the first embodiment above or any specific embodiments descried therein can be prepared according to any methods known in the art, see, for example, WO 2012/128868 and WO2012/112687.

In certain embodiments, the immunoconjugates of the first embodiment can be prepared by a first method comprising the steps of reacting the CBA with the cytotoxic agent having an amine reactive group.

In one embodiment, for the first method described above, the reaction is carried out in the presence of an imine reactive reagent, such as $NaHSO_3$.

In one embodiment, for the first method described above the cytotoxic agent having an imine reactive group is represented by the following formula:

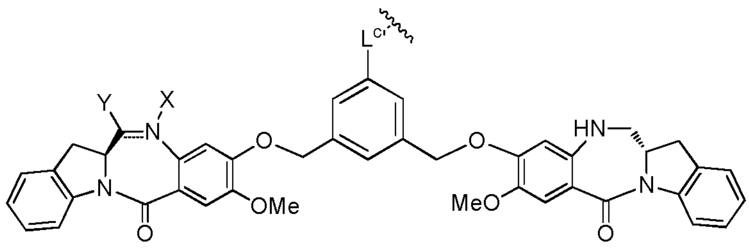
(L1a'),

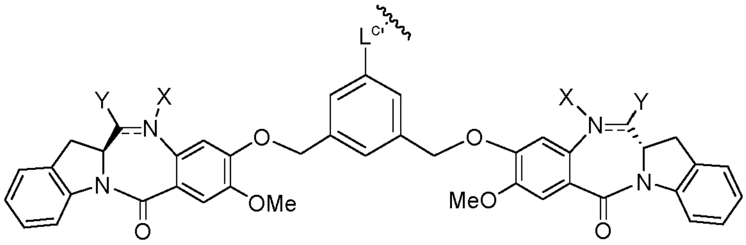
(L1a'1),

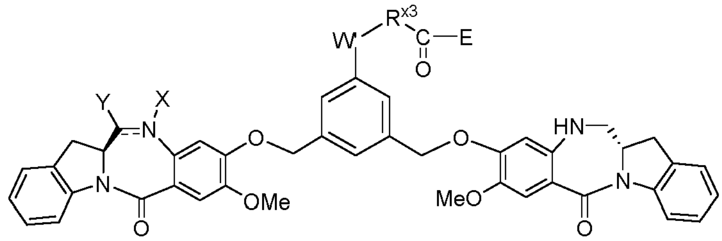
(L1b'),

, or

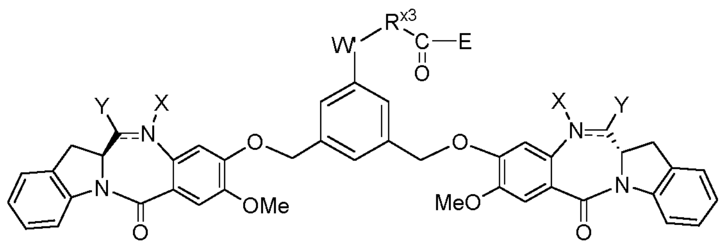
(L1b'1);

or a pharmaceutically acceptable salt thereof, wherein the definitions for the variables are described above for formulas (L1a'), (L1a'1), (L1b') and (L1b'1).

In certain embodiments, the immunoconjugates of the first embodiment can be prepared by a second method comprising the steps of:

(a) reacting the cytotoxic agent with a linker compound having an amine reactive group and a thiol reactive group to form a cytotoxic agent-linker compound having the amine reactive group bound thereto; and

(b) reacting the CBA with the cytotoxic agent-linker compound.

In one embodiment, for the second method described above, the reaction in step (a) is carried out in the presence of an imine reactive reagent (*e.g.*, $NaHSO_3$).

In one embodiment, for the second method described above, the cytotoxic agent-linker compound is reacted with the CBA without purification. Alternatively, the cytotoxic agent-linker compound is first purified before reacting with the CBA.

In certain embodiments, the immunoconjugates of the first embodiment can be prepared by a third method

comprising the steps of:

(a) reacting the CBA with a linker compound having an amine reactive group and a thiol reactive group to form a modified CBA having a thiol reactive group bound thereto; and

(b) reacting the modified CBA with the cytotoxic agent.

In one embodiment, for the third method described above, the reaction in step (b) is carried out in the presence of an imine reactive reagent (*e.g.*, $NaHSO_3$).

In certain embodiments, the immunoconjugates of the first embodiment can be prepared by a fourth method comprising the steps of reacting the CBA, a cytotoxic compound and a linker compound having an amine reactive group and a thiol reactive group.

In one embodiment, for the fourth method, the reaction is carried out in the presence of an imine reactive agent (*e.g.,* $NaHSO_3$).

In certain embodiments, for the second, third or fourth method, described above, the linker compound having an amine reactive group and a thiol reactive group is represented by the following formula:

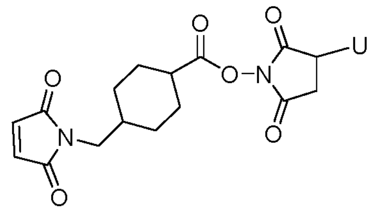
(a1L);
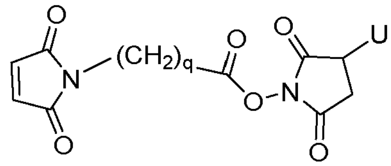
(a2L);

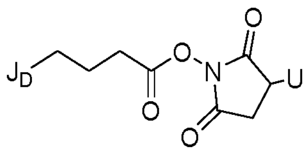
(a3L);

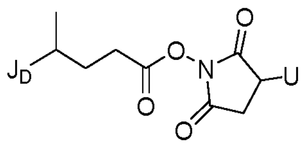
(a4L);
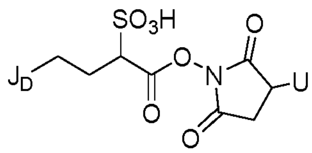
(a5L),

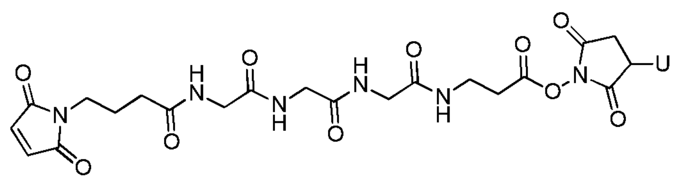
(a6L),

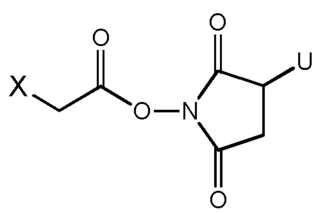
(a7L);
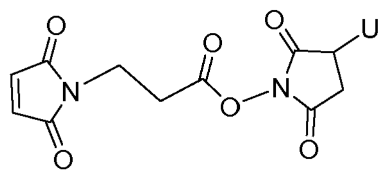
(a8L);

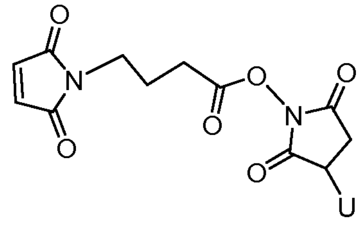
(a9L);

and

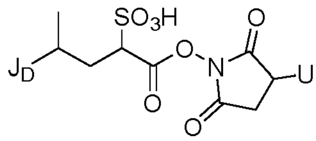
(a10L),

wherein X is halogen; $J_D$ -SH, -$SSR^d$, or -SC(=O)$R^g$; $R^d$ is phenyl, nitrophenyl, dinitrophenyl, carboxynitrophenyl, pyridyl or nitropyridyl; $R^g$ is an alkyl; and the remaining variables are as described above for formula (a1) - (a10); and the cytotoxic agent is represented by the following formula:

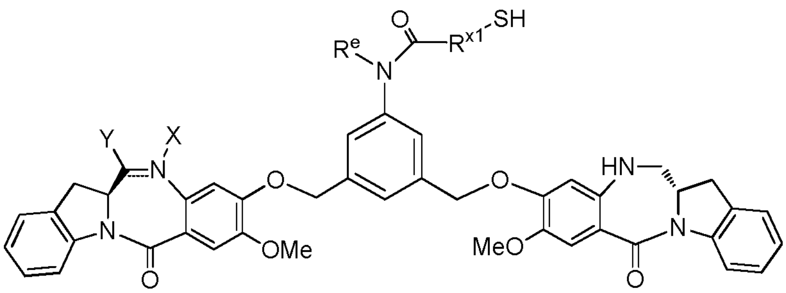
(L2a');

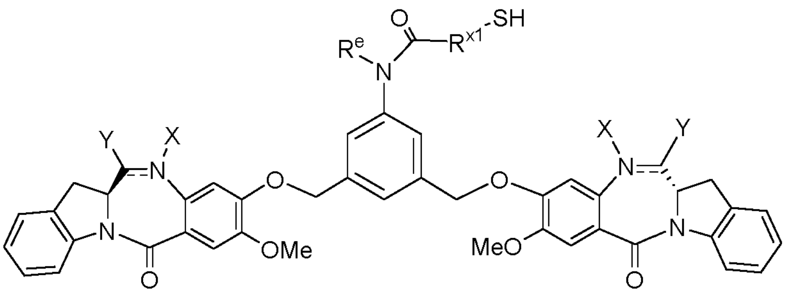
(L2a'1);

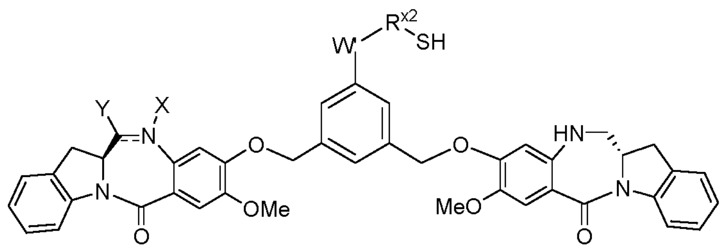
(L2b');

or

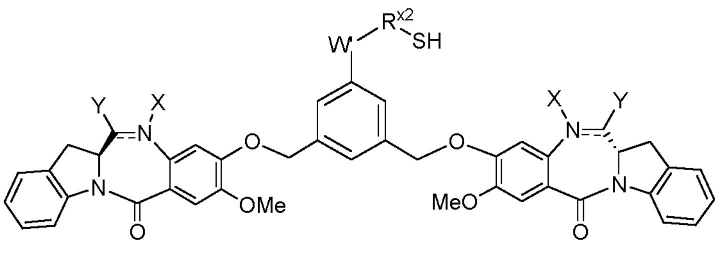

(L2b'1),

or a pharmaceutically acceptable salt thereof, wherein the variables are as described above for formulas (L1a'), (L1a'1), (L1b'), (L1b'1), (L2a'), (L2a'1), (L2b') and (L2b'1).

In certain embodiments, for the second, third or fourth methods described above, the linker compound having an amine reactive group and a thiol reactive group is represented by any one of the formula (a1L) - (a10L) and the cytotoxic agent is represented by the following formula:

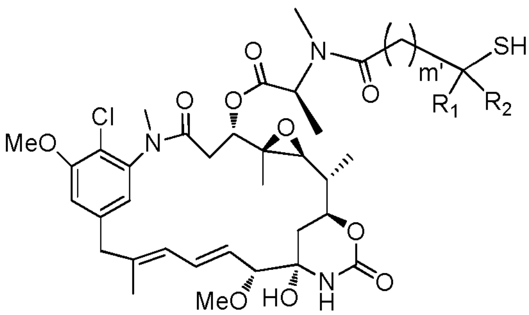

wherein the variables are as described above in any one of the 13th to 17th specific embodiments of the first embodiment described above and any more specific embodiments described therein.

In a specific embodiment, for the second, third or fourth methods described above, the linker is sulfo-SPDB, the cytotoxic agent is DM4 and the immunoconjugate is represented by the following formula:

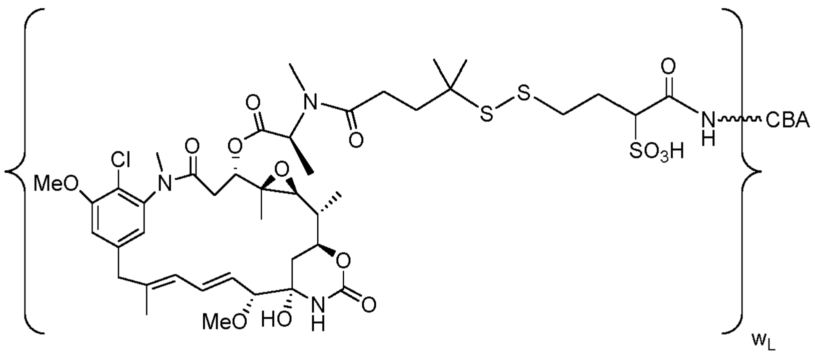

,

or a pharmaceutically acceptable salt thereof, wherein $W_L$ is an integer from 1 to 10.

The immunoconjugates comprising an anti-ADAM9 antibody or an ADAM9-binding fragment thereof covalently linked to a cytotoxic agent through the thiol group (-SH) of one or more cysteine residues located on the anti-ADAM9 antibody or an ADAM9-binding fragment thereof as described in the second embodiment above (e.g., immunoconjugates of any one of the 1st to 23rd specific embodiments or any more specific embodiments described therein) can be prepared by reacting the CBA having one or more free cysteine with a cytotoxic agent having a thiol-reactive group described herein.

In a preferred embodiment, the immunoconjugates of the present invention possesses a variant IgG Fc Region comprising an additional cysteine residue, so as to permit the conjugation of a pharmacological agent to such molecule (*e.g.,* **SEQ ID NOs: 79** and **80**).

In one embodiment, the cytotoxic agent having a thiol-reactive group is represented by the following formula:

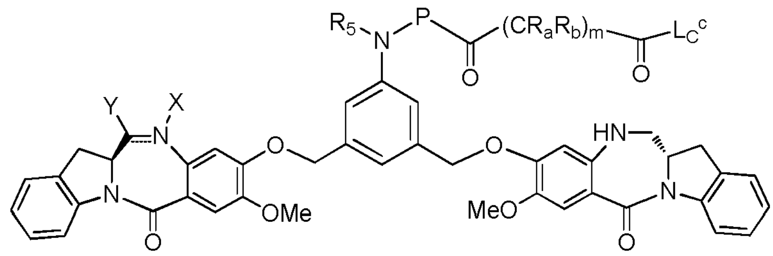

(C1a');

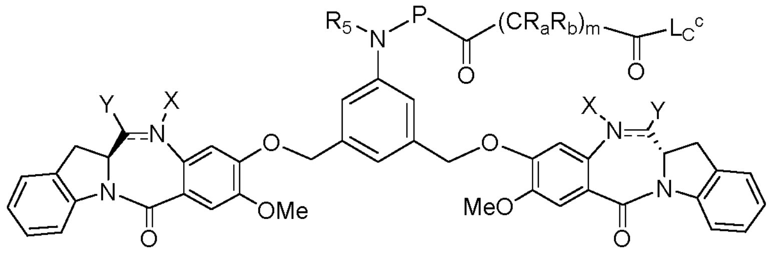

(C1a'1)

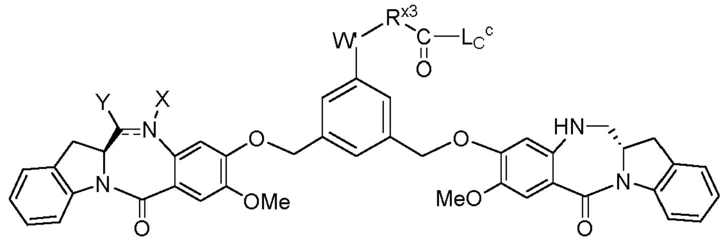

(C1b');

or

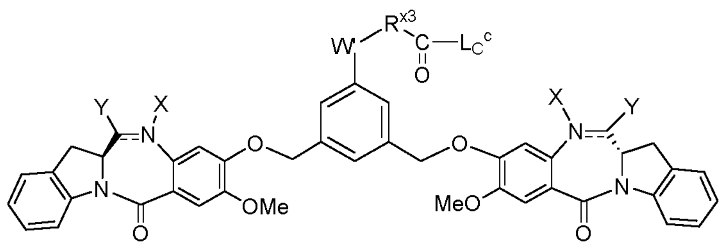

(C1b'1),

or a pharmaceutically acceptable salt thereof, wherein $—L_C^c$ is represented by the following formula:

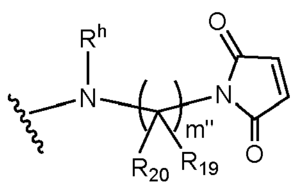

wherein the variables are as described above in any one of the 1st to 9th and 23rd specific embodiments of the second embodiment or any more specific embodiments described therein.

In another embodiment, the cytotoxic agent having a thiol-reactive group is represented by the following formula:

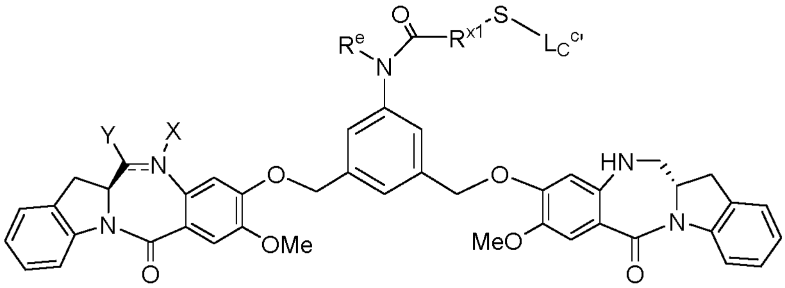

(C2a"),

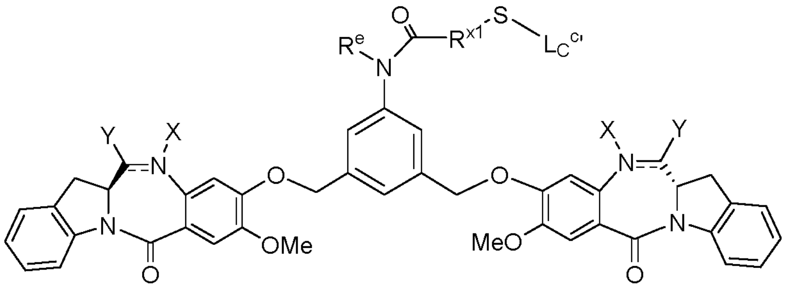

(C2a"1),

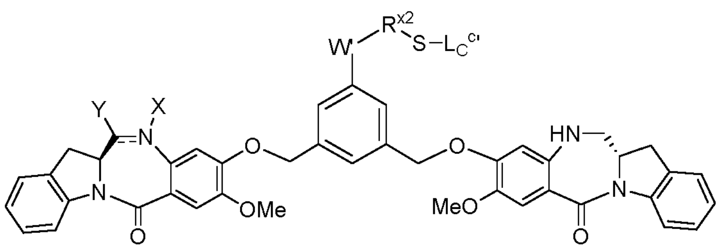

(C2b"),

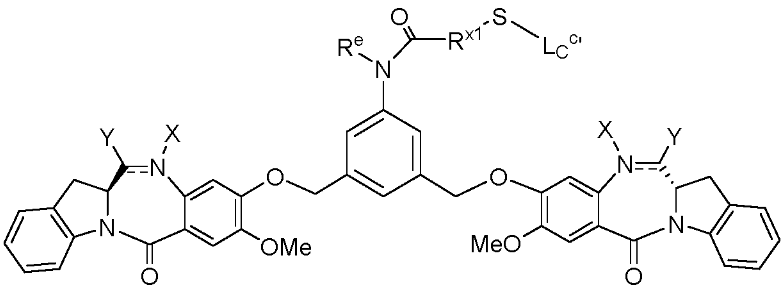

(C2b"1)

or a pharmaceutically acceptable salt thereof, wherein $L_C^{c'}$ is represented by the following formula:

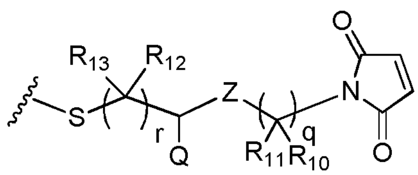

;

or

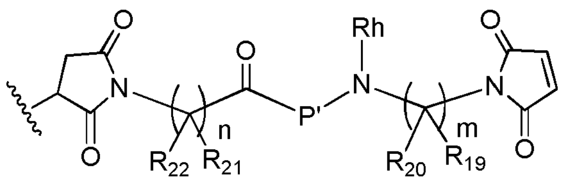

;

wherein the variables are as described above in any one of the 10th to 16th and 23rd specific embodiment of the second embodiment or any more specific embodiments described therein.

In yet another embodiment, the cytotoxic agent having a thiol-reactive group is represented by the following formula:

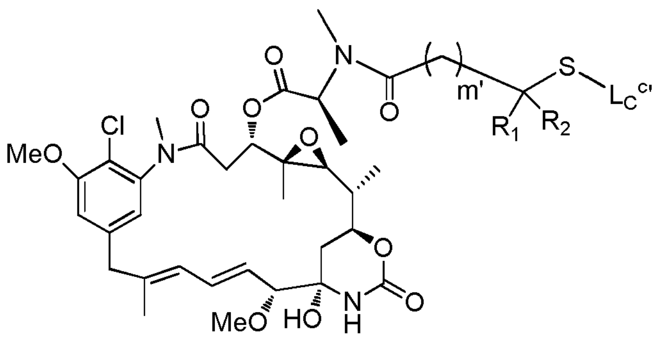

(C3a'),

or a pharmaceutically acceptable salt thereof, wherein $L_C^{c'}$ is described above and the remaining variables are as described above in any one of the 17th to 23rd specific embodiments of the second embodiment or any more specific embodiments described therein.

In a specific embodiment, the cytotoxic agent having a thiol-reactive group is represented by the following formula:

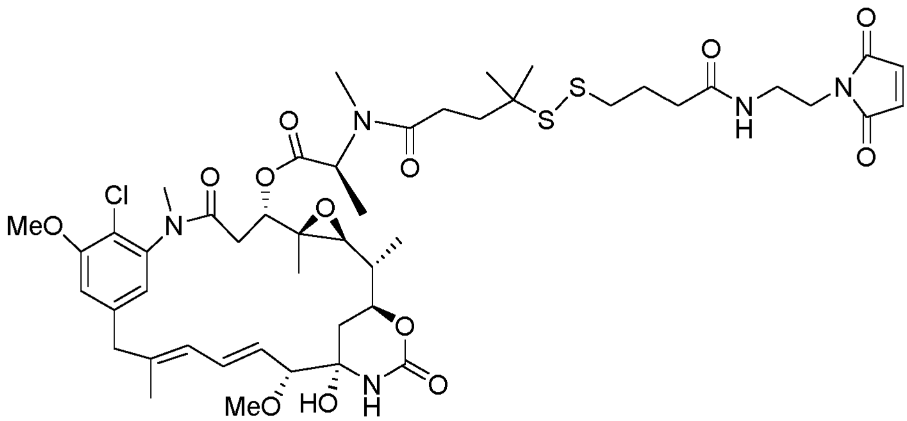

;

or a pharmaceutically acceptable salt thereof.

In another specific embodiment, the cytotoxic agent having a thiol-reactive group is represented by the following formula:

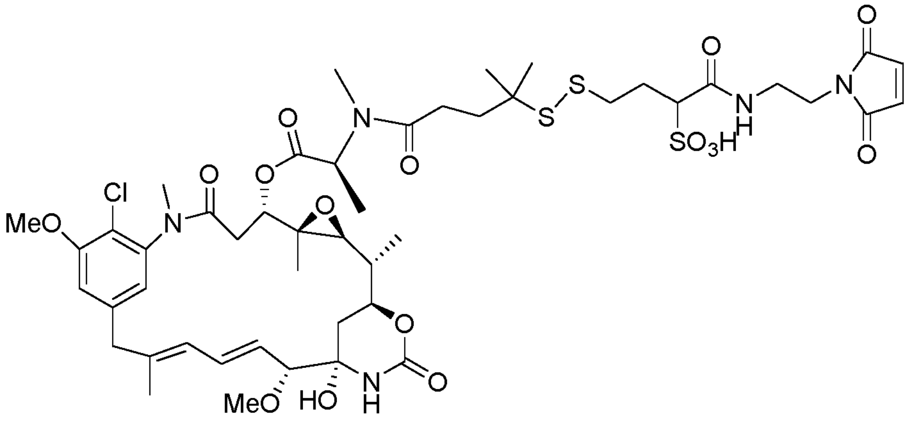

;

or a pharmaceutically acceptable salt thereof.

In certain embodiments, organic solvents are used in the reaction of the CBA and the cytotoxic agent to solubilize the cytotoxic agent. Exemplary organic solvents include dimethylacetamide (DMA), propylene glycol, *etc.* In one embodiment, the reaction of the CBA and the cytotoxic agent is carried out in the presence of DMA and propylene glycol.

In a specific embodiment, the cytotoxic agent represented by the following formula:

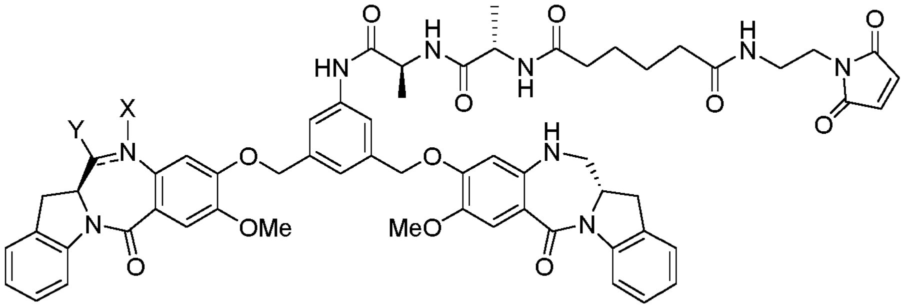

or a pharmaceutically acceptable salt thereof, is reacted with a CBA *(e.g.,* an anti-ADAM9 antibody or an ADAM9-binding fragment thereof) to form the immunoconjugate represented by the following formula:

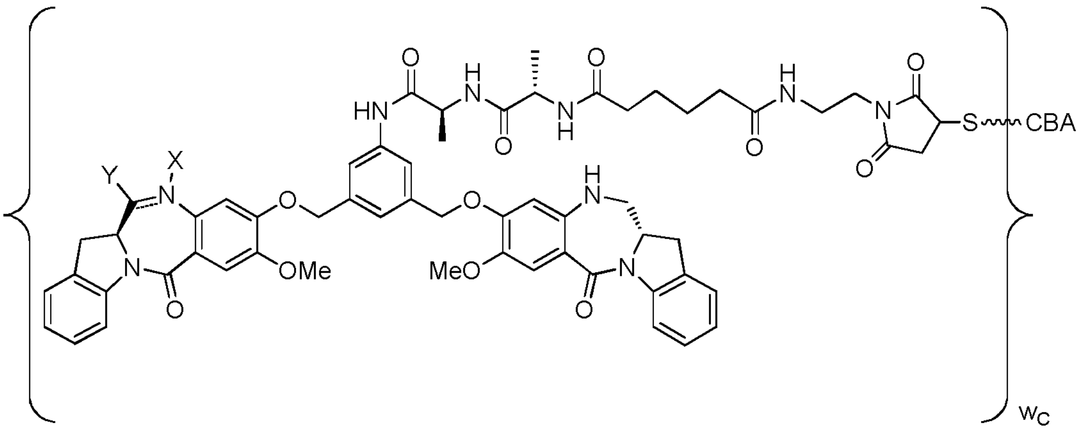

,

or a pharmaceutically acceptable salt thereof, wherein:

the double line

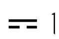

between N and C represents a single bond or a double bond, provided that when it is a double bond, X is absent and

Y is -H, and when it is a single bond, X is -H, and Y is $-SO_3H$ or a pharmaceutically acceptable salt thereof; and $W_C$ is 1 or 2. In a more specific embodiment, the double line



between N and C represents a double bond, X is absent and Y is -H. In another more specific embodiment, the double line



between N and C represents a single bond, X is -H and Y is $-SO_3H$ or a pharmaceutically acceptable salt thereof. Even more specifically, the pharmaceutically acceptable salt is a sodium or a potassium salt.

In certain embodiments, when Y is $—SO_3H$ or a pharmaceutically acceptable salt thereof, the immunoconjugates can be prepared by (a) reacting the imine-moiety in the imine-containing cytotoxic agent having a thiol-reactive group described above (*i.e.*, formula (C1a'), (C1a'1), (C1b'), (C1b'1), (C2a"), (C2a"1), (C2b") or (C2b"1), wherein the double line



between N and C represents a double bond, X is absent and Y is -H) with a sulfur dioxide, bisulfite salt or a metabisulfite salt in an aqueous solution at a pH of 1.9 to 5.0 to form a modified cytotoxic agent comprising a modified imine moiety represented by the following formula:

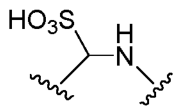

or a pharmaceutically acceptable salt thereof; and (b) reacting the modified cytotoxic agent with the anti-ADAM9 antibody or an ADAM9-binding fragment thereof described herein to form the immunoconjugate.

In a 1st aspect, for the method described above, the reaction of step (a) is carried out at a pH of 1.9 to 5.0. More specifically, the pH is 2.5 to 4.9, 1.9 to 4.8, 2.0 to 4.8, 2.5 to 4.5, 2.9 to 4.5, 2.9 to 4.0, 2.9 to 3.7, 3.1 to 3.5, or 3.2 to 3.4. In another specific embodiment, the reaction of step (a) is carried out at a pH of 1.9, 2.0, 2.1, 2.2, 2.3, 2.4, 2.5, 2.6, 2.7, 2.8, 2.9, 3.0, 3.1, 3.2, 3.3, 3.4, 3.5, 3.6, 3.7, 3.8, 3.9, 4.0, 4.1, 4.2, 4.3, 4.5, 4.6, 4.7, 4.8, 4.9 or 5.0. In yet another specific embodiment, the reaction of step (a) is carried out at a pH of 3.3.

As used herein, a specific pH value means the specific value $\pm$ 0.05.

In some embodiments, the reaction of step (a) is carried out in the presence of a buffer solution. Any suitable buffer solution known in the art can be used in the methods of the present invention. Suitable buffer solutions include, for example, a citrate buffer, an acetate buffer, a succinate buffer, a phosphate buffer, a glycine-containing buffer (e.g., glycine-HCl buffer), a phthalate buffer (e.g., a buffer solution comprising sodium or potassium hydrogen phthalate), and a combination thereof. In some embodiments, the buffer solution is a succinate buffer. In some embodiments, the buffer solution is a phosphate buffer. In some embodiments, the buffer is a citrate-phosphate buffer. In some embodiments, the buffer is a citrate-phosphate buffer comprising citric acid and $Na_2HPO_4$. In other embodiments, the buffer is a citrate-phosphate buffer comprising citric acid and $K_2HPO_4$. In some embodiments, the concentration of the buffer solution described above can be in the range of 10 to 250 mM, 10 to 200 mM, 10 to 150 mM, 10 to 100 mM, 25 to 100 mM, 25 to 75 mM, 10 to 50 mM, or 20 to 50 mM.

In a 2nd aspect, the reaction step (a) is carried out in the absence of a buffer solution (e.g., the buffers described in the 1st aspect). In some embodiments, the present method comprises the steps of: (a) reacting the imine-moiety in the imine-containing cytotoxic agent having a thiol-reactive group described above (*i.e.,* formula (C1a'), (C1a'1), (C1b'), (C1b'1), (C2a"), (C2a"1), (C2b") or (C2b"1), wherein the double line



between N and C represents a double bond, X is absent and Y is -H) with sulfur dioxide, a bisulfite salt or a metabisulfite salt in an aqueous solution to form a modified cytotoxic agent comprising a modified imine moiety represented by the following formula:

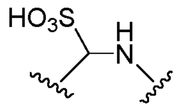

or a pharmaceutically acceptable salt thereof, wherein the aqueous solution does not comprise a buffer; and (b) reacting the modified cytotoxic agent with the anti-ADAM9 antibody or an ADAM9-binding fragment thereof described herein to form the immunoconjugate. In some embodiments, the reaction of step (a) is carried out in a mixture of an organic solvent and water. More specifically, the reaction of step (a) is carried out in a mixture of dimethyacetamide (DMA) and water. In some embodiments, the mixture of DMA and water comprises less than 60% of DMA by volume. Even more specifically, the volume ratio of DMA and water is 1:1.

In a 3rd aspect, for the methods described above or in the 1st or 2nd aspect, 0.5 to 5.0 equivalents of the bisulfite salt or 0.25 or 2.5 equivalents of the metabisulfite salt is used for every 1 equivalent of the imine-containing cytotoxic agent in the reaction of step (a). In some embodiments, 0.5 to 4.5, 0.5 to 4.0, 0.5 to 3.5, 0.5 to 4.0, 0.5 to 3.5, 0.5 to 3.0, 0.5 to 2.5, 0.8 to 2.0, 0.9 to 1.8, 1.0 to 1.7, 1.1 to 1.6, or 1.2 to 1.5 equivalents of the bisulfite salt or 0.25 to 2.25, 0.25 to 2.0, 0.25 to 1.75, 0.25 to 2.0, 0.25 to 1.75, 0.25 to 1.5, 0.25 to 1.25, 0.4 to 1.0, 0.45 to 0.9, 0.5 to 0.85, 0.55 to 0.8, or 0.6 to 0.75 equivalents of the metabisulfite salt is used for every 1 equivalent of the imine-containing cytotoxic agent. In other embodiments, 0.5, 0.6, 0.7, 0.8, 0.9, 1.0, 1.1, 1.2, 1.3 1.4, 1.5, 1.6, 1.7, 1.8, 1.9, 2.0, 2.1, 2.2, 2.3, 2.4, 2.5, 2.6, 2.7, 2.8, 2.9, 3.0, 3.1, 3.2, 3.3, 3.4, 3.5, 4.0, 4.5 or 5.0 equivalents of the bisulfite salt or 0.25, 0.3, 0.35, 0.4, 0.45, 0.5, 0.55, 0.6, 0.65 0.7, 0.75, 0.8, 0.85, 0.9, 0.95, 1.0, 1.05, 1.1, 1.15, 1.2, 1.25, 1.3, 1.35, 1.4, 1.45, 1.5, 1.55, 1.6, 1.65, 1.7, 1.75, 2.0, 2.25 or 2.5 equivalents of the metabisulfite salt is used for every 1 equivalent of the imine-containing cytotoxic agent. In yet other embodiments, 1.4 equivalents of the bisulfite salt or 0.7 equivalent of the metabisulfite salt is used for every 1 equivalent of the imine-containing cytotoxic agent. In other embodiments, 1.2 equivalents of the bisulfite salt or 0.6 equivalent of the metabisulfite salt is used for every 1 equivalent of the imine-containing cytotoxic agent.

As used herein, a specific equivalent means the specific value ± 0.05.

In a 4th aspect, for methods described above, the reaction of step (a) is carried out at a pH of 2.9 to 3.7 and 1.0 to 1.8 equivalents of the bisulfite salt or 0.5 to 0.9 equivalents of the metabisulfite salt is reacted with 1 equivalent of the imine-containing cytotoxic agent. In some embodiments, the reaction of step (a) is carried out at a pH of 3.1 to 3.5 and 1.1 to 1.6 equivalents of the bisulfite salt or 0.55 to 0.8 equivalents of the metabisulfite salt is reacted with 1 equivalent of the imine-containing cytotoxic agent. In other embodiments, the reaction of step (a) is carried out at a pH of 3.2 to 3.4 and 1.3 to 1.5 equivalents of the bisulfite salt or 0.65 to 0.75 equivalents of the metabisulfite is reacted with 1 equivalent of the imine-containing cytotoxic agent. In other embodiments, the reaction of step (a) is carried out at a pH of 3.3 and 1.4 equivalents of the bisulfite salt or 0.7 equivalent of the metabisulfite salt is reacted with 1 equivalent of the imine-containing cytotoxic agent. In yet other embodiments, the reaction of step (a) is carried out at a pH of 3.3 and 1.4 equivalents of sodium bisulfite is reacted with 1 equivalent of the imine-containing cytotoxic agent.

In a 5th aspect, for the methods described above or in the 1st, 2nd, 3rd or 4th aspect, the reaction of step (a) is carried out in a mixture of an organic solvent and water. Any suitable organic solvent can be used. Exemplary organic solvents include alcohols (e.g., methanol, ethanol, propanol, etc.), dimethylformamide (DMF), dimethylsulfoxide (DMSO), acetonitrile, acetone, methylene chloride, etc. In some embodiments, the organic solvent is miscible with water. In other embodiments, the organic solvent is not miscible with water, i.e., the reaction of step (a) is carried out in a biphasic solution. In some embodiments, the organic solvent is dimethylacetamide (DMA). The organic solvent (e.g., DMA) can be present in the amount of 1%-99%, 1-95%, 10-80%, 20-70%, 30-70%, 1-60%, 5-60%, 10-60%, 20-60%, 30-60%, 40-60%, 45-55%, 10-50%, or 20-40%, by volume of the total volume of water and the organic solvent. In some embodiments, the reaction of step (a) is carried out in a mixture of DMA and water, wherein the volume ratio of DMA and water is 1:1.

In a 6th aspect, for the methods described above or in the 1st, 2nd, 3rd, 4th or 5th aspect, the reaction of step (a) can be carried out at any suitable temperature. In some embodiments, the reaction is carried out at a temperature from 0 °C to 50 °C, from 10 °C to 50 °C, from 10 °C to 40 °C, or from 10 °C to 30 °C. In other embodiments, the reaction is carried out at a temperature from 15 °C to 30 °C, from 20 °C to 30 °C, from 15 °C to 25 °C, from 16 °C to 24 °C, from 17 °C to 23 °C, from 18 °C to 22 °C or from 19 °C to 21 °C. In yet other embodiments, the reaction can be carried out at 15 °C, 16 °C, 17 °C, 18 °C, 19 °C, 20 °C, 21 °C, 22 °C, 23 °C, 24 °C or 25 °C. In some embodiments, the reaction can be carried out from 0 °C to 15 °C, from 0 °C to 10 °C, from 1°C to 10 °C, 5 °C to 15 °C, or from 5 °C to 10 °C.

In a 7th aspect, for the methods described above or in the 1st, 2nd, 3rd, 4th, 5th or 6th aspect, the reaction of step (a) is carried out for 1 minute to 48 hours, 5 minutes to 36 hours, 10 minutes to 24 hours, 30 minutes to 24 hours, 30 minutes to 20 hours, 1 hour to 20 hours, 1 hour to 15 hours, 1 hour to 10 hours, 2 hours to 10 hours, 3 hours to 9 hours, 3 hours to 8 hours, 4 hours to 6 hours, or 1 hour to 4 hours. In some embodiments, the reaction is allowed to proceed for 4 to 6 hours. In other embodiments, the reaction is allowed to proceed for 10 minutes, 15 minutes, 20 minutes, 30 minutes, 1 hours, 2 hours, 3 hours, 4 hours, 5 hours, 6 hours, 7 hours, 8 hours, 9 hours, 10 hours, 11 hours, 12 hours,

13 hours, 14 hours, 15 hours, etc. In other embodiments, the reaction is allowed to proceed for 4 hours. In yet other embodiments, the reaction is allowed to proceed for 2 hours.

In a $8^{th}$ aspect, for the methods of the present invention described herein or in the $1^{st}$, $2^{nd}$, $3^{rd}$, $4^{th}$, $5^{th}$, $6^{th}$ or $7^{th}$ aspect, the reaction of step (b) is carried out at a pH of 4 to 9. In some embodiments, the reaction of step (b) is carried out at a pH of 4.5 to 8.5, 5 to 8.5, 5 to 8, 5 to 7.5, 5 to 7, 5 to 6.5, or 5.5 to 6.5. In other embodiments, the reaction of step (b) is carried out at pH 5.0, 5.1, 5.2, 5.3, 5.4, 5.5, 5.6, 5.7, 5.8, 5.9, 6.0, 6.1, 6.2, 6.3, 6.4, 6.5, 6.6, 6.7, 6.8, 6.9, 7.0, 7.1, 7.2, 7.3, 7.4, 7.5, 7.6, 7.7, 7.8, 7.9, or 8.0.

In some embodiments, for the methods described above or in the $1^{st}$, $2^{nd}$, $3^{rd}$, $4^{th}$, $5^{th}$, $6^{th}$, $7^{th}$ or $8^{th}$ aspect, the reaction of step (b) is carried out in an aqueous solution comprising a mixture of water and an organic solvent. Any suitable organic solvent described above can be used. More specifically, the organic solvent is DMA. In some embodiments, the aqueous solution comprises less than 50%, less than 40%, less than 30%, less than 25%, less than 20%, less than 15%, less than 10%, less than 5%, less than 3%, less than 2%, or less than 1% of the organic solvent (e.g. DMA) by volume.

In some embodiments, for the methods described herein or in the $1^{st}$, $2^{nd}$, $3^{rd}$, $4^{th}$, $5^{th}$, $6^{th}$, $7^{th}$ or $8^{th}$ aspect, the bisulfite salt is sodium or potassium bisulfite and the metabisulfite salt is sodium or potassium metabisulfite. In a specific embodiment, the bisulfite salt is sodium bisulfite and the metabisulfite salt is sodium metabisulfite.

In some embodiments, for the methods described herein or in the $1^{st}$, $2^{nd}$, $3^{rd}$, $4^{th}$, $5^{th}$, $6^{th}$, $7^{th}$ or $8^{th}$ aspect, the modified cytotoxic agent is not purified before reacting with the cell-binding agent in step (b). Alternatively, the modified cytotoxic agent is purified before reacting with the cell-binding agent in step (b). Any suitable methods described herein can be used to purify the modified cytotoxic agent.

In some embodiments, for the methods described above, the reaction of step (a) results in no substantial sulfonation of the maleimide group. In some embodiments, less than 50%, 40%, 30%, 20%, 10%, 9%, 8%, 7%, 6%, 5%, 4%, 3%, 2%, or 1% of the maleimide group is sulfonated. The percentage of maleimide sulfonation is equal to the total amount of the maleimide-sulfonated cytotoxic agent (the cytotoxic agent having sulfonation on the maleimide only) and the di-sulfonated cytotoxic agent (the cytotoxic agent having sulfonation on both the maleimide and the imine moieties) divided by the starting amount of the imine-containing cytotoxic agent before its reaction with the bisulfite salt or the metabisulfite salt.

In some embodiments, the immunoconjugates prepared by any methods described above is subject to a purification step. In this regard, the immunoconjugate can be purified from the other components of the mixture using tangential flow filtration (TFF), non-adsorptive chromatography, adsorptive chromatography, adsorptive filtration, selective precipitation, or any other suitable purification process, as well as combinations thereof.

In some embodiments, the immunoconjugate is purified using a single purification step (e.g., TFF). Preferably, the conjugate is purified and exchanged into the appropriate formulation using a single purification step (e.g., TFF). In other embodiments of the invention, the immunoconjugate is purified using two sequential purification steps. For example, the immunoconjugate can be first purified by selective precipitation, adsorptive filtration, absorptive chromatography or non-absorptive chromatography, followed by purification with TFF. One of ordinary skill in the art will appreciate that purification of the immunoconjugate enables the isolation of a stable conjugate comprising the cell-binding agent chemically coupled to the cytotoxic agent.

Any suitable TFF systems may be utilized for purification, including a Pellicon type system (Millipore, Billerica, Mass.), a Sartocon Cassette system (Sartorius AG, Edgewood, N.Y.), and a Centrasette type system (Pall Corp., East Hills, N.Y.)

Any suitable adsorptive chromatography resin may be utilized for purification. Preferred adsorptive chromatography resins include hydroxyapatite chromatography, hydrophobic charge induction chromatography (HCIC), hydrophobic interaction chromatography (HIC), ion exchange chromatography, mixed mode ion exchange chromatography, immobilized metal affinity chromatography (IMAC), dye ligand chromatography, affinity chromatography, reversed phase chromatography, and combinations thereof. Examples of suitable hydroxyapatite resins include ceramic hydroxyapatite (CHT Type I and Type II, Bio-Rad Laboratories, Hercules, Calif.), HA Ultrogel hydroxyapatite (Pall Corp., East Hills, N.Y.), and ceramic fluoroapatite (CFT Type I and Type II, Bio-Rad Laboratories, Hercules, Calif.). An example of a suitable HCIC resin is MEP Hypercel resin (Pall Corp., East Hills, N.Y.). Examples of suitable HIC resins include Butyl-Sepharose, Hexyl-Sepharose, Phenyl-Sepharose, and Octyl Sepharose resins (all from GE Healthcare, Piscataway, N.J.), as well as Macro-prep Methyl and Macro-Prep t-Butyl resins (Biorad Laboratories, Hercules, Calif.). Examples of suitable ion exchange resins include SP-Sepharose, CM-Sepharose, and Q-Sepharose resins (all from GE Healthcare, Piscataway, N.J.), and Unosphere S resin (Bio-Rad Laboratories, Hercules, Calif.). Examples of suitable mixed mode ion exchangers include Bakerbond ABx resin (JT Baker, Phillipsburg N.J.) Examples of suitable IMAC resins include Chelating Sepharose resin (GE Healthcare, Piscataway, N.J.) and Profinity IMAC resin (Bio-Rad Laboratories, Hercules, Calif.). Examples of suitable dye ligand resins include Blue Sepharose resin (GE Healthcare, Piscataway, N.J.) and Affigel Blue resin (Bio-Rad Laboratories, Hercules, Calif.). Examples of suitable affinity resins include Protein A Sepharose resin (e.g., MabSelect, GE Healthcare, Piscataway, N.J.), where the cell-binding agent is an antibody, and lectin affinity

resins, e.g. Lentil Lectin Sepharose resin (GE Healthcare, Piscataway, N.J.), where the cell-binding agent bears appropriate lectin binding sites. Alternatively an antibody specific to the cell-binding agent may be used. Such an antibody can be immobilized to, for instance, Sepharose 4 Fast Flow resin (GE Healthcare, Piscataway, N.J.). Examples of suitable reversed phase resins include C4, C8, and C18 resins (Grace Vydac, Hesperia, Calif.).

Any suitable non-adsorptive chromatography resin may be utilized for purification. Examples of suitable non-adsorptive chromatography resins include SEPHADEXTM G-25, G-50, G-100, SEPHACRYLTM resins (e.g., S-200 and S-300), SUPERDEXTM resins (e.g., SUPERDEXTM 75 and SUPERDEXTM 200), BIO-GEL® resins (e.g., P-6, P-10, P-30, P-60, and P-100), and others known to those of ordinary skill in the art.

## VI. Uses of the Immunoconjugates of the Present Invention

The present invention encompasses pharmaceutical compositions comprising the immunoconjugates of the present invention.

As provided herein, the immunoconjugates of the present invention, comprising the humanized/optimized anti-ADAM9-VL and/or VH Domains provided herein, have the ability to bind ADAM9 present on the surface of a cell and mediate cell killing. In particular, the immunoconjugates of the present invention comprising a pharmacological agent, are internalized and mediate cell killing via the activity of the pharmacological agent. Such cell killing activity may be augmented by the immunoconjugate inducing antibody-dependent cell-mediated cytotoxicity (ADCC) and/or complement dependent cytotoxicity (CDC)

Thus, immunoconjugates of the present invention, comprising the humanized/optimized anti-ADAM9-VL and/or VH Domains provided herein, have the ability to treat any disease or condition associated with or characterized by the expression of ADAM9. As discussed above, ADAM9 is an onco-embryonic antigen expressed in numerous blood and solid malignancies that is associated with high-grade tumors exhibiting a less-differentiated morphology, and is correlated with poor clinical outcomes. Thus, without limitation, the immunoconjugates of the present invention may be employed in the treatment of cancer, particularly a cancer characterized by the expression of ADAM9.

In other particular embodiments, immunoconjugates of the present invention may be useful in the treatment of lung cancer (e.g., non-small-cell lung cancer), colorectal cancer, bladder cancer, gastric cancer, pancreatic cancer, renal cell carcinoma, prostate cancer, esophageal cancer, breast cancer, head and neck cancer, uterine cancer, ovarian cancer, liver cancer, cervical cancer, thyroid cancer, testicular cancer, myeloid cancer, melanoma, and lymphoid cancer.

In further embodiments, immunoconjugates of the present invention may be useful in the treatment of non-small-cell lung cancer (squamous cell, adenocarcinoma, or large-cell undifferentiated carcinoma) and colorectal cancer (adenocarcinoma, gastrointestinal carcinoid tumors, gastrointestinal stromal tumors, primary colorectal lymphoma, leiomyosarcoma, or squamous cell carcinoma).

In addition to their utility in therapy, the immunoconjugates of the present invention may be detectably labeled and used in the diagnosis of cancer or in the imaging of tumors and tumor cells.

## VII. Pharmaceutical Compositions

The compositions of the invention include bulk drug compositions useful in the manufacture of pharmaceutical compositions (e.g., impure or non-sterile compositions) and pharmaceutical compositions (*i.e.,* compositions that are suitable for administration to a subject or patient) that can be used in the preparation of unit dosage forms. Such compositions comprise a prophylactically or therapeutically effective amount of the immunoconjugates of the present invention, or a combination of such agents and a pharmaceutically acceptable carrier. Preferably, compositions of the invention comprise a prophylactically or therapeutically effective amount of immunoconjugates of the present invention and a pharmaceutically acceptable carrier. The invention also encompasses such pharmaceutical compositions that additionally include a second therapeutic antibody (e.g., tumor-specific monoclonal antibody) that is specific for a particular cancer antigen, and a pharmaceutically acceptable carrier.

In a specific embodiment, the term **"pharmaceutically acceptable"** means approved by a regulatory agency of the Federal or a state government or listed in the U.S. Pharmacopeia or other generally recognized pharmacopeia for use in animals, and more particularly in humans. The term **"carrier"** refers to a diluent, adjuvant (*e.g.*, Freund's adjuvant (complete and incomplete), excipient, or vehicle with which the therapeutic is administered. Generally, the ingredients of compositions of the invention are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

The invention also provides a pharmaceutical pack or kit comprising one or more containers filled with an

immunoconjugates of the present invention, alone or with such pharmaceutically acceptable carrier. Additionally, one or more other prophylactic or therapeutic agents useful for the treatment of a disease can also be included in the pharmaceutical pack or kit. The invention also provides a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects approval by the agency of manufacture, use or sale for human administration.

The present invention provides kits that can be used in the above methods. A kit can comprise any of the immunoconjugates of the present invention. The kit can further comprise one or more other prophylactic and/or therapeutic agents useful for the treatment of cancer, in one or more containers.

### VIII. Methods of Administration

The compositions of the present invention may be provided for use in the treatment, prophylaxis, and amelioration of one or more symptoms associated with a disease, disorder by administering to a subject an effective amount an immunoconjugate of the invention. In a preferred aspect, such compositions are substantially purified (*i.e.,* substantially free from substances that limit its effect or produce undesired side effects). In a specific embodiment, the subject is an animal, preferably a mammal such as non-primate (*e.g.,* bovine, equine, feline, canine, rodent, *etc.*) or a primate (*e.g.,* monkey such as, a cynomolgus monkey, human, *etc.*). In a preferred embodiment, the subject is a human.

Various delivery systems are known and can be used to administer the compositions, *e.g.*, encapsulation in liposomes, microparticles, microcapsules, recombinant cells capable of expressing the antibody or fusion protein, receptor-mediated endocytosis (See, *e.g.,* Wu et al. (1987) "Receptor-Mediated In Vitro Gene Transformation By A Soluble DNA Carrier System, " J. Biol. Chem. 262:4429-4432), construction of a nucleic acid as part of a retroviral or other vector, *etc.*

Methods of administering the immunoconjugates for use according to the invention include parenteral administration (*e.g.*, intradermal, intramuscular, intraperitoneal, intravenous and subcutaneous), epidural, and mucosal (*e.g.*, intranasal and oral routes). In a specific embodiment, the immunoconjugates for use according to the present invention are administered intramuscularly, intravenously, or subcutaneously. The compositions may be administered by any convenient route, for example, by infusion or bolus injection, and may be administered together with other biologically active agents. Administration can be systemic or local.

The invention also provides that preparations of the immunoconjugates of the present invention are packaged in a hermetically sealed container such as an ampoule or sachette indicating the quantity of the molecule. In one embodiment, such molecules are supplied as a dry sterilized lyophilized powder or water free concentrate in a hermetically sealed container and can be reconstituted, *e.g.*, with water or saline to the appropriate concentration for administration to a subject. Preferably, the immunoconjugates of the present invention are supplied as a dry sterile lyophilized powder in a hermetically sealed container.

The lyophilized preparations of the immunoconjugates of the present invention should be stored at between 2°C and 8°C in their original container and the molecules should be administered within 12 hours, preferably within 6 hours, within 5 hours, within 3 hours, or within 1 hour after being reconstituted. In an alternative embodiment, such molecules are supplied in liquid form in a hermetically sealed container indicating the quantity and concentration of the molecule, fusion protein, or conjugated molecule. Preferably, such immunoconjugates when provided in liquid form are supplied in a hermetically sealed container.

As used herein, an **"effective amount"** of a pharmaceutical composition is an amount sufficient to effect beneficial or desired results including clinical results such as decreasing symptoms resulting from the disease, attenuating a symptom of infection (e.g., viral load, fever, pain, sepsis, etc.) or a symptom of cancer (e.g., the proliferation, of cancer cells, tumor presence, tumor metastases, *etc.*), thereby increasing the quality of life of those suffering from the disease, decreasing the dose of other medications required to treat the disease, enhancing the effect of another medication such as via targeting and/or internalization, delaying the progression of the disease, and/ or prolonging survival of individuals.

An effective amount can be administered in one or more administrations. For purposes of this invention, an effective amount of drug, compound, or pharmaceutical composition is an amount sufficient: to kill and/or reduce the proliferation of cancer cells, and/or to eliminate, reduce and/or delay the development of metastasis from a primary site of cancer. In some embodiments, an effective amount of a drug, compound, or pharmaceutical composition may or may not be achieved in conjunction with another drug, compound, or pharmaceutical composition. Thus, an "effective amount" may be considered in the context of administering one or more chemotherapeutic agents, and a single agent may be considered to be given in an effective amount if, in conjunction with one or more other agents, a desirable result may be or is achieved.

For the immunoconjugates for use according to the invention, the dosage administered to a patient is preferably determined based upon the body weight (kg) of the recipient subject.

The dosage and frequency of administration of an immunoconjugate for use according to the present invention may be reduced or altered by enhancing uptake and tissue penetration of the molecule by modifications such as, for example, lipidation.

The dosage of an immunoconjugate for use according to the invention administered to a patient may be calculated for use as a single agent therapy. Alternatively, the molecule may be used in combination with other therapeutic compositions and the dosage administered to a patient are lower than when said molecules are used as a single agent therapy.

The pharmaceutical compositions for use according to the invention may be administered locally to the area in need of treatment; this may be achieved by, for example, local infusion, by injection, or by means of an implant, said implant being of a porous, nonporous, or gelatinous material, including membranes, such as sialastic membranes, or fibers. Preferably, when administering an immunoconjugate for use according to the invention, care must be taken to use materials to which the molecule does not absorb.

The compositions for use according to the invention can be delivered in a vesicle, in particular a liposome (*See* Langer (1990) "New Methods Of Drug Delivery," Science 249: 1527-1533); Treat et al., in LIPOSOMES IN THE THERAPY OF INFECTIOUS DISEASE AND CANCER, Lopez-Berestein and Fidler (eds.), Liss, New York, pp. 353- 365 (1989); Lopez-Berestein, ibid., pp. 3 17-327).

Treatment of a subject with a therapeutically or prophylactically effective amount of an immunoconjugate of the present invention can include a single treatment or, preferably, can include a series of treatments. It will also be appreciated that the effective dosage of the molecules used for treatment may increase or decrease over the course of a particular treatment.

**EXAMPLES**

Having now generally described the invention, the same will be more readily understood through reference to the following Examples. The following examples illustrate various methods for compositions in the diagnostic or treatment methods of the invention. The examples are intended to illustrate, but in no way limit, the scope of the invention.

**Example 1**

**Tumor Cell Specificity of the Anti-ADAM9 Antibody MAB-A**

A murine anti-ADAM9 antibody (designated herein as MAB-A) was identified that: (1) blocks the target protein processing activity of ADAM9; (2) is internalized; and (3) has anti-tumor activity (see, *e.g.,* US Patent No. 8361475). The tumor cell specificity of MAB-A was investigated by IHC. Tumor tissue was contacted with MAB-A (0.4 μg/mL) or an isotype control (0.4 μg/mL) and the extent of staining was visualized. MAB-A was found to strongly label a variety of large cell carcinoma, squamous cell carcinoma, and adenocarcinoma non-small cell lung cancer cell types **(FIG. 1A),** breast cancer cells, prostate cancer cells, gastric cancer cells **(FIG. 1B)**, as well as colon cancer samples **(FIG. 1C).** Normal tissue was contacted with MAB-A (1.25 μg/mL) and the extent of staining was visualized. As summarized in **Table 2** above, MAB-A exhibited little or no staining of a wide variety of normal tissues. It will be noted that the concentration of MAB-A used in these studies was nearly 3-times that used for staining of tumor cells. The results of these IHC studies indicate that MAB-A exhibits strong preferential binding to tumor cells over normal cells.

**Example 2**

**Species Cross Reactivity**

The binding of MAB-A to human ADAM9 (huADAM9) and cynomolgus monkey ADAM9 (cynoADAM9) was examined. Briefly, 293-FT and CHO-K cells transiently expressing huADAM9, cynoADAM9, an unrelated antigen, or the untransfected parental cells were incubated with MAB-A followed by goat anti-murine-PE secondary antibody and analyzed by FACS. As shown in **FIG. 2,** MAB-A exhibits strong binding to huADAM9 transiently expressed on both cells types. MAB-A exhibits poor binding to cynoADAM9. MAB-A did not bind to the parental cells or cells expressing an irrelevant antigen. Similar low levels of binding to cynoADAM were seen in ELISA assays.

**Example 3**

**Humanization and Initial Optimization**

Humanization of MAB-A yielded a humanized VH Domain, designated herein as **"hMAB-A VH(1)"** and a

humanized VL Domain designated herein as "hMAB-A VL(1)." The humanized Variable Domains were then optimized to enhance binding activity and/or to remove potentially labile amino acid residues as described in more detail below. This first round of optimization yielded three additional humanized VH Domains, designated herein as **"hMAB-A VH(2)," "hMAB-A VH(3),"** and **"hMAB-A VH(4),"** and three additional humanized VL Domains designated herein as **"hMAB-A VL(2)," "hMAB-A VL(3),"** and **"hMAB-A VL(4)**." In addition, a chimeric version of MAB-A **("chMAB-A")** having the murine VH and VL Domains and human constant regions was generated. The amino acid sequences of the murine and the humanized/optimized VH and VL Domains are provided above, an alignment is provided in **FIGs. 3A** and **3B.** The consensus sequence of these humanized/optimized VH and VL Domains is provided above. Where multiple humanized Variable Domains were generated the humanized heavy and light chain Variable Domains of a particular anti-ADAM9 antibody (*e.g.*, MAB-A) may be used in any combination and particular combinations of humanized chains are referred to by reference to the specific VH/VL Domains, for example an antibody comprising hMAB-A VH(1) and hMAB-A VL(2) is specifically referred to as "hMAB-A (1.2)."

In the following examples, any immunoconjugate comprising the following VH Domains is shown for illustration purposes only and does not form part of the presently-claimed invention:

hMAB-A VH (1)

hMAB-A VH (2)

hMAB-A VH (3)

hMAB-A VH (4).

In the following examples, any immunoconjugate comprising the chimeric version of MAB-A (chMAB-A) is shown for illustration purposes only and does not form part of the presently-claimed invention.

hMAB-A VH(1) was generated having framework regions derived from human germlines VH3-21 and VH3-64, and hMAB-A VL(1) was generated having framework regions derived from human germlines B3 and L6. The murine CDRs were retained in these humanized variable domains.

A potential deamidation site was identified in the $CDR_H2$ (shown in single underlining in **FIG. 3A**) and a potential aspartic acid isomerization site was identified in $CDR_L1$ (shown in single underlining in **FIG. 3B**). Amino acid substitutions at these positions were examined to identify substitutions to remove these sites while maintaining binding affinity. A substitution of phenylalanine at position 54 (N54F) of $CDR_H2$ (present in hMAB-A VH(2)) and at serine at position 28 (D28S) of $CDR_L1$ (present in hMAB-A VL(2)) were selected, wherein the numbering is accordingly to Kabat. The identified substitutions may be used separately or in combination. Surprisingly, antibodies comprising the N54F substitution were found to exhibit about a 2-fold increase in affinity for human ADAM9 (see, *e.g.,* **Table 3,** below) and slightly improved binding to cynomolgus ADAM9.

Additional, optimized variants were generated to minimize the number of lysine residues present in the CDRs. Two lysine residues are present in $CDR_H2$ (indicated with a double underline in **FIG. 3A**), and one lysine is present in $CDR_L1$ (indicated with a double underline in **FIG. 3B**). Amino acid substitutions at these positions were examined to identify substitutions that maintained binding affinity. Substitutions of arginine at position 62 (K62R), of glutamine at position 64 (K64Q), and serine at position 65 (S65G) were selected for $CDR_H2$ (present in hMAB-A VH(3)), wherein the numbering is accordingly to Kabat. A substitution of an arginine at position 24 (K24R) was selected for $CDR_L1$ (present in hMAB-A VL(3)). The identified substitutions may be used separately or in combination.

Other potentially labile resides present in the CDRs were identified (indicated with a dotted underline in **FIGs. 3A-3B),** one methionine residue within $CDR_H1$ at position 34 (M34), one methionine residue within $CDR_L1$ at position 33 (M33), and histidine, glutamic acid, and aspartic acid residues at positions 92 (H93), 93 (E93), and 94 (D94), within $CDR_L3$, wherein the numbering is accordingly to Kabat. Amino acid substitutions at these positions were examined to identify substitutions that maintained binding affinity. Substitution of isoleucine at position 34 (M34I) was selected for $CDR_H1$ and substitutions of leucine, tyrosine, serine and threonine were selected for positions 33 (M33L), 92 (H93Y), 93 (E93S), and 94 (D94T) of $CDR_L3$, wherein the numbering is according to Kabat. Each of these positions could readily be substituted in combination with all of the substitutions detailed above to yield hMAB-A VH(4) and hMAB-A VL(4), which when paired together generate an antibody that retained a small improvement in affinity as compared to the parental murine antibody, and that has a greatly reduced potential for deamidation or oxidation and no lysine residues in the CDRs.

The relative binding affinity of the humanized/optimized antibodies hMAB-A (1.1), hMAB-A (2.2), hMAB-A (2.3), hMAB-A (3.3), hMAB-A (4.4) and the chimeric chMAB-A (having murine VH/VL Domains) to huADAM was investigated using BIACORE® analysis, in which His-tagged soluble human ADAM9 **("shADAM9-His,"** containing an extracellular portion of human ADAM9 fused to a histidine-containing protein) was passed over a surface coated with immobilized

antibody. Briefly, each antibody was captured on a Fab2 goat anti-human Fc surface and then incubated in the presence of different concentrations (6.25-100 nM) of the shADAM9-His protein. The kinetics of binding were determined via BIACORE® analysis binding (normalized 1:1 Langmuir binding model). The calculated $k_a$, $k_d$ and $K_D$ from these studies are presented in **Table 3.** Binding to cynoADAM9 was examined by FACS as described above and by ELISA.

**Table 3.**

| Antibody | pI | huADAM9 | | |
|---|---|---|---|---|
| | | $\mathbf{k_a}$ (x$10^6$) | $\mathbf{k_d}$ (x$10^{-3}$) | **KD** (nM) |
| chMAB-A | 6.61 | 1.3 | 4.7 | 3.6 |
| hMAB-A (1.1) | 6.44 | 1.5 | 5.2 | 3.5 |
| hMAB-A (2.2) | 6.58 | 1.1 | 1.5 | 1.4 |
| hMAB-A (2.3) | 6.58 | 1.3 | 1.7 | 1.3 |
| hMAB-A (3.3) | 6.44 | 1.1 | 1.5 | 1.4 |
| hMAB-A (4.4) | 6.73 | 1.0 | 2.0 | 2.0 |

The results of these studies demonstrate that the humanized/optimized antibodies have the same or higher binding affinity to human ADAM9 than the parental murine antibody. In particular, it was observed that the introduction of the N54F mutation in the humanized antibodies resulted in improved binding to huADAM9 (i.e., hMAB-A (2.2), hMAB-A (2.3), and hMAB-A (3.3)). This mutation also provided a slight improvement in binding to cynoADAM9 as determined by FACS and ELISA, however, these antibodies continued to exhibit poor binding to cynoADAM9. These studies also identified additional substitutions that could be introduced to remove lysine residues from the CDRs without reducing affinity. Additional substitutions were identified to remove other potentially labile residues with a minimal impact on affinity.

## Example 4

## Optimization of Binding to Non-Human Primate ADAM9

Random mutagenesis was used to introduce substitutions within the Heavy Chain $CDR_H2$ (Kabat positions 53-58) and $CDR_H3$ (Kabat positions 95-100 and 100a-100f) domains of hMAB-A (2.2). The mutants were screened to identify clones having enhanced binding to non-human primate ADAM9 (e.g., cynoADAM9) and that retained high affinity binding to huADAM9. 48 clones were selected from two independent screens of mutations within $CDR_H3$ (Kabat positions 100a-100f). **Table 4** provides an alignment of the amino acid sequence of $CDR_H3$ Kabat residues 100a-f from hMAB-A **(2.2)** clones selected for enhanced binding to cynoADAM9 from two independent screens. Additional clone alignments are provided in **Table 5.** As indicated in such Tables, similar clones emerged in each experiment, which fell into discrete substitution patterns.

**Table 4 Substitutions within Sub-Domain of the Heavy Chain CDRH3 of MAB-A (Kabat Positions 100a-100f)**

| Screen 1 | | | Screen 2 | | |
|---|---|---|---|---|---|
| **Clone ID** | **SEQ ID NO** | **$CDR_H3$ Sub-Domain Sequence** | **Clone ID** | **SEQ ID NO** | **$CDR_H3$ Sub-Domain Sequence** |
| MAB-A | 81 | GSRDYF | MAB-A | 81 | GSRDYF |
| 1 | 82 | DGEGVM | 1 | 112 | DGKAVL |
| 2 | 82 | DGEGVM | 2 | 113 | FNKAVL |
| 3 | 83 | FHSGLL | 3 | 84 | FNSATL |
| 4 | 84 | FNSATL | 4 | 114 | FNSGTW |
| 5 | 85 | FNSGTL | 5 | 115 | FNTGVF |
| 6 | 86 | FNSSTL | 6 | 116 | GKSRFH |
| 7 | 87 | GKSKWL | 7 | 91 | IGKGVF |

(continued)

| Table 4 Substitutions within Sub-Domain of the Heavy Chain CDRH3 of MAB-A (Kabat Positions 100a-100f) | | | | | |
|---|---|---|---|---|---|
| **Screen 1** | | | **Screen 2** | | |
| **Clone ID** | **SEQ ID NO** | **CDR$_H$3 Sub-Domain Sequence** | **Clone ID** | **SEQ ID NO** | **CDR$_H$3 Sub-Domain Sequence** |
| 8 | 88 | GMGGTL | 8 | 92 | IGKGVL |
| 9 | 89 | HAKGGM | 9 | 117 | IGKNVY |
| 10 | 90 | IGEAVL | 10 | 118 | MGKGVM |
| 11 | 91 | IGKGVF | 11 | 119 | NGESVF |
| 12 | 91 | IGKGVF | 12 | 120 | PDFGWM |
| 13 | 92 | IGKGVL | 13 | 121 | PGSGVM |
| 14 | 93 | KHDSVL | 14 | 122 | PKDAWL |
| 15 | 94 | LNTAVM | 15 | 99 | PKFGWK |
| 16 | 95 | NGEGTL | 16 | 99 | PKFGWK |
| 17 | 96 | NGKNTL | 17 | 123 | PKFGWL |
| 18 | 97 | NSAGIL | 18 | 124 | PKIGWH |
| 19 | 98 | PKEGWM | 19 | 124 | PKIGWH |
| 20 | 99 | PKFGWK | 20 | 124 | PKIGWH |
| 21 | 100 | PKMGWV | 21 | 125 | PKMGWA |
| 22 | 101 | PRLGHL | 22 | 126 | PKMGWM |
| 23 | 102 | PSFGWA | 23 | 126 | PKMGWM |
| 24 | 103 | QAKGTM | 24 | 126 | PKMGWM |
| 25 | 104 | RGMGVM | 25 | 126 | PKMGWM |
| 26 | 105 | RKEGWM | 26 | 127 | PQMGWL |
| 27 | 106 | TGKGVL | 27 | 128 | PRFGWL |
| 28 | 107 | TGMGTL | 28 | 128 | PRFGWL |
| 29 | 108 | TGNGVM | 29 | 128 | PRFGWL |
| 30 | 108 | TGNGVM | 30 | 129 | PRMGFL |
| 31 | 109 | WNAGTF | 31 | 130 | PRMGFM |
| 32 | 110 | YHHTPL | 32 | 131 | PSFGWM |
| 33 | 110 | YHHTPL | 33 | 132 | RREGWM |
| 34 | 111 | YQSATL | 34 | 133 | SGEGVL |
| | | | 35 | 134 | SGNGVM |
| | | | 36 | 135 | VGKAVL |

| Table 5 Substitutions within Sub-Domain of the Heavy Chain CDRH3 of MAB-A (Kabat Positions 100a-100f) | | |
|---|---|---|
| **Clone ID** | **SEQ ID NO** | **CDR$_H$3 Sub-Domain Sequence** |
| MAB-A **VH (2A)** | 85 | FNSGTL |
| MAB-A **VH (2B)** | 92 | IGKGVL |

(continued)

| Table 5 Substitutions within Sub-Domain of the Heavy Chain CDRH3 of MAB-A (Kabat Positions 100a-100f) | | |
|---|---|---|
| **Clone ID** | **SEQ ID NO** | **$CDR_H3$ Sub-Domain Sequence** |
| MAB-A **VH (2C)** | 128 | PRFGWL |
| MAB-A **VH (2D)** | 106 | TGKGVL |
| MAB-A **VH (2E)** | 136 | DSNAVL |
| MAB-A **VH (2F)** | 137 | FHSGTL |
| MAB-A **VH (2G)** | 113 | FNKAVL |
| MAB-A **VH (2H)** | 138 | GGSGVL |
| MAB-A **VH (2I)** | 139 | PRQGFL |
| MAB-A **VH (2J)** | 140 | YNSGTL |

For all the clones examined, Gly and Ala are the preferred amino acid residues at positon 4 (P4) and Leu, Met, and Phe are the preferred amino acid residues at position 6 (P6). The preferred amino acid residues at other positions (*e.g.*, position 2 (P2), position 3 (P3) and position 5 (P5)) depend on the amino acid residue found at P1. For clones having a Pro residue at position 1 (PI), Lys and Arg were preferred at P2, Phe and Met at P3, Gly at P4, and Trp or Phe at P5. For clones having a Phe, Tyr or Trp at P1, Asn and His were preferred at P2, Ser and His at P3, and Leu at P6. For clones having Ile, Leu or Val at P1, Gly was preferred at P2, Lys at P3, Val at P5 and hydrophobic at P6. In addition, as can be seen in **Table 4,** for clones having a Thr residue at P1, Gly was preferred at P2, Lys, Met, and Asn were preferred at P3, Gly was preferred at P4, Val or Thr were preferred at P5 and Leu and Met at P6. Additional clones having an Asp, Gly, Arg, His, or Ser residue at P1 were also identified at lower frequencies (see **Table 4** and **Table 5**).

The VH Domain of the ten clones shown in **Table 5** were used to generate further optimized variants of hMAB-A (2.2) designated hMAB-A (2A.2)-(2J.2). The binding of the selected clones was examined by ELISA assay. Briefly, antibodies that bind to histidine-containing peptides, and that had been coated onto microtiter plates, were used to capture His peptide-tagged soluble cynoADAM9 (**"cynoADAM9-His"**) (1 μg/mL) or His peptide-tagged soluble huADAM9 (1 μg/mL), and the binding of serial dilutions of the parental hMAB-A (2.2) and the ten $CDR_H3$ hMAB-A (2A.2) variants was examined. The binding curves cynoADAM9 and huADAM9 are presented in **FIG. 4A** and **FIG. 4B,** respectively. hMAB-A (2A.2) variants comprising each of the selected VH Domains exhibited improved binding to cynoADAM9 with MAB-A VH(2B), MAB-A VH(2C), MAB-A VH(2D), and MAB-A VH(2I), showing the greatest enhancement in cynoADAM9 binding while maintaining similar binding to huADAM9 as the parental hMAB-A (2.2) antibody.

The relative binding affinity of the humanized/further optimized antibodies MAB-A VH(2B.2), MAB-A VH(2C.2), MAB-A VH(2D.2), and MAB-A VH(2I.2), and the parental hMAB-A (2.2), to huADAM9-His and cynoADAM9-His was investigated using BIACORE® analysis essentially as described above. The calculated $k_a$, $k_d$ and $K_D$ from these studies are presented in **Table 6.**

| **Table 6** | | | | | | |
|---|---|---|---|---|---|---|
| Antibody | huADAM9 | | | cynoADAM9 | | |
| | $\mathbf{k_a}$ (x$10^5$ ) ($M^{-1}s^{-1}$) | $\mathbf{k_d}$ (x$10^{-4}$) ($s^{-1}$) | KD (nM) | $\mathbf{k_a}$ (x$10^5$) ($M^{-1}s^{-1}$) | $\mathbf{k_d}$ (x$10^{-4}$) ($s^{-1}$) | KD (nM) |
| hMAB-A (2.2) | 9.0 | 5.5 | 0.6 | 2.0 | 220 | 110 |
| hMAB-A (2B. 2) | 6.1 | 3.9 | 0.6 | 3.4 | 0.66 | 0.2 |
| hMAB-A (2C. 2) | 5.9 | 8.1 | 1.4 | 3.5 | <0.1 | <0.3 |
| hMAB-A (2D. 2) | 6.9 | 5.8 | 0.8 | 4.2 | 3.0 | 0.7 |
| hMAB-A (2I.2) | 6.6 | 2.3 | 0.4 | 4.0 | 0.85 | 0.2 |

The binding studies demonstrate that the four top clones exhibited between 150-550-fold enhancement in

binding affinity to cynoADAM9 while maintaining the same high affinity binding to huADAM9 as the parental antibody. hMAB-A (2C.2) and hMAB-A (21.2) was selected for further studies.

**Example 5**

**Immunohistochemistry Study of Antibody hMAB-A (2I.2)**

The cell specificity of hMAB-A (21.2) was investigated by IHC. Positive and negative control cells, and normal human and cynomolgus monkey tissues were contacted with hMAB-A (21.2) (2.5 μg/mL) or an isotype control (2.5 μg/mL) and the extent of staining was visualized. The results of the study are summarized in **Table 7.**

**Table 7**

| Cell/Tissue | hMAB-A **(2I.2) (2.5 μg/ml)** | **IgG1 Negative Control (2.5 μg/ml)** |
|---|---|---|
| Cho-K parental cells | - | - |
| Cho-K/huADAM9 medium expression P:1 | 2-4+ (gr c > m) rare to occasional and 1+ (gr c > m) occasional | - |
| Cho-K/huADAM9 high expression | 2-4+ (gr c > m) frequent | - |
| Cho-K/ cynoADAM9 clone 2 | 2-4+ (gr c > m) frequent | - |
| Cho-K/cynoADAM9 clone #16 | 2-4+ (gr c > m) frequent | - |
| A498 cells | 2-4+ (gr c > m) rare to occasional and 1+ (gr c > m) occasional to frequent | - |
| Colon MG06-CHTN-96 B | - | numerous 2-4+ (gr c) cells consistent with macrophages |
| Lung MG06-CHtN-162B1 A | - | occasional 2-4+ (gr c) cells consistent with macrophages |
| Liver ILS11103 B | - | hepatocytes 1+ (gr c) rare to occasional |
| Pancreas ILS10266 | - | - |
| Heart Life Legacy 0910035D | - | cardiac muscle cells with numerous 1-3+ small foci of (gr c) consistent with lipofuscin pigment |
| Kidney ILS10241 B | - | tubule epi 1+ (gr c) rare |
| Bladder ILSD8011 J | - | occasional 2-4+ (gr c) cells consistent with macrophages |
| Cyno Colon #1 | - | mucosal epi (luminal m) 2-4+ rare to occasional and 1+ rare to occasional; numerous 2-3+ (gr c) cells consistent with macrophages predominantly within LP |
| Cyno Lung #1 | - | very rare 2-4+ (gr c) cells consistent with macrophages |
| Cyno Liver #1 | - | - |
| Cyno Pancreas #1 | - | - |
| Cyno Heart #1 | - | - |
| Cyno Kidney #070368M | - | tubule epi 2+ (gr c) rare and 1+ (gr c) rare to occasional |
| Cyno Bladder #1 | transitional cell epi ± (gr c) rare | rare 1-4+ (gr c) cells consistent with macrophages |
| Lung CA ILS10108 | H score 150 | tu - |

(continued)

| Table 7 | | |
|---|---|---|
| **Cell/Tissue** | hMAB-A **(2I.2) (2.5 μg/ml)** | **IgG1 Negative Control (2.5 μg/ml)** |
| Lung CA ILS7223 | H score 180 | tu - |
| Lung CA ILS2156 A | H score 80 | tu - |
| Lung CA ILS7295 A | H score 60 | tu - |

IHC studies were also conducted to assess binding of humanized/optimized hMAB-A (21.2) at a concentration of 12.5 μg/mL (5x optimal staining concentration). Positive and negative control cells, and normal human and cynomolgus monkey tissues were employed in this study. The results of the study are summarized in **Table 8.**

| Table 8 | | |
|---|---|---|
| **Cell/Tissue** | hMAB-A **(2I.2) (12.5 μg/ml)** | **IgG1 Negative Control (12.5 μg/ml)** |
| Cho-K parental cells | - | - |
| Cho-K/huADAM9 medium expression P: 1 | 2-4+ (gr c > m) occasional to frequent | - |
| Cho-K/huADAM9 high expression | 3-4+ (gr c > m) occasional to frequent | - |
| Cho-K/ cynoADAM9 clone 2 | 3-4+ (gr c > m) frequent | - |
| Cho-K/ cynoADAM9 clone #16 | 3-4+ (gr c > m) frequent | - |
| A498 cells | 2-4+ (gr c > m) occasional to frequent | - |
| Colon MG06-CHTN-96 B | epi ± - 1+ rare to occasional | numerous 2-4+ (gr c) cells consistent with macrophages predominantly within LP in test article and negative control |
| Lung MG06-CHtN-162B1 A | alveolar cells (favor pneumocytes) 2-3+ (gr c > m) rare, 1+ (gr c > m) rare to occasional; EC 2-4+ (c,m) rare, 1+ (c,m) rare | occasional scattered 2-4+ (gr c) cells consistent with macrophages in test article and negative control |
| Liver ILS11103 B | - | occasional scattered 2-4+ (gr c) cells consistent with macrophages in test article and negative control |
| Pancreas ILS10266 | ductal epi 1+ (gr c > m) very rare | cells (favor acinar cells) 1+ (gr c) very rare; occasional scattered 2-4+ (gr c) cells consistent with macrophages in test article and negative |
| Heart Life Legacy 0910035D | - | numerous small foci 1-3+ granular staining with cardiac muscle cells consistent with lipofuscin pigment consistent with artifact in test article and negative control |
| Kidney ILS10241 B | tubule epi 1+ (gr c) rare to occasional | tubule epi ± (gr c) rare |
| Bladder ILSD8011 J | transitional cell epi 1+ (gr c) rare | rare 2-4+ (gr c) cells consistent with macrophages in test article and negative control |

(continued)

| Table 8 | | |
|---|---|---|
| **Cell/Tissue** | hMAB-A **(2I.2) (12.5 μg/ml)** | **IgG1 Negative Control (12.5 μg/ml)** |
| Cyno Colon #1 | - | mucosal epi (luminal m) 2-4+ occasional and 1+ rare to occasional |
| Cyno Lung #1 | bronchial epi 1+ (gr c > m) rare to occasional and ± (gr c > m) occasional to frequent | - |
| Cyno Liver #1 | - | - |
| Cyno Pancreas #1 | - | - |
| Cyno Heart #1 | - | - |
| Cyno Kidney #070368M | - | tubule epi 1+ (gr c) rare and ± (gr c) rare |
| Cyno Bladder #1 | transitional cell epi 2+ (gr c > m) rare and 1+ (gr c > m) rare to occasional | - |
| Lung CA ILS10108 | H score 180 | tu - |
| Lung CA ILS7223 | H score 180 | tu - |
| Lung CA ILS2156 A | H score 115 | tu - |
| Lung CA ILS7295 A | H score 115 | tu - |

A comparative IHC study was conducted in order to assess differences in binding by hMAB-A (2.2), hMAB-A (2.3), hMAB-A (2C.2), and hMAB-A (2I.2) at 2.5 μg/mL or 5 μg/mL. Positive and negative control cells, and normal human and cynomolgus monkey tissues were employed in this study. The results of the study are summarized in **Table 9.**

| Table 9 | | | | | |
|---|---|---|---|---|---|
| **Tissue** | hMAB-A **(2.3) 5 ug/mL** | hMAB-A **(2.2) 2.5 μg/mL** | hMAB-A **(2C.2) 2.5 μg/mL** | hMAB-A **(2I.2) 2.5 μg/mL** | **Isotype control 5 μg/mL** |
| Cho-K parental P:3 | - | - | - | - | - |
| Cho-K/hu ADAM9.2 medium expression P:1 | 1+ (c) occasional | 2-4+ (gr c > m) rare and 1+ (gr c > m) rare to occasional | 2-4+ (gr c > m) rare to occasional and 1+ (gr c > m) rare to occasional | 2-4+ (gr c > m) rare to occasional and 1+ (gr c > m) occasional | - |
| Cho-K/hu ADAM9.18 high expression P:1 | 3+ (m,c) frequent | 2-4+ (gr c > m) occasional to frequent and 1+ (gr c > m) occasional | 2-4+ (gr c > m) occasional to frequent and 1+ (gr c > m) occasional | 2-4+ (gr c > m) frequent | - |
| Cho-K Cyno #2 | 1+(c) occasional | - | 3-4+ (gr c > m) frequent | 2-4+ (gr c > m) frequent | - |
| Cho-K Cyno #16 | 2+ (c,m) occasional to frequent | 2-4+ (gr c > m) rare and 1+ (gr c > m) rare to occasional | 3-4+ (gr c > m) frequent | 2-4+ (gr c > m) frequent | - |

(continued)

| Table 9 | | | | | |
|---|---|---|---|---|---|
| **Tissue** | hMAB-A **(2.3) 5 ug/mL** | hMAB-A **(2.2) 2.5 μg/mL** | hMAB-A **(2C.2) 2.5 μg/mL** | hMAB-A **(2I.2) 2.5 μg/mL** | **Isotype control 5 μg/mL** |
| A498 072210 | 3-4+ (c,m) frequent | 2-4+ (gr c > m) rare and 1+ (gr c > m) occasional to frequent | 2-4+ (gr c > m) rare and 1+ (gr c > m) occasional | 2-4+ (gr c > m) rare to occasional and 1+ (gr c > m) occasional to frequent | - |
| Lung CA ILS10108 | IHC score 3 | H Score 55 | H Score 17 | H score 150 | - |
| Lung CA ILS7223 | IHC score 3 | H Score 205 | H Score 160 | H score 180 | - |
| Lung CA ILS2156 A | IHC score 1 | H Score 5 | H Score 0 | H score 80 | - |
| Lung CA ILS7295 A | IHC score 1 | H Score 1 | H Score 0 | H score 60 | - |

A further comparative IHC study was conducted in order to assess differences in binding by hMAB-A (2.2), hMAB-A (2.3), hMAB-A (2C.2), and hMAB-A (21.2) and murine MAB-A at 2.5 μg/mL 5 μg/mL or 12.5 μg/mL. Positive and negative control cells, and normal human and cynomolgus monkey tissues were employed in this study. The results of the study are summarized in **Table 10.**

| Table 10 | | | | | |
|---|---|---|---|---|---|
| **Tissue** | hMAB-A **(2.3) 5 ug/mL** | hMAB-A **(2.2) 2.5 μg/mL** | hMAB-A **(2C.2) 2.5 μg/mL** | hMAB-A **(2I.2) 12.5 μg/ml** | MAB-A **5 μg/mL** |
| Colon MG06-CHTN-96 B | epi 1+ (c,m) rare; sm negative | - | - | epi ± - 1+ rare to occasional | Epithelium 1-3+ [m, c] (occas to freq); Others (Neg) |
| Lung MG06-CHtN-162B1 A | pneumocytes/ macr ophages 2+ (c,m) occasional | - | - | alveolar cells (favor pneumocytes) 2-3+ (gr c > m) rare, 1+ (gr c > m) rare to occasional; EC 2-4+ (c,m) rare, 1+ (c,m) rare | Monoctyes 1+ [c] (rare to occas); Others (Neg) |
| Liver ILS11103 B | hepatocytes 1+ (c) rare to occasional | hepatocytes 1+ (gr c) frequent | hepatocytes 2+ (gr c) rare and 1+ (gr c) frequent | - | Kupffer cells 3+ [c] (occas); Others (Neg) |
| Pancreas ILS 10266 | epi 1+ (c) rare; Islet Cells 1+ (c) very rare | - | - | ductal epi 1+ (gr c > m) very rare | Ductal epithelium 1-2+ [c, m] (rare to occas); Fibril 2+ (rare); Others (Neg) |
| Heart Life Legacy 0910035D | ± | - | - | - | Neg |

(continued)

Table 10

| Tissue | hMAB-A **(2.3) 5 ug/mL** | hMAB-A **(2.2) 2.5 μg/mL** | hMAB-A **(2C.2) 2.5 μg/mL** | hMAB-A **(2I.2) 12.5 μg/ml** | MAB-A **5 μg/mL** |
|---|---|---|---|---|---|
| Kidney ILS10241 B | epi 2-3+ (c,m) frequent | tubule epi 2+ (gr c) rare to occasional and 1+ (gr c) occasional to frequent | tubule epi 2+ (gr c) rare to occasional and 1+ (gr c) occasional to frequent | tubule epi 1+ (gr c) rare to occasional | Epithelium 1+ [c] (rare); Others (Neg) |
| Bladder ILSD8011 J | transitional epi 1+ (c) rare to occasional | - | - | transitional cell epi 1+ (gr c) rare | Transitional epithelium 2+ [c, m] (occas to freq); Stromal cells 3+ [c] (rare); Others (Neg) |
| Cyno Colon #1 | epi 1+ (c,m) rare | - | - | - | |
| Cyno Lung #1 | Macrophage and pneumocytes 1+ (c) very rare | - | bronchial epi 3-4+ (gr c) rare, 2+ (gr c) occasional, and 1+ (gr c) occasional | bronchial epi 1+ (gr c > m) rare to occasional and ± (gr c > m) occasional to frequent | - |
| Cyno Liver #1 | hepatocytes 1+ (c) frequent | hepatocytes 2+ (gr c) rare to occasional and 1+ (gr c) rare to occasional | hepatocytes 2+ (gr c) rare to occasional and 1+ (gr c) occasional; ductal epi 1+ (gr c) occasional | - | - |
| Cyno Pancreas #1 | epi and Islet Cells 1+ (c) very rare | - | islet cells ± (gr c) frequent; ductal epi 1+ (gr c) rare to occasional | - | positive |
| Cyno Heart #1 | myocardium 1+ (c) frequent | - | - | - | - |
| Cyno Kidney #070368M | epi 2+ (c) frequent | tubule epi 2+ (gr c) rare to occasional and 1+ (gr c) rare to occasional | tubule epi 2+ (gr c) rare to occasional and 1+ (gr c) occasional to frequent | - | positive |
| Cyno Bladder #1 | transitional epi ± (c); macrophages very rare | - | transitional cell epi 2-3+ (gr c > m) rare and 1+ (gr c > m) occasional | transitional cell epi 2+ (gr c > m) rare and 1+ (gr c > m) rare to occasional | |

The results thus demonstrate that hMAB-A (2.2) exhibited an overall low level staining of human hepatocytes and kidney tubules at optimal concentration, with a lower staining intensity/frequency of reactivity in hepatocytes and kidney tubules observed in the negative control. hMAB-A (2.2) exhibited similar low level staining of cyno hepatocytes

and kidney tubules at optimal concentration, with lower staining intensity/frequency of reactivity in kidney tubules observed in the negative control.

The results also demonstrate that hMAB-A (2C.2) exhibited an overall low level staining of human hepatocytes and kidney tubules at optimal concentration, with lower staining intensity/frequency of reactivity in hepatocytes and kidney tubules observed in the negative control. hMAB-A (2C.2) exhibited similar low level staining in cyno hepatocytes and kidney tubules at optimal concentration. Additional minimal findings in cyno lung epithelium, pancreas islets/ epithelium and bladder epithelium for hMAB-A (2C.2) was not observed in the corresponding human tissue; lower staining intensity/frequency of reactivity was observed in lung epithelium, kidney tubules, bladder epithelium in negative control. The results also demonstrate that hMAB-A (21.2) exhibited no staining of human or cyno tissues at optimal concentration, with rare +/- bladder transitional cell epithelium staining. hMAB-A (21.2) also exhibited overall low level and frequency staining of human lung alveolar cells, pancreas ductal epithelium, kidney tubule, bladder transitional cell epithelium at 5x optimal concentration, and overall low level staining of cyno bronchial epithelium and bladder transitional cell epithelium at 5x optimal concentration. hMAB-A (21.2) exhibits an overall favorable IHC profile on the human normal tissues tested and a similar profile on corresponding cynomolgus monkey tissues.

**Example 6**

**hMAB-A (21.2) Comprising Variant Fc Regions**

hMAB-A (21.2) comprises a light chain **(SEQ ID NO:68)** having a kappa light chain constant region and a heavy chain **(SEQ ID NO:52)** having wild-type IgG heavy chain constant regions. Fc variants were generated by introducing the following substitutions into the Fc Region: L234A/L235A (see, *e.g.,* **SEQ ID NO: 78)** designated hMAB-A (2I.2)(AA); S442C (see, *e.g.*, **SEQ ID NO: 79)** designated hMAB-A (2I.2)(C); and L234A/L235A/S442C (see, *e.g.,* **SEQ ID NO: 80)** designated hMAB-A (2I.2)(AA/C). The binding of each Fc variant to huADAM9-His and cynoADAM9-His was examined by ELISA assay. Briefly, antibodies that bind to histidine-containing peptides, and that had been coated onto microtiter plates, were used to capture His peptide-tagged soluble cynoADAM9 or His peptide-tagged soluble huADAM9 (0.5 μg/mL), and the binding of serial dilutions of the parental hMAB-A (2.2) and the Fc variants was examined. The binding curves huADAM9 and cynoADAM9 are presented in **FIG. 5A** and **FIG. 5B,** respectively and show that each of the Fc variants retained the binding affinity of hMAB-A (21.2) having a wild-type Fc region.

**Example 7**

**Target Expression Analysis**

To evaluate ADAM9 expression across different indications, a tissue microarray (TMA) with 20 different tumor types was first evaluated using an ADAM9 IHC assay developed at ImmunoGen for preliminary research use.

All samples analyzed were FFPE (Formalin fixed & paraffin embedded) samples. The 500 core 20 carcinoma TMA was purchased from Folio Biosciences (Cat# ARY-HH0212). The NSCLC TMA with 80 cores for adenocarcinoma and 80 cores for squamous cell carcinoma was purchased from US Biomax (Cat# LC1921A). The colorectal cancer TMA with 80 cores for adenocarcinoma was purchased from Pantomics Inc. (Cat# COC1261). The gastric cancer samples were purchased from Avaden Biosciences.

Immunohistochemical staining for ADAM9 was carried out using the Ventana Discovery Ultra autostainer. The primary antibody for ADAM9 was a commercially available rabbit monoclonal antibody. All samples were evaluated and scored by a board certified pathologist trained in the scoring algorithm. The presence of at least 100 viable tumor cells was required for scoring. Staining intensity was scored on a semi-quantitative integer scale from 0 to 3, with 0 representing no staining, 1 representing weak staining, 2 representing moderate and 3 representing strong staining. The percentage of cells staining positively at each intensity level was recorded. Scoring was based on localization of Adam9 to the cell membrane only, as well as evaluation of localization to both cytoplasm and membrane. The staining results were analyzed by H score, which combines components of staining intensity with the percentage of positive cells. It has a value between 0 and 300 and is defined as:

$$\begin{gathered} 1 * (\text{percentage of cells staining at 1+ intensity}) \\ + 2 * (\text{percentage of cells staining at 2+ intensity}) \\ + 3 * (\text{percentage of cells staining at 3+ intensity}) \\ = \text{H score.} \end{gathered}$$

The 500 core 20 carcinoma TMA with 5 normal tissue controls for each tumor type was stained and scored in two different ways: (1) based on membrane staining alone or (2) based on membrane and cytoplasmic staining. Table 11 below and FIG. 6A summarize the prevalence of ADAM9 based on membrane staining for all twenty indications and Table 12 and FIG. 6B summarize the results of membrane and cytoplasmic staining for eight selected indications.

Based on the results from the multi carcinoma TMA, three indications for an expanded prevalence analysis were chosen: non-small cell lung cancer (NSCLC), colorectal cancer (CRC) and gastric cancer. For NSCLC, one TMA with 80 cores for adenocarcinoma and 80 cores for squamous cell carcinoma was stained and evaluated. For CRC, one TMA with 80 cores for adenocarcinoma was analyzed, of which 78 were evaluable. For gastric cancer, 15 whole tissue sections of adenocarcinoma were analyzed. All of these samples were scored for membrane and cytoplasmic staining, and the results are summarized in Table 13. The results of these preliminary studies show that ADAM9 is expressed in a wide range of solid cancers and support the use of anti-ADAM9 drug conjugates in many different ADAM9-expressing solid tumors.

**Table 11: Prevalence of ADAM9 in 20 different indications based on membrane staining**

| Tumor type | % Positive (H score >=1) | H score: 1-100 | H score: 101-200 |
|---|---|---|---|
| Pancreas (n=17) | 95% | 24% | 71% |
| Uterus (n=18) | 89% | 67% | 22% |
| Thyroid (n=17) | 88% | 88% | 0% |
| Kidney (n=17) | 88% | 59% | 29% |
| Testis (n=17) | 83% | 65% | 18% |
| Prostate (n=20) | 80% | 45% | 35% |
| Colon (n=16) | 76% | 38% | 38% |
| Bladder (n=16) | 76% | 63% | 13% |
| Breast(n=20) | 75% | 65% | 10% |
| Brain (n=19) | 68% | 63% | 5% |
| Stomach (n=17) | 59% | 24% | 35% |
| Lung (n=19) | 58% | 58% | 0% |
| Esophagus (n=19) | 43% | 32% | 11% |
| Cervix (n=20) | 40% | 40% | 0% |
| Ovary (n=18) | 39% | 33% | 6% |
| Head and Neck (n=20) | 35% | 30% | 2% |
| Liver (n=19) | 32% | 32% | 0% |
| Skin (n=20) | 10% | 10% | 0% |
| Soft Tissue (n=20) | 0% | 0% | 0% |
| Lymphoma (n=20) | 0% | 0% | 0% |

**Table 12: Prevalence of ADAM9 in 8 selected indications based on membrane and cytoplasmic staining**

| Tumor type | % Positive (H score >=1) | H score: 1-100 | H score: 101-200 | H score: 201-300 |
|---|---|---|---|---|
| Colon | 100% | 31% | 63% | 6% |
| Lung | 100% | 58% | 42% | 0% |
| Pancreas | 100% | 18% | 76% | 6% |
| Prostate | 95% | 25% | 55% | 15% |
| Esophagus | 95% | 58% | 37% | 0% |
| Stomach | 94% | 18% | 71% | 6% |

(continued)

| Tumor type | % Positive (H score >=1) | H score: 1-100 | H score: 101-200 | H score: 201-300 |
|---|---|---|---|---|
| Breast | 70% | 50% | 20% | 0% |
| Ovarian | 61% | 44% | 17% | 0% |

**Table 13: Prevalence of ADAM9 in additional samples for NSCLC, CRC and Gastric Cancer based on membrane and cytoplasmic staining**

| Tumor Type | Number of Samples | % positive (H score >=1) | H-Score Distribution | | |
|---|---|---|---|---|---|
| | | | 1-100 | 101-200 | 201-300 |
| NSCLC Adenocarcinoma | 80 | 90% | 46% | 39% | 5% |
| NSCLC Squamous | 80 | 81% | 65% | 13% | 3% |
| CRC Adenocarcinoma | 78 | 91% | 51% | 41% | 0% |
| Gastric Adenocarcinoma | 15 | 100% | 27% | 53% | 20% |

**Example 8**

**Anti-ADAM9 Antibody Internalization Studies**

To assess the internalization of the anti-ADAM9 antibodies of the invention, flow cytometry-based internalization experiments were performed on hMAB-A(2.2), hMAB-A(21.2), and hMAB-A(2I.2)-S442C antibodies conjugated to Alexa Fluor 488.

Anti-ADAM9 Alexa488 antibody conjugates for hMAB-A(2.2), hMAB-A(21.2), hMAB-A(2I.2)-S442C were generated using Alexa Fluor 488 tetrafluorophenyl ester according to the manufacturer's instructions (Thermofisher). The conjugates were eluted in sodium azide free PBS, pH7.2 to enable internalization assays. The concentration and degree of labeling were calculated from absorption measurements at 280 nm and 494 nm. FACS binding assays were performed to ensure that Alexa488-labeling did not adversely affect target binding.

The internalization of anti-ADAM9-Alexa488 conjugates was determined following both continuous and pulse exposure to the fluorescent conjugates. For continuous experiments, NCI-HI703 cells were treated with a saturating concentration of the indicated Alexa488-labeled antibody on ice or at 37°C for the entire time indicated. While for pulse experiments anti-ADAM9-Alexa488 conjugates were prebound to the cells on ice and the excess conjugate washed away before shifting to 37°C and monitoring internalization. At the indicated time points following either continuous or pulse exposure, cells were lifted with versene (Thermofisher) and washed with ice-cold PBS twice, and replicate wells were resuspended in ice-cold PBS without (unquenched samples) or with 300nM anti-A488 antibody (quenched samples). All samples were incubated for 30 m on ice. Cells were then pelleted, fixed in 1% paraformaldehyde and analyzed by flow cytometry. The fluorescence of cells incubated on ice for 30 minutes and then incubated with anti-Alexa488 antibody represents the unquenchable fluorescent fraction and was subtracted from all other samples prior to calculating internalization. The percent internalization was calculated as fluorescence of quenched samples corrected for incomplete surface quenching (intracellular fluorescence) divided by that of unquenched cells (total fluorescence). The internalization of anti-ADAM9 antibody conjugates were graphed and the data was fitted using a single-phase exponential decay equation (GraphPad Prism, ver. 5.01).

The internalization of surface bound Alexa488-labeled anti-ADAM9 antibodies was evaluated after both pulse and continuous treatment in NCI-HI703 cells. All three anti-ADAM9-Alexa488 conjugates tested showed rapid internalization, with ~39% of the conjugates internalized in the first 15 minutes and a total of~77% internalized after 6 hours (FIG. 7A). Interestingly, after continuous exposure for 24 hours, the internalized fluorescent signal was ~7-fold greater than the total initial fluorescent signal bound to the cell surface after 30 minutes (FIG. 7B). Thus, ADAM9 is likely replenished at the cell surface from an intracellular pool during incubations at 37°C, allowing for multiple rounds of anti-ADAM9 antibody conjugate internalization. The efficacy of anti-ADAM9 immunoconjugates rely on of the internalization, intracellular trafficking, and degradation of the immunoconjugates. The potency of anti-ADAM9 immunoconjugates can in part be explained by the high internalization of anti-ADAM9 immunoconjugates which likely leads to generation of

high amounts of cytotoxic catabolites.

**Example 9**

**Anti-ADAM9 Antibody Cell Processing Studies**

To assess the on-cell target binding, uptake and lysosomal degradation of chMAB-A, a previously-described $^3$H-propionamide-labeled antibody processing method was used (Lai et al., Pharm Res. 2015 Nov; 32(11):3593-603). Using this method, the ADAM9-targeting chMAB-A antibody was trace labeled with tritiated propionate via lysine residues. It has previously been shown that upon cellular binding, uptake, and trafficking to the lysosome, [$^3$H]propionate labeled-Ab ($^3$H-Ab) is degraded and lysine-[$^3$H]propionamide is released into the cell growth medium. Addition of organic solvent precipitates the intact, labeled antibody and leaves the lysine-[$^3$H]propinoamide in solution, allowing convenient and accurate measurement of the extent of antibody processing.

chMAB-A was labeled with [$^3$H]-propionate as previously described. The NSCLC line, NCI-H1703, and the CRC line, DLD-1, were treated with 10 nM $^3$H-chMAB-A antibody after determination of antigen saturation via binding curve. Some cell samples were treated with the non-targeting, tritiated isotype control antibody, $^3$H-chKTI, while others were untreated. Cells were plated and grown overnight in 6-well plates and then pulse-treated with reagent(s) as previously described. Briefly, cells were incubated with either $^3$H-chMAB-A antibody or $^3$H-chKTI for 20 minutes before washing 3 times in fresh media. Cells were incubated overnight at 37°C with 5% $CO_2$. After a 20-24 hour incubation cells were harvested and protein precipitated with 4:3 volume acetone: media/cell mixture. Samples were frozen at -80 °C for a minimum of 1 hour before thawing and separating by centrifuge. Pellets were treated to solubilize protein prior to counting for 5 minutes in a Tri-Carb liquid scintillation counter (LSC). Per manufacturer's protocol, 1 mL of SOLVABLE (Perkin Elmer) was added to each pellet sample and incubated in a 50 °C water bath overnight. Samples were removed from the water bath, transferred to 20 mL glass scintillation vials and EDTA and $H_2O_2$ were added to samples followed by an additional 1 hour 50 °C incubation. Samples were quenched with HCl, 15 mL of Optima Gold liquid scintillation fluid (Perkin Elmer) was added, and samples were vortexed thoroughly. Samples were kept in the dark for a minimum of 4 hour before counting by LSC. Protein-free acetone extract samples were dried to <1 mL volumes under vacuum and processed using Solvable as described above prior to LSC. The amount of bound, degraded, and intact labeled antibody were calculated from the resulting sample CPM values.

The level of processing of $^3$H-chMAB-A antibody was determined after pulse-treatment and overnight incubation at 37 °C. NCI-HI703 cells showed 93% of $^3$H-chMAB-A processed within 24 hours, and DLD-1 cells showed 92% of $^3$H-chMAB-A processed in the same time period. Binding and processing of $^3$H-chKTI was negligible (>100-fold lower total CPM than for targeted antibody). The processing values for these cell lines are high, especially compared to the 24 hour pulse processing values previously reported for other ADC targets/antibodies supporting the anti-ADAM9 antibodies described herein as effective drug conjugates.

**Example 10**

**Binding Affinity Studies of Humanized/Optimized Anti-ADAM9 Antibodies and Drug Conjugates**

To evaluate the consequence of conjugation on antigen binding, the relative binding affinity of each anti-ADAM9 immunoconjugate and its respective unconjugated antibody to ADAM9 was determined by FACS analysis on 300-19 cells ectopically-expressing either human ADAM9 or cynomolgus ADAM9. Briefly, the ADAM9-expressing 300-19 cells were incubated with dilution series of anti-ADAM9 antibodies or immunoconjugates for 30 min @ 4°C in FACS buffer (PBS, 0.1% BSA, 0.01% $NaN_3$). Samples were then washed and incubated with fluorescently-labeled secondary antibody for 30 minutes at 4°C. The normalized mean of fluorescence intensity at each concentration was plotted and the EC50 of binding was calculated using a nonlinear regression analysis (GraphPad Prims 4.0). The results from this study are summarized in Table 14.

All of the anti-ADAM9 antibodies and immunoconjugates tested bound with similar affinity to human ADAM9 with an EC50 of approximately 1.4 nM measured by flow cytometry, indicating that conjugation did not appreciably alter antibody binding affinity (see,

FIGs. 8A-8D). While humanization improved binding to cynomolgus ADAM9 relative to the chimeric antibody (chMAB-A), further optimized variants (hMAB-A(2C.2), hMAB-A(21.2), and hMAB-A(2I.2) S442C) and their immunoconjugates exhibited dramatically improved binding to cynomolgus ADAM9. The dramatic improvement in binding to cynomolgus ADAM9 facilitates toxicology and safety studies to validate the use of ADAM9 immunoconjugates as drug therapies.

**Table 14**

| Anti-ADAM9 antibody/immunoconjugate | EC50 (nM) by FACS | | |
|---|---|---|---|
| | 300-19: huADAM9 | 300-19: cynoADAM9 | cyno/ human |
| **chMAB-A** | 0.41 | 112.2 | 273 |
| chMAB-A-sSPDB-DM4 | 0.95 | 275.3 | 290 |
| chMAB-A-DGN-549 | 1.24 | 153.2 | 123 |
| **hMAB-A(2.2)** | 0.69 | 32.7 | 47 |
| hMAB-A(2.2)-sSPDB-DM4 | 1.11 | 13.3 | 12 |
| hMAB-A(2.2)-DGN549 | 1.55 | 22.3 | 14 |
| **hMAB-A(2C.2)** | 1.22 | 3.8 | 3 |
| hMAB-A(2C.2)-sSPDB-DM4 | 1.50 | 4.4 | 3 |
| hMAB-A(2C.2)-DGN549 | 1.99 | 5.6 | 3 |
| **hMAB-A(2I.2)** | 1.49 | 4.1 | 3 |
| hMAB-A(2I.2)-sSPDB-DM4 | 1.55 | 4.9 | 3 |
| hMAB-A(2I.2)-DGN549 | 2.25 | 6.6 | 3 |
| **hMAB-A(2I.2) S442C** | 1.33 | 3.8 | 3 |
| hMAB-A(2I.2) S442C-DGN549 | 2.49 | 6.2 | 2 |

**Example 11**

**Synthesis of N-(2-(2,5-dioxo-2,5-dihydro-1H-pyrrol-1-yl)ethyl)-11-(3-((((S)-8-methoxy-6-oxo-11,12,12a,13-tetrahydro-6H-benzo[5,6][1,4]diazepino[1,2-a]indol-9-yl)oxy)methyl)-5-((((S)-8-methoxy-6-oxo-12a,13-dihydro-6H-benzo[5,6][1,4]diazepino[1,2-a]indol-9-yl)oxy)methyl)phenyl)-13,13-dimethyl-2,5,8-trioxa-14,15-dithia-11-azanonadecan-19-amide, Compound D6**

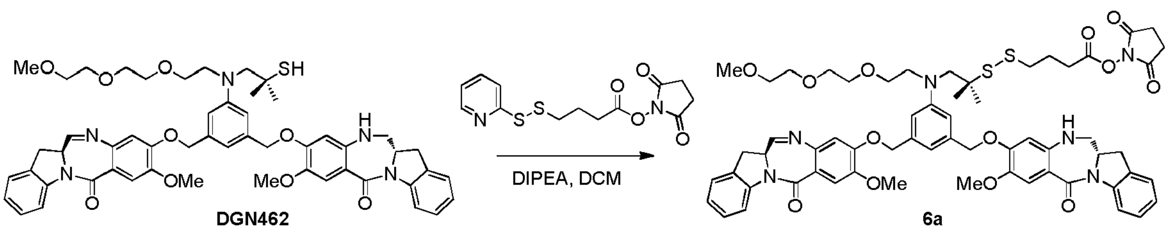

DGN462 6a

Step 1: To a solution of the free thiol DGN462 (40 mg, 0.042 mmol) and NHS 4-(2-pyridyldithio)butanate (35 mg, 80% purity, 0.085 mmol) in anhydrous dichloromethane (0.5 mL) was added anhydrous diisopropylethylamine (0.015 mL, 0.085 mmol) and was stirred at room temperature for 16 hours. The reaction mixture was quenched with saturated ammonium chloride and diluted with dichloromethane. The obtained mixture was separated in a separatory funnel. The organic layer was washed with brine, dried over anhydrous sodium sulfate, filtered and stripped under reduced pressure. The residue was purified by semi-preparative reverse phase HPLC (CI8 column, $CH_3CN/H_2O$). The fractions that contained pure product were combined, frozen and lyophilized to give the desired NHS ester, compound 6a (29.7 mg, 60% yield). LCMS = 9.1 min (15 min method). MS (m/z): 1157.3 $(M + 1)^+$.

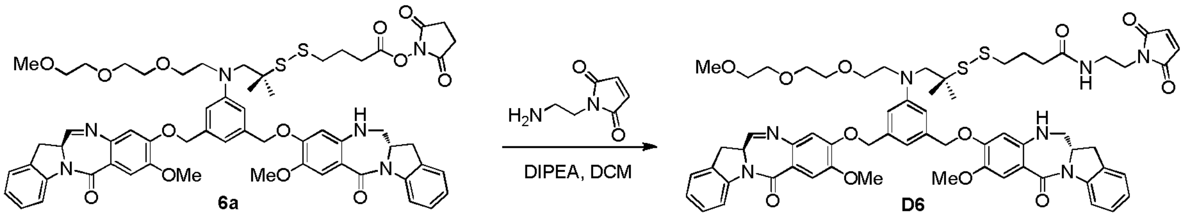

6a
D6

Step 2: To a solution of the NHS ester, compound 6a (12.3 mg, 0.011 mmol) and N-(2-aminoethyl)maleimide hydrochloride (2.0 mg, 0.011 mmol) in anhydrous dichloromethane (0.3 mL) was added DIPEA (0.0022 mL, 0.013 mmol). The mixture was stirred at room temperature for 3 hours then it was stripped under reduced pressure. The residue was purified by semi-preparative reverse phase HPLC (CI8 column, $CH_3CN/H_2O$). The fractions that contained pure product were combined, frozen and lyophilized to give the desired maleimide, compound D6 (10 mg, 80% yield). LCMS = 8.3 min (15 min method). MS (m/z): 1181.8 $(M + 1)^+$.

## Example 12

**Synthesis of N1-(2-(2,5-dioxo-2,5-dihydro-1H-pyrrol-1-yl)ethyl)-N6-((S)-1-(((S)-1-((3-((((S)-8-methoxy-6-oxo-11,12,12a,13-tetrahydro-6H-benzo[5,6][1,4]diazepino[1,2-a]indol-9-yl)oxy)methyl)-5-((((S)-8-methoxy-6-oxo-12a,13-dihydro-6H-benzo[5,6][1,4]diazepino[1,2-a]indol-9-yl)oxy)methyl)phenyl)amino)-1-oxopropan-2-yl)amino)-1-oxopropan-2-yl)adipamide, compound D5**

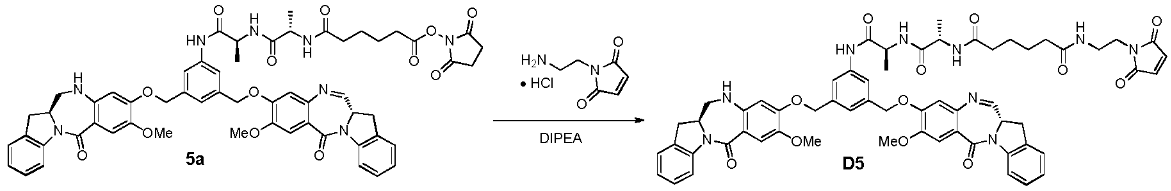

5a
D5

NHS ester, compound 5a (8.2 mg, 7.6 μmol) and 1-(2-aminoethyl)-1H-pyrrole-2,5-dione hydrochloride (2.2 mg, 0.011 mmol) were dissolved in anhydrous dichloromethane (305 μL) at room temperature. DIPEA (2.66 μL, 0.015mmol) was added and the reaction and was stirred for 3.5 hours. The reaction mixture was concentrated and was purified by RPHPLC (C18 column, $CH_3CN/H_2O$, gradient, 35% to 55%). The desired product fractions were frozen and lyophilized to give maleimide, compound D5 as a solid white powder (5.3 mg, 58% yield). LCMS = 5.11 min (8 min method). MS (m/z): 1100.6 $(M + 1)^+$.

## Example 13

**Synthesis of 1-((2-(2,5-dioxo-2,5-dihydro-1H-pyrrol-1-yl)ethyl)amino)-4-((5-((3-((((S)-8-methoxy-6-oxo-11,12,12a,13-tetrahydro-6H-benzo[5,6][1,4]diazepino[1,2-a]indol-9-yl)oxy)methyl)-5-((((S)-8-methoxy-6-oxo-12a,13-dihydro-6H-benzo[5,6][1,4]diazepino[1,2-a]indol-9-yl)oxy)methyl)phenyl)amino)-2-methyl-5-oxopentan-2-yl)disulfanyl)-1-oxobutane-2-sulfonic acid, compound D4**

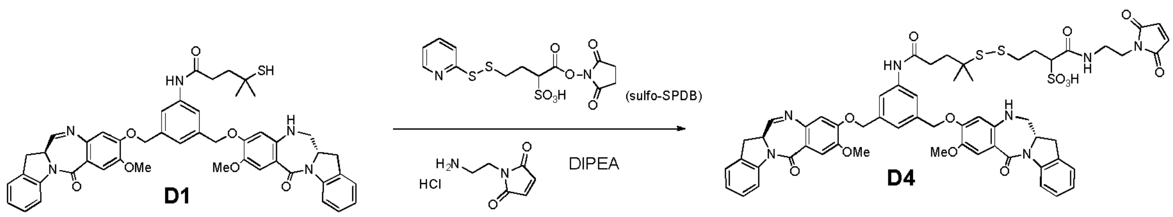

D1
D4

To a suspension of the free thiol, D1 (88 mg, 0.105 mmol) and 1-((2,5-dioxopyrrolidin-1-yl)oxy)-1-oxo-4-(pyridin-2-yldisulfanyl)butane-2-sulfonic acid (sulfo-SPDB) (64.0 mg, 0.158 mmol) in anhydrous dichloromethane (2.10 mL) was

added DIPEA (55.0 μL, 0.315 mmol) under nitrogen at room temperature. The mixture stirred for 16 hours and then 1-(2-aminoethyl)-1H-pyrrole-2,5-dione hydrochloride (55.6 mg, 0.315 mmol), anhydrous dichloromethane (1.0 mL) and DIPEA (0.055 mL, 0.315 mmol) were added. The mixture stirred for an additional 5 hours at room temperature upon which the reaction was concentrated *in vacuo.* The resulting residue was purified by RP-HPLC (Cl8, $CH_3CN/H_2O$). Fractions containing desired product were frozen and lyophilized to give maleimide, D4 (20 mg, 16% yield) as a white solid. LCMS = 4.92 min (8 min method). MS (m/z): 1158.6 $(M + 1)^+$.

**Example 14**

**Synthesis of N-(2-(2,5-dioxo-2,5-dihydro-1H-pyrrol-1-yl)ethyl)-11-(3-((((S)-8-methoxy-6-oxo-11,12,12a,13-tetrahydro-6H-benzo[5,6][1,4]diazepino[1,2-a]indol-9-yl)oxy)methyl)-5-((((S)-8-methoxy-6-oxo-12a,13-dihydro-6H-benzo[5,6][1,4]diazepino[1,2-a]indol-9-yl)oxy)methyl)phenyl)-2,5,8-triobxa-11-azapentadecan-15-amide, compound D7**

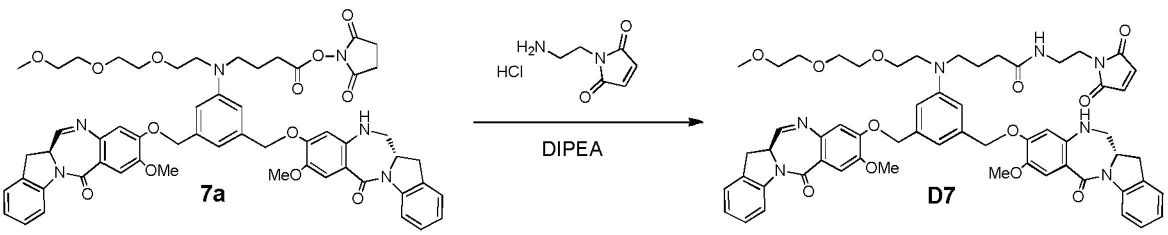


7a D7

To a solution of NHS ester, **7a** (5 mg, 4.82 μmol) and 1-(2-aminoethyl)-1H-pyrrole-2,5-dione hydrochloride (1.7 mg, 9.64 μmol) in anhydrous dichloromethane (200 μL) was added DIPEA (1.512 μL, 8.68 μmol) under nitrogen. The mixture was stirred at room temperature for 4 hours and then concentrated *in vacuo.* The resulting residue was purified by RP-HPLC (C18, $CH_3CN$ / $H_2O$). Fractions containing desired product were frozen and lyophilized to give maleimide, compound **D7** (3.5 mg, 68% yield). LCMS = 4.61 min (15 min method). MS (m/z): 1062.8 $(M + 1)^+$.

**Example 15**

**Preparation of Cysteine Site-Specific Conjugate of the anti-ADAM9 Antibody hMAB-A(2I.2)-S442C-DGN549**

hMAB-A(2I.2)-S442C is a humanized/optimized antibody with a light chain sequence of SEQ ID NO:68 (comprising a VL sequence of SEQ ID NO:55), and a heavy chain sequence of SEQ ID NO:142 (comprising a VH sequence of SEQ ID NO:28), which includes an engineered Cys corresponding to the $6^{th}$ to the last residue of SEQ ID NO:142, when X is a lysine, or corresponding to the $5^{th}$ to the last residue of SEQ ID NO: 142, when X is absent.

The two unpaired cysteine residues in the hMAB-A(2I.2)-S442C antibody were reduced using standard procedures. To a solution of this intermediate in phosphate buffered saline (PBS), 5 mM *N*,*N*,*N*',*N*'-ethylenediaminetetracetic acid (EDTA) pH 6.0 was added *N*,*N*-dimethylacetamide (DMA), propylene glycol, and 10 molar equivalents of DGN549-C (compound D5) as a stock solution in DMA to give a reaction mixture with a final solvent composition of 2% v/v DMA and 38% v/v propylene glycol in PBS 5mM EDTA pH 6.0 and the reaction was allowed to proceed overnight at 25°C.

The conjugate was purified into 20 mM succinate, 8.5% sucrose, 0.01% Tween-20, 50 μM sodium bisulfite, pH 4.2 using a fast protein liquid chromatography (FPLC) system fitted with Sephadex G-25 Fine desalting columns with the flow rate set to 5 mL/min. The conjugate was then filtered through a 0.22 μm syringe filter. The conjugate was found to have an average of 1.9 mol DGN549/mol antibody by UV-vis, 97.6% monomer by SEC, and 0.8% unconjugated DGN549 by tandem SEC/RP-UPLC. LC-MS of the deglycosylated conjugate is not shown.

**Example 16**

**Preparation of Lysine-Linked IGN Conjugates of anti-ADAM9 Antibodies**

**a. Preparation of hMAB-A(2I.2)-DGN549**

hMAB-A(2I.2) is a humanized/optimized antibody with a light chain sequence of SEQ ID NO:68 and a heavy chain sequence of SEQ ID NO:52. To a solution containing the hMAB-A(2I.2)antibody buffered at pH 8.5 with 15 mM

2-[4-(hydroxyethyl)piperzin-1-yl]ethanesulfonic acid (HEPES), N,N-dimethylacetamide (DMA) was added along with 4.8 equivalents of DGN549-L (compound D2) as a stock solution in DMA such that the final solvent composition was 10% (v/v) DMA and 90% (v/v) aqueous buffer. The reaction was allowed to proceed for 4 hr. at 25°C.

The conjugate was purified into 20 mM succinate, 8.5% sucrose, 0.01% Tween-20, 50 μM sodium bisulfite, pH 5.2 over Sephadex G-25 desalting columns, dialzyed against this buffer using a membrane with a 10 kDa molecular weight cutoff, and filtered through a 0.22 μm syringe filter.

The conjugate had an average of 2.8 mol DGN549/mol antibody by UV-vis, 98.6% monomer by SEC, and < 0.5% unconjugated DGN549 by tandem SEC/RP-UPLC. LC-MS of the deglycosylated conjugate is not shown.

**b. Preparation of hMAB-A(2.2)-DGN549**

hMAB-A(2.2) is a humanized antibody with a light chain sequence of SEQ ID NO:68 and a heavy chain sequence of SEQ ID NO:50. The hMAB-A(2.2) antibody was conjugated to DGN549 using the same conditions as those described for hMAB-A(2I.2)-DGN549, except that 4.4 equivalents of DGN549-L (compound D2) were added to the reaction. hMAB-A(2.2)-DGN549 was purified in the same manner as described for hMAB-A(2I.2)-DGN549.

Purified hMAB-A(2.2)-DGN549 had an average of 2.7 mol DGN549/mol antibody by UV-vis, 95.6% monomer by SEC, and 0.6% unconjugated DGN549 by tandem SEC/RP-UPLC. LC-MS of the deglycosylated conjugate is not shown.

**c. Preparation of hMAB-A(2C.2)-DGN549**

hMAB-A(2C.2) is a humanized/optimized antibody with a light chain sequence of SEQ ID NO:68 and a heavy chain sequence of SEQ ID NO:51.

The hMAB-A(2C.2) antibody was conjugated to DGN549 using the same conditions as those described for hMAB-A(2I.2)-DGN549, except that 4.9 equivalents of DGN549-L (compound D2) were added to the reaction. hMAB-A(2C.2)-DGN549 was purified in the same manner as described for hMAB-A(2I.2)-DGN549.

Purified hMAB-A(2C.2)-DGN549 had an average of 2.8 mol DGN549/mol antibody by UV-vis, 98.6% monomer by SEC, and < 0.6% unconjugated DGN549 by tandem SEC/RP-UPLC. LC-MS of the deglycosylated conjugate is not shown.

**d. Preparation of chMAB-A-DGN549, chMAB-A-sSPDB-IGN137, chMAB-A-IGN23, and chMAB-A-sSPDB-DGN462**

The chMAB-A antibody was conjugated to DGN549 using the same conditions as those described for hMAB-A(2I.2)-DGN549, except that 3.4 equivalents of DGN549-L (compound D2) were added to the reaction. chMAB-A-DGN549 was purified in the same manner as described for hMAB-A(2I.2)-DGN549, except that the conjugate was purified into 20 mM histidine, 50 mM sodium chloride, 8.5% sucrose, 0.01% Tween-20, 50 μM sodium bisulfite, pH 6.2. Purified chMAB-A-DGN549 had an average of 2.7 mol DGN549/mol antibody by UV-vis, 94.4% monomer by SEC, and < 0.4% unconjugated DGN549 by tandem SEC/RP-UPLC. LC-MS of the deglycosylated conjugate is not shown.

The chMAB-A antibody was conjugated to IGN137 (compound D1) via the sulfo-SPDB linker as described previously in U.S. Patent No. 9,381,256. The conjugate was purified in the same manner as described for hMAB-A(2I.2)-DGN549, except that the conjugate was purified into 20 mM histidine, 50 mM sodium chloride, 8.5% sucrose, 0.01% Tween-20, 50 μM sodium bisulfite, pH 6.2. The conjugate had an average of 2.9 mol IGN137/mol antibody by UV-vis, 88.4% monomer by SEC, and ≤0.4% unconjugated IGN137 by tandem SEC/RP-UPLC. LC-MS of the deglycosylated conjugate is not shown.

The chMAB-A antibody was conjugated to IGN23 (compound D3) as described previously in U.S. Patent No. 8,426,402. The conjugate was purified in the same manner as described for hMAB-A(2I.2)-DGN549, except that the conjugate was purified into 20 mM histidine, 50 mM sodium chloride, 8.5% sucrose, 0.01% Tween-20, 50 μM sodium bisulfite, pH 6.2 and dialysis was not performed. The conjugate had an average of 3.2 mol IGN23/mol antibody by UV-vis, 97.9% monomer by SEC, and 1.2% unconjugated IGN23 by reversed phase HPLC analysis. LC-MS of the deglycosylated conjugate is not shown.

The chMAB-A antibody was conjugated to DGN462 via sulfo-SPDB as described previously in U.S. Patent No. 8765740. The conjugate was purified in the same manner as described for hMAB-A(2I.2)-DGN549, except that the conjugate was purified into 20 mM histidine, 50 mM sodium chloride, 8.5% sucrose, 0.01% Tween-20, 50 μM sodium bisulfite, pH 6.2 and dialysis was not performed. The conjugate had an average of 2.8 mol DGN462/mol antibody by UV-vis, 95.6% monomer by SEC, and 0.5% unconjugated DGN462 by reversed phase HPLC analysis. LC-MS of the deglycosylated conjugate is not shown.

**Example 17**

**Preparation of Lysine-Linked DM Conjugates of anti-ADAM9 Antibodies**

**a. Preparation of hMAB-A(2I.2)-sSPDB-DM4**

hMAB-A(2I.2) is a humanized/optimized antibody with a light chain sequence of SEQ ID NO:68 and a heavy chain sequence of SEQ ID NO:52.

To prepare the **hMAB-A(2I.2)-sSPDB-DM4** conjugate, sulfo-SPDB (sSPDB) and DM4 additions were performed in a step-wise manner. First, a solution containing hMAB-A(2I.2) antibody buffered at pH 8.1 with 50 mM 4-(2-Hydroxyethyl)-1-piperazinepropanesulfonic acid (EPPS), 50 mM sodium chloride was mixed with DMA and 11.5 equivalents of sSPDB from a DMA stock solution such that the final solvent composition was 10% (v/v) DMA and 90% (v/v) aqueous buffer. After allowing the first reaction step to proceed for 4 hr. at 25°C, 17.3 equivalents of DM4 from a DMA stock solution, DMA, and 500 mM EPPS/500 mM sodium chloride pH 8.1 buffer were added to the reaction mixture such that the final solvent composition of 10% (v/v) DMA and 90% (v/v) aqueous buffer from the first reaction step was maintained. The second reaction step was allowed to proceed overnight at 25°C.

The conjugate was purified into 10 mM succinate, 250 mM glycine, 0.5% sucrose, 0.01% Tween-20, pH 5.5 over Sephadex G-25 desalting columns, dialzyed against this buffer using a membrane with a 10 kDa molecular weight cutoff, and filtered through a 0.22 μm syringe filter.

The conjugate had an average of 3.5 mol DM4/mol antibody by UV-vis, 99.2% monomer by SEC, and ≤ 1.7% unconjugated DM4 by mixed-mode HPLC. LC-MS of the deglycosylated conjugate is not shown.

**b. Preparation of hMAB-A(2C.2)-sSPDB-DM4**

hMAB-A(2C.2) is a humanized/optimized antibody with a light chain sequence of SEQ ID NO:68 and a heavy chain sequence of SEQ ID NO:51.

The hMAB-A(2C.2) antibody was conjugated to DM4 via the sSPDB linker using the same method as that described for hMAB-A(2I.2)-sSPDB-DM4. The conjugate was purified in the same manner as that described for hMAB-A(2I.2)-sSPDB-DM4.

The conjugate had an average of 3.2 mol DM4/mol antibody by UV-vis, 98.9% monomer by SEC, and 2.6% unconjugated DM4 by mixed-mode HPLC. LC-MS of the deglycosylated conjugate is not shown.

**c. Preparation of hMAB-A(2.2)-sSPDB-DM4**

hMAB-A(2.2) is a humanized antibody with a light chain sequence of SEQ ID NO:68 and a heavy chain sequence of SEQ ID NO:50.

The hMAB-A(2.2) antibody was conjugated to DM4 via the sSPDB linker using the same method as that described for hMAB-A(2I.2)-sSPDB-DM4. The conjugate was purified into 10 mM succinate, 250 mM glycine, 0.5% sucrose, 0.01% Tween-20, pH 5.5 using a fast protein liquid chromatography (FPLC) system fitted with Sephadex G-25 Fine desalting columns and then filtered through a 0.22 μm syringe filter

The conjugate had an average of 3.2 mol DM4/mol antibody by UV-vis, 97.4% monomer by SEC, and ≤ 1.1% unconjugated DM4 by mixed-mode HPLC. LC-MS of the deglycosylated conjugate is not shown.

**d. Preparation of chMAB-A-sSPDB-DM4 and chMAB-A-SMCC-DM1**

The chMAB-A antibody was conjugated to DM4 via the sulfo-SPDB linker using the same method as that described for hMAB-A(2I.2)-sSPDB-DM4, except that 6.4 equivalents of sSPDB and 9.6 equivalents of DM4 were used in the respective reaction steps. The conjugate was purified in the same manner as that described for hMAB-A(2I.2)-sSPDB-DM4, except that dialysis was not performed. The conjugate had an average of 3.4 mol DM4/mol antibody by UV-vis, 97.0% monomer by SEC, and 4.7% unconjugated DM4 by mixed-mode HPLC. LC-MS of the deglycosylated conjugate is not shown.

The chMAB-A antibody was conjugated to DM1 via the SMCC linker using previously described methods in U.S. Patent No. 8624003. The conjugate was purified in the same manner as that described for hMAB-A(2I.2)-sSPDB-DM4, except that dialysis was not performed. The conjugate had an average of 3.9 mol DM1/mol antibody by UV-vis, 99.7% monomer by SEC, and 4.9% unconjugated DM1 by mixed-mode HPLC. LC-MS of the deglycosylated conjugate is not shown.

**Example 18**

***In vitro* Cytotoxicity of anti-ADAM9 Antibody Drug Conjugates**

The *in vitro* cytotoxicty of various anti-ADAM9 antibody immunoconjugates against a panel of ADAM9-expressing cell lines was compared to non-targeting IgG1 conjugates. Specifically, 500 to 2000 cells/well were plated in 96-well plates 24 hours prior to treatment. Conjugates were diluted into the culture medium using 3-fold dilution series and 100μL were added per well. Control wells containing cells but lacking conjugate, as well as wells contained medium only, were included in each assay plate. Assays were performed in triplicate for each data point. The plates were incubated at 37°C in a humidified 5% $CO_2$ incubator for 4 to 5 days. Then the relative number of viable cells in each well was determined using the WST-8 based Cell Counting Kit-8 (Dojindo Molecular Technologies). The surviving fraction of cells in each well was calculated by first correcting for the medium background absorbance, and then dividing each value by the average of the values in the control wells (non-treated cells). The percentage of surviving cells was plotted against conjugate concentration and the EC50 of activity was calculated using a nonlinear regression analysis (GraphPad Prims 4.0).

These results show that anti-ADAM9 immunoconjugates can kill a broad panel of ADAM9-positive tumor cell lines, including lung, pancreatic, renal, prostate, and colon tumor cell lines (see FIGs. 9A-9H). In each case tested, a good specificity window is observed, suggesting that cytotoxicity is a result of specific anti-ADAM9 antibody binding to target cells. In particular, the anti-ADAM9-DGN549 conjugates (hMAB-A(2.2)-DGN549 and hMAB-A(2I.2)-S442C-DGN549) were extremely potent with IC50 values ranging from 1 to 115 pM and were ~3 logs more active than a non-targeting IgG1 conjugate (see, Table 15).

## Table 15

| Cell Line | Disease Type | anti-ADAM9 ADC | In Vitro Cytotoxicity ($IC_{50}$ nM) | Fold-specificity ($IC_{50}$ control ADC/ $IC_{50}$ anti-ADAM9 ADC) |
|---|---|---|---|---|
| **NCI-H1703** | NSCLS - Squamous Cell | hMAB-A(2.2)-sSPDB-DM4 | 0.024 | 152 |
| | | hMAB-A(2I.2)-sSPDB-DM4 | 0.028 | 132 |
| | | hMAB-A(2.2)-DGN549 | 0.002 | >1000 |
| | | hMAB-A(2I.2) S442C-DGN549 | 0.004 | >1000 |
| **DLD-1** | Colorectal Adenocarcinoma | hMAB-A(2.2)-sSPDB-DM4 | 2.729 | 9 |
| | | hMAB-A(2I.2)-sSPDB-DM4 | 0.386 | 63 |
| | | hMAB-A(2.2)-DGN549 | 0.010 | >1000 |
| | | hMAB-A(2I.2) S442C-DGN549 | 0.045 | >1000 |
| **A498** | Kidney Carcinoma | hMAB-A(2.2)-sSPDB-DM4 | 0.119 | 27 |

| | | | | |
|---|---|---|---|---|
| | | hMAB-A(2I.2)-sSPDB-DM4 | 0.108 | 30 |
| | | hMAB-A(2.2)-DGN549 | 0.001 | >1000 |
| | | hMAB-A(2I.2) S442C-DGN549 | 0.004 | >1000 |
| DU145 | Prostate Carcinoma | hMAB-A(2.2)-sSPDB-DM4 | 0.570 | 25 |
| | | hMAB-A(2I.2)-sSPDB-DM4 | 0.439 | 32 |
| | | hMAB-A(2.2)-DGN549 | 0.002 | >1000 |
| | | hMAB-A(2I.2) S442C-DGN549 | 0.004 | >1000 |
| HPAF2 | Pancreatic Adenocarcinoma | hMAB-A(2.2)-sSPDB-DM4 | 0.622 | 9 |
| | | hMAB-A(2I.2)-sSPDB-DM4 | 0.422 | 13 |
| | | hMAB-A(2.2)-DGN549 | 0.043 | >1000 |
| | | hMAB-A(2I.2) S442C-DGN549 | 0.115 | >1000 |
| IGROV1 | Ovarian Adenocarcinoma | hMAB-A(2.2)-sSPDB-DM4 | 4.078 | 3 |
| | | hMAB-A(2I.2)-sSPDB-DM4 | 1.411 | 8 |
| | | hMAB-A(2.2)-DGN549 | 0.001 | >1000 |
| | | hMAB-A(2I.2) S442C-DGN549 | 0.003 | >1000 |
| KATO III | Gastric Carcinoma | hMAB-A(2.2)-sSPDB-DM4 | 1.154 | 5 |
| | | hMAB-A(2I.2)-sSPDB-DM4 | 0.480 | 11 |
| | | hMAB-A(2.2)-DGN549 | 0.031 | >1000 |
| | | hMAB-A(2I.2) S442C-DGN549 | 0.065 | >1000 |
| HS578T | Breast Carcinoma | hMAB-A(2.2)-sSPDB-DM4 | 0.119 | 16 |
| | | hMAB-A(2I.2)-sSPDB-DM4 | 0.094 | 20 |
| | | hMAB-A(2.2)-DGN549 | 0.009 | >1000 |
| | | hMAB-A(2I.2) S442C-DGN549 | 0.017 | 646 |

### Example 19

**Anti-tumor Activity of anti-ADAM9 Antibody Drug Conjugates in SCID Mice Bearing Calu3 Human Non-Small Cell Lung Adenocarcinoma Xenografts**

The antitumor activity of varying doses of hMAB-A(2.2)-sSPDB-DM4 and a single dose of chMAB-A-DGN549 conjugates was evaluated in female SCID mice bearing Calu3 cells, a human lung adenocarcinoma subcutaneous xenograft model.

Calu3 cells were harvested for inoculation, with 99% viability determined by trypan blue exclusion. Mice were inoculated with 5 x $10^6$ Calu3 cells in 0.2ml 50% Matrigel/ 50% serum free medium by subcutaneous injection in the area on the right hind flank. Eighty-four female SCID Mice (6 weeks of age) were obtained from Charles River Laboratories. Upon receipt, the animals were observed for 9 days prior to study initiation. Animals showed no sign of disease or illness upon arrival, or prior to treatment.

Eighty-four mice were randomized into 8 groups (8 mice per group) by tumor volume. The tumor volumes ranged from 74.09 to 150.21 (106.41 ± 18.69, Mean ± SD) $mm^3$. The mice were measured and randomized based on the tumor volume on day 6 post implantation. The mice were dosed on day 7 post implantation. Body weight of the mice ranged from 16.05 to 21.72 (18.64 ± 1.03, Mean ± SD) grams. Mice in each group were identified by punch method. Administration of the test agents and vehicle were carried out intravenously by using a 1.0 ml syringe fitted with a 27 gauge, ½ inch needle. The groups included: a control group dosed with vehicle (PBS), chKTI-sSPDB-DM4 at 5 mg/kg, qdx1, hMAB-A(2.2)-sSPDB-DM4 at 1.25 mg/kg (by antibody), qdx1, hMAB-A(2.2)-sSPDB-DM4 at 2.5 mg/kg (by antibody), qdx1, hMAB-A(2.2)-sSPDB-DM4 at 5 mg/kg (by antibody), qdx1, and chMAB-A-DGN549 at 10μg/kg (by payload; 0.6 mg/kg by antibody), qdx1.

Tumor size was measured two times per week in three dimensions using a caliper. The tumor volume was

expressed in $mm^3$ using the formula V = Length x Width x Height x ½. A mouse was considered to have a partial regression (PR) when tumor volume was reduced by 50% or greater, complete tumor regression (CR) when no palpable tumor could be detected and tumor-free survivor (TFS) is the number of mice tumor free at the end of the study. Tumor volume and body weight were determined by StudyLog software. $Log_{10}$ cell kill (LCK) was calculated with the formula LCK = (T - C) / $T_d$ x 3.32, where (*T* - C), or tumor growth delay (TGD), is the median time (in days) for the treatment group and control group tumors to reach a predetermined size of 815 $mm^3$ (tumor-free survivors excluded) [1], $T_d$ is the tumor doubling time in mice (estimated from nonlinear exponential curve fit of daily median of control tumor growth) and x is the number of cell doublings per log of cell growth. %T/C (tumor growth inhibition) is the ratio of the median tumor volume (TV) of the treatment group at a predetermined time (i.e., time when median TV for control tumors reach to the maximum tumor volume, i.e., TV@ ~1000$mm^3$, or when tumors became necrotic, thus this is the time point when all control group mice came off the study) to the median TV of the controls at the end point for controls.

Body weight of all the mice was measured two times per week as a rough index of drug toxicity. Body weights (BW) of mice were expressed as percent change in body weight from the pre-treatment body weight as follows: % BW change = [(BW post / BW pre) - 1] x 100, where BW post is weight after treatment and BW pre is the starting body weight prior to treatment. Percent body weight loss (BWL) was expressed as the mean change in body weight post treatment. Animals were sacrificed when the tumor volume was larger than 1000 mm3 or necrotic, or if body weight dropped by 20% more at any point in the study.

The results of the study are shown in FIG. 10. hMAB-A(2.2)-sSPDB-DM4 conjugate was active at all doses tested. hMAB-A(2.2)-sSPDB-DM4 conjugate had a tumor growth inhibition (T/C) value of 39%, T-C value of 17, and LCK value of 0.5 at 1.25mg/kg dose (with 3 partial regression out of 8mice and complete regressions); a T/C value of 25%, T-C value of 22, and LCK value of 0.7 at 2.5mg/kg (with 3 partial regression out of 8 mice and no complete regressions), and a T/C value of 14%, T-C value of 36, and LCK value of 1.1 at 5mg/kg dose ( with 4 partial regressions out of 8 mice and 1 complete regressions out of 8 mice). chMAB-A-DGN549 was highly active at 10μg/kg (by payload; 0.6 mg/kg by antibody) with 8 complete regressions out of 8 mice. No significant body weight loss was observed with either the hMAB-A(2.2)-sSPDB-DM4, or the chMAB-DGN549, or the chKTI-sSPDB-DM4 conjugates at the indicated doses, and thus all three conjugates were well tolerated in mice at the indicated doses in this study. The results show that hMAB-A(2.2)-sSPDB-DM4 conjugate is active at varying doses in this model. The results also show that chMAB-A-DGN549 is highly active at 10 μg/kg (by payload; 0.6 mg/kg by antibody).

The antitumor activity of various doses of hMAB-A(2I.2)-S442C-DGN549 as a single i.v. dose was also evaluated in female SCID mice bearing Calu-3 tumor xenografts, a NSCLC model. Six days post inoculation, mice were randomized into 8 groups (n=8 per group) by tumor volume. The treatment groups included a control group dosed with vehicle (PBS), hMAB-A(2I.2)-S442C-DGN549 conjugate dosed at 0.5 (by payload; 0.04 mg/kg by antibody), 1 (by payload; 0.08 mg/kg by antibody), 3 (by payload; 0.25 mg/kg by antibody) and 10μg/kg (by payload; 0.8 mg/kg by antibody) as a single i.v. dose, as well as a non-targeting control of huKTI-S442C-DGN549 conjugate at 1 (by payload; 0.08 mg/kg by antibody), 3 (by payload; 0.25 mg/kg by antibody) and 10μg/kg (by payload; 0.8 mg/kg by antibody) dosed as a single i.v. dose.

Tumor volumes and body weights were measured as described above and the results are shown in FIG. 11. Briefly, hMAB-A(2I.2)-S442C-DGN549 was highly active at even the lowest dose of 0.5μg/kg (by payload; 0.04 mg/kg by antibody) and potent activity was observed at 1 (by payload; 0.08 mg/kg by antibody), 3 (by payload; 0.25 mg/kg by antibody), 10μg/kg (by payload; 0.8 mg/kg by antibody) doses. Non-targeting control, huKTI-S442C-DGN549 was inactive in this model at all three doses tested, i.e. 1 (by payload; 0.08 mg/kg by antibody), 3 (by payload; 0.25 mg/kg by antibody) and 10ug/kg (by payload; 0.8 mg/kg by antibody). hMAB-A(2I.2)-S442C-DGN549 at 0.5μg/kg (by payload; 0.04 mg/kg by antibody) had a T/C of 7 %, with 7/8 PR, and 0/8 CR at the study endpoint. hMAB-A(2I.2)-S442C-DGN549 at 1μg/kg (by payload; 0.08 mg/kg by antibody) had a T/C of 2 %, with 8/8 PR, and 2/8 CR at the study endpoint. hMAB-A(2I.2)-S442C-DGN549 at 3μg/kg (by payload; 0.25 mg/kg by antibody) had a T/C of 0 %, with 8/8 PR, and 8/8 CR at the study endpoint, and hMAB-A(2I.2)-S442C-DGN549 at 10μg/kg (by payload; 0.8 mg/kg by antibody) had a T/C of 0 %, with 8/8 PR, and 8/8 CR at the study endpoint.

Both hMAB-A(2I.2)-S442C-DGN549 (at 0.5, 1, 3, 10μg/kg, by payload), and huKTI-S442C-DGN549 (at 1,3,10μg/kg, by payload) were well tolerated in mice at all the doses indicated in this study. No significant body weight loss was observed with any of the two conjugates at the indicated doses. The results from this study suggest that hMAB-A(21.2)-S442C-DGN549 conjugate demonstrated compelling anti-tumor activity and is highly efficacious in Calu-3 non-small cell lung cancer tumor xenograft model.

**Example 20**

**Anti-tumor Activity of anti-ADAM9 Antibody Drug Conjugates in SCID Mice Bearing H1703 Non-Small Cell Lung Squamous Cell Carcinoma Xenografts**

The antitumor activity of varying doses of hMAB-A(2.2)-sSPDB-DM4 conjugate was evaluated in female SCID

mice bearing H1703 cells, a NSCLC squamous cell carcinoma xenograft model.

H1703 cells were harvested for inoculation, with 100% viability determined by trypan blue exclusion. Mice were inoculated with 5 x $10^6$ H1703 cells in 0.2ml 50% Matrigel/ 50% serum free medium by subcutaneous injection in the area on the right hind flank. Seventy-two female nude mice (6 weeks of age) were obtained from Charles River Laboratories on 3/24/16. Upon receipt, the animals were observed for 8 days prior to study initiation. Animals showed no sign of disease or illness upon arrival, or prior to treatment. Forty eight mice were randomized into 8 groups (6 mice per group) by tumor volume. The tumor volumes ranged from 76.18 to 159.00 (115.19 ± 22.08, Mean ± SD) $mm^3$. The mice were measured and randomized based on the tumor volume on day 19 post implantation. The mice were dosed on day 20 post implantation (4/21/16). Body weight of the mice ranged from 20.20 to 29.15 (23.88 ± 1.95, Mean ± SD) grams. Mice in each group were identified by punch method. Administration of the test agents and vehicle were carried out intravenously by using a 1.0 ml syringe fitted with a 27 gauge, ½ inch needle. The groups included: a control group dosed with vehicle (PBS) at 150μL/mouse, qdx1, chKTI-sSPDB-DM4 at 5 mg/kg, qdx1, hMAB-A(2.2)-sSPDB-DM4 at 1.25 mg/kg, qdx1, hMAB-A(2.2)-sSPDB-DM4 at 2.5 mg/kg, qdx1, and hMAB-A(2.2)-sSPDB-DM4 at 5 mg/kg, qdx1.

Tumor size and body weight measurements were determined as described in Example 19. The results of the study are shown in FIG. 12. hMAB-A(2.2)-sSPDB-DM4 was highly active at a low dose of 2.5 mg/kg with a T/C of 6%, T-C of 28, and LCK of 1.4 (with 3 partial regressions out of 6 mice and no complete regressions). and highly active at 5mg/kg doses with a T/C of 1%, T-C of 63, and LCK of 3.2 (with 6 partial regressions out of 6 mice, 3 complete regressions out of 6 mice and 1 tumor free survivor). hMAB-A(2.2)-sSPDB-DM4 conjugate was inactive at 1.25mg/kg dose, and had a tumor growth inhibition (T/C) of 79%, T-C of 4, and LCK of 0.2 (with 1 partial regression out of 6 mice and no complete regressions). No significant body weight loss was observed with either the hMAB-A(2.2)-sSPDB-DM4 or the chKTI-sSPDB-DM4 conjugates at the indicated doses, and thus both conjugates were well tolerated in mice at the indicated doses in this study. The results show that hMAB-A(2.2)-sSPDB-DM4 conjugate is highly active at doses as low as 2.5 mg/kg in this model.

## Example 21

### Anti-tumor Activity of anti-ADAM9 Antibody Drug Conjugates in SCID Mice Bearing Detroit562 Squamous Cell Carcinoma of the Head and Neck Xenografts

The antitumor activity of varying doses of chMAB-A-sSPDB-DM4 conjugates was evaluated in female CD-1 immunodeficient mice bearing Detroit562 cells, a pharyngeal carcinoma xenograft model.

Mice (6 weeks of age) were obtained from Charles River Laboratories on 7/1/14. Upon receipt, the animals were observed for 15 days prior to study initiation. Animals showed no sign of disease or illness upon arrival, or prior to study start. Detroit562 cells were harvested for inoculation, with greater than 90% viability determined by trypan blue exclusion. Mice were inoculated with 5 x $10^6$ Detroit562 cells in 0.2ml 50% Matrigel/50% serum free medium by subcutaneous injection in the area on the right hind flank.

Forty-two mice were randomized into 6 groups (7 mice per group) by tumor volume. The tumor volume ranged from 95.76 to 450.83 $mm^3$. The mice were measured and randomized based on the tumor volume on day 26 post implantation. Administration of the test agents and vehicle were carried out on the day following randomization (Study Day 0) by intraperitoneal injection using a 1.0 ml syringe fitted with a 27 gauge, ½ inch needle. The groups included: a control group dosed with vehicle (PBS), chKTI-sSPDB-DM4 at 5 mg/kg, qdx1, chKTI-sSPDB-DM4 at 2.5 mg/kg, qdx1, chMAB-A-sSPDB-DM4 at 5 mg/kg, qdx1, chMAB-A-sSPDB-DM4 at 2.5 mg/kg, qdx1, chMAB-A-sSPDB-DM4 at 1.25 mg/kg, qdx1.

Tumor size and body weight measurements were determined as described in Example 19. The results of the study are shown in FIG. 13. Briefly, chMAB-A-sSPDB-DM4 was active at all 3 of the tested dose levels (5, 2.5 and 1.25 mg/kg) over the course of the study. chMAB-A-sSPDB-DM4 at 5 mg/kg had a T/C of 32%, and 6/7 PR and 1/7 CR at the study endpoint. chMAB-A-sSPDB-DM4 at 2.5 mg/kg had a T/C of 27%, and 6/7 PR and 2/7 CR at the study endpoint. chMAB-A-sSPDB-DM4 at 1.25 mg/kg had a T/C of 37%, and 5/7 PR and 0/7 CR at the study endpoint. chKTI-sSPDB-DM4 at 5 mg/kg had a T/C of 89%, and 3/7 PR and 0/7 CR at the study endpoint. chKTI-sSPDB-DM4 at 5 mg/kg had a T/C of 80%, and 3/7 PR and 1/7 CR at the study endpoint.

chMAB-A-sSPDB-DM4 was well tolerated in mice at all doses examined in this study. No significant body weight loss was observed at the indicated doses. The results from this study suggest that chMAB-A-sSPDB-DM4 conjugate demonstrates anti-tumor activity and is efficacious in Detroit562 pharyngeal carcinoma tumor xenograft model.

In a second study, the antitumor activity of varying doses of chMAB-A-sSPDB-DM4 conjugates was evaluated in female CD-1 immunodeficient mice bearing larger volume Detroit562 tumor xenografts.

Mice (5 weeks of age) were obtained from Charles River Laboratories on 9/17/14. Upon receipt, the animals were observed for 7 days prior to study initiation. Animals showed no sign of disease or illness upon arrival, or prior to study start. Detroit562 cells were harvested for inoculation, with greater than 90% viability determined by trypan blue

exclusion. Mice were inoculated with 5 x $10^6$ Detroit562 cells in 0.2ml 50% Matrigel/50% serum free medium by subcutaneous injection in the area on the right hind flank.

Eighteen mice were randomized into 3 groups (6 mice per group) by tumor volume. The tumor volume ranged from 277.51 to 503.49 $mm^3$. The mice were measured and randomized based on the tumor volume on day 35 post implantation. Administration of the test agents and vehicle were carried out two days following randomization (Study Day 1) by intraperitoneal injection using a 1.0 ml syringe fitted with a 27 gauge, ½ inch needle. The groups included: a control group dosed with vehicle (PBS), chMAB-A-sSPDB-DM4 at 5 mg/kg, qdx1 and chMAB-A-sSPDB-DM4 at 1.25 mg/kg, qdx1.

Tumor size and body weight measurements were determined as described in Example 19. The results of the study are shown in FIG. 14. Briefly, chMAB-A-sSPDB-DM4 was active at the 2 tested dose levels (5 and 1.25 mg/kg) over the course of the study. chMAB-A-sSPDB-DM4 at 5 mg/kg had a T/C of 3%, and 6/6 PR and 1/7 CR at the study endpoint. chMAB-A-sSPDB-DM4 at 1.25 mg/kg had a T/C of 14%, and 6/6 PR and 0/7 CR at the study endpoint.

chMAB-A-sSPDB-DM4 was well tolerated in mice at both doses examined in this study. No significant body weight loss was observed at the indicated doses. The results from this study suggest that chMAB-A-sSPDB-DM4 conjugate demonstrates anti-tumor activity and is efficacious in larger volume Detroit562 pharyngeal carcinoma tumor xenograft model.

**Example 22**

**Anti-tumor Activity of anti-ADAM9 Antibody Drug Conjugates in Nude Mice Bearing SNU-5 Gastric Carcinoma Xenografts**

The antitumor activity of varying doses of huMAB-A(2.2)-sSPDB-DM4 and a single dose of huMAB-A(2I.2)-S442C-DGN549 conjugates was evaluated in female Nude mice bearing Snu-5 cells, a human gastric carcinoma xenograft model.

Snu-5 cells were harvested for inoculation, with 100% viability determined by trypan blue exclusion. Mice were inoculated with 5 x $10^6$ Snu-5 cells in 0.1ml 50% Matrigel/ 50% serum free medium by subcutaneous injection in the area on the right hind flank. Fifty-six female Nude Mice (6 weeks of age) were obtained from Charles River Laboratories on 1/25/17. Upon receipt, the animals were observed for 8 days prior to study initiation. Animals showed no sign of disease or illness upon arrival, or prior to treatment.

Fifty-six mice were randomized into 7 groups (8 mice per group) by tumor volume. The tumor volumes ranged from 76.29 to 129.12 (130.58 $\pm$ 13.28, Mean $\pm$ SD) $mm^3$. The mice were measured and randomized based on the tumor volume on day 13 post implantation. The mice were dosed on day 14 post implantation (2/17/17). Body weight of the mice ranged from 19.50 to 26.35 (22.69 $\pm$ 1.40, Mean $\pm$ SD) grams. Mice in each group were identified by punch method. Administration of the test agents and vehicle were carried out intravenously by using a 1.0 ml syringe fitted with a 27 gauge, ½ inch needle. The groups included: a control group dosed with vehicle (PBS), chKTI-sSPDB-DM4 at 5 mg/kg, huMAB-A(2.2)-sSPDB-DM4 at 2.5 mg/kg, huMAB-A(2.2)-sSPDB-DM4 at 5 mg/kg, huKTI-S442C-DGN549 at 10$\mu$g/kg, huMAB-A(2I.2)-S442C-DGN549 at 3$\mu$g/kg and huMAB-A(2I.2)-S442C-DGN549 at 10$\mu$g/kg.

Tumor size was measured two times per week in three dimensions using a caliper. The tumor volume was expressed in $mm^3$ using the formula V = Length x Width x Height x ½. A mouse was considered to have a partial regression (PR) when tumor volume was reduced by 50% or greater, complete tumor regression (CR) when no palpable tumor could be detected and tumor-free survivor (TFS) is the number of mice tumor free at the end of the study. Tumor volume and body weight were determined by StudyLog software. $Log_{10}$ cell kill (LCK) was calculated with the formula LCK = (T - C) / $T_d$ x 3.32, where ($T$ - C), or tumor growth delay (TGD), is the median time (in days) for the treatment group and control group tumors to reach a predetermined size of 798 $mm^3$ (tumor-free survivors excluded) [1], $T_d$ is the tumor doubling time in mice (estimated from nonlinear exponential curve fit of daily median of control tumor growth) and x is the number of cell doublings per log of cell growth. %T/C (tumor growth inhibition) is the ratio of the median tumor volume (TV) of the treatment group at a predetermined time (i.e., time when median TV for control tumors reach to the maximum tumor volume, i.e., TV@ ~1000$mm^3$, or when tumors became necrotic, thus this is the time point when all control group mice came off the study) to the median TV of the controls at the end point for controls.

Body weight of all the mice was measured two times per week as a rough index of drug toxicity. Body weights (BW) of mice were expressed as percent change in body weight from the pre-treatment body weight as follows: % BW change = [(BW post / BW pre) - 1] x 100, where BW post is weight after treatment and BW pre is the starting body weight prior to treatment. Percent body weight loss (BWL) was expressed as the mean change in body weight post treatment. Animals were sacrificed when the tumor volume was larger than 1000 mm3 or necrotic, or if body weight dropped by 20% more at any point in the study.

The results of the study are shown in FIG. 15 and FIG. 16. huMAB-A(2.2)-sSPDB-DM4 conjugate was highly active at both doses tested. huMAB-A(2.2)-sSPDB-DM4 conjugate had a tumor growth inhibition (T/C) value of 5%, at

2.5mg/kg dose (with 8 partial regression out of 8mice and 2 complete regressions); a T/C value of 1%, 5mg/kg (with 8 partial regression out of 8 mice and 6 complete regressions). huMAB-A(2I.2)-S442C-DGN549 was highly active at both doses tested. huMAB-A(2I.2)-S442C-DGN549 conjugate had a (T/C) value of 7%, at 3μg/kg dose (with 8 partial regression out of 8 mice and 4 complete regressions); a T/C value of 4%, 5mg/kg (with 8 partial regression out of 8 mice and 5 complete regressions). No significant body weight loss was observed with either the huMAB-A(2.2)-sSPDB-DM4, or the huMAB-A(2I.2)-S442C-DGN549, or the chKTI-sSPDB-DM4, or the huKTI-S442C-DGN549 conjugates at the indicated doses, and thus all conjugates were well tolerated in mice at the indicated doses in this study.

**Example 23**

**Anti-tumor Activity of anti-ADAM9 Antibody Drug Conjugates in Mice Bearing SW48 Colorectal Cancer Cell Line Xenografts**

The antitumor activity of varying doses of hMAB-A(2.2)-sSPDB-DM4 and huMAB-A(2I.2)-S442C-DGN649 conjugates were evaluated in female Nude mice bearing SW48 cells, a colorectal cancer cell line xenograft model.

SW48 cells were harvested for inoculation. Mice were inoculated with SW48 cells by subcutaneous injection in the area on the right hind flank. Animals showed no sign of disease or illness upon arrival from laboratories, or prior to treatment.

Fourty-two mice were randomized into 7 groups (6 mice per group) by tumor volume. The tumor volumes ranged from 89 to 169 $mm^3$. The mice were measured, randomized and dosed based on the tumor volume on day 17 post implantation. Mice in each group were identified by ear tags. Administration of the test agents and vehicle were carried out intravenously. The groups included: a control group dosed with vehicle (PBS), chKTI-sSPDB-DM4 at 10 mg/kg, huMAB-A(2.2)-sSPDB-DM4 at 5 mg/kg, huMAB-A(2.2)-sSPDB-DM4 at 10mg/kg, huKTI-S442C-DGN549 at 5μg/kg, huMAB-A(2I.2)-S442C-DGN549 at 2.5 μg/kg and huMAB-A(2I.2)-S442C-DGN549 at 5μg/kg.

Tumor size and body weight measurements were determined as described in Example 22. The results of the study are shown in FIG. 17 and FIG. 18. huMAB-A(2.2)-sSPDB-DM4 conjugate was active at both doses tested. huMAB-A(2.2)-sSPDB-DM4 conjugate had a tumor growth inhibition (T/C) value of 13%, at 5mg/kg dose (with no partial or complete regressions); a T/C value of 5%, 10mg/kg (with 6 partial regression out of 6 mice). huMAB-A(2I.2)-S442C-DGN549 was active at 5μg/kg dose and inactive at 2.5μg/kg dose tested. huMAB-A(2I.2)-S442C-DGN549 conjugate had a (T/C) value of 92%, at 2.5μg/kg dose; a T/C value of 41%, 5μg/kg (with 2 partial regression out of 6 mice). No significant body weight loss was observed with either the huMAB-A(2.2)-sSPDB-DM4, or the huMAB-A(2I.2)-S442C-DGN549, or the chKTI-sSPDB-DM4, or the huKTI-S442C-DGN549 conjugates at the indicated doses, and thus all conjugates were well tolerated in mice at the indicated doses in this study (needs to be confirmed by MGNX).

**Example 24**

**Extending the Exposure of Anti-ADAM9 Antibody Maytansinoid Conjugates by Improving FcRn Binding**

To maximize the potential of anti-ADAM9 antibody maytansinoid conjugates to drive efficacy, substitutions M252Y, S254T, and T256E ("the YTE mutation") were introduced in the Fc region of anti-ADAM9 antibodies, hMAB-A(2I.2) and hMAB-A(2I.2)-S442C, to extend exposure of the antibody drug conjugates by improving FcRn binding. FcRn plays a major role in the maintenance of serum IgG levels. IgGs that are non-specifically endocytosed bind to FcRn in the endosome due to a pH-dependent interaction. FcRn-bound IgG is then sorted into recycling endosomes and transported back to the cell surface where the IgG is released due to the neutral pH. Enhancing FcRn binding can improve sorting and improved pharmacokinetics.

hMAB-A(2I.2)-YTE-sSPDB-DM4 conjugate was prepared as detailed in Example 17a. hMAB-A(2I.2)-S442C-YTE-sSPDB-DM4 conjugate was prepared as previously described in PCT Publication No. WO2017/004025. As seen in Figures 19A and 19B, the YTE mutation increased binding affinity and avidity of the hMAB-A(2I.2) antibody to human and cyno FcRn. Moreover, the hMAB-A(2I.2)-YTE-sSPDB-DM4 conjugate showed similar in vitro and in vivo activity in NCI-H1703, SNU5, and Calu3 as the parental hMAB-A(2I.2)-sSPDB-DM4 conjugate.

Cynomolgus PK and tolerability studies were performed to characterize the tolerability and pharmacokinetic profile of the hMAB-A(2I.2)-YTE-sSPDB-DM4 conjugate compared to the parental hMAB-A(2I.2)-sSPDB-DM4 conjugate. Both conjugates were administered as 10-minute IV infusions to male cynomolgus monkeys at a dose of 4 and 12 mg/kg (antibody dose) to assess both PK profile and acute tolerability. In addition, the naked antibodies, hMAB-A(2I.2) and hMAB-A(2I.2)-YTE, were administered to male cynomolgus monkeys at a dose of 4 mg/kg to address potential effects of conjugation on PK parameters. Blood was collected over the course of 28 days to allow for the measurement of ADC, total antibody and free payload concentrations. Tolerability was determined based on survival, clinical observations, body weights, and clinical pathology.

As seen in Figures 20, cynomolgus PK data of hMAB-A(2I.2)-YTE-sSPDB-DM4 conjugate compared to parental conjugate revealed that the YTE mutation increased the exposure compared to the parental conjugate. In addition, cynomolgus PK data of the naked hMAB-A(2I.2) antibody and hMAB-A(2I.2)-YTE antibody showed that conjugation has minimal effect on the PK parameters and that the YTE mutation improves both naked antibody and conjugate PK parameters. Furthermore, hMAB-A(2I.2)-sSPDB-DM4 and hMAB-A(2I.2)-YTE-sSPDB-DM4 conjugate were well tolerated up to a dose of 12 mg/kg with minimal skin clinical observations and dose dependent effects on hematology parameters.

While the invention has been described in connection with specific embodiments thereof, it will be understood that it is capable of further modifications within the scope of the appended claims.

## Claims

1. An immunoconjugate comprising a humanized anti-ADAM9 antibody or ADAM9-binding fragment thereof that specifically binds to human ADAM9 and cyno ADAM9:

    (I) wherein said humanized anti-ADAM9 antibody or ADAM9-binding fragment thereof is conjugated to a pharmacological agent; and
    (II) wherein said humanized anti-ADAM9 antibody or ADAM9-binding fragment thereof comprises a Light Chain Variable (VL) Domain and a Heavy Chain Variable (VH) Domain, wherein said Heavy Chain Variable Domain comprises a $CDR_H1$ Domain, a $CDR_H2$ Domain and a $CDR_H3$ Domain, and said Light Chain Variable Domain comprises a $CDR_L1$ Domain, a $CDR_L2$ Domain, and a $CDR_L3$ Domain, wherein:

        (A) said $CDR_H1$ Domain comprises the amino acid sequence **of SEQ ID NO:8** or **SEQ ID NO:34;** and
        (B) said $CDR_H2$ Domain comprises the amino acid sequence of **SEQ ID NO: 9, SEQ ID NO:35** or **SEQ ID NO:36;** and
        (C) said $CDR_H3$ Domain comprises amino acid sequence of any one **of SEQ ID NO:37, SEQ ID NO:38, SEQ ID NO:39, SEQ ID NO:40, SEQ ID NO:41, SEQ ID NO:42, SEQ ID NO:43, SEQ ID NO:44, SEQ ID NO:45 or SEQ ID NO:46;** and

    wherein:

        (A) said $CDR_L1$ Domain has the amino acid sequence **of SEQ ID NO:12, SEQ ID NO:62, SEQ ID NO:63,** or **SEQ ID NO:64;** and
        (B) said $CDR_L2$ Domain has the amino acid sequence of **SEQ ID NO:13;** and
        (C) said $CDR_L3$ Domain has the amino acid sequence of **SEQ ID NO:14** or **SEQ ID NO:65.**

2. The immunoconjugate of claim 1, wherein said humanized anti-ADAM9 antibody or ADAM9-binding fragment thereof comprises a $CDR_H1$ domain, a $CDR_H2$ domain, and a $CDR_H3$ domain and a $CDR_L1$ domain, a $CDR_L2$ domain, and a $CDR_L3$ domain having the sequences selected from the group consisting of:

    (a) SEQ ID NOs: 8, 35, and 45 and SEQ ID NOs: 62, 13, 14, respectively;
    (b) SEQ ID NOs: 8, 35, and 38 and SEQ ID NOs: 62, 13, 14, respectively;
    (c) SEQ ID NOs: 8, 35, and 39 and SEQ ID NOs: 62, 13, 14, respectively;
    (d) SEQ ID NOs: 8, 35, and 40 and SEQ ID NOs: 62, 13, 14, respectively;
    (e) SEQ ID NOs: 8, 35, and 41 and SEQ ID NOs: 62, 13, 14, respectively;
    (f) SEQ ID NOs: 8, 35, and 42 and SEQ ID NOs: 62, 13, 14, respectively;
    (g) SEQ ID NOs: 8, 35, and 43 and SEQ ID NOs: 62, 13, 14, respectively;
    (h) SEQ ID NOs: 8, 35, and 44 and SEQ ID NOs: 62, 13, 14, respectively;
    (i) SEQ ID NOs: 8, 35, and 37 and SEQ ID NOs: 62, 13, 14, respectively; and
    (j) SEQ ID NOs: 8, 35, and 46 and SEQ ID NOs: 62, 13, 14, respectively.

3. The immunoconjugate of claim 1 or 2, wherein:

    (A) said VH Domain comprises **SEQ ID NO:20, SEQ ID NO:21, SEQ ID NO:22, SEQ ID NO:23, SEQ ID NO:24, SEQ ID NO:25, SEQ ID NO:26, SEQ ID NO:27, SEQ ID NO:28** or **SEQ ID NO:29;** and
    (B) said VL Domain comprises **SEQ ID NO:54, SEQ ID NO:55, SEQ ID NO:56** or **SEQ ID NO:57.**

**4.** The immunoconjugate of claim 1, wherein said $CDR_H1$ Domain comprises the amino acid sequence SYWMH **(SEQ ID NO:8),** said $CDR_H2$ Domain comprises the amino acid sequence EIIPIFGHTNYNEKFKS **(SEQ ID NO:35),** said $CDR_H3$ Domain comprises the amino acid sequence GGYYYYPRQGFLDY **(SEQ ID NO:45),** said $CDR_L1$ Domain comprises the amino acid sequence KASQSVDYSGDSYMN **(SEQ ID NO:62),** said $CDR_L2$ Domain comprises the amino acid sequence AASDLES **(SEQ ID NO:13),** and said $CDR_L3$ Domain comprises the amino acid sequence QQSHEDPFT **(SEQ ID NO:14).**

**5.** The immunoconjugate of claim 4, wherein said immunoconjugate comprises:

(A) the Heavy Chain Variable (VH) Domain of hMAB-A **(2I.2) (SEQ ID NO:28);** and/or
(B) the Light Chain Variable (VL) Domain of hMAB-A **(2I.2)** (**SEQ ID NO:55**); and/or
(C) the Heavy Chain Variable (VH) Domain of hMAB-A **(2I.2) (SEQ ID NO:28)** and the Light Chain Variable (VL) Domain of hMAB-A **(2I.2) (SEQ ID NO:55).**

**6.** The immunoconjugate of any one of claims 1-5, wherein said immunoconjugate comprises an Fc Region, optionally wherein said Fc Region is a variant Fc Region that comprises:

(a) one or more amino acid modification(s) that reduce(s) the affinity of the variant Fc Region for an FcγR; and/or
(b) one or more amino acid modification(s) that introduces a cysteine residue; and/or
(c) one or more amino acid modification(s) that extends(s) the serum half-life.

**7.** The immunoconjugate of claim 6, wherein

(I) said one or more amino acid modification(s) that reduce(s) the affinity of the variant Fc Region for an FcγR comprise:

(A) L234A;
(B) L235A; or
(C) L234A and L235A; and

(II) said one or more amino acid modification(s) that that introduces a cysteine residue comprises S442C; and
(III) said one or more amino acid modification(s) that that extends(s) the serum half-life comprise:

(A) M252Y;
(B) M252Y and S254T;
(C) M252Y and T256E;
(D) M252Y, S254T and T256E; or
(E) K288D and H435K; and

wherein said numbering is that of the EU index as in Kabat.

**8.** The immunoconjugate of any one of claims 1-7, wherein said humanized anti-ADAM9 antibody comprises a heavy chain and a light chain having the sequences selected from the group consisting of:

(a) SEQ ID NO:152 and SEQ ID NO:68, respectively;
(b) SEQ ID NO:141 and SEQ ID NO:68, respectively;
(c) SEQ ID NO:142 and SEQ ID NO:68, respectively;
(d) SEQ ID NO:143 and SEQ ID NO:68, respectively;
(e) SEQ ID NO:151 and SEQ ID NO:68, respectively;
(f) SEQ ID NO:52 and SEQ ID NO:68, respectively;
(g) SEQ ID NO:153 and SEQ ID NO:68, respectively; and
(h) SEQ ID NO:154 and SEQ ID NO:68, respectively.

**9.** The immunoconjugate of claim 8, wherein X in SEQ ID NO:141, SEQ ID NO:142, SEQ ID NO:143, SEQ ID NO:151, SEQ ID NO:152, SEQ ID NO:153 or SEQ ID NO:154 is absent.

**10.** The immunoconjugate of any one of claims 1-9, wherein said the humanized anti-ADAM9 antibody comprises a heavy chain and a light chain having the sequences of SEQ ID NO:152 and SEQ ID NO:68, respectively.

**11.** The immunoconjugate of claim 1, wherein said humanized anti-ADAM9 antibody or ADAM9 binding fragment thereof comprises a heavy chain variable domain (VH) and a light chain variable domain (VL) having the sequences of SEQ ID NO:28 and SEQ ID NO:55, respectively.

**12.** The immunoconjugate of any one of claims 1-11, wherein said pharmacological agent is a maytansinoid compound, a pyrrolobenzodiazepine compound, or an indolinobenzodiazepine compound.

**13.** The immunoconjugate of claim 12, wherein said pharmacological agent is a maytansinoid compound.

**14.** The immunoconjugate of any one of claims 1-13, wherein the immunoconjugate is represented by a formula selected from the group consisting of:
(I)

$$CBA\text{—}(\text{—}Cy^{L1})_{W_L},$$

wherein:

CBA is said humanized anti-ADAM9 antibody or ADAM9-binding fragment thereof that is covalently linked to $Cy^{L1}$ through a lysine residue;
$W_L$ is an integer from 1 to 20; and
$Cy^{L1}$ is represented by the following formula:

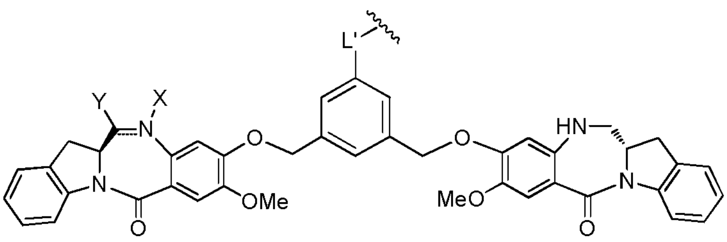

, or

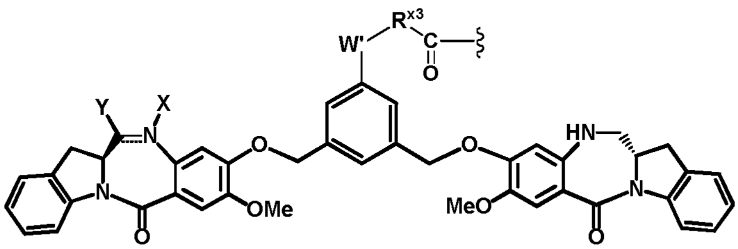

or a pharmaceutically acceptable salt thereof, wherein:

the double line



between N and C represents a single bond or a double bond, provided that when it is a double bond, X is absent and Y is -H or a $(C_1\text{-}C_4)$alkyl; and when it is a single bond, X is -H or an amine protecting moiety, and Y is -OH or $-SO_3H$ or a pharmaceutically acceptable salt thereof;
W' is $-NR^{e'}$,
$R^{e'}$ is $-(CH_2\text{-}CH_2\text{-}O)_n\text{-}R^k$;
n is an integer from 2 to 6;
$R^k$ is -H or -Me;
$R^{x3}$ is a $(C_1\text{-}C_6)$alkyl;

L' is represented by the following formula:

- $NR_5$-P-C(=O)-$(CR_aR_b)_m$-C(=O)- (B1'); or
- $NR_5$-P-C(=O)-$(CR_aR_b)_m$-S-$Z^{s1}$- (B2');

$R_5$ is -H or a $(C_1-C_3)$alkyl;
P is an amino acid residue or a peptide containing between 2 to 20 amino acid residues;
$R_a$ and $R_b$, for each occurrence, are each independently -H, $(C_1-C_3)$alkyl, or a charged substituent or an ionizable group Q;
m is an integer from 1 to 6; and
$Z^{s1}$ is selected from any one of the following formulas:

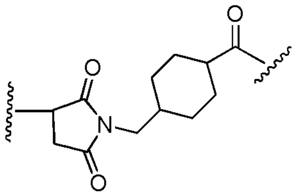
(b1);
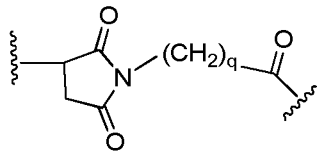
(b2);
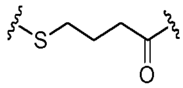
(b3);
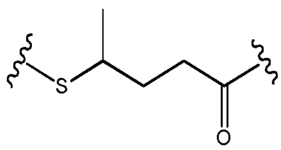
(b4);
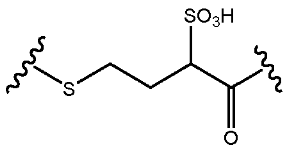
(b5),
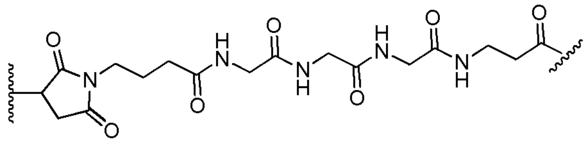
(b6),
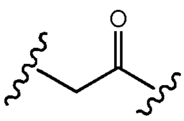
(b7);
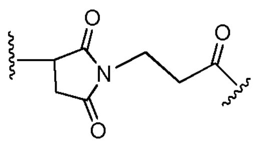
(b8);
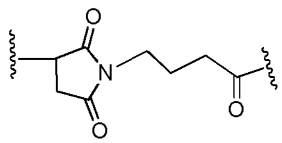
(b9);

and

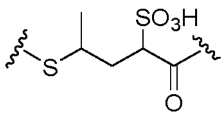
(b10),

wherein q is an integer from 1 to 5, and optionally wherein the pharmaceutically acceptable salt is a sodium or a potassium salt;

(II)

$$CBA\left(-Cy^{L2}\right)_{W_L},$$

wherein:

CBA is said humanized anti-ADAM9 antibody or ADAM9-binding fragment thereof that is covalently linked to $Cy^{L2}$ through a lysine residue;
$W_L$ is an integer from 1 to 20; and

$Cy^{L2}$ is represented by the following formula:

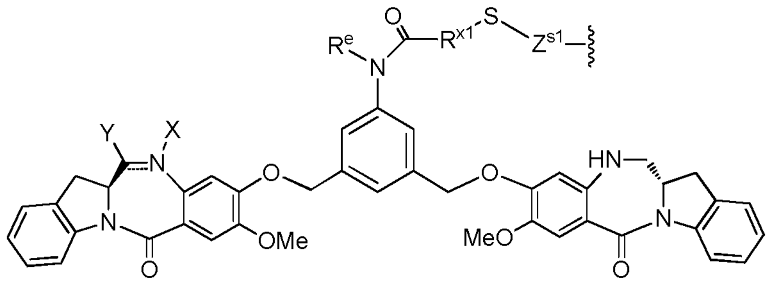

;

or

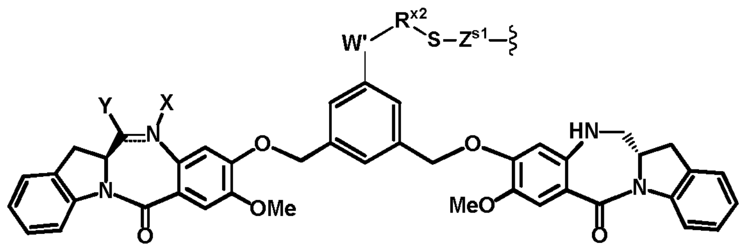

;

or a pharmaceutically acceptable salt thereof, wherein:

the double line



between N and C represents a single bond or a double bond, provided that when it is a double bond, X is absent and Y is -H or a $(C_1\text{-}C_4)$alkyl; and when it is a single bond, X is -H or an amine protecting moiety, and Y is -OH or $-SO_3H$;

$R^{x1}$ and $R^{x2}$ are independently $(C_1\text{-}C_6)$alkyl;

$R^e$ is -H or a $(C_1\text{-}C_6)$alkyl;

W' is $-NR^{e'}$,

$R^{e'}$ is $-(CH_2\text{-}CH_2\text{-}O)_n\text{-}R^k$;

n is an integer from 2 to 6;

$R^k$ is -H or -Me;

$Z^{s1}$ is selected from any one of the following formulas:

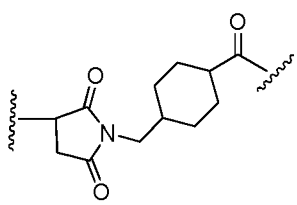

(b1);

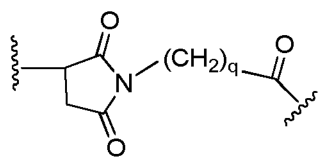

(b2);

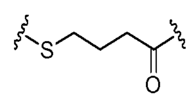

(b3);

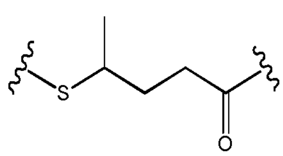

(b4);

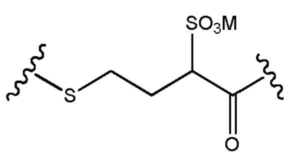

(b5),

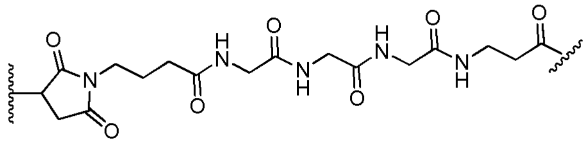

(b6),

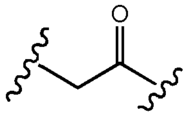 (b7); 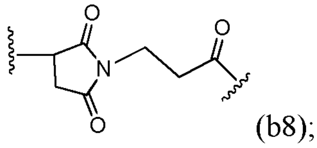 (b8); 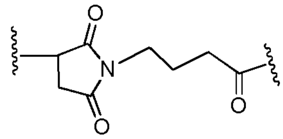 (b9);

and

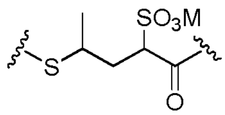

(b10),

wherein q is an integer from 1 to 5, and optionally wherein the pharmaceutically acceptable salt is a sodium or a potassium salt;

(III)

$$CBA\left(-Cy^{L3}\right)_{W_L},$$

wherein:

CBA is said humanized anti-ADAM9 antibody or ADAM9-binding fragment thereof that is covalently linked to $Cy^{L3}$ through a lysine residue;
$W_L$ is an integer from 1 to 20;
$Cy^{L3}$ is represented by the following formula:

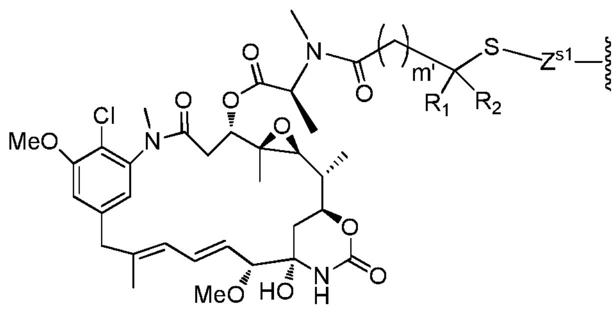

,

m' is 1 or 2;
$R_1$ and $R_2$, are each independently H or a $(C_1-C_3)$alkyl; and
$Z^{s1}$ is selected from any one of the following formulas:

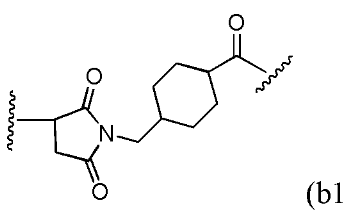 (b1); 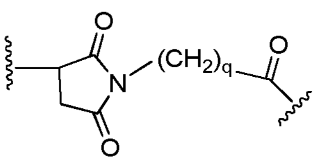 (b2); 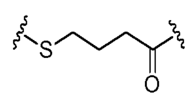 (b3);

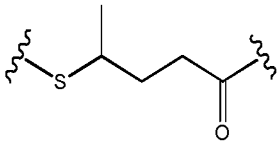 (b4); 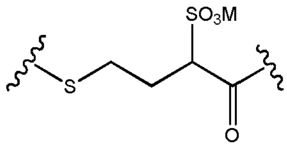 (b5),

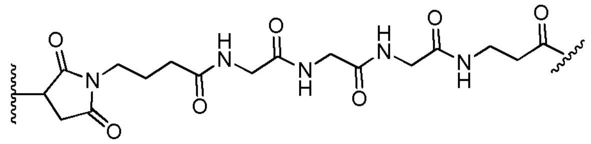 (b6),

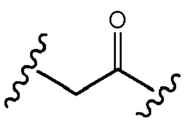 (b7); 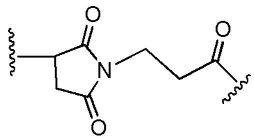 (b8); 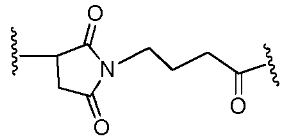 (b9),

and

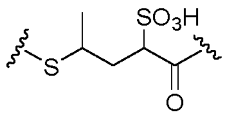 (b10),

wherein q is an integer from 1 to 5, and optionally wherein the pharmaceutically acceptable salt is a sodium or a potassium salt;

(IV)

$$CBA\left(-Cy^{C1}\right)_{W_C},$$

wherein:

CBA is said humanized anti-ADAM9 antibody or ADAM9-binding fragment thereof that is covalently linked to $Cy^{C1}$ through a cysteine residue;
$W_C$ is 1 or 2;
$Cy^{C1}$ is represented by the following formula:

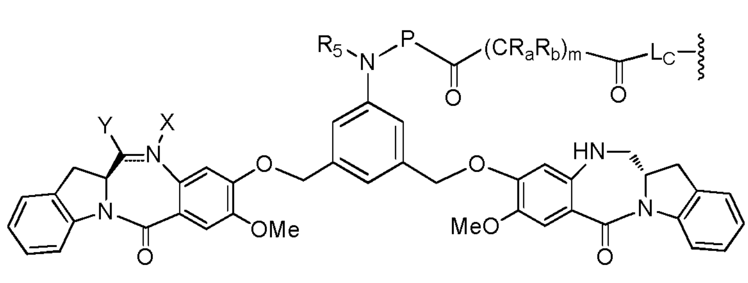

; or

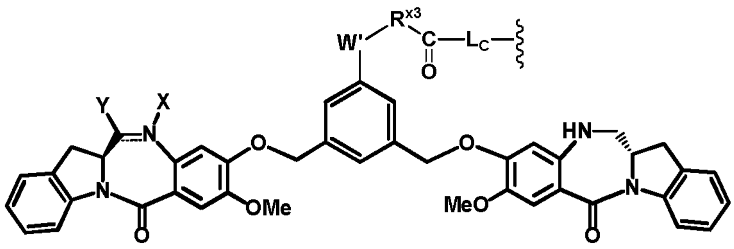

,

or a pharmaceutically acceptable salt thereof, wherein:

the double line ⎓ between N and C represents a single bond or a double bond, provided that when it is a double bond, X is absent and Y is -H or a $(C_1\text{-}C_4)$alkyl; and when it is a single bond, X is -H or an amine protecting moiety, Y is -OH or $-SO_3H$ or a pharmaceutically acceptable salt thereof;
$R_5$ is -H or a $(C_1\text{-}C_3)$alkyl;
P is an amino acid residue or a peptide containing 2 to 20 amino acid residues;
$R_a$ and $R_b$, for each occurrence, are independently -H, $(C_1\text{-}C_3)$alkyl, or a charged substituent or an ionizable group Q;
W' is $-NR^{e'}$,
$R^e$ is $-(CH_2\text{-}CH_2\text{-}O)_n\text{-}R^k$;
n is an integer from 2 to 6;
$R^k$ is -H or -Me;
$R^{x3}$ is a $(C_1\text{-}C_6)$alkyl; and,
$L_C$ is represented by

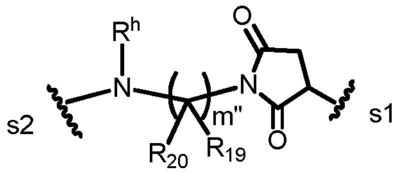

,

s1 is the site covalently linked to CBA, and s2 is the site covalently linked to the -C(=O)- group on $Cy^{C1}$; wherein:

$R_{19}$ and $R_{20}$, for each occurrence, are independently -H or a $(C_1\text{-}C_3)$alkyl;
m" is an integer between 1 and 10; and

$R^h$ is -H or a $(C_1\text{-}C_3)$alkyl, and optionally wherein the pharmaceutically acceptable salt is a sodium or a potassium salt;

(V)

$$CBA\text{-}\left(\text{-}Cy^{C2}\right)_{W_C},$$

wherein:

CBA is said humanized anti-ADAM9 antibody or ADAM9-binding fragment thereof that is covalently linked to $Cy^{C2}$ through a cysteine residue;
$W_C$ is 1 or 2;
$Cy^{C2}$ is represented by the following formula:

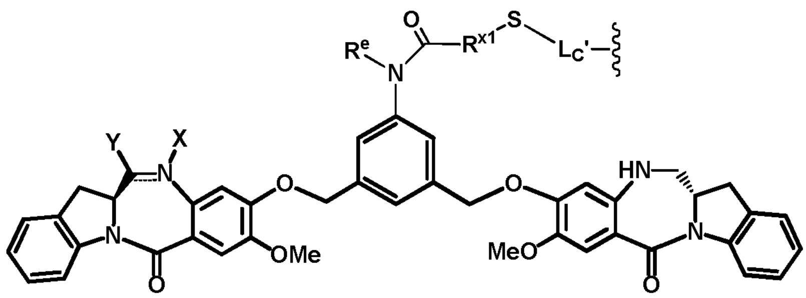

,

or

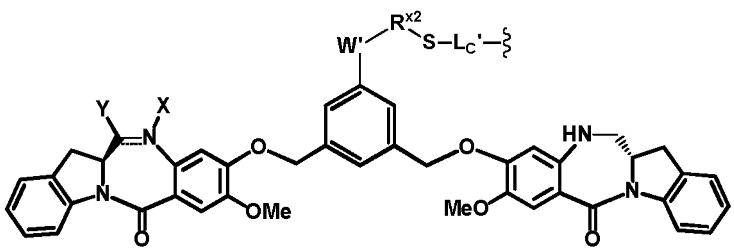

,

or a pharmaceutically acceptable salt thereof, wherein:

the double line



between N and C represents a single bond or a double bond, provided that when it is a double bond, X is absent and Y is -H or a $(C_1-C_4)$alkyl; and when it is a single bond, X is -H or an amine protecting moiety, Y is -OH or $-SO_3H$ or a pharmaceutically acceptable salt thereof;
$R^{x1}$ is a $(C_1-C_6)$alkyl;
$R^e$ is -H or a $(C_1-C_6)$alkyl;
W' is $-NR^{e'}$;
$R^{e'}$ is $-(CH_2-CH_2-O)_n-R^k$;
n is an integer from 2 to 6;
$R^k$ is -H or -Me;
$R^{x2}$ is a $(C_1-C_6)$alkyl;
$L_C'$ is represented by the following formula:

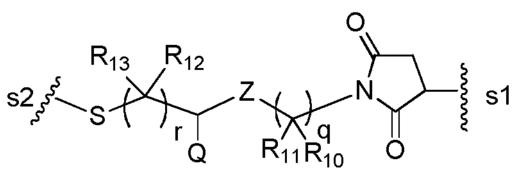

;

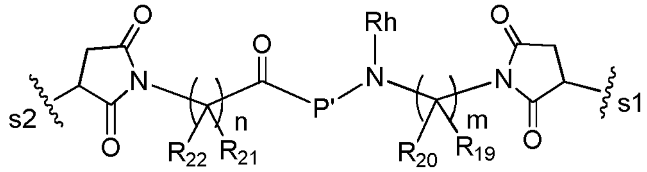

;

wherein:

s1 is the site covalently linked to the CBA and s2 is the site covalently linked to -S- group on $Cy^{C2}$;
Z is -C(=O)-$NR_9$-,or -$NR_9$-C(=O)-;
Q is -H, a charged substituent, or an ionizable group;
$R_9$, $R_{10}$, $R_{11}$, $R_{12}$, $R_{13}$, $R_{19}$, $R_{20}$, $R_{21}$ and $R_{22}$, for each occurrence, are independently -H or a $(C_1\text{-}C_3)$alkyl;
q and r, for each occurrence, are independently an integer between 0 and 10;
m and n are each independently an integer between 0 and 10;
$R^h$ is -H or a $(C_1\text{-}C_3)$alkyl; and

P' is an amino acid residue or a peptide containing 2 to 20 amino acid residues, and optionally wherein the pharmaceutically acceptable salt is a sodium or a potassium salt;
and

(VI)

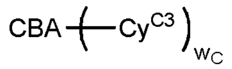

wherein:

CBA is said humanized anti-ADAM9 antibody or ADAM9-binding fragment thereof that is covalently linked to $Cy^{C3}$ through a cysteine residue;
$W_C$ is 1 or 2;
$Cy^{C3}$ is represented by the following formula:

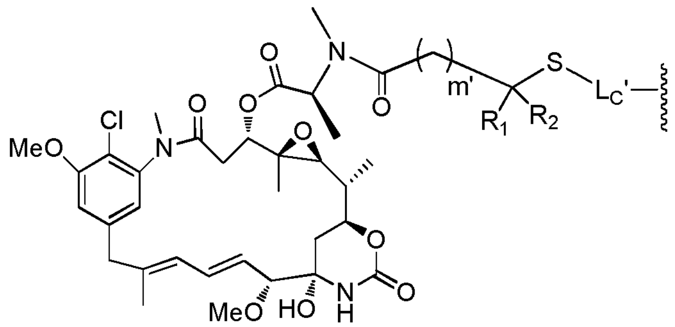

wherein:

m' is 1 or 2;
$R_1$ and $R_2$, are each independently -H or a $(C_1\text{-}C_3)$alkyl;
$L_C$' is represented by the following formula:

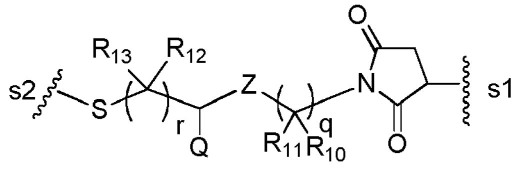

;

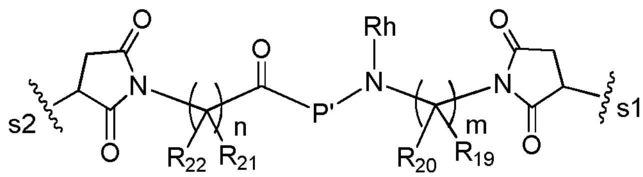

;

wherein:

s1 is the site covalently linked to the CBA and s2 is the site covalently linked to -S- group on $Cy^{C3}$;
Z is $-C(=O)-NR_9-$,or $-NR_9-C(=O)-$;
Q is H, a charged substituent, or an ionizable group;
$R_9$, $R_{10}$, $R_{11}$, $R_{12}$, $R_{13}$, $R_{19}$, $R_{20}$, $R_{21}$ and $R_{22}$, for each occurrence, are independently -H or a $(C_1-C_3)$alkyl;
q and r, for each occurrence, are independently an integer between 0 and 10;
m and n are each independently an integer between 0 and 10;
$R^h$ is -H or a $(C_1-C_3)$alkyl;

P' is an amino acid residue or a peptide containing 2 to 20 amino acid residues, and optionally wherein the pharmaceutically acceptable salt is a sodium or a potassium salt.

**15.** A pharmaceutical composition comprising an effective amount of the immunoconjugate of any of claims 1-14 and a pharmaceutically acceptable carrier, excipient or diluent.

**16.** An immunoconjugate of any one of claims 1-14 or the pharmaceutical composition of claim 15 for use in the treatment of a disease or condition associated with, or **characterized by**, the expression of ADAM9.

**17.** The immunoconjugate or pharmaceutical composition for use of claim 16, wherein said disease or condition associated with, or **characterized by**, the expression of ADAM9 is cancer, optionally wherein said cancer is selected from the group consisting of non-small-cell lung cancer, colorectal cancer, bladder cancer, gastric cancer, pancreatic cancer, renal cell carcinoma, prostate cancer, esophageal cancer, breast cancer, head and neck cancer, uterine cancer, ovarian cancer, liver cancer, cervical cancer, thyroid cancer, testicular cancer, myeloid cancer, melanoma, and lymphoid cancer.

**Patentansprüche**

**1.** Immunkonjugat, umfassend einen humanisierten Anti-ADAM9-Antikörper oder ein ADAM9-bindendes Fragment davon, das spezifisch an Human-ADAM9 und Cyno-ADAM9 bindet:

(I) wobei der humanisierte Anti-ADAM9-Antikörper oder das ADAM9-bindende Fragment davon mit einem pharmakologischen Mittel konjugiert ist; und
(II) wobei der humanisierte Anti-ADAM9-Antikörper oder das ADAM9-bindende Fragment davon eine variable Domäne der leichten Kette (VL) und eine variable Domäne der schweren Kette (VH) umfasst, wobei die variable Domäne der schweren Kette eine $CDR_H1$-Domäne, eine $CDR_H2$-Domäne und eine $CDR_H3$-Domäne umfasst und die variable Domäne der leichten Kette eine $CDR_L1$-Domäne, eine $CDR_L2$-Domäne und eine $CDR_L3$-Domäne umfasst, wobei:

(A) die $CDR_H1$-Domäne die Aminosäuresequenz von SEQ ID NR.: 8 oder SEQ ID NR.: 34 umfasst; und
(B) die $CDR_H2$-Domäne die Aminosäuresequenz von SEQ ID NR.: 9, SEQ ID NR.: 35 oder SEQ ID NR.: 36 umfasst; und
(C) die $CDR_H3$-Domäne die Aminosäuresequenz von einem von SEQ ID Nr. 37, SEQ ID Nr. 38, SEQ ID Nr. 39, SEQ ID Nr. 40, SEQ ID Nr. 41, SEQ ID Nr. 42, SEQ ID Nr. 43, SEQ ID Nr. 44, SEQ ID Nr. 45 oder SEQ ID Nr. 46 umfasst; und

wobei:

(A) die $CDR_L1$-Domäne die Aminosäuresequenz von SEQ ID NR.: 12, SEQ ID NR.: 62, SEQ ID NR.: 63 oder SEQ ID NR.: 64 aufweist; und
(B) die $CDR_L2$-Domäne die Aminosäuresequenz von SEQ ID NR.: 13 aufweist;
und
(C) die $CDR_L3$-Domäne die Aminosäuresequenz von SEQ ID NR.: 14 oder SEQ ID NR.: 65 aufweist.

**2.** Immunkonjugat nach Anspruch 1, wobei der humanisierte Anti-ADAM9-Antikörper oder das ADAM9-bindende Fragment davon eine $CDR_H1$-Domäne, eine $CDR_H2$-Domäne und eine $CDR_H3$-Domäne und eine $CDR_L1$-Domäne, eine $CDR_L2$-Domäne und eine $CDR_L3$-Domäne mit den Sequenzen umfasst, die ausgewählt sind aus der Gruppe,

bestehend aus:

(a) SEQ ID NRn.: 8, 35 und 45 bzw. SEQ ID NRn.: 62, 13, 14;
(b) SEQ ID NRn.: 8, 35 und 38 bzw. SEQ ID NRn.: 62, 13, 14;
(c) SEQ ID NRn.: 8, 35 und 39 bzw. SEQ ID NRn.: 62, 13, 14;
(d) SEQ ID NRn.: 8, 35 und 40 bzw. SEQ ID NRn.: 62, 13, 14;
(e) SEQ ID NRn.: 8, 35 und 41 bzw. SEQ ID NRn.: 62, 13, 14
(f) SEQ ID NRn.: 8, 35 und 42 bzw. SEQ ID NRn.: 62, 13, 14;
(g) SEQ ID NRn.: 8, 35 und 43 bzw. SEQ ID NRn.: 62, 13, 14;
(h) SEQ ID NRn.: 8,35 und 44 bzw. SEQ ID NRn.: 62, 13, 14;
(i) SEQ ID NRn.: 8,35 und 37 bzw. SEQ ID NRn.: 62, 13, 14; und
(j) SEQ ID NRn.: 8,35 und 46 bzw. SEQ ID NRn.: 62, 13, 14.

**3.** Immunkonjugat nach Anspruch 1 oder 2, wobei:

(A) die VH-Domäne SEQ ID Nr. 20, SEQ ID Nr. 21, SEQ ID Nr. 22, SEQ ID Nr. 23, SEQ ID Nr. 24, SEQ ID Nr. 25, SEQ ID Nr. 26, SEQ ID Nr. 27, SEQ ID Nr. 28 oder SEQ ID Nr. 29 umfasst; und
(B) die VL-Domäne SEQ ID NR.: 54, SEQ ID NR.: 55, SEQ ID NR.: 56 oder SEQ ID NR.: 57 umfasst.

**4.** Immunkonjugat nach Anspruch 1, wobei die $CDR_H1$-Domäne die Aminosäuresequenz SYWMH (SEQ ID NR.: 8) umfasst, die $CDR_H2$-Domäne die Aminosäuresequenz EIIPIFGHTNYNEKFKS (SEQ ID NR.: 35) umfasst, die $CDR_H3$-Domäne die Aminosäuresequenz GGYYYYPRQGFLDY (SEQ ID NR. 45) umfasst, die $CDR_L1$-Domäne die Aminosäuresequenz KASQSVDYSGDSYMN (SEQ ID NR.: 62) umfasst, die $CDR_L2$-Domäne die Aminosäuresequenz AASDLES (SEQ ID NR.: 13) umfasst und die $CDR_L3$-Domäne die Aminosäuresequenz QQSHEDPFT (SEQ ID NR.: 14) umfasst.

**5.** Immunkonjugat nach Anspruch 4, wobei das Immunkonjugat umfasst:

(A) die variable Domäne der schweren Kette (VH) von hMAB-A (2I.2) (SEQ ID NR.: 28); und/oder
(B) die variable Domäne der leichten Kette (VL) von hMAB-A (2I.2) (SEQ ID NR.: 55); und/oder
(C) die variable Domäne der schweren Kette (VH) von hMAB-A (2I.2) (SEQ ID NR.: 28) und die variable Domäne der leichten Kette (VL) von hMAB-A (2I.2) (SEQ ID NR.: 55).

**6.** Immunkonjugat nach einem der Ansprüche 1-5, wobei das Immunkonjugat eine Fc-Region umfasst, wahlweise wobei die Fc-Region eine variante Fc-Region ist, die umfasst:

(a) eine oder mehrere Aminosäuremodifizierung(en), die die Affinität der varianten Fc-Region für einen FcγR verringert/verringern; und/oder
(b) eine oder mehrere Aminosäuremodifizierung(en), die einen Cysteinrest einführt/einführen; und/oder
(c) eine oder mehrere Aminosäuremodifizierung(en), die die Serum-Halbwertszeit verlängert/verlängern.

**7.** Immunkonjugat nach Anspruch 6, wobei

(I) die eine oder mehreren Aminosäuremodifizierung(en), die die Affinität der varianten Fc-Region für einen FcγR verringert/verringern, umfasst/umfassen:

(A) L234A;
(B) L235A oder
(C) L234A und L235A; und

(II) die eine oder mehreren Aminosäuremodifizierung(en), die einen Cysteinrest einführt/einführen, S442C umfassen; und
(III) die eine oder mehreren Aminosäuremodifizierung(en), die die Serum-Halbwertszeit verlängert/verlängern, umfasst/umfassen:

(A) M252Y;
(B) M252Y und S254T;
(C) M252Y und T256E;

(D) M252Y, S254T und T256E; oder
(E) K288D und H435K; und

wobei die Nummerierung die des EU-Indexes wie in Kabat ist.

**8.** Immunkonjugat nach einem der Ansprüche 1-7, wobei der humanisierte Anti-ADAM9-Antikörper eine schwere Kette und eine leichte Kette mit den Sequenzen umfasst, die ausgewählt sind aus der Gruppe, bestehend aus:

(a) SEQ ID NR.: 152 bzw. SEQ ID NR.: 68;
(b) SEQ ID NR.: 141 bzw. SEQ ID NR.: 68;
(c) SEQ ID NR.: 142 bzw. SEQ ID NR.: 68;
(d) SEQ ID NR.: 143 bzw. SEQ ID NR.: 68;
(e) SEQ ID NR.: 151 bzw. SEQ ID NR.: 68;
(f) SEQ ID NR.: 52 bzw. SEQ ID NR.: 68;
(g) SEQ ID NR.: 153 bzw. SEQ ID NR.: 68 und
(h) SEQ ID NR.: 154 bzw. SEQ ID NR.: 68.

**9.** Immunkonjugat nach Anspruch 8, wobei X in SEQ ID NR.: 141, SEQ ID NR.: 142, SEQ ID NR.: 143, SEQ ID NR.: 151, SEQ ID NR.: 152, SEQ ID NR.: 153 oder SEQ ID NR.: 154 nicht vorhanden ist.

**10.** Immunkonjugat nach einem der Ansprüche 1-9, wobei der humanisierte Anti-ADAM9-Antikörper eine schwere Kette und eine leichte Kette mit den Sequenzen von SEQ ID NR.: 152 bzw. SEQ ID NR.: 68 umfasst.

**11.** Immunkonjugat nach Anspruch 1, wobei der humanisierte Anti-ADAM9-Antikörper oder das ADAM9-bindende Fragment davon eine variable Domäne der schweren Kette (VH) und eine variable Domäne der leichten Kette (VL) mit den Sequenzen SEQ ID NR.: 28 bzw. SEQ ID NR.: 55 umfasst.

**12.** Immunkonjugat nach einem der Ansprüche 1-11, wobei das pharmakologische Mittel eine Maytansinoidverbindung, eine Pyrrolobenzodiazepinverbindung oder eine Indolinobenzodiazepinverbindung ist.

**13.** Immunkonjugat nach Anspruch 12, wobei das pharmakologische Mittel eine Maytansinoidverbindung ist.

**14.** Immunkonjugat nach einem der Ansprüche 1-13, wobei das Immunkonjugat durch eine Formel dargestellt wird, die ausgewählt ist aus der Gruppe, bestehend aus:

(I)

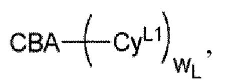

, wobei:

CBA der humanisierte Anti-ADAM9-Antikörper oder das ADAM9-bindende Fragment davon ist, der/das kovalent über einen Lysinrest an $Cy^{L1}$ gebunden ist;
$W_L$ eine ganze Zahl von 1 bis 20 ist; und
$Cy^{L1}$ durch die nachstehende Formel dargestellt wird:

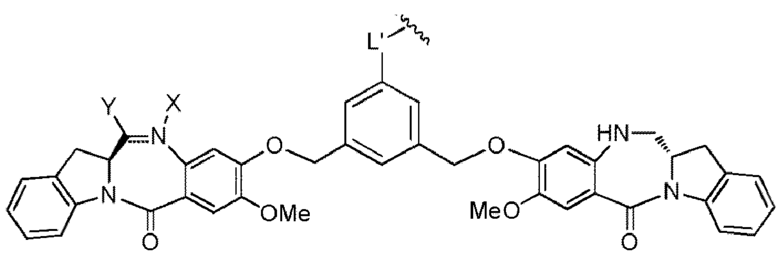

oder

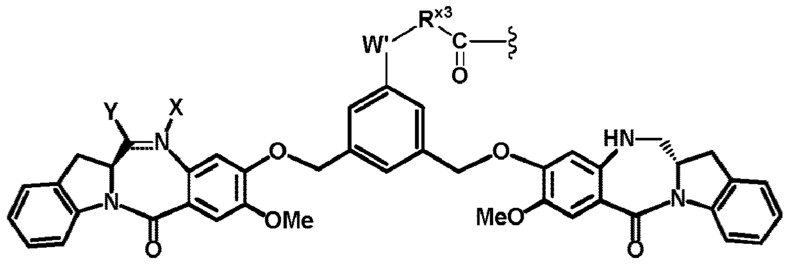

oder ein pharmazeutisch verträgliches Salz davon, wobei:

die Doppellinie

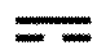

zwischen N und C eine Einfachbindung oder eine Doppelbindung darstellt, mit der Maßgabe, dass, wenn es sich um eine Doppelbindung handelt, X nicht vorhanden ist und Y -H oder ein $(C_1-C_4)$-Alkyl ist; und wenn es sich um eine Einfachbindung handelt, X -H oder eine Aminschutzeinheit ist und Y -OH oder $-SO_3H$ oder ein pharmazeutisch verträgliches Salz davon ist;
W' $-NR^{e'}$ ist,
$R^e$ $-(CH_2-CH_2-O)_n-R^k$ ist;
n eine ganze Zahl von 2 bis 6 ist;
$R^k$ -H oder -Me ist;
$R^{x3}$ ein $(C_1-C_6)$-Alkyl ist;
L' durch die nachstehende Formel dargestellt wird:

- $NR_5-P-C(=O)-(CR_aR_b)_m-C(=O)-$ (B1'); oder
- $NR_5-P-C(=O)-(CR_aR_b)_m-S-Z^{s1}-$ (B2');

$R_5$ -H oder ein $(C_1-C_3)$-Alkyl ist;
P ein Aminosäurerest oder ein Peptid ist, das zwischen 2 bis 20 Aminosäurereste enthält;
$R_a$ und $R_b$ bei jedem Auftreten jeweils unabhängig voneinander -H, $(C_1-C_3)$-Alkyl oder ein geladener Substituent oder eine ionisierbare Gruppe Q sind;
m eine ganze Zahl von 1 bis 6 ist; und
$Z^{s1}$ ausgewählt ist aus einer der nachstehenden Formeln:

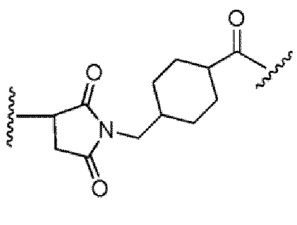 (b1); 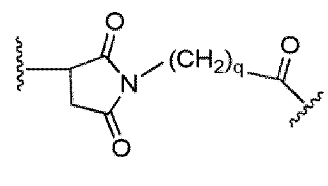 (b2); 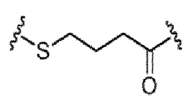 (b3);

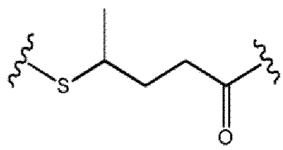 (b4); 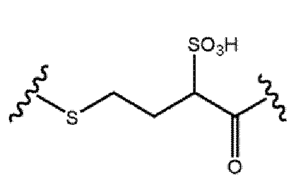 (b5),

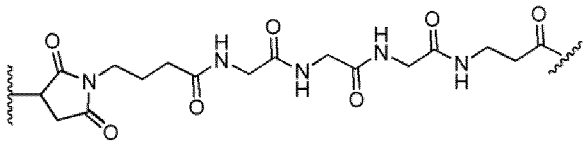
(b6),

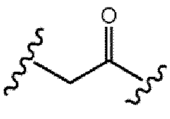
(b7);
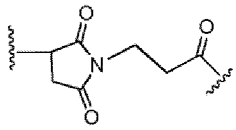
(b8);
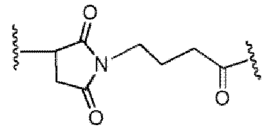
(b9)

und

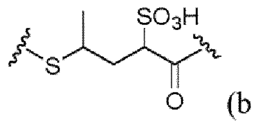
(b10)

wobei q eine ganze Zahl von 1 bis 5 ist, und wahlweise wobei das pharmazeutisch verträgliche Salz ein Natrium- oder Kaliumsalz ist;

(II)

$$CBA \left( - Cy^{L2} \right)_{W_L} ,$$

, wobei:

CBA der humanisierte Anti-ADAM9-Antikörper oder das ADAM9-bindende Fragment davon ist, der/das kovalent über einen Lysinrest an $Cy^{L2}$ gebunden ist;
$W_L$ eine ganze Zahl von 1 bis 20 ist; und
$Cy^{L2}$ durch die nachstehende Formel dargestellt wird:

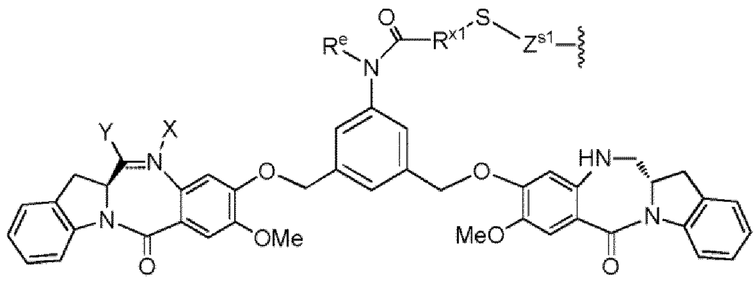

oder

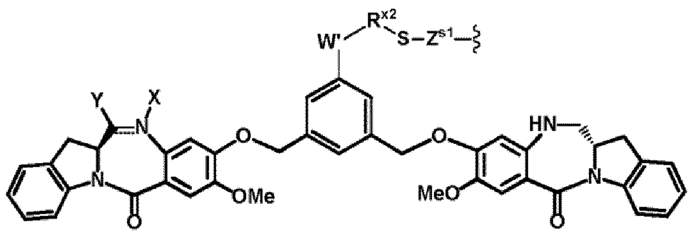
;

oder ein pharmazeutisch verträgliches Salz davon, wobei:

die Doppellinie

zwischen N und C eine Einfachbindung oder eine Doppelbindung darstellt, mit der Maßgabe, dass, wenn es sich um eine Doppelbindung handelt, X nicht vorhanden ist und Y -H oder ein ($C_1$-$C_4$-Alkyl) ist; und wenn es sich um eine Einfachbindung handelt, X -H oder eine Aminschutzeinheit ist und Y -OH oder -$SO_3H$ ist;
$R^{x1}$ und $R^{x2}$ unabhängig voneinander ($C_1$-$C_6$)-Alkyl sind;
$R^e$ -H oder ein ($C_1$-$C_6$)-Alkyl ist;
W' -$NR^{e'}$ ist,
$R^e$ -$(CH_2$-$CH_2$-$O)_n$-$R^k$ ist;
n eine ganze Zahl von 2 bis 6 ist;
$R^k$ -H oder -Me ist;
$Z^{s1}$ ausgewählt ist aus einer der nachstehenden Formeln:

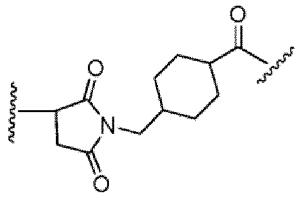 (b1); 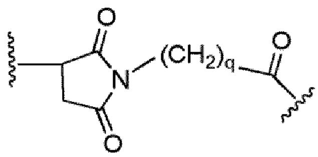 (b2); 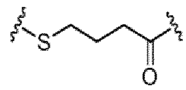 (b3);

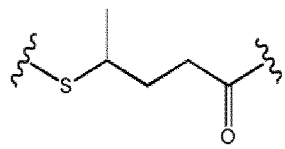 (b4); 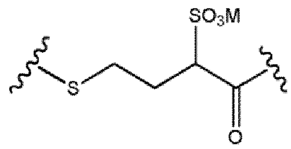 (b5),

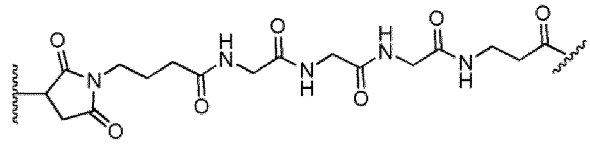 (b6),

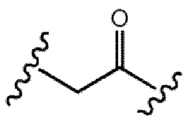 (b7); 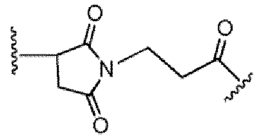 (b8); 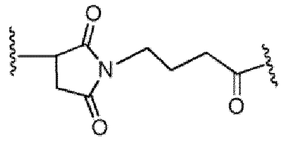 (b9)

und

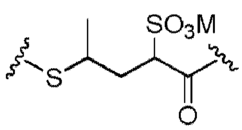 (b10),

wobei q eine ganze Zahl von 1 bis 5 ist, und wahlweise wobei das pharmazeutisch verträgliche Salz ein Natrium- oder Kaliumsalz ist;

(III)

$CBA\left(-Cy^{L3}\right)_{W_L}$,

wobei:

CBA der humanisierte Anti-ADAM9-Antikörper oder das ADAM9-bindende Fragment davon ist, der/das kovalent über einen Lysinrest an $Cy^{L3}$ gebunden ist;
$W_L$ eine ganze Zahl von 1 bis 20 ist;
$Cy^{L3}$ durch die nachstehende Formel dargestellt wird:

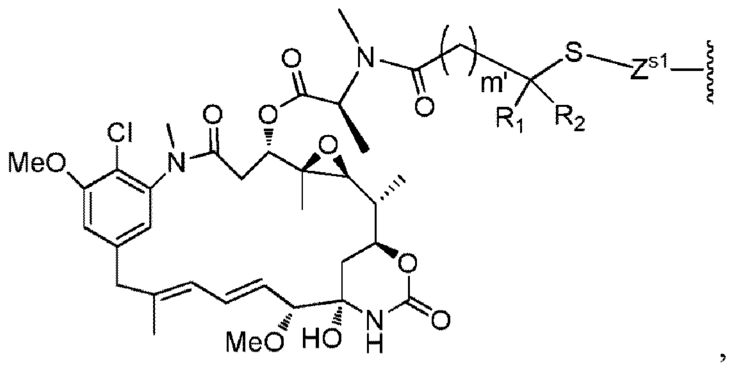

,

m' 1 oder 2 ist;
$R_1$ und $R_2$ jeweils unabhängig voneinander H oder ein $(C_1\text{-}C_3)$-Alkyl sind; und
$Z^{s1}$ ausgewählt ist aus einer der nachstehenden Formeln:

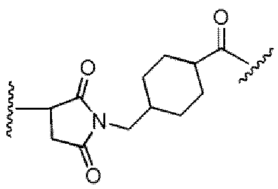 (b1); 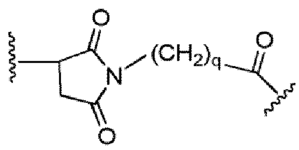 (b2); 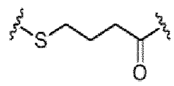 (b3);

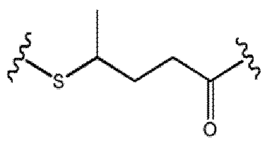 (b4); 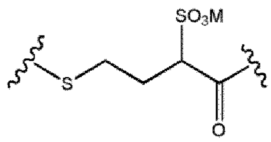 (b5),

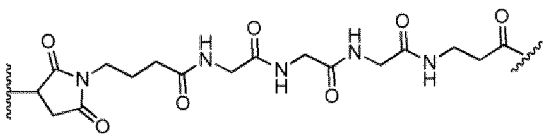 (b6),

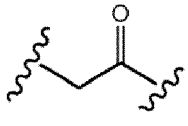 (b7); 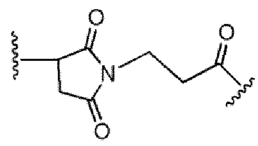 (b8); 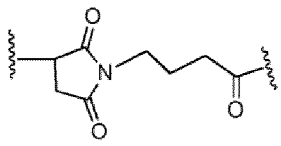 (b9)

und

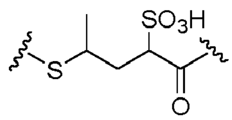

(b10)

wobei q eine ganze Zahl von 1 bis 5 ist, und wahlweise wobei das pharmazeutisch verträgliche Salz ein Natrium- oder Kaliumsalz ist;

(IV)

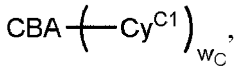

wobei:

CBA der humanisierte Anti-ADAM9-Antikörper oder das ADAM9-bindende Fragment davon ist, der/das kovalent über einen Cysteinrest an $Cy^{C1}$ gebunden ist;
Wc 1 oder 2 ist;
$Cy^{C1}$ durch die nachstehende Formel dargestellt wird:

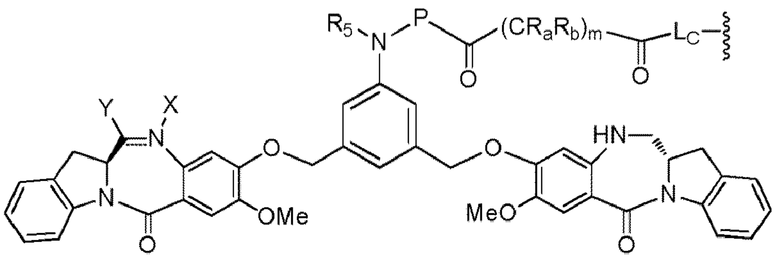

oder

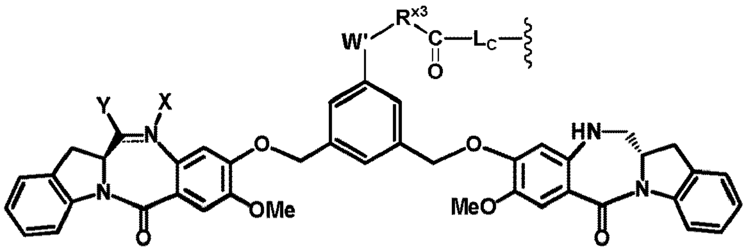

oder ein pharmazeutisch verträgliches Salz davon, wobei:

die Doppellinie =-= zwischen N und C eine Einfachbindung oder eine Doppelbindung darstellt, mit der Maßgabe, dass, wenn es sich um eine Doppelbindung handelt, X nicht vorhanden ist und Y -H oder ein $(C_1$-$C_4)$-Alkyl ist; und wenn es sich um eine Einfachbindung handelt, X -H oder eine Aminschutzeinheit ist, Y -OH oder $-SO_3H$ oder ein pharmazeutisch verträgliches Salz davon ist;
$R_5$ -H oder ein $(C_1$-$C_3)$-Alkyl ist;
P ein Aminosäurerest oder ein Peptid ist, das 2 bis 20 Aminosäurereste enthält;
$R_a$ und $R_b$ bei jedem Auftreten unabhängig voneinander -H, $(C_1$-$C_3)$-Alkyl oder ein geladener Substituent oder eine ionisierbare Gruppe Q sind;
W' $-NR^{e'}$ ist,
$R^{e'}$ $-(CH_2$-$CH_2$-$O)_n$-$R^k$ ist;
n eine ganze Zahl von 2 bis 6 ist;
$R^k$ -H oder -Me ist;
$R^{x3}$ ein $(C_1$-$C_6)$-Alkyl ist; und
Lc dargestellt wird durch

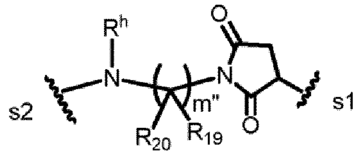

, s1 die Stelle ist, die kovalent an CBA gebunden ist, und s2 die Stelle ist, die kovalent an die Gruppe -C(=O)- an $Cy^{C1}$ gebunden ist;
wobei:

$R_{19}$ und $R_{20}$ bei jedem Auftreten unabhängig voneinander -H oder ein ($C_1$-$C_3$)-Alkyl sind;
m" eine ganze Zahl zwischen 1 und 10 ist; und
$R^h$ -H oder ein ($C_1$-$C_3$)-Alkyl ist, und wahlweise wobei das pharmazeutisch verträgliche Salz ein Natrium- oder ein Kaliumsalz ist;

(V)

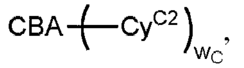

wobei:

CBA der humanisierte Anti-ADAM9-Antikörper oder das ADAM9-bindende Fragment davon ist, der/das kovalent über einen Cysteinrest an $Cy^{C2}$ gebunden ist;
Wc 1 oder 2 ist;
$Cy^{C2}$ durch die nachstehende Formel dargestellt wird:

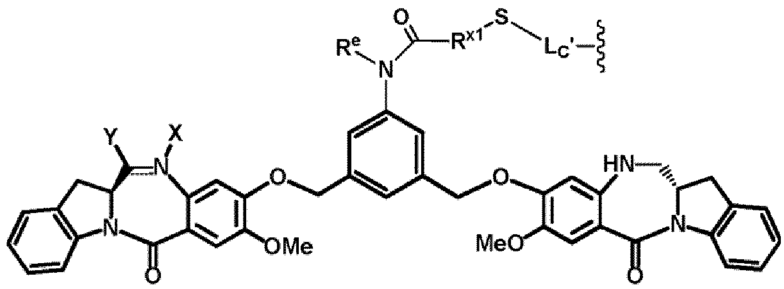

oder

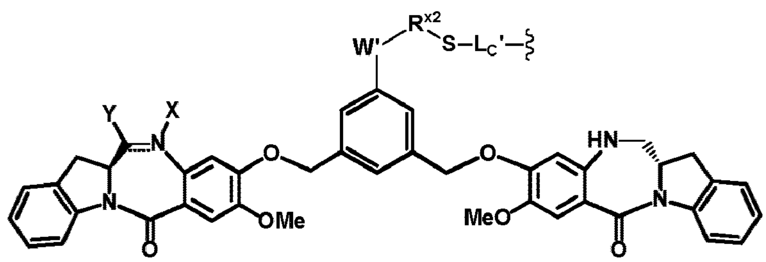

oder ein pharmazeutisch verträgliches Salz davon, wobei:

die Doppellinie ⎓ zwischen N und C eine Einfachbindung oder eine Doppelbindung darstellt, mit der Maßgabe, dass, wenn es sich um eine Doppelbindung handelt, X nicht vorhanden ist und Y -H oder ein ($C_1$-$C_4$)-Alkyl ist; und wenn es sich um eine Einfachbindung handelt, X -H oder eine Aminschutzeinheit ist, Y -OH oder -$SO_3H$ oder ein pharmazeutisch verträgliches Salz davon ist;

$R^{x1}$ ein ($C_1$-$C_6$)-Alkyl ist;
$R^e$ -H oder ein ($C_1$-$C_6$)-Alkyl ist;
W' $-NR^{e'}$ ist;
$R^{e'}$ $-(CH_2-CH_2-O)_n-R^k$ ist;
n eine ganze Zahl von 2 bis 6 ist;
$R^k$ -H oder -Me ist;
$R^{x2}$ ein ($C_1$-$C_6$)-Alkyl ist;
Lc' durch die nachstehende Formel dargestellt wird:

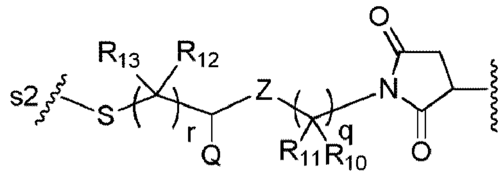

;

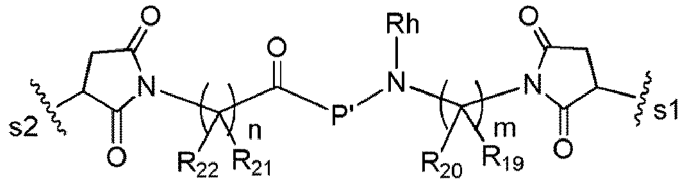

;

wobei:

s1 die Stelle ist, die kovalent an CBA gebunden ist, und s2 die Stelle ist, die kovalent an die Gruppe -S- an $Cy^{C2}$ gebunden ist;
Z $-C(=O)-NR_9-$ oder $-NR_9-C(=O)-$ ist;
Q -H, ein geladener Substituent oder eine ionisierbare Gruppe ist;
$R_9$, $R_{10}$, $R_{11}$, $R_{12}$, $R_{13}$, $R_{19}$, $R_{20}$, $R_{21}$ und $R_{22}$ bei jedem Auftreten unabhängig voneinander -H oder ein ($C_1$-$C_3$)-Alkyl sind;
q und r bei jedem Auftreten unabhängig voneinander eine ganze Zahl zwischen 0 und 10 sind;
m und n jeweils unabhängig voneinander eine ganze Zahl zwischen 0 und 10 sind;
$R^h$ -H oder ein ($C_1$-$C_3$)-Alkyl ist; und

P' ein Aminosäurerest oder ein Peptid ist, das 2 bis 20 Aminosäurereste enthält, und wahlweise wobei das pharmazeutisch verträgliche Salz ein Natrium- oder Kaliumsalz ist; und

(VI)

$$CBA\left(-Cy^{C3}\right)_{W_C},$$

wobei:

CBA der humanisierte Anti-ADAM9-Antikörper oder das ADAM9-bindende Fragment davon ist, der/das kovalent über einen Cysteinrest an $Cy^{C3}$ gebunden ist;
Wc 1 oder 2 ist;
$Cy^{C3}$ durch die nachstehende Formel dargestellt wird:

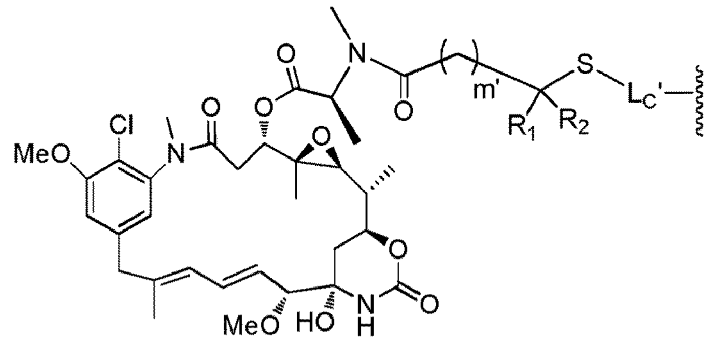

wobei:

m' 1 oder 2 ist;
$R_1$ und $R_2$ jeweils unabhängig voneinander -H oder ein ($C_1$-$C_3$)-Alkyl sind;
Lc' durch die nachstehende Formel dargestellt wird:

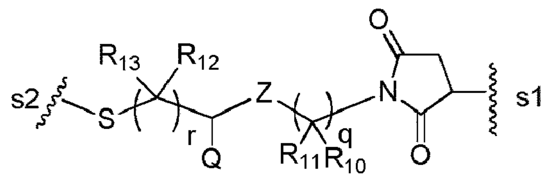

;

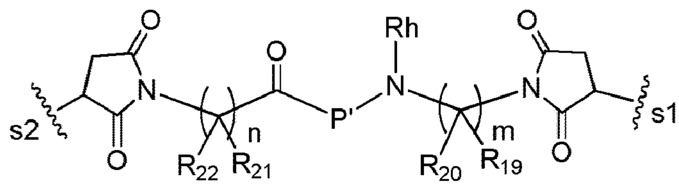

wobei:

s1 die Stelle ist, die kovalent an CBA gebunden ist, und s2 die Stelle ist, die kovalent an die Gruppe -S- an $Cy^{C3}$ gebunden ist;
Z -C(=O)-$NR_9$- oder -$NR_9$-C(=O)- ist;
Q H, ein geladener Substituent oder eine ionisierbare Gruppe ist;
$R_9$, $R_{10}$, $R_{11}$, $R_{12}$, $R_{13}$, $R_{19}$, $R_{20}$, $R_{21}$ und $R_{22}$ bei jedem Auftreten unabhängig voneinander -H oder ein ($C_1$-$C_3$)-Alkyl sind;
q und r bei jedem Auftreten unabhängig voneinander eine ganze Zahl zwischen 0 und 10 sind;
m und n jeweils unabhängig voneinander eine ganze Zahl zwischen 0 und 10 sind;
$R^h$ -H oder ein ($C_1$-$C_3$)-Alkyl ist;
P' ein Aminosäurerest oder ein Peptid ist, das 2 bis 20 Aminosäurereste enthält, und wahlweise wobei das pharmazeutisch verträgliche Salz ein Natrium- oder ein Kaliumsalz ist.

**15.** Pharmazeutische Zusammensetzung, umfassend eine wirksame Menge des Immunkonjugats nach einem der Ansprüche 1-14 und einen pharmazeutisch verträglichen Träger, Exzipienten oder Verdünnungsmittel.

**16.** Immunkonjugat nach einem der Ansprüche 1-14 oder die pharmazeutische Zusammensetzung nach Anspruch 15 zur Verwendung bei der Behandlung einer Krankheit oder eines Zustands, der der Expression von ADAM9 zugeordnet oder **dadurch gekennzeichnet** ist.

**17.** Immunkonjugat oder pharmazeutische Zusammensetzung zur Verwendung nach Anspruch 16, wobei die Krankheit oder der Zustand, der der Expression von ADAM9 zugeordnet oder **dadurch gekennzeichnet** ist, Krebs ist, wahlweise wobei der Krebs ausgewählt ist aus der Gruppe, bestehend aus nicht-kleinzelligem Lungenkrebs, kolorektalem Krebs, Blasenkrebs, Magenkrebs, Bauchspeicheldrüsenkrebs, Nierenzellkarzinom, Prostatakrebs, Speiseröhrenkrebs, Brustkrebs, Kopf- und Halskrebs, Gebärmutterkrebs, Eierstockkrebs, Leberkrebs, Gebärmutterhalskrebs,

Schilddrüsenkrebs, Hodenkrebs, myeloischer Krebs, Melanom und lymphatischer Krebs.

**Revendications**

1. Immunoconjugué comprenant un anticorps anti ADAM9 humanisé ou un fragment de liaison à ADAM9 de celui-ci qui se lie spécifiquement à une ADAM9 humaine et à une cyno ADAM9:

    (I) dans lequel ledit anticorps anti ADAM9 humanisé ou le fragment de liaison à ADAM9 de celui-ci est conjugué à un agent pharmacologique; et
    (II) dans lequel ledit anticorps anti ADAM9 humanisé ou le fragment de liaison à ADAM9 de celui-ci comprend un domaine variable de chaîne légère (VL) et un domaine variable de chaîne lourde (VH), dans lequel ledit domaine variable de chaîne lourde comprend un domaine $CDR_H1$, un domaine $CDR_H2$ et un domaine $CDR_H3$, et ledit domaine variable de chaîne légère comprend un domaine $CDR_L1$, un domaine $CDR_L2$ et un domaine $CDR_L3$, dans lequel:

        (A) ledit domaine $CDR_H1$ comprend la séquence d'acides aminés de la SEQ ID NO: 8 ou de la SEQ ID NO: 34; et
        (B) ledit domaine $CDR_H2$ comprend la séquence d'acides aminés de la SEQ ID NO: 9, de la SEQ ID NO: 35 ou de la SEQ ID NO: 36; et
        (C) ledit domaine $CDR_H3$ comprend la séquence d'acides aminés de l'une quelconque des SEQ ID NO: 37, SEQ ID NO: 38, SEQ ID NO: 39, SEQ ID NO: 40, SEQ ID NO: 41, SEQ ID NO: 42, SEQ ID NO: 43, SEQ ID NO: 44, SEQ ID NO: 45 ou SEQ ID NO: 46; et

    dans lequel:

        (A) ledit domaine $CDR_L1$ a la séquence d'acides aminés de la SEQ ID NO: 12, de la SEQ ID NO: 62, de la SEQ ID NO: 63 ou de la SEQ ID NO: 64; et
        (B) ledit domaine $CDR_L2$ a la séquence d'acides aminés de la SEQ ID NO: 13; et
        (C) ledit domaine $CDR_L3$ a la séquence d'acides aminés de la SEQ ID NO: 14 ou de la SEQ ID NO: 65.

2. Immunoconjugué selon la revendication 1, dans lequel ledit anticorps anti ADAM9 humanisé ou le fragment de liaison à ADAM9 de celui-ci comprend un domaine $CDR_H1$, un domaine $CDR_H2$ et un domaine $CDR_H3$, et un domaine $CDR_L1$, un domaine $CDR_L2$ et un domaine $CDR_L3$ ayant les séquences sélectionnées parmi le groupe constitué:

    (a) des SEQ ID NO: 8,35 et 45 et des SEQ ID NO: 62, 13, 14, respectivement;
    (b) des SEQ ID NO: 8,35 et 38 et des SEQ ID NO: 62, 13, 14, respectivement;
    (c) des SEQ ID NO: 8,35 et 39 et des SEQ ID NO: 62, 13, 14, respectivement;
    (d) des SEQ ID NO: 8,35 et 40 et des SEQ ID NO: 62, 13, 14, respectivement;
    (e) des SEQ ID NO: 8,35 et 41 et des SEQ ID NO: 62, 13, 14, respectivement;
    (f) des SEQ ID NO: 8, 35 et 42 et des SEQ ID NO: 62, 13, 14, respectivement;
    (g) des SEQ ID NO: 8, 35 et 43 et des SEQ ID NO: 62, 13, 14, respectivement;
    (h) des SEQ ID NO: 8, 35 et 44 et des SEQ ID NO: 62, 13, 14, respectivement;
    (i) des SEQ ID NO: 8, 35 et 37 et des SEQ ID NO: 62, 13, 14, respectivement; et
    (j) des SEQ ID NO: 8,35 et 46 et des SEQ ID NO: 62, 13, 14, respectivement.

3. Immunoconjugué selon la revendication 1 ou 2, dans lequel:

    (A) ledit domaine VH comprend les SEQ ID NO: 20, SEQ ID NO: 21, SEQ ID NO: 22, SEQ ID NO: 23, SEQ ID NO: 24, SEQ ID NO: 25, SEQ ID NO: 26, SEQ ID NO: 27, SEQ ID NO: 28 ou SEQ ID NO: 29; et
    (B) ledit domaine VL comprend les SEQ ID NO: 54, SEQ ID NO: 55, SEQ ID NO: 56 ou SEQ ID NO: 57.

4. Immunoconjugué selon la revendication 1, dans lequel ledit domaine $CDR_H1$ comprend la séquence d'acides aminés SYWMH (SEQ ID NO: 8), ledit domaine $CDR_H2$ comprend la séquence d'acides aminés EIIPIFGHTNYNEKFKS (SEQ ID NO: 35), ledit domaine $CDR_H3$ comprend la séquence d'acides aminés GGYYYYPRQGFLDY (SEQ ID NO: 45), ledit domaine $CDR_L1$ comprend la séquence d'acides aminés KASQSVDYSGDSYMN (SEQ ID NO: 62), ledit domaine $CDR_L2$ comprend la séquence d'acides aminés AASDLES (SEQ ID NO: 13) et ledit domaine $CDR_L3$

comprend la séquece d'acides aminés QQSHEDPFT (SEQ ID NO: 14).

**5.** Immunoconjugué selon la revendication 4, dans lequel ledit immunoconjugué comprend:

(A) le domaine variable de chaîne lourde (VH) de hMAB-A (2I.2) (SEQ ID NO: 28); et/ou
(B) le domaine variable de chaîne légère (VL) de hMAB-A (2I.2) (SEQ ID NO: 55); et/ou
(C) le domaine variable de chaîne lourde (VH) de hMAB-A (2I.2) (SEQ ID NO: 28) et le domaine variable de chaîne légère (VL) de hMAB-A (2I.2) (SEQ ID NO: 55).

**6.** Immunoconjugué selon l'une quelconque des revendications 1-5, où ledit immunoconjugué comprend une région Fc, optionnellement dans lequel ladite région Fc est une région Fc variante qui comprend:

(a) une ou plusieurs modifications d'acides aminés qui réduisent l'affinité de la région Fc variante pour FcyR; et/ou
(b) une ou plusieurs modifications d'acides aminés qui introduisent un résidu cystéine; et/ou
(c) une ou plusieurs modifictions d'acides aminés qui prolongent la demi-vie sérique

**7.** Immunoconjugué selon la revendication 6, dans lequel

(I) ladite une ou lesdites plusieurs modifications d'acides aminés qui réduisent l'affinité de la région Fc variante pour un FcyR comprennent:

(A) L234A;
(B) L235A; ou
(C) L234A et L235A; et

(II) ladite une ou lesdites plusieurs modifications d'acides aminés qui introduisent un résidu cystéine comprennent S442C; et
(III) ladite une ou lesdites plusieurs modifications d'acides aminés qui prolongent la demi-vie sérique comprennent

(A) M252Y;
(B) M252Y et S254T;
(C) M252Y et T256E;
(D) M252Y, S254T et T256E; ou
(E) K288D et H435K; et

dans lequel ladite numérotation est celle de l'index EU comme dans Kabat.

**8.** Immunoconjugué selon l'une quelconque des revendications 1-7, dans lequel ledit anticorps anti ADAM9 humanisé comprend une chaîne lourde et une chaîne légère ayant les séquences sélectionnées parmi le groupe constitué:

(a) de la SEQ ID NO: 152 et de la SEQ ID NO: 68, respectivement;
(b) de la SEQ ID NO: 141 et de la SEQ ID NO: 68, respectivement;
(c) de la SEQ ID NO: 142 et de la SEQ ID NO: 68, respectivement;
(d) de la SEQ ID NO: 143 et de la SEQ ID NO: 68, respectivement;
(e) de la SEQ ID NO: 151 et de la SEQ ID NO: 68, respectivement;
(f) de la SEQ ID NO: 52 et de la SEQ ID NO: 68, respectivement;
(g) de la SEQ ID NO: 153 et de la SEQ ID NO: 68, respectivement; et
(h) de la SEQ ID NO: 154 et de la SEQ ID NO: 68, respectivement.

**9.** Immunoconjugué selon la revendication 8, dans lequel X dans les SEQ ID NO: 141, SEQ ID NO: 142, SEQ ID NO: 143, SEQ ID NO: 151, SEQ ID NO: 152, SEQ ID NO: 153 ou SEQ ID NO: 154 est absent.

**10.** Immunoconjugué selon l'une quelconque des revendications 1-9, dans lequel ledit anticorps anti ADAM9 humanisé comprend une chaîne lourde et une chaîne légère ayant les séquences de la SEQ ID NO: 152 et de la SEQ ID NO: 68, respectivement.

**11.** Immunoconjugué selon la revendication 1, dans lequel ledit anticorps anti ADAM9 humanisé ou le fragment de

liaison à ADAM9 de celui-ci, comprend un domaine variable de chaîne lourde (VH) et un domaine variable de chaîne légère (VL) ayant les séquences de la SEQ ID NO: 28 et de la SEQ ID NO: 55, respectivement.

**12.** Immunoconjugué selon l'une quelconque des revendications 1-11, dans lequel ledit agent pharmacologique est un composé maytansinoïde, un composé de pyrrolobenzodiazépine ou un composé d'indolinobenzodiazépine.

**13.** Immunoconjugué selon la revendication 12, dans lequel ledit agent pharmacologique est un composé maytansinoïde.

**14.** Immunoconjugué selon l'une quelconque des revendications 1-13, où l'immunoconjugué est représenté par une formule sélectionnée parmi le group constitué de:

(I)

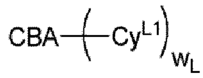

dans lequel:

CBA est ledit anticorps anti ADAM9 humanisé ou le fragment de liaison à ADAM9 de celui-ci qui est lié de manière covalente à $Cy^{L1}$ par un résidu lysine;
$W_L$ est un entier de 1 à 20; et
$Cy^{L1}$ est représenté par la formule suivante:

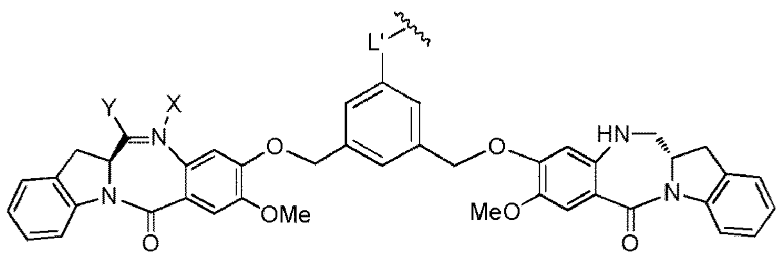

ou

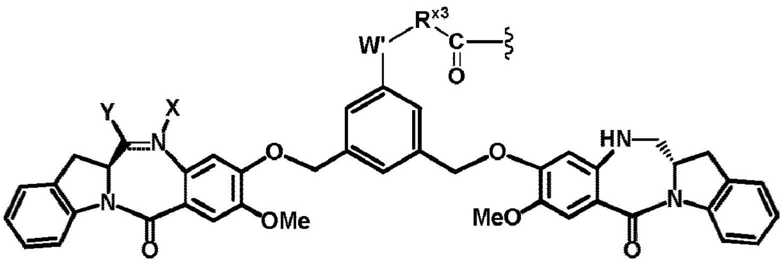

ou un sel pharmaceutiquement acceptable de celui-ci, dans lequel:

la double ligne ⎓ entre N et C représente une liaison simple ou une liaison double, à condition que lorsque c'est une liaison double, X est absent et Y est -H ou un alkyle ($C_1$-$C_4$); et lorsque c'est une liaison simple, X est -H ou une fraction protectrice d'amine, et Y est -OH ou -$SO_3H$, ou un sel pharmaceutiquement acceptable de celui-ci;
W' est -$NR^{e'}$,
$R^e$ est -$(CH_2$-$CH_2$-$O)_n$-$R^k$;
n est un entier de 2 à 6;
$R^k$ est -H ou -Me;
$R^{x3}$ est un alkyle ($C_1$-$C_6$);
L' est représenté par la formule suivante:

- $NR_5$-P-C(=O)-$(CR_aR_b)_m$-C(=O)- (B1'); ou
- $NR_5$-P-C(=O)-$(CR_aR_b)_m$-S-$Z^{s1}$- (B2');

$R_5$ est -H ou un alkyle ($C_1$-$C_3$);
P est un résidu d'acide aminé ou un peptide contenant d'entre 2 à 20 résidus d'acides aminés;
$R_a$ et $R_b$ sont chacun indépendamment à chaque occurrence -H, un alkyle ($C_1$-$C_3$) ou un substituant chargé ou un groupe Q ionisable;
m est un entier de 1 à 6; et
$Z^{s1}$ est sélectionné parmi l'une quelconque des formules suivantes;

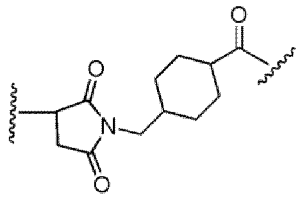 (b1); 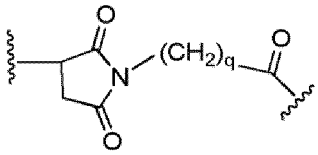 (b2); 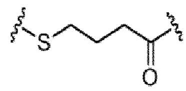 (b3);

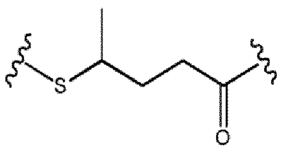 (b4); 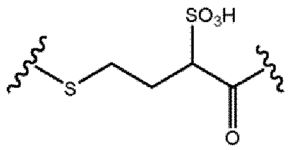 (b5),

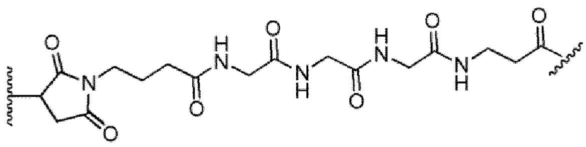 (b6),

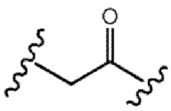 (b7); 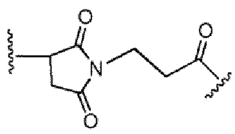 (b8); 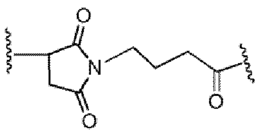 (b9)

et

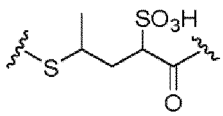 (b10)

dans lequel q est un entier de 1 à 5, et optionnellement dans lequel le sel pharmaceutiquement acceptable est un sel de sodium ou un sel de potassium;

(II)

$$CBA-\left(Cy^{L2}\right)_{W_L}$$

dans lequel:

CBA est ledit anticorps anti ADAM9 humanisé ou le fragment de liaison à ADAM9 de celui-ci qui est lié de manière covalente à $Cy^{L2}$ par un résidu lysine;
$W_L$ est un entier de 1 à 20; et

$Cy^{L2}$ est représenté par la formule suivante:

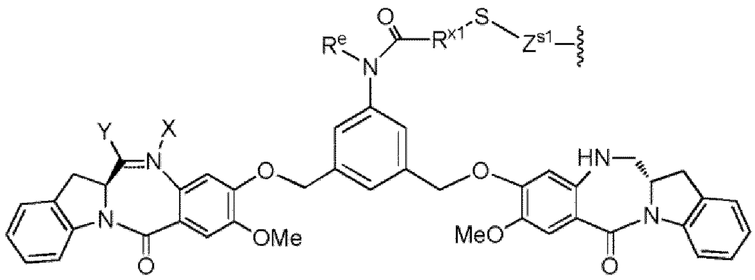

ou

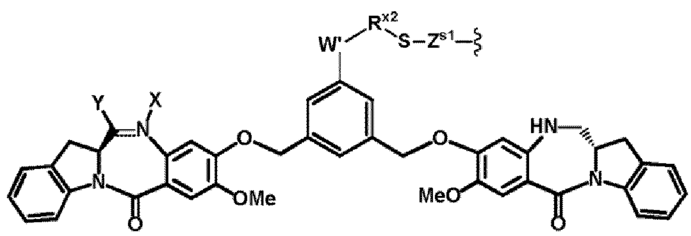

;

ou un sel pharmaceutiquement acceptable de celui-ci, dans lequel:

la double ligne ⎓ entre N et C représente une liaison simple ou une liaison double, à condition que lorsque c'est une liaison double, X est absent et Y est -H ou un alkyle ($C_1$-$C_4$); et lorsque c'est une liaison simple, X est -H ou une fraction protectrice d'amine, et Y est -OH ou -$SO_3H$;

$R^{x1}$ et $R^{x2}$ sont indépendamment un alkyle ($C_1$-$C_6$);

$R^e$ est -H ou un alkyle ($C_1$-$C_6$);

W' est -$NR^{e'}$,

$R^e$ est -$(CH_2$-$CH_2$-$O)_n$-$R^k$;

n est un entier de 2 à 6;

$R^k$ est -H ou -Me;

$Z^{s1}$ est sélectionné parmi l'une quelconque des formules suivantes;

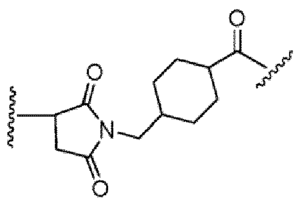

(b1);

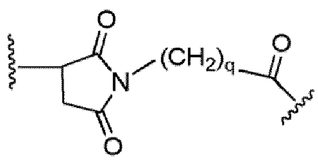

(b2);

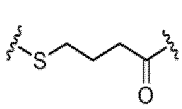

(b3);

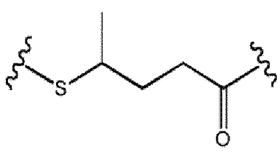

(b4);

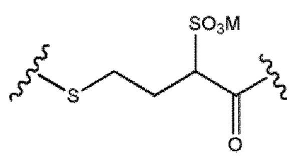

(b5),

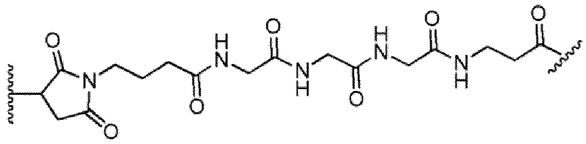

(b6),

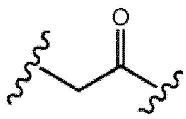 (b7); 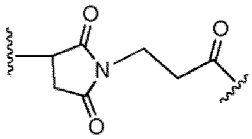 (b8); 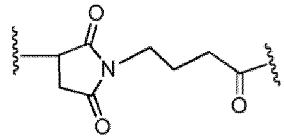 (b9)

et

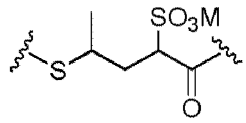 (b10)

dans lequel q est un entier de 1 à 5, et optionnellement dans lequel le sel pharmaceutiquement acceptable est un sel de sodium ou un sel de potassium;

(III)

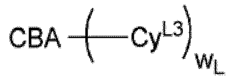

dans lequel:

CBA est ledit anticorps anti ADAM9 humanisé ou le fragment de liaison à ADAM9 de celui-ci qui est lié de manière covalente à $Cy^{L3}$ par un résidu lysine;
$W_L$ est un entier de 1 à 20; et
$Cy^{L3}$ est représenté par la formule suivante:

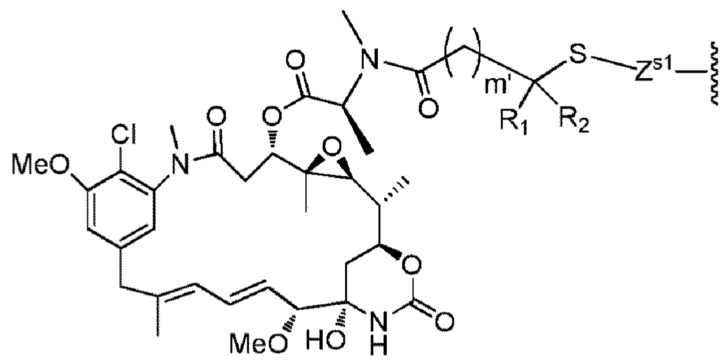,

m' est 1 ou 2;
$R_1$ et $R_2$ sont chacun indépendamment H ou un alkyle ($C_1$-$C_3$); et
$Z^{s1}$ est sélectionné parmi l'une quelconque des formules suivantes;

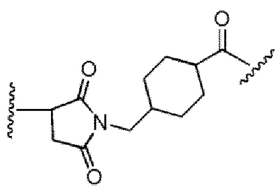 (b1); 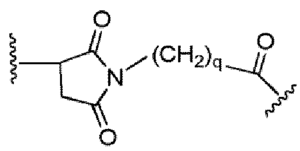 (b2); 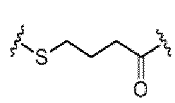 (b3);

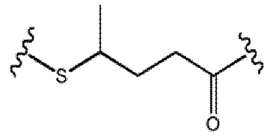 (b4); 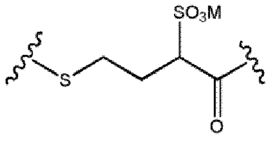 (b5),

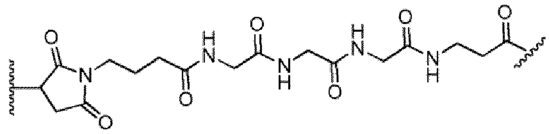 (b6),

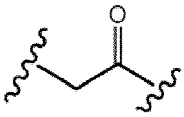 (b7); 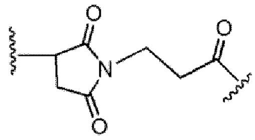 (b8); 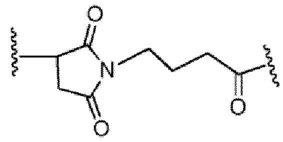 (b9)

et

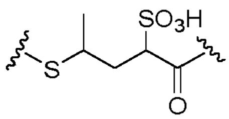 (b10)

dans lequel q est un entier de 1 à 5, et optionnellement dans lequel le sel pharmaceutiquement acceptable est un sel de sodium ou un sel de potassium;

(IV)

$$CBA\left(-Cy^{C1}\right)_{w_C}$$

dans lequel:

CBA est ledit anticorps anti ADAM9 humanisé ou le fragment de liaison à ADAM9 de celui-ci qui est lié de manière covalente à $Cy^{C1}$ par un résidu cystéine;
Wc est 1 ou 2;
$Cy^{C1}$ est représenté par la formule suivante:

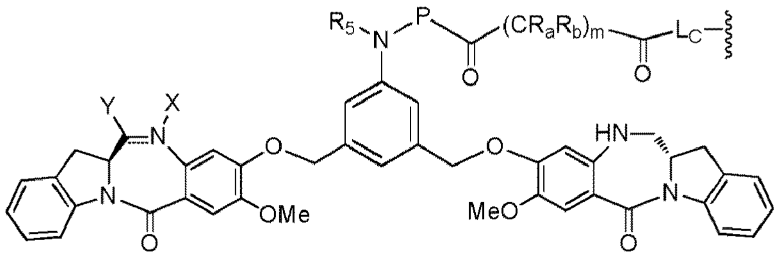

ou

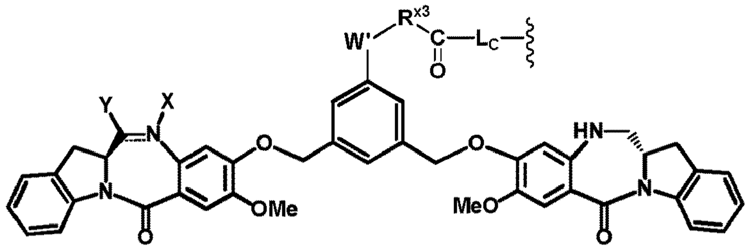

ou un sel pharmaceutiquement acceptable de celui-ci, dans lequel:

la double ligne ⎓ entre N et C représente une liaison simple ou une liaison double, à condition que lorsque c'est une liaison double, X est absent et Y est -H ou un alkyle ($C_1$-$C_4$); et lorsque c'est une liaison simple, X est -H ou une fraction protectrice d'amine, et Y est -OH ou -$SO_3H$ ou un sel pharmaceutiquement acceptable de celui-ci;
$R_5$ est -H ou un alkyle ($C_1$-$C_3$);
P est un résidu d'acide aminé ou un peptide contenant de 2 à 20 résidus d'acides aminés;
$R_a$ et $R_b$ sont indépendamment à chaque occurrence -H, un alkyle ($C_1$-$C_3$), ou un substituant chargé ou un groupe Q ionisable;
W' est -$NR^{e'}$,
$R^e$ est -($CH_2$-$CH_2$-O)$_n$-$R^k$;
n est un entier de 2 à 6;
$R^k$ est -H ou -Me;
$R^{x3}$ est un alkyle ($C_1$-$C_6$); et
$L_C$ est représenté par

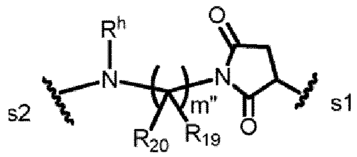

,

s1 est le site lié de manière covalente à CBA, et s2 est le site lié de manière covalente au groupe -C(=O)- sur $Cy^{C1}$;
dans lequel:

$R_{19}$ et $R_{20}$ sont indépendamment à chaque occurrence -H ou un alkyle ($C_1$-$C_3$);
m" est un entier entre 1 et 10; et
$R^h$ est -H ou un alkyle ($C_1$-$C_3$), et optionnellement dans lequel le sel pharmaceutiquement acceptable est un sel de sodium ou un sel de potassium;

(V)

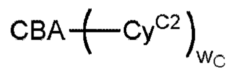

dans lequel:

CBA est ledit anticorps anti ADAM9 humanisé ou le fragment de liaison à ADAM9 de celui-ci qui est lié de manière covalente à $Cy^{C2}$ par un résidu cystéine;
Wc est 1 ou 2;
$Cy^{C2}$ est représenté par la formule suivante:

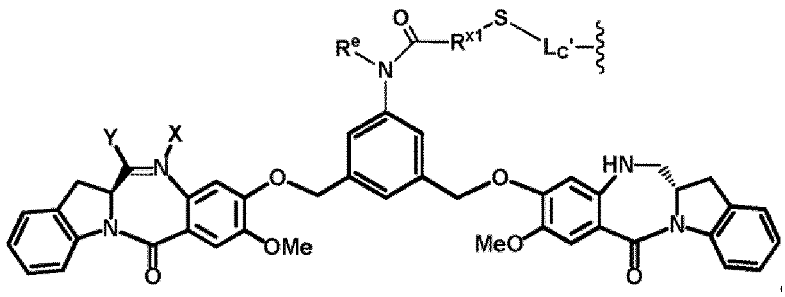

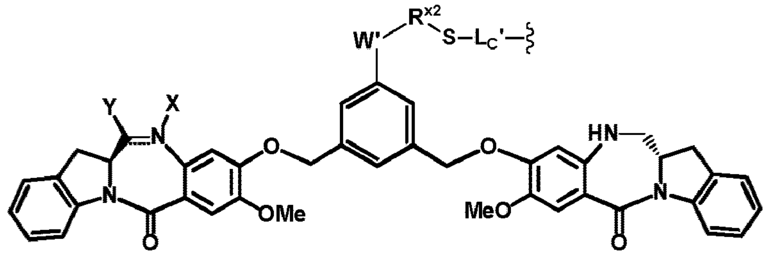

ou un sel pharmaceutiquement acceptable de celui-ci, dans lequel:

la double ligne ═ entre N et C représente une liaison simple ou une liaison double, à condition que lorsque c'est une liaison double, X est absent et Y est -H ou un alkyle ($C_1$-$C_4$); et lorsque c'est une liaison simple, X est -H ou une fraction protectrice d'amine, Y est -OH ou -$SO_3H$ ou un sel pharmaceutiquement acceptable de celui-ci;
$R^{x1}$ est un alkyle ($C_1$-$C_6$);
$R^e$ est -H ou un alkyle ($C_1$-$C_6$);
W' est -$NR^{e'}$,
$R^e$ est -($CH_2$-$CH_2$-O)$_n$-$R^k$;
n est un entier de 2 à 6;
$R^k$ est -H ou -Me;
$R^{x2}$ est un alkyle ($C_1$-$C_6$);
$L_C$' est représenté par la formule suivante:

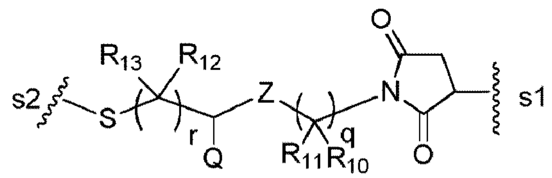

;

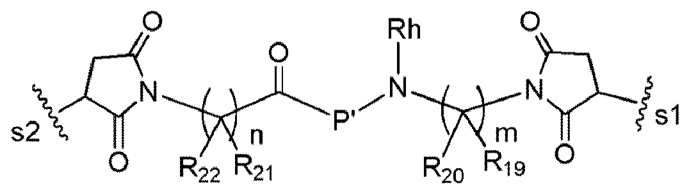

dans lequel:

s1 est le site lié de manière covalente à CBA et s2 est le site lié de manière covalente au groupe -S- sur $Cy^{C2}$;
Z est -C(=O)-$NR_9$ ou -$NR_9$-C(=O)-;
Q est -H, un substituant chargé ou un groupe ionisable;

$R_9$, $R_{10}$, $R_{11}$, $R_{12}$, $R_{13}$, $R_{19}$, $R_{20}$, $R_{21}$ et $R_{22}$ sont indépendamment à chaque occurrence -H ou un alkyle ($C_1$-$C_3$);
q et r sont indépendamment à chaque occurrence un entier d'entre 0 et 10;
m et n sont chacun indépendamment un entier d'entre 0 et 10;
$R^h$ est -H ou un alkyle ($C_1$-$C_3$), et

P' est un résidu d'acide aminé ou un peptide contenant de 2 à 20 résidus d'acides aminés, et optionnellement dans lequel le sel pharmaceutiquement acceptable est un sel de sodium ou un sel de potassium; et

(VI)

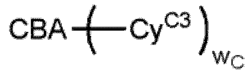

dans lequel:

CBA est ledit anticorps anti ADAM9 humanisé ou le fragment de liaison à ADAM9 de celui-ci qui est lié de manière covalente à $Cy^{C3}$ par un résidu cystéine;
Wc est 1 ou 2;
$Cy^{C3}$ est représenté par la formule suivante:

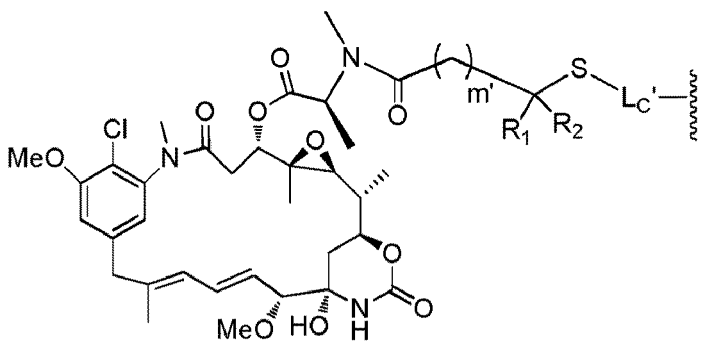

dans lequel:

m' est 1 ou 2;
$R_1$ et $R_2$ sont chacun indépendamment -H ou un alkyle ($C_1$-$C_3$);
$L_C$' est représenté par la formule suivante:

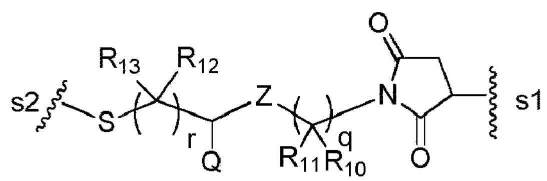

;

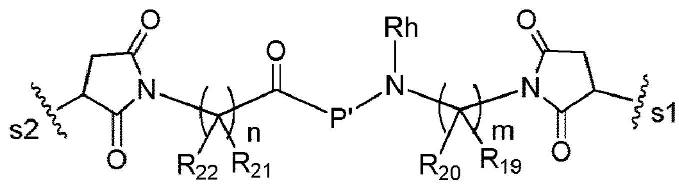

dans lequel:

s1 est le site lié de manière covalente à CBA et s2 est le site lié de manière covalente au groupe -S- sur $Cy^{C3}$;

Z est -C(=O)-$NR_9$ ou -$NR_9$-C(=O)-;
Q est H, un substituant chargé ou un groupe ionisable;
$R_9$, $R_{10}$, $R_{11}$, $R_{12}$, $R_{13}$, $R_{19}$, $R_{20}$, $R_{21}$ et $R_{22}$ sont indépendamment à chaque occurrence -H ou un alkyle ($C_1$-$C_3$);
q et r sont indépendamment à chaque occurrence un entier d'entre 0 et 10;
m et n sont chacun indépendamment un entier d'entre 0 et 10;
$R^h$ est -H ou un alkyle ($C_1$-$C_3$),
P' est un résidu d'acide aminé ou un peptide contenant de 2 à 20 résidus d'acides aminés, et optionnellement dans lequel le sel pharmaceutiquement acceptable est un sel de sodium ou un sel de potassium.

**15.** Composition pharmaceutique comprenant une quantité efficace de l'immunoconjugué selon l'une quelconque des revendications 1-14, et un véhicule, un excipient ou un diluant pharmaceutiquement acceptables.

**16.** Immunoconjugué selon l'une quelconque des revendications 1-14 ou composition pharmaceutique selon la revendication 15, pour utilisation dans le traitement d'une maladie ou d'une condition associée à, ou **caractérisée par**, l'expression d'ADAM9.

**17.** Immunoconjugué ou composition pharmaceutique pour utilisation selon la revendication 16, où ladite maladie ou la condition associée à, ou **caractérisée par**, l'expression d'ADAM9 est un cancer, optionnellement où ledit cancer est sélectionné parmi le groupe constitué de cancer du poumon non à petites cellules, cancer colorectal, cancer de la vessie, cancer de l'estomac, cancer du pancréas, carcinome à cellules rénales, cancer de la prostate, cancer de l'œsophage, cancer du sein, cancer de la tête et du cou, cancer de l'utérus, cancer des ovaires, cancer du foie, cancer du col de l'utérus, cancer de la thyroïde, cancer testiculaire, cancer myéloïde, mélanome et cancer lymphoïde.

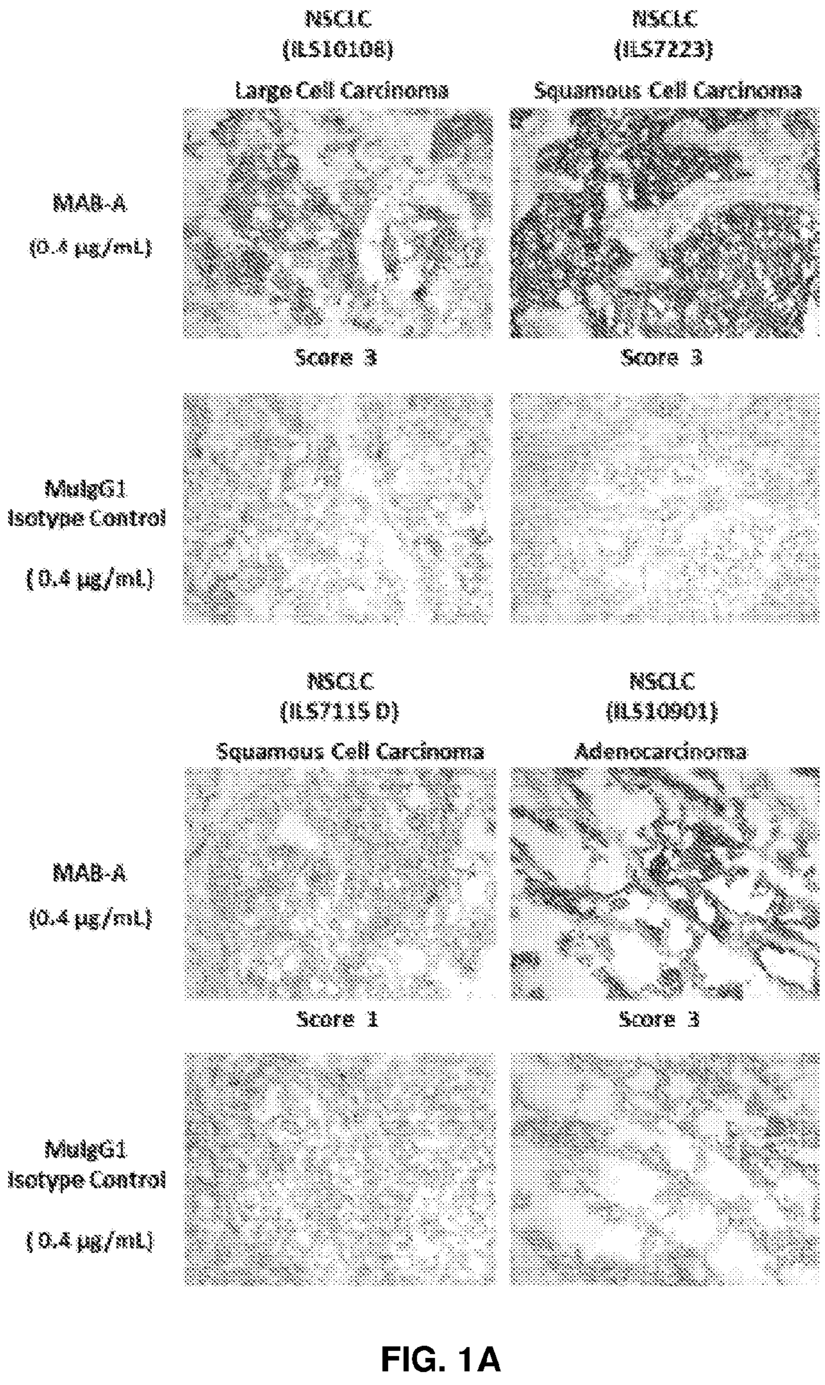
NSCLC
(ILS10108)
Large Cell Carcinoma
NSCLC
(ILS7223)
Squamous Cell Carcinoma
MAB-A
(0.4 μg/mL)
Score 3
Score 3
MuIgG1
Isotype Control
( 0.4 μg/mL)
NSCLC
(ILS7115 D)
Squamous Cell Carcinoma
NSCLC
(ILS10901)
Adenocarcinoma
MAB-A
(0.4 μg/mL)
Score 1
Score 3
MuIgG1
Isotype Control
( 0.4 μg/mL)

**FIG. 1A**

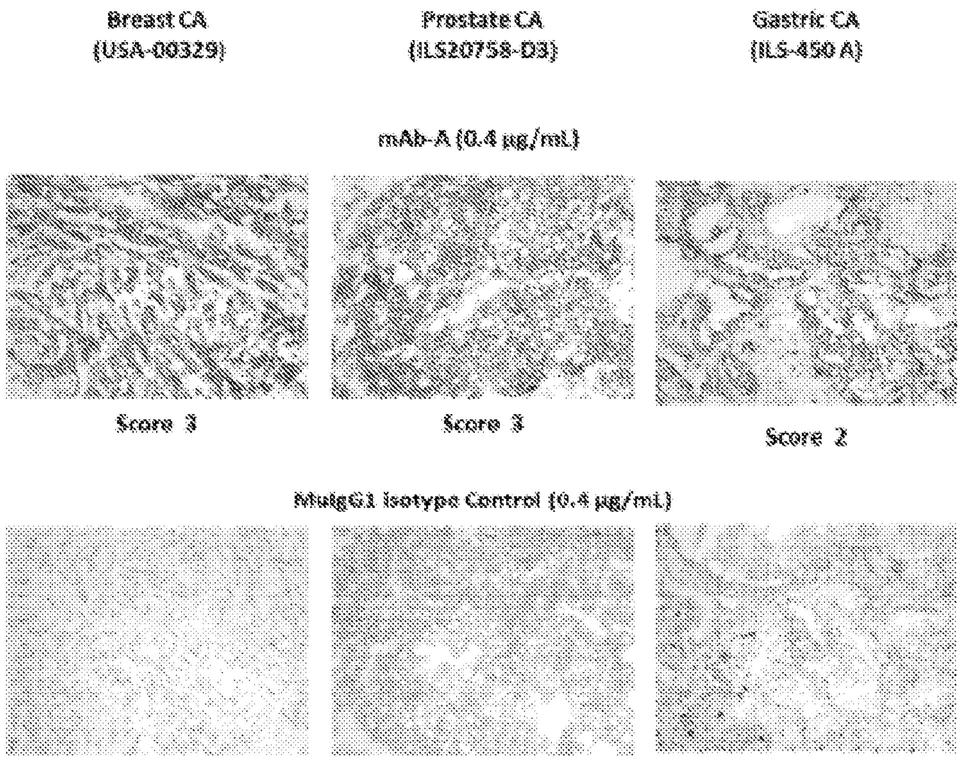
Breast CA
(USA-00329)
Prostate CA
(ILS20758-D3)
Gastric CA
(ILS-450 A)
mAb-A (0.4 μg/mL)
Score 3
Score 3
Score 2
MuIgG1 Isotype Control (0.4 μg/mL)

FIG. 1B

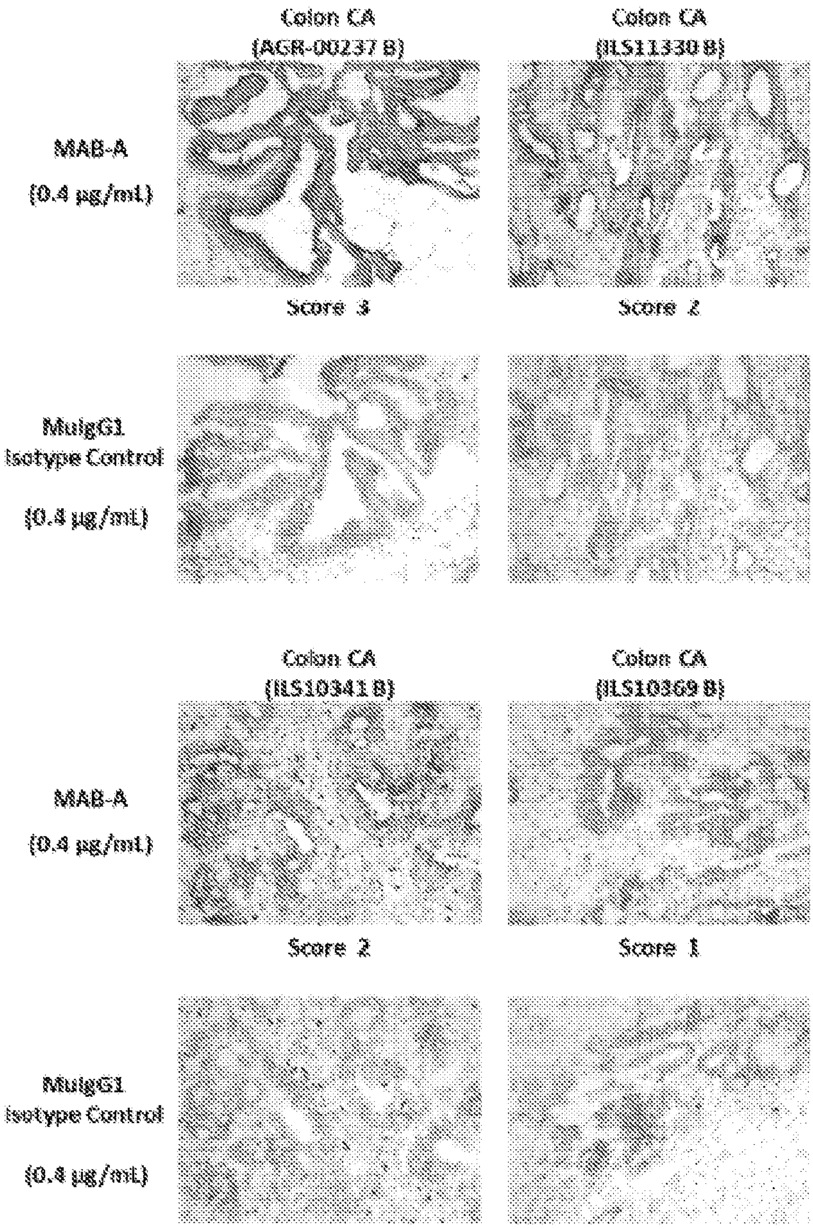
Colon CA
(AGR-00237 B)
Colon CA
(ILS11330 B)
MAB-A
(0.4 µg/mL)
Score 3
Score 2
MuIgG1
Isotype Control
(0.4 µg/mL)
Colon CA
(ILS10341 B)
Colon CA
(ILS10369 B)
MAB-A
(0.4 µg/mL)
Score 2
Score 1
MuIgG1
Isotype Control
(0.4 µg/mL)

FIG. 1C

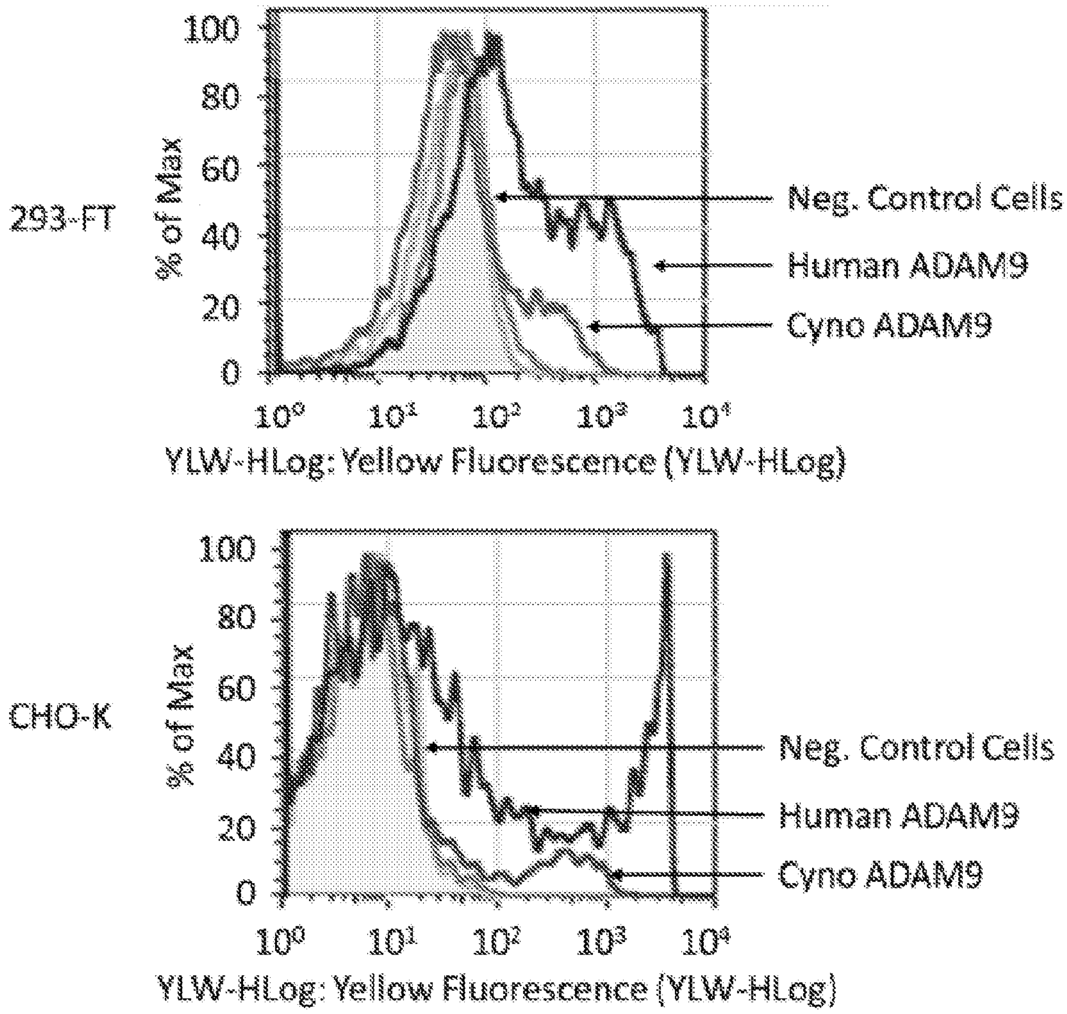
293-FT
100
80
60
40
20
0
% of Max
Neg. Control Cells
Human ADAM9
Cyno ADAM9
10⁰ 10¹ 10² 10³ 10⁴
YLW-HLog: Yellow Fluorescence (YLW-HLog)
CHO-K
100
80
60
40
20
0
% of Max
Neg. Control Cells
Human ADAM9
Cyno ADAM9
10⁰ 10¹ 10² 10³ 10⁴
YLW-HLog: Yellow Fluorescence (YLW-HLog)

FIG. 2

| | | FR1 | CDR1 |
|---|---|---|---|
| Murine MAB-A VH | **SEQ ID NO:7** | QVQLQQPGAELVKPGASVKLSCKASGYTFT | SYWMH |
| hMAB-A VH(1) | **SEQ ID NO:16** | E...VES.GG.....G.LR...A...F..S | ..... |
| hMAB-A VH(2) | **SEQ ID NO:17** | E...VES.GG.....G.LR...A...F..S | ..... |
| hMAB-A VH(3) | **SEQ ID NO:18** | E...VES.GG.....G.LR...A...F..S | ..... |
| hMAB-A VH(4) | **SEQ ID NO:19** | E...VES.GG.....G.LR...A...F..S | ...I. |
| hMAB-A VH(2B) | **SEQ ID NO:21** | E...VES.GG.....G.LR...A...F..S | ..... |
| hMAB-A VH(2C) | **SEQ ID NO:22** | E...VES.GG.....G.LR...A...F..S | ..... |
| hMAB-A VH(2D) | **SEQ ID NO:23** | E...VES.GG.....G.LR...A...F..S | ..... |
| hMAB-A VH(2I) | **SEQ ID NO:28** | E...VES.GG.....G.LR...A...F..S | ..... |

| FR2 | CDR2 | FR3 |
|---|---|---|
| WVKQRPGQGLEWIG | EIIPINGHTNYNEKFKS | KATLTLDKSSSTAYMQLSSLASEDSAVYYCAR |
| ..R.A..K....V. | ................. | RF.IS..N.KN.L.L.MG..RA..T....... |
| ..R.A..K....V. | .....F........... | RF.IS..N.KN.L.L.MG..RA..T....... |
| ..R.A..K....V. | .....F.......R.QG | RF.IS..N.KN.L.L.MG..RA..T....... |
| ..R.A..K....V. | .....F.......R.QG | RF.IS..N.KN.L.L.MG..RA..T....... |
| ..R.A..K....V. | .....F........... | RF.IS..N.KN.L.L.MG..RA..T....... |
| ..R.A..K....V. | .....F........... | RF.IS..N.KN.L.L.MG..RA..T....... |
| ..R.A..K....V. | .....F........... | RF.IS..N.KN.L.L.MG..RA..T....... |
| ..R.A..K....V. | .....F........... | RF.IS..N.KN.L.L.MG..RA..T....... |

| CDR3 | FR4 |
|---|---|
| GGYYYYGSRDYFDY | WGQGTTLTVSS |
| .............. | ......V.... |
| .............. | ......V.... |
| .............. | ......V.... |
| .............. | ......V.... |
| ......TGKGVL.. | ......V.... |
| ......FRFGWL.. | ......V.... |
| ......TGKGVL.. | ......V.... |
| ......FRQGFL.. | ......V.... |

**FIG. 3A**

```
                                                           FR1                      CDR1
Murine MAB-A VL     SEQ ID NO:11    DIVLTQSPASLAVSLGQRATISC  KASQSVDYDGDSYMN
hMAB-A VL(1)        SEQ ID NO:54    ...M....D.......E......  ...............
hMAB-A VL(2)        SEQ ID NO:55    ...M....D.......E......  ........S......
hMAB-A VL(3)        SEQ ID NO:56    ...M....D.......E......  R.......S......
hMAB-A VL(4)        SEQ ID NO:57    ...M....D.......E......  R.......S....L.

      FR2             CDR2                   FR3                         CDR3        FR4
WYQQIPGQPPKLLIY     AASNLES   GIPARFSGSGSGTDFTLNIHPVEEEDAATYYC    QQSHEDPFT   FGGGTKLEIK
....K..........     .......   .................T.SSL.P..F.....    .........   ..........
....K..........     .......   .................T.SSL.P..F.....    .........   ..........
....K..........     .......   .................T.SSL.P..F.....    .........   ..........
....K..........     .......   .................T.SSL.P..F.....    ...YST...   ..........
```

**FIG. 3B**

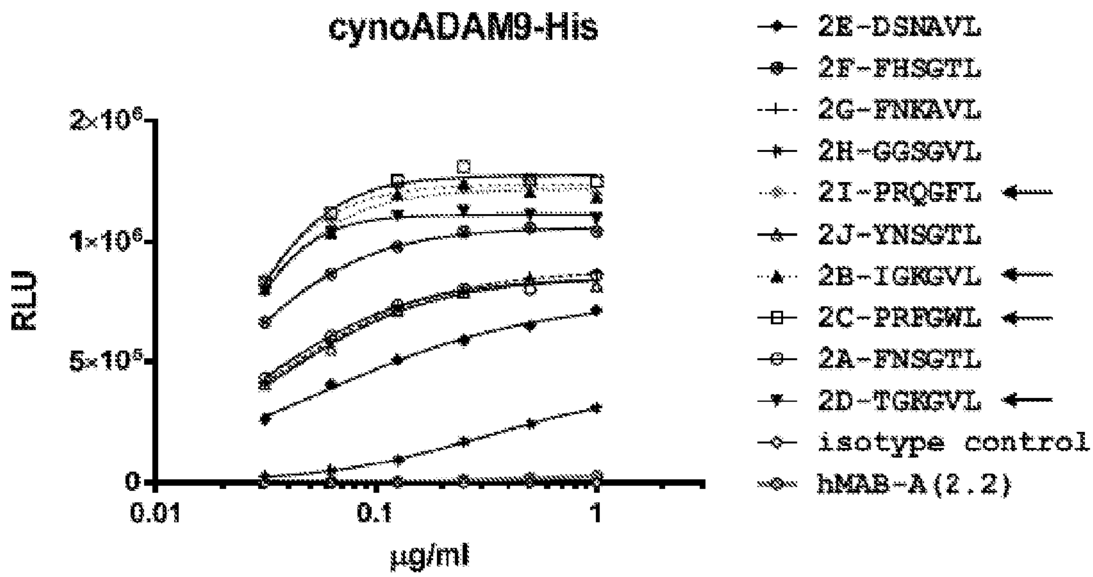
cynoADAM9-His
2×10^6
1×10^6
5×10^5
0
RLU
0.01
0.1
1
µg/ml
2E-DSNAVL
2F-FHSGTL
2G-FNKAVL
2H-GGSGVL
2I-PRQGFL
2J-YNSGTL
2B-IGKGVL
2C-PRFGWL
2A-FNSGTL
2D-TGKGVL
isotype control
hMAB-A(2.2)

FIG. 4A

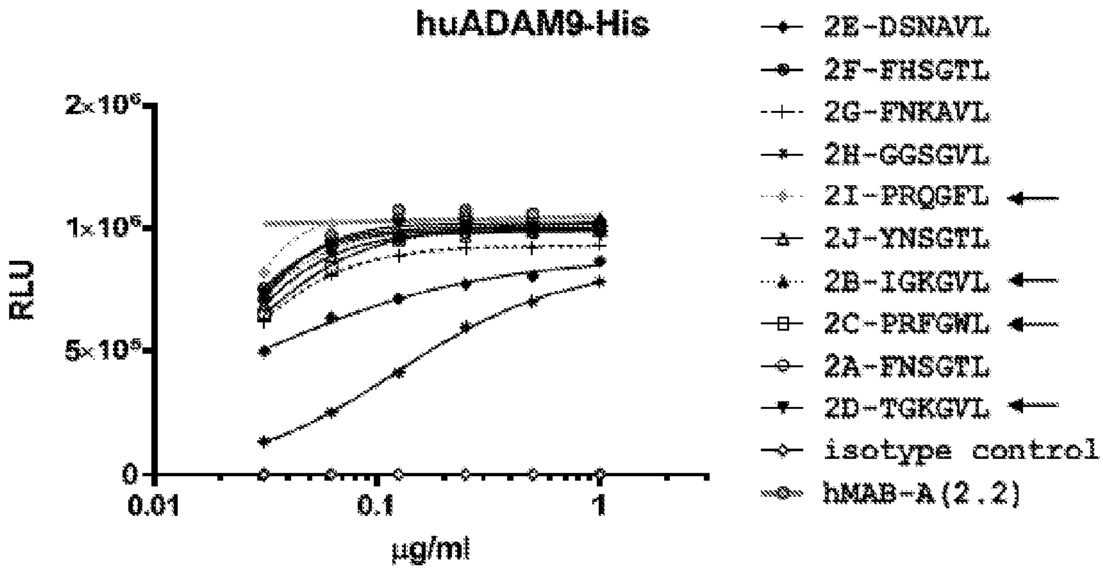
huADAM9-His
2×10^6
1×10^6
5×10^5
0
RLU
0.01
0.1
1
µg/ml
2E-DSNAVL
2F-FHSGTL
2G-FNKAVL
2H-GGSGVL
2I-PRQGFL
2J-YNSGTL
2B-IGKGVL
2C-PRFGWL
2A-FNSGTL
2D-TGKGVL
isotype control
hMAB-A(2.2)

FIG. 4B

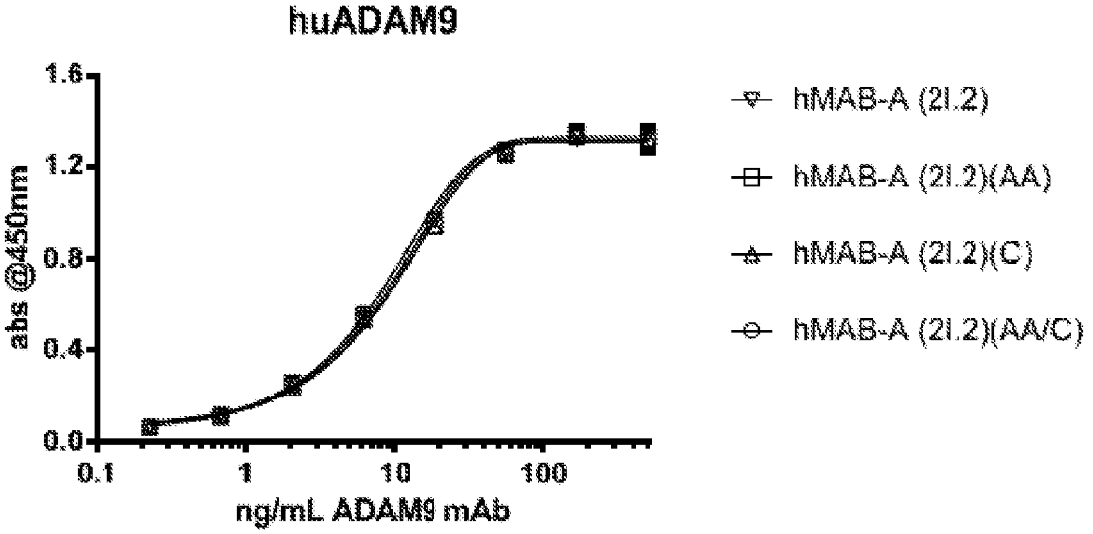
huADAM9
abs @450nm
ng/mL ADAM9 mAb
0.0
0.4
0.8
1.2
1.6
0.1
1
10
100
hMAB-A (2I.2)
hMAB-A (2I.2)(AA)
hMAB-A (2I.2)(C)
hMAB-A (2I.2)(AA/C)

FIG. 5A

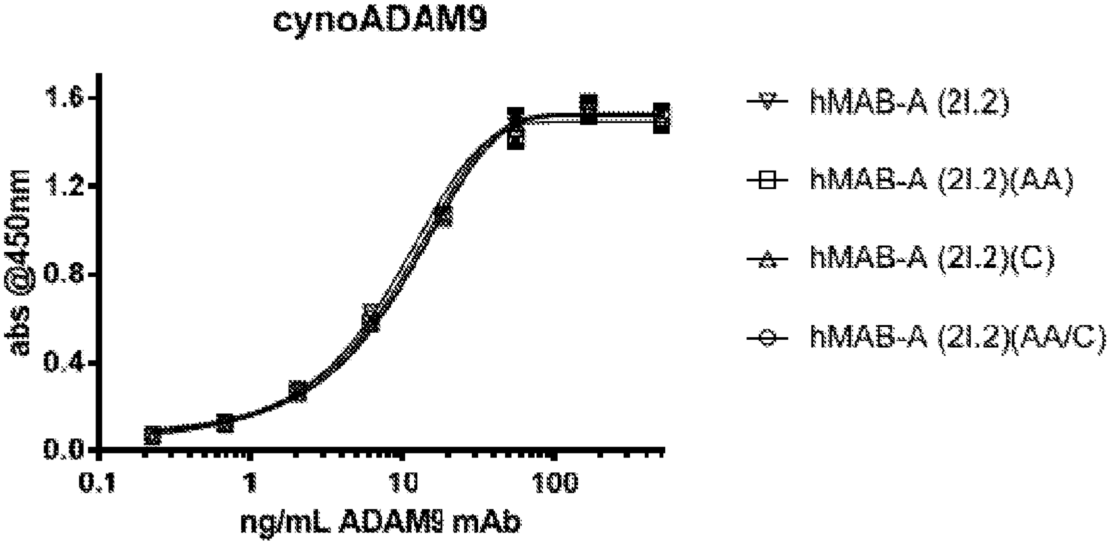
cynoADAM9
abs @450nm
ng/mL ADAM9 mAb
0.0
0.4
0.8
1.2
1.6
0.1
1
10
100
hMAB-A (2I.2)
hMAB-A (2I.2)(AA)
hMAB-A (2I.2)(C)
hMAB-A (2I.2)(AA/C)

FIG. 5B

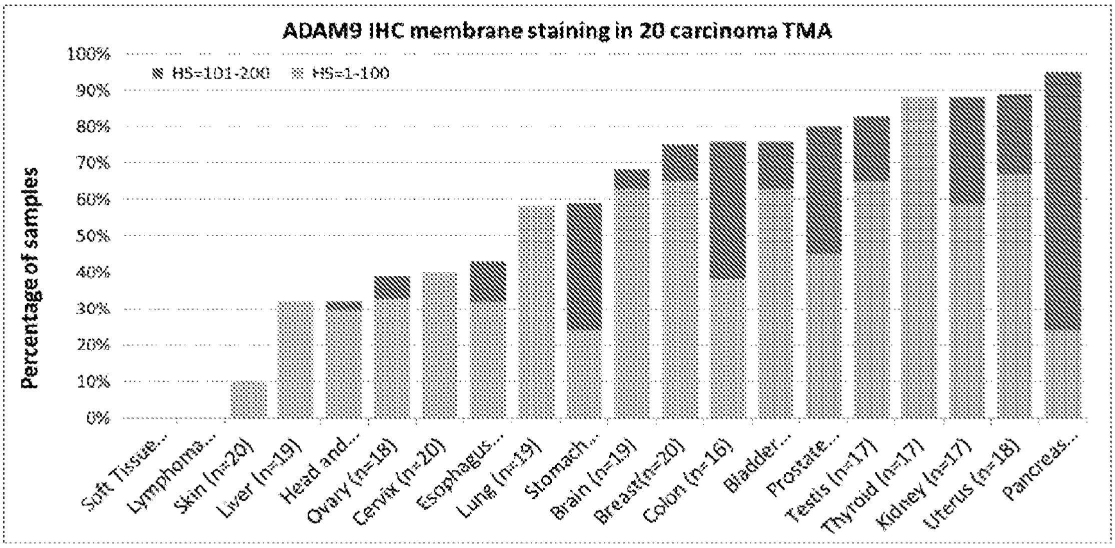
ADAM9 IHC membrane staining in 20 carcinoma TMA
HS=101-200
HS=1-100
Percentage of samples
100%
90%
80%
70%
60%
50%
40%
30%
20%
10%
0%
Soft Tissue...
Lymphoma...
Skin (n=20)
Liver (n=19)
Head and...
Ovary (n=18)
Cervix (n=20)
Esophagus...
Lung (n=19)
Stomach...
Brain (n=19)
Breast(n=20)
Colon (n=16)
Bladder...
Prostate...
Testis (n=17)
Thyroid (n=17)
Kidney (n=17)
Uterus (n=18)
Pancreas...

FIG. 6A

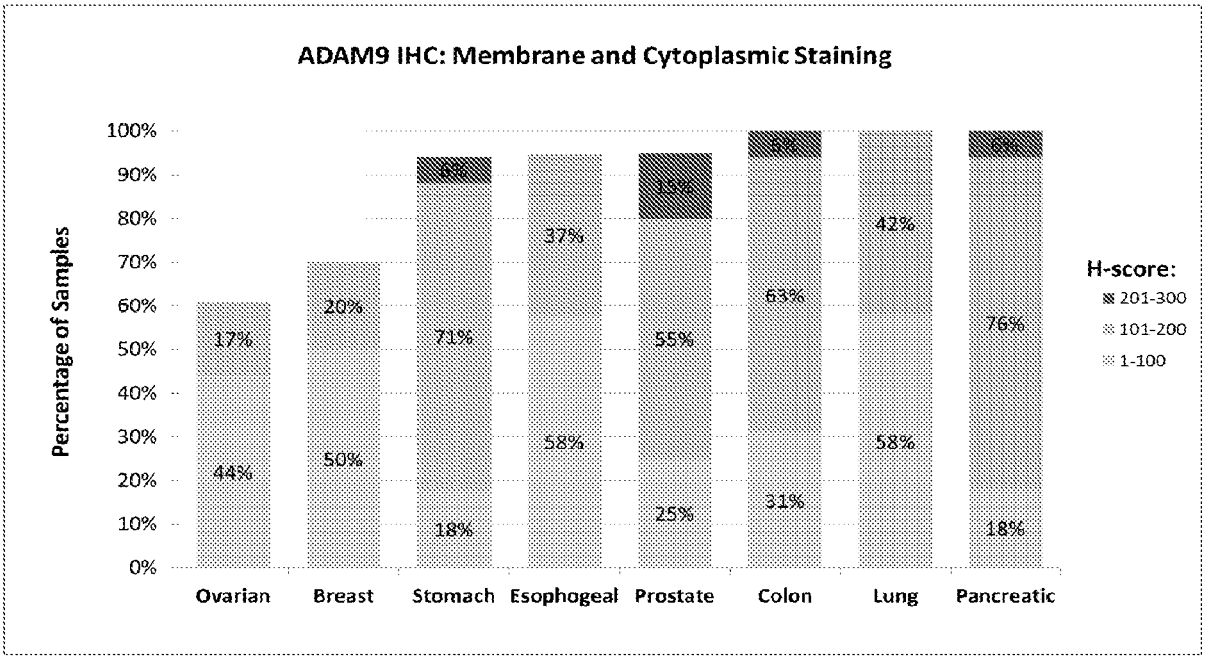
ADAM9 IHC: Membrane and Cytoplasmic Staining
Percentage of Samples
100%
90%
80%
70%
60%
50%
40%
30%
20%
10%
0%
17%
44%
20%
50%
6%
71%
18%
37%
58%
15%
55%
25%
6%
63%
31%
42%
58%
6%
76%
18%
Ovarian
Breast
Stomach
Esophogeal
Prostate
Colon
Lung
Pancreatic
H-score:
201-300
101-200
1-100

FIG. 6B

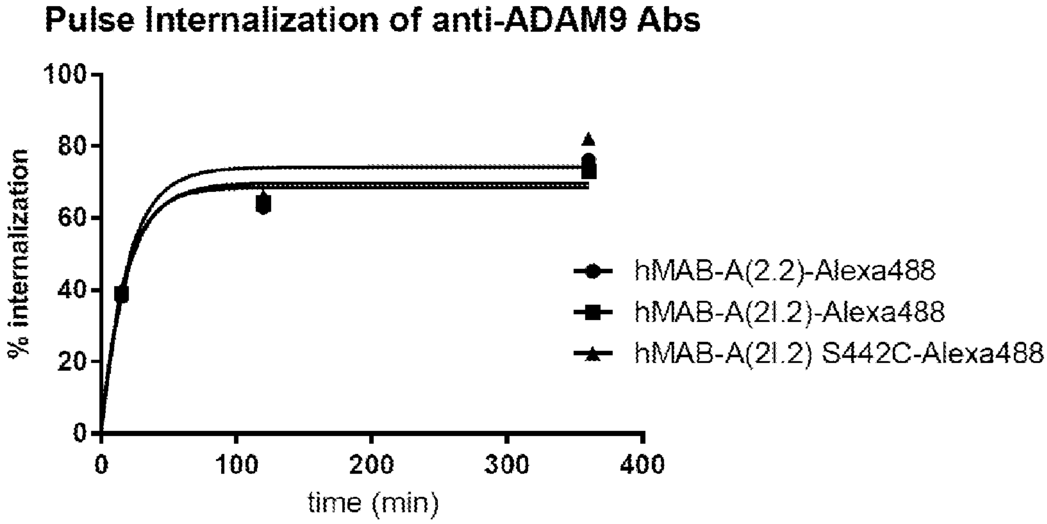
Pulse Internalization of anti-ADAM9 Abs
100
80
60
40
20
0
% internalization
0
100
200
300
400
time (min)
hMAB-A(2.2)-Alexa488
hMAB-A(2I.2)-Alexa488
hMAB-A(2I.2) S442C-Alexa488

FIG. 7A

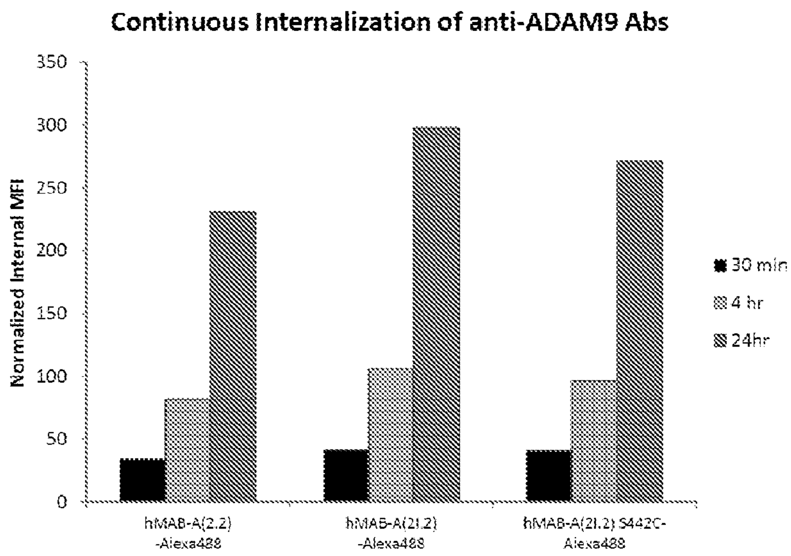
Continuous Internalization of anti-ADAM9 Abs
350
300
250
200
150
100
50
0
Normalized Internal MFI
30 min
4 hr
24hr
hMAB-A(2.2)
-Alexa488
hMAB-A(2I.2)
-Alexa488
hMAB-A(2I.2) S442C-
Alexa488

FIG. 7B

FIG. 8A

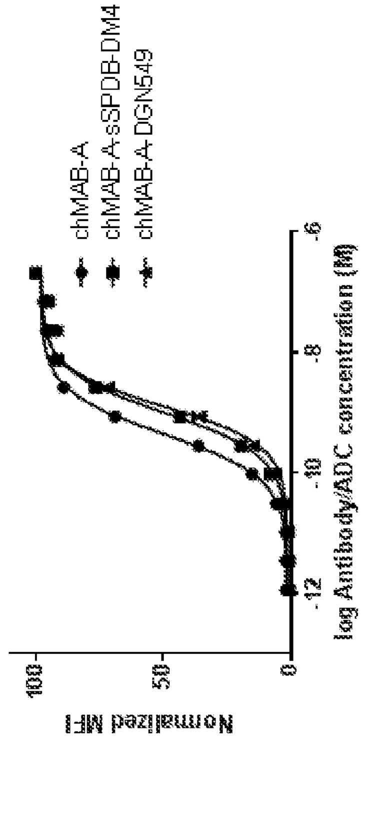


FIG. 8B

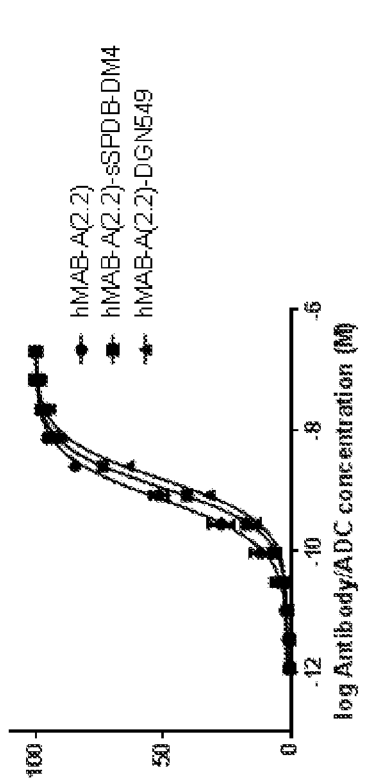


FIG. 8C

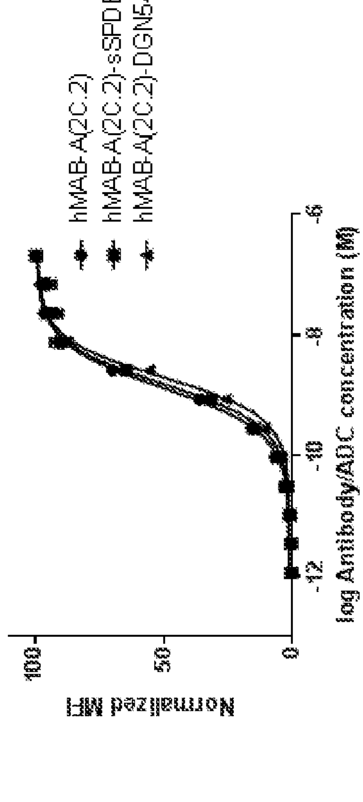


FIG. 8D

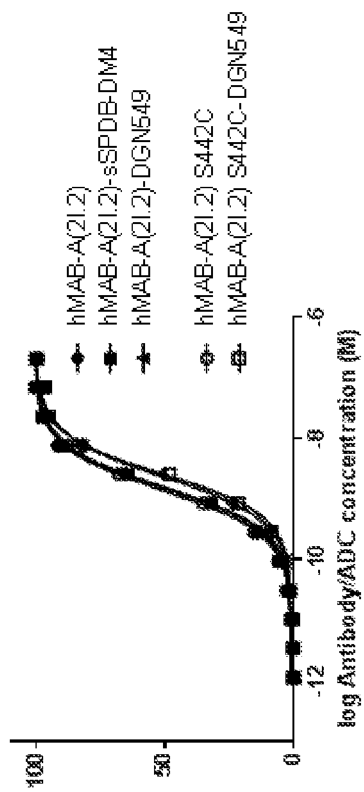

FIG. 9A

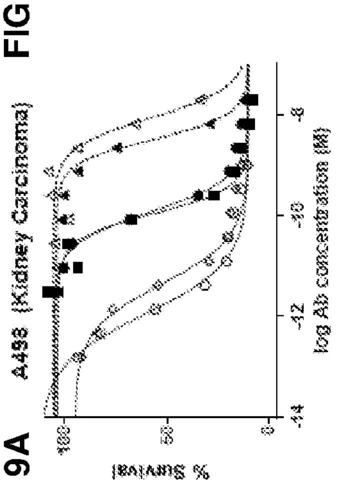


FIG. 9B

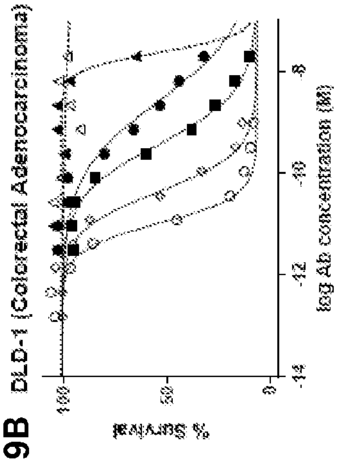


FIG. 9C

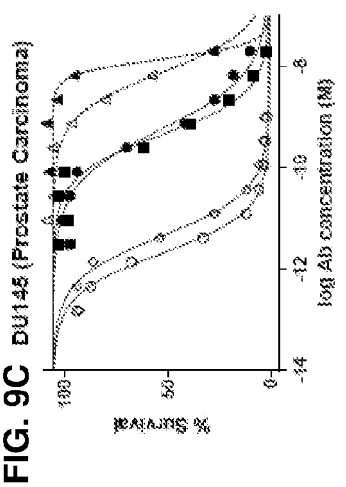


FIG. 9D

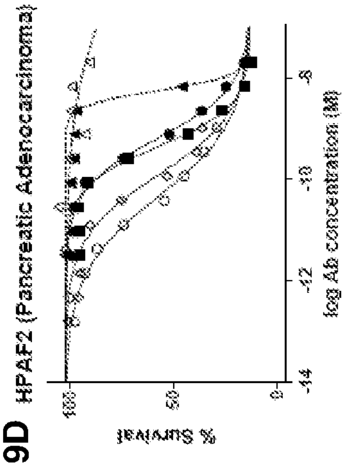


FIG. 9E

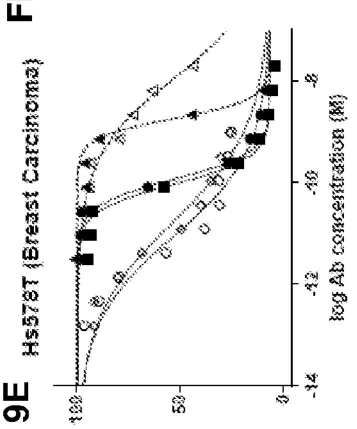


FIG. 9F

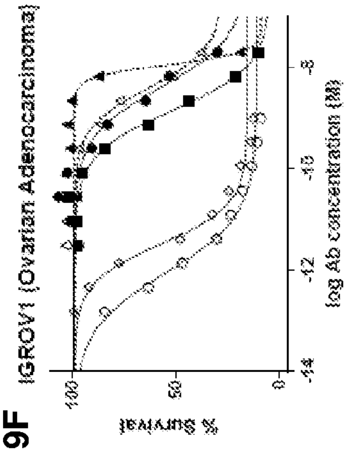


FIG. 9G

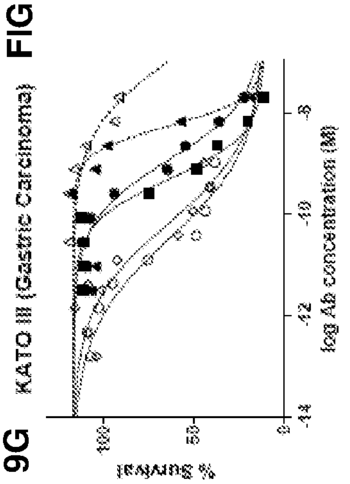


FIG. 9H

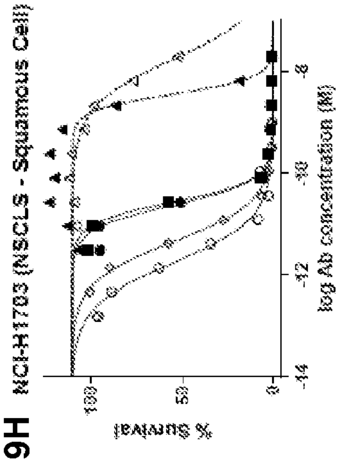


hMAB-A(2.2)-sSPDB-DM4
hMAB-A(2I.2)-sSPDB-DM4
non-targeting IgG1-sSPDB-DM4
hMAB-A(2.2)-DGN549
hMAB-A(2I.2) S442C-DGN549
non-targeting IgG1-DGN549

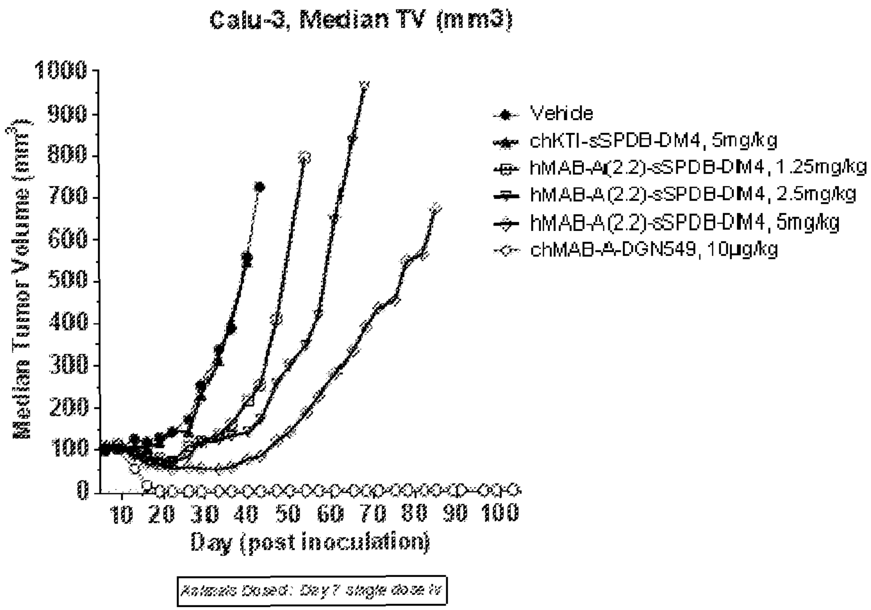


| Group | Agent | Route | Dosage per injection (mg/kg) | Dose schedule | Tx day | Total dose (mg/kg) | Time to reach $1000mm^3$ (days) | T/C (%) | T-C (days) | LCK | Regressions Day | | Results |
|---|---|---|---|---|---|---|---|---|---|---|---|---|---|
| | | | | | | | | | | | Partial | Complete | |
| A | Vehicle | iv | - | qdx1 | 7 | - | 43 | - | - | - | - | - | - |
| B | chKTI-s-SPDB-DM4 | iv | 5 | qdx1 | 7 | 5 | 50 | 98% | 7 | 0.2 | 0/8 | 0/8 | Inactive |
| C | hMAB-A(2.2)-sSPDB-DM4 | iv | 1.25 | qdx1 | 7 | 1.25 | 60 | 39% | 17 | 0.5 | 3/8 | 0/8 | Active |
| D | hMAB-A(2.2)-sSPDB-DM4 | iv | 2.5 | qdx1 | 7 | 2.5 | 65 | 25% | 22 | 0.7 | 3/8 | 0/8 | Active |
| E | hMAB-A(2.2)-sSPDB-DM4 | iv | 5 | qdx1 | 7 | 5 | 79 | 14% | 36 | 1.1 | 4/8 | 1/8 | Active |
| F | chMAB-A-DGN549 | iv | 10μg/kg | qdx1 | 7 | 10μg/kg | CBD | 0% | CBD | CBD | 8/8 | 8/8 | Highly Active |
| Tumor doubling time ($T_d$) = | 9.774 | (nonlinear exponential curve fit; $R^2$ = 0.9782) | | | | | | | | | | | |

FIG. 10

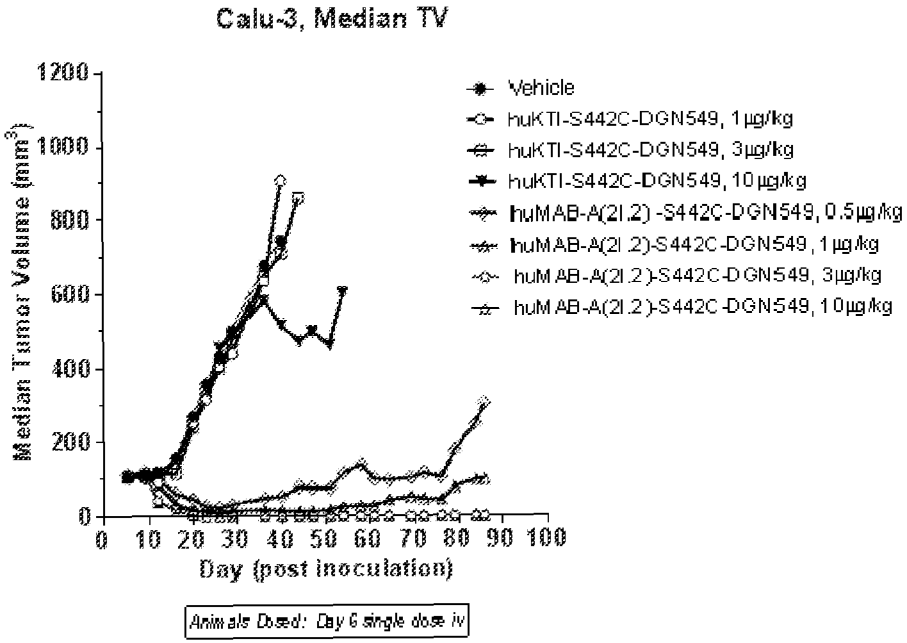


| GROUP | | %T/C | Regressions | | Results |
|---|---|---|---|---|---|
| | | | PR | CR | |
| A | Vehicle | | - | - | - |
| B | huKTI-S442C-DGN549, 1µg/kg | 121% | 0/8 | 0/8 | Inactive |
| C | huKTI-S442C-DGN549, 3µg/kg | 95% | 0/8 | 0/8 | Inactive |
| D | huKTI-S442C-DGN549, 10µg/kg | 69% | 0/8 | 0/8 | Inactive |
| E | huMAB-A(2I.2)-S442C-DGN549, 0.5µg/kg | 7% | 7/8 | 0/8 | Highly Active |
| F | huMAB-A(2I.2)-S442C-DGN549, 1µg/kg | 2% | 8/8 | 2/8 | Highly Active |
| G | huMAB-A(2I.2)-S442C-DGN549, 3µg/kg | 0% | 8/8 | 8/8 | Highly Active |
| H | huMAB-A(2I.2)-S442C-DGN549, 10µg/kg | 0% | 8/8 | 8/8 | Highly Active |

FIG. 11

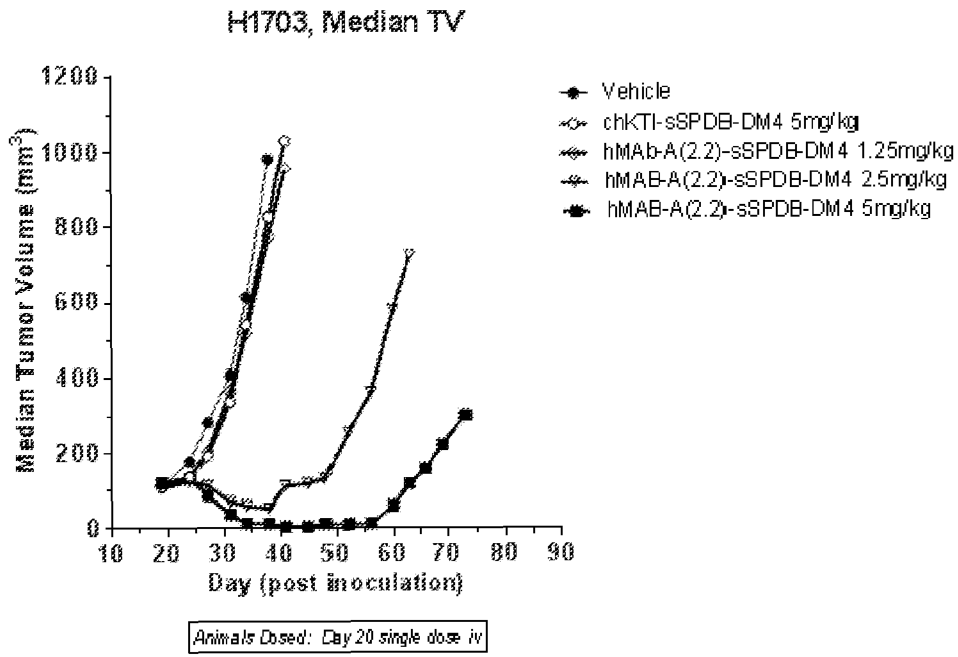


| Group | Agent | Route | Dosage per injection (mg/kg) | Dose schedule | Tx day | Total dose (mg/kg) | Time to reach $1000mm^3$ (days) | T/C (%) | T-C (days) | LCK | Regressions Day | | Results |
|---|---|---|---|---|---|---|---|---|---|---|---|---|---|
| | | | | | | | | | | | Partial | Complete | |
| A | Vehicle | iv | - | qdx1 | 20 | - | 38 | - | - | - | - | - | - |
| B | chKTI-sSPDB-DM4 | iv | 5 | qdx1 | 20 | 5 | 40 | 84% | 2 | 0.1 | 0/6 | 0/6 | Inactive |
| C | hMAB-A(2.2)-sSPDB-DM4 | iv | 1.25 | qdx1 | 20 | 1.25 | 42 | 79% | 4 | 0.2 | 1/6 | 0/6 | Inactive |
| D | hMAB-A(2.2)-sSPDB-DM4 | iv | 2.5 | qdx1 | 20 | 2.5 | 66 | 6% | 28 | 1.4 | 3/6 | 0/6 | Highly Active |
| E | hMAB-A(2.2)-sSPDB-DM4 | iv | 5 | qdx1 | 20 | 5 | NA | 1% | NA | NA | 6/6 | 3/6 | Highly Active |
| Tumor doubling time ($T_d$) = 5.94 (nonlinear exponential curve fit; $R^2$ = 0.9943) | | | | | | | | | | | | | |

**FIG. 12**

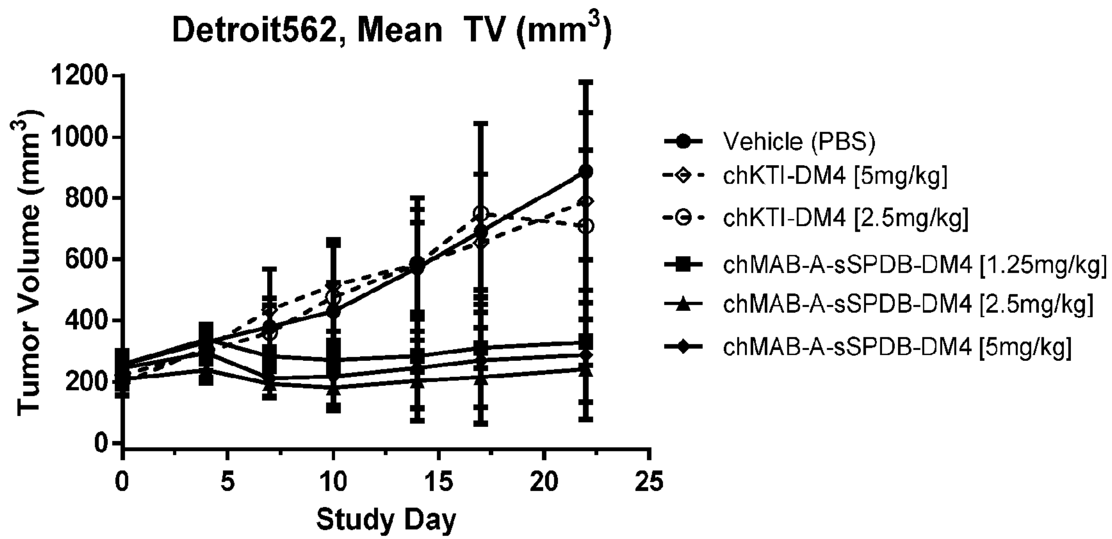


| Group | Agent | Route | Dosage per injection (mg/kg) | Dose Schedule | Tx day | Total dose (mg/kg) | T/C (%) | Regressions Day | |
|---|---|---|---|---|---|---|---|---|---|
| | | | | | | | | Partial | Complete |
| 1 | Vehicle | ip | - | qdx1 | 0 | - | - | - | - |
| 2 | chKTI-sSPDB-DM4 | ip | 5 | qdx1 | 0 | 5 | 89 | 3/7 | 0/7 |
| 3 | chKTI-sSPDB-DM4 | ip | 2.5 | qdx1 | 0 | 2.5 | 80 | 3/7 | 1/7 |
| 4 | chMAB-A-sSPDB-DM4 | ip | 5 | qdx1 | 0 | 5 | 32 | 6/7 | 1/7 |
| 5 | chMAB-A-sSPDB-DM4 | ip | 2.5 | qdx1 | 0 | 2.5 | 27 | 6/7 | 2/7 |
| 6 | chMAB-A-sSPDB-DM4 | ip | 1.25 | qdx1 | 0 | 1.25 | 37 | 5/7 | 0/7 |

FIG. 13

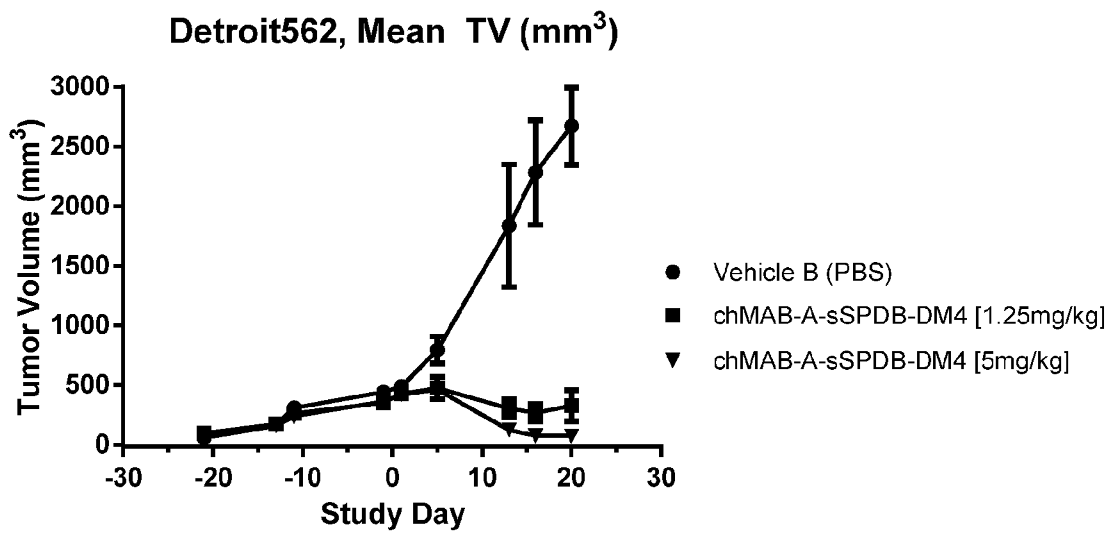


| Group | Agent | Route | Dosage per injection (mg/kg) | Dose Schedule | Tx day | Total dose (mg/kg) | T/C (%) | Regressions Day | |
|---|---|---|---|---|---|---|---|---|---|
| | | | | | | | | Partial | Complete |
| 1 | Vehicle | ip | - | qdx1 | 1 | - | - | - | - |
| 2 | chMAB-A-sSPDB-DM4 | ip | 5 | qdx1 | 1 | 5 | 3 | 6/6 | 1/7 |
| 3 | chMAB-A-sSPDB-DM4 | ip | 1.25 | qdx1 | 1 | 1.25 | 14 | 6/6 | 0/7 |

FIG. 14

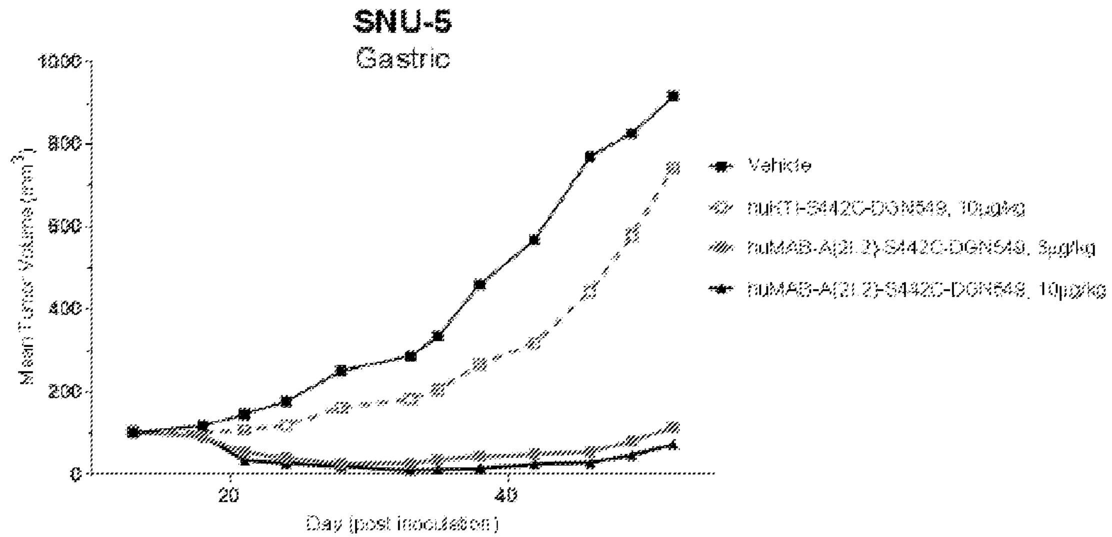


| Treatment | %T/C (Day 46) | Regressions | | Results |
|---|---|---|---|---|
| | | PR | CR | |
| huKTI-S442C-DGN549, 10μg/kg | 58% | 0/8 | 0/8 | Inactive |
| huMAB-A(2I.2) -S442C-DGN549, 3μg/kg | 7% | 8/8 | 4/8 | Highly Active |
| huMAB-A(2I.2) -S442C-DGN549, 10μg/kg | 4% | 8/8 | 5/8 | Highly Active |

FIG. 15

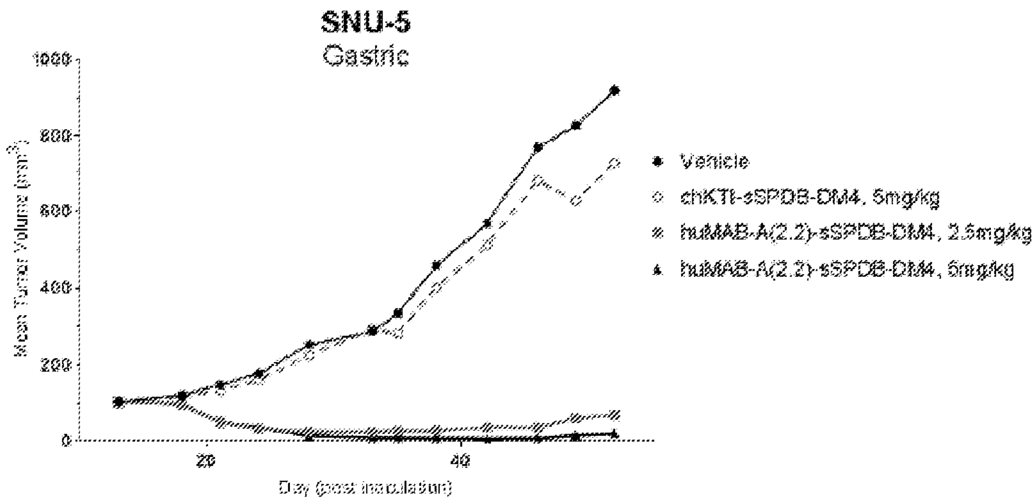


| Treatment | %T/C (Day 46) | Regressions | | Results |
|---|---|---|---|---|
| | | PR | CR | |
| chKTI-sSPDB-DM4, 5mg/kg | 89% | 0/8 | 0/8 | Inactive |
| huMAB-A(2.2)-sSPDB-DM4, 2.5mg/kg | 5% | 8/8 | 2/8 | Highly Active |
| huMAB-A(2.2)-sSPDB-DM4, 5mg/kg | 1% | 8/8 | 6/8 | Highly Active |

FIG. 16

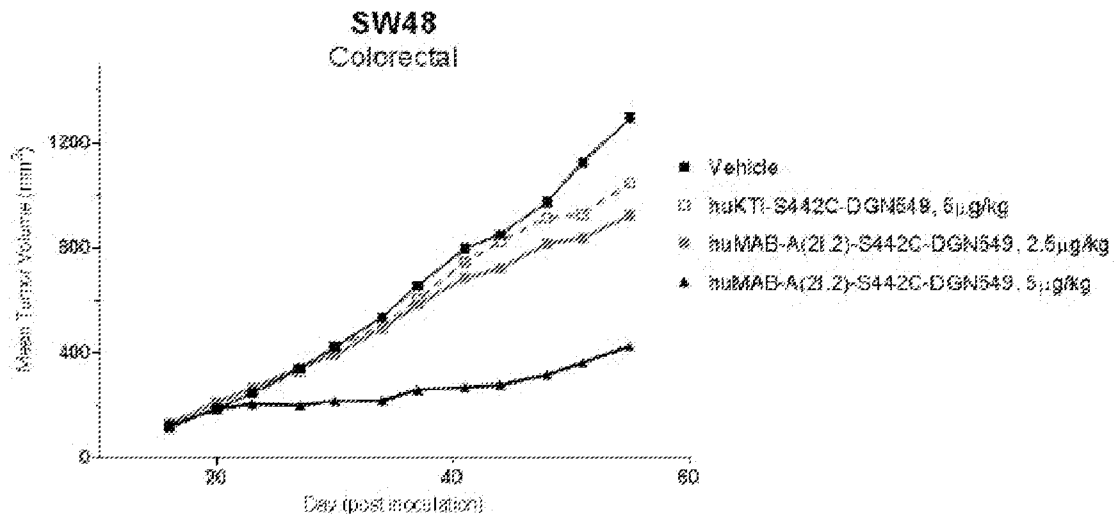


| Treatment | %T/C (Day 34) | Regressions | | Results |
|---|---|---|---|---|
| | | PR | CR | |
| huKTI-S442C-DGN549, 5μg/kg | 94% | 0/6 | 0/6 | Inactive |
| huMAB-A(2I.2)-S442C-DGN549, 2.5μg/kg | 92% | 0/6 | 0/6 | Inactive |
| huMAB-A(2I.2)-S442C-DGN549, 5μg/kg | 41% | 2/6 | 0/6 | Active |

FIG. 17

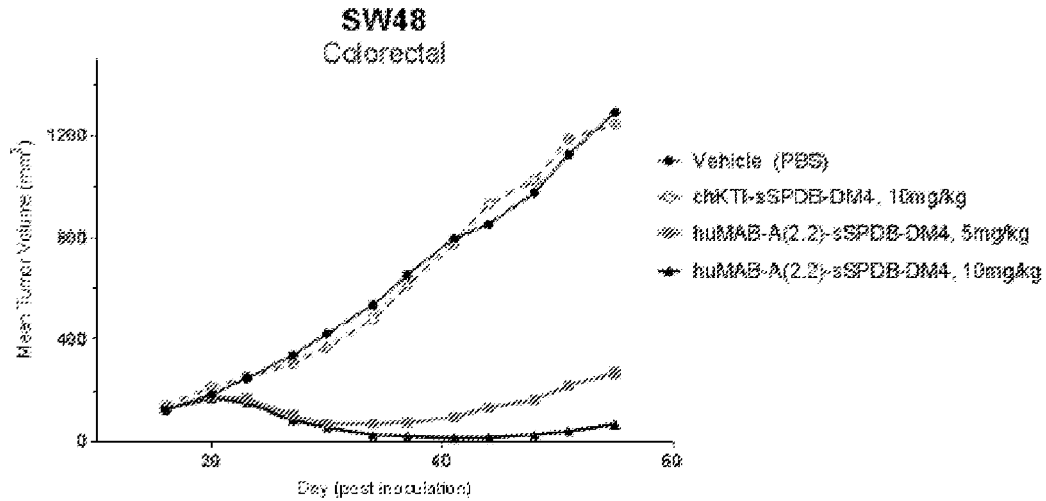


| Treatment | %T/C (Day 34) | Regressions | | Results |
|---|---|---|---|---|
| | | PR | CR | |
| chKTI-sSPDB-DM4, 10mg/kg | 90% | 0/6 | 0/6 | Inactive |
| huMAB-A(2.2)-sSPDB-DM4, 5mg/kg | 13% | 0/6 | 0/6 | Active |
| huMAB-A(2.2)-sSPDB-DM4, 10mg/kg | 5% | 6/6 | 0/6 | Highly Active |

**FIG. 18**

FIG. 19A

hMAB-A(2I.2)

hMAB-A(2I.2)-YTE

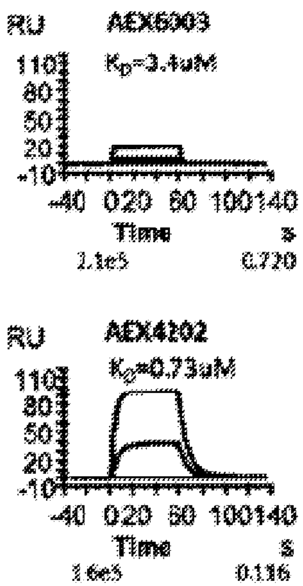


FIG. 19B

hMAB-A(2I.2)

hMAB-A(2I.2)-YTE

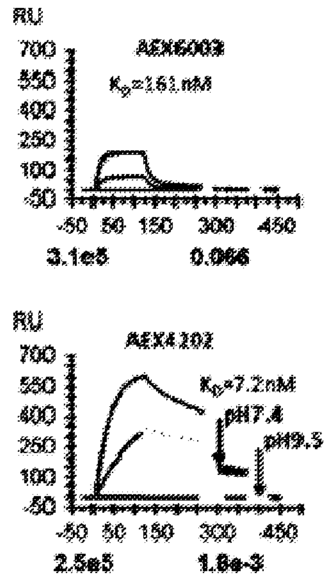

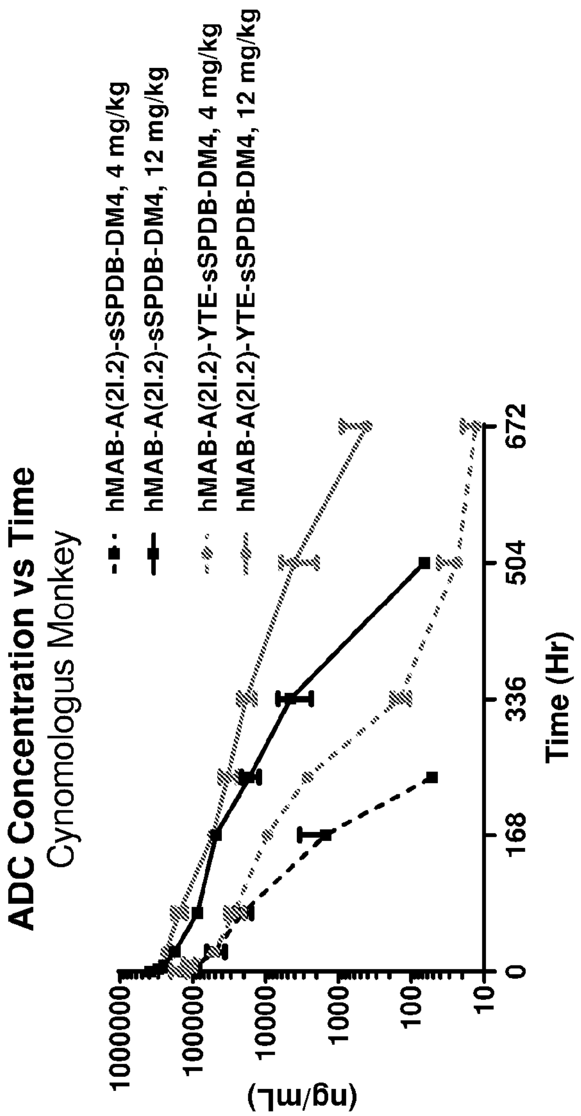
ADC Concentration vs Time
Cynomologus Monkey
hMAB-A(2I.2)-sSPDB-DM4, 4 mg/kg
hMAB-A(2I.2)-sSPDB-DM4, 12 mg/kg
hMAB-A(2I.2)-YTE-sSPDB-DM4, 4 mg/kg
hMAB-A(2I.2)-YTE-sSPDB-DM4, 12 mg/kg
Concentration (ng/mL)
1000000
100000
10000
1000
100
10
0
168
336
504
672
Time (Hr)

FIG. 20

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20130045244 A
- US 9150656 B
- US 7585634 B
- US 7829277 B
- US 8101361 B
- US 8445198 B
- US 20090023149 A
- US 20160138113 A
- US 20160068909 A
- US 20160024582 A
- US 20150368352 A
- US 20150337356 A
- US 20150337048 A
- US 20150010575 A
- US 20140342946 A
- US 20120077694 A
- US 20110151536 A
- US 20110129450 A
- US 20100291063 A
- US 20100233079 A
- US 20100112713 A
- US 20090203051 A
- US 20040092466 A
- US 20030091568 A
- US 20020068062 A
- WO 2016077505 A
- WO 2014205293 A
- WO 2014186364 A
- WO 2014124326 A
- WO 2014108480 A
- WO 2013119960 A
- WO 2013098797 A
- WO 2013049704 A
- WO 2011100362 A
- US 20090285840 A1
- DE 10337368 A1
- US 20120149595 A
- US 20090233300 A
- US 20060270618 A
- US 20090142301 A
- US 4816567 A
- US 5807715 A
- US 5866692 A
- US 6331415 B
- EP 519596 A
- US 6180377 B
- US 6054297 A
- US 5997867 A
- US 6277375 B
- US 7083784 B
- US 7217797 B
- US 8088376 B
- US 20020147311 A
- US 20070148164 A
- WO 9823289 A
- WO 2009058492 A
- WO 2010033279 A
- US 8361475 B
- US 7674619 B
- US 5208020 A
- US 7276497 B
- WO 2010043880 A
- WO 2011130616 A
- WO 2009016516 A
- WO 2013177481 A
- WO 2012112708 A
- WO 2010091150 A
- WO 2012128868 A
- US 19526916
- US 20080050310 A
- US 20050169933 A
- US 6913748 B
- US 6716821 B
- US 20090274713 A
- US 20100129314 A
- US 7276497 A
- US 9381256 B
- US 8765740 B
- US 8426402 B
- US 9353127 B
- US 20160082114 A
- US 5565332 A
- US 5580717 A
- US 5733743 A
- US 6265150 B
- WO 2012112687 A
- US 8624003 B
- WO 2017004025 A
- WO 12116203920 A
- WO 62480201 A
- WO 62438488 A

**Non-patent literature cited in the description**


- **AMENDOLA, R.S. et al.** ADAM9 Disintegrin Domain Activates Human Neutrophils Through An Autocrine Circuit Involving Integrins And CXCR2,. *J. Leukocyte Biol.,* 2015, vol. 97 (5), 951-962
- **EDWARS, D.R. et al.** The ADAM Metalloproteases,. *Molec. Aspects Med.,* 2008, vol. 29, 258-289
- **LI, J et al.** Overexpression of ADAM9 Promotes Colon Cancer Cells Invasion,. *J. Invest. Surg.,* 2016, vol. 26 (3), 127-133
- **DUFFY, M.J. et al.** The ADAMs Family Of Proteases: New Biomarkers And Therapeutic Targets For Cancer?,. *Clin. Proteomics,* 2011, vol. 8 (9), 1-13
- **DUFFY, M.J. et al.** The Role Of ADAMs In Disease Pathophysiology,. *Clin. Chim. Acta,* 2009, vol. 403, 31-36
- **ITO, N et al.** ADAMs, A Disintegrin And Metalloproteinases, Mediate Shedding Of Oxytocinase,. *Biochem. Biophys. Res. Commun.,* 2004, vol. 314, 1008-1013
- **ZIGRINO, P et al.** The Disintegrin-Like And Cysteine-Rich Domains Of ADAM-9 Mediate Interactions Between Melanoma Cells And Fibroblasts,. *J. Biol. Chem.,* 2011, vol. 286, 6801-6807
- **SUN, C et al.** ADAM15 Regulates Endothelial Permeability And Neutrophil Migration Via Src/ERK1/2 Signalling,. *Cardiovasc. Res.,* 2010, vol. 87, 348-355
- **NAMBA, K et al.** Involvement Of ADAM9 In Multinucleated Giant Cell Formation Of Blood Monocytes,. *Cell. Immunol.,* 2001, vol. 213, 104-113
- **OKSALA, N et al.** ADAM-9, ADAM-15, And ADAM-17 Are Upregulated In Macrophages In Advanced Human Atherosclerotic Plaques In Aorta And Carotid And Femoral Arteries - Tampere Vascular Study,. *Ann. Med.,* 2009, vol. 41, 279-290
- **KARADAG, A et al.** ADAM-9 (MDC-9/Meltringamma), A Member Of The A Disintegrin And Metalloproteinase Family, Regulates Myeloma-Cell-Induced Interleukin-6 Production In Osteoblasts By Direct Interaction With The Alpha(v)Beta5 Integrin,. *Blood,* 2006, vol. 107, 3271-3278
- **COMINETTI, M.R. et al.** Inhibition Of Platelets And Tumor Cell Adhesion By The Disintegrin Domain Of Human ADAM9 To Collagen I Under Dynamic Flow Conditions,. *Biochimie,* 2009, vol. 91, 1045-1052
- **YOSHIMASU, T et al.** Overexpression Of ADAM9 In Non-Small Cell Lung Cancer Correlates With Brain Metastasis,. *Cancer Res,* 2004, vol. 64, 4190-4196
- **PEDUTO, L et al.** Critical Function For ADAM9 In Mouse Prostate Cancer,. *Cancer Res.,* 2005, vol. 65, 9312-9319
- **ZIGRINO, P et al.** ADAM-9 Expression And Regulation In Human Skin Melanoma And Melanoma Cell Lines,. *Int. J. Cancer,* 2005, vol. 116, 853-859
- **FRITZSCHE, F.R. et al.** ADAM9 Is Highly Expressed In Renal Cell Cancer And Is Associated With Tumour Progression,. *BMC Cancer,* 2008, vol. 8 (179), 1-9
- **FRY, J.L. et al.** Secreted And Membrane-Bound Isoforms Of Protease ADAM9 Have Opposing Effects On Breast Cancer Cell Migration,. *Cancer Res,* 2010, vol. 70, 8187-8198
- **CHANG, L et al.** Combined Rnai Targeting Human Stat3 And ADAM9 As Gene Therapy For Non-Small Cell Lung Cancer,. *Oncology Letters,* 2016, vol. 11, 1242-1250
- **FAN, X et al.** ADAM9 Expression Is Associate with Glioma Tumor Grade and Histological Type, and Acts as a Prognostic Factor in Lower-Grade Gliomas,. *Int. J. Mol. Sci.,* 2016, vol. 17 (1276), 1-11
- **FRY, J.L. et al.** Secreted And Membrane-Bound Isoforms Of Protease ADAM9 Have Opposing Effects On Breast Cancer Cell Migration,. *Cancer Res.,* 2010, vol. 70, 8187-8198
- **MAZZOCCA, A.** A Secreted Form OfADAM9 Promotes Carcinoma Invasion Through Tumor-Stromal Interactions,. *Cancer Res.,* 2005, vol. 65, 4728-4738
- **PEDUTO, L.** ADAM9 As A Potential Target Molecule In Cancer,. *Curr. Pharm. Des.,* 2009, vol. 15, 2282-2287
- **DUFFY, M.J. et al.** Role Of ADAMs In Cancer Formation And Progression. *Clin. Cancer Res.,* 2009, vol. 15, 1140-1144
- **JOSSON, S et al.** Inhibition of ADAM9 Expression Induces Epithelial Phenotypic Alterations and Sensitizes Human Prostate Cancer Cells to Radiation and Chemotherapy,. *Prostate,* 2011, vol. 71 (3), 232-240
- **CHAN, C.E. et al.** The Use Of Antibodies In The Treatment Of Infectious Diseases,. *Singapore Med. J.,* 2009, vol. 50 (7), 663-666
- **KOHLER, G et al.** Continuous Cultures Of Fused Cells Secreting Antibody Of Predefined Specificity,. *Nature,* 1975, vol. 256, 495-497
- **JENNINGS, V.M.** Review of Selected Adjuvants Used in Antibody Production,. *ILAR J,* 1995, vol. 37 (3), 119-125
- **CHOTHIA, C. ; LESK, A. M.** Canonical structures for the hypervariable regions of immunoglobulins,. *J. Mol. Biol.,* 1987, vol. 196, 901-917
- **LOBUGLIO, A.F. et al.** Mouse/Human Chimeric Monoclonal Antibody In Man: Kinetics And Immune Response,. *Proc. Natl. Acad. Sci. (U.S.A.),* 1989, vol. 86, 4220-4224
- **SATO, K et al.** *Cancer Res,* 1993, vol. 53, 851-856

- **RIECHMANN, L et al.** Reshaping Human Antibodies for Therapy. *Nature,* 1988, vol. 332, 323-327
- **VERHOEYEN, M et al.** Reshaping Human Antibodies: Grafting An Antilysozyme Activity. *Science,* 1988, vol. 239, 1534-1536
- **KETTLEBOROUGH, C. A. et al.** Humanization Of A Mouse Monoclonal Antibody By CDR-Grafting: The Importance Of Framework Residues On Loop Conformation. *Protein Engineering,* 1991, vol. 4, 773-3783
- **MAEDA, H et al.** Construction Of Reshaped Human Antibodies With HIV-Neutralizing Activity. *Human Antibodies Hybridoma,* 1991, vol. 2, 124-134
- **GORMAN, S. D. et al.** Reshaping A Therapeutic CD4 Antibody. *Proc. Natl. Acad. Sci. (U.S.A.),* 1991, vol. 88, 4181-4185
- **TEMPEST, P.R. et al.** Reshaping A Human Monoclonal Antibody To Inhibit Human Respiratory Syncytial Virus Infection in vivo. *Bio/Technology,* 1991, vol. 9, 266-271
- **CO, M. S. et al.** HumanizedAntibodies For Antiviral Therapy. *Proc. Natl. Acad. Sci. (U.S.A.),* 1991, vol. 88, 2869-2873
- **CARTER, P et al.** Humanization Of An Anti-p185her2 Antibody For Human Cancer Therapy,. *Proc. Natl. Acad. Sci. (U.S.A.),* 1992, vol. 89, 4285-4289
- **CO, M.S. et al.** Chimeric And Humanized Antibodies With Specificity For The CD33 Antigen,. *J. Immunol.,* 1992, vol. 148, 1149-1154
- **WINTER et al.** Man-made Antibodies. *Nature,* 1991, vol. 349, 293-299
- **LOBUGLIO et al.** Mouse/Human Chimeric Monoclonal Antibody In Man: Kinetics And Immune Response. *Proc. Natl. Acad. Sci. (U.S.A.),* 1989, vol. 86, 4220-4224
- **SHAW et al.** Characterization Of A Mouse/Human Chimeric Monoclonal Antibody (17-1A) To A Colon Cancer Tumor-Associated Antigen. *J. Immunol.,* 1987, vol. 138, 4534-4538
- **BROWN et al.** Tumor-Specific Genetically Engineered Murine/Human Chimeric Monoclonal Antibody,. *Cancer Res,* 1987, vol. 47, 3577-3583
- **JONES et al.** Replacing The Complementarity-Determining Regions In A Human Antibody With Those From A Mouse. *Nature,* 1986, vol. 321, 522-525
- **DAUGHERTY et al.** Polymerase Chain Reaction Facilitates The Cloning, CDR-Grafting, And Rapid Expression Of A Murine Monoclonal Antibody Directed Against The CD18 Component Of Leukocyte Integrins. *Nucl. Acids Res.,* 1991, vol. 19, 2471-2476
- **KABAT et al.** SEQUENCES OF PROTEINS OF IMMUNOLOGICAL INTEREST. Public Health Service, 1991
- **LEFRANC et al.** The Human IgG Subclasses: Molecular Analysis of Structure, Function And Regulation. Pergamon, 1990, 43-78
- **LEFRANC, G et al.** *Hum. Genet.,* 1979, vol. 50, 199-211
- **STAVENHAGEN, J.B. et al.** Fc Optimization Of Therapeutic Antibodies Enhances Their Ability To Kill Tumor Cells In Vitro And Controls Tumor Expansion In Vivo Via Low-Affinity Activating Fcgamma Receptors. *Cancer Res,* 2007, vol. 57 (18), 8882-8890
- Dictionary of Chemical Terms. McGraw-Hill Book Company, 1984
- **ELIEL, E. ; WILEN, S.** Stereochemistry of Organic Compounds. John Wiley & Sons, Inc, 1994
- **P. WUTS ; T. GREENE.** Protective Groups in Organic Synthesis. J. Wiley & Sons, 2007
- **P. G.M. WUTS ; T. W. GREENE.** Protective Groups in Organic Synthesis. John Wiley & Sons, 2007
- **ISALM ; DENT.** Bioconjugation. Groves Dictionaries Inc, 1999, 218-363
- **MERRIFIELD, B.** Solid Phase Synthesis,. *Science,* 1986, vol. 232 (4748), 341-347
- **HOUGHTEN, R.A.** General Method For The Rapid Solid-Phase Synthesis Of Large Numbers Of Peptides: Specificity Of Antigen-Antibody Interaction At The Level Of Individual Amino Acids,. *Proc. Natl. Acad. Sci. (U.S.A.),* 1985, vol. 82 (15), 5131-5135
- **GANESAN, A.** Solid-Phase Synthesis In The Twenty-First Century,. *Mini Rev. Med. Chem.,* 2006, vol. 6 (1), 3-10
- **PEETERS et al.** Production Of Antibodies And Antibody Fragments In Plants,. *Vaccine,* 2001, vol. 19, 2756
- **LONBERG, N et al.** Human Antibodies From Transgenic Mice,. *Int. Rev. Immunol,* 1995, vol. 13, 65-93
- **POLLOCK et al.** Transgenic Milk As A Method For The Production Of Recombinant Antibodies,. *J. Immunol Methods,* 1999, vol. 231, 147-157
- **WINTER, G et al.** Making Antibodies By Phage Display Technology,. *Annu. Rev. Immunol.,* 1994, vol. 12, 433-455
- **WU et al.** Receptor-Mediated In Vitro Gene Transformation By A Soluble DNA Carrier System,. *J. Biol. Chem.,* 1987, vol. 262, 4429-4432
- **LANGER.** New Methods Of Drug Delivery,. *Science,* 1990, vol. 249, 1527-1533
- **TREAT et al.** LIPOSOMES IN THE THERAPY OF INFECTIOUS DISEASE AND CANCER. Liss, 1989, 353-365
- **LAI et al.** *Pharm Res,* November 2015, vol. 32 (11), 3593-603

SEQUENCE LISTING

<110> IMMUNOGEN, INC. MACROGENICS, INC.

<120> IMMUNOCONJUGATES TARGETING ADAM9 AND METHODS OF USE THEREOF

<130> 121162-03920

<140>
<141>

<150> 62/480,201 <151> 2017-03-31

<150> 62/438,488 <151> 2016-12-23

<160> 154

<170> PatentIn version 3.5

<210> 1
<211> 217
<212> PRT
<213> Homo sapiens

<220>
<221> VARIANT
<222> (217)..(217)
<223> /replace=" "

<220>
<221> MISC_FEATURE
<222> (1)..(217)
<223> /note="Variant residues given in the sequence have no preference with respect to those in the annotations for variant positions"

<400> 1

```
Ala Pro Glu Leu Leu Gly Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Val Val Asp Val Ser His Glu Asp Pro Glu Val Lys Phe Asn Trp Tyr
        35                  40                  45

Val Asp Gly Val Glu Val His Asn Ala Lys Thr Lys Pro Arg Glu Glu
    50                  55                  60

Gln Tyr Asn Ser Thr Tyr Arg Val Val Ser Val Leu Thr Val Leu His
65                  70                  75                  80

Gln Asp Trp Leu Asn Gly Lys Glu Tyr Lys Cys Lys Val Ser Asn Lys
                85                  90                  95

Ala Leu Pro Ala Pro Ile Glu Lys Thr Ile Ser Lys Ala Lys Gly Gln
            100                 105                 110

Pro Arg Glu Pro Gln Val Tyr Thr Leu Pro Pro Ser Arg Glu Glu Met
        115                 120                 125

Thr Lys Asn Gln Val Ser Leu Thr Cys Leu Val Lys Gly Phe Tyr Pro
    130                 135                 140

Ser Asp Ile Ala Val Glu Trp Glu Ser Asn Gly Gln Pro Glu Asn Asn
145                 150                 155                 160

Tyr Lys Thr Thr Pro Pro Val Leu Asp Ser Asp Gly Ser Phe Phe Leu
                165                 170                 175

Tyr Ser Lys Leu Thr Val Asp Lys Ser Arg Trp Gln Gln Gly Asn Val
            180                 185                 190

Phe Ser Cys Ser Val Met His Glu Ala Leu His Asn His Tyr Thr Gln
        195                 200                 205

Lys Ser Leu Ser Leu Ser Pro Gly Lys
    210                 215
```

<210> 2
<211> 216
<212> PRT
<213> Homo sapiens

<220>

<221> VARIANT
<222> (216)..(216)
<223> /replace=" "

<220>
<221> MISC_FEATURE
<222> (1)..(216)
<223> /note="Variant residues given in the sequence have no preference with respect to those in the annotations for variant positions"

<400> 2

```
Ala Pro Pro Val Ala Gly Pro Ser Val Phe Leu Phe Pro Pro Lys Pro
1               5                   10                  15

Lys Asp Thr Leu Met Ile Ser Arg Thr Pro Glu Val Thr Cys Val Val
            20                  25                  30

Val Asp Val Ser His Glu Asp Pro Glu Val Gln Phe Asn Trp Tyr Val
        35                  40                  45
```

```
Asp Gly Val Glu Val His Asn Ala Lys Thr Lys Pro Arg Glu Glu Gln
    50                  55                  60

Phe Asn Ser Thr Phe Arg Val Val Ser Val Leu Thr Val Val His Gln
65                  70                  75                  80

Asp Trp Leu Asn Gly Lys Glu Tyr Lys Cys Lys Val Ser Asn Lys Gly
                85                  90                  95

Leu Pro Ala Pro Ile Glu Lys Thr Ile Ser Lys Thr Lys Gly Gln Pro
            100                 105                 110

Arg Glu Pro Gln Val Tyr Thr Leu Pro Pro Ser Arg Glu Glu Met Thr
        115                 120                 125

Lys Asn Gln Val Ser Leu Thr Cys Leu Val Lys Gly Phe Tyr Pro Ser
    130                 135                 140

Asp Ile Ser Val Glu Trp Glu Ser Asn Gly Gln Pro Glu Asn Asn Tyr
145                 150                 155                 160

Lys Thr Thr Pro Pro Met Leu Asp Ser Asp Gly Ser Phe Phe Leu Tyr
                165                 170                 175

Ser Lys Leu Thr Val Asp Lys Ser Arg Trp Gln Gln Gly Asn Val Phe
            180                 185                 190

Ser Cys Ser Val Met His Glu Ala Leu His Asn His Tyr Thr Gln Lys
        195                 200                 205

Ser Leu Ser Leu Ser Pro Gly Lys
    210                 215
```

<210> 3
<211> 217
<212> PRT
<213> Homo sapiens

<220>
<221> VARIANT
<222> (217)..(217)
<223> /replace=" "

<220>
<221> MISC_FEATURE
<222> (1)..(217)
<223> /note="Variant residues given in the sequence have no preference with respect to those in the annotations for variant positions"

<400> 3

```
Ala Pro Glu Leu Leu Gly Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Val Val Asp Val Ser His Glu Asp Pro Glu Val Gln Phe Lys Trp Tyr
        35                  40                  45

Val Asp Gly Val Glu Val His Asn Ala Lys Thr Lys Pro Arg Glu Glu
    50                  55                  60

Gln Tyr Asn Ser Thr Phe Arg Val Val Ser Val Leu Thr Val Leu His
65                  70                  75                  80

Gln Asp Trp Leu Asn Gly Lys Glu Tyr Lys Cys Lys Val Ser Asn Lys
                85                  90                  95

Ala Leu Pro Ala Pro Ile Glu Lys Thr Ile Ser Lys Thr Lys Gly Gln
            100                 105                 110

Pro Arg Glu Pro Gln Val Tyr Thr Leu Pro Pro Ser Arg Glu Glu Met
        115                 120                 125

Thr Lys Asn Gln Val Ser Leu Thr Cys Leu Val Lys Gly Phe Tyr Pro
    130                 135                 140

Ser Asp Ile Ala Val Glu Trp Glu Ser Ser Gly Gln Pro Glu Asn Asn
145                 150                 155                 160

Tyr Asn Thr Thr Pro Pro Met Leu Asp Ser Asp Gly Ser Phe Phe Leu
                165                 170                 175

Tyr Ser Lys Leu Thr Val Asp Lys Ser Arg Trp Gln Gln Gly Asn Ile
            180                 185                 190

Phe Ser Cys Ser Val Met His Glu Ala Leu His Asn Arg Phe Thr Gln
        195                 200                 205

Lys Ser Leu Ser Leu Ser Pro Gly Lys
    210                 215
```

<210> 4
<211> 217
<212> **PRT**
<213> Homo sapiens

<220>

<221> VARIANT
<222> (217)..(217)
<223> /replace=" "

<220>
<221> MISC_FEATURE
<222> (1)..(217)
<223> /note="Variant residues given in the sequence have no preference with respect to those in the annotations for variant positions"

<400> 4

```
Ala Pro Glu Phe Leu Gly Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Val Val Asp Val Ser Gln Glu Asp Pro Glu Val Gln Phe Asn Trp Tyr
        35                  40                  45

Val Asp Gly Val Glu Val His Asn Ala Lys Thr Lys Pro Arg Glu Glu
    50                  55                  60

Gln Phe Asn Ser Thr Tyr Arg Val Val Ser Val Leu Thr Val Leu His
65                  70                  75                  80

Gln Asp Trp Leu Asn Gly Lys Glu Tyr Lys Cys Lys Val Ser Asn Lys
                85                  90                  95

Gly Leu Pro Ser Ser Ile Glu Lys Thr Ile Ser Lys Ala Lys Gly Gln
            100                 105                 110

Pro Arg Glu Pro Gln Val Tyr Thr Leu Pro Pro Ser Gln Glu Glu Met
        115                 120                 125

Thr Lys Asn Gln Val Ser Leu Thr Cys Leu Val Lys Gly Phe Tyr Pro
    130                 135                 140

Ser Asp Ile Ala Val Glu Trp Glu Ser Asn Gly Gln Pro Glu Asn Asn
145                 150                 155                 160

Tyr Lys Thr Thr Pro Pro Val Leu Asp Ser Asp Gly Ser Phe Phe Leu
                165                 170                 175

Tyr Ser Arg Leu Thr Val Asp Lys Ser Arg Trp Gln Glu Gly Asn Val
            180                 185                 190

Phe Ser Cys Ser Val Met His Glu Ala Leu His Asn His Tyr Thr Gln
```

195 200 205

Lys Ser Leu Ser Leu Ser Leu Gly Lys
210 215

<210> 5
<211> 819
<212> PRT
<213> Homo sapiens

<400> 5

```
Met Gly Ser Gly Ala Arg Phe Pro Ser Gly Thr Leu Arg Val Arg Trp
1               5                   10                  15

Leu Leu Leu Leu Gly Leu Val Gly Pro Val Leu Gly Ala Ala Arg Pro
            20                  25                  30

Gly Phe Gln Gln Thr Ser His Leu Ser Ser Tyr Glu Ile Ile Thr Pro
        35                  40                  45

Trp Arg Leu Thr Arg Glu Arg Arg Glu Ala Pro Arg Pro Tyr Ser Lys
    50                  55                  60

Gln Val Ser Tyr Val Ile Gln Ala Glu Gly Lys Glu His Ile Ile His
65                  70                  75                  80

Leu Glu Arg Asn Lys Asp Leu Leu Pro Glu Asp Phe Val Val Tyr Thr
                85                  90                  95

Tyr Asn Lys Glu Gly Thr Leu Ile Thr Asp His Pro Asn Ile Gln Asn
            100                 105                 110

His Cys His Tyr Arg Gly Tyr Val Glu Gly Val His Asn Ser Ser Ile
        115                 120                 125

Ala Leu Ser Asp Cys Phe Gly Leu Arg Gly Leu Leu His Leu Glu Asn
    130                 135                 140

Ala Ser Tyr Gly Ile Glu Pro Leu Gln Asn Ser Ser His Phe Glu His
145                 150                 155                 160

Ile Ile Tyr Arg Met Asp Asp Val Tyr Lys Glu Pro Leu Lys Cys Gly
                165                 170                 175

Val Ser Asn Lys Asp Ile Glu Lys Glu Thr Ala Lys Asp Glu Glu Glu
            180                 185                 190

Glu Pro Pro Ser Met Thr Gln Leu Leu Arg Arg Arg Arg Ala Val Leu
        195                 200                 205
```

Pro Gln Thr Arg Tyr Val Glu Leu Phe Ile Val Val Asp Lys Glu Arg
210 215 220

Tyr Asp Met Met Gly Arg Asn Gln Thr Ala Val Arg Glu Glu Met Ile
225 230 235 240

Leu Leu Ala Asn Tyr Leu Asp Ser Met Tyr Ile Met Leu Asn Ile Arg
245 250 255

Ile Val Leu Val Gly Leu Glu Ile Trp Thr Asn Gly Asn Leu Ile Asn
260 265 270

Ile Val Gly Gly Ala Gly Asp Val Leu Gly Asn Phe Val Gln Trp Arg
275 280 285

Glu Lys Phe Leu Ile Thr Arg Arg Arg His Asp Ser Ala Gln Leu Val
290 295 300

Leu Lys Lys Gly Phe Gly Gly Thr Ala Gly Met Ala Phe Val Gly Thr
305 310 315 320

Val Cys Ser Arg Ser His Ala Gly Gly Ile Asn Val Phe Gly Gln Ile
325 330 335

Thr Val Glu Thr Phe Ala Ser Ile Val Ala His Glu Leu Gly His Asn
340 345 350

Leu Gly Met Asn His Asp Asp Gly Arg Asp Cys Ser Cys Gly Ala Lys
355 360 365

Ser Cys Ile Met Asn Ser Gly Ala Ser Gly Ser Arg Asn Phe Ser Ser
370 375 380

Cys Ser Ala Glu Asp Phe Glu Lys Leu Thr Leu Asn Lys Gly Gly Asn
385 390 395 400

Cys Leu Leu Asn Ile Pro Lys Pro Asp Glu Ala Tyr Ser Ala Pro Ser
405 410 415

Cys Gly Asn Lys Leu Val Asp Ala Gly Glu Glu Cys Asp Cys Gly Thr
420 425 430

Pro Lys Glu Cys Glu Leu Asp Pro Cys Cys Glu Gly Ser Thr Cys Lys
435 440 445

Leu Lys Ser Phe Ala Glu Cys Ala Tyr Gly Asp Cys Cys Lys Asp Cys

```
    450                 455                 460

Arg Phe Leu Pro Gly Gly Thr Leu Cys Arg Gly Lys Thr Ser Glu Cys
465                 470                 475                 480

Asp Val Pro Glu Tyr Cys Asn Gly Ser Ser Gln Phe Cys Gln Pro Asp
                485                 490                 495

Val Phe Ile Gln Asn Gly Tyr Pro Cys Gln Asn Asn Lys Ala Tyr Cys
            500                 505                 510

Tyr Asn Gly Met Cys Gln Tyr Tyr Asp Ala Gln Cys Gln Val Ile Phe
        515                 520                 525

Gly Ser Lys Ala Lys Ala Ala Pro Lys Asp Cys Phe Ile Glu Val Asn
    530                 535                 540

Ser Lys Gly Asp Arg Phe Gly Asn Cys Gly Phe Ser Gly Asn Glu Tyr
545                 550                 555                 560

Lys Lys Cys Ala Thr Gly Asn Ala Leu Cys Gly Lys Leu Gln Cys Glu
                565                 570                 575

Asn Val Gln Glu Ile Pro Val Phe Gly Ile Val Pro Ala Ile Ile Gln
            580                 585                 590

Thr Pro Ser Arg Gly Thr Lys Cys Trp Gly Val Asp Phe Gln Leu Gly
        595                 600                 605

Ser Asp Val Pro Asp Pro Gly Met Val Asn Glu Gly Thr Lys Cys Gly
    610                 615                 620

Ala Gly Lys Ile Cys Arg Asn Phe Gln Cys Val Asp Ala Ser Val Leu
625                 630                 635                 640

Asn Tyr Asp Cys Asp Val Gln Lys Lys Cys His Gly His Gly Val Cys
                645                 650                 655

Asn Ser Asn Lys Asn Cys His Cys Glu Asn Gly Trp Ala Pro Pro Asn
            660                 665                 670

Cys Glu Thr Lys Gly Tyr Gly Gly Ser Val Asp Ser Gly Pro Thr Tyr
        675                 680                 685

Asn Glu Met Asn Thr Ala Leu Arg Asp Gly Leu Leu Val Phe Phe Phe
    690                 695                 700
```

Leu Ile Val Pro Leu Ile Val Cys Ala Ile Phe Ile Phe Ile Lys Arg
705 710 715 720

Asp Gln Leu Trp Arg Ser Tyr Phe Arg Lys Lys Arg Ser Gln Thr Tyr
725 730 735

Glu Ser Asp Gly Lys Asn Gln Ala Asn Pro Ser Arg Gln Pro Gly Ser
740 745 750

Val Pro Arg His Val Ser Pro Val Thr Pro Pro Arg Glu Val Pro Ile
755 760 765

Tyr Ala Asn Arg Phe Ala Val Pro Thr Tyr Ala Ala Lys Gln Pro Gln
770 775 780

Gln Phe Pro Ser Arg Pro Pro Pro Pro Gln Pro Lys Val Ser Ser Gln
785 790 795 800

Gly Asn Leu Ile Pro Ala Arg Pro Ala Pro Ala Pro Pro Leu Tyr Ser
805 810 815

Ser Leu Thr

<210> 6
<211> 819
<212> PRT
<213> Macaca fascicularis

<400> 6

Met Gly Ser Gly Val Gly Ser Pro Ser Gly Thr Leu Arg Val Arg Trp
1               5                   10                  15

Leu Leu Leu Leu Cys Leu Val Gly Pro Val Leu Gly Ala Ala Arg Pro
            20                  25                  30

Gly Phe Gln Gln Thr Ser His Leu Ser Ser Tyr Glu Ile Ile Thr Pro
        35                  40                  45

Trp Arg Leu Thr Arg Glu Arg Arg Glu Ala Pro Arg Pro Tyr Ser Lys
    50                  55                  60

Gln Val Ser Tyr Leu Ile Gln Ala Glu Gly Lys Glu His Ile Ile His
65                  70                  75                  80

Leu Glu Arg Asn Lys Asp Leu Leu Pro Glu Asp Phe Val Val Tyr Thr
                85                  90                  95

Tyr Asn Lys Glu Gly Thr Val Ile Thr Asp His Pro Asn Ile Gln Asn

            100                 105                 110

His Cys His Phe Arg Gly Tyr Val Glu Gly Val Tyr Asn Ser Ser Val
        115                 120                 125

Ala Leu Ser Asn Cys Phe Gly Leu Arg Gly Leu Leu His Leu Glu Asn
    130                 135                 140

Ala Ser Tyr Gly Ile Glu Pro Leu Gln Asn Ser Ser His Phe Glu His
145                 150                 155                 160

Ile Ile Tyr Arg Met Asp Asp Val His Lys Glu Pro Leu Lys Cys Gly
                165                 170                 175

Val Ser Asn Lys Asp Ile Glu Lys Glu Thr Thr Lys Asp Glu Glu Glu
            180                 185                 190

Glu Pro Pro Ser Met Thr Gln Leu Leu Arg Arg Arg Arg Ala Val Leu
        195                 200                 205

Pro Gln Thr Arg Tyr Val Glu Leu Phe Ile Val Val Asp Lys Glu Arg
    210                 215                 220

Tyr Asp Met Met Gly Arg Asn Gln Thr Ala Val Arg Glu Glu Met Ile
225                 230                 235                 240

Leu Leu Ala Asn Tyr Leu Asp Ser Met Tyr Ile Met Leu Asn Ile Arg
                245                 250                 255

Ile Val Leu Val Gly Leu Glu Ile Trp Thr Asn Gly Asn Leu Ile Asn
            260                 265                 270

Ile Ala Gly Gly Ala Gly Asp Val Leu Gly Asn Phe Val Gln Trp Arg
        275                 280                 285

Glu Lys Phe Leu Ile Thr Arg Arg Arg His Asp Ser Ala Gln Leu Val
    290                 295                 300

Leu Lys Lys Gly Phe Gly Gly Thr Ala Gly Met Ala Phe Val Gly Thr
305                 310                 315                 320

Val Cys Ser Arg Ser His Ala Gly Gly Ile Asn Val Phe Gly His Ile
                325                 330                 335

Thr Val Glu Thr Phe Ala Ser Ile Val Ala His Glu Leu Gly His Asn
            340                 345                 350

Leu Gly Met Asn His Asp Asp Gly Arg Asp Cys Ser Cys Gly Ala Lys
355 360 365

Ser Cys Ile Met Asn Ser Gly Ala Ser Gly Ser Arg Asn Phe Ser Ser
370 375 380

Cys Ser Ala Glu Asp Phe Glu Lys Leu Thr Leu Asn Lys Gly Gly Asn
385 390 395 400

Cys Leu Leu Asn Ile Pro Lys Pro Asp Glu Ala Tyr Ser Ala Pro Ser
405 410 415

Cys Gly Asn Lys Leu Val Asp Ala Gly Glu Glu Cys Asp Cys Gly Thr
420 425 430

Pro Lys Glu Cys Glu Leu Asp Pro Cys Cys Glu Gly Ser Thr Cys Lys
435 440 445

Leu Lys Ser Phe Ala Glu Cys Ala Tyr Gly Asp Cys Cys Lys Asp Cys
450 455 460

Arg Phe Leu Pro Gly Gly Thr Leu Cys Arg Gly Lys Thr Ser Glu Cys
465 470 475 480

Asp Val Pro Glu Tyr Cys Asn Gly Ser Ser Gln Phe Cys Gln Pro Asp
485 490 495

Val Phe Ile Gln Asn Gly Tyr Pro Cys Gln Asn Asn Lys Ala Tyr Cys
500 505 510

Tyr Asn Gly Met Cys Gln Tyr Tyr Asp Ala Gln Cys Gln Val Ile Phe
515 520 525

Gly Ser Lys Ala Lys Ala Ala Pro Lys Asp Cys Phe Ile Glu Val Asn
530 535 540

Ser Lys Gly Asp Arg Phe Gly Asn Cys Gly Phe Ser Gly Asn Glu Tyr
545 550 555 560

Lys Lys Cys Ala Thr Gly Asn Ala Leu Cys Gly Lys Leu Gln Cys Glu
565 570 575

Asn Val Gln Glu Ile Pro Val Phe Gly Ile Val Pro Ala Ile Ile Gln
580 585 590

Thr Pro Ser Arg Gly Thr Lys Cys Trp Gly Val Asp Phe Gln Leu Gly
595 600 605

Ser Asp Val Pro Asp Pro Gly Met Val Asn Glu Gly Thr Lys Cys Gly
610 615 620

Ala Asp Lys Ile Cys Arg Asn Phe Gln Cys Val Asp Ala Ser Val Leu
625 630 635 640

Asn Tyr Asp Cys Asp Ile Gln Lys Lys Cys His Gly His Gly Val Cys
645 650 655

Asn Ser Asn Lys Asn Cys His Cys Glu Asn Gly Trp Ala Pro Pro Asn
660 665 670

Cys Glu Thr Lys Gly Tyr Gly Gly Ser Val Asp Ser Gly Pro Thr Tyr
675 680 685

Asn Glu Met Asn Thr Ala Leu Arg Asp Gly Leu Leu Val Phe Phe Phe
690 695 700

Leu Ile Val Pro Leu Ile Val Cys Ala Ile Phe Ile Phe Ile Lys Arg
705 710 715 720

Asp Gln Leu Trp Arg Arg Tyr Phe Arg Lys Lys Arg Ser Gln Thr Tyr
725 730 735

Glu Ser Asp Gly Lys Asn Gln Ala Asn Pro Ser Arg Gln Pro Val Ser
740 745 750

Val Pro Arg His Val Ser Pro Val Thr Pro Pro Arg Glu Val Pro Ile
755 760 765

Tyr Ala Asn Arg Phe Pro Val Pro Thr Tyr Ala Ala Lys Gln Pro Gln
770 775 780

Gln Phe Pro Ser Arg Pro Pro Pro Pro Gln Pro Lys Val Ser Ser Gln
785 790 795 800

Gly Asn Leu Ile Pro Ala Arg Pro Ala Pro Ala Pro Pro Leu Tyr Ser
805 810 815

Ser Leu Thr

<210> 7
<211> 123
<212> PRT
<213> Artificial Sequence

<220>
<221> source

<223> /note="Description of Artificial Sequence: Synthetic polypeptide"

<400> 7

```
Gln Val Gln Leu Gln Gln Pro Gly Ala Glu Leu Val Lys Pro Gly Ala
1               5                   10                  15

Ser Val Lys Leu Ser Cys Lys Ala Ser Gly Tyr Thr Phe Thr Ser Tyr
            20                  25                  30

Trp Met His Trp Val Lys Gln Arg Pro Gly Gln Gly Leu Glu Trp Ile
        35                  40                  45

Gly Glu Ile Ile Pro Ile Asn Gly His Thr Asn Tyr Asn Glu Lys Phe
    50                  55                  60

Lys Ser Lys Ala Thr Leu Thr Leu Asp Lys Ser Ser Ser Thr Ala Tyr
65                  70                  75                  80

Met Gln Leu Ser Ser Leu Ala Ser Glu Asp Ser Ala Val Tyr Tyr Cys
                85                  90                  95

Ala Arg Gly Gly Tyr Tyr Tyr Tyr Gly Ser Arg Asp Tyr Phe Asp Tyr
            100                 105                 110

Trp Gly Gln Gly Thr Thr Leu Thr Val Ser Ser
        115                 120
```

<210> 8
<211> 5
<212> PRT
<213> Artificial Sequence

<220>
<221> source
<223> /note="Description of Artificial Sequence: Synthetic peptide"

<400> 8
Ser Tyr Trp Met His
1 5

<210> 9
<211> 17
<212> PRT
<213> Artificial Sequence

<220>
<221> source
<223> /note="Description of Artificial Sequence: Synthetic peptide"

<400> 9

Glu Ile Ile Pro Ile Asn Gly His Thr Asn Tyr Asn Glu Lys Phe Lys
1               5                   10                  15

Ser

<210> 10
<211> 14
<212> PRT
<213> Artificial Sequence

<220>
<221> source
<223> /note="Description of Artificial Sequence: Synthetic peptide"

<400> 10
Gly Gly Tyr Tyr Tyr Tyr Gly Ser Arg Asp Tyr Phe Asp Tyr
1 5 10

<210> 11
<211> 111
<212> PRT
<213> Artificial Sequence

<220>
<221> source
<223> /note="Description of Artificial Sequence: Synthetic polypeptide"

<400> 11

Asp Ile Val Leu Thr Gln Ser Pro Ala Ser Leu Ala Val Ser Leu Gly
1               5                   10                  15

Gln Arg Ala Thr Ile Ser Cys Lys Ala Ser Gln Ser Val Asp Tyr Asp
            20                  25                  30

Gly Asp Ser Tyr Met Asn Trp Tyr Gln Gln Ile Pro Gly Gln Pro Pro
        35                  40                  45

Lys Leu Leu Ile Tyr Ala Ala Ser Asp Leu Glu Ser Gly Ile Pro Ala
    50                  55                  60

Arg Phe Ser Gly Ser Gly Ser Gly Thr Asp Phe Thr Leu Asn Ile His
65                  70                  75                  80

Pro Val Glu Glu Glu Asp Ala Ala Thr Tyr Tyr Cys Gln Gln Ser His
                85                  90                  95

Glu Asp Pro Phe Thr Phe Gly Gly Gly Thr Lys Leu Glu Ile Lys
            100                 105                 110

<210> 12
<211> 15

<212> PRT
<213> Artificial Sequence

<220>
<221> source
<223> /note="Description of Artificial Sequence: Synthetic peptide"

<400> 12
Lys Ala Ser Gln Ser Val Asp Tyr Asp Gly Asp Ser Tyr Met Asn
1 5 10 15

<210> 13
<211> 7
<212> PRT
<213> Artificial Sequence

<220>
<221> source
<223> /note="Description of Artificial Sequence: Synthetic peptide"

<400> 13
Ala Ala Ser Asp Leu Glu Ser
1 5

<210> 14
<211> 9
<212> PRT
<213> Artificial Sequence

<220>
<221> source
<223> /note="Description of Artificial Sequence: Synthetic peptide"

<400> 14
Gln Gln Ser His Glu Asp Pro Phe Thr
1 5

<210> 15
<211> 123
<212> PRT
<213> Artificial Sequence

<220>
<221> source
<223> /note="Description of Artificial Sequence: Synthetic polypeptide"

<220>
<221> VARIANT
<222> (34)..(34)
<223> /replace="Ile"

<220>
<221> VARIANT
<222> (55)..(55)
<223> /replace="Phe"

<220>
<221> VARIANT

<222> (63)..(63)
<223> /replace="Arg"

<220>
<221> VARIANT
<222> (65)..(65)
<223> /replace="Gln"

<220>
<221> VARIANT
<222> (66)..(66)
<223> /replace="Gly"

<220>
<221> VARIANT
<222> (105)..(105)
<223> /replace="Phe" or "Tyr" or "Trp" or "Ile" or "Leu" "Val" or "Thr" or "Gly" or "Asp"

<220>
<221> VARIANT
<222> (106)..(106)
<223> /replace="Arg" or "Asn" or "His" or "Gly" or "Ser"

<220>
<221> VARIANT
<222> (107)..(107)
<223> /replace="Met" or "Ser" or "Lys" or "Asn"

<220>
<221> VARIANT
<222> (108)..(108)
<223> /replace="Ala"

<220>
<221> VARIANT
<222> (109)..(109)
<223> /replace="Phe" or "Thr" or "Val"

<220>
<221> VARIANT
<222> (110)..(110)
<223> /replace="Leu" or "Lys"

<220>
<221> MISC_FEATURE
<222> (1)..(123)
<223> /note="Variant residues given in the sequence have no preference with respect to those in the annotations for variant positions"

<220>
<221> source
<223> /note="See specification as filed for detailed description of substitutions and preferred embodiments"

<400> 15

```
Glu Val Gln Leu Val Glu Ser Gly Gly Gly Leu Val Lys Pro Gly Gly
1               5                   10                  15
```

Ser Leu Arg Leu Ser Cys Ala Ala Ser Gly Phe Thr Phe Ser Ser Tyr
20 25 30

Trp Met His Trp Val Arg Gln Ala Pro Gly Lys Gly Leu Glu Trp Val
35 40 45

Gly Glu Ile Ile Pro Ile Asn Gly His Thr Asn Tyr Asn Glu Lys Phe
50 55 60

Lys Ser Arg Phe Thr Ile Ser Leu Asp Asn Ser Lys Asn Thr Leu Tyr
65 70 75 80

Leu Gln Met Gly Ser Leu Arg Ala Glu Asp Thr Ala Val Tyr Tyr Cys
85 90 95

Ala Arg Gly Gly Tyr Tyr Tyr Tyr Pro Lys Phe Gly Trp Met Asp Tyr
100 105 110

Trp Gly Gln Gly Thr Thr Val Thr Val Ser Ser
115 120

<210> 16
<211> 123
<212> PRT
<213> Artificial Sequence

<220>
<221> source
<223> /note="Description of Artificial Sequence: Synthetic polypeptide"

<400> 16

Glu Val Gln Leu Val Glu Ser Gly Gly Gly Leu Val Lys Pro Gly Gly
1 5 10 15

Ser Leu Arg Leu Ser Cys Ala Ala Ser Gly Phe Thr Phe Ser Ser Tyr
20 25 30

Trp Met His Trp Val Arg Gln Ala Pro Gly Lys Gly Leu Glu Trp Val
35 40 45

Gly Glu Ile Ile Pro Ile Asn Gly His Thr Asn Tyr Asn Glu Lys Phe
50 55 60

Lys Ser Arg Phe Thr Ile Ser Leu Asp Asn Ser Lys Asn Thr Leu Tyr
65 70 75 80

Leu Gln Met Gly Ser Leu Arg Ala Glu Asp Thr Ala Val Tyr Tyr Cys
85 90 95

Ala Arg Gly Gly Tyr Tyr Tyr Tyr Gly Ser Arg Asp Tyr Phe Asp Tyr
100 105 110

Trp Gly Gln Gly Thr Thr Val Thr Val Ser Ser
115 120

<210> 17
<211> 123
<212> PRT
<213> Artificial Sequence

<220>
<221> source
<223> /note="Description of Artificial Sequence: Synthetic polypeptide"

<400> 17

Glu Val Gln Leu Val Glu Ser Gly Gly Gly Leu Val Lys Pro Gly Gly
1 5 10 15

Ser Leu Arg Leu Ser Cys Ala Ala Ser Gly Phe Thr Phe Ser Ser Tyr
20 25 30

Trp Met His Trp Val Arg Gln Ala Pro Gly Lys Gly Leu Glu Trp Val
35 40 45

Gly Glu Ile Ile Pro Ile Phe Gly His Thr Asn Tyr Asn Glu Lys Phe
50 55 60

Lys Ser Arg Phe Thr Ile Ser Leu Asp Asn Ser Lys Asn Thr Leu Tyr
65 70 75 80

Leu Gln Met Gly Ser Leu Arg Ala Glu Asp Thr Ala Val Tyr Tyr Cys
85 90 95

Ala Arg Gly Gly Tyr Tyr Tyr Tyr Gly Ser Arg Asp Tyr Phe Asp Tyr
100 105 110

Trp Gly Gln Gly Thr Thr Val Thr Val Ser Ser
115 120

<210> 18
<211> 123
<212> PRT
<213> Artificial Sequence

<220>
<221> source
<223> /note="Description of Artificial Sequence: Synthetic polypeptide"

<400> 18

Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Trp Met His Trp Val Arg Gln Ala Pro Gly Lys Gly Leu Glu Trp Val
        35                  40                  45

Gly Glu Ile Ile Pro Ile Phe Gly His Thr Asn Tyr Asn Glu Arg Phe
    50                  55                  60

Gln Gly Arg Phe Thr Ile Ser Leu Asp Asn Ser Lys Asn Thr Leu Tyr
65                  70                  75                  80

Leu Gln Met Gly Ser Leu Arg Ala Glu Asp Thr Ala Val Tyr Tyr Cys
                85                  90                  95

Ala Arg Gly Gly Tyr Tyr Tyr Tyr Gly Ser Arg Asp Tyr Phe Asp Tyr
            100                 105                 110

Trp Gly Gln Gly Thr Thr Val Thr Val Ser Ser
        115                 120

<210> 19
<211> 123
<212> PRT
<213> Artificial Sequence

<220>
<221> source
<223> /note="Description of Artificial Sequence: Synthetic polypeptide"

<400> 19

Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Trp Ile His Trp Val Arg Gln Ala Pro Gly Lys Gly Leu Glu Trp Val
        35                  40                  45

Gly Glu Ile Ile Pro Ile Phe Gly His Thr Asn Tyr Asn Glu Arg Phe
    50                  55                  60

Gln Gly Arg Phe Thr Ile Ser Leu Asp Asn Ser Lys Asn Thr Leu Tyr
65                  70                  75                  80

Leu Gln Met Gly Ser Leu Arg Ala Glu Asp Thr Ala Val Tyr Tyr Cys
85 90 95

Ala Arg Gly Gly Tyr Tyr Tyr Tyr Gly Ser Arg Asp Tyr Phe Asp Tyr
100 105 110

Trp Gly Gln Gly Thr Thr Val Thr Val Ser Ser
115 120

<210> 20
<211> 123
<212> PRT
<213> Artificial Sequence

<220>
<221> source
<223> /note="Description of Artificial Sequence: Synthetic polypeptide"

<400> 20

Glu Val Gln Leu Val Glu Ser Gly Gly Gly Leu Val Lys Pro Gly Gly
1 5 10 15

Ser Leu Arg Leu Ser Cys Ala Ala Ser Gly Phe Thr Phe Ser Ser Tyr
20 25 30

Trp Met His Trp Val Arg Gln Ala Pro Gly Lys Gly Leu Glu Trp Val
35 40 45

Gly Glu Ile Ile Pro Ile Phe Gly His Thr Asn Tyr Asn Glu Lys Phe
50 55 60

Lys Ser Arg Phe Thr Ile Ser Leu Asp Asn Ser Lys Asn Thr Leu Tyr
65 70 75 80

Leu Gln Met Gly Ser Leu Arg Ala Glu Asp Thr Ala Val Tyr Tyr Cys
85 90 95

Ala Arg Gly Gly Tyr Tyr Tyr Tyr Phe Asn Ser Gly Thr Leu Asp Tyr
100 105 110

Trp Gly Gln Gly Thr Thr Val Thr Val Ser Ser
115 120

<210> 21
<211> 123
<212> PRT
<213> Artificial Sequence

<220>

<221> source
<223> /note="Description of Artificial Sequence: Synthetic polypeptide"

<400> 21

```
Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Trp Met His Trp Val Arg Gln Ala Pro Gly Lys Gly Leu Glu Trp Val
        35                  40                  45

Gly Glu Ile Ile Pro Ile Phe Gly His Thr Asn Tyr Asn Glu Lys Phe
    50                  55                  60

Lys Ser Arg Phe Thr Ile Ser Leu Asp Asn Ser Lys Asn Thr Leu Tyr
65                  70                  75                  80

Leu Gln Met Gly Ser Leu Arg Ala Glu Asp Thr Ala Val Tyr Tyr Cys
                85                  90                  95

Ala Arg Gly Gly Tyr Tyr Tyr Tyr Ile Gly Lys Gly Val Leu Asp Tyr
            100                 105                 110

Trp Gly Gln Gly Thr Thr Val Thr Val Ser Ser
        115                 120
```

<210> 22
<211> 123
<212> PRT
<213> Artificial Sequence

<220>
<221> source
<223> /note="Description of Artificial Sequence: Synthetic polypeptide"

<400> 22

```
Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Trp Met His Trp Val Arg Gln Ala Pro Gly Lys Gly Leu Glu Trp Val
        35                  40                  45

Gly Glu Ile Ile Pro Ile Phe Gly His Thr Asn Tyr Asn Glu Lys Phe
    50                  55                  60

Lys Ser Arg Phe Thr Ile Ser Leu Asp Asn Ser Lys Asn Thr Leu Tyr
65                  70                  75                  80

Leu Gln Met Gly Ser Leu Arg Ala Glu Asp Thr Ala Val Tyr Tyr Cys
                85                  90                  95

Ala Arg Gly Gly Tyr Tyr Tyr Tyr Pro Arg Phe Gly Trp Leu Asp Tyr
            100                 105                 110

Trp Gly Gln Gly Thr Thr Val Thr Val Ser Ser
        115                 120
```

<210> 23
<211> 123
<212> PRT
<213> Artificial Sequence

<220>
<221> source
<223> /note="Description of Artificial Sequence: Synthetic polypeptide"

<400> 23

```
Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Trp Met His Trp Val Arg Gln Ala Pro Gly Lys Gly Leu Glu Trp Val
        35                  40                  45

Gly Glu Ile Ile Pro Ile Phe Gly His Thr Asn Tyr Asn Glu Lys Phe
    50                  55                  60

Lys Ser Arg Phe Thr Ile Ser Leu Asp Asn Ser Lys Asn Thr Leu Tyr
65                  70                  75                  80

Leu Gln Met Gly Ser Leu Arg Ala Glu Asp Thr Ala Val Tyr Tyr Cys
                85                  90                  95

Ala Arg Gly Gly Tyr Tyr Tyr Tyr Thr Gly Lys Gly Val Leu Asp Tyr
            100                 105                 110

Trp Gly Gln Gly Thr Thr Val Thr Val Ser Ser
        115                 120
```

<210> 24
<211> 123
<212> PRT
<213> Artificial Sequence

<220>
<221> source
<223> /note="Description of Artificial Sequence: Synthetic polypeptide"

<400> 24

Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Trp Met His Trp Val Arg Gln Ala Pro Gly Lys Gly Leu Glu Trp Val
        35                  40                  45

Gly Glu Ile Ile Pro Ile Phe Gly His Thr Asn Tyr Asn Glu Lys Phe
    50                  55                  60

Lys Ser Arg Phe Thr Ile Ser Leu Asp Asn Ser Lys Asn Thr Leu Tyr
65                  70                  75                  80

Leu Gln Met Gly Ser Leu Arg Ala Glu Asp Thr Ala Val Tyr Tyr Cys
                85                  90                  95

Ala Arg Gly Gly Tyr Tyr Tyr Tyr Asp Ser Asn Ala Val Leu Asp Tyr
            100                 105                 110

Trp Gly Gln Gly Thr Thr Val Thr Val Ser Ser
        115                 120

<210> 25
<211> 123
<212> PRT
<213> Artificial Sequence

<220>
<221> source
<223> /note="Description of Artificial Sequence: Synthetic polypeptide"

<400> 25

Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Trp Met His Trp Val Arg Gln Ala Pro Gly Lys Gly Leu Glu Trp Val
        35                  40                  45

Gly Glu Ile Ile Pro Ile Phe Gly His Thr Asn Tyr Asn Glu Lys Phe

```
    50                  55                  60

Lys Ser Arg Phe Thr Ile Ser Leu Asp Asn Ser Lys Asn Thr Leu Tyr
65                  70                  75                  80

Leu Gln Met Gly Ser Leu Arg Ala Glu Asp Thr Ala Val Tyr Tyr Cys
                85                  90                  95

Ala Arg Gly Gly Tyr Tyr Tyr Tyr Phe His Ser Gly Thr Leu Asp Tyr
            100                 105                 110

Trp Gly Gln Gly Thr Thr Val Thr Val Ser Ser
        115                 120
```

<210> 26
<211> 123
<212> PRT
<213> Artificial Sequence

<220>
<221> source
<223> /note="Description of Artificial Sequence: Synthetic polypeptide"

<400> 26

```
Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Trp Met His Trp Val Arg Gln Ala Pro Gly Lys Gly Leu Glu Trp Val
        35                  40                  45

Gly Glu Ile Ile Pro Ile Phe Gly His Thr Asn Tyr Asn Glu Lys Phe
    50                  55                  60

Lys Ser Arg Phe Thr Ile Ser Leu Asp Asn Ser Lys Asn Thr Leu Tyr
65                  70                  75                  80

Leu Gln Met Gly Ser Leu Arg Ala Glu Asp Thr Ala Val Tyr Tyr Cys
                85                  90                  95

Ala Arg Gly Gly Tyr Tyr Tyr Tyr Phe Asn Lys Ala Val Leu Asp Tyr
            100                 105                 110

Trp Gly Gln Gly Thr Thr Val Thr Val Ser Ser
        115                 120
```

<210> 27
<211> 123
<212> PRT
<213> Artificial Sequence

<220>
<221> source
<223> /note="Description of Artificial Sequence: Synthetic polypeptide"

<400> 27

```
Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Trp Met His Trp Val Arg Gln Ala Pro Gly Lys Gly Leu Glu Trp Val
        35                  40                  45

Gly Glu Ile Ile Pro Ile Phe Gly His Thr Asn Tyr Asn Glu Lys Phe
    50                  55                  60

Lys Ser Arg Phe Thr Ile Ser Leu Asp Asn Ser Lys Asn Thr Leu Tyr
65                  70                  75                  80

Leu Gln Met Gly Ser Leu Arg Ala Glu Asp Thr Ala Val Tyr Tyr Cys
                85                  90                  95

Ala Arg Gly Gly Tyr Tyr Tyr Tyr Gly Gly Ser Gly Val Leu Asp Tyr
            100                 105                 110

Trp Gly Gln Gly Thr Thr Val Thr Val Ser Ser
        115                 120
```

<210> 28
<211> 123
<212> PRT
<213> Artificial Sequence

<220>
<221> source
<223> /note="Description of Artificial Sequence: Synthetic polypeptide"

<400> 28

```
Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Trp Met His Trp Val Arg Gln Ala Pro Gly Lys Gly Leu Glu Trp Val
        35                  40                  45

Gly Glu Ile Ile Pro Ile Phe Gly His Thr Asn Tyr Asn Glu Lys Phe
    50                  55                  60

Lys Ser Arg Phe Thr Ile Ser Leu Asp Asn Ser Lys Asn Thr Leu Tyr
65                  70                  75                  80

Leu Gln Met Gly Ser Leu Arg Ala Glu Asp Thr Ala Val Tyr Tyr Cys
                85                  90                  95

Ala Arg Gly Gly Tyr Tyr Tyr Tyr Pro Arg Gln Gly Phe Leu Asp Tyr
            100                 105                 110

Trp Gly Gln Gly Thr Thr Val Thr Val Ser Ser
        115                 120
```

<210> 29
<211> 123
<212> PRT
<213> Artificial Sequence

<220>
<221> source
<223> /note="Description of Artificial Sequence: Synthetic polypeptide"

<400> 29

```
Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Trp Met His Trp Val Arg Gln Ala Pro Gly Lys Gly Leu Glu Trp Val
        35                  40                  45

Gly Glu Ile Ile Pro Ile Phe Gly His Thr Asn Tyr Asn Glu Lys Phe
    50                  55                  60

Lys Ser Arg Phe Thr Ile Ser Leu Asp Asn Ser Lys Asn Thr Leu Tyr
65                  70                  75                  80

Leu Gln Met Gly Ser Leu Arg Ala Glu Asp Thr Ala Val Tyr Tyr Cys
                85                  90                  95

Ala Arg Gly Gly Tyr Tyr Tyr Tyr Tyr Asn Ser Gly Thr Leu Asp Tyr
            100                 105                 110

Trp Gly Gln Gly Thr Thr Val Thr Val Ser Ser
        115                 120
```

<210> 30
<211> 30
<212> PRT
<213> Artificial Sequence

<220>
<221> source
<223> /note="Description of Artificial Sequence: Synthetic polypeptide"

<400> 30

```
Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser
            20                  25                  30
```

<210> 31
<211> 14
<212> PRT
<213> Artificial Sequence

<220>
<221> source
<223> /note="Description of Artificial Sequence: Synthetic peptide"

<400> 31

Trp Val Arg Gln Ala Pro Gly Lys Gly Leu Glu Trp Val Gly
1 5 10

<210> 32
<211> 32
<212> PRT
<213> Artificial Sequence

<220>
<221> source
<223> /note="Description of Artificial Sequence: Synthetic polypeptide"

<400> 32

Arg Phe Thr Ile Ser Leu Asp Asn Ser Lys Asn Thr Leu Tyr Leu Gln
1 5 10 15

Met Gly Ser Leu Arg Ala Glu Asp Thr Ala Val Tyr Tyr Cys Ala Arg
20 25 30

<210> 33
<211> 11
<212> PRT
<213> Artificial Sequence

<220>
<221> source
<223> /note="Description of Artificial Sequence: Synthetic peptide"

<400> 33

Trp Gly Gln Gly Thr Thr Val Thr Val Ser Ser
1 5 10

<210> 34
<211> 5
<212> PRT
<213> Artificial Sequence

<220>
<221> source
<223> /note="Description of Artificial Sequence: Synthetic peptide"

<400> 34
Ser Tyr Trp Ile His
1 5

<210> 35
<211> 17
<212> PRT
<213> Artificial Sequence

<220>
<221> source
<223> /note="Description of Artificial Sequence: Synthetic peptide"

<400> 35

Glu Ile Ile Pro Ile Phe Gly His Thr Asn Tyr Asn Glu Lys Phe Lys
1 5 10 15

Ser

<210> 36
<211> 17
<212> PRT
<213> Artificial Sequence

<220>
<221> source
<223> /note="Description of Artificial Sequence: Synthetic peptide"

<400> 36

Glu Ile Ile Pro Ile Phe Gly His Thr Asn Tyr Asn Glu Arg Phe Gln
1 5 10 15

Gly

<210> 37
<211> 14
<212> PRT
<213> Artificial Sequence

<220>
<221> source
<223> /note="Description of Artificial Sequence: Synthetic peptide"

<400> 37

Gly Gly Tyr Tyr Tyr Tyr Phe Asn Ser Gly Thr Leu Asp Tyr
1 5 10

<210> 38
<211> 14
<212> PRT
<213> Artificial Sequence

<220>
<221> source
<223> /note="Description of Artificial Sequence: Synthetic peptide"

<400> 38

Gly Gly Tyr Tyr Tyr Tyr Ile Gly Lys Gly Val Leu Asp Tyr
1 5 10

<210> 39
<211> 14
<212> PRT
<213> Artificial Sequence

<220>
<221> source

<223> /note="Description of Artificial Sequence: Synthetic peptide"

<400> 39

```
Gly Gly Tyr Tyr Tyr Tyr Pro Arg Phe Gly Trp Leu Asp Tyr
1               5                   10
```

<210> 40
<211> 14
<212> PRT
<213> Artificial Sequence

<220>
<221> source
<223> /note="Description of Artificial Sequence: Synthetic peptide"

<400> 40

```
Gly Gly Tyr Tyr Tyr Tyr Thr Gly Lys Gly Val Leu Asp Tyr
1               5                   10
```

<210> 41
<211> 14
<212> PRT
<213> Artificial Sequence

<220>
<221> source
<223> /note="Description of Artificial Sequence: Synthetic peptide"

<400> 41

```
Gly Gly Tyr Tyr Tyr Tyr Asp Ser Asn Ala Val Leu Asp Tyr
1               5                   10
```

<210> 42
<211> 14
<212> PRT
<213> Artificial Sequence

<220>
<221> source
<223> /note="Description of Artificial Sequence: Synthetic peptide"

<400> 42

```
Gly Gly Tyr Tyr Tyr Tyr Phe His Ser Gly Thr Leu Asp Tyr
1               5                   10
```

<210> 43
<211> 14
<212> PRT
<213> Artificial Sequence

<220>
<221> source
<223> /note="Description of Artificial Sequence: Synthetic peptide"

<400> 43

Gly Gly Tyr Tyr Tyr Tyr Phe Asn Lys Ala Val Leu Asp Tyr
1 5 10

<210> 44
<211> 14
<212> PRT
<213> Artificial Sequence

<220>
<221> source
<223> /note="Description of Artificial Sequence: Synthetic peptide"

<400> 44

Gly Gly Tyr Tyr Tyr Tyr Gly Gly Ser Gly Val Leu Asp Tyr
1 5 10

<210> 45
<211> 14
<212> PRT
<213> Artificial Sequence

<220>
<221> source
<223> /note="Description of Artificial Sequence: Synthetic peptide"

<400> 45

Gly Gly Tyr Tyr Tyr Tyr Pro Arg Gln Gly Phe Leu Asp Tyr
1 5 10

<210> 46
<211> 14
<212> PRT
<213> Artificial Sequence

<220>
<221> source
<223> /note="Description of Artificial Sequence: Synthetic peptide"

<400> 46

Gly Gly Tyr Tyr Tyr Tyr Tyr Asn Ser Gly Thr Leu Asp Tyr
1 5 10

<210> 47
<211> 5
<212> PRT
<213> Artificial Sequence

<220>
<221> source
<223> /note="Description of Artificial Sequence: Synthetic peptide"

<220>
<221> VARIANT

<222> (4)..(4)
<223> /replace="Ile"

<220>
<221> MISC_FEATURE
<222> (1)..(5)
<223> /note="Variant residues given in the sequence have no preference with respect to those in the annotations for variant positions"

<400> 47
Ser Tyr Trp Met His
1 5

<210> 48
<211> 17
<212> PRT
<213> Artificial Sequence

<220>
<221> source
<223> /note="Description of Artificial Sequence: Synthetic peptide"

<220>
<221> VARIANT
<222> (6)..(6)
<223> /replace="Phe"

<220>
<221> VARIANT
<222> (14)..(14)
<223> /replace="Arg"

<220>
<221> VARIANT
<222> (16)..(16)
<223> /replace="Gln"

<220>
<221> VARIANT
<222> (17)..(17)
<223> /replace="Gly"

<220>
<221> MISC_FEATURE
<222> (1)..(17)
<223> /note="Variant residues given in the sequence have no preference with respect to those in the annotations for variant positions"

<400> 48

```
Glu Ile Ile Pro Ile Asn Gly His Thr Asn Tyr Asn Glu Lys Phe Lys
1               5                   10                  15
```

Ser

<210> 49
<211> 14
<212> PRT

<213> Artificial Sequence

<220>
<221> source
<223> /note="Description of Artificial Sequence: Synthetic peptide"

<220>
<221> VARIANT
<222> (7)..(7)
<223> /replace="Phe" or "Tyr" or "Trp" or "Ile" or "Leu" "Val" or "Thr" or "Gly" or "Asp"

<220>
<221> VARIANT
<222> (8)..(8)
<223> /replace="Arg" or "Asn" or "His" or "Gly" or "Ser"

<220>
<221> VARIANT
<222> (9)..(9)
<223> /replace="Met" or "Ser" or "Lys" or Asn"

<220>
<221> VARIANT
<222> (10)..(10)
<223> /replace="Ala"

<220>
<221> VARIANT
<222> (11)..(11)
<223> /replace="Phe" or "Thr" or "Val"

<220>
<221> VARIANT
<222> (12)..(12)
<223> /replace="Leu" or "Lys"

<220>
<221> MISC_FEATURE
<222> (1)..(14)
<223> /note="Variant residues given in the sequence have no preference with respect to those in the annotations for variant positions"

<220>
<221> source
<223> /note="See specification as filed for detailed description of substitutions and preferred embodiments"

<400> 49

```
Gly Gly Tyr Tyr Tyr Tyr Pro Lys Phe Gly Trp Met Asp Tyr
1               5                   10
```

<210> 50
<211> 453
<212> PRT
<213> Artificial Sequence

<220>
<221> source

<223> /note="Description of Artificial Sequence: Synthetic polypeptide"

<220>
<221> VARIANT
<222> (453)..(453)
<223> /replace=" "

<220>
<221> MISC_FEATURE
<222> (1)..(453)
<223> /note="Variant residues given in the sequence have no preference with respect to those in the annotations for variant positions"

<400> 50

```
Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Trp Met His Trp Val Arg Gln Ala Pro Gly Lys Gly Leu Glu Trp Val
        35                  40                  45
```

Gly Glu Ile Ile Pro Ile Phe Gly His Thr Asn Tyr Asn Glu Lys Phe
    50                  55                  60

Lys Ser Arg Phe Thr Ile Ser Leu Asp Asn Ser Lys Asn Thr Leu Tyr
65                  70                  75                  80

Leu Gln Met Gly Ser Leu Arg Ala Glu Asp Thr Ala Val Tyr Tyr Cys
                85                  90                  95

Ala Arg Gly Gly Tyr Tyr Tyr Tyr Gly Ser Arg Asp Tyr Phe Asp Tyr
            100                 105                 110

Trp Gly Gln Gly Thr Thr Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Arg Val Glu Pro Lys
    210                 215                 220

Ser Cys Asp Lys Thr His Thr Cys Pro Pro Cys Pro Ala Pro Glu Leu
225                 230                 235                 240

Leu Gly Gly Pro Ser Val Phe Leu Phe Pro Pro Lys Pro Lys Asp Thr
                245                 250                 255

Leu Met Ile Ser Arg Thr Pro Glu Val Thr Cys Val Val Val Asp Val
            260                 265                 270

Ser His Glu Asp Pro Glu Val Lys Phe Asn Trp Tyr Val Asp Gly Val
        275                 280                 285

Glu Val His Asn Ala Lys Thr Lys Pro Arg Glu Glu Gln Tyr Asn Ser

290 295 300

Thr Tyr Arg Val Val Ser Val Leu Thr Val Leu His Gln Asp Trp Leu
305 310 315 320

Asn Gly Lys Glu Tyr Lys Cys Lys Val Ser Asn Lys Ala Leu Pro Ala
325 330 335

Pro Ile Glu Lys Thr Ile Ser Lys Ala Lys Gly Gln Pro Arg Glu Pro
340 345 350

Gln Val Tyr Thr Leu Pro Pro Ser Arg Glu Glu Met Thr Lys Asn Gln
355 360 365

Val Ser Leu Thr Cys Leu Val Lys Gly Phe Tyr Pro Ser Asp Ile Ala
370 375 380

Val Glu Trp Glu Ser Asn Gly Gln Pro Glu Asn Asn Tyr Lys Thr Thr
385 390 395 400

Pro Pro Val Leu Asp Ser Asp Gly Ser Phe Phe Leu Tyr Ser Lys Leu
405 410 415

Thr Val Asp Lys Ser Arg Trp Gln Gln Gly Asn Val Phe Ser Cys Ser
420 425 430

Val Met His Glu Ala Leu His Asn His Tyr Thr Gln Lys Ser Leu Ser
435 440 445

Leu Ser Pro Gly Lys
450

<210> 51
<211> 453
<212> PRT
<213> Artificial Sequence

<220>
<221> source
<223> /note="Description of Artificial Sequence: Synthetic polypeptide"

<220>
<221> VARIANT
<222> (453)..(453)
<223> /replace=" "

<220>
<221> MISC_FEATURE
<222> (1)..(453)
<223> /note="Variant residues given in the sequence have no preference with respect to those in the annotations

for variant positions"

<400> 51

```
Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Trp Met His Trp Val Arg Gln Ala Pro Gly Lys Gly Leu Glu Trp Val
        35                  40                  45

Gly Glu Ile Ile Pro Ile Phe Gly His Thr Asn Tyr Asn Glu Lys Phe
    50                  55                  60

Lys Ser Arg Phe Thr Ile Ser Leu Asp Asn Ser Lys Asn Thr Leu Tyr
65                  70                  75                  80

Leu Gln Met Gly Ser Leu Arg Ala Glu Asp Thr Ala Val Tyr Tyr Cys
                85                  90                  95

Ala Arg Gly Gly Tyr Tyr Tyr Tyr Pro Arg Phe Gly Trp Leu Asp Tyr
            100                 105                 110

Trp Gly Gln Gly Thr Thr Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Arg Val Glu Pro Lys
    210                 215                 220

Ser Cys Asp Lys Thr His Thr Cys Pro Pro Cys Pro Ala Pro Glu Leu
225                 230                 235                 240
```

```
Leu Gly Gly Pro Ser Val Phe Leu Phe Pro Pro Lys Pro Lys Asp Thr
                245                 250                 255

Leu Met Ile Ser Arg Thr Pro Glu Val Thr Cys Val Val Val Asp Val
            260                 265                 270

Ser His Glu Asp Pro Glu Val Lys Phe Asn Trp Tyr Val Asp Gly Val
        275                 280                 285

Glu Val His Asn Ala Lys Thr Lys Pro Arg Glu Glu Gln Tyr Asn Ser
    290                 295                 300

Thr Tyr Arg Val Val Ser Val Leu Thr Val Leu His Gln Asp Trp Leu
305                 310                 315                 320

Asn Gly Lys Glu Tyr Lys Cys Lys Val Ser Asn Lys Ala Leu Pro Ala
                325                 330                 335

Pro Ile Glu Lys Thr Ile Ser Lys Ala Lys Gly Gln Pro Arg Glu Pro
            340                 345                 350

Gln Val Tyr Thr Leu Pro Pro Ser Arg Glu Glu Met Thr Lys Asn Gln
        355                 360                 365

Val Ser Leu Thr Cys Leu Val Lys Gly Phe Tyr Pro Ser Asp Ile Ala
    370                 375                 380

Val Glu Trp Glu Ser Asn Gly Gln Pro Glu Asn Asn Tyr Lys Thr Thr
385                 390                 395                 400

Pro Pro Val Leu Asp Ser Asp Gly Ser Phe Phe Leu Tyr Ser Lys Leu
                405                 410                 415

Thr Val Asp Lys Ser Arg Trp Gln Gln Gly Asn Val Phe Ser Cys Ser
            420                 425                 430

Val Met His Glu Ala Leu His Asn His Tyr Thr Gln Lys Ser Leu Ser
        435                 440                 445

Leu Ser Pro Gly Lys
    450
```

<210> 52
<211> 453
<212> PRT
<213> Artificial Sequence

<220>

<221> source
<223> /note="Description of Artificial Sequence: Synthetic polypeptide"

<220>
<221> VARIANT
<222> (453)..(453)
<223> /replace="

<220>
<221> MISC_FEATURE
<222> (1)..(453)
<223> /note="Variant residues given in the sequence have no preference with respect to those in the annotations for variant positions"

<400> 52

```
Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Trp Met His Trp Val Arg Gln Ala Pro Gly Lys Gly Leu Glu Trp Val
        35                  40                  45

Gly Glu Ile Ile Pro Ile Phe Gly His Thr Asn Tyr Asn Glu Lys Phe
    50                  55                  60

Lys Ser Arg Phe Thr Ile Ser Leu Asp Asn Ser Lys Asn Thr Leu Tyr
65                  70                  75                  80

Leu Gln Met Gly Ser Leu Arg Ala Glu Asp Thr Ala Val Tyr Tyr Cys
                85                  90                  95

Ala Arg Gly Gly Tyr Tyr Tyr Tyr Pro Arg Gln Gly Phe Leu Asp Tyr
            100                 105                 110

Trp Gly Gln Gly Thr Thr Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
```

180 185 190

Thr Val Pro Ser Ser Ser Leu Gly Thr Gln Thr Tyr Ile Cys Asn Val
195 200 205

Asn His Lys Pro Ser Asn Thr Lys Val Asp Lys Arg Val Glu Pro Lys
210 215 220

Ser Cys Asp Lys Thr His Thr Cys Pro Pro Cys Pro Ala Pro Glu Leu
225 230 235 240

Leu Gly Gly Pro Ser Val Phe Leu Phe Pro Pro Lys Pro Lys Asp Thr
245 250 255

Leu Met Ile Ser Arg Thr Pro Glu Val Thr Cys Val Val Val Asp Val
260 265 270

Ser His Glu Asp Pro Glu Val Lys Phe Asn Trp Tyr Val Asp Gly Val
275 280 285

Glu Val His Asn Ala Lys Thr Lys Pro Arg Glu Glu Gln Tyr Asn Ser
290 295 300

Thr Tyr Arg Val Val Ser Val Leu Thr Val Leu His Gln Asp Trp Leu
305 310 315 320

Asn Gly Lys Glu Tyr Lys Cys Lys Val Ser Asn Lys Ala Leu Pro Ala
325 330 335

Pro Ile Glu Lys Thr Ile Ser Lys Ala Lys Gly Gln Pro Arg Glu Pro
340 345 350

Gln Val Tyr Thr Leu Pro Pro Ser Arg Glu Glu Met Thr Lys Asn Gln
355 360 365

Val Ser Leu Thr Cys Leu Val Lys Gly Phe Tyr Pro Ser Asp Ile Ala
370 375 380

Val Glu Trp Glu Ser Asn Gly Gln Pro Glu Asn Asn Tyr Lys Thr Thr
385 390 395 400

Pro Pro Val Leu Asp Ser Asp Gly Ser Phe Phe Leu Tyr Ser Lys Leu
405 410 415

Thr Val Asp Lys Ser Arg Trp Gln Gln Gly Asn Val Phe Ser Cys Ser
420 425 430

Val Met His Glu Ala Leu His Asn His Tyr Thr Gln Lys Ser Leu Ser
435 440 445

Leu Ser Pro Gly Lys
450

<210> 53
<211> 111
<212> PRT
<213> Artificial Sequence

<220>
<221> source
<223> /note="Description of Artificial Sequence: Synthetic polypeptide"

<220>
<221> VARIANT
<222> (24)..(24)
<223> /replace="Arg"

<220>
<221> VARIANT
<222> (32)..(32)
<223> /replace="Ser"

<220>
<221> VARIANT
<222> (37)..(37)
<223> /replace="Leu"

<220>
<221> VARIANT
<222> (96)..(96)
<223> /replace="Tyr"

<220>
<221> VARIANT
<222> (97)..(97)
<223> /replace="Ser"

<220>
<221> VARIANT
<222> (98)..(98)
<223> /replace="Thr"

<220>
<221> MISC_FEATURE
<222> (1)..(111)
<223> /note="Variant residues given in the sequence have no preference with respect to those in the annotations for variant positions"

<400> 53

Asp Ile Val Met Thr Gln Ser Pro Asp Ser Leu Ala Val Ser Leu Gly
1 5 10 15

Glu Arg Ala Thr Ile Ser Cys Lys Ala Ser Gln Ser Val Asp Tyr Asp
20 25 30

Gly Asp Ser Tyr Met Asn Trp Tyr Gln Gln Lys Pro Gly Gln Pro Pro
35 40 45

Lys Leu Leu Ile Tyr Ala Ala Ser Asp Leu Glu Ser Gly Ile Pro Ala
50 55 60

Arg Phe Ser Gly Ser Gly Ser Gly Thr Asp Phe Thr Leu Thr Ile Ser
65 70 75 80

Ser Leu Glu Pro Glu Asp Phe Ala Thr Tyr Tyr Cys Gln Gln Ser His
85 90 95

Glu Asp Pro Phe Thr Phe Gly Gln Gly Thr Lys Leu Glu Ile Lys
100 105 110

<210> 54
<211> 111
<212> PRT
<213> Artificial Sequence

<220>
<221> source
<223> /note="Description of Artificial Sequence: Synthetic polypeptide"

<400> 54

```
Asp Ile Val Met Thr Gln Ser Pro Asp Ser Leu Ala Val Ser Leu Gly
1               5                   10                  15

Glu Arg Ala Thr Ile Ser Cys Lys Ala Ser Gln Ser Val Asp Tyr Asp
            20                  25                  30

Gly Asp Ser Tyr Met Asn Trp Tyr Gln Gln Lys Pro Gly Gln Pro Pro
        35                  40                  45

Lys Leu Leu Ile Tyr Ala Ala Ser Asp Leu Glu Ser Gly Ile Pro Ala
    50                  55                  60

Arg Phe Ser Gly Ser Gly Ser Gly Thr Asp Phe Thr Leu Thr Ile Ser
65                  70                  75                  80

Ser Leu Glu Pro Glu Asp Phe Ala Thr Tyr Tyr Cys Gln Gln Ser His
                85                  90                  95

Glu Asp Pro Phe Thr Phe Gly Gln Gly Thr Lys Leu Glu Ile Lys
            100                 105                 110
```

<210> 55
<211> 111
<212> PRT
<213> Artificial Sequence

<220>
<221> source
<223> /note="Description of Artificial Sequence: Synthetic polypeptide"

<400> 55

```
Asp Ile Val Met Thr Gln Ser Pro Asp Ser Leu Ala Val Ser Leu Gly
1               5                   10                  15

Glu Arg Ala Thr Ile Ser Cys Lys Ala Ser Gln Ser Val Asp Tyr Ser
            20                  25                  30

Gly Asp Ser Tyr Met Asn Trp Tyr Gln Gln Lys Pro Gly Gln Pro Pro
        35                  40                  45

Lys Leu Leu Ile Tyr Ala Ala Ser Asp Leu Glu Ser Gly Ile Pro Ala
    50                  55                  60

Arg Phe Ser Gly Ser Gly Ser Gly Thr Asp Phe Thr Leu Thr Ile Ser
65                  70                  75                  80

Ser Leu Glu Pro Glu Asp Phe Ala Thr Tyr Tyr Cys Gln Gln Ser His
                85                  90                  95

Glu Asp Pro Phe Thr Phe Gly Gln Gly Thr Lys Leu Glu Ile Lys
            100                 105                 110
```

<210> 56
<211> 111
<212> PRT
<213> Artificial Sequence

<220>
<221> source
<223> /note="Description of Artificial Sequence: Synthetic polypeptide"

<400> 56

```
Asp Ile Val Met Thr Gln Ser Pro Asp Ser Leu Ala Val Ser Leu Gly
1               5                   10                  15

Glu Arg Ala Thr Ile Ser Cys Arg Ala Ser Gln Ser Val Asp Tyr Ser
            20                  25                  30

Gly Asp Ser Tyr Met Asn Trp Tyr Gln Gln Lys Pro Gly Gln Pro Pro
        35                  40                  45

Lys Leu Leu Ile Tyr Ala Ala Ser Asp Leu Glu Ser Gly Ile Pro Ala
    50                  55                  60
```

Arg Phe Ser Gly Ser Gly Ser Gly Thr Asp Phe Thr Leu Thr Ile Ser
65 70 75 80

Ser Leu Glu Pro Glu Asp Phe Ala Thr Tyr Tyr Cys Gln Gln Ser His
85 90 95

Glu Asp Pro Phe Thr Phe Gly Gln Gly Thr Lys Leu Glu Ile Lys
100 105 110

<210> 57
<211> 111
<212> PRT
<213> Artificial Sequence

<220>
<221> source
<223> /note="Description of Artificial Sequence: Synthetic polypeptide"

<400> 57

Asp Ile Val Met Thr Gln Ser Pro Asp Ser Leu Ala Val Ser Leu Gly
1 5 10 15

Glu Arg Ala Thr Ile Ser Cys Arg Ala Ser Gln Ser Val Asp Tyr Ser
20 25 30

Gly Asp Ser Tyr Leu Asn Trp Tyr Gln Gln Lys Pro Gly Gln Pro Pro
35 40 45

Lys Leu Leu Ile Tyr Ala Ala Ser Asp Leu Glu Ser Gly Ile Pro Ala
50 55 60

Arg Phe Ser Gly Ser Gly Ser Gly Thr Asp Phe Thr Leu Thr Ile Ser
65 70 75 80

Ser Leu Glu Pro Glu Asp Phe Ala Thr Tyr Tyr Cys Gln Gln Ser Tyr
85 90 95

Ser Thr Pro Phe Thr Phe Gly Gln Gly Thr Lys Leu Glu Ile Lys
100 105 110

<210> 58
<211> 23
<212> PRT
<213> Artificial Sequence

<220>
<221> source
<223> /note="Description of Artificial Sequence: Synthetic peptide"

<400> 58

Asp Ile Val Met Thr Gln Ser Pro Asp Ser Leu Ala Val Ser Leu Gly
1 5 10 15

Glu Arg Ala Thr Ile Ser Cys
20

<210> 59
<211> 15
<212> PRT
<213> Artificial Sequence

<220>
<221> source
<223> /note="Description of Artificial Sequence: Synthetic peptide"

<400> 59

Trp Tyr Gln Gln Lys Pro Gly Gln Pro Pro Lys Leu Leu Ile Tyr
1 5 10 15

<210> 60
<211> 32
<212> PRT
<213> Artificial Sequence

<220>
<221> source
<223> /note="Description of Artificial Sequence: Synthetic polypeptide"

<400> 60

Gly Ile Pro Ala Arg Phe Ser Gly Ser Gly Ser Gly Thr Asp Phe Thr
1 5 10 15

Leu Thr Ile Ser Ser Leu Glu Pro Glu Asp Phe Ala Thr Tyr Tyr Cys
20 25 30

<210> 61
<211> 10
<212> PRT
<213> Artificial Sequence

<220>
<221> source
<223> /note="Description of Artificial Sequence: Synthetic peptide"

<400> 61

Phe Gly Gln Gly Thr Lys Leu Glu Ile Lys
1 5 10

<210> 62
<211> 15
<212> PRT
<213> Artificial Sequence

<220>
<221> source
<223> /note="Description of Artificial Sequence: Synthetic peptide"

<400> 62

**Lys Ala Ser Gln Ser Val Asp Tyr Ser Gly Asp Ser Tyr Met Asn**
**1 5 10 15**

<210> 63
<211> 15
<212> PRT
<213> Artificial Sequence

<220>
<221> source
<223> /note="Description of Artificial Sequence: Synthetic peptide"

<400> 63

**Arg Ala Ser Gln Ser Val Asp Tyr Ser Gly Asp Ser Tyr Met Asn**
**1 5 10 15**

<210> 64
<211> 15
<212> PRT
<213> Artificial Sequence

<220>
<221> source
<223> /note="Description of Artificial Sequence: Synthetic peptide"

<400> 64

**Arg Ala Ser Gln Ser Val Asp Tyr Ser Gly Asp Ser Tyr Leu Asn**
**1 5 10 15**

<210> 65
<211> 9
<212> PRT
<213> Artificial Sequence

<220>
<221> source
<223> /note="Description of Artificial Sequence: Synthetic peptide"

<400> 65
Gln Gln Ser Tyr Ser Thr Pro Phe Thr
1 5

<210> 66
<211> 15
<212> PRT
<213> Artificial Sequence

<220>
<221> source
<223> /note="Description of Artificial Sequence: Synthetic peptide"

<220>
<221> VARIANT
<222> (1)..(1)
<223> /replace="Arg"

<220>
<221> VARIANT
<222> (9)..(9)
<223> /replace="Ser"

<220>
<221> VARIANT
<222> (14)..(14)
<223> /replace="Leu"

<220>
<221> MISC_FEATURE
<222> (1)..(15)
<223> /note="Variant residues given in the sequence have no preference with respect to those in the annotations for variant positions"

<400> 66

```
Lys Ala Ser Gln Ser Val Asp Tyr Asp Gly Asp Ser Tyr Met Asn
1               5                   10                  15
```

<210> 67
<211> 9
<212> PRT
<213> Artificial Sequence

<220>
<221> source
<223> /note="Description of Artificial Sequence: Synthetic peptide"

<220>
<221> VARIANT
<222> (4)..(4)
<223> /replace="Tyr"

<220>
<221> VARIANT
<222> (5)..(5)
<223> /replace="Ser"

<220>
<221> VARIANT
<222> (6)..(6)
<223> /replace="Thr"

<220>
<221> MISC_FEATURE
<222> (1)..(9)
<223> /note="Variant residues given in the sequence have no preference with respect to those in the annotations for variant positions"

<400> 67

Gln Gln Ser His Glu Asp Pro Phe Thr
1               5

<210> 68
<211> 218
<212> PRT
<213> Artificial Sequence

<220>
<221> source
<223> /note="Description of Artificial Sequence: Synthetic polypeptide"

<400> 68

```
Asp Ile Val Met Thr Gln Ser Pro Asp Ser Leu Ala Val Ser Leu Gly
1               5                   10                  15

Glu Arg Ala Thr Ile Ser Cys Lys Ala Ser Gln Ser Val Asp Tyr Ser
            20                  25                  30

Gly Asp Ser Tyr Met Asn Trp Tyr Gln Gln Lys Pro Gly Gln Pro Pro
        35                  40                  45

Lys Leu Leu Ile Tyr Ala Ala Ser Asp Leu Glu Ser Gly Ile Pro Ala
    50                  55                  60

Arg Phe Ser Gly Ser Gly Ser Gly Thr Asp Phe Thr Leu Thr Ile Ser
65                  70                  75                  80

Ser Leu Glu Pro Glu Asp Phe Ala Thr Tyr Tyr Cys Gln Gln Ser His
                85                  90                  95

Glu Asp Pro Phe Thr Phe Gly Gln Gly Thr Lys Leu Glu Ile Lys Arg
            100                 105                 110

Thr Val Ala Ala Pro Ser Val Phe Ile Phe Pro Pro Ser Asp Glu Gln
        115                 120                 125

Leu Lys Ser Gly Thr Ala Ser Val Val Cys Leu Leu Asn Asn Phe Tyr
    130                 135                 140

Pro Arg Glu Ala Lys Val Gln Trp Lys Val Asp Asn Ala Leu Gln Ser
145                 150                 155                 160

Gly Asn Ser Gln Glu Ser Val Thr Glu Gln Asp Ser Lys Asp Ser Thr
                165                 170                 175

Tyr Ser Leu Ser Ser Thr Leu Thr Leu Ser Lys Ala Asp Tyr Glu Lys
            180                 185                 190
```

```
His Lys Val Tyr Ala Cys Glu Val Thr His Gln Gly Leu Ser Ser Pro
        195                 200                 205

Val Thr Lys Ser Phe Asn Arg Gly Glu Cys
    210                 215
```

<210> 69
<211> 107
<212> PRT
<213> Homo sapiens

<400> 69

```
Arg Thr Val Ala Ala Pro Ser Val Phe Ile Phe Pro Pro Ser Asp Glu
1               5                   10                  15

Gln Leu Lys Ser Gly Thr Ala Ser Val Val Cys Leu Leu Asn Asn Phe
            20                  25                  30

Tyr Pro Arg Glu Ala Lys Val Gln Trp Lys Val Asp Asn Ala Leu Gln
        35                  40                  45

Ser Gly Asn Ser Gln Glu Ser Val Thr Glu Gln Asp Ser Lys Asp Ser
    50                  55                  60

Thr Tyr Ser Leu Ser Ser Thr Leu Thr Leu Ser Lys Ala Asp Tyr Glu
65                  70                  75                  80

Lys His Lys Val Tyr Ala Cys Glu Val Thr His Gln Gly Leu Ser Ser
                85                  90                  95

Pro Val Thr Lys Ser Phe Asn Arg Gly Glu Cys
            100                 105
```

<210> 70
<211> 104
<212> PRT
<213> Homo sapiens

<400> 70

```
Gln Pro Lys Ala Ala Pro Ser Val Thr Leu Phe Pro Pro Ser Ser Glu
1               5                   10                  15

Glu Leu Gln Ala Asn Lys Ala Thr Leu Val Cys Leu Ile Ser Asp Phe
            20                  25                  30

Tyr Pro Gly Ala Val Thr Val Ala Trp Lys Ala Asp Ser Ser Pro Val
        35                  40                  45

Lys Ala Gly Val Glu Thr Thr Pro Ser Lys Gln Ser Asn Asn Lys Tyr
    50                  55                  60

Ala Ala Ser Ser Tyr Leu Ser Leu Thr Pro Glu Gln Trp Lys Ser His
65                  70                  75                  80

Arg Ser Tyr Ser Cys Gln Val Thr His Glu Gly Ser Thr Val Glu Lys
                85                  90                  95

Thr Val Ala Pro Thr Glu Cys Ser
            100
```

<210> 71
<211> 98
<212> PRT
<213> Homo sapiens

<400> 71

```
Ala Ser Thr Lys Gly Pro Ser Val Phe Pro Leu Ala Pro Ser Ser Lys
1               5                   10                  15

Ser Thr Ser Gly Gly Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Ser Leu Gly Thr Gln Thr
65                  70                  75                  80

Tyr Ile Cys Asn Val Asn His Lys Pro Ser Asn Thr Lys Val Asp Lys
                85                  90                  95

Arg Val
```

<210> 72
<211> 98
<212> PRT
<213> Homo sapiens

<400> 72

```
Ala Ser Thr Lys Gly Pro Ser Val Phe Pro Leu Ala Pro Cys Ser Arg
1               5                   10                  15

Ser Thr Ser Glu Ser Thr Ala Ala Leu Gly Cys Leu Val Lys Asp Tyr
            20                  25                  30

Phe Pro Glu Pro Val Thr Val Ser Trp Asn Ser Gly Ala Leu Thr Ser
        35                  40                  45

Gly Val His Thr Phe Pro Ala Val Leu Gln Ser Ser Gly Leu Tyr Ser
    50                  55                  60

Leu Ser Ser Val Val Thr Val Pro Ser Ser Asn Phe Gly Thr Gln Thr
65                  70                  75                  80

Tyr Thr Cys Asn Val Asp His Lys Pro Ser Asn Thr Lys Val Asp Lys
                85                  90                  95

Thr Val
```

<210> 73
<211> 98
<212> PRT
<213> Homo sapiens

<400> 73

Ala Ser Thr Lys Gly Pro Ser Val Phe Pro Leu Ala Pro Cys Ser Arg
1 5 10 15

Ser Thr Ser Glu Ser Thr Ala Ala Leu Gly Cys Leu Val Lys Asp Tyr
20 25 30

Phe Pro Glu Pro Val Thr Val Ser Trp Asn Ser Gly Ala Leu Thr Ser
35 40 45

Gly Val His Thr Phe Pro Ala Val Leu Gln Ser Ser Gly Leu Tyr Ser
50 55 60

Leu Ser Ser Val Val Thr Val Pro Ser Ser Ser Leu Gly Thr Lys Thr
65 70 75 80

Tyr Thr Cys Asn Val Asp His Lys Pro Ser Asn Thr Lys Val Asp Lys
85 90 95

Arg Val

<210> 74
<211> 15
<212> PRT
<213> Homo sapiens

<400> 74

Glu Pro Lys Ser Cys Asp Lys Thr His Thr Cys Pro Pro Cys Pro
1 5 10 15

<210> 75
<211> 12
<212> PRT
<213> Homo sapiens

<400> 75

Glu Arg Lys Cys Cys Val Glu Cys Pro Pro Cys Pro
1 5 10

<210> 76
<211> 12
<212> PRT
<213> Homo sapiens

<400> 76

Glu Ser Lys Tyr Gly Pro Pro Cys Pro Ser Cys Pro
1 5 10

<210> 77
<211> 12
<212> PRT

<213> Artificial Sequence

<220>
<221> source
<223> /note="Description of Artificial Sequence: Synthetic peptide"

<400> 77

```
Glu Ser Lys Tyr Gly Pro Pro Cys Pro Pro Cys Pro
1               5                   10
```

<210> 78
<211> 217
<212> PRT
<213> Artificial Sequence

<220>
<221> source
<223> /note="Description of Artificial Sequence: Synthetic polypeptide"

<220>
<221> VARIANT
<222> (217)..(217)
<223> /replace=" "

<220>
<221> MISC_FEATURE
<222> (1)..(217)
<223> /note="Variant residues given in the sequence have no preference with respect to those in the annotations for variant positions"

<400> 78

```
Ala Pro Glu Ala Ala Gly Gly Pro Ser Val Phe Leu Phe Pro Pro Lys
1               5                   10                  15

Pro Lys Asp Thr Leu Met Ile Ser Arg Thr Pro Glu Val Thr Cys Val
```

20 25 30

Val Val Asp Val Ser His Glu Asp Pro Glu Val Lys Phe Asn Trp Tyr
35 40 45

Val Asp Gly Val Glu Val His Asn Ala Lys Thr Lys Pro Arg Glu Glu
50 55 60

Gln Tyr Asn Ser Thr Tyr Arg Val Val Ser Val Leu Thr Val Leu His
65 70 75 80

Gln Asp Trp Leu Asn Gly Lys Glu Tyr Lys Cys Lys Val Ser Asn Lys
85 90 95

Ala Leu Pro Ala Pro Ile Glu Lys Thr Ile Ser Lys Ala Lys Gly Gln
100 105 110

Pro Arg Glu Pro Gln Val Tyr Thr Leu Pro Pro Ser Arg Glu Glu Met
115 120 125

Thr Lys Asn Gln Val Ser Leu Thr Cys Leu Val Lys Gly Phe Tyr Pro
130 135 140

Ser Asp Ile Ala Val Glu Trp Glu Ser Asn Gly Gln Pro Glu Asn Asn
145 150 155 160

Tyr Lys Thr Thr Pro Pro Val Leu Asp Ser Asp Gly Ser Phe Phe Leu
165 170 175

Tyr Ser Lys Leu Thr Val Asp Lys Ser Arg Trp Gln Gln Gly Asn Val
180 185 190

Phe Ser Cys Ser Val Met His Glu Ala Leu His Asn His Tyr Thr Gln
195 200 205

Lys Ser Leu Ser Leu Ser Pro Gly Lys
210 215

<210> 79
<211> 217
<212> PRT
<213> Artificial Sequence

<220>
<221> source
<223> /note="Description of Artificial Sequence: Synthetic polypeptide"

<220>
<221> VARIANT

<222> (217)..(217)
<223> /replace=" "

<220>
<221> MISC_FEATURE
<222> (1)..(217)
<223> /note="Variant residues given in the sequence have no preference with respect to those in the annotations for variant positions"

<400> 79

```
Ala Pro Glu Leu Leu Gly Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Val Val Asp Val Ser His Glu Asp Pro Glu Val Lys Phe Asn Trp Tyr
        35                  40                  45

Val Asp Gly Val Glu Val His Asn Ala Lys Thr Lys Pro Arg Glu Glu
    50                  55                  60

Gln Tyr Asn Ser Thr Tyr Arg Val Val Ser Val Leu Thr Val Leu His
65                  70                  75                  80

Gln Asp Trp Leu Asn Gly Lys Glu Tyr Lys Cys Lys Val Ser Asn Lys
                85                  90                  95

Ala Leu Pro Ala Pro Ile Glu Lys Thr Ile Ser Lys Ala Lys Gly Gln
            100                 105                 110

Pro Arg Glu Pro Gln Val Tyr Thr Leu Pro Pro Ser Arg Glu Glu Met
        115                 120                 125

Thr Lys Asn Gln Val Ser Leu Thr Cys Leu Val Lys Gly Phe Tyr Pro
    130                 135                 140

Ser Asp Ile Ala Val Glu Trp Glu Ser Asn Gly Gln Pro Glu Asn Asn
145                 150                 155                 160

Tyr Lys Thr Thr Pro Pro Val Leu Asp Ser Asp Gly Ser Phe Phe Leu
                165                 170                 175

Tyr Ser Lys Leu Thr Val Asp Lys Ser Arg Trp Gln Gln Gly Asn Val
            180                 185                 190

Phe Ser Cys Ser Val Met His Glu Ala Leu His Asn His Tyr Thr Gln
        195                 200                 205

Lys Ser Leu Cys Leu Ser Pro Gly Lys
    210                 215
```

<210> 80
<211> 217
<212> PRT
<213> Artificial Sequence

<220>

<221> source
<223> /note="Description of Artificial Sequence: Synthetic polypeptide"

<220>
<221> VARIANT
<222> (217)..(217)
<223> /replace=" "

<220>
<221> MISC_FEATURE
<222> (1)..(217)
<223> /note="Variant residues given in the sequence have no preference with respect to those in the annotations for variant positions"

<400> 80

```
Ala Pro Glu Ala Ala Gly Gly Pro Ser Val Phe Leu Phe Pro Pro Lys
1               5                   10                  15

Pro Lys Asp Thr Leu Met Ile Ser Arg Thr Pro Glu Val Thr Cys Val
            20                  25                  30

Val Val Asp Val Ser His Glu Asp Pro Glu Val Lys Phe Asn Trp Tyr
        35                  40                  45

Val Asp Gly Val Glu Val His Asn Ala Lys Thr Lys Pro Arg Glu Glu
    50                  55                  60

Gln Tyr Asn Ser Thr Tyr Arg Val Val Ser Val Leu Thr Val Leu His
65                  70                  75                  80

Gln Asp Trp Leu Asn Gly Lys Glu Tyr Lys Cys Lys Val Ser Asn Lys
                85                  90                  95

Ala Leu Pro Ala Pro Ile Glu Lys Thr Ile Ser Lys Ala Lys Gly Gln
            100                 105                 110

Pro Arg Glu Pro Gln Val Tyr Thr Leu Pro Pro Ser Arg Glu Glu Met
        115                 120                 125

Thr Lys Asn Gln Val Ser Leu Thr Cys Leu Val Lys Gly Phe Tyr Pro
    130                 135                 140
```

Ser Asp Ile Ala Val Glu Trp Glu Ser Asn Gly Gln Pro Glu Asn Asn
145 150 155 160

Tyr Lys Thr Thr Pro Pro Val Leu Asp Ser Asp Gly Ser Phe Phe Leu
165 170 175

Tyr Ser Lys Leu Thr Val Asp Lys Ser Arg Trp Gln Gln Gly Asn Val
180 185 190

Phe Ser Cys Ser Val Met His Glu Ala Leu His Asn His Tyr Thr Gln
195 200 205

Lys Ser Leu Cys Leu Ser Pro Gly Lys
210 215

<210> 81
<211> 6
<212> PRT
<213> Artificial Sequence

<220>
<221> source
<223> /note="Description of Artificial Sequence: Synthetic peptide"

<220>
<221> MISC_FEATURE
<222> (1)..(6)
<223> /note="Variant residues given in the sequence have no preference with respect to those in the annotations for variant positions"

<400> 81
Gly Ser Arg Asp Tyr Phe
1 5

<210> 82
<211> 6
<212> PRT
<213> Artificial Sequence

<220>
<221> source
<223> /note="Description of Artificial Sequence: Synthetic peptide"

<400> 82

Asp Gly Glu Gly Val Met
1 5

<210> 83
<211> 6
<212> PRT
<213> Artificial Sequence

<220>
<221> source

<223> /note="Description of Artificial Sequence: Synthetic peptide"

<400> 83

Phe His Ser Gly Leu Leu
1               5

<210> 84
<211> 6
<212> PRT
<213> Artificial Sequence

<220>
<221> source
<223> /note="Description of Artificial Sequence: Synthetic peptide"

<400> 84

Phe Asn Ser Ala Thr Leu
1               5

<210> 85
<211> 6
<212> PRT
<213> Artificial Sequence

<220>
<221> source
<223> /note="Description of Artificial Sequence: Synthetic peptide"

<400> 85

Phe Asn Ser Gly Thr Leu
1               5

<210> 86
<211> 6
<212> PRT
<213> Artificial Sequence

<220>
<221> source
<223> /note="Description of Artificial Sequence: Synthetic peptide"

<400> 86

Phe Asn Ser Ser Thr Leu
1               5

<210> 87
<211> 6
<212> PRT
<213> Artificial Sequence

<220>
<221> source
<223> /note="Description of Artificial Sequence: Synthetic peptide"

<400> 87

Gly Lys Ser Lys Trp Leu
1               5

<210> 88
<211> 6
<212> PRT
<213> Artificial Sequence

<220>
<221> source
<223> /note="Description of Artificial Sequence: Synthetic peptide"

<400> 88

Gly Met Gly Gly Thr Leu
1               5

<210> 89
<211> 6
<212> PRT
<213> Artificial Sequence

<220>
<221> source
<223> /note="Description of Artificial Sequence: Synthetic peptide"

<400> 89

His Ala Lys Gly Gly Met
1               5

<210> 90
<211> 6
<212> PRT
<213> Artificial Sequence

<220>
<221> source
<223> /note="Description of Artificial Sequence: Synthetic peptide"

<400> 90

Ile Gly Glu Ala Val Leu
1               5

<210> 91
<211> 6
<212> PRT
<213> Artificial Sequence

<220>
<221> source
<223> /note="Description of Artificial Sequence: Synthetic peptide"

<400> 91

Ile Gly Lys Gly Val Phe
1 5

<210> 92
<211> 6
<212> PRT
<213> Artificial Sequence

<220>
<221> source
<223> /note="Description of Artificial Sequence: Synthetic peptide"

<400> 92

Ile Gly Lys Gly Val Leu
1 5

<210> 93
<211> 6
<212> PRT
<213> Artificial Sequence

<220>
<221> source
<223> /note="Description of Artificial Sequence: Synthetic peptide"

<400> 93

Lys His Asp Ser Val Leu
1 5

<210> 94
<211> 6
<212> PRT
<213> Artificial Sequence

<220>
<221> source
<223> /note="Description of Artificial Sequence: Synthetic peptide"

<400> 94

Leu Asn Thr Ala Val Met
1 5

<210> 95
<211> 6
<212> PRT
<213> Artificial Sequence

<220>
<221> source
<223> /note="Description of Artificial Sequence: Synthetic peptide"

<400> 95

Asn Gly Glu Gly Thr Leu
1 5

<210> 96
<211> 6
<212> PRT
<213> Artificial Sequence

<220>
<221> source
<223> /note="Description of Artificial Sequence: Synthetic peptide"

<400> 96

Asn Gly Lys Asn Thr Leu
1               5

<210> 97
<211> 6
<212> PRT
<213> Artificial Sequence

<220>
<221> source
<223> /note="Description of Artificial Sequence: Synthetic peptide"

<400> 97

Asn Ser Ala Gly Ile Leu
1               5

<210> 98
<211> 6
<212> PRT
<213> Artificial Sequence

<220>
<221> source
<223> /note="Description of Artificial Sequence: Synthetic peptide"

<400> 98

Pro Lys Glu Gly Trp Met
1               5

<210> 99
<211> 6
<212> PRT
<213> Artificial Sequence

<220>
<221> source
<223> /note="Description of Artificial Sequence: Synthetic peptide"

<400> 99

Pro Lys Phe Gly Trp Lys
1               5

<210> 100
<211> 6

<212> PRT
<213> Artificial Sequence

<220>
<221> source
<223> /note="Description of Artificial Sequence: Synthetic peptide"

<400> 100

```
Pro Lys Met Gly Trp Val
1               5
```

<210> 101
<211> 6
<212> PRT
<213> Artificial Sequence

<220>
<221> source
<223> /note="Description of Artificial Sequence: Synthetic peptide"

<400> 101

```
Pro Arg Leu Gly His Leu
1               5
```

<210> 102
<211> 6
<212> PRT
<213> Artificial Sequence

<220>
<221> source
<223> /note="Description of Artificial Sequence: Synthetic peptide"

<400> 102

```
Pro Ser Phe Gly Trp Ala
1               5
```

<210> 103
<211> 6
<212> PRT
<213> Artificial Sequence

<220>
<221> source
<223> /note="Description of Artificial Sequence: Synthetic peptide"

<400> 103

```
Gln Ala Lys Gly Thr Met
1               5
```

<210> 104
<211> 6
<212> PRT
<213> Artificial Sequence

<220>
<221> source
<223> /note="Description of Artificial Sequence: Synthetic peptide"

<400> 104

**Arg Gly Met Gly Val Met**
**1 5**

<210> 105
<211> 6
<212> PRT
<213> Artificial Sequence

<220>
<221> source
<223> /note="Description of Artificial Sequence: Synthetic peptide"

<400> 105

**Arg Lys Glu Gly Trp Met**
**1 5**

<210> 106
<211> 6
<212> PRT
<213> Artificial Sequence

<220>
<221> source
<223> /note="Description of Artificial Sequence: Synthetic peptide"

<400> 106

**Thr Gly Lys Gly Val Leu**
**1 5**

<210> 107
<211> 6
<212> PRT
<213> Artificial Sequence

<220>
<221> source
<223> /note="Description of Artificial Sequence: Synthetic peptide"

<400> 107

**Thr Gly Met Gly Thr Leu**
**1 5**

<210> 108
<211> 6
<212> PRT
<213> Artificial Sequence

<220>
<221> source

<223> /note="Description of Artificial Sequence: Synthetic peptide"

<400> 108

```
Thr Gly Asn Gly Val Met
1               5
```

<210> 109
<211> 6
<212> PRT
<213> Artificial Sequence

<220>
<221> source
<223> /note="Description of Artificial Sequence: Synthetic peptide"

<400> 109

```
Trp Asn Ala Gly Thr Phe
1               5
```

<210> 110
<211> 6
<212> PRT
<213> Artificial Sequence

<220>
<221> source
<223> /note="Description of Artificial Sequence: Synthetic peptide"

<400> 110

```
Tyr His His Thr Pro Leu
1               5
```

<210> 111
<211> 6
<212> PRT
<213> Artificial Sequence

<220>
<221> source
<223> /note="Description of Artificial Sequence: Synthetic peptide"

<400> 111

```
Tyr Gln Ser Ala Thr Leu
1               5
```

<210> 112
<211> 6
<212> PRT
<213> Artificial Sequence

<220>
<221> source
<223> /note="Description of Artificial Sequence: Synthetic peptide"

<400> 112

Asp Gly Lys Ala Val Leu
1 5

<210> 113
<211> 6
<212> PRT
<213> Artificial Sequence

<220>
<221> source
<223> /note="Description of Artificial Sequence: Synthetic peptide"

<400> 113

Phe Asn Lys Ala Val Leu
1 5

<210> 114
<211> 6
<212> PRT
<213> Artificial Sequence

<220>
<221> source
<223> /note="Description of Artificial Sequence: Synthetic peptide"

<400> 114

Phe Asn Ser Gly Thr Trp
1 5

<210> 115
<211> 6
<212> PRT
<213> Artificial Sequence

<220>
<221> source
<223> /note="Description of Artificial Sequence: Synthetic peptide"

<400> 115

Phe Asn Thr Gly Val Phe
1 5

<210> 116
<211> 6
<212> PRT
<213> Artificial Sequence

<220>
<221> source
<223> /note="Description of Artificial Sequence: Synthetic peptide"

<400> 116

Gly Lys Ser Arg Phe His
1 5

<210> 117
<211> 6
<212> PRT
<213> Artificial Sequence

<220>
<221> source
<223> /note="Description of Artificial Sequence: Synthetic peptide"

<400> 117

Ile Gly Lys Asn Val Tyr
1 5

<210> 118
<211> 6
<212> PRT
<213> Artificial Sequence

<220>
<221> source
<223> /note="Description of Artificial Sequence: Synthetic peptide"

<400> 118

Met Gly Lys Gly Val Met
1 5

<210> 119
<211> 6
<212> PRT
<213> Artificial Sequence

<220>
<221> source
<223> /note="Description of Artificial Sequence: Synthetic peptide"

<400> 119

Asn Gly Glu Ser Val Phe
1 5

<210> 120
<211> 6
<212> PRT
<213> Artificial Sequence

<220>
<221> source
<223> /note="Description of Artificial Sequence: Synthetic peptide"

<400> 120

Pro Asp Phe Gly Trp Met
1 5

<210> 121
<211> 6
<212> PRT
<213> Artificial Sequence

<220>
<221> source
<223> /note="Description of Artificial Sequence: Synthetic peptide"

<400> 121

```
Pro Gly Ser Gly Val Met
1               5
```

<210> 122
<211> 6
<212> PRT
<213> Artificial Sequence

<220>
<221> source
<223> /note="Description of Artificial Sequence: Synthetic peptide"

<400> 122

```
Pro Lys Asp Ala Trp Leu
1               5
```

<210> 123
<211> 6
<212> PRT
<213> Artificial Sequence

<220>
<221> source
<223> /note="Description of Artificial Sequence: Synthetic peptide"

<400> 123

```
Pro Lys Phe Gly Trp Leu
1               5
```

<210> 124
<211> 6
<212> PRT
<213> Artificial Sequence

<220>
<221> source
<223> /note="Description of Artificial Sequence: Synthetic peptide"

<400> 124

```
Pro Lys Ile Gly Trp His
1               5
```

<210> 125
<211> 6

<212> PRT
<213> Artificial Sequence

<220>
<221> source
<223> /note="Description of Artificial Sequence: Synthetic peptide"

<400> 125

Pro Lys Met Gly Trp Ala
1 5

<210> 126
<211> 6
<212> PRT
<213> Artificial Sequence

<220>
<221> source
<223> /note="Description of Artificial Sequence: Synthetic peptide"

<400> 126

Pro Lys Met Gly Trp Met
1 5

<210> 127
<211> 6
<212> PRT
<213> Artificial Sequence

<220>
<221> source
<223> /note="Description of Artificial Sequence: Synthetic peptide"

<400> 127

Pro Gln Met Gly Trp Leu
1 5

<210> 128
<211> 6
<212> PRT
<213> Artificial Sequence

<220>
<221> source
<223> /note="Description of Artificial Sequence: Synthetic peptide"

<400> 128

Pro Arg Phe Gly Trp Leu
1 5

<210> 129
<211> 6
<212> PRT
<213> Artificial Sequence

<220>
<221> source
<223> /note="Description of Artificial Sequence: Synthetic peptide"

<400> 129

Pro Arg Met Gly Phe Leu
1 5

<210> 130
<211> 6
<212> PRT
<213> Artificial Sequence

<220>
<221> source
<223> /note="Description of Artificial Sequence: Synthetic peptide"

<400> 130

Pro Arg Met Gly Phe Met
1 5

<210> 131
<211> 6
<212> PRT
<213> Artificial Sequence

<220>
<221> source
<223> /note="Description of Artificial Sequence: Synthetic peptide"

<400> 131

Pro Ser Phe Gly Trp Met
1 5

<210> 132
<211> 6
<212> PRT
<213> Artificial Sequence

<220>
<221> source
<223> /note="Description of Artificial Sequence: Synthetic peptide"

<400> 132

Arg Arg Glu Gly Trp Met
1 5

<210> 133
<211> 6
<212> PRT
<213> Artificial Sequence

<220>

<221> source
<223> /note="Description of Artificial Sequence: Synthetic peptide"

<400> 133

```
Ser Gly Glu Gly Val Leu
1               5
```

<210> 134
<211> 6
<212> PRT
<213> Artificial Sequence

<220>
<221> source
<223> /note="Description of Artificial Sequence: Synthetic peptide"

<400> 134

```
Ser Gly Asn Gly Val Met
1               5
```

<210> 135
<211> 6
<212> PRT
<213> Artificial Sequence

<220>
<221> source
<223> /note="Description of Artificial Sequence: Synthetic peptide"

<400> 135

```
Val Gly Lys Ala Val Leu
1               5
```

<210> 136
<211> 6
<212> PRT
<213> Artificial Sequence

<220>
<221> source
<223> /note="Description of Artificial Sequence: Synthetic peptide"

<400> 136

```
Asp Ser Asn Ala Val Leu
1               5
```

<210> 137
<211> 6
<212> PRT
<213> Artificial Sequence

<220>
<221> source
<223> /note="Description of Artificial Sequence: Synthetic peptide"

<400> 137

Phe His Ser Gly Thr Leu
1               5

<210> 138
<211> 6
<212> PRT
<213> Artificial Sequence

<220>
<221> source
<223> /note="Description of Artificial Sequence: Synthetic peptide"

<400> 138

Gly Gly Ser Gly Val Leu
1               5

<210> 139
<211> 6
<212> PRT
<213> Artificial Sequence

<220>
<221> source
<223> /note="Description of Artificial Sequence: Synthetic peptide"

<400> 139

Pro Arg Gln Gly Phe Leu
1               5

<210> 140
<211> 6
<212> PRT
<213> Artificial Sequence

<220>
<221> source
<223> /note="Description of Artificial Sequence: Synthetic peptide"

<400> 140

Tyr Asn Ser Gly Thr Leu
1               5

<210> 141
<211> 453
<212> PRT
<213> Artificial Sequence

<220>
<221> source
<223> /note="Description of Artificial Sequence: Synthetic polypeptide"

<220>
<221> VARIANT

<222> (453)..(453)
<223> /replace=" "

<220>
<221> MISC_FEATURE
<222> (1)..(453)
<223> /note="Variant residues given in the sequence have no preference with respect to those in the annotations for variant positions"

<400> 141

Glu Val Gln Leu Val Glu Ser Gly Gly Gly Leu Val Lys Pro Gly Gly
1 5 10 15

Ser Leu Arg Leu Ser Cys Ala Ala Ser Gly Phe Thr Phe Ser Ser Tyr
20 25 30

Trp Met His Trp Val Arg Gln Ala Pro Gly Lys Gly Leu Glu Trp Val
35 40 45

Gly Glu Ile Ile Pro Ile Phe Gly His Thr Asn Tyr Asn Glu Lys Phe
50 55 60

Lys Ser Arg Phe Thr Ile Ser Leu Asp Asn Ser Lys Asn Thr Leu Tyr
65 70 75 80

Leu Gln Met Gly Ser Leu Arg Ala Glu Asp Thr Ala Val Tyr Tyr Cys
85 90 95

Ala Arg Gly Gly Tyr Tyr Tyr Tyr Pro Arg Gln Gly Phe Leu Asp Tyr
100 105 110

Trp Gly Gln Gly Thr Thr Val Thr Val Ser Ser Ala Ser Thr Lys Gly
115 120 125

Pro Ser Val Phe Pro Leu Ala Pro Ser Ser Lys Ser Thr Ser Gly Gly
130 135 140

Thr Ala Ala Leu Gly Cys Leu Val Lys Asp Tyr Phe Pro Glu Pro Val
145 150 155 160

Thr Val Ser Trp Asn Ser Gly Ala Leu Thr Ser Gly Val His Thr Phe
165 170 175

Pro Ala Val Leu Gln Ser Ser Gly Leu Tyr Ser Leu Ser Ser Val Val
180 185 190

Thr Val Pro Ser Ser Ser Leu Gly Thr Gln Thr Tyr Ile Cys Asn Val
195 200 205

Asn His Lys Pro Ser Asn Thr Lys Val Asp Lys Arg Val Glu Pro Lys
210 215 220

Ser Cys Asp Lys Thr His Thr Cys Pro Pro Cys Pro Ala Pro Glu Ala
225 230 235 240

Ala Gly Gly Pro Ser Val Phe Leu Phe Pro Pro Lys Pro Lys Asp Thr
245 250 255

Leu Met Ile Ser Arg Thr Pro Glu Val Thr Cys Val Val Val Asp Val
260 265 270

Ser His Glu Asp Pro Glu Val Lys Phe Asn Trp Tyr Val Asp Gly Val
275 280 285

Glu Val His Asn Ala Lys Thr Lys Pro Arg Glu Glu Gln Tyr Asn Ser
290 295 300

Thr Tyr Arg Val Val Ser Val Leu Thr Val Leu His Gln Asp Trp Leu
305 310 315 320

Asn Gly Lys Glu Tyr Lys Cys Lys Val Ser Asn Lys Ala Leu Pro Ala
325 330 335

Pro Ile Glu Lys Thr Ile Ser Lys Ala Lys Gly Gln Pro Arg Glu Pro
340 345 350

Gln Val Tyr Thr Leu Pro Pro Ser Arg Glu Glu Met Thr Lys Asn Gln
355 360 365

Val Ser Leu Thr Cys Leu Val Lys Gly Phe Tyr Pro Ser Asp Ile Ala
370 375 380

Val Glu Trp Glu Ser Asn Gly Gln Pro Glu Asn Asn Tyr Lys Thr Thr
385 390 395 400

Pro Pro Val Leu Asp Ser Asp Gly Ser Phe Phe Leu Tyr Ser Lys Leu
405 410 415

Thr Val Asp Lys Ser Arg Trp Gln Gln Gly Asn Val Phe Ser Cys Ser
420 425 430

Val Met His Glu Ala Leu His Asn His Tyr Thr Gln Lys Ser Leu Ser
435 440 445

Leu Ser Pro Gly Lys
450

<210> 142
<211> 453

<212> PRT
<213> Artificial Sequence

<220>
<221> source
<223> /note="Description of Artificial Sequence: Synthetic polypeptide"

<220>
<221> VARIANT
<222> (453)..(453)
<223> /replace=" "

<220>
<221> MISC_FEATURE
<222> (1)..(453)
<223> /note="Variant residues given in the sequence have no preference with respect to those in the annotations for variant positions"

<400> 142

```
Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Trp Met His Trp Val Arg Gln Ala Pro Gly Lys Gly Leu Glu Trp Val
        35                  40                  45

Gly Glu Ile Ile Pro Ile Phe Gly His Thr Asn Tyr Asn Glu Lys Phe
    50                  55                  60

Lys Ser Arg Phe Thr Ile Ser Leu Asp Asn Ser Lys Asn Thr Leu Tyr
65                  70                  75                  80

Leu Gln Met Gly Ser Leu Arg Ala Glu Asp Thr Ala Val Tyr Tyr Cys
                85                  90                  95

Ala Arg Gly Gly Tyr Tyr Tyr Tyr Pro Arg Gln Gly Phe Leu Asp Tyr
            100                 105                 110

Trp Gly Gln Gly Thr Thr Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
```

165 170 175

Pro Ala Val Leu Gln Ser Ser Gly Leu Tyr Ser Leu Ser Ser Val Val
180 185 190

Thr Val Pro Ser Ser Ser Leu Gly Thr Gln Thr Tyr Ile Cys Asn Val
195 200 205

Asn His Lys Pro Ser Asn Thr Lys Val Asp Lys Arg Val Glu Pro Lys
210 215 220

Ser Cys Asp Lys Thr His Thr Cys Pro Pro Cys Pro Ala Pro Glu Leu
225 230 235 240

Leu Gly Gly Pro Ser Val Phe Leu Phe Pro Pro Lys Pro Lys Asp Thr
245 250 255

Leu Met Ile Ser Arg Thr Pro Glu Val Thr Cys Val Val Val Asp Val
260 265 270

Ser His Glu Asp Pro Glu Val Lys Phe Asn Trp Tyr Val Asp Gly Val
275 280 285

Glu Val His Asn Ala Lys Thr Lys Pro Arg Glu Glu Gln Tyr Asn Ser
290 295 300

Thr Tyr Arg Val Val Ser Val Leu Thr Val Leu His Gln Asp Trp Leu
305 310 315 320

Asn Gly Lys Glu Tyr Lys Cys Lys Val Ser Asn Lys Ala Leu Pro Ala
325 330 335

Pro Ile Glu Lys Thr Ile Ser Lys Ala Lys Gly Gln Pro Arg Glu Pro
340 345 350

Gln Val Tyr Thr Leu Pro Pro Ser Arg Glu Glu Met Thr Lys Asn Gln
355 360 365

Val Ser Leu Thr Cys Leu Val Lys Gly Phe Tyr Pro Ser Asp Ile Ala
370 375 380

Val Glu Trp Glu Ser Asn Gly Gln Pro Glu Asn Asn Tyr Lys Thr Thr
385 390 395 400

Pro Pro Val Leu Asp Ser Asp Gly Ser Phe Phe Leu Tyr Ser Lys Leu
405 410 415

Thr Val Asp Lys Ser Arg Trp Gln Gln Gly Asn Val Phe Ser Cys Ser
420 425 430

Val Met His Glu Ala Leu His Asn His Tyr Thr Gln Lys Ser Leu Cys
435 440 445

Leu Ser Pro Gly Lys
450

<210> 143
<211> 453
<212> PRT
<213> Artificial Sequence

<220>
<221> source
<223> /note="Description of Artificial Sequence: Synthetic polypeptide"

<220>
<221> VARIANT
<222> (453)..(453)
<223> /replace=" "

<220>
<221> MISC_FEATURE
<222> (1)..(453)
<223> /note="Variant residues given in the sequence have no preference with respect to those in the annotations for variant positions"

<400> 143

Glu Val Gln Leu Val Glu Ser Gly Gly Gly Leu Val Lys Pro Gly Gly
1 5 10 15

Ser Leu Arg Leu Ser Cys Ala Ala Ser Gly Phe Thr Phe Ser Ser Tyr
20 25 30

Trp Met His Trp Val Arg Gln Ala Pro Gly Lys Gly Leu Glu Trp Val
35 40 45

Gly Glu Ile Ile Pro Ile Phe Gly His Thr Asn Tyr Asn Glu Lys Phe
50 55 60

Lys Ser Arg Phe Thr Ile Ser Leu Asp Asn Ser Lys Asn Thr Leu Tyr
65 70 75 80

Leu Gln Met Gly Ser Leu Arg Ala Glu Asp Thr Ala Val Tyr Tyr Cys
85 90 95

Ala Arg Gly Gly Tyr Tyr Tyr Tyr Pro Arg Gln Gly Phe Leu Asp Tyr
100 105 110

```
Trp Gly Gln Gly Thr Thr Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Arg Val Glu Pro Lys
    210                 215                 220

Ser Cys Asp Lys Thr His Thr Cys Pro Pro Cys Pro Ala Pro Glu Ala
225                 230                 235                 240

Ala Gly Gly Pro Ser Val Phe Leu Phe Pro Pro Lys Pro Lys Asp Thr
                245                 250                 255

Leu Met Ile Ser Arg Thr Pro Glu Val Thr Cys Val Val Val Asp Val
            260                 265                 270

Ser His Glu Asp Pro Glu Val Lys Phe Asn Trp Tyr Val Asp Gly Val
        275                 280                 285

Glu Val His Asn Ala Lys Thr Lys Pro Arg Glu Glu Gln Tyr Asn Ser
    290                 295                 300

Thr Tyr Arg Val Val Ser Val Leu Thr Val Leu His Gln Asp Trp Leu
305                 310                 315                 320

Asn Gly Lys Glu Tyr Lys Cys Lys Val Ser Asn Lys Ala Leu Pro Ala
                325                 330                 335

Pro Ile Glu Lys Thr Ile Ser Lys Ala Lys Gly Gln Pro Arg Glu Pro
            340                 345                 350

Gln Val Tyr Thr Leu Pro Pro Ser Arg Glu Glu Met Thr Lys Asn Gln
        355                 360                 365
```

```
Val Ser Leu Thr Cys Leu Val Lys Gly Phe Tyr Pro Ser Asp Ile Ala
    370                 375                 380

Val Glu Trp Glu Ser Asn Gly Gln Pro Glu Asn Asn Tyr Lys Thr Thr
385                 390                 395                 400

Pro Pro Val Leu Asp Ser Asp Gly Ser Phe Phe Leu Tyr Ser Lys Leu
                405                 410                 415

Thr Val Asp Lys Ser Arg Trp Gln Gln Gly Asn Val Phe Ser Cys Ser
            420                 425                 430

Val Met His Glu Ala Leu His Asn His Tyr Thr Gln Lys Ser Leu Cys
        435                 440                 445

Leu Ser Pro Gly Lys
    450
```

<210> 144
<211> 4
<212> PRT
<213> Artificial Sequence

<220>
<221> source
<223> /note="Description of Artificial Sequence: Synthetic peptide"

<400> 144
Ala Leu Ala Leu

1

<210> 145
<211> 4
<212> PRT
<213> Artificial Sequence

<220>
<221> source
<223> /note="Description of Artificial Sequence: Synthetic peptide"

<220>
<221> MOD_RES
<222> (1)..(1)
<223> Beta-Ala

<400> 145
Ala Leu Ala Leu

1

<210> 146
<211> 4
<212> PRT

<213> Artificial Sequence

<220>
<221> source
<223> /note="Description of Artificial Sequence: Synthetic peptide"

<400> 146

```
Gly Phe Leu Gly
1
```

<210> 147
<211> 217
<212> PRT
<213> Artificial Sequence

<220>
<221> source
<223> /note="Description of Artificial Sequence: Synthetic polypeptide"

<220>
<221> VARIANT
<222> (217)..(217)
<223> /replace=" "

<220>
<221> MISC_FEATURE
<222> (1)..(217)
<223> /note="Variant residues given in the sequence have no preference with respect to those in the annotations for variant positions"

<400> 147

```
Ala Pro Glu Leu Leu Gly Gly Pro Ser Val Phe Leu Phe Pro Pro Lys
1               5                   10                  15

Pro Lys Asp Thr Leu Tyr Ile Thr Arg Glu Pro Glu Val Thr Cys Val
            20                  25                  30

Val Val Asp Val Ser His Glu Asp Pro Glu Val Lys Phe Asn Trp Tyr
        35                  40                  45

Val Asp Gly Val Glu Val His Asn Ala Lys Thr Lys Pro Arg Glu Glu
    50                  55                  60

Gln Tyr Asn Ser Thr Tyr Arg Val Val Ser Val Leu Thr Val Leu His
65                  70                  75                  80

Gln Asp Trp Leu Asn Gly Lys Glu Tyr Lys Cys Lys Val Ser Asn Lys
                85                  90                  95

Ala Leu Pro Ala Pro Ile Glu Lys Thr Ile Ser Lys Ala Lys Gly Gln
```

100 105 110

Pro Arg Glu Pro Gln Val Tyr Thr Leu Pro Pro Ser Arg Glu Glu Met
115 120 125

Thr Lys Asn Gln Val Ser Leu Thr Cys Leu Val Lys Gly Phe Tyr Pro
130 135 140

Ser Asp Ile Ala Val Glu Trp Glu Ser Asn Gly Gln Pro Glu Asn Asn
145 150 155 160

Tyr Lys Thr Thr Pro Pro Val Leu Asp Ser Asp Gly Ser Phe Phe Leu
165 170 175

Tyr Ser Lys Leu Thr Val Asp Lys Ser Arg Trp Gln Gln Gly Asn Val
180 185 190

Phe Ser Cys Ser Val Met His Glu Ala Leu His Asn His Tyr Thr Gln
195 200 205

Lys Ser Leu Ser Leu Ser Pro Gly Lys
210 215

<210> 148
<211> 217
<212> PRT
<213> Artificial Sequence

<220>
<221> source
<223> /note="Description of Artificial Sequence: Synthetic polypeptide"

<220>
<221> VARIANT
<222> (217)..(217)
<223> /replace=" "

<220>
<221> MISC_FEATURE
<222> (1)..(217)
<223> /note="Variant residues given in the sequence have no preference with respect to those in the annotations for variant positions"

<400> 148

Ala Pro Glu Leu Leu Gly Gly Pro Ser Val Phe Leu Phe Pro Pro Lys
1 5 10 15

Pro Lys Asp Thr Leu Tyr Ile Thr Arg Glu Pro Glu Val Thr Cys Val
20 25 30

```
Val Val Asp Val Ser His Glu Asp Pro Glu Val Lys Phe Asn Trp Tyr
        35                  40                  45

Val Asp Gly Val Glu Val His Asn Ala Lys Thr Lys Pro Arg Glu Glu
    50                  55                  60

Gln Tyr Asn Ser Thr Tyr Arg Val Val Ser Val Leu Thr Val Leu His
65                  70                  75                  80

Gln Asp Trp Leu Asn Gly Lys Glu Tyr Lys Cys Lys Val Ser Asn Lys
                85                  90                  95

Ala Leu Pro Ala Pro Ile Glu Lys Thr Ile Ser Lys Ala Lys Gly Gln
            100                 105                 110

Pro Arg Glu Pro Gln Val Tyr Thr Leu Pro Pro Ser Arg Glu Glu Met
        115                 120                 125

Thr Lys Asn Gln Val Ser Leu Thr Cys Leu Val Lys Gly Phe Tyr Pro
    130                 135                 140

Ser Asp Ile Ala Val Glu Trp Glu Ser Asn Gly Gln Pro Glu Asn Asn
145                 150                 155                 160

Tyr Lys Thr Thr Pro Pro Val Leu Asp Ser Asp Gly Ser Phe Phe Leu
                165                 170                 175

Tyr Ser Lys Leu Thr Val Asp Lys Ser Arg Trp Gln Gln Gly Asn Val
            180                 185                 190

Phe Ser Cys Ser Val Met His Glu Ala Leu His Asn His Tyr Thr Gln
        195                 200                 205

Lys Ser Leu Cys Leu Ser Pro Gly Lys
    210                 215
```

<210> 149
<211> 217
<212> PRT
<213> Artificial Sequence

<220>
<221> source
<223> /note="Description of Artificial Sequence: Synthetic polypeptide"

<220>
<221> VARIANT
<222> (217)..(217)
<223> /replace=" "

<220>
<221> MISC_FEATURE
<222> (1)..(217)
<223> /note="Variant residues given in the sequence have no preference with respect to those in the annotations for variant positions"

<400> 149

```
Ala Pro Glu Ala Ala Gly Gly Pro Ser Val Phe Leu Phe Pro Pro Lys
1               5                   10                  15

Pro Lys Asp Thr Leu Tyr Ile Thr Arg Glu Pro Glu Val Thr Cys Val
            20                  25                  30

Val Val Asp Val Ser His Glu Asp Pro Glu Val Lys Phe Asn Trp Tyr
        35                  40                  45

Val Asp Gly Val Glu Val His Asn Ala Lys Thr Lys Pro Arg Glu Glu
    50                  55                  60

Gln Tyr Asn Ser Thr Tyr Arg Val Val Ser Val Leu Thr Val Leu His
65                  70                  75                  80

Gln Asp Trp Leu Asn Gly Lys Glu Tyr Lys Cys Lys Val Ser Asn Lys
                85                  90                  95

Ala Leu Pro Ala Pro Ile Glu Lys Thr Ile Ser Lys Ala Lys Gly Gln
            100                 105                 110

Pro Arg Glu Pro Gln Val Tyr Thr Leu Pro Pro Ser Arg Glu Glu Met
        115                 120                 125

Thr Lys Asn Gln Val Ser Leu Thr Cys Leu Val Lys Gly Phe Tyr Pro
    130                 135                 140

Ser Asp Ile Ala Val Glu Trp Glu Ser Asn Gly Gln Pro Glu Asn Asn
145                 150                 155                 160

Tyr Lys Thr Thr Pro Pro Val Leu Asp Ser Asp Gly Ser Phe Phe Leu
                165                 170                 175

Tyr Ser Lys Leu Thr Val Asp Lys Ser Arg Trp Gln Gln Gly Asn Val
            180                 185                 190

Phe Ser Cys Ser Val Met His Glu Ala Leu His Asn His Tyr Thr Gln
        195                 200                 205

Lys Ser Leu Ser Leu Ser Pro Gly Lys
    210                 215
```

<210> 150
<211> 217
<212> PRT
<213> Artificial Sequence

<220>

<221> source
<223> /note="Description of Artificial Sequence: Synthetic polypeptide"

<220>
<221> VARIANT
<222> (217)..(217)
<223> /replace=" "

<220>
<221> MISC_FEATURE
<222> (1)..(217)
<223> /note="Variant residues given in the sequence have no preference with respect to those in the annotations for variant positions"

<400> 150

```
Ala Pro Glu Ala Ala Gly Gly Pro Ser Val Phe Leu Phe Pro Pro Lys
1               5                   10                  15

Pro Lys Asp Thr Leu Tyr Ile Thr Arg Glu Pro Glu Val Thr Cys Val
            20                  25                  30

Val Val Asp Val Ser His Glu Asp Pro Glu Val Lys Phe Asn Trp Tyr
        35                  40                  45

Val Asp Gly Val Glu Val His Asn Ala Lys Thr Lys Pro Arg Glu Glu
    50                  55                  60

Gln Tyr Asn Ser Thr Tyr Arg Val Val Ser Val Leu Thr Val Leu His
65                  70                  75                  80

Gln Asp Trp Leu Asn Gly Lys Glu Tyr Lys Cys Lys Val Ser Asn Lys
                85                  90                  95

Ala Leu Pro Ala Pro Ile Glu Lys Thr Ile Ser Lys Ala Lys Gly Gln
            100                 105                 110

Pro Arg Glu Pro Gln Val Tyr Thr Leu Pro Pro Ser Arg Glu Glu Met
        115                 120                 125

Thr Lys Asn Gln Val Ser Leu Thr Cys Leu Val Lys Gly Phe Tyr Pro
    130                 135                 140

Ser Asp Ile Ala Val Glu Trp Glu Ser Asn Gly Gln Pro Glu Asn Asn
```

```
145                 150                 155                 160

Tyr Lys Thr Thr Pro Pro Val Leu Asp Ser Asp Gly Ser Phe Phe Leu
                165                 170                 175

Tyr Ser Lys Leu Thr Val Asp Lys Ser Arg Trp Gln Gln Gly Asn Val
            180                 185                 190

Phe Ser Cys Ser Val Met His Glu Ala Leu His Asn His Tyr Thr Gln
        195                 200                 205

Lys Ser Leu Cys Leu Ser Pro Gly Lys
    210                 215
```

<210> 151
<211> 453
<212> PRT
<213> Artificial Sequence

<220>
<221> source
<223> /note="Description of Artificial Sequence: Synthetic polypeptide"

<220>
<221> VARIANT
<222> (453)..(453)
<223> /replace=" "

<220>
<221> MISC_FEATURE
<222> (1)..(453)
<223> /note="Variant residues given in the sequence have no preference with respect to those in the annotations for variant positions"

<400> 151

```
Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Trp Met His Trp Val Arg Gln Ala Pro Gly Lys Gly Leu Glu Trp Val
        35                  40                  45

Gly Glu Ile Ile Pro Ile Phe Gly His Thr Asn Tyr Asn Glu Lys Phe
    50                  55                  60

Lys Ser Arg Phe Thr Ile Ser Leu Asp Asn Ser Lys Asn Thr Leu Tyr
65                  70                  75                  80
```

```
Leu Gln Met Gly Ser Leu Arg Ala Glu Asp Thr Ala Val Tyr Tyr Cys
                85                  90                  95

Ala Arg Gly Gly Tyr Tyr Tyr Tyr Pro Arg Gln Gly Phe Leu Asp Tyr
            100                 105                 110

Trp Gly Gln Gly Thr Thr Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Arg Val Glu Pro Lys
    210                 215                 220

Ser Cys Asp Lys Thr His Thr Cys Pro Pro Cys Pro Ala Pro Glu Leu
225                 230                 235                 240

Leu Gly Gly Pro Ser Val Phe Leu Phe Pro Pro Lys Pro Lys Asp Thr
                245                 250                 255

Leu Tyr Ile Thr Arg Glu Pro Glu Val Thr Cys Val Val Val Asp Val
            260                 265                 270

Ser His Glu Asp Pro Glu Val Lys Phe Asn Trp Tyr Val Asp Gly Val
        275                 280                 285

Glu Val His Asn Ala Lys Thr Lys Pro Arg Glu Glu Gln Tyr Asn Ser
    290                 295                 300

Thr Tyr Arg Val Val Ser Val Leu Thr Val Leu His Gln Asp Trp Leu
305                 310                 315                 320

Asn Gly Lys Glu Tyr Lys Cys Lys Val Ser Asn Lys Ala Leu Pro Ala
                325                 330                 335
```

```
Pro Ile Glu Lys Thr Ile Ser Lys Ala Lys Gly Gln Pro Arg Glu Pro
            340                 345                 350

Gln Val Tyr Thr Leu Pro Pro Ser Arg Glu Glu Met Thr Lys Asn Gln
        355                 360                 365

Val Ser Leu Thr Cys Leu Val Lys Gly Phe Tyr Pro Ser Asp Ile Ala
    370                 375                 380

Val Glu Trp Glu Ser Asn Gly Gln Pro Glu Asn Asn Tyr Lys Thr Thr
385                 390                 395                 400

Pro Pro Val Leu Asp Ser Asp Gly Ser Phe Phe Leu Tyr Ser Lys Leu
                405                 410                 415

Thr Val Asp Lys Ser Arg Trp Gln Gln Gly Asn Val Phe Ser Cys Ser
            420                 425                 430

Val Met His Glu Ala Leu His Asn His Tyr Thr Gln Lys Ser Leu Ser
        435                 440                 445

Leu Ser Pro Gly Lys
    450
```

<210> 152
<211> 453
<212> PRT
<213> Artificial Sequence

<220>
<221> source
<223> /note="Description of Artificial Sequence: Synthetic polypeptide"

<220>
<221> VARIANT
<222> (453)..(453)
<223> /replace=" "

<220>
<221> MISC_FEATURE
<222> (1)..(453)
<223> /note="Variant residues given in the sequence have no preference with respect to those in the annotations for variant positions"

<400> 152

```
Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30
```

Trp Met His Trp Val Arg Gln Ala Pro Gly Lys Gly Leu Glu Trp Val
35 40 45

Gly Glu Ile Ile Pro Ile Phe Gly His Thr Asn Tyr Asn Glu Lys Phe
50 55 60

Lys Ser Arg Phe Thr Ile Ser Leu Asp Asn Ser Lys Asn Thr Leu Tyr
65 70 75 80

Leu Gln Met Gly Ser Leu Arg Ala Glu Asp Thr Ala Val Tyr Tyr Cys
85 90 95

Ala Arg Gly Gly Tyr Tyr Tyr Tyr Pro Arg Gln Gly Phe Leu Asp Tyr
100 105 110

Trp Gly Gln Gly Thr Thr Val Thr Val Ser Ser Ala Ser Thr Lys Gly
115 120 125

Pro Ser Val Phe Pro Leu Ala Pro Ser Ser Lys Ser Thr Ser Gly Gly
130 135 140

Thr Ala Ala Leu Gly Cys Leu Val Lys Asp Tyr Phe Pro Glu Pro Val
145 150 155 160

Thr Val Ser Trp Asn Ser Gly Ala Leu Thr Ser Gly Val His Thr Phe
165 170 175

Pro Ala Val Leu Gln Ser Ser Gly Leu Tyr Ser Leu Ser Ser Val Val
180 185 190

Thr Val Pro Ser Ser Ser Leu Gly Thr Gln Thr Tyr Ile Cys Asn Val
195 200 205

Asn His Lys Pro Ser Asn Thr Lys Val Asp Lys Arg Val Glu Pro Lys
210 215 220

Ser Cys Asp Lys Thr His Thr Cys Pro Pro Cys Pro Ala Pro Glu Leu
225 230 235 240

Leu Gly Gly Pro Ser Val Phe Leu Phe Pro Pro Lys Pro Lys Asp Thr
245 250 255

Leu Tyr Ile Thr Arg Glu Pro Glu Val Thr Cys Val Val Val Asp Val
260 265 270

Ser His Glu Asp Pro Glu Val Lys Phe Asn Trp Tyr Val Asp Gly Val

275 280 285

Glu Val His Asn Ala Lys Thr Lys Pro Arg Glu Glu Gln Tyr Asn Ser
290 295 300

Thr Tyr Arg Val Val Ser Val Leu Thr Val Leu His Gln Asp Trp Leu
305 310 315 320

Asn Gly Lys Glu Tyr Lys Cys Lys Val Ser Asn Lys Ala Leu Pro Ala
325 330 335

Pro Ile Glu Lys Thr Ile Ser Lys Ala Lys Gly Gln Pro Arg Glu Pro
340 345 350

Gln Val Tyr Thr Leu Pro Pro Ser Arg Glu Glu Met Thr Lys Asn Gln
355 360 365

Val Ser Leu Thr Cys Leu Val Lys Gly Phe Tyr Pro Ser Asp Ile Ala
370 375 380

Val Glu Trp Glu Ser Asn Gly Gln Pro Glu Asn Asn Tyr Lys Thr Thr
385 390 395 400

Pro Pro Val Leu Asp Ser Asp Gly Ser Phe Phe Leu Tyr Ser Lys Leu
405 410 415

Thr Val Asp Lys Ser Arg Trp Gln Gln Gly Asn Val Phe Ser Cys Ser
420 425 430

Val Met His Glu Ala Leu His Asn His Tyr Thr Gln Lys Ser Leu Cys
435 440 445

Leu Ser Pro Gly Lys
450

<210> 153
<211> 453
<212> PRT
<213> Artificial Sequence

<220>
<221> source
<223> /note="Description of Artificial Sequence: Synthetic polypeptide"

<220>
<221> VARIANT
<222> (453)..(453)
<223> /replace=" "

<220>
<221> MISC_FEATURE
<222> (1)..(453)
<223> /note="Variant residues given in the sequence have no preference with respect to those in the annotations for variant positions"

<400> 153

```
Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Trp Met His Trp Val Arg Gln Ala Pro Gly Lys Gly Leu Glu Trp Val
        35                  40                  45

Gly Glu Ile Ile Pro Ile Phe Gly His Thr Asn Tyr Asn Glu Lys Phe
    50                  55                  60

Lys Ser Arg Phe Thr Ile Ser Leu Asp Asn Ser Lys Asn Thr Leu Tyr
65                  70                  75                  80

Leu Gln Met Gly Ser Leu Arg Ala Glu Asp Thr Ala Val Tyr Tyr Cys
                85                  90                  95

Ala Arg Gly Gly Tyr Tyr Tyr Tyr Pro Arg Gln Gly Phe Leu Asp Tyr
            100                 105                 110

Trp Gly Gln Gly Thr Thr Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
                165                 170                 175

Pro Ala Val Leu Gln Ser Ser Gly Leu Tyr Ser Leu Ser Ser Val Val
            180                 185                 190

Thr Val Pro Ser Ser Ser Leu Gly Thr Gln Thr Tyr Ile Cys Asn Val
        195                 200                 205

Asn His Lys Pro Ser Asn Thr Lys Val Asp Lys Arg Val Glu Pro Lys
    210                 215                 220
```

Ser Cys Asp Lys Thr His Thr Cys Pro Pro Cys Pro Ala Pro Glu Ala
225 230 235 240

Ala Gly Gly Pro Ser Val Phe Leu Phe Pro Pro Lys Pro Lys Asp Thr
245 250 255

Leu Tyr Ile Thr Arg Glu Pro Glu Val Thr Cys Val Val Val Asp Val
260 265 270

Ser His Glu Asp Pro Glu Val Lys Phe Asn Trp Tyr Val Asp Gly Val
275 280 285

Glu Val His Asn Ala Lys Thr Lys Pro Arg Glu Glu Gln Tyr Asn Ser
290 295 300

Thr Tyr Arg Val Val Ser Val Leu Thr Val Leu His Gln Asp Trp Leu
305 310 315 320

Asn Gly Lys Glu Tyr Lys Cys Lys Val Ser Asn Lys Ala Leu Pro Ala
325 330 335

Pro Ile Glu Lys Thr Ile Ser Lys Ala Lys Gly Gln Pro Arg Glu Pro
340 345 350

Gln Val Tyr Thr Leu Pro Pro Ser Arg Glu Glu Met Thr Lys Asn Gln
355 360 365

Val Ser Leu Thr Cys Leu Val Lys Gly Phe Tyr Pro Ser Asp Ile Ala
370 375 380

Val Glu Trp Glu Ser Asn Gly Gln Pro Glu Asn Asn Tyr Lys Thr Thr
385 390 395 400

Pro Pro Val Leu Asp Ser Asp Gly Ser Phe Phe Leu Tyr Ser Lys Leu
405 410 415

Thr Val Asp Lys Ser Arg Trp Gln Gln Gly Asn Val Phe Ser Cys Ser
420 425 430

Val Met His Glu Ala Leu His Asn His Tyr Thr Gln Lys Ser Leu Ser
435 440 445

Leu Ser Pro Gly Lys
450

<210> 154
<211> 453

<212> PRT
<213> Artificial Sequence

<220>
<221> source
<223> /note="Description of Artificial Sequence: Synthetic polypeptide"

<220>
<221> VARIANT
<222> (453)..(453)
<223> /replace="

<220>
<221> MISC_FEATURE
<222> (1)..(453)
<223> /note="Variant residues given in the sequence have no preference with respect to those in the annotations for variant positions"

<400> 154

```
Glu Val Gln Leu Val Glu Ser Gly Gly Gly Leu Val Lys Pro Gly Gly
1               5                   10                  15

Ser Leu Arg Leu Ser Cys Ala Ala Ser Gly Phe Thr Phe Ser Ser Tyr
            20                  25                  30

Trp Met His Trp Val Arg Gln Ala Pro Gly Lys Gly Leu Glu Trp Val
        35                  40                  45

Gly Glu Ile Ile Pro Ile Phe Gly His Thr Asn Tyr Asn Glu Lys Phe
    50                  55                  60

Lys Ser Arg Phe Thr Ile Ser Leu Asp Asn Ser Lys Asn Thr Leu Tyr
65                  70                  75                  80

Leu Gln Met Gly Ser Leu Arg Ala Glu Asp Thr Ala Val Tyr Tyr Cys
                85                  90                  95

Ala Arg Gly Gly Tyr Tyr Tyr Tyr Pro Arg Gln Gly Phe Leu Asp Tyr
            100                 105                 110

Trp Gly Gln Gly Thr Thr Val Thr Val Ser Ser Ala Ser Thr Lys Gly
        115                 120                 125

Pro Ser Val Phe Pro Leu Ala Pro Ser Ser Lys Ser Thr Ser Gly Gly
    130                 135                 140

Thr Ala Ala Leu Gly Cys Leu Val Lys Asp Tyr Phe Pro Glu Pro Val
145                 150                 155                 160

Thr Val Ser Trp Asn Ser Gly Ala Leu Thr Ser Gly Val His Thr Phe
```

165 170 175

Pro Ala Val Leu Gln Ser Ser Gly Leu Tyr Ser Leu Ser Ser Val Val
180 185 190

Thr Val Pro Ser Ser Ser Leu Gly Thr Gln Thr Tyr Ile Cys Asn Val
195 200 205

Asn His Lys Pro Ser Asn Thr Lys Val Asp Lys Arg Val Glu Pro Lys
210 215 220

Ser Cys Asp Lys Thr His Thr Cys Pro Pro Cys Pro Ala Pro Glu Ala
225 230 235 240

Ala Gly Gly Pro Ser Val Phe Leu Phe Pro Pro Lys Pro Lys Asp Thr
245 250 255

Leu Tyr Ile Thr Arg Glu Pro Glu Val Thr Cys Val Val Val Asp Val
260 265 270

Ser His Glu Asp Pro Glu Val Lys Phe Asn Trp Tyr Val Asp Gly Val
275 280 285

Glu Val His Asn Ala Lys Thr Lys Pro Arg Glu Glu Gln Tyr Asn Ser
290 295 300

Thr Tyr Arg Val Val Ser Val Leu Thr Val Leu His Gln Asp Trp Leu
305 310 315 320

Asn Gly Lys Glu Tyr Lys Cys Lys Val Ser Asn Lys Ala Leu Pro Ala
325 330 335

Pro Ile Glu Lys Thr Ile Ser Lys Ala Lys Gly Gln Pro Arg Glu Pro
340 345 350

Gln Val Tyr Thr Leu Pro Pro Ser Arg Glu Glu Met Thr Lys Asn Gln
355 360 365

Val Ser Leu Thr Cys Leu Val Lys Gly Phe Tyr Pro Ser Asp Ile Ala
370 375 380

Val Glu Trp Glu Ser Asn Gly Gln Pro Glu Asn Asn Tyr Lys Thr Thr
385 390 395 400

Pro Pro Val Leu Asp Ser Asp Gly Ser Phe Phe Leu Tyr Ser Lys Leu
405 410 415

```
Thr Val Asp Lys Ser Arg Trp Gln Gln Gly Asn Val Phe Ser Cys Ser
            420                 425                 430

Val Met His Glu Ala Leu His Asn His Tyr Thr Gln Lys Ser Leu Cys
        435                 440                 445

Leu Ser Pro Gly Lys
    450
```